(19) Europäisches Patentamt
European Patent Office
Office européen des brevets

(11) EP 2 623 584 B1

(12) **EUROPÄISCHE PATENTSCHRIFT**

(45) Veröffentlichungstag und Bekanntmachung des Hinweises auf die Patenterteilung:
**10.04.2019 Patentblatt 2019/15**

(51) Int Cl.:
***C11B 1/02*** (2006.01) ***C12N 9/02*** (2006.01)
***C12N 9/10*** (2006.01) ***A01K 67/027*** (2006.01)
***C12N 15/82*** (2006.01)

(21) Anmeldenummer: **13162907.3**

(22) Anmeldetag: **23.02.2005**

(54) **Verfahren zur Herstellung mehrfach ungesättigter Fettsäuren in transgenen Pflanzen**

Method for producing polyunsatured fatty acids in transgenic plants

Procédé de fabrication de plusieurs acides gras insaturés dans des plantes transgéniques

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität:
**27.02.2004 DE 102004009457**
**13.03.2004 DE 102004012370**
**08.04.2004 DE 102004017518**
**14.05.2004 DE 102004024014**
**16.07.2004 PCT/EP2004/007957**
**24.12.2004 DE 102004062543**

(43) Veröffentlichungstag der Anmeldung:
**07.08.2013 Patentblatt 2013/32**

(60) Teilanmeldung:
**19155597.8**

(62) Dokumentnummer(n) der früheren Anmeldung(en) nach Art. 76 EPÜ:
**05715462.7 / 1 723 220**

(73) Patentinhaber: **BASF Plant Science GmbH**
**67056 Ludwigshafen (DE)**

(72) Erfinder:
- **Cirpus, Petra**
  **67165 Waldsee (DE)**
- **Bauer, Jörg**
  **Durham, NC 27713 (US)**
- **Qiu, Xiao**
  **Saskatoon, Saskatchewan S7N 3S5 (CA)**
- **Wu, Guohai**
  **Saskatoon, Saskatchewan S7N 4A9 (CA)**
- **Datla, Nagamani**
  **Saskatoon, Saskatchewan S7V 1B6 (CA)**

(74) Vertreter: **BASF IP Association**
**BASF SE**
**G-FLP-C006**
**67056 Ludwigshafen (DE)**

(56) Entgegenhaltungen:
**WO-A2-2005/012316 DE-A1- 10 219 203**

- **BEAUDOIN FREDERIC ET AL: "Heterologous reconstitution in yeast of the polyunsaturated fatty acid biosynthetic pathway", PROCEEDINGS OF THE NATIONAL ACADEMY OF SCIENCES OF USA, NATIONAL ACADEMY OF SCIENCE. WASHINGTON, US, Bd. 97, Nr. 12, 6. Juni 2000 (2000-06-06), Seiten 6421-6426, XP002200201, ISSN: 0027-8424**
- **DOMERGUE F ET AL: "Cloning and functional characterization of Phaeodactylum tricornutum front-end desaturases involved in eicosapentaenoic acid biosynthesis", EUROPEAN JOURNAL OF BIOCHEMISTRY, BERLIN, DE, Bd. 269, Nr. 16, August 2002 (2002-08), Seiten 4105-4113, XP002228745, ISSN: 0014-2956**
- **ZANK T K ET AL: "Cloning and functional expression of the first plant fatty acid elongase specific for DELTA6-polyunsaturated fatty acids", BIOCHEMICAL SOCIETY TRANSACTIONS, COLCHESTER, ESSEX, GB, Bd. 28, Nr. 6, Dezember 2000 (2000-12), Seiten 654-658, XP002174836, ISSN: 0300-5127**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- DREXLER H ET AL: "Metabolic engineering of fatty acids for breeding of new oilseed crops: Strategies, problems and first results", JOURNAL OF PLANT PHYSIOLOGY, FISCHER, STUTTGART, DE, Bd. 160, Nr. 7, Juli 2003 (2003-07), Seiten 779-802, XP002266491, ISSN: 0176-1617
- MEYER ASTRID ET AL: "Novel fatty acid elongases and their use for the reconstitution of docosahexaenoic acid biosynthesis.", JOURNAL OF LIPID RESEARCH. OCT 2004, Bd. 45, Nr. 10, Oktober 2004 (2004-10), Seiten 1899-1909, XP009046591, ISSN: 0022-2275
- Drummond: "Re. GRAS Notification - Tuna Oil", , 15 February 2002 (2002-02-15), XP55389590, Retrieved from the Internet: URL:https://www.fda.gov/downloads/Food/IngredientsPackagingLabeling/GRAS/NoticeInventory/ucm258378.pdf [retrieved on 2017-07-11]
- Drummond: "Re. GRAS Notification - Tuna Oil", , 15 February 2002 (2002-02-15), XP055389590, Retrieved from the Internet: URL:https://www.fda.gov/downloads/Food/IngredientsPackagingLabeling/GRAS/NoticeInventory/ucm258378.pdf [retrieved on 2017-07-11]

SEQUENCE LISTING

<110> BASF Plant Science GmbH

<120> Verfahren zur Herstellung von mehrfach ungesättigten Fettsäuren in transgenen Pflanzen

<130> PF56186

<140> 20041035
<141> 2004-12-22

<160> 202

<170> PatentIn version 3.1

<210> 1
<211> 1266
<212> DNA
<213> Euglena gracilis

<220>
<221> CDS
<222> (1)..(1266)
<223> Delta-8-Desaturase

<400> 1

```
atg aag tca aag cgc caa gcg ctt ccc ctt aca att gat gga aca aca      48
Met Lys Ser Lys Arg Gln Ala Leu Pro Leu Thr Ile Asp Gly Thr Thr
1               5                   10                  15

tat gat gtg tct gcc tgg gtc aat ttc cac cct ggt ggt gcg gaa att      96
Tyr Asp Val Ser Ala Trp Val Asn Phe His Pro Gly Gly Ala Glu Ile
            20                  25                  30

ata gag aat tac caa gga agg gat gcc act gat gcc ttc atg gtt atg     144
Ile Glu Asn Tyr Gln Gly Arg Asp Ala Thr Asp Ala Phe Met Val Met
        35                  40                  45

cac tct caa gaa gcc ttc gac aag ctc aag cgc atg ccc aaa atc aat     192
His Ser Gln Glu Ala Phe Asp Lys Leu Lys Arg Met Pro Lys Ile Asn
    50                  55                  60

ccc agt tct gag ttg cca ccc cag gct gca gtg aat gaa gct caa gag     240
Pro Ser Ser Glu Leu Pro Pro Gln Ala Ala Val Asn Glu Ala Gln Glu
65                  70                  75                  80

gat ttc cgg aag ctc cga gaa gag ttg atc gca act ggc atg ttt gat     288
Asp Phe Arg Lys Leu Arg Glu Glu Leu Ile Ala Thr Gly Met Phe Asp
                85                  90                  95

gcc tcc ccc ctc tgg tac tca tac aaa atc agc acc aca ctg ggc ctt     336
Ala Ser Pro Leu Trp Tyr Ser Tyr Lys Ile Ser Thr Thr Leu Gly Leu
```

```
                100                 105                 110

gga gtg ctg ggt tat ttc ctg atg gtt cag tat cag atg tat ttc att      384
Gly Val Leu Gly Tyr Phe Leu Met Val Gln Tyr Gln Met Tyr Phe Ile
        115                 120                 125

ggg gca gtg ttg ctt ggg atg cac tat caa cag atg ggc tgg ctt tct      432
Gly Ala Val Leu Leu Gly Met His Tyr Gln Gln Met Gly Trp Leu Ser
    130                 135                 140

cat gac att tgc cac cac cag act ttc aag aac cgg aac tgg aac aac      480
His Asp Ile Cys His His Gln Thr Phe Lys Asn Arg Asn Trp Asn Asn
145                 150                 155                 160

ctc gtg gga ctg gta ttt ggc aat ggt ctg caa ggt ttt tcc gtg aca      528
Leu Val Gly Leu Val Phe Gly Asn Gly Leu Gln Gly Phe Ser Val Thr
                165                 170                 175

tgc tgg aag gac aga cac aat gca cat cat tcg gca acc aat gtt caa      576
Cys Trp Lys Asp Arg His Asn Ala His His Ser Ala Thr Asn Val Gln
            180                 185                 190

ggg cac gac cct gat att gac aac ctc ccc ctc tta gcc tgg tct gag      624
Gly His Asp Pro Asp Ile Asp Asn Leu Pro Leu Leu Ala Trp Ser Glu
        195                 200                 205

gat gac gtc aca cgg gcg tca ccg att tcc cgc aag ctc att cag ttc      672
Asp Asp Val Thr Arg Ala Ser Pro Ile Ser Arg Lys Leu Ile Gln Phe
    210                 215                 220

cag cag tat tat ttc ttg gtc atc tgt atc ttg ttg cgg ttc att tgg      720
Gln Gln Tyr Tyr Phe Leu Val Ile Cys Ile Leu Leu Arg Phe Ile Trp
225                 230                 235                 240

tgt ttc cag agc gtg ttg acc gtg cgc agt ctg aag gac aga gat aac      768
Cys Phe Gln Ser Val Leu Thr Val Arg Ser Leu Lys Asp Arg Asp Asn
                245                 250                 255

caa ttc tat cgc tct cag tat aag aag gag gcc att ggc ctc gcc ctg      816
Gln Phe Tyr Arg Ser Gln Tyr Lys Lys Glu Ala Ile Gly Leu Ala Leu
            260                 265                 270

cat tgg aca ttg aag gcc ctg ttc cac tta ttc ttt atg ccc agc atc      864
His Trp Thr Leu Lys Ala Leu Phe His Leu Phe Phe Met Pro Ser Ile
        275                 280                 285

ctc aca tcg ctg ttg gta ttt ttc gtt tcg gag ctg gtt ggc ggc ttc      912
Leu Thr Ser Leu Leu Val Phe Phe Val Ser Glu Leu Val Gly Gly Phe
    290                 295                 300

ggc att gcg atc gtg gtg ttc atg aac cac tac cca ctg gag aag atc      960
Gly Ile Ala Ile Val Val Phe Met Asn His Tyr Pro Leu Glu Lys Ile
305                 310                 315                 320

ggg gac tcg gtc tgg gat ggc cat gga ttc tcg gtt ggc cag atc cat     1008
Gly Asp Ser Val Trp Asp Gly His Gly Phe Ser Val Gly Gln Ile His
                325                 330                 335

gag acc atg aac att cgg cga ggg att atc aca gat tgg ttt ttc gga     1056
Glu Thr Met Asn Ile Arg Arg Gly Ile Ile Thr Asp Trp Phe Phe Gly
            340                 345                 350

ggc ttg aac tac cag atc gag cac cat ttg tgg ccg acc ctc cct cgc     1104
Gly Leu Asn Tyr Gln Ile Glu His His Leu Trp Pro Thr Leu Pro Arg
        355                 360                 365

cac aac ctg aca gcg gtt agc tac cag gtg gaa cag ctg tgc cag aag     1152
His Asn Leu Thr Ala Val Ser Tyr Gln Val Glu Gln Leu Cys Gln Lys
```

370 375 380

cac aac ctg ccg tat cgg aac ccg ctg ccc cat gaa ggg ttg gtc atc 1200
His Asn Leu Pro Tyr Arg Asn Pro Leu Pro His Glu Gly Leu Val Ile
385 390 395 400

ctg ctg cgc tat ctg gcg gtg ttc gcc cgg atg gcg gag aag caa ccc 1248
Leu Leu Arg Tyr Leu Ala Val Phe Ala Arg Met Ala Glu Lys Gln Pro
405 410 415

gcg ggg aag gct cta taa 1266
Ala Gly Lys Ala Leu
420

<210> 2
<211> 421
<212> PRT
<213> Euglena gracilis

<400> 2

Met Lys Ser Lys Arg Gln Ala Leu Pro Leu Thr Ile Asp Gly Thr Thr
1 5 10 15

Tyr Asp Val Ser Ala Trp Val Asn Phe His Pro Gly Gly Ala Glu Ile
20 25 30

Ile Glu Asn Tyr Gln Gly Arg Asp Ala Thr Asp Ala Phe Met Val Met
35 40 45

His Ser Gln Glu Ala Phe Asp Lys Leu Lys Arg Met Pro Lys Ile Asn
50 55 60

Pro Ser Ser Glu Leu Pro Pro Gln Ala Ala Val Asn Glu Ala Gln Glu
65 70 75 80

Asp Phe Arg Lys Leu Arg Glu Glu Leu Ile Ala Thr Gly Met Phe Asp
85 90 95

Ala Ser Pro Leu Trp Tyr Ser Tyr Lys Ile Ser Thr Thr Leu Gly Leu
100 105 110

Gly Val Leu Gly Tyr Phe Leu Met Val Gln Tyr Gln Met Tyr Phe Ile
115 120 125

Gly Ala Val Leu Leu Gly Met His Tyr Gln Gln Met Gly Trp Leu Ser
130 135 140

His Asp Ile Cys His His Gln Thr Phe Lys Asn Arg Asn Trp Asn Asn
145 150 155 160

Leu Val Gly Leu Val Phe Gly Asn Gly Leu Gln Gly Phe Ser Val Thr

165 170 175

Cys Trp Lys Asp Arg His Asn Ala His His Ser Ala Thr Asn Val Gln
180 185 190

Gly His Asp Pro Asp Ile Asp Asn Leu Pro Leu Leu Ala Trp Ser Glu
195 200 205

Asp Asp Val Thr Arg Ala Ser Pro Ile Ser Arg Lys Leu Ile Gln Phe
210 215 220

Gln Gln Tyr Tyr Phe Leu Val Ile Cys Ile Leu Leu Arg Phe Ile Trp
225 230 235 240

Cys Phe Gln Ser Val Leu Thr Val Arg Ser Leu Lys Asp Arg Asp Asn
245 250 255

Gln Phe Tyr Arg Ser Gln Tyr Lys Lys Glu Ala Ile Gly Leu Ala Leu
260 265 270

His Trp Thr Leu Lys Ala Leu Phe His Leu Phe Phe Met Pro Ser Ile
275 280 285

Leu Thr Ser Leu Leu Val Phe Phe Val Ser Glu Leu Val Gly Gly Phe
290 295 300

Gly Ile Ala Ile Val Val Phe Met Asn His Tyr Pro Leu Glu Lys Ile
305 310 315 320

Gly Asp Ser Val Trp Asp Gly His Gly Phe Ser Val Gly Gln Ile His
325 330 335

Glu Thr Met Asn Ile Arg Arg Gly Ile Ile Thr Asp Trp Phe Phe Gly
340 345 350

Gly Leu Asn Tyr Gln Ile Glu His His Leu Trp Pro Thr Leu Pro Arg
355 360 365

His Asn Leu Thr Ala Val Ser Tyr Gln Val Glu Gln Leu Cys Gln Lys
370 375 380

His Asn Leu Pro Tyr Arg Asn Pro Leu Pro His Glu Gly Leu Val Ile
385 390 395 400

Leu Leu Arg Tyr Leu Ala Val Phe Ala Arg Met Ala Glu Lys Gln Pro
405 410 415

Ala Gly Lys Ala Leu
420

<210> 3
<211> 777
<212> DNA
<213> Isochrysis galbana

<220>
<221> CDS
<222> (1)..(777)
<223> Delta-9-Elongase

<400> 3

```
atg gcc ctc gca aac gac gcg gga gag cgc atc tgg gcg gct gtg acc      48
Met Ala Leu Ala Asn Asp Ala Gly Glu Arg Ile Trp Ala Ala Val Thr
1               5                   10                  15

gac ccg gaa atc ctc att ggc acc ttc tcg tac ttg cta ctc aaa ccg      96
Asp Pro Glu Ile Leu Ile Gly Thr Phe Ser Tyr Leu Leu Leu Lys Pro
            20                  25                  30

ctg ctc cgc aat tcc ggg ctg gtg gat gag aag aag ggc gca tac agg      144
Leu Leu Arg Asn Ser Gly Leu Val Asp Glu Lys Lys Gly Ala Tyr Arg
        35                  40                  45

acg tcc atg atc tgg tac aac gtt ctg ctg gcg ctc ttc tct gcg ctg      192
Thr Ser Met Ile Trp Tyr Asn Val Leu Leu Ala Leu Phe Ser Ala Leu
    50                  55                  60

agc ttc tac gtg acg gcg acc gcc ctc ggc tgg gac tat ggt acg ggc      240
Ser Phe Tyr Val Thr Ala Thr Ala Leu Gly Trp Asp Tyr Gly Thr Gly
65                  70                  75                  80

gcg tgg ctg cgc agg caa acc ggc gac aca ccg cag ccg ctc ttc cag      288
Ala Trp Leu Arg Arg Gln Thr Gly Asp Thr Pro Gln Pro Leu Phe Gln
                85                  90                  95

tgc ccg tcc ccg gtt tgg gac tcg aag ctc ttc aca tgg acc gcc aag      336
Cys Pro Ser Pro Val Trp Asp Ser Lys Leu Phe Thr Trp Thr Ala Lys
            100                 105                 110

gca ttc tat tac tcc aag tac gtg gag tac ctc gac acg gcc tgg ctg      384
Ala Phe Tyr Tyr Ser Lys Tyr Val Glu Tyr Leu Asp Thr Ala Trp Leu
        115                 120                 125

agg gtc tcc ttt ctc cag gcc ttc cac cac ttt ggc gcg ccg tgg gat      432
Arg Val Ser Phe Leu Gln Ala Phe His His Phe Gly Ala Pro Trp Asp
    130                 135                 140

gtg tac ctc ggc att cgg ctg cac aac gag ggc gta tgg atc ttc atg      480
Val Tyr Leu Gly Ile Arg Leu His Asn Glu Gly Val Trp Ile Phe Met
145                 150                 155                 160

ttt ttc aac tcg ttc att cac acc atc atg tac acc tac tac ggc ctc      528
Phe Phe Asn Ser Phe Ile His Thr Ile Met Tyr Thr Tyr Tyr Gly Leu
                165                 170                 175

acc gcc gcc ggg tat aag ttc aag gcc aag ccg ctc atc acc gcg atg      576
```

```
Thr Ala Ala Gly Tyr Lys Phe Lys Ala Lys Pro Leu Ile Thr Ala Met
            180                 185                 190

cag atc tgc cag ttc gtg ggc ggc ttc ctg ttg gtc tgg gac tac atc       624
Gln Ile Cys Gln Phe Val Gly Gly Phe Leu Leu Val Trp Asp Tyr Ile
        195                 200                 205

aac gtc ccc tgc ttc aac tcg gac aaa ggg aag ttg ttc agc tgg gct       672
Asn Val Pro Cys Phe Asn Ser Asp Lys Gly Lys Leu Phe Ser Trp Ala
    210                 215                 220

ttc aac tat gca tac gtc ggc tcg gtc ttc ttg ctc ttc tgc cac ttt       720
Phe Asn Tyr Ala Tyr Val Gly Ser Val Phe Leu Leu Phe Cys His Phe
225                 230                 235                 240

ttc tac cag gac aac ttg gca acg aag aaa tcg gcc aag gcg ggc aag       768
Phe Tyr Gln Asp Asn Leu Ala Thr Lys Lys Ser Ala Lys Ala Gly Lys
                245                 250                 255

cag ctc tag                                                           777
Gln Leu
```

<210> 4
<211> 258
<212> PRT
<213> Isochrysis galbana

<400> 4

```
Met Ala Leu Ala Asn Asp Ala Gly Glu Arg Ile Trp Ala Ala Val Thr
1               5                   10                  15

Asp Pro Glu Ile Leu Ile Gly Thr Phe Ser Tyr Leu Leu Leu Lys Pro
            20                  25                  30

Leu Leu Arg Asn Ser Gly Leu Val Asp Glu Lys Lys Gly Ala Tyr Arg
        35                  40                  45

Thr Ser Met Ile Trp Tyr Asn Val Leu Leu Ala Leu Phe Ser Ala Leu
    50                  55                  60

Ser Phe Tyr Val Thr Ala Thr Ala Leu Gly Trp Asp Tyr Gly Thr Gly
65                  70                  75                  80

Ala Trp Leu Arg Arg Gln Thr Gly Asp Thr Pro Gln Pro Leu Phe Gln
                85                  90                  95

Cys Pro Ser Pro Val Trp Asp Ser Lys Leu Phe Thr Trp Thr Ala Lys
            100                 105                 110

Ala Phe Tyr Tyr Ser Lys Tyr Val Glu Tyr Leu Asp Thr Ala Trp Leu
        115                 120                 125
```

```
Arg Val Ser Phe Leu Gln Ala Phe His His Phe Gly Ala Pro Trp Asp
    130                 135                 140

Val Tyr Leu Gly Ile Arg Leu His Asn Glu Gly Val Trp Ile Phe Met
145                 150                 155                 160

Phe Phe Asn Ser Phe Ile His Thr Ile Met Tyr Thr Tyr Tyr Gly Leu
                165                 170                 175

Thr Ala Ala Gly Tyr Lys Phe Lys Ala Lys Pro Leu Ile Thr Ala Met
            180                 185                 190

Gln Ile Cys Gln Phe Val Gly Gly Phe Leu Leu Val Trp Asp Tyr Ile
        195                 200                 205

Asn Val Pro Cys Phe Asn Ser Asp Lys Gly Lys Leu Phe Ser Trp Ala
    210                 215                 220

Phe Asn Tyr Ala Tyr Val Gly Ser Val Phe Leu Leu Phe Cys His Phe
225                 230                 235                 240

Phe Tyr Gln Asp Asn Leu Ala Thr Lys Lys Ser Ala Lys Ala Gly Lys
                245                 250                 255

Gln Leu
```

<210> 5
<211> 1410
<212> DNA
<213> Phaeodactylum tricornutum

<220>
<221> CDS
<222> (1)..(1410)
<223> Delta-5-Desaturase

<400> 5

```
atg gct ccg gat gcg gat aag ctt cga caa cgc cag acg act gcg gta      48
Met Ala Pro Asp Ala Asp Lys Leu Arg Gln Arg Gln Thr Thr Ala Val
1               5                   10                  15

gcg aag cac aat gct gct acc ata tcg acg cag gaa cgc ctt tgc agt      96
Ala Lys His Asn Ala Ala Thr Ile Ser Thr Gln Glu Arg Leu Cys Ser
            20                  25                  30

ctg tct tcg ctc aaa ggc gaa gaa gtc tgc atc gac gga atc atc tat     144
Leu Ser Ser Leu Lys Gly Glu Glu Val Cys Ile Asp Gly Ile Ile Tyr
```

```
        35                  40                  45

gac ctc caa tca ttc gat cat ccc ggg ggt gaa acg atc aaa atg ttt      192
Asp Leu Gln Ser Phe Asp His Pro Gly Gly Glu Thr Ile Lys Met Phe
    50                  55                  60

ggt ggc aac gat gtc act gta cag tac aag atg att cac ccg tac cat      240
Gly Gly Asn Asp Val Thr Val Gln Tyr Lys Met Ile His Pro Tyr His
65                  70                  75                  80

acc gag aag cat ttg gaa aag atg aag cgt gtc ggc aag gtg acg gat      288
Thr Glu Lys His Leu Glu Lys Met Lys Arg Val Gly Lys Val Thr Asp
                85                  90                  95

ttc gtc tgc gag tac aag ttc gat acc gaa ttt gaa cgc gaa atc aaa      336
Phe Val Cys Glu Tyr Lys Phe Asp Thr Glu Phe Glu Arg Glu Ile Lys
            100                 105                 110

cga gaa gtc ttc aag att gtg cga cga ggc aag gat ttc ggt act ttg      384
Arg Glu Val Phe Lys Ile Val Arg Arg Gly Lys Asp Phe Gly Thr Leu
        115                 120                 125

gga tgg ttc ttc cgt gcg ttt tgc tac att gcc att ttc ttc tac ctg      432
Gly Trp Phe Phe Arg Ala Phe Cys Tyr Ile Ala Ile Phe Phe Tyr Leu
    130                 135                 140

cag tac cat tgg gtc acc acg gga acc tct tgg ctg ctg gcc gtg gcc      480
Gln Tyr His Trp Val Thr Thr Gly Thr Ser Trp Leu Leu Ala Val Ala
145                 150                 155                 160

tac gga atc tcc caa gcg atg att ggc atg aat gtc cag cac gat gcc      528
Tyr Gly Ile Ser Gln Ala Met Ile Gly Met Asn Val Gln His Asp Ala

                165                 170                 175

aac cac ggg gcc acc tcc aag cgt ccc tgg gtc aac gac atg cta ggc      576
Asn His Gly Ala Thr Ser Lys Arg Pro Trp Val Asn Asp Met Leu Gly
            180                 185                 190

ctc ggt gcg gat ttt att ggt ggt tcc aag tgg ctc tgg cag gaa caa      624
Leu Gly Ala Asp Phe Ile Gly Gly Ser Lys Trp Leu Trp Gln Glu Gln
        195                 200                 205

cac tgg acc cac cac gct tac acc aat cac gcc gag atg gat ccc gat      672
His Trp Thr His His Ala Tyr Thr Asn His Ala Glu Met Asp Pro Asp
    210                 215                 220

agc ttt ggt gcc gaa cca atg ctc cta ttc aac gac tat ccc ttg gat      720
Ser Phe Gly Ala Glu Pro Met Leu Leu Phe Asn Asp Tyr Pro Leu Asp
225                 230                 235                 240

cat ccc gct cgt acc tgg cta cat cgc ttt caa gca ttc ttt tac atg      768
His Pro Ala Arg Thr Trp Leu His Arg Phe Gln Ala Phe Phe Tyr Met
                245                 250                 255

ccc gtc ttg gct gga tac tgg ttg tcc gct gtc ttc aat cca caa att      816
Pro Val Leu Ala Gly Tyr Trp Leu Ser Ala Val Phe Asn Pro Gln Ile
            260                 265                 270

ctt gac ctc cag caa cgc ggc gca ctt tcc gtc ggt atc cgt ctc gac      864
Leu Asp Leu Gln Gln Arg Gly Ala Leu Ser Val Gly Ile Arg Leu Asp
        275                 280                 285

aac gct ttc att cac tcg cga cgc aag tat gcg gtt ttc tgg cgg gct      912
Asn Ala Phe Ile His Ser Arg Arg Lys Tyr Ala Val Phe Trp Arg Ala

    290                 295                 300
```

```
gtg tac att gcg gtg aac gtg att gct ccg ttt tac aca aac tcc ggc      960
Val Tyr Ile Ala Val Asn Val Ile Ala Pro Phe Tyr Thr Asn Ser Gly
305                 310                 315                 320

ctc gaa tgg tcc tgg cgt gtc ttt gga aac atc atg ctc atg ggt gtg     1008
Leu Glu Trp Ser Trp Arg Val Phe Gly Asn Ile Met Leu Met Gly Val
                325                 330                 335

gcg gaa tcg ctc gcg ctg gcg gtc ctg ttt tcg ttg tcg cac aat ttc     1056
Ala Glu Ser Leu Ala Leu Ala Val Leu Phe Ser Leu Ser His Asn Phe
            340                 345                 350

gaa tcc gcg gat cgc gat ccg acc gcc cca ctg aaa aag acg gga gaa     1104
Glu Ser Ala Asp Arg Asp Pro Thr Ala Pro Leu Lys Lys Thr Gly Glu
        355                 360                 365

cca gtc gac tgg ttc aag aca cag gtc gaa act tcc tgc act tac ggt     1152
Pro Val Asp Trp Phe Lys Thr Gln Val Glu Thr Ser Cys Thr Tyr Gly
    370                 375                 380

gga ttc ctt tcc ggt tgc ttc acg gga ggt ctc aac ttt cag gtt gaa     1200
Gly Phe Leu Ser Gly Cys Phe Thr Gly Gly Leu Asn Phe Gln Val Glu
385                 390                 395                 400

cac cac ttg ttc cca cgc atg agc agc gct tgg tat ccc tac att gcc     1248
His His Leu Phe Pro Arg Met Ser Ser Ala Trp Tyr Pro Tyr Ile Ala
                405                 410                 415

ccc aag gtc cgc gaa att tgc gcc aaa cac ggc gtc cac tac gcc tac     1296
Pro Lys Val Arg Glu Ile Cys Ala Lys His Gly Val His Tyr Ala Tyr

            420                 425                 430

tac ccg tgg atc cac caa aac ttt ctc tcc acc gtc cgc tac atg cac     1344
Tyr Pro Trp Ile His Gln Asn Phe Leu Ser Thr Val Arg Tyr Met His
        435                 440                 445

gcg gcc ggg acc ggt gcc aac tgg cgc cag atg gcc aga gaa aat ccc     1392
Ala Ala Gly Thr Gly Ala Asn Trp Arg Gln Met Ala Arg Glu Asn Pro
    450                 455                 460

ttg acc gga cgg gcg taa                                             1410
Leu Thr Gly Arg Ala
465
```

<210> 6
<211> 469
<212> PRT
<213> Phaeodactylum tricornutum

<400> 6

```
Met Ala Pro Asp Ala Asp Lys Leu Arg Gln Arg Gln Thr Thr Ala Val
1               5                   10                  15

Ala Lys His Asn Ala Ala Thr Ile Ser Thr Gln Glu Arg Leu Cys Ser
            20                  25                  30

Leu Ser Ser Leu Lys Gly Glu Glu Val Cys Ile Asp Gly Ile Ile Tyr
        35                  40                  45
```

Asp Leu Gln Ser Phe Asp His Pro Gly Gly Glu Thr Ile Lys Met Phe
50 55 60

Gly Gly Asn Asp Val Thr Val Gln Tyr Lys Met Ile His Pro Tyr His
65 70 75 80

Thr Glu Lys His Leu Glu Lys Met Lys Arg Val Gly Lys Val Thr Asp
85 90 95

Phe Val Cys Glu Tyr Lys Phe Asp Thr Glu Phe Glu Arg Glu Ile Lys
100 105 110

Arg Glu Val Phe Lys Ile Val Arg Arg Gly Lys Asp Phe Gly Thr Leu
115 120 125

Gly Trp Phe Phe Arg Ala Phe Cys Tyr Ile Ala Ile Phe Phe Tyr Leu
130 135 140

Gln Tyr His Trp Val Thr Thr Gly Thr Ser Trp Leu Leu Ala Val Ala
145 150 155 160

Tyr Gly Ile Ser Gln Ala Met Ile Gly Met Asn Val Gln His Asp Ala
165 170 175

Asn His Gly Ala Thr Ser Lys Arg Pro Trp Val Asn Asp Met Leu Gly
180 185 190

Leu Gly Ala Asp Phe Ile Gly Gly Ser Lys Trp Leu Trp Gln Glu Gln
195 200 205

His Trp Thr His His Ala Tyr Thr Asn His Ala Glu Met Asp Pro Asp
210 215 220

Ser Phe Gly Ala Glu Pro Met Leu Leu Phe Asn Asp Tyr Pro Leu Asp
225 230 235 240

His Pro Ala Arg Thr Trp Leu His Arg Phe Gln Ala Phe Phe Tyr Met
245 250 255

Pro Val Leu Ala Gly Tyr Trp Leu Ser Ala Val Phe Asn Pro Gln Ile
260 265 270

Leu Asp Leu Gln Gln Arg Gly Ala Leu Ser Val Gly Ile Arg Leu Asp
275 280 285

Asn Ala Phe Ile His Ser Arg Arg Lys Tyr Ala Val Phe Trp Arg Ala
290 295 300

Val Tyr Ile Ala Val Asn Val Ile Ala Pro Phe Tyr Thr Asn Ser Gly
305 310 315 320

```
Leu Glu Trp Ser Trp Arg Val Phe Gly Asn Ile Met Leu Met Gly Val
                325                 330                 335

Ala Glu Ser Leu Ala Leu Ala Val Leu Phe Ser Leu Ser His Asn Phe
            340                 345                 350

Glu Ser Ala Asp Arg Asp Pro Thr Ala Pro Leu Lys Lys Thr Gly Glu
        355                 360                 365

Pro Val Asp Trp Phe Lys Thr Gln Val Glu Thr Ser Cys Thr Tyr Gly
    370                 375                 380

Gly Phe Leu Ser Gly Cys Phe Thr Gly Gly Leu Asn Phe Gln Val Glu
385                 390                 395                 400

His His Leu Phe Pro Arg Met Ser Ser Ala Trp Tyr Pro Tyr Ile Ala
                405                 410                 415

Pro Lys Val Arg Glu Ile Cys Ala Lys His Gly Val His Tyr Ala Tyr
            420                 425                 430

Tyr Pro Trp Ile His Gln Asn Phe Leu Ser Thr Val Arg Tyr Met His
        435                 440                 445

Ala Ala Gly Thr Gly Ala Asn Trp Arg Gln Met Ala Arg Glu Asn Pro
    450                 455                 460

Leu Thr Gly Arg Ala
465
```

<210> 7
<211> 1344
<212> DNA
<213> Ceratodon purpureus

<220>
<221> CDS
<222> (1)..(1344)
<223> Delta-5-Desaturase

<400> 7

```
atg gta tta cga gag caa gag cat gag cca ttc ttc att aaa att gat      48
Met Val Leu Arg Glu Gln Glu His Glu Pro Phe Phe Ile Lys Ile Asp
1               5                   10                  15

gga aaa tgg tgt caa att gac gat gct gtc ctg aga tca cat cca ggt      96
```

```
Gly Lys Trp Cys Gln Ile Asp Asp Ala Val Leu Arg Ser His Pro Gly
            20                  25                  30

ggt agt gca att act acc tat aaa aat atg gat gcc act acc gta ttc      144
Gly Ser Ala Ile Thr Thr Tyr Lys Asn Met Asp Ala Thr Thr Val Phe
        35                  40                  45

cac aca ttc cat act ggt tct aaa gaa gcg tat caa tgg ctg aca gaa      192
His Thr Phe His Thr Gly Ser Lys Glu Ala Tyr Gln Trp Leu Thr Glu
    50                  55                  60

ttg aaa aaa gag tgc cct aca caa gaa cca gag atc cca gat att aag      240
Leu Lys Lys Glu Cys Pro Thr Gln Glu Pro Glu Ile Pro Asp Ile Lys
65                  70                  75                  80

gat gac cca atc aaa gga att gat gat gtg aac atg gga act ttc aat      288
Asp Asp Pro Ile Lys Gly Ile Asp Asp Val Asn Met Gly Thr Phe Asn
                85                  90                  95

att tct gag aaa cga tct gcc caa ata aat aaa agt ttc act gat cta      336
Ile Ser Glu Lys Arg Ser Ala Gln Ile Asn Lys Ser Phe Thr Asp Leu
            100                 105                 110

cgt atg cga gtt cgt gca gaa gga ctt atg gat gga tct cct ttg ttc      384
Arg Met Arg Val Arg Ala Glu Gly Leu Met Asp Gly Ser Pro Leu Phe
        115                 120                 125

tac att aga aaa att ctt gaa aca atc ttc aca att ctt ttt gca ttc      432
Tyr Ile Arg Lys Ile Leu Glu Thr Ile Phe Thr Ile Leu Phe Ala Phe
    130                 135                 140

tac ctt caa tac cac aca tat tat ctt cca tca gct att cta atg gga      480
Tyr Leu Gln Tyr His Thr Tyr Tyr Leu Pro Ser Ala Ile Leu Met Gly
145                 150                 155                 160

gtt gcg tgg caa caa ttg gga tgg tta atc cat gaa ttc gca cat cat      528
Val Ala Trp Gln Gln Leu Gly Trp Leu Ile His Glu Phe Ala His His
                165                 170                 175

cag ttg ttc aaa aac aga tac tac aat gat ttg gcc agc tat ttc gtt      576
Gln Leu Phe Lys Asn Arg Tyr Tyr Asn Asp Leu Ala Ser Tyr Phe Val
            180                 185                 190

gga aac ttt tta caa gga ttc tca tct ggt ggt tgg aaa gag cag cac      624
Gly Asn Phe Leu Gln Gly Phe Ser Ser Gly Gly Trp Lys Glu Gln His
        195                 200                 205

aat gtg cat cac gca gcc aca aat gtt gtt gga cga gac gga gat ctt      672
Asn Val His His Ala Ala Thr Asn Val Val Gly Arg Asp Gly Asp Leu
    210                 215                 220

gat tta gtc cca ttc tat gct aca gtg gca gaa cat ctc aac aat tat      720
Asp Leu Val Pro Phe Tyr Ala Thr Val Ala Glu His Leu Asn Asn Tyr
225                 230                 235                 240

tct cag gat tca tgg gtt atg act cta ttc aga tgg caa cat gtt cat      768
Ser Gln Asp Ser Trp Val Met Thr Leu Phe Arg Trp Gln His Val His
                245                 250                 255

tgg aca ttc atg tta cca ttc ctc cgt ctc tcg tgg ctt ctt cag tca      816
Trp Thr Phe Met Leu Pro Phe Leu Arg Leu Ser Trp Leu Leu Gln Ser
            260                 265                 270

atc att ttt gtt agt cag atg cca act cat tat tat gac tat tac aga      864
Ile Ile Phe Val Ser Gln Met Pro Thr His Tyr Tyr Asp Tyr Tyr Arg
        275                 280                 285

aat act gcg att tat gaa cag gtt ggt ctc tct ttg cac tgg gct tgg      912
```

```
Asn Thr Ala Ile Tyr Glu Gln Val Gly Leu Ser Leu His Trp Ala Trp
    290                 295                 300

tca ttg ggt caa ttg tat ttc cta ccc gat tgg tca act aga ata atg      960
Ser Leu Gly Gln Leu Tyr Phe Leu Pro Asp Trp Ser Thr Arg Ile Met
305                 310                 315                 320

ttc ttc ctt gtt tct cat ctt gtt gga ggt ttc ctg ctc tct cat gta     1008
Phe Phe Leu Val Ser His Leu Val Gly Gly Phe Leu Leu Ser His Val
                325                 330                 335

gtt act ttc aat cat tat tca gtg gag aag ttt gca ttg agc tcg aac     1056
Val Thr Phe Asn His Tyr Ser Val Glu Lys Phe Ala Leu Ser Ser Asn
            340                 345                 350

atc atg tca aat tac gct tgt ctt caa atc atg acc aca aga aat atg     1104
Ile Met Ser Asn Tyr Ala Cys Leu Gln Ile Met Thr Thr Arg Asn Met
        355                 360                 365

aga cct gga aga ttc att gac tgg ctt tgg gga ggt ctt aac tat cag     1152
Arg Pro Gly Arg Phe Ile Asp Trp Leu Trp Gly Gly Leu Asn Tyr Gln
    370                 375                 380

att gag cac cat ctt ttc cca acg atg cca cga cac aac ttg aac act     1200
Ile Glu His His Leu Phe Pro Thr Met Pro Arg His Asn Leu Asn Thr
385                 390                 395                 400

gtt atg cca ctt gtt aag gag ttt gca gca gca aat ggt tta cca tac     1248
Val Met Pro Leu Val Lys Glu Phe Ala Ala Ala Asn Gly Leu Pro Tyr
                405                 410                 415

atg gtc gac gat tat ttc aca gga ttc tgg ctt gaa att gag caa ttc     1296
Met Val Asp Asp Tyr Phe Thr Gly Phe Trp Leu Glu Ile Glu Gln Phe
            420                 425                 430

cga aat att gca aat gtt gct gct aaa ttg act aaa aag att gcc tag     1344
Arg Asn Ile Ala Asn Val Ala Ala Lys Leu Thr Lys Lys Ile Ala
        435                 440                 445
```

<210> 8
<211> 447
<212> PRT
<213> Ceratodon purpureus

<400> 8

```
Met Val Leu Arg Glu Gln Glu His Glu Pro Phe Phe Ile Lys Ile Asp
1               5                   10                  15

Gly Lys Trp Cys Gln Ile Asp Asp Ala Val Leu Arg Ser His Pro Gly
            20                  25                  30

Gly Ser Ala Ile Thr Thr Tyr Lys Asn Met Asp Ala Thr Thr Val Phe
        35                  40                  45

His Thr Phe His Thr Gly Ser Lys Glu Ala Tyr Gln Trp Leu Thr Glu
    50                  55                  60
```

Leu Lys Lys Glu Cys Pro Thr Gln Glu Pro Glu Ile Pro Asp Ile Lys
65 70 75 80

Asp Asp Pro Ile Lys Gly Ile Asp Asp Val Asn Met Gly Thr Phe Asn
85 90 95

Ile Ser Glu Lys Arg Ser Ala Gln Ile Asn Lys Ser Phe Thr Asp Leu
100 105 110

Arg Met Arg Val Arg Ala Glu Gly Leu Met Asp Gly Ser Pro Leu Phe
115 120 125

Tyr Ile Arg Lys Ile Leu Glu Thr Ile Phe Thr Ile Leu Phe Ala Phe
130 135 140

Tyr Leu Gln Tyr His Thr Tyr Tyr Leu Pro Ser Ala Ile Leu Met Gly
145 150 155 160

Val Ala Trp Gln Gln Leu Gly Trp Leu Ile His Glu Phe Ala His His
165 170 175

Gln Leu Phe Lys Asn Arg Tyr Tyr Asn Asp Leu Ala Ser Tyr Phe Val
180 185 190

Gly Asn Phe Leu Gln Gly Phe Ser Ser Gly Gly Trp Lys Glu Gln His
195 200 205

Asn Val His His Ala Ala Thr Asn Val Val Gly Arg Asp Gly Asp Leu
210 215 220

Asp Leu Val Pro Phe Tyr Ala Thr Val Ala Glu His Leu Asn Asn Tyr
225 230 235 240

Ser Gln Asp Ser Trp Val Met Thr Leu Phe Arg Trp Gln His Val His
245 250 255

Trp Thr Phe Met Leu Pro Phe Leu Arg Leu Ser Trp Leu Leu Gln Ser
260 265 270

Ile Ile Phe Val Ser Gln Met Pro Thr His Tyr Tyr Asp Tyr Tyr Arg
275 280 285

Asn Thr Ala Ile Tyr Glu Gln Val Gly Leu Ser Leu His Trp Ala Trp
290 295 300

Ser Leu Gly Gln Leu Tyr Phe Leu Pro Asp Trp Ser Thr Arg Ile Met
305 310 315 320

Phe Phe Leu Val Ser His Leu Val Gly Gly Phe Leu Leu Ser His Val
325 330 335

```
Val Thr Phe Asn His Tyr Ser Val Glu Lys Phe Ala Leu Ser Ser Asn
            340                 345                 350

Ile Met Ser Asn Tyr Ala Cys Leu Gln Ile Met Thr Thr Arg Asn Met
        355                 360                 365

Arg Pro Gly Arg Phe Ile Asp Trp Leu Trp Gly Gly Leu Asn Tyr Gln
    370                 375                 380

Ile Glu His His Leu Phe Pro Thr Met Pro Arg His Asn Leu Asn Thr
385                 390                 395                 400

Val Met Pro Leu Val Lys Glu Phe Ala Ala Ala Asn Gly Leu Pro Tyr
                405                 410                 415

Met Val Asp Asp Tyr Phe Thr Gly Phe Trp Leu Glu Ile Glu Gln Phe
            420                 425                 430

Arg Asn Ile Ala Asn Val Ala Ala Lys Leu Thr Lys Lys Ile Ala
        435                 440                 445
```

<210> 9
<211> 1443
<212> DNA
<213> Physcomitrella patens

<220>
<221> CDS
<222> (1)..(1443)
<223> Delta-5-Desaturase

<400> 9

```
atg gcg ccc cac tct gcg gat act gct ggg ctc gtg cct tct gac gaa       48
Met Ala Pro His Ser Ala Asp Thr Ala Gly Leu Val Pro Ser Asp Glu
1               5                   10                  15

ttg agg cta cga acg tcg aat tca aag ggt ccc gaa caa gag caa act       96
Leu Arg Leu Arg Thr Ser Asn Ser Lys Gly Pro Glu Gln Glu Gln Thr
            20                  25                  30

ttg aag aag tac acc ctt gaa gat gtc agc cgc cac aac acc cca gca      144
Leu Lys Lys Tyr Thr Leu Glu Asp Val Ser Arg His Asn Thr Pro Ala
        35                  40                  45

gat tgt tgg ttg gtg ata tgg ggc aaa gtc tac gat gtc aca agc tgg      192
Asp Cys Trp Leu Val Ile Trp Gly Lys Val Tyr Asp Val Thr Ser Trp
    50                  55                  60

att ccc aat cat ccg ggg ggc agt ctc atc cac gta aaa gca ggg cag      240
Ile Pro Asn His Pro Gly Gly Ser Leu Ile His Val Lys Ala Gly Gln
```

```
65                  70                  75                  80

gat tcc act cag ctt ttc gat tcc tat cac ccc ctt tat gtc agg aaa      288
Asp Ser Thr Gln Leu Phe Asp Ser Tyr His Pro Leu Tyr Val Arg Lys
                85                  90                  95

atg ctc gcg aag tac tgt att ggg gaa tta gta ccg tct gct ggt gat      336
Met Leu Ala Lys Tyr Cys Ile Gly Glu Leu Val Pro Ser Ala Gly Asp
            100                 105                 110

gac aag ttt aag aaa gca act ctg gag tat gca gat gcc gaa aat gaa      384
Asp Lys Phe Lys Lys Ala Thr Leu Glu Tyr Ala Asp Ala Glu Asn Glu
        115                 120                 125

gat ttc tat ttg gtt gtg aag caa cga gtt gaa tct tat ttc aag agt      432
Asp Phe Tyr Leu Val Val Lys Gln Arg Val Glu Ser Tyr Phe Lys Ser
    130                 135                 140

aac aag ata aac ccc caa att cat cca cat atg atc ctg aag tca ttg      480
Asn Lys Ile Asn Pro Gln Ile His Pro His Met Ile Leu Lys Ser Leu
145                 150                 155                 160

ttc att ctt ggg gga tat ttc gcc agt tac tat tta gcg ttc ttc tgg      528
Phe Ile Leu Gly Gly Tyr Phe Ala Ser Tyr Tyr Leu Ala Phe Phe Trp
                165                 170                 175

tct tca agt gtc ctt gtt tct ttg ttt ttc gca ttg tgg atg ggg ttc      576
Ser Ser Ser Val Leu Val Ser Leu Phe Phe Ala Leu Trp Met Gly Phe
            180                 185                 190

ttc gca gcg gaa gtc ggc gtg tcg att caa cat gat gga aat cat ggt      624
Phe Ala Ala Glu Val Gly Val Ser Ile Gln His Asp Gly Asn His Gly
        195                 200                 205

tca tac act aaa tgg cgt ggc ttt gga tat atc atg gga gcc tcc cta      672
Ser Tyr Thr Lys Trp Arg Gly Phe Gly Tyr Ile Met Gly Ala Ser Leu
    210                 215                 220

gat cta gtc gga gcc agt agc ttc atg tgg aga cag caa cac gtt gtg      720
Asp Leu Val Gly Ala Ser Ser Phe Met Trp Arg Gln Gln His Val Val
225                 230                 235                 240

gga cat cac tcg ttt aca aat gtg gac aac tac gat cct gat att cgt      768
Gly His His Ser Phe Thr Asn Val Asp Asn Tyr Asp Pro Asp Ile Arg
                245                 250                 255

gtg aaa gat cca gat gtc agg agg gtt gcg acc aca caa cca aga caa      816
Val Lys Asp Pro Asp Val Arg Arg Val Ala Thr Thr Gln Pro Arg Gln
            260                 265                 270

tgg tat cat gcg tat cag cat atc tac ctg gca gta tta tat gga act      864
Trp Tyr His Ala Tyr Gln His Ile Tyr Leu Ala Val Leu Tyr Gly Thr
        275                 280                 285

cta gct ctt aag agt att ttt cta gat gat ttc ctt gcg tac ttc aca      912
Leu Ala Leu Lys Ser Ile Phe Leu Asp Asp Phe Leu Ala Tyr Phe Thr
    290                 295                 300

gga tca att ggc cct gtc aag gtg gcg aaa atg acc ccc ctg gag ttc      960
Gly Ser Ile Gly Pro Val Lys Val Ala Lys Met Thr Pro Leu Glu Phe
305                 310                 315                 320

aac atc ttc ttt cag gga aag ctg cta tat gcg ttc tac atg ttc gtg     1008
Asn Ile Phe Phe Gln Gly Lys Leu Leu Tyr Ala Phe Tyr Met Phe Val
                325                 330                 335

ttg cca tct gtg tac ggt gtt cac tcc gga gga act ttc ttg gca cta     1056
Leu Pro Ser Val Tyr Gly Val His Ser Gly Gly Thr Phe Leu Ala Leu
```

```
        340                 345                 350

tat gtg gct tct cag ctc att aca ggt tgg atg tta gct ttt ctt ttt      1104
Tyr Val Ala Ser Gln Leu Ile Thr Gly Trp Met Leu Ala Phe Leu Phe
        355                 360                 365

caa gta gca cat gtc gtg gat gat gtt gca ttt cct aca cca gaa ggt      1152
Gln Val Ala His Val Val Asp Asp Val Ala Phe Pro Thr Pro Glu Gly
    370                 375                 380

ggg aag gtg aag gga gga tgg gct gca atg cag gtt gca aca act acg      1200
Gly Lys Val Lys Gly Gly Trp Ala Ala Met Gln Val Ala Thr Thr Thr
385                 390                 395                 400

gat ttc agt cca cgc tca tgg ttc tgg ggt cat gtc tct gga gga tta      1248
Asp Phe Ser Pro Arg Ser Trp Phe Trp Gly His Val Ser Gly Gly Leu
                405                 410                 415

aac aac caa att gag cat cat ctg ttt cca gga gtg tgc cat gtt cat      1296
Asn Asn Gln Ile Glu His His Leu Phe Pro Gly Val Cys His Val His
            420                 425                 430

tat cca gcc att cag cct att gtc gag aag acg tgc aag gaa ttc gat      1344
Tyr Pro Ala Ile Gln Pro Ile Val Glu Lys Thr Cys Lys Glu Phe Asp
        435                 440                 445

gtg cct tat gta gcc tac cca act ttt tgg act gcg ttg aga gcc cac      1392
Val Pro Tyr Val Ala Tyr Pro Thr Phe Trp Thr Ala Leu Arg Ala His
    450                 455                 460

ttt gcg cat ttg aaa aag gtt gga ttg aca gag ttt cgg ctc gat ggc      1440
Phe Ala His Leu Lys Lys Val Gly Leu Thr Glu Phe Arg Leu Asp Gly
465                 470                 475                 480

tga                                                                  1443
```

<210> 10
<211> 480
<212> PRT
<213> Physcomitrella patens

<400> 10

```
Met Ala Pro His Ser Ala Asp Thr Ala Gly Leu Val Pro Ser Asp Glu
1               5                   10                  15

Leu Arg Leu Arg Thr Ser Asn Ser Lys Gly Pro Glu Gln Glu Gln Thr
            20                  25                  30

Leu Lys Lys Tyr Thr Leu Glu Asp Val Ser Arg His Asn Thr Pro Ala
        35                  40                  45

Asp Cys Trp Leu Val Ile Trp Gly Lys Val Tyr Asp Val Thr Ser Trp
    50                  55                  60

Ile Pro Asn His Pro Gly Gly Ser Leu Ile His Val Lys Ala Gly Gln
65                  70                  75                  80
```

Asp Ser Thr Gln Leu Phe Asp Ser Tyr His Pro Leu Tyr Val Arg Lys
85 90 95

Met Leu Ala Lys Tyr Cys Ile Gly Glu Leu Val Pro Ser Ala Gly Asp
100 105 110

Asp Lys Phe Lys Lys Ala Thr Leu Glu Tyr Ala Asp Ala Glu Asn Glu
115 120 125

Asp Phe Tyr Leu Val Val Lys Gln Arg Val Glu Ser Tyr Phe Lys Ser
130 135 140

Asn Lys Ile Asn Pro Gln Ile His Pro His Met Ile Leu Lys Ser Leu
145 150 155 160

Phe Ile Leu Gly Gly Tyr Phe Ala Ser Tyr Tyr Leu Ala Phe Phe Trp
165 170 175

Ser Ser Ser Val Leu Val Ser Leu Phe Phe Ala Leu Trp Met Gly Phe
180 185 190

Phe Ala Ala Glu Val Gly Val Ser Ile Gln His Asp Gly Asn His Gly
195 200 205

Ser Tyr Thr Lys Trp Arg Gly Phe Gly Tyr Ile Met Gly Ala Ser Leu
210 215 220

Asp Leu Val Gly Ala Ser Ser Phe Met Trp Arg Gln Gln His Val Val
225 230 235 240

Gly His His Ser Phe Thr Asn Val Asp Asn Tyr Asp Pro Asp Ile Arg
245 250 255

Val Lys Asp Pro Asp Val Arg Arg Val Ala Thr Thr Gln Pro Arg Gln
260 265 270

Trp Tyr His Ala Tyr Gln His Ile Tyr Leu Ala Val Leu Tyr Gly Thr
275 280 285

Leu Ala Leu Lys Ser Ile Phe Leu Asp Asp Phe Leu Ala Tyr Phe Thr
290 295 300

Gly Ser Ile Gly Pro Val Lys Val Ala Lys Met Thr Pro Leu Glu Phe
305 310 315 320

Asn Ile Phe Phe Gln Gly Lys Leu Leu Tyr Ala Phe Tyr Met Phe Val
325 330 335

Leu Pro Ser Val Tyr Gly Val His Ser Gly Gly Thr Phe Leu Ala Leu
340 345 350

```
Tyr Val Ala Ser Gln Leu Ile Thr Gly Trp Met Leu Ala Phe Leu Phe
        355                 360                 365

Gln Val Ala His Val Val Asp Asp Val Ala Phe Pro Thr Pro Glu Gly
    370                 375                 380

Gly Lys Val Lys Gly Gly Trp Ala Ala Met Gln Val Ala Thr Thr Thr
385                 390                 395                 400

Asp Phe Ser Pro Arg Ser Trp Phe Trp Gly His Val Ser Gly Gly Leu
                405                 410                 415

Asn Asn Gln Ile Glu His His Leu Phe Pro Gly Val Cys His Val His
            420                 425                 430

Tyr Pro Ala Ile Gln Pro Ile Val Glu Lys Thr Cys Lys Glu Phe Asp
        435                 440                 445

Val Pro Tyr Val Ala Tyr Pro Thr Phe Trp Thr Ala Leu Arg Ala His
    450                 455                 460

Phe Ala His Leu Lys Lys Val Gly Leu Thr Glu Phe Arg Leu Asp Gly
465                 470                 475                 480
```

<210> 11
<211> 1320
<212> DNA
<213> Thraustrochytrium

<220>
<221> CDS
<222> (1)..(1320)
<223>

<400> 11

```
atg ggc aag ggc agc gag ggc cgc agc gcg gcg cgc gag atg acg gcc      48
Met Gly Lys Gly Ser Glu Gly Arg Ser Ala Ala Arg Glu Met Thr Ala
1               5                   10                  15

gag gcg aac ggc gac aag cgg aaa acg att ctg atc gag ggc gtc ctg      96
Glu Ala Asn Gly Asp Lys Arg Lys Thr Ile Leu Ile Glu Gly Val Leu
            20                  25                  30

tac gac gcg acg aac ttt aag cac ccg ggc ggt tcg atc atc aac ttc     144
Tyr Asp Ala Thr Asn Phe Lys His Pro Gly Gly Ser Ile Ile Asn Phe
        35                  40                  45

ttg acc gag ggc gag gcc ggc gtg gac gcg acg cag gcg tac cgc gag     192
Leu Thr Glu Gly Glu Ala Gly Val Asp Ala Thr Gln Ala Tyr Arg Glu
```

```
        50                  55                  60

ttt cat cag cgg tcc ggc aag gcc gac aag tac ctc aag tcg ctg ccg       240
Phe His Gln Arg Ser Gly Lys Ala Asp Lys Tyr Leu Lys Ser Leu Pro
65                  70                  75                  80

aag ctg gat gcg tcc aag gtg gag tcg cgg ttc tcg gcc aaa gag cag       288
Lys Leu Asp Ala Ser Lys Val Glu Ser Arg Phe Ser Ala Lys Glu Gln
                85                  90                  95

gcg cgg cgc gac gcc atg acg cgc gac tac gcg gcc ttt cgc gag gag       336
Ala Arg Arg Asp Ala Met Thr Arg Asp Tyr Ala Ala Phe Arg Glu Glu
            100                 105                 110

ctc gtc gcc gag ggg tac ttt gac ccg tcg atc ccg cac atg att tac       384
Leu Val Ala Glu Gly Tyr Phe Asp Pro Ser Ile Pro His Met Ile Tyr
        115                 120                 125

cgc gtc gtg gag atc gtg gcg ctc ttc gcg ctc tcg ttc tgg ctc atg       432
Arg Val Val Glu Ile Val Ala Leu Phe Ala Leu Ser Phe Trp Leu Met
    130                 135                 140

tcc aag gcc tcg ccc acc tcg ctc gtg ctg ggc gtg gtg atg aac ggc       480
Ser Lys Ala Ser Pro Thr Ser Leu Val Leu Gly Val Val Met Asn Gly
145                 150                 155                 160

att gcg cag ggc cgc tgc ggc tgg gtc atg cac gag atg ggc cac ggg       528
Ile Ala Gln Gly Arg Cys Gly Trp Val Met His Glu Met Gly His Gly
                165                 170                 175

tcg ttc acg ggc gtc atc tgg ctc gac gac cgg atg tgc gag ttc ttc       576
Ser Phe Thr Gly Val Ile Trp Leu Asp Asp Arg Met Cys Glu Phe Phe
            180                 185                 190

tac ggc gtc ggc tgc ggc atg agc ggg cac tac tgg aag aac cag cac       624
Tyr Gly Val Gly Cys Gly Met Ser Gly His Tyr Trp Lys Asn Gln His
        195                 200                 205

agc aag cac cac gcc gcg ccc aac cgc ctc gag cac gat gtc gat ctc       672
Ser Lys His His Ala Ala Pro Asn Arg Leu Glu His Asp Val Asp Leu
    210                 215                 220

aac acg ctg ccc ctg gtc gcc ttt aac gag cgc gtc gtg cgc aag gtc       720
Asn Thr Leu Pro Leu Val Ala Phe Asn Glu Arg Val Val Arg Lys Val
225                 230                 235                 240

aag ccg gga tcg ctg ctg gcg ctc tgg ctg cgc gtg cag gcg tac ctc       768
Lys Pro Gly Ser Leu Leu Ala Leu Trp Leu Arg Val Gln Ala Tyr Leu
                245                 250                 255

ttt gcg ccc gtc tcg tgc ctg ctc atc ggc ctt ggc tgg acg ctc tac       816
Phe Ala Pro Val Ser Cys Leu Leu Ile Gly Leu Gly Trp Thr Leu Tyr
            260                 265                 270

ctg cac ccg cgc tac atg ctg cgc acc aag cgg cac atg gag ttc gtc       864
Leu His Pro Arg Tyr Met Leu Arg Thr Lys Arg His Met Glu Phe Val
        275                 280                 285

tgg atc ttc gcg cgc tac att ggc tgg ttc tcg ctc atg ggc gct ctc       912
Trp Ile Phe Ala Arg Tyr Ile Gly Trp Phe Ser Leu Met Gly Ala Leu
    290                 295                 300

ggc tac tcg ccg ggc acc tcg gtc ggg atg tac ctg tgc tcg ttc ggc       960
Gly Tyr Ser Pro Gly Thr Ser Val Gly Met Tyr Leu Cys Ser Phe Gly
305                 310                 315                 320

ctc ggc tgc att tac att ttc ctg cag ttc gcc gtc agc cac acg cac      1008
Leu Gly Cys Ile Tyr Ile Phe Leu Gln Phe Ala Val Ser His Thr His
```

```
                    325                 330                 335

ctg ccg gtg acc aac ccg gag gac cag ctg cac tgg ctc gag tac gcg      1056
Leu Pro Val Thr Asn Pro Glu Asp Gln Leu His Trp Leu Glu Tyr Ala
                340                 345                 350

gcc gac cac acg gtg aac att agc acc aag tcc tgg ctc gtc acg tgg      1104
Ala Asp His Thr Val Asn Ile Ser Thr Lys Ser Trp Leu Val Thr Trp
            355                 360                 365

tgg atg tcg aac ctg aac ttt cag atc gag cac cac ctc ttc ccc acg      1152
Trp Met Ser Asn Leu Asn Phe Gln Ile Glu His His Leu Phe Pro Thr
        370                 375                 380

gcg ccg cag ttc cgc ttc aag gaa atc agt cct cgc gtc gag gcc ctc      1200
Ala Pro Gln Phe Arg Phe Lys Glu Ile Ser Pro Arg Val Glu Ala Leu
385                 390                 395                 400

ttc aag cgc cac aac ctc ccg tac tac gac ctg ccc tac acg agc gcg      1248
Phe Lys Arg His Asn Leu Pro Tyr Tyr Asp Leu Pro Tyr Thr Ser Ala
                405                 410                 415

gtc tcg acc acc ttt gcc aat ctt tat tcc gtc ggc cac tcg gtc ggc      1296
Val Ser Thr Thr Phe Ala Asn Leu Tyr Ser Val Gly His Ser Val Gly
                420                 425                 430

gcc gac acc aag aag cag gac tga                                      1320
Ala Asp Thr Lys Lys Gln Asp
            435
```

<210> 12
<211> 439
<212> PRT
<213> Thraustrochytrium

<400> 12

```
Met Gly Lys Gly Ser Glu Gly Arg Ser Ala Ala Arg Glu Met Thr Ala
1               5                   10                  15

Glu Ala Asn Gly Asp Lys Arg Lys Thr Ile Leu Ile Glu Gly Val Leu
            20                  25                  30

Tyr Asp Ala Thr Asn Phe Lys His Pro Gly Gly Ser Ile Ile Asn Phe
        35                  40                  45

Leu Thr Glu Gly Glu Ala Gly Val Asp Ala Thr Gln Ala Tyr Arg Glu
    50                  55                  60

Phe His Gln Arg Ser Gly Lys Ala Asp Lys Tyr Leu Lys Ser Leu Pro
65                  70                  75                  80

Lys Leu Asp Ala Ser Lys Val Glu Ser Arg Phe Ser Ala Lys Glu Gln
                85                  90                  95

Ala Arg Arg Asp Ala Met Thr Arg Asp Tyr Ala Ala Phe Arg Glu Glu
```

100 105 110

Leu Val Ala Glu Gly Tyr Phe Asp Pro Ser Ile Pro His Met Ile Tyr
115 120 125

Arg Val Val Glu Ile Val Ala Leu Phe Ala Leu Ser Phe Trp Leu Met
130 135 140

Ser Lys Ala Ser Pro Thr Ser Leu Val Leu Gly Val Val Met Asn Gly
145 150 155 160

Ile Ala Gln Gly Arg Cys Gly Trp Val Met His Glu Met Gly His Gly
165 170 175

Ser Phe Thr Gly Val Ile Trp Leu Asp Asp Arg Met Cys Glu Phe Phe
180 185 190

Tyr Gly Val Gly Cys Gly Met Ser Gly His Tyr Trp Lys Asn Gln His
195 200 205

Ser Lys His His Ala Ala Pro Asn Arg Leu Glu His Asp Val Asp Leu
210 215 220

Asn Thr Leu Pro Leu Val Ala Phe Asn Glu Arg Val Val Arg Lys Val
225 230 235 240

Lys Pro Gly Ser Leu Leu Ala Leu Trp Leu Arg Val Gln Ala Tyr Leu
245 250 255

Phe Ala Pro Val Ser Cys Leu Leu Ile Gly Leu Gly Trp Thr Leu Tyr
260 265 270

Leu His Pro Arg Tyr Met Leu Arg Thr Lys Arg His Met Glu Phe Val
275 280 285

Trp Ile Phe Ala Arg Tyr Ile Gly Trp Phe Ser Leu Met Gly Ala Leu
290 295 300

Gly Tyr Ser Pro Gly Thr Ser Val Gly Met Tyr Leu Cys Ser Phe Gly
305 310 315 320

Leu Gly Cys Ile Tyr Ile Phe Leu Gln Phe Ala Val Ser His Thr His
325 330 335

Leu Pro Val Thr Asn Pro Glu Asp Gln Leu His Trp Leu Glu Tyr Ala
340 345 350

Ala Asp His Thr Val Asn Ile Ser Thr Lys Ser Trp Leu Val Thr Trp
355 360 365

Trp Met Ser Asn Leu Asn Phe Gln Ile Glu His His Leu Phe Pro Thr

```
                370                 375                 380

Ala Pro Gln Phe Arg Phe Lys Glu Ile Ser Pro Arg Val Glu Ala Leu
385                 390                 395                 400

Phe Lys Arg His Asn Leu Pro Tyr Tyr Asp Leu Pro Tyr Thr Ser Ala
                405                 410                 415

Val Ser Thr Thr Phe Ala Asn Leu Tyr Ser Val Gly His Ser Val Gly
            420                 425                 430

Ala Asp Thr Lys Lys Gln Asp
        435
```

<210> 13
<211> 1341
<212> DNA
<213> Mortierella alpina

<220>
<221> CDS
<222> (1)..(1341)
<223> Delta-5-Desaturase

<400> 13

```
atg gga acg gac caa gga aaa acc ttc acc tgg gaa gag ctg gcg gcc      48
Met Gly Thr Asp Gln Gly Lys Thr Phe Thr Trp Glu Glu Leu Ala Ala
1               5                   10                  15

cat aac acc aag gac gac cta ctc ttg gcc atc cgc ggc agg gtg tac      96
His Asn Thr Lys Asp Asp Leu Leu Leu Ala Ile Arg Gly Arg Val Tyr
            20                  25                  30

gat gtc aca aag ttc ttg agc cgc cat cct ggt gga gtg gac act ctc     144
Asp Val Thr Lys Phe Leu Ser Arg His Pro Gly Gly Val Asp Thr Leu
        35                  40                  45

ctg ctc gga gct ggc cga gat gtt act ccg gtc ttt gag atg tat cac     192
Leu Leu Gly Ala Gly Arg Asp Val Thr Pro Val Phe Glu Met Tyr His
    50                  55                  60

gcg ttt ggg gct gca gat gcc att atg aag aag tac tat gtc ggt aca     240
Ala Phe Gly Ala Ala Asp Ala Ile Met Lys Lys Tyr Tyr Val Gly Thr
65                  70                  75                  80

ctg gtc tcg aat gag ctg ccc atc ttc ccg gag cca acg gtg ttc cac     288
Leu Val Ser Asn Glu Leu Pro Ile Phe Pro Glu Pro Thr Val Phe His
                85                  90                  95

aaa acc atc aag acg aga gtc gag ggc tac ttt acg gat cgg aac att     336
Lys Thr Ile Lys Thr Arg Val Glu Gly Tyr Phe Thr Asp Arg Asn Ile
            100                 105                 110
```

```
gat ccc aag aat aga cca gag atc tgg gga cga tac gct ctt atc ttt      384
Asp Pro Lys Asn Arg Pro Glu Ile Trp Gly Arg Tyr Ala Leu Ile Phe
        115                 120                 125

gga tcc ttg atc gct tcc tac tac gcg cag ctc ttt gtg cct ttc gtt      432
Gly Ser Leu Ile Ala Ser Tyr Tyr Ala Gln Leu Phe Val Pro Phe Val
    130                 135                 140

gtc gaa cgc aca tgg ctt cag gtg gtg ttt gca atc atc atg gga ttt      480
Val Glu Arg Thr Trp Leu Gln Val Val Phe Ala Ile Ile Met Gly Phe
145                 150                 155                 160

gcg tgc gca caa gtc gga ctc aac cct ctt cat gat gcg tct cac ttt      528
Ala Cys Ala Gln Val Gly Leu Asn Pro Leu His Asp Ala Ser His Phe
                165                 170                 175

tca gtg acc cac aac ccc act gtc tgg aag att ctg gga gcc acg cac      576
Ser Val Thr His Asn Pro Thr Val Trp Lys Ile Leu Gly Ala Thr His
            180                 185                 190

gac ttt ttc aac gga gca tcg tac ctg gtg tgg atg tac caa cat atg      624
Asp Phe Phe Asn Gly Ala Ser Tyr Leu Val Trp Met Tyr Gln His Met
        195                 200                 205

ctc ggc cat cac ccc tac acc aac att gct gga gca gat ccc gac gtg      672
Leu Gly His His Pro Tyr Thr Asn Ile Ala Gly Ala Asp Pro Asp Val
    210                 215                 220

tcg acg tct gag ccc gat gtt cgt cgt atc aag ccc aac caa aag tgg      720
Ser Thr Ser Glu Pro Asp Val Arg Arg Ile Lys Pro Asn Gln Lys Trp
225                 230                 235                 240

ttt gtc aac cac atc aac cag cac atg ttt gtt cct ttc ctg tac gga      768
Phe Val Asn His Ile Asn Gln His Met Phe Val Pro Phe Leu Tyr Gly
                245                 250                 255

ctg ctg gcg ttc aag gtg cgc att cag gac atc aac att ttg tac ttt      816
Leu Leu Ala Phe Lys Val Arg Ile Gln Asp Ile Asn Ile Leu Tyr Phe
            260                 265                 270

gtc aag acc aat gac gct att cgt gtc aat ccc atc tcg aca tgg cac      864
Val Lys Thr Asn Asp Ala Ile Arg Val Asn Pro Ile Ser Thr Trp His
        275                 280                 285

act gtg atg ttc tgg ggc ggc aag gct ttc ttt gtc tgg tat cgc ctg      912
Thr Val Met Phe Trp Gly Gly Lys Ala Phe Phe Val Trp Tyr Arg Leu
    290                 295                 300

att gtt ccc ctg cag tat ctg ccc ctg ggc aag gtg ctg ctc ttg ttc      960
Ile Val Pro Leu Gln Tyr Leu Pro Leu Gly Lys Val Leu Leu Leu Phe
305                 310                 315                 320

acg gtc gcg gac atg gtg tcg tct tac tgg ctg gcg ctg acc ttc cag     1008
Thr Val Ala Asp Met Val Ser Ser Tyr Trp Leu Ala Leu Thr Phe Gln
                325                 330                 335

gcg aac cac gtt gtt gag gaa gtt cag tgg ccg ttg cct gac gag aac     1056
Ala Asn His Val Val Glu Glu Val Gln Trp Pro Leu Pro Asp Glu Asn
            340                 345                 350

ggg atc atc caa aag gac tgg gca gct atg cag gtc gag act acg cag     1104
Gly Ile Ile Gln Lys Asp Trp Ala Ala Met Gln Val Glu Thr Thr Gln
        355                 360                 365

gat tac gca cac gat tcg cac ctc tgg acc agc atc act ggc agc ttg     1152
Asp Tyr Ala His Asp Ser His Leu Trp Thr Ser Ile Thr Gly Ser Leu
    370                 375                 380
```

```
aac tac cag gct gtg cac cat ctg ttc ccc aac gtg tcg cag cac cat      1200
Asn Tyr Gln Ala Val His His Leu Phe Pro Asn Val Ser Gln His His
385                 390                 395                 400

tat ccc gat att ctg gcc atc atc aag aac acc tgc agc gag tac aag      1248
Tyr Pro Asp Ile Leu Ala Ile Ile Lys Asn Thr Cys Ser Glu Tyr Lys
                405                 410                 415

gtt cca tac ctt gtc aag gat acg ttt tgg caa gca ttt gct tca cat      1296
Val Pro Tyr Leu Val Lys Asp Thr Phe Trp Gln Ala Phe Ala Ser His
            420                 425                 430

ttg gag cac ttg cgt gtt ctt gga ctc cgt ccc aag gaa gag tag          1341
Leu Glu His Leu Arg Val Leu Gly Leu Arg Pro Lys Glu Glu
        435                 440                 445
```

<210> 14
<211> 446
<212> PRT
<213> Mortierella alpina

<400> 14

```
Met Gly Thr Asp Gln Gly Lys Thr Phe Thr Trp Glu Glu Leu Ala Ala
1               5                   10                  15

His Asn Thr Lys Asp Asp Leu Leu Leu Ala Ile Arg Gly Arg Val Tyr
            20                  25                  30

Asp Val Thr Lys Phe Leu Ser Arg His Pro Gly Gly Val Asp Thr Leu
        35                  40                  45

Leu Leu Gly Ala Gly Arg Asp Val Thr Pro Val Phe Glu Met Tyr His
    50                  55                  60

Ala Phe Gly Ala Ala Asp Ala Ile Met Lys Lys Tyr Tyr Val Gly Thr
65                  70                  75                  80

Leu Val Ser Asn Glu Leu Pro Ile Phe Pro Glu Pro Thr Val Phe His
                85                  90                  95

Lys Thr Ile Lys Thr Arg Val Glu Gly Tyr Phe Thr Asp Arg Asn Ile
            100                 105                 110

Asp Pro Lys Asn Arg Pro Glu Ile Trp Gly Arg Tyr Ala Leu Ile Phe
        115                 120                 125

Gly Ser Leu Ile Ala Ser Tyr Tyr Ala Gln Leu Phe Val Pro Phe Val
    130                 135                 140

Val Glu Arg Thr Trp Leu Gln Val Val Phe Ala Ile Ile Met Gly Phe
145                 150                 155                 160
```

```
Ala Cys Ala Gln Val Gly Leu Asn Pro Leu His Asp Ala Ser His Phe
                165                 170                 175

Ser Val Thr His Asn Pro Thr Val Trp Lys Ile Leu Gly Ala Thr His
            180                 185                 190

Asp Phe Phe Asn Gly Ala Ser Tyr Leu Val Trp Met Tyr Gln His Met
        195                 200                 205

Leu Gly His His Pro Tyr Thr Asn Ile Ala Gly Ala Asp Pro Asp Val
    210                 215                 220

Ser Thr Ser Glu Pro Asp Val Arg Arg Ile Lys Pro Asn Gln Lys Trp
225                 230                 235                 240

Phe Val Asn His Ile Asn Gln His Met Phe Val Pro Phe Leu Tyr Gly
                245                 250                 255

Leu Leu Ala Phe Lys Val Arg Ile Gln Asp Ile Asn Ile Leu Tyr Phe
            260                 265                 270

Val Lys Thr Asn Asp Ala Ile Arg Val Asn Pro Ile Ser Thr Trp His
        275                 280                 285

Thr Val Met Phe Trp Gly Gly Lys Ala Phe Phe Val Trp Tyr Arg Leu
    290                 295                 300

Ile Val Pro Leu Gln Tyr Leu Pro Leu Gly Lys Val Leu Leu Leu Phe
305                 310                 315                 320

Thr Val Ala Asp Met Val Ser Ser Tyr Trp Leu Ala Leu Thr Phe Gln
                325                 330                 335

Ala Asn His Val Val Glu Glu Val Gln Trp Pro Leu Pro Asp Glu Asn
            340                 345                 350

Gly Ile Ile Gln Lys Asp Trp Ala Ala Met Gln Val Glu Thr Thr Gln
        355                 360                 365

Asp Tyr Ala His Asp Ser His Leu Trp Thr Ser Ile Thr Gly Ser Leu
    370                 375                 380

Asn Tyr Gln Ala Val His His Leu Phe Pro Asn Val Ser Gln His His
385                 390                 395                 400

Tyr Pro Asp Ile Leu Ala Ile Ile Lys Asn Thr Cys Ser Glu Tyr Lys
                405                 410                 415

Val Pro Tyr Leu Val Lys Asp Thr Phe Trp Gln Ala Phe Ala Ser His
            420                 425                 430
```

```
Leu Glu His Leu Arg Val Leu Gly Leu Arg Pro Lys Glu Glu
        435                 440                 445
```

<210> 15
<211> 1344
<212> DNA
<213> Caenorhabditis elegans

<220>
<221> CDS
<222> (1)..(1344)
<223> Delta-5-Desaturase

<400> 15

```
atg gta tta cga gag caa gag cat gag cca ttc ttc att aaa att gat      48
Met Val Leu Arg Glu Gln Glu His Glu Pro Phe Phe Ile Lys Ile Asp
1               5                   10                  15

gga aaa tgg tgt caa att gac gat gct gtc ctg aga tca cat cca ggt      96
Gly Lys Trp Cys Gln Ile Asp Asp Ala Val Leu Arg Ser His Pro Gly
            20                  25                  30

ggt agt gca att act acc tat aaa aat atg gat gcc act acc gta ttc     144
Gly Ser Ala Ile Thr Thr Tyr Lys Asn Met Asp Ala Thr Thr Val Phe
        35                  40                  45

cac aca ttc cat act ggt tct aaa gaa gcg tat caa tgg ctg aca gaa     192
His Thr Phe His Thr Gly Ser Lys Glu Ala Tyr Gln Trp Leu Thr Glu
    50                  55                  60

ttg aaa aaa gag tgc cct aca caa gaa cca gag atc cca gat att aag     240
Leu Lys Lys Glu Cys Pro Thr Gln Glu Pro Glu Ile Pro Asp Ile Lys
65                  70                  75                  80

gat gac cca atc aaa gga att gat gat gtg aac atg gga act ttc aat     288
Asp Asp Pro Ile Lys Gly Ile Asp Asp Val Asn Met Gly Thr Phe Asn
                85                  90                  95

att tct gag aaa cga tct gcc caa ata aat aaa agt ttc act gat cta     336
Ile Ser Glu Lys Arg Ser Ala Gln Ile Asn Lys Ser Phe Thr Asp Leu
            100                 105                 110

cgt atg cga gtt cgt gca gaa gga ctt atg gat gga tct cct ttg ttc     384
Arg Met Arg Val Arg Ala Glu Gly Leu Met Asp Gly Ser Pro Leu Phe
        115                 120                 125

tac att aga aaa att ctt gaa aca atc ttc aca att ctt ttt gca ttc     432
Tyr Ile Arg Lys Ile Leu Glu Thr Ile Phe Thr Ile Leu Phe Ala Phe
    130                 135                 140

tac ctt caa tac cac aca tat tat ctt cca tca gct att cta atg gga     480
Tyr Leu Gln Tyr His Thr Tyr Tyr Leu Pro Ser Ala Ile Leu Met Gly
145                 150                 155                 160

gtt gcg tgg caa caa ttg gga tgg tta atc cat gaa ttc gca cat cat     528
Val Ala Trp Gln Gln Leu Gly Trp Leu Ile His Glu Phe Ala His His
```

```
            165                 170                 175

cag ttg ttc aaa aac aga tac tac aat gat ttg gcc agc tat ttc gtt      576
Gln Leu Phe Lys Asn Arg Tyr Tyr Asn Asp Leu Ala Ser Tyr Phe Val
        180                 185                 190

gga aac ttt tta caa gga ttc tca tct ggt ggt tgg aaa gag cag cac      624
Gly Asn Phe Leu Gln Gly Phe Ser Ser Gly Gly Trp Lys Glu Gln His
        195                 200                 205

aat gtg cat cac gca gcc aca aat gtt gtt gga cga gac gga gat ctt      672
Asn Val His His Ala Ala Thr Asn Val Val Gly Arg Asp Gly Asp Leu
    210                 215                 220

gat tta gtc cca ttc tat gct aca gtg gca gaa cat ctc aac aat tat      720
Asp Leu Val Pro Phe Tyr Ala Thr Val Ala Glu His Leu Asn Asn Tyr
225                 230                 235                 240

tct cag gat tca tgg gtt atg act cta ttc aga tgg caa cat gtt cat      768
Ser Gln Asp Ser Trp Val Met Thr Leu Phe Arg Trp Gln His Val His
            245                 250                 255

tgg aca ttc atg tta cca ttc ctc cgt ctc tcg tgg ctt ctt cag tca      816
Trp Thr Phe Met Leu Pro Phe Leu Arg Leu Ser Trp Leu Leu Gln Ser
        260                 265                 270

atc att ttt gtt agt cag atg cca act cat tat tat gac tat tac aga      864
Ile Ile Phe Val Ser Gln Met Pro Thr His Tyr Tyr Asp Tyr Tyr Arg
        275                 280                 285

aat act gcg att tat gaa cag gtt ggt ctc tct ttg cac tgg gct tgg      912
Asn Thr Ala Ile Tyr Glu Gln Val Gly Leu Ser Leu His Trp Ala Trp
    290                 295                 300

tca ttg ggt caa ttg tat ttc cta ccc gat tgg tca act aga ata atg      960
Ser Leu Gly Gln Leu Tyr Phe Leu Pro Asp Trp Ser Thr Arg Ile Met
305                 310                 315                 320

ttc ttc ctt gtt tct cat ctt gtt gga ggt ttc ctg ctc tct cat gta     1008
Phe Phe Leu Val Ser His Leu Val Gly Gly Phe Leu Leu Ser His Val
            325                 330                 335

gtt act ttc aat cat tat tca gtg gag aag ttt gca ttg agc tcg aac     1056
Val Thr Phe Asn His Tyr Ser Val Glu Lys Phe Ala Leu Ser Ser Asn
        340                 345                 350

atc atg tca aat tac gct tgt ctt caa atc atg acc aca aga aat atg     1104
Ile Met Ser Asn Tyr Ala Cys Leu Gln Ile Met Thr Thr Arg Asn Met
        355                 360                 365

aga cct gga aga ttc att gac tgg ctt tgg gga ggt ctt aac tat cag     1152
Arg Pro Gly Arg Phe Ile Asp Trp Leu Trp Gly Gly Leu Asn Tyr Gln
    370                 375                 380

att gag cac cat ctt ttc cca acg atg cca cga cac aac ttg aac act     1200
Ile Glu His His Leu Phe Pro Thr Met Pro Arg His Asn Leu Asn Thr
385                 390                 395                 400

gtt atg cca ctt gtt aag gag ttt gca gca gca aat ggt tta cca tac     1248
Val Met Pro Leu Val Lys Glu Phe Ala Ala Ala Asn Gly Leu Pro Tyr
            405                 410                 415

atg gtc gac gat tat ttc aca gga ttc tgg ctt gaa att gag caa ttc     1296
Met Val Asp Asp Tyr Phe Thr Gly Phe Trp Leu Glu Ile Glu Gln Phe
        420                 425                 430

cga aat att gca aat gtt gct gct aaa ttg act aaa aag att gcc tag     1344
Arg Asn Ile Ala Asn Val Ala Ala Lys Leu Thr Lys Lys Ile Ala

          435                 440                 445
```

<210> 16
<211> 447
<212> PRT
<213> Caenorhabditis elegans

<400> 16

```
Met Val Leu Arg Glu Gln Glu His Glu Pro Phe Phe Ile Lys Ile Asp
1               5                   10                  15

Gly Lys Trp Cys Gln Ile Asp Asp Ala Val Leu Arg Ser His Pro Gly
            20                  25                  30

Gly Ser Ala Ile Thr Thr Tyr Lys Asn Met Asp Ala Thr Thr Val Phe
        35                  40                  45

His Thr Phe His Thr Gly Ser Lys Glu Ala Tyr Gln Trp Leu Thr Glu
    50                  55                  60

Leu Lys Lys Glu Cys Pro Thr Gln Glu Pro Glu Ile Pro Asp Ile Lys
65                  70                  75                  80

Asp Asp Pro Ile Lys Gly Ile Asp Asp Val Asn Met Gly Thr Phe Asn
                85                  90                  95

Ile Ser Glu Lys Arg Ser Ala Gln Ile Asn Lys Ser Phe Thr Asp Leu
            100                 105                 110

Arg Met Arg Val Arg Ala Glu Gly Leu Met Asp Gly Ser Pro Leu Phe
        115                 120                 125

Tyr Ile Arg Lys Ile Leu Glu Thr Ile Phe Thr Ile Leu Phe Ala Phe
    130                 135                 140

Tyr Leu Gln Tyr His Thr Tyr Tyr Leu Pro Ser Ala Ile Leu Met Gly
145                 150                 155                 160

Val Ala Trp Gln Gln Leu Gly Trp Leu Ile His Glu Phe Ala His His
                165                 170                 175

Gln Leu Phe Lys Asn Arg Tyr Tyr Asn Asp Leu Ala Ser Tyr Phe Val
            180                 185                 190

Gly Asn Phe Leu Gln Gly Phe Ser Ser Gly Gly Trp Lys Glu Gln His
        195                 200                 205

Asn Val His His Ala Ala Thr Asn Val Val Gly Arg Asp Gly Asp Leu
```

```
            210                 215                 220

Asp Leu Val Pro Phe Tyr Ala Thr Val Ala Glu His Leu Asn Asn Tyr
225                 230                 235                 240

Ser Gln Asp Ser Trp Val Met Thr Leu Phe Arg Trp Gln His Val His
                245                 250                 255

Trp Thr Phe Met Leu Pro Phe Leu Arg Leu Ser Trp Leu Leu Gln Ser
            260                 265                 270

Ile Ile Phe Val Ser Gln Met Pro Thr His Tyr Tyr Asp Tyr Tyr Arg
        275                 280                 285

Asn Thr Ala Ile Tyr Glu Gln Val Gly Leu Ser Leu His Trp Ala Trp
    290                 295                 300

Ser Leu Gly Gln Leu Tyr Phe Leu Pro Asp Trp Ser Thr Arg Ile Met
305                 310                 315                 320

Phe Phe Leu Val Ser His Leu Val Gly Gly Phe Leu Leu Ser His Val
                325                 330                 335

Val Thr Phe Asn His Tyr Ser Val Glu Lys Phe Ala Leu Ser Ser Asn
            340                 345                 350

Ile Met Ser Asn Tyr Ala Cys Leu Gln Ile Met Thr Thr Arg Asn Met
        355                 360                 365

Arg Pro Gly Arg Phe Ile Asp Trp Leu Trp Gly Gly Leu Asn Tyr Gln
    370                 375                 380

Ile Glu His His Leu Phe Pro Thr Met Pro Arg His Asn Leu Asn Thr
385                 390                 395                 400

Val Met Pro Leu Val Lys Glu Phe Ala Ala Ala Asn Gly Leu Pro Tyr
                405                 410                 415

Met Val Asp Asp Tyr Phe Thr Gly Phe Trp Leu Glu Ile Glu Gln Phe
            420                 425                 430

Arg Asn Ile Ala Asn Val Ala Ala Lys Leu Thr Lys Lys Ile Ala
        435                 440                 445
```

<210> 17
<211> 1683
<212> DNA
<213> Borago officinalis

<220>
<221> CDS
<222> (42)..(1388)
<223> Delta-6-Desaturase

<400> 17

```
tatctgccta ccctcccaaa gagagtagtc atttttcatc a atg gct gct caa atc      56
                                              Met Ala Ala Gln Ile
                                              1               5

aag aaa tac att acc tca gat gaa ctc aag aac cac gat aaa ccc gga       104
Lys Lys Tyr Ile Thr Ser Asp Glu Leu Lys Asn His Asp Lys Pro Gly
                10                  15                  20

gat cta tgg atc tcg att caa ggg aaa gcc tat gat gtt tcg gat tgg       152
Asp Leu Trp Ile Ser Ile Gln Gly Lys Ala Tyr Asp Val Ser Asp Trp
            25                  30                  35

gtg aaa gac cat cca ggt ggc agc ttt ccc ttg aag agt ctt gct ggt       200
Val Lys Asp His Pro Gly Gly Ser Phe Pro Leu Lys Ser Leu Ala Gly
        40                  45                  50

caa gag gta act gat gca ttt gtt gca ttc cat cct gcc tct aca tgg       248
Gln Glu Val Thr Asp Ala Phe Val Ala Phe His Pro Ala Ser Thr Trp
    55                  60                  65

aag aat ctt gat aag ttt ttc act ggg tat tat ctt aaa gat tac tct       296
Lys Asn Leu Asp Lys Phe Phe Thr Gly Tyr Tyr Leu Lys Asp Tyr Ser
70                  75                  80                  85

gtt tct gag gtt tct aaa gat tat agg aag ctt gtg ttt gag ttt tct       344
Val Ser Glu Val Ser Lys Asp Tyr Arg Lys Leu Val Phe Glu Phe Ser
                90                  95                  100

aaa atg ggt ttg tat gac aaa aaa ggt cat att atg ttt gca act ttg       392
Lys Met Gly Leu Tyr Asp Lys Lys Gly His Ile Met Phe Ala Thr Leu
            105                 110                 115

tgc ttt ata gca atg ctg ttt gct atg agt gtt tat ggg gtt ttg ttt       440
Cys Phe Ile Ala Met Leu Phe Ala Met Ser Val Tyr Gly Val Leu Phe
        120                 125                 130

tgt gag ggt gtt ttg gta cat ttg ttt tct ggg tgt ttg atg ggg ttt       488
Cys Glu Gly Val Leu Val His Leu Phe Ser Gly Cys Leu Met Gly Phe
    135                 140                 145

ctt tgg att cag agt ggt tgg att gga cat gat gct ggg cat tat atg       536
Leu Trp Ile Gln Ser Gly Trp Ile Gly His Asp Ala Gly His Tyr Met
150                 155                 160                 165

gta gtg tct gat tca agg ctt aat aag ttt atg ggt att ttt gct gca       584
Val Val Ser Asp Ser Arg Leu Asn Lys Phe Met Gly Ile Phe Ala Ala
                170                 175                 180

aat tgt ctt tca gga ata agt att ggt tgg tgg aaa tgg aac cat aat       632
Asn Cys Leu Ser Gly Ile Ser Ile Gly Trp Trp Lys Trp Asn His Asn
            185                 190                 195

gca cat cac att gcc tgt aat agc ctt gaa tat gac cct gat tta caa       680
Ala His His Ile Ala Cys Asn Ser Leu Glu Tyr Asp Pro Asp Leu Gln
        200                 205                 210
```

```
tat ata cca ttc ctt gtt gtg tct tcc aag ttt ttt ggt tca ctc acc      728
Tyr Ile Pro Phe Leu Val Val Ser Ser Lys Phe Phe Gly Ser Leu Thr
    215                 220                 225

tct cat ttc tat gag aaa agg ttg act ttt gac tct tta tca aga ttc      776
Ser His Phe Tyr Glu Lys Arg Leu Thr Phe Asp Ser Leu Ser Arg Phe
230                 235                 240                 245

ttt gta agt tat caa cat tgg aca ttt tac cct att atg tgt gct gct      824
Phe Val Ser Tyr Gln His Trp Thr Phe Tyr Pro Ile Met Cys Ala Ala
                250                 255                 260

agg ctc aat atg tat gta caa tct ctc ata atg ttg ttg acc aag aga      872
Arg Leu Asn Met Tyr Val Gln Ser Leu Ile Met Leu Leu Thr Lys Arg
            265                 270                 275

aat gtg tcc tat cga gct cag gaa ctc ttg gga tgc cta gtg ttc tcg      920
Asn Val Ser Tyr Arg Ala Gln Glu Leu Leu Gly Cys Leu Val Phe Ser
        280                 285                 290

att tgg tac ccg ttg ctt gtt tct tgt ttg cct aat tgg ggt gaa aga      968
Ile Trp Tyr Pro Leu Leu Val Ser Cys Leu Pro Asn Trp Gly Glu Arg
    295                 300                 305

att atg ttt gtt att gca agt tta tca gtg act gga atg caa caa gtt     1016
Ile Met Phe Val Ile Ala Ser Leu Ser Val Thr Gly Met Gln Gln Val
310                 315                 320                 325

cag ttc tcc ttg aac cac ttc tct tca agt gtt tat gtt gga aag cct     1064
Gln Phe Ser Leu Asn His Phe Ser Ser Ser Val Tyr Val Gly Lys Pro
                330                 335                 340

aaa ggg aat aat tgg ttt gag aaa caa acg gat ggg aca ctt gac att     1112
Lys Gly Asn Asn Trp Phe Glu Lys Gln Thr Asp Gly Thr Leu Asp Ile
            345                 350                 355

tct tgt cct cct tgg atg gat tgg ttt cat ggt gga ttg caa ttc caa     1160
Ser Cys Pro Pro Trp Met Asp Trp Phe His Gly Gly Leu Gln Phe Gln
        360                 365                 370

att gag cat cat ttg ttt ccc aag atg cct aga tgc aac ctt agg aaa     1208
Ile Glu His His Leu Phe Pro Lys Met Pro Arg Cys Asn Leu Arg Lys
    375                 380                 385

atc tcg ccc tac gtg atc gag tta tgc aag aaa cat aat ttg cct tac     1256
Ile Ser Pro Tyr Val Ile Glu Leu Cys Lys Lys His Asn Leu Pro Tyr
390                 395                 400                 405

aat tat gca tct ttc tcc aag gcc aat gaa atg aca ctc aga aca ttg     1304
Asn Tyr Ala Ser Phe Ser Lys Ala Asn Glu Met Thr Leu Arg Thr Leu
                410                 415                 420

agg aac aca gca ttg cag gct agg gat ata acc aag ccg ctc ccg aag     1352
Arg Asn Thr Ala Leu Gln Ala Arg Asp Ile Thr Lys Pro Leu Pro Lys
            425                 430                 435

aat ttg gta tgg gaa gct ctt cac act cat ggt taa aattaccctt          1398
Asn Leu Val Trp Glu Ala Leu His Thr His Gly
        440                 445

agttcatgta ataatttgag attatgtatc tcctatgttt gtgtcttgtc ttggttctac   1458

ttgttggagt cattgcaact tgtcttttat ggtttattag atgtttttta atatatttta   1518

gaggttttgc tttcatctcc attattgatg aataaggagt tgcatattgt caattgttgt   1578

gctcaatatc tgatattttg gaatgtactt tgtaccactg tgttttcagt tgaagctcat   1638

gtgtacttct atagactttg tttaaatggt tatgtcatgt tattt                   1683
```

<210> 18
<211> 448
<212> PRT
<213> Borago officinalis

<400> 18

```
Met Ala Ala Gln Ile Lys Lys Tyr Ile Thr Ser Asp Glu Leu Lys Asn
1               5                   10                  15

His Asp Lys Pro Gly Asp Leu Trp Ile Ser Ile Gln Gly Lys Ala Tyr
            20                  25                  30

Asp Val Ser Asp Trp Val Lys Asp His Pro Gly Gly Ser Phe Pro Leu
        35                  40                  45

Lys Ser Leu Ala Gly Gln Glu Val Thr Asp Ala Phe Val Ala Phe His
    50                  55                  60

Pro Ala Ser Thr Trp Lys Asn Leu Asp Lys Phe Phe Thr Gly Tyr Tyr
65                  70                  75                  80

Leu Lys Asp Tyr Ser Val Ser Glu Val Ser Lys Asp Tyr Arg Lys Leu
                85                  90                  95

Val Phe Glu Phe Ser Lys Met Gly Leu Tyr Asp Lys Lys Gly His Ile
            100                 105                 110

Met Phe Ala Thr Leu Cys Phe Ile Ala Met Leu Phe Ala Met Ser Val
        115                 120                 125

Tyr Gly Val Leu Phe Cys Glu Gly Val Leu Val His Leu Phe Ser Gly
    130                 135                 140

Cys Leu Met Gly Phe Leu Trp Ile Gln Ser Gly Trp Ile Gly His Asp
145                 150                 155                 160

Ala Gly His Tyr Met Val Val Ser Asp Ser Arg Leu Asn Lys Phe Met
                165                 170                 175

Gly Ile Phe Ala Ala Asn Cys Leu Ser Gly Ile Ser Ile Gly Trp Trp
            180                 185                 190

Lys Trp Asn His Asn Ala His His Ile Ala Cys Asn Ser Leu Glu Tyr
        195                 200                 205
```

```
Asp Pro Asp Leu Gln Tyr Ile Pro Phe Leu Val Val Ser Ser Lys Phe
    210                 215                 220

Phe Gly Ser Leu Thr Ser His Phe Tyr Glu Lys Arg Leu Thr Phe Asp
225                 230                 235                 240

Ser Leu Ser Arg Phe Phe Val Ser Tyr Gln His Trp Thr Phe Tyr Pro
                245                 250                 255

Ile Met Cys Ala Ala Arg Leu Asn Met Tyr Val Gln Ser Leu Ile Met
            260                 265                 270

Leu Leu Thr Lys Arg Asn Val Ser Tyr Arg Ala Gln Glu Leu Leu Gly
        275                 280                 285

Cys Leu Val Phe Ser Ile Trp Tyr Pro Leu Leu Val Ser Cys Leu Pro
    290                 295                 300

Asn Trp Gly Glu Arg Ile Met Phe Val Ile Ala Ser Leu Ser Val Thr
305                 310                 315                 320

Gly Met Gln Gln Val Gln Phe Ser Leu Asn His Phe Ser Ser Ser Val
                325                 330                 335

Tyr Val Gly Lys Pro Lys Gly Asn Asn Trp Phe Glu Lys Gln Thr Asp
            340                 345                 350

Gly Thr Leu Asp Ile Ser Cys Pro Pro Trp Met Asp Trp Phe His Gly
        355                 360                 365

Gly Leu Gln Phe Gln Ile Glu His His Leu Phe Pro Lys Met Pro Arg
    370                 375                 380

Cys Asn Leu Arg Lys Ile Ser Pro Tyr Val Ile Glu Leu Cys Lys Lys
385                 390                 395                 400

His Asn Leu Pro Tyr Asn Tyr Ala Ser Phe Ser Lys Ala Asn Glu Met
                405                 410                 415

Thr Leu Arg Thr Leu Arg Asn Thr Ala Leu Gln Ala Arg Asp Ile Thr
            420                 425                 430

Lys Pro Leu Pro Lys Asn Leu Val Trp Glu Ala Leu His Thr His Gly
        435                 440                 445
```

<210> 19
<211> 1563
<212> DNA
<213> Ceratodon purpureus

<220>
<221> CDS
<222> (1)..(1563)
<223> Delta-6-Desaturase

<400> 19

```
atg gtg tcc cag ggc ggc ggt ctc tcg cag ggt tcc att gaa gaa aac      48
Met Val Ser Gln Gly Gly Gly Leu Ser Gln Gly Ser Ile Glu Glu Asn
1               5                   10                  15

att gac gtt gag cac ttg gca acg atg ccc ctc gtc agt gac ttc cta      96
Ile Asp Val Glu His Leu Ala Thr Met Pro Leu Val Ser Asp Phe Leu
            20                  25                  30

aat gtc ctg gga acg act ttg ggc cag tgg agt ctt tcc act aca ttc     144
Asn Val Leu Gly Thr Thr Leu Gly Gln Trp Ser Leu Ser Thr Thr Phe
        35                  40                  45

gct ttc aag agg ctc acg act aag aaa cac agt tcg gac atc tcg gtg     192
Ala Phe Lys Arg Leu Thr Thr Lys Lys His Ser Ser Asp Ile Ser Val
    50                  55                  60

gag gca caa aaa gaa tcg gtt gcg cgg ggg cca gtt gag aat att tct     240
Glu Ala Gln Lys Glu Ser Val Ala Arg Gly Pro Val Glu Asn Ile Ser
65                  70                  75                  80

caa tcg gtt gcg cag ccc atc agg cgg agg tgg gtg cag gat aaa aag     288
Gln Ser Val Ala Gln Pro Ile Arg Arg Arg Trp Val Gln Asp Lys Lys
                85                  90                  95

ccg gtt act tac agc ctg aag gat gta gct tcg cac gat atg ccc cag     336
Pro Val Thr Tyr Ser Leu Lys Asp Val Ala Ser His Asp Met Pro Gln
            100                 105                 110

gac tgc tgg att ata atc aaa gag aag gtg tat gat gtg agc acc ttc     384
Asp Cys Trp Ile Ile Ile Lys Glu Lys Val Tyr Asp Val Ser Thr Phe
        115                 120                 125

gct gag cag cac cct gga ggc acg gtt atc aac acc tac ttc gga cga     432
Ala Glu Gln His Pro Gly Gly Thr Val Ile Asn Thr Tyr Phe Gly Arg
    130                 135                 140

gac gcc aca gat gtt ttc tct act ttc cac gca tcc acc tca tgg aag     480
Asp Ala Thr Asp Val Phe Ser Thr Phe His Ala Ser Thr Ser Trp Lys
145                 150                 155                 160

att ctt cag aat ttc tac atc ggg aac ctt gtt agg gag gag ccg act     528
Ile Leu Gln Asn Phe Tyr Ile Gly Asn Leu Val Arg Glu Glu Pro Thr
                165                 170                 175

ttg gag ctg ctg aag gag tac aga gag ttg aga gcc ctt ttc ttg aga     576
Leu Glu Leu Leu Lys Glu Tyr Arg Glu Leu Arg Ala Leu Phe Leu Arg
            180                 185                 190

gaa cag ctt ttc aag agt tcc aaa tcc tac tac ctt ttc aag act ctc     624
Glu Gln Leu Phe Lys Ser Ser Lys Ser Tyr Tyr Leu Phe Lys Thr Leu
        195                 200                 205

ata aat gtt tcc att gtt gcc aca agc att gcg ata atc agt ctg tac     672
Ile Asn Val Ser Ile Val Ala Thr Ser Ile Ala Ile Ile Ser Leu Tyr
    210                 215                 220
```

```
aag tct tac cgg gcg gtt ctg tta tca gcc agt ttg atg ggc ttg ttt      720
Lys Ser Tyr Arg Ala Val Leu Leu Ser Ala Ser Leu Met Gly Leu Phe
225                 230                 235                 240

att caa cag tgc gga tgg ttg tct cac gat ttt cta cac cat cag gta      768
Ile Gln Gln Cys Gly Trp Leu Ser His Asp Phe Leu His His Gln Val
                245                 250                 255

ttt gag aca cgc tgg ctc aat gac gtt gtt ggc tat gtg gtc ggc aac      816
Phe Glu Thr Arg Trp Leu Asn Asp Val Val Gly Tyr Val Val Gly Asn
            260                 265                 270

gtt gtt ctg gga ttc agt gtc tcg tgg tgg aag acc aag cac aac ctg      864
Val Val Leu Gly Phe Ser Val Ser Trp Trp Lys Thr Lys His Asn Leu
        275                 280                 285

cat cat gct gct ccg aat gaa tgc gac caa aag tac aca ccg att gat      912
His His Ala Ala Pro Asn Glu Cys Asp Gln Lys Tyr Thr Pro Ile Asp
    290                 295                 300

gag gat att gat act ctc ccc atc att gct tgg agt aaa gat ctc ttg      960
Glu Asp Ile Asp Thr Leu Pro Ile Ile Ala Trp Ser Lys Asp Leu Leu
305                 310                 315                 320

gcc act gtt gag agc aag acc atg ttg cga gtt ctt cag tac cag cac     1008
Ala Thr Val Glu Ser Lys Thr Met Leu Arg Val Leu Gln Tyr Gln His
                325                 330                 335

cta ttc ttt ttg gtt ctt ttg acg ttt gcc cgg gcg agt tgg cta ttt     1056
Leu Phe Phe Leu Val Leu Leu Thr Phe Ala Arg Ala Ser Trp Leu Phe
            340                 345                 350

tgg agc gcg gcc ttc act ctc agg ccc gag ttg acc ctt ggc gag aag     1104
Trp Ser Ala Ala Phe Thr Leu Arg Pro Glu Leu Thr Leu Gly Glu Lys
        355                 360                 365

ctt ttg gag agg gga acg atg gct ttg cac tac att tgg ttt aat agt     1152
Leu Leu Glu Arg Gly Thr Met Ala Leu His Tyr Ile Trp Phe Asn Ser
    370                 375                 380

gtt gcg ttt tat ctg ctc ccc gga tgg aaa cca gtt gta tgg atg gtg     1200
Val Ala Phe Tyr Leu Leu Pro Gly Trp Lys Pro Val Val Trp Met Val
385                 390                 395                 400

gtc agc gag ctc atg tct ggt ttc ctg ctg gga tac gta ttt gta ctc     1248
Val Ser Glu Leu Met Ser Gly Phe Leu Leu Gly Tyr Val Phe Val Leu
                405                 410                 415

agt cac aat gga atg gag gtg tac aat acg tca aag gac ttc gtg aat     1296
Ser His Asn Gly Met Glu Val Tyr Asn Thr Ser Lys Asp Phe Val Asn
            420                 425                 430

gcc cag att gca tcg act cgc gac atc aaa gca ggg gtg ttt aat gat     1344
Ala Gln Ile Ala Ser Thr Arg Asp Ile Lys Ala Gly Val Phe Asn Asp
        435                 440                 445

tgg ttc acc gga ggt ctc aac aga cag att gag cat cat cta ttt cca     1392
Trp Phe Thr Gly Gly Leu Asn Arg Gln Ile Glu His His Leu Phe Pro
    450                 455                 460

acg atg ccc agg cac aac ctt aat aaa att tct cct cac gtg gag act     1440
Thr Met Pro Arg His Asn Leu Asn Lys Ile Ser Pro His Val Glu Thr
465                 470                 475                 480

ttg tgc aag aag cat gga ctg gtc tac gaa gac gtg agc atg gct tcg     1488
Leu Cys Lys Lys His Gly Leu Val Tyr Glu Asp Val Ser Met Ala Ser
                485                 490                 495
```

```
ggc act tac cgg gtt ttg aaa aca ctt aag gac gtt gcc gat gct gct      1536
Gly Thr Tyr Arg Val Leu Lys Thr Leu Lys Asp Val Ala Asp Ala Ala
            500                 505                 510

tca cac cag cag ctt gct gcg agt tga                                  1563
Ser His Gln Gln Leu Ala Ala Ser
        515                 520
```

<210> 20
<211> 520
<212> PRT
<213> Ceratodon purpureus

<400> 20

```
Met Val Ser Gln Gly Gly Gly Leu Ser Gln Gly Ser Ile Glu Glu Asn
1               5                   10                  15

Ile Asp Val Glu His Leu Ala Thr Met Pro Leu Val Ser Asp Phe Leu
            20                  25                  30

Asn Val Leu Gly Thr Thr Leu Gly Gln Trp Ser Leu Ser Thr Thr Phe
        35                  40                  45

Ala Phe Lys Arg Leu Thr Thr Lys Lys His Ser Ser Asp Ile Ser Val
    50                  55                  60

Glu Ala Gln Lys Glu Ser Val Ala Arg Gly Pro Val Glu Asn Ile Ser
65                  70                  75                  80

Gln Ser Val Ala Gln Pro Ile Arg Arg Arg Trp Val Gln Asp Lys Lys
                85                  90                  95

Pro Val Thr Tyr Ser Leu Lys Asp Val Ala Ser His Asp Met Pro Gln
            100                 105                 110

Asp Cys Trp Ile Ile Ile Lys Glu Lys Val Tyr Asp Val Ser Thr Phe
        115                 120                 125

Ala Glu Gln His Pro Gly Gly Thr Val Ile Asn Thr Tyr Phe Gly Arg
    130                 135                 140

Asp Ala Thr Asp Val Phe Ser Thr Phe His Ala Ser Thr Ser Trp Lys
145                 150                 155                 160

Ile Leu Gln Asn Phe Tyr Ile Gly Asn Leu Val Arg Glu Glu Pro Thr
                165                 170                 175

Leu Glu Leu Leu Lys Glu Tyr Arg Glu Leu Arg Ala Leu Phe Leu Arg
            180                 185                 190
```

```
Glu Gln Leu Phe Lys Ser Ser Lys Ser Tyr Tyr Leu Phe Lys Thr Leu
        195                 200                 205

Ile Asn Val Ser Ile Val Ala Thr Ser Ile Ala Ile Ile Ser Leu Tyr
    210                 215                 220

Lys Ser Tyr Arg Ala Val Leu Leu Ser Ala Ser Leu Met Gly Leu Phe
225                 230                 235                 240

Ile Gln Gln Cys Gly Trp Leu Ser His Asp Phe Leu His His Gln Val
                245                 250                 255

Phe Glu Thr Arg Trp Leu Asn Asp Val Val Gly Tyr Val Val Gly Asn
            260                 265                 270

Val Val Leu Gly Phe Ser Val Ser Trp Trp Lys Thr Lys His Asn Leu
        275                 280                 285

His His Ala Ala Pro Asn Glu Cys Asp Gln Lys Tyr Thr Pro Ile Asp
    290                 295                 300

Glu Asp Ile Asp Thr Leu Pro Ile Ile Ala Trp Ser Lys Asp Leu Leu
305                 310                 315                 320

Ala Thr Val Glu Ser Lys Thr Met Leu Arg Val Leu Gln Tyr Gln His
                325                 330                 335

Leu Phe Phe Leu Val Leu Leu Thr Phe Ala Arg Ala Ser Trp Leu Phe
            340                 345                 350

Trp Ser Ala Ala Phe Thr Leu Arg Pro Glu Leu Thr Leu Gly Glu Lys
        355                 360                 365

Leu Leu Glu Arg Gly Thr Met Ala Leu His Tyr Ile Trp Phe Asn Ser
    370                 375                 380

Val Ala Phe Tyr Leu Leu Pro Gly Trp Lys Pro Val Val Trp Met Val
385                 390                 395                 400

Val Ser Glu Leu Met Ser Gly Phe Leu Leu Gly Tyr Val Phe Val Leu
                405                 410                 415

Ser His Asn Gly Met Glu Val Tyr Asn Thr Ser Lys Asp Phe Val Asn
            420                 425                 430

Ala Gln Ile Ala Ser Thr Arg Asp Ile Lys Ala Gly Val Phe Asn Asp
        435                 440                 445

Trp Phe Thr Gly Gly Leu Asn Arg Gln Ile Glu His His Leu Phe Pro
    450                 455                 460
```

Thr Met Pro Arg His Asn Leu Asn Lys Ile Ser Pro His Val Glu Thr
465 470 475 480

Leu Cys Lys Lys His Gly Leu Val Tyr Glu Asp Val Ser Met Ala Ser
485 490 495

Gly Thr Tyr Arg Val Leu Lys Thr Leu Lys Asp Val Ala Asp Ala Ala
500 505 510

Ser His Gln Gln Leu Ala Ala Ser
515 520

<210> 21
<211> 1434
<212> DNA
<213> Phaeodactylum tricornutum

<220>
<221> CDS
<222> (1)..(1434)
<223> Delta-6-Desaturase

<400> 21

atg ggc aaa gga ggg gac gct cgg gcc tcg aag ggc tca acg gcg gct 48
Met Gly Lys Gly Gly Asp Ala Arg Ala Ser Lys Gly Ser Thr Ala Ala
1 5 10 15

cgc aag atc agt tgg cag gaa gtc aag acc cac gcg tct ccg gag gac 96
Arg Lys Ile Ser Trp Gln Glu Val Lys Thr His Ala Ser Pro Glu Asp
20 25 30

gcc tgg atc att cac tcc aat aag gtc tac gac gtg tcc aac tgg cac 144
Ala Trp Ile Ile His Ser Asn Lys Val Tyr Asp Val Ser Asn Trp His
35 40 45

gaa cat ccc gga ggc gcc gtc att ttc acg cac gcc ggt gac gac atg 192
Glu His Pro Gly Gly Ala Val Ile Phe Thr His Ala Gly Asp Asp Met
50 55 60

acg gac att ttc gct gcc ttt cac gca ccc gga tcg cag tcg ctc atg 240
Thr Asp Ile Phe Ala Ala Phe His Ala Pro Gly Ser Gln Ser Leu Met
65 70 75 80

aag aag ttc tac att ggc gaa ttg ctc ccg gaa acc acc ggc aag gag 288
Lys Lys Phe Tyr Ile Gly Glu Leu Leu Pro Glu Thr Thr Gly Lys Glu
85 90 95

ccg cag caa atc gcc ttt gaa aag ggc tac cgc gat ctg cgc tcc aaa 336
Pro Gln Gln Ile Ala Phe Glu Lys Gly Tyr Arg Asp Leu Arg Ser Lys
100 105 110

ctc atc atg atg ggc atg ttc aag tcc aac aag tgg ttc tac gtc tac 384

```
Leu Ile Met Met Gly Met Phe Lys Ser Asn Lys Trp Phe Tyr Val Tyr
        115                 120                 125

aag tgc ctc agc aac atg gcc att tgg gcc gcc gcc tgt gct ctc gtc      432
Lys Cys Leu Ser Asn Met Ala Ile Trp Ala Ala Ala Cys Ala Leu Val
    130                 135                 140

ttt tac tcg gac cgc ttc tgg gta cac ctg gcc agc gcc gtc atg ctg      480
Phe Tyr Ser Asp Arg Phe Trp Val His Leu Ala Ser Ala Val Met Leu
145                 150                 155                 160

gga aca ttc ttt cag cag tcg gga tgg ttg gca cac gac ttt ctg cac      528
Gly Thr Phe Phe Gln Gln Ser Gly Trp Leu Ala His Asp Phe Leu His
                165                 170                 175

cac cag gtc ttc acc aag cgc aag cac ggg gat ctc gga gga ctc ttt      576
His Gln Val Phe Thr Lys Arg Lys His Gly Asp Leu Gly Gly Leu Phe
            180                 185                 190

tgg ggg aac ctc atg cag ggt tac tcc gta cag tgg tgg aaa aac aag      624
Trp Gly Asn Leu Met Gln Gly Tyr Ser Val Gln Trp Trp Lys Asn Lys
        195                 200                 205

cac aac gga cac cac gcc gtc ccc aac ctc cac tgc tcc tcc gca gtc      672
His Asn Gly His His Ala Val Pro Asn Leu His Cys Ser Ser Ala Val
    210                 215                 220

gcg caa gat ggg gac ccg gac atc gat acc atg ccc ctt ctc gcc tgg      720
Ala Gln Asp Gly Asp Pro Asp Ile Asp Thr Met Pro Leu Leu Ala Trp
225                 230                 235                 240

tcc gtc cag caa gcc cag tct tac cgg gaa ctc caa gcc gac gga aag      768
Ser Val Gln Gln Ala Gln Ser Tyr Arg Glu Leu Gln Ala Asp Gly Lys
                245                 250                 255

gat tcg ggt ttg gtc aag ttc atg atc cgt aac caa tcc tac ttt tac      816
Asp Ser Gly Leu Val Lys Phe Met Ile Arg Asn Gln Ser Tyr Phe Tyr
            260                 265                 270

ttt ccc atc ttg ttg ctc gcc cgc ctg tcg tgg ttg aac gag tcc ttc      864
Phe Pro Ile Leu Leu Leu Ala Arg Leu Ser Trp Leu Asn Glu Ser Phe
        275                 280                 285

aag tgc gcc ttt ggg ctt gga gct gcg tcg gag aac gct gct ctc gaa      912
Lys Cys Ala Phe Gly Leu Gly Ala Ala Ser Glu Asn Ala Ala Leu Glu
    290                 295                 300

ctc aag gcc aag ggt ctt cag tac ccc ctt ttg gaa aag gct ggc atc      960
Leu Lys Ala Lys Gly Leu Gln Tyr Pro Leu Leu Glu Lys Ala Gly Ile
305                 310                 315                 320

ctg ctg cac tac gct tgg atg ctt aca gtt tcg tcc ggc ttt gga cgc     1008
Leu Leu His Tyr Ala Trp Met Leu Thr Val Ser Ser Gly Phe Gly Arg
                325                 330                 335

ttc tcg ttc gcg tac acc gca ttt tac ttt cta acc gcg acc gcg tcc     1056
Phe Ser Phe Ala Tyr Thr Ala Phe Tyr Phe Leu Thr Ala Thr Ala Ser
            340                 345                 350

tgt gga ttc ttg ctc gcc att gtc ttt ggc ctc ggc cac aac ggc atg     1104
Cys Gly Phe Leu Leu Ala Ile Val Phe Gly Leu Gly His Asn Gly Met
        355                 360                 365

gcc acc tac aat gcc gac gcc cgt ccg gac ttc tgg aag ctc caa gtc     1152
Ala Thr Tyr Asn Ala Asp Ala Arg Pro Asp Phe Trp Lys Leu Gln Val
    370                 375                 380

acc acg act cgc aac gtc acg ggc gga cac ggt ttc ccc caa gcc ttt     1200
```

```
Thr Thr Thr Arg Asn Val Thr Gly Gly His Gly Phe Pro Gln Ala Phe
385                 390                 395                 400

gtc gac tgg ttc tgt ggt ggc ctc cag tac caa gtc gac cac cac tta      1248
Val Asp Trp Phe Cys Gly Gly Leu Gln Tyr Gln Val Asp His His Leu
            405                 410                 415

ttc ccc agc ctg ccc cga cac aat ctg gcc aag aca cac gca ctg gtc      1296
Phe Pro Ser Leu Pro Arg His Asn Leu Ala Lys Thr His Ala Leu Val
        420                 425                 430

gaa tcg ttc tgc aag gag tgg ggt gtc cag tac cac gaa gcc gac ctt      1344
Glu Ser Phe Cys Lys Glu Trp Gly Val Gln Tyr His Glu Ala Asp Leu
        435                 440                 445

gtg gac ggg acc atg gaa gtc ttg cac cat ttg ggc agc gtg gcc ggc      1392
Val Asp Gly Thr Met Glu Val Leu His His Leu Gly Ser Val Ala Gly
    450                 455                 460

gaa ttc gtc gtg gat ttt gta cgc gat gga ccc gcc atg taa              1434
Glu Phe Val Val Asp Phe Val Arg Asp Gly Pro Ala Met
465                 470                 475
```

<210> 22
<211> 477
<212> PRT
<213> Phaeodactylum tricornutum

<400> 22

```
Met Gly Lys Gly Gly Asp Ala Arg Ala Ser Lys Gly Ser Thr Ala Ala
1               5                   10                  15

Arg Lys Ile Ser Trp Gln Glu Val Lys Thr His Ala Ser Pro Glu Asp
            20                  25                  30

Ala Trp Ile Ile His Ser Asn Lys Val Tyr Asp Val Ser Asn Trp His
        35                  40                  45

Glu His Pro Gly Gly Ala Val Ile Phe Thr His Ala Gly Asp Asp Met
    50                  55                  60

Thr Asp Ile Phe Ala Ala Phe His Ala Pro Gly Ser Gln Ser Leu Met
65                  70                  75                  80

Lys Lys Phe Tyr Ile Gly Glu Leu Leu Pro Glu Thr Thr Gly Lys Glu
                85                  90                  95

Pro Gln Gln Ile Ala Phe Glu Lys Gly Tyr Arg Asp Leu Arg Ser Lys
            100                 105                 110

Leu Ile Met Met Gly Met Phe Lys Ser Asn Lys Trp Phe Tyr Val Tyr
        115                 120                 125
```

Lys Cys Leu Ser Asn Met Ala Ile Trp Ala Ala Ala Cys Ala Leu Val
    130                 135                 140

Phe Tyr Ser Asp Arg Phe Trp Val His Leu Ala Ser Ala Val Met Leu
145                 150                 155                 160

Gly Thr Phe Phe Gln Gln Ser Gly Trp Leu Ala His Asp Phe Leu His
                165                 170                 175

His Gln Val Phe Thr Lys Arg Lys His Gly Asp Leu Gly Gly Leu Phe
            180                 185                 190

Trp Gly Asn Leu Met Gln Gly Tyr Ser Val Gln Trp Trp Lys Asn Lys
        195                 200                 205

His Asn Gly His His Ala Val Pro Asn Leu His Cys Ser Ser Ala Val
    210                 215                 220

Ala Gln Asp Gly Asp Pro Asp Ile Asp Thr Met Pro Leu Leu Ala Trp
225                 230                 235                 240

Ser Val Gln Gln Ala Gln Ser Tyr Arg Glu Leu Gln Ala Asp Gly Lys
                245                 250                 255

Asp Ser Gly Leu Val Lys Phe Met Ile Arg Asn Gln Ser Tyr Phe Tyr
            260                 265                 270

Phe Pro Ile Leu Leu Leu Ala Arg Leu Ser Trp Leu Asn Glu Ser Phe
        275                 280                 285

Lys Cys Ala Phe Gly Leu Gly Ala Ala Ser Glu Asn Ala Ala Leu Glu
    290                 295                 300

Leu Lys Ala Lys Gly Leu Gln Tyr Pro Leu Leu Glu Lys Ala Gly Ile
305                 310                 315                 320

Leu Leu His Tyr Ala Trp Met Leu Thr Val Ser Ser Gly Phe Gly Arg
                325                 330                 335

Phe Ser Phe Ala Tyr Thr Ala Phe Tyr Phe Leu Thr Ala Thr Ala Ser
            340                 345                 350

Cys Gly Phe Leu Leu Ala Ile Val Phe Gly Leu Gly His Asn Gly Met
        355                 360                 365

Ala Thr Tyr Asn Ala Asp Ala Arg Pro Asp Phe Trp Lys Leu Gln Val
    370                 375                 380

Thr Thr Thr Arg Asn Val Thr Gly Gly His Gly Phe Pro Gln Ala Phe
385                 390                 395                 400

```
Val Asp Trp Phe Cys Gly Gly Leu Gln Tyr Gln Val Asp His His Leu
                405                 410                 415

Phe Pro Ser Leu Pro Arg His Asn Leu Ala Lys Thr His Ala Leu Val
            420                 425                 430

Glu Ser Phe Cys Lys Glu Trp Gly Val Gln Tyr His Glu Ala Asp Leu
        435                 440                 445

Val Asp Gly Thr Met Glu Val Leu His His Leu Gly Ser Val Ala Gly
    450                 455                 460

Glu Phe Val Val Asp Phe Val Arg Asp Gly Pro Ala Met
465                 470                 475
```

<210> 23
<211> 1578
<212> DNA
<213> Physcomitrella patens

<220>
<221> CDS
<222> (1)..(1578)
<223> Delta-6-Desaturase

<400> 23

```
atg gta ttc gcg ggc ggt gga ctt cag cag ggc tct ctc gaa gaa aac      48
Met Val Phe Ala Gly Gly Gly Leu Gln Gln Gly Ser Leu Glu Glu Asn
1               5                   10                  15

atc gac gtc gag cac att gcc agt atg tct ctc ttc agc gac ttc ttc      96
Ile Asp Val Glu His Ile Ala Ser Met Ser Leu Phe Ser Asp Phe Phe
            20                  25                  30

agt tat gtg tct tca act gtt ggt tcg tgg agc gta cac agt ata caa     144
Ser Tyr Val Ser Ser Thr Val Gly Ser Trp Ser Val His Ser Ile Gln
        35                  40                  45

cct ttg aag cgc ctg acg agt aag aag cgt gtt tcg gaa agc gct gcc     192
Pro Leu Lys Arg Leu Thr Ser Lys Lys Arg Val Ser Glu Ser Ala Ala
    50                  55                  60

gtg caa tgt ata tca gct gaa gtt cag aga aat tcg agt acc cag gga     240
Val Gln Cys Ile Ser Ala Glu Val Gln Arg Asn Ser Ser Thr Gln Gly
65                  70                  75                  80

act gcg gag gca ctc gca gaa tca gtc gtg aag ccc acg aga cga agg     288
Thr Ala Glu Ala Leu Ala Glu Ser Val Val Lys Pro Thr Arg Arg Arg
                85                  90                  95

tca tct cag tgg aag aag tcg aca cac ccc cta tca gaa gta gca gta     336
Ser Ser Gln Trp Lys Lys Ser Thr His Pro Leu Ser Glu Val Ala Val
            100                 105                 110
```

```
cac aac aag cca agc gat tgc tgg att gtt gta aaa aac aag gtg tat      384
His Asn Lys Pro Ser Asp Cys Trp Ile Val Val Lys Asn Lys Val Tyr
        115                 120                 125

gat gtt tcc aat ttt gcg gac gag cat ccc gga gga tca gtt att agt      432
Asp Val Ser Asn Phe Ala Asp Glu His Pro Gly Gly Ser Val Ile Ser
    130                 135                 140

act tat ttt gga cga gac ggc aca gat gtt ttc tct agt ttt cat gca      480
Thr Tyr Phe Gly Arg Asp Gly Thr Asp Val Phe Ser Ser Phe His Ala
145                 150                 155                 160

gct tct aca tgg aaa att ctt caa gac ttt tac att ggt gac gtg gag      528
Ala Ser Thr Trp Lys Ile Leu Gln Asp Phe Tyr Ile Gly Asp Val Glu
                165                 170                 175

agg gtg gag ccg act cca gag ctg ctg aaa gat ttc cga gaa atg aga      576
Arg Val Glu Pro Thr Pro Glu Leu Leu Lys Asp Phe Arg Glu Met Arg
            180                 185                 190

gct ctt ttc ctg agg gag caa ctt ttc aaa agt tcg aaa ttg tac tat      624
Ala Leu Phe Leu Arg Glu Gln Leu Phe Lys Ser Ser Lys Leu Tyr Tyr
        195                 200                 205

gtt atg aag ctg ctc acg aat gtt gct att ttt gct gcg agc att gca      672
Val Met Lys Leu Leu Thr Asn Val Ala Ile Phe Ala Ala Ser Ile Ala
    210                 215                 220

ata ata tgt tgg agc aag act att tca gcg gtt ttg gct tca gct tgt      720
Ile Ile Cys Trp Ser Lys Thr Ile Ser Ala Val Leu Ala Ser Ala Cys
225                 230                 235                 240

atg atg gct ctg tgt ttc caa cag tgc gga tgg cta tcc cat gat ttt      768
Met Met Ala Leu Cys Phe Gln Gln Cys Gly Trp Leu Ser His Asp Phe
                245                 250                 255

ctc cac aat cag gtg ttt gag aca cgc tgg ctt aat gaa gtt gtc ggg      816
Leu His Asn Gln Val Phe Glu Thr Arg Trp Leu Asn Glu Val Val Gly
            260                 265                 270

tat gtg atc ggc aac gcc gtt ctg ggg ttt agt aca ggg tgg tgg aag      864
Tyr Val Ile Gly Asn Ala Val Leu Gly Phe Ser Thr Gly Trp Trp Lys
        275                 280                 285

gag aag cat aac ctt cat cat gct gct cca aat gaa tgc gat cag act      912
Glu Lys His Asn Leu His His Ala Ala Pro Asn Glu Cys Asp Gln Thr
    290                 295                 300

tac caa cca att gat gaa gat att gat act ctc ccc ctc att gcc tgg      960
Tyr Gln Pro Ile Asp Glu Asp Ile Asp Thr Leu Pro Leu Ile Ala Trp
305                 310                 315                 320

agc aag gac ata ctg gcc aca gtt gag aat aag aca ttc ttg cga atc     1008
Ser Lys Asp Ile Leu Ala Thr Val Glu Asn Lys Thr Phe Leu Arg Ile
                325                 330                 335

ctc caa tac cag cat ctg ttc ttc atg ggt ctg tta ttt ttc gcc cgt     1056
Leu Gln Tyr Gln His Leu Phe Phe Met Gly Leu Leu Phe Phe Ala Arg
            340                 345                 350

ggt agt tgg ctc ttt tgg agc tgg aga tat acc tct aca gca gtg ctc     1104
Gly Ser Trp Leu Phe Trp Ser Trp Arg Tyr Thr Ser Thr Ala Val Leu
        355                 360                 365

tca cct gtc gac agg ttg ttg gag aag gga act gtt ctg ttt cac tac     1152
Ser Pro Val Asp Arg Leu Leu Glu Lys Gly Thr Val Leu Phe His Tyr
    370                 375                 380
```

```
ttt tgg ttc gtc ggg aca gcg tgc tat ctt ctc cct ggt tgg aag cca      1200
Phe Trp Phe Val Gly Thr Ala Cys Tyr Leu Leu Pro Gly Trp Lys Pro
385                 390                 395                 400

tta gta tgg atg gcg gtg act gag ctc atg tcc ggc atg ctg ctg ggc      1248
Leu Val Trp Met Ala Val Thr Glu Leu Met Ser Gly Met Leu Leu Gly
                405                 410                 415

ttt gta ttt gta ctt agc cac aat ggg atg gag gtt tat aat tcg tct      1296
Phe Val Phe Val Leu Ser His Asn Gly Met Glu Val Tyr Asn Ser Ser
            420                 425                 430

aaa gaa ttc gtg agt gca cag atc gta tcc aca cgg gat atc aaa gga      1344
Lys Glu Phe Val Ser Ala Gln Ile Val Ser Thr Arg Asp Ile Lys Gly
        435                 440                 445

aac ata ttc aac gac tgg ttc act ggt ggc ctt aac agg caa ata gag      1392
Asn Ile Phe Asn Asp Trp Phe Thr Gly Gly Leu Asn Arg Gln Ile Glu
    450                 455                 460

cat cat ctt ttc cca aca atg ccc agg cat aat tta aac aaa ata gca      1440
His His Leu Phe Pro Thr Met Pro Arg His Asn Leu Asn Lys Ile Ala
465                 470                 475                 480

cct aga gtg gag gtg ttc tgt aag aaa cac ggt ctg gtg tac gaa gac      1488
Pro Arg Val Glu Val Phe Cys Lys Lys His Gly Leu Val Tyr Glu Asp
                485                 490                 495

gta tct att gct acc ggc act tgc aag gtt ttg aaa gca ttg aag gaa      1536
Val Ser Ile Ala Thr Gly Thr Cys Lys Val Leu Lys Ala Leu Lys Glu
            500                 505                 510

gtc gcg gag gct gcg gca gag cag cat gct acc acc agt taa              1578
Val Ala Glu Ala Ala Ala Glu Gln His Ala Thr Thr Ser
        515                 520                 525
```

<210> 24
<211> 525
<212> PRT
<213> Physcomitrella patens

<400> 24

```
Met Val Phe Ala Gly Gly Gly Leu Gln Gln Gly Ser Leu Glu Glu Asn
1               5                   10                  15

Ile Asp Val Glu His Ile Ala Ser Met Ser Leu Phe Ser Asp Phe Phe
            20                  25                  30

Ser Tyr Val Ser Ser Thr Val Gly Ser Trp Ser Val His Ser Ile Gln
        35                  40                  45

Pro Leu Lys Arg Leu Thr Ser Lys Lys Arg Val Ser Glu Ser Ala Ala
    50                  55                  60

Val Gln Cys Ile Ser Ala Glu Val Gln Arg Asn Ser Ser Thr Gln Gly
65                  70                  75                  80
```

Thr Ala Glu Ala Leu Ala Glu Ser Val Val Lys Pro Thr Arg Arg Arg
85 90 95

Ser Ser Gln Trp Lys Lys Ser Thr His Pro Leu Ser Glu Val Ala Val
100 105 110

His Asn Lys Pro Ser Asp Cys Trp Ile Val Val Lys Asn Lys Val Tyr
115 120 125

Asp Val Ser Asn Phe Ala Asp Glu His Pro Gly Gly Ser Val Ile Ser
130 135 140

Thr Tyr Phe Gly Arg Asp Gly Thr Asp Val Phe Ser Ser Phe His Ala
145 150 155 160

Ala Ser Thr Trp Lys Ile Leu Gln Asp Phe Tyr Ile Gly Asp Val Glu
165 170 175

Arg Val Glu Pro Thr Pro Glu Leu Leu Lys Asp Phe Arg Glu Met Arg
180 185 190

Ala Leu Phe Leu Arg Glu Gln Leu Phe Lys Ser Ser Lys Leu Tyr Tyr
195 200 205

Val Met Lys Leu Leu Thr Asn Val Ala Ile Phe Ala Ala Ser Ile Ala
210 215 220

Ile Ile Cys Trp Ser Lys Thr Ile Ser Ala Val Leu Ala Ser Ala Cys
225 230 235 240

Met Met Ala Leu Cys Phe Gln Gln Cys Gly Trp Leu Ser His Asp Phe
245 250 255

Leu His Asn Gln Val Phe Glu Thr Arg Trp Leu Asn Glu Val Val Gly
260 265 270

Tyr Val Ile Gly Asn Ala Val Leu Gly Phe Ser Thr Gly Trp Trp Lys
275 280 285

Glu Lys His Asn Leu His His Ala Ala Pro Asn Glu Cys Asp Gln Thr
290 295 300

Tyr Gln Pro Ile Asp Glu Asp Ile Asp Thr Leu Pro Leu Ile Ala Trp
305 310 315 320

Ser Lys Asp Ile Leu Ala Thr Val Glu Asn Lys Thr Phe Leu Arg Ile
325 330 335

Leu Gln Tyr Gln His Leu Phe Phe Met Gly Leu Leu Phe Phe Ala Arg
340 345 350

Gly Ser Trp Leu Phe Trp Ser Trp Arg Tyr Thr Ser Thr Ala Val Leu
355 360 365

Ser Pro Val Asp Arg Leu Leu Glu Lys Gly Thr Val Leu Phe His Tyr
370 375 380

Phe Trp Phe Val Gly Thr Ala Cys Tyr Leu Leu Pro Gly Trp Lys Pro
385 390 395 400

Leu Val Trp Met Ala Val Thr Glu Leu Met Ser Gly Met Leu Leu Gly
405 410 415

Phe Val Phe Val Leu Ser His Asn Gly Met Glu Val Tyr Asn Ser Ser
420 425 430

Lys Glu Phe Val Ser Ala Gln Ile Val Ser Thr Arg Asp Ile Lys Gly
435 440 445

Asn Ile Phe Asn Asp Trp Phe Thr Gly Gly Leu Asn Arg Gln Ile Glu
450 455 460

His His Leu Phe Pro Thr Met Pro Arg His Asn Leu Asn Lys Ile Ala
465 470 475 480

Pro Arg Val Glu Val Phe Cys Lys Lys His Gly Leu Val Tyr Glu Asp
485 490 495

Val Ser Ile Ala Thr Gly Thr Cys Lys Val Leu Lys Ala Leu Lys Glu
500 505 510

Val Ala Glu Ala Ala Ala Glu Gln His Ala Thr Thr Ser
515 520 525

<210> 25
<211> 1332
<212> DNA
<213> Caenorhabditis elegans

<220>
<221> CDS
<222> (1)..(1332)
<223> Delta-6-Desaturase

<400> 25

atg gtc gtc gac aag aat gcc tcc ggg ctt cga atg aag gtc gat ggc 48

```
Met Val Val Asp Lys Asn Ala Ser Gly Leu Arg Met Lys Val Asp Gly
1               5                   10                  15

aaa tgg ctc tac ctt agc gag gaa ttg gtg aag aaa cat cca gga gga        96
Lys Trp Leu Tyr Leu Ser Glu Glu Leu Val Lys Lys His Pro Gly Gly
            20                  25                  30

gct gtt att gaa caa tat aga aat tcg gat gct act cat att ttc cac       144
Ala Val Ile Glu Gln Tyr Arg Asn Ser Asp Ala Thr His Ile Phe His
        35                  40                  45

gct ttc cac gaa gga tct tct cag gct tat aag caa ctt gac ctt ctg       192
Ala Phe His Glu Gly Ser Ser Gln Ala Tyr Lys Gln Leu Asp Leu Leu
    50                  55                  60

aaa aag cac gga gag cac gat gaa ttc ctt gag aaa caa ttg gaa aag       240
Lys Lys His Gly Glu His Asp Glu Phe Leu Glu Lys Gln Leu Glu Lys
65                  70                  75                  80

aga ctt gac aaa gtt gat atc aat gta tca gca tat gat gtc agt gtt       288
Arg Leu Asp Lys Val Asp Ile Asn Val Ser Ala Tyr Asp Val Ser Val
                85                  90                  95

gca caa gaa aag aaa atg gtt gaa tca ttc gaa aaa cta cga cag aag       336
Ala Gln Glu Lys Lys Met Val Glu Ser Phe Glu Lys Leu Arg Gln Lys
            100                 105                 110

ctt cat gat gat gga tta atg aaa gca aat gaa aca tat ttc ctg ttt       384
Leu His Asp Asp Gly Leu Met Lys Ala Asn Glu Thr Tyr Phe Leu Phe
        115                 120                 125

aaa gcg att tca aca ctt tca att atg gca ttt gca ttt tat ctt cag       432
Lys Ala Ile Ser Thr Leu Ser Ile Met Ala Phe Ala Phe Tyr Leu Gln
    130                 135                 140

tat ctt gga tgg tat att act tct gca tgt tta tta gca ctt gca tgg       480
Tyr Leu Gly Trp Tyr Ile Thr Ser Ala Cys Leu Leu Ala Leu Ala Trp
145                 150                 155                 160

caa caa ttc gga tgg tta aca cat gag ttc tgc cat caa cag cca aca       528
Gln Gln Phe Gly Trp Leu Thr His Glu Phe Cys His Gln Gln Pro Thr
                165                 170                 175

aag aac aga cct ttg aat gat act att tct ttg ttc ttt ggt aat ttc       576
Lys Asn Arg Pro Leu Asn Asp Thr Ile Ser Leu Phe Phe Gly Asn Phe
            180                 185                 190

tta caa gga ttt tca aga gat tgg tgg aag gac aag cat aac act cat       624
Leu Gln Gly Phe Ser Arg Asp Trp Trp Lys Asp Lys His Asn Thr His
        195                 200                 205

cac gct gcc aca aat gta att gat cat gac ggt gat atc gac ttg gca       672
His Ala Ala Thr Asn Val Ile Asp His Asp Gly Asp Ile Asp Leu Ala
    210                 215                 220

cca ctt ttc gca ttt att cca gga gat ttg tgc aag tat aag gcc agc       720
Pro Leu Phe Ala Phe Ile Pro Gly Asp Leu Cys Lys Tyr Lys Ala Ser
225                 230                 235                 240

ttt gaa aaa gca att ctc aag att gta cca tat caa cat ctc tat ttc       768
Phe Glu Lys Ala Ile Leu Lys Ile Val Pro Tyr Gln His Leu Tyr Phe
                245                 250                 255

acc gca atg ctt cca atg ctc cgt ttc tca tgg act ggt cag tca gtt       816
Thr Ala Met Leu Pro Met Leu Arg Phe Ser Trp Thr Gly Gln Ser Val
            260                 265                 270

caa tgg gta ttc aaa gag aat caa atg gag tac aag gtc tat caa aga       864
```

```
Gln Trp Val Phe Lys Glu Asn Gln Met Glu Tyr Lys Val Tyr Gln Arg
        275                 280                 285

aat gca ttc tgg gag caa gca aca att gtt gga cat tgg gct tgg gta      912
Asn Ala Phe Trp Glu Gln Ala Thr Ile Val Gly His Trp Ala Trp Val
    290                 295                 300

ttc tat caa ttg ttc tta tta cca aca tgg cca ctt cgg gtt gct tat      960
Phe Tyr Gln Leu Phe Leu Leu Pro Thr Trp Pro Leu Arg Val Ala Tyr
305                 310                 315                 320

ttc att att tca caa atg gga gga ggc ctt ttg att gct cac gta gtc     1008
Phe Ile Ile Ser Gln Met Gly Gly Gly Leu Leu Ile Ala His Val Val
                325                 330                 335

act ttc aac cat aac tct gtt gat aag tat cca gcc aat tct cga att     1056
Thr Phe Asn His Asn Ser Val Asp Lys Tyr Pro Ala Asn Ser Arg Ile
            340                 345                 350

tta aac aac ttc gcc gct ctt caa att ttg acc aca cgc aac atg act     1104
Leu Asn Asn Phe Ala Ala Leu Gln Ile Leu Thr Thr Arg Asn Met Thr
        355                 360                 365

cca tct cca ttc att gat tgg ctt tgg ggt gga ctc aat tat cag atc     1152
Pro Ser Pro Phe Ile Asp Trp Leu Trp Gly Gly Leu Asn Tyr Gln Ile
    370                 375                 380

gag cac cac ttg ttc cca aca atg cca cgt tgc aat ctg aat gct tgc     1200
Glu His His Leu Phe Pro Thr Met Pro Arg Cys Asn Leu Asn Ala Cys
385                 390                 395                 400

gtg aaa tat gtg aaa gaa tgg tgc aaa gag aat aat ctt cct tac ctc     1248
Val Lys Tyr Val Lys Glu Trp Cys Lys Glu Asn Asn Leu Pro Tyr Leu
                405                 410                 415

gtc gat gac tac ttt gac gga tat gca atg aat ttg caa caa ttg aaa     1296
Val Asp Asp Tyr Phe Asp Gly Tyr Ala Met Asn Leu Gln Gln Leu Lys
            420                 425                 430

aat atg gct gag cac att caa gct aaa gct gcc taa                     1332
Asn Met Ala Glu His Ile Gln Ala Lys Ala Ala
        435                 440
```

<210> 26
<211> 443
<212> PRT
<213> Caenorhabditis elegans

<400> 26

```
Met Val Val Asp Lys Asn Ala Ser Gly Leu Arg Met Lys Val Asp Gly
1               5                   10                  15

Lys Trp Leu Tyr Leu Ser Glu Glu Leu Val Lys Lys His Pro Gly Gly
            20                  25                  30

Ala Val Ile Glu Gln Tyr Arg Asn Ser Asp Ala Thr His Ile Phe His
        35                  40                  45
```

Ala Phe His Glu Gly Ser Ser Gln Ala Tyr Lys Gln Leu Asp Leu Leu
50 55 60

Lys Lys His Gly Glu His Asp Glu Phe Leu Glu Lys Gln Leu Glu Lys
65 70 75 80

Arg Leu Asp Lys Val Asp Ile Asn Val Ser Ala Tyr Asp Val Ser Val
85 90 95

Ala Gln Glu Lys Lys Met Val Glu Ser Phe Glu Lys Leu Arg Gln Lys
100 105 110

Leu His Asp Asp Gly Leu Met Lys Ala Asn Glu Thr Tyr Phe Leu Phe
115 120 125

Lys Ala Ile Ser Thr Leu Ser Ile Met Ala Phe Ala Phe Tyr Leu Gln
130 135 140

Tyr Leu Gly Trp Tyr Ile Thr Ser Ala Cys Leu Leu Ala Leu Ala Trp
145 150 155 160

Gln Gln Phe Gly Trp Leu Thr His Glu Phe Cys His Gln Gln Pro Thr
165 170 175

Lys Asn Arg Pro Leu Asn Asp Thr Ile Ser Leu Phe Phe Gly Asn Phe
180 185 190

Leu Gln Gly Phe Ser Arg Asp Trp Trp Lys Asp Lys His Asn Thr His
195 200 205

His Ala Ala Thr Asn Val Ile Asp His Asp Gly Asp Ile Asp Leu Ala
210 215 220

Pro Leu Phe Ala Phe Ile Pro Gly Asp Leu Cys Lys Tyr Lys Ala Ser
225 230 235 240

Phe Glu Lys Ala Ile Leu Lys Ile Val Pro Tyr Gln His Leu Tyr Phe
245 250 255

Thr Ala Met Leu Pro Met Leu Arg Phe Ser Trp Thr Gly Gln Ser Val
260 265 270

Gln Trp Val Phe Lys Glu Asn Gln Met Glu Tyr Lys Val Tyr Gln Arg
275 280 285

Asn Ala Phe Trp Glu Gln Ala Thr Ile Val Gly His Trp Ala Trp Val
290 295 300

Phe Tyr Gln Leu Phe Leu Leu Pro Thr Trp Pro Leu Arg Val Ala Tyr
305 310 315 320

```
Phe Ile Ile Ser Gln Met Gly Gly Gly Leu Leu Ile Ala His Val Val
                325                 330                 335

Thr Phe Asn His Asn Ser Val Asp Lys Tyr Pro Ala Asn Ser Arg Ile
            340                 345                 350

Leu Asn Asn Phe Ala Ala Leu Gln Ile Leu Thr Thr Arg Asn Met Thr
        355                 360                 365

Pro Ser Pro Phe Ile Asp Trp Leu Trp Gly Gly Leu Asn Tyr Gln Ile
    370                 375                 380

Glu His His Leu Phe Pro Thr Met Pro Arg Cys Asn Leu Asn Ala Cys
385                 390                 395                 400

Val Lys Tyr Val Lys Glu Trp Cys Lys Glu Asn Asn Leu Pro Tyr Leu
                405                 410                 415

Val Asp Asp Tyr Phe Asp Gly Tyr Ala Met Asn Leu Gln Gln Leu Lys
            420                 425                 430

Asn Met Ala Glu His Ile Gln Ala Lys Ala Ala
        435                 440
```

<210> 27
<211> 873
<212> DNA
<213> Physcomitrella patens

<220>
<221> CDS
<222> (1)..(873)
<223> Delta-6-Elongase

<400> 27

```
atg gag gtc gtg gag aga ttc tac ggt gag ttg gat ggg aag gtc tcg       48
Met Glu Val Val Glu Arg Phe Tyr Gly Glu Leu Asp Gly Lys Val Ser
1               5                   10                  15

cag ggc gtg aat gca ttg ctg ggt agt ttt ggg gtg gag ttg acg gat       96
Gln Gly Val Asn Ala Leu Leu Gly Ser Phe Gly Val Glu Leu Thr Asp
            20                  25                  30

acg ccc act acc aaa ggc ttg ccc ctc gtt gac agt ccc aca ccc atc      144
Thr Pro Thr Thr Lys Gly Leu Pro Leu Val Asp Ser Pro Thr Pro Ile
        35                  40                  45

gtc ctc ggt gtt tct gta tac ttg act att gtc att gga ggg ctt ttg      192
Val Leu Gly Val Ser Val Tyr Leu Thr Ile Val Ile Gly Gly Leu Leu
    50                  55                  60
```

```
tgg ata aag gcc agg gat ctg aaa ccg cgc gcc tcg gag cca ttt ttg      240
Trp Ile Lys Ala Arg Asp Leu Lys Pro Arg Ala Ser Glu Pro Phe Leu
65                  70                  75                  80

ctc caa gct ttg gtg ctt gtg cac aac ctg ttc tgt ttt gcg ctc agt      288
Leu Gln Ala Leu Val Leu Val His Asn Leu Phe Cys Phe Ala Leu Ser
                85                  90                  95

ctg tat atg tgc gtg ggc atc gct tat cag gct att acc tgg cgg tac      336
Leu Tyr Met Cys Val Gly Ile Ala Tyr Gln Ala Ile Thr Trp Arg Tyr
            100                 105                 110

tct ctc tgg ggc aat gca tac aat cct aaa cat aaa gag atg gcg att      384
Ser Leu Trp Gly Asn Ala Tyr Asn Pro Lys His Lys Glu Met Ala Ile
        115                 120                 125

ctg gta tac ttg ttc tac atg tct aag tac gtg gaa ttc atg gat acc      432
Leu Val Tyr Leu Phe Tyr Met Ser Lys Tyr Val Glu Phe Met Asp Thr
    130                 135                 140

gtt atc atg ata ctg aag cgc agc acc agg caa ata agc ttc ctc cac      480
Val Ile Met Ile Leu Lys Arg Ser Thr Arg Gln Ile Ser Phe Leu His
145                 150                 155                 160

gtt tat cat cat tct tca att tcc ctc att tgg tgg gct att gct cat      528
Val Tyr His His Ser Ser Ile Ser Leu Ile Trp Trp Ala Ile Ala His
                165                 170                 175

cac gct cct ggc ggt gaa gca tat tgg tct gcg gct ctg aac tca gga      576
His Ala Pro Gly Gly Glu Ala Tyr Trp Ser Ala Ala Leu Asn Ser Gly
            180                 185                 190

gtg cat gtt ctc atg tat gcg tat tac ttc ttg gct gcc tgc ctt cga      624
Val His Val Leu Met Tyr Ala Tyr Tyr Phe Leu Ala Ala Cys Leu Arg
        195                 200                 205

agt agc cca aag tta aaa aat aag tac ctt ttt tgg ggc agg tac ttg      672
Ser Ser Pro Lys Leu Lys Asn Lys Tyr Leu Phe Trp Gly Arg Tyr Leu
    210                 215                 220

aca caa ttc caa atg ttc cag ttt atg ctg aac tta gtg cag gct tac      720
Thr Gln Phe Gln Met Phe Gln Phe Met Leu Asn Leu Val Gln Ala Tyr
225                 230                 235                 240

tac gac atg aaa acg aat gcg cca tat cca caa tgg ctg atc aag att      768
Tyr Asp Met Lys Thr Asn Ala Pro Tyr Pro Gln Trp Leu Ile Lys Ile
                245                 250                 255

ttg ttc tac tac atg atc tcg ttg ctg ttt ctt ttc ggc aat ttt tac      816
Leu Phe Tyr Tyr Met Ile Ser Leu Leu Phe Leu Phe Gly Asn Phe Tyr
            260                 265                 270

gta caa aaa tac atc aaa ccc tct gac gga aag caa aag gga gct aaa      864
Val Gln Lys Tyr Ile Lys Pro Ser Asp Gly Lys Gln Lys Gly Ala Lys
        275                 280                 285

act gag tga                                                          873
Thr Glu
    290
```

<210> 28
<211> 290
<212> PRT
<213> Physcomitrella patens

<400> 28

Met Glu Val Val Glu Arg Phe Tyr Gly Glu Leu Asp Gly Lys Val Ser
1 5 10 15

Gln Gly Val Asn Ala Leu Leu Gly Ser Phe Gly Val Glu Leu Thr Asp
20 25 30

Thr Pro Thr Thr Lys Gly Leu Pro Leu Val Asp Ser Pro Thr Pro Ile
35 40 45

Val Leu Gly Val Ser Val Tyr Leu Thr Ile Val Ile Gly Gly Leu Leu
50 55 60

Trp Ile Lys Ala Arg Asp Leu Lys Pro Arg Ala Ser Glu Pro Phe Leu
65 70 75 80

Leu Gln Ala Leu Val Leu Val His Asn Leu Phe Cys Phe Ala Leu Ser
85 90 95

Leu Tyr Met Cys Val Gly Ile Ala Tyr Gln Ala Ile Thr Trp Arg Tyr
100 105 110

Ser Leu Trp Gly Asn Ala Tyr Asn Pro Lys His Lys Glu Met Ala Ile
115 120 125

Leu Val Tyr Leu Phe Tyr Met Ser Lys Tyr Val Glu Phe Met Asp Thr
130 135 140

Val Ile Met Ile Leu Lys Arg Ser Thr Arg Gln Ile Ser Phe Leu His
145 150 155 160

Val Tyr His His Ser Ser Ile Ser Leu Ile Trp Trp Ala Ile Ala His
165 170 175

His Ala Pro Gly Gly Glu Ala Tyr Trp Ser Ala Ala Leu Asn Ser Gly
180 185 190

Val His Val Leu Met Tyr Ala Tyr Tyr Phe Leu Ala Ala Cys Leu Arg
195 200 205

Ser Ser Pro Lys Leu Lys Asn Lys Tyr Leu Phe Trp Gly Arg Tyr Leu
210 215 220

Thr Gln Phe Gln Met Phe Gln Phe Met Leu Asn Leu Val Gln Ala Tyr
225 230 235 240

Tyr Asp Met Lys Thr Asn Ala Pro Tyr Pro Gln Trp Leu Ile Lys Ile
245 250 255

```
Leu Phe Tyr Tyr Met Ile Ser Leu Leu Phe Leu Phe Gly Asn Phe Tyr
          260                 265                 270

Val Gln Lys Tyr Ile Lys Pro Ser Asp Gly Lys Gln Lys Gly Ala Lys
        275                 280                 285

Thr Glu
    290
```

<210> 29
<211> 1049
<212> DNA
<213> Thraustochytrium

<220>
<221> CDS
<222> (43)..(858)
<223> Delta-6-Elongase

<400> 29

```
gaattcggca cgagagcgcg cggagcggag acctcggccg cg atg atg gag ccg      54
                                               Met Met Glu Pro
                                               1

ctc gac agg tac agg gcg ctg gcg gag ctc gcc gcg agg tac gcc agc     102
Leu Asp Arg Tyr Arg Ala Leu Ala Glu Leu Ala Ala Arg Tyr Ala Ser
5                   10                  15                  20

tcg gcg gcc ttc aag tgg caa gtc acg tac gac gcc aag gac agc ttc     150
Ser Ala Ala Phe Lys Trp Gln Val Thr Tyr Asp Ala Lys Asp Ser Phe
                25                  30                  35

gtc ggg ccc ctg gga atc cgg gag ccg ctc ggg ctc ctg gtg ggc tcc     198
Val Gly Pro Leu Gly Ile Arg Glu Pro Leu Gly Leu Leu Val Gly Ser
            40                  45                  50

gtg gtc ctc tac ctg agc ctg ctg gcc gtg gtc tac gcg ctg cgg aac     246
Val Val Leu Tyr Leu Ser Leu Leu Ala Val Val Tyr Ala Leu Arg Asn
        55                  60                  65

tac ctt ggc ggc ctc atg gcg ctc cgc agc gtg cat aac ctc ggg ctc     294
Tyr Leu Gly Gly Leu Met Ala Leu Arg Ser Val His Asn Leu Gly Leu
    70                  75                  80

tgc ctc ttc tcg ggc gcc gtg tgg atc tac acg agc tac ctc atg atc     342
Cys Leu Phe Ser Gly Ala Val Trp Ile Tyr Thr Ser Tyr Leu Met Ile
85                  90                  95                  100

cag gat ggg cac ttt cgc agc ctc gag gcg gca acg tgc gag ccg ctc     390
Gln Asp Gly His Phe Arg Ser Leu Glu Ala Ala Thr Cys Glu Pro Leu
                105                 110                 115

aag cat ccg cac ttc cag ctc atc agc ttg ctc ttt gcg ctg tcc aag     438
```

```
Lys His Pro His Phe Gln Leu Ile Ser Leu Leu Phe Ala Leu Ser Lys
            120                 125                 130

atc tgg gag tgg ttc gac acg gtg ctc ctc atc gtc aag ggc aac aag      486
Ile Trp Glu Trp Phe Asp Thr Val Leu Leu Ile Val Lys Gly Asn Lys
        135                 140                 145

ctc cgc ttc ctg cac gtc ttg cac cac gcc acg acc ttt tgg ctc tac      534
Leu Arg Phe Leu His Val Leu His His Ala Thr Thr Phe Trp Leu Tyr
    150                 155                 160

gcc atc gac cac atc ttt ctc tcg tcc atc aag tac ggc gtc gcg gtc      582
Ala Ile Asp His Ile Phe Leu Ser Ser Ile Lys Tyr Gly Val Ala Val
165                 170                 175                 180

aat gct ttc atc cac acc gtc atg tac gcg cac tac ttc cgc cca ttc      630
Asn Ala Phe Ile His Thr Val Met Tyr Ala His Tyr Phe Arg Pro Phe
                185                 190                 195

ccg aag ggc ttg cgc ccg ctt att acg cag ttg cag atc gtc cag ttc      678
Pro Lys Gly Leu Arg Pro Leu Ile Thr Gln Leu Gln Ile Val Gln Phe
            200                 205                 210

att ttc agc atc ggc atc cat acc gcc att tac tgg cac tac gac tgc      726
Ile Phe Ser Ile Gly Ile His Thr Ala Ile Tyr Trp His Tyr Asp Cys
        215                 220                 225

gag ccg ctc gtg cat acc cac ttt tgg gaa tac gtc acg ccc tac ctt      774
Glu Pro Leu Val His Thr His Phe Trp Glu Tyr Val Thr Pro Tyr Leu
    230                 235                 240

ttc gtc gtg ccc ttc ctc atc ctc ttt ttc aat ttt tac ctg cag cag      822
Phe Val Val Pro Phe Leu Ile Leu Phe Phe Asn Phe Tyr Leu Gln Gln
245                 250                 255                 260

tac gtc ctc gcg ccc gca aaa acc aag aag gca tag ccacgtaaca           868
Tyr Val Leu Ala Pro Ala Lys Thr Lys Lys Ala
                265                 270

gtagaccagc agcgccgagg acgcgtgccg cgttatcgcg aagcacgaaa taaagaagat    928

catttgattc aacgaggcta cttgcggcca cgagaaaaaa aaaaaaaaaa aaaaaaaaaa    988

aaaaaaaaaa aaaaaaaaaa aaaaaaaaaa aaaaaaaaaa aaaaaaaaaa aaaaaaaaaa   1048

c                                                                   1049
```

<210> 30
<211> 271
<212> PRT
<213> Thraustochytrium

<400> 30

```
Met Met Glu Pro Leu Asp Arg Tyr Arg Ala Leu Ala Glu Leu Ala Ala
1               5                   10                  15

Arg Tyr Ala Ser Ser Ala Ala Phe Lys Trp Gln Val Thr Tyr Asp Ala
            20                  25                  30
```

```
Lys Asp Ser Phe Val Gly Pro Leu Gly Ile Arg Glu Pro Leu Gly Leu
        35                  40                  45

Leu Val Gly Ser Val Val Leu Tyr Leu Ser Leu Leu Ala Val Val Tyr
    50                  55                  60

Ala Leu Arg Asn Tyr Leu Gly Gly Leu Met Ala Leu Arg Ser Val His
65                  70                  75                  80

Asn Leu Gly Leu Cys Leu Phe Ser Gly Ala Val Trp Ile Tyr Thr Ser
                85                  90                  95

Tyr Leu Met Ile Gln Asp Gly His Phe Arg Ser Leu Glu Ala Ala Thr
            100                 105                 110

Cys Glu Pro Leu Lys His Pro His Phe Gln Leu Ile Ser Leu Leu Phe
        115                 120                 125

Ala Leu Ser Lys Ile Trp Glu Trp Phe Asp Thr Val Leu Leu Ile Val
    130                 135                 140

Lys Gly Asn Lys Leu Arg Phe Leu His Val Leu His His Ala Thr Thr
145                 150                 155                 160

Phe Trp Leu Tyr Ala Ile Asp His Ile Phe Leu Ser Ser Ile Lys Tyr
                165                 170                 175

Gly Val Ala Val Asn Ala Phe Ile His Thr Val Met Tyr Ala His Tyr
            180                 185                 190

Phe Arg Pro Phe Pro Lys Gly Leu Arg Pro Leu Ile Thr Gln Leu Gln
        195                 200                 205

Ile Val Gln Phe Ile Phe Ser Ile Gly Ile His Thr Ala Ile Tyr Trp
    210                 215                 220

His Tyr Asp Cys Glu Pro Leu Val His Thr His Phe Trp Glu Tyr Val
225                 230                 235                 240

Thr Pro Tyr Leu Phe Val Val Pro Phe Leu Ile Leu Phe Phe Asn Phe
                245                 250                 255

Tyr Leu Gln Gln Tyr Val Leu Ala Pro Ala Lys Thr Lys Lys Ala
            260                 265                 270
```

<210> 31
<211> 837
<212> DNA
<213> Phytophthora infestans

<220>
<221> CDS
<222> (1)..(837)
<223> Delta-6-Elongase

<400> 31

```
atg tcg act gag cta ctg cag agc tac tac gcg tgg gcc aac gcc acg      48
Met Ser Thr Glu Leu Leu Gln Ser Tyr Tyr Ala Trp Ala Asn Ala Thr
1               5                   10                  15

gag gcc aag ctg ctg gac tgg gtc gac cct gag ggc ggc tgg aag gtg      96
Glu Ala Lys Leu Leu Asp Trp Val Asp Pro Glu Gly Gly Trp Lys Val
            20                  25                  30

cat cct atg gca gac tac ccc cta gcc aac ttc tcc agc gtc tac gcc     144
His Pro Met Ala Asp Tyr Pro Leu Ala Asn Phe Ser Ser Val Tyr Ala
        35                  40                  45

atc tgc gtc gga tac ttg ctc ttc gta atc ttc ggc acg gcc ctg atg     192
Ile Cys Val Gly Tyr Leu Leu Phe Val Ile Phe Gly Thr Ala Leu Met
    50                  55                  60

aaa atg gga gtc ccc gcc atc aag acc agt cca tta cag ttt gtg tac     240
Lys Met Gly Val Pro Ala Ile Lys Thr Ser Pro Leu Gln Phe Val Tyr
65                  70                  75                  80

aac ccc atc caa gtc att gcc tgc tct tat atg tgc gtg gag gcc gcc     288
Asn Pro Ile Gln Val Ile Ala Cys Ser Tyr Met Cys Val Glu Ala Ala
                85                  90                  95

atc cag gcc tac cgc aac ggc tac acc gcc gcc ccg tgc aac gcc ttt     336
Ile Gln Ala Tyr Arg Asn Gly Tyr Thr Ala Ala Pro Cys Asn Ala Phe
            100                 105                 110

aag tcc gac gac ccc gtc atg ggc aac gtt ctg tac ctc ttc tat ctc     384
Lys Ser Asp Asp Pro Val Met Gly Asn Val Leu Tyr Leu Phe Tyr Leu
        115                 120                 125

tcc aag atg ctc gac ctg tgc gac aca gtc ttc att atc cta gga aag     432
Ser Lys Met Leu Asp Leu Cys Asp Thr Val Phe Ile Ile Leu Gly Lys
    130                 135                 140

aag tgg aaa cag ctt tcc atc ttg cac gtg tac cac cac ctt acc gtg     480
Lys Trp Lys Gln Leu Ser Ile Leu His Val Tyr His His Leu Thr Val
145                 150                 155                 160

ctt ttc gtc tac tat gtg acg ttc cgc gcc gct cag gac ggg gac tca     528
Leu Phe Val Tyr Tyr Val Thr Phe Arg Ala Ala Gln Asp Gly Asp Ser
                165                 170                 175

tat gct acc atc gtg ctc aac ggc ttc gtg cac acc atc atg tac act     576
Tyr Ala Thr Ile Val Leu Asn Gly Phe Val His Thr Ile Met Tyr Thr
            180                 185                 190

tac tac ttc gtc agc gcc cac acg cgc aac att tgg tgg aag aag tac     624
Tyr Tyr Phe Val Ser Ala His Thr Arg Asn Ile Trp Trp Lys Lys Tyr
        195                 200                 205

ctc acg cgc att cag ctt atc cag ttc gtg acc atg aac gtg cag ggc     672
Leu Thr Arg Ile Gln Leu Ile Gln Phe Val Thr Met Asn Val Gln Gly
    210                 215                 220
```

```
tac ctg acc tac tct cga cag tgc cca ggc atg cct cct aag gtg ccg      720
Tyr Leu Thr Tyr Ser Arg Gln Cys Pro Gly Met Pro Pro Lys Val Pro
225                 230                 235                 240

ctc atg tac ctt gtg tac gtg cag tca ctc ttc tgg ctc ttc atg aat      768
Leu Met Tyr Leu Val Tyr Val Gln Ser Leu Phe Trp Leu Phe Met Asn
                245                 250                 255

ttc tac att cgc gcg tac gtg ttc ggc ccc aag aaa ccg gcc gtg gag      816
Phe Tyr Ile Arg Ala Tyr Val Phe Gly Pro Lys Lys Pro Ala Val Glu
            260                 265                 270

gaa tcg aag aag aag ttg taa                                          837
Glu Ser Lys Lys Lys Leu
        275
```

<210> 32
<211> 278
<212> PRT
<213> Phytophthora infestans

<400> 32

```
Met Ser Thr Glu Leu Leu Gln Ser Tyr Tyr Ala Trp Ala Asn Ala Thr
1               5                   10                  15

Glu Ala Lys Leu Leu Asp Trp Val Asp Pro Glu Gly Gly Trp Lys Val
            20                  25                  30

His Pro Met Ala Asp Tyr Pro Leu Ala Asn Phe Ser Ser Val Tyr Ala
        35                  40                  45

Ile Cys Val Gly Tyr Leu Leu Phe Val Ile Phe Gly Thr Ala Leu Met
    50                  55                  60

Lys Met Gly Val Pro Ala Ile Lys Thr Ser Pro Leu Gln Phe Val Tyr
65                  70                  75                  80

Asn Pro Ile Gln Val Ile Ala Cys Ser Tyr Met Cys Val Glu Ala Ala
                85                  90                  95

Ile Gln Ala Tyr Arg Asn Gly Tyr Thr Ala Ala Pro Cys Asn Ala Phe
            100                 105                 110

Lys Ser Asp Asp Pro Val Met Gly Asn Val Leu Tyr Leu Phe Tyr Leu
        115                 120                 125

Ser Lys Met Leu Asp Leu Cys Asp Thr Val Phe Ile Ile Leu Gly Lys
    130                 135                 140

Lys Trp Lys Gln Leu Ser Ile Leu His Val Tyr His His Leu Thr Val
145                 150                 155                 160
```

```
Leu Phe Val Tyr Tyr Val Thr Phe Arg Ala Ala Gln Asp Gly Asp Ser
                165                 170                 175

Tyr Ala Thr Ile Val Leu Asn Gly Phe Val His Thr Ile Met Tyr Thr
            180                 185                 190

Tyr Tyr Phe Val Ser Ala His Thr Arg Asn Ile Trp Trp Lys Lys Tyr
        195                 200                 205

Leu Thr Arg Ile Gln Leu Ile Gln Phe Val Thr Met Asn Val Gln Gly
    210                 215                 220

Tyr Leu Thr Tyr Ser Arg Gln Cys Pro Gly Met Pro Pro Lys Val Pro
225                 230                 235                 240

Leu Met Tyr Leu Val Tyr Val Gln Ser Leu Phe Trp Leu Phe Met Asn
                245                 250                 255

Phe Tyr Ile Arg Ala Tyr Val Phe Gly Pro Lys Lys Pro Ala Val Glu
            260                 265                 270

Glu Ser Lys Lys Lys Leu
        275
```

<210> 33
<211> 954
<212> DNA
<213> Mortierella alpina

<220>
<221> CDS
<222> (1)..(954)
<223> Delta-6-Elongase

<400> 33

```
atg gcc gcc gca atc ttg gac aag gtc aac ttc ggc att gat cag ccc      48
Met Ala Ala Ala Ile Leu Asp Lys Val Asn Phe Gly Ile Asp Gln Pro
1               5                   10                  15

ttc gga atc aag ctc gac acc tac ttt gct cag gcc tat gaa ctc gtc      96
Phe Gly Ile Lys Leu Asp Thr Tyr Phe Ala Gln Ala Tyr Glu Leu Val
            20                  25                  30

acc gga aag tcc atc gac tcc ttc gtc ttc cag gag ggc gtc acg cct     144
Thr Gly Lys Ser Ile Asp Ser Phe Val Phe Gln Glu Gly Val Thr Pro
        35                  40                  45

ctc tcg acc cag aga gag gtc gcc atg tgg act atc act tac ttc gtc     192
```

```
Leu Ser Thr Gln Arg Glu Val Ala Met Trp Thr Ile Thr Tyr Phe Val
    50                  55                  60

gtc atc ttt ggt ggt cgc cag atc atg aag agc cag gac gcc ttc aag      240
Val Ile Phe Gly Gly Arg Gln Ile Met Lys Ser Gln Asp Ala Phe Lys
65                  70                  75                  80

ctc aag ccc ctc ttc atc ctc cac aac ttc ctc ctg acg atc gcg tcc      288
Leu Lys Pro Leu Phe Ile Leu His Asn Phe Leu Leu Thr Ile Ala Ser
                85                  90                  95

gga tcg ctg ttg ctc ctg ttc atc gag aac ctg gtc ccc atc ctc gcc      336
Gly Ser Leu Leu Leu Leu Phe Ile Glu Asn Leu Val Pro Ile Leu Ala
            100                 105                 110

aga aac gga ctt ttc tac gcc atc tgc gac gac ggt gcc tgg acc cag      384
Arg Asn Gly Leu Phe Tyr Ala Ile Cys Asp Asp Gly Ala Trp Thr Gln
        115                 120                 125

cgc ctc gag ctc ctc tac tac ctc aac tac ctg gtc aag tac tgg gag      432
Arg Leu Glu Leu Leu Tyr Tyr Leu Asn Tyr Leu Val Lys Tyr Trp Glu
    130                 135                 140

ttg gcc gac acc gtc ttt ttg gtc ctc aag aag aag cct ctt gag ttc      480
Leu Ala Asp Thr Val Phe Leu Val Leu Lys Lys Lys Pro Leu Glu Phe
145                 150                 155                 160

ctg cac tac ttc cac cac tcg atg acc atg gtt ctc tgc ttt gtc cag      528
Leu His Tyr Phe His His Ser Met Thr Met Val Leu Cys Phe Val Gln
                165                 170                 175

ctt gga gga tac act tca gtg tcc tgg gtc cct att acc ctc aac ttg      576
Leu Gly Gly Tyr Thr Ser Val Ser Trp Val Pro Ile Thr Leu Asn Leu
            180                 185                 190

act gtc cac gtc ttc atg tac tac tac tac atg cgc tcc gct gcc ggt      624
Thr Val His Val Phe Met Tyr Tyr Tyr Tyr Met Arg Ser Ala Ala Gly
        195                 200                 205

gtt cgc atc tgg tgg aag cag tac ttg acc act ctc cag atc gtc cag      672
Val Arg Ile Trp Trp Lys Gln Tyr Leu Thr Thr Leu Gln Ile Val Gln
    210                 215                 220

ttc gtt ctt gac ctc gga ttc atc tac ttc tgc gcc tac acc tac ttc      720
Phe Val Leu Asp Leu Gly Phe Ile Tyr Phe Cys Ala Tyr Thr Tyr Phe
225                 230                 235                 240

gcc ttc acc tac ttc ccc tgg gct ccc aac gtc ggc aag tgc gcc ggt      768
Ala Phe Thr Tyr Phe Pro Trp Ala Pro Asn Val Gly Lys Cys Ala Gly
                245                 250                 255

acc gag ggt gct gct ctc ttt ggc tgc gga ctc ctc tcc agc tat ctc      816
Thr Glu Gly Ala Ala Leu Phe Gly Cys Gly Leu Leu Ser Ser Tyr Leu
            260                 265                 270

ttg ctc ttt atc aac ttc tac cgc att acc tac aat gcc aag gcc aag      864
Leu Leu Phe Ile Asn Phe Tyr Arg Ile Thr Tyr Asn Ala Lys Ala Lys
        275                 280                 285

gca gcc aag gag cgt gga agc aac ttt acc ccc aag act gtc aag tcc      912
Ala Ala Lys Glu Arg Gly Ser Asn Phe Thr Pro Lys Thr Val Lys Ser
    290                 295                 300

ggc gga tcg ccc aag aag ccc tcc aag agc aag cac atc taa              954
Gly Gly Ser Pro Lys Lys Pro Ser Lys Ser Lys His Ile
305                 310                 315
```

<210> 34
<211> 317
<212> PRT

<213> Mortierella alpina

<400> 34

```
Met Ala Ala Ala Ile Leu Asp Lys Val Asn Phe Gly Ile Asp Gln Pro
1               5                   10                  15

Phe Gly Ile Lys Leu Asp Thr Tyr Phe Ala Gln Ala Tyr Glu Leu Val
            20                  25                  30

Thr Gly Lys Ser Ile Asp Ser Phe Val Phe Gln Glu Gly Val Thr Pro
        35                  40                  45

Leu Ser Thr Gln Arg Glu Val Ala Met Trp Thr Ile Thr Tyr Phe Val
    50                  55                  60

Val Ile Phe Gly Gly Arg Gln Ile Met Lys Ser Gln Asp Ala Phe Lys
65                  70                  75                  80

Leu Lys Pro Leu Phe Ile Leu His Asn Phe Leu Leu Thr Ile Ala Ser
                85                  90                  95

Gly Ser Leu Leu Leu Leu Phe Ile Glu Asn Leu Val Pro Ile Leu Ala
            100                 105                 110

Arg Asn Gly Leu Phe Tyr Ala Ile Cys Asp Asp Gly Ala Trp Thr Gln
        115                 120                 125

Arg Leu Glu Leu Leu Tyr Tyr Leu Asn Tyr Leu Val Lys Tyr Trp Glu
    130                 135                 140

Leu Ala Asp Thr Val Phe Leu Val Leu Lys Lys Lys Pro Leu Glu Phe
145                 150                 155                 160

Leu His Tyr Phe His His Ser Met Thr Met Val Leu Cys Phe Val Gln
                165                 170                 175

Leu Gly Gly Tyr Thr Ser Val Ser Trp Val Pro Ile Thr Leu Asn Leu
            180                 185                 190

Thr Val His Val Phe Met Tyr Tyr Tyr Tyr Met Arg Ser Ala Ala Gly
        195                 200                 205

Val Arg Ile Trp Trp Lys Gln Tyr Leu Thr Thr Leu Gln Ile Val Gln
    210                 215                 220
```

Phe Val Leu Asp Leu Gly Phe Ile Tyr Phe Cys Ala Tyr Thr Tyr Phe
225 230 235 240

Ala Phe Thr Tyr Phe Pro Trp Ala Pro Asn Val Gly Lys Cys Ala Gly
245 250 255

Thr Glu Gly Ala Ala Leu Phe Gly Cys Gly Leu Leu Ser Ser Tyr Leu
260 265 270

Leu Leu Phe Ile Asn Phe Tyr Arg Ile Thr Tyr Asn Ala Lys Ala Lys
275 280 285

Ala Ala Lys Glu Arg Gly Ser Asn Phe Thr Pro Lys Thr Val Lys Ser
290 295 300

Gly Gly Ser Pro Lys Lys Pro Ser Lys Ser Lys His Ile
305 310 315

<210> 35
<211> 957
<212> DNA
<213> Mortierella alpina

<220>
<221> CDS
<222> (1)..(957)
<223> Delta-6-Elongase

<400> 35

atg gag tcg att gcg cca ttc ctc cca tca aag atg ccg caa gat ctg 48
Met Glu Ser Ile Ala Pro Phe Leu Pro Ser Lys Met Pro Gln Asp Leu
1 5 10 15

ttt atg gac ctt gcc acc gct atc ggt gtc cgg gcc gcg ccc tat gtc 96
Phe Met Asp Leu Ala Thr Ala Ile Gly Val Arg Ala Ala Pro Tyr Val
20 25 30

gat cct ctc gag gcc gcg ctg gtg gcc cag gcc gag aag tac atc ccc 144
Asp Pro Leu Glu Ala Ala Leu Val Ala Gln Ala Glu Lys Tyr Ile Pro
35 40 45

acg att gtc cat cac acg cgt ggg ttc ctg gtc gcg gtg gag tcg cct 192
Thr Ile Val His His Thr Arg Gly Phe Leu Val Ala Val Glu Ser Pro
50 55 60

ttg gcc cgt gag ctg ccg ttg atg aac ccg ttc cac gtg ctg ttg atc 240
Leu Ala Arg Glu Leu Pro Leu Met Asn Pro Phe His Val Leu Leu Ile
65 70 75 80

gtg ctc gct tat ttg gtc acg gtc ttt gtg ggc atg cag atc atg aag 288
Val Leu Ala Tyr Leu Val Thr Val Phe Val Gly Met Gln Ile Met Lys
85 90 95

```
aac ttt gag cgg ttc gag gtc aag acg ttt tcg ctc ctg cac aac ttt      336
Asn Phe Glu Arg Phe Glu Val Lys Thr Phe Ser Leu Leu His Asn Phe
            100                 105                 110

tgt ctg gtc tcg atc agc gcc tac atg tgc ggt ggg atc ctg tac gag      384
Cys Leu Val Ser Ile Ser Ala Tyr Met Cys Gly Gly Ile Leu Tyr Glu
        115                 120                 125

gct tat cag gcc aac tat gga ctg ttt gag aac gct gct gat cat acc      432
Ala Tyr Gln Ala Asn Tyr Gly Leu Phe Glu Asn Ala Ala Asp His Thr
    130                 135                 140

ttc aag ggt ctt cct atg gcc aag atg atc tgg ctc ttc tac ttc tcc      480
Phe Lys Gly Leu Pro Met Ala Lys Met Ile Trp Leu Phe Tyr Phe Ser
145                 150                 155                 160

aag atc atg gag ttt gtc gac acc atg atc atg gtc ctc aag aag aac      528
Lys Ile Met Glu Phe Val Asp Thr Met Ile Met Val Leu Lys Lys Asn
                165                 170                 175

aac cgc cag atc tcc ttc ttg cac gtt tac cac cac agc tcc atc ttc      576
Asn Arg Gln Ile Ser Phe Leu His Val Tyr His His Ser Ser Ile Phe
            180                 185                 190

acc atc tgg tgg ttg gtc acc ttt gtt gca ccc aac ggt gaa gcc tac      624
Thr Ile Trp Trp Leu Val Thr Phe Val Ala Pro Asn Gly Glu Ala Tyr
        195                 200                 205

ttc tct gct gcg ttg aac tcg ttc atc cat gtg atc atg tac ggc tac      672
Phe Ser Ala Ala Leu Asn Ser Phe Ile His Val Ile Met Tyr Gly Tyr
    210                 215                 220

tac ttc ttg tcg gcc ttg ggc ttc aag cag gtg tcg ttc atc aag ttc      720
Tyr Phe Leu Ser Ala Leu Gly Phe Lys Gln Val Ser Phe Ile Lys Phe
225                 230                 235                 240

tac atc acg cgc tcg cag atg aca cag ttc tgc atg atg tcg gtc cag      768
Tyr Ile Thr Arg Ser Gln Met Thr Gln Phe Cys Met Met Ser Val Gln
                245                 250                 255

tct tcc tgg gac atg tac gcc atg aag gtc ctt ggc cgc ccc gga tac      816
Ser Ser Trp Asp Met Tyr Ala Met Lys Val Leu Gly Arg Pro Gly Tyr
            260                 265                 270

ccc ttc ttc atc acg gct ctg ctt tgg ttc tac atg tgg acc atg ctc      864
Pro Phe Phe Ile Thr Ala Leu Leu Trp Phe Tyr Met Trp Thr Met Leu
        275                 280                 285

ggt ctc ttc tac aac ttt tac aga aag aac gcc aag ttg gcc aag cag      912
Gly Leu Phe Tyr Asn Phe Tyr Arg Lys Asn Ala Lys Leu Ala Lys Gln
    290                 295                 300

gcc aag gcc gac gct gcc aag gag aag gca agg aag ttg cag taa          957
Ala Lys Ala Asp Ala Ala Lys Glu Lys Ala Arg Lys Leu Gln
305                 310                 315
```

<210> 36
<211> 318
<212> PRT
<213> Mortierella alpina

<400> 36

```
Met Glu Ser Ile Ala Pro Phe Leu Pro Ser Lys Met Pro Gln Asp Leu
1               5                   10                  15

Phe Met Asp Leu Ala Thr Ala Ile Gly Val Arg Ala Ala Pro Tyr Val
            20                  25                  30

Asp Pro Leu Glu Ala Ala Leu Val Ala Gln Ala Glu Lys Tyr Ile Pro
        35                  40                  45

Thr Ile Val His His Thr Arg Gly Phe Leu Val Ala Val Glu Ser Pro
    50                  55                  60

Leu Ala Arg Glu Leu Pro Leu Met Asn Pro Phe His Val Leu Leu Ile
65                  70                  75                  80

Val Leu Ala Tyr Leu Val Thr Val Phe Val Gly Met Gln Ile Met Lys
                85                  90                  95

Asn Phe Glu Arg Phe Glu Val Lys Thr Phe Ser Leu Leu His Asn Phe
            100                 105                 110

Cys Leu Val Ser Ile Ser Ala Tyr Met Cys Gly Gly Ile Leu Tyr Glu
        115                 120                 125

Ala Tyr Gln Ala Asn Tyr Gly Leu Phe Glu Asn Ala Ala Asp His Thr
    130                 135                 140

Phe Lys Gly Leu Pro Met Ala Lys Met Ile Trp Leu Phe Tyr Phe Ser
145                 150                 155                 160

Lys Ile Met Glu Phe Val Asp Thr Met Ile Met Val Leu Lys Lys Asn
                165                 170                 175

Asn Arg Gln Ile Ser Phe Leu His Val Tyr His His Ser Ser Ile Phe
            180                 185                 190

Thr Ile Trp Trp Leu Val Thr Phe Val Ala Pro Asn Gly Glu Ala Tyr
        195                 200                 205

Phe Ser Ala Ala Leu Asn Ser Phe Ile His Val Ile Met Tyr Gly Tyr
    210                 215                 220

Tyr Phe Leu Ser Ala Leu Gly Phe Lys Gln Val Ser Phe Ile Lys Phe
225                 230                 235                 240

Tyr Ile Thr Arg Ser Gln Met Thr Gln Phe Cys Met Met Ser Val Gln
                245                 250                 255

Ser Ser Trp Asp Met Tyr Ala Met Lys Val Leu Gly Arg Pro Gly Tyr
            260                 265                 270
```

```
Pro Phe Phe Ile Thr Ala Leu Leu Trp Phe Tyr Met Trp Thr Met Leu
        275                 280                 285

Gly Leu Phe Tyr Asn Phe Tyr Arg Lys Asn Ala Lys Leu Ala Lys Gln
    290                 295                 300

Ala Lys Ala Asp Ala Ala Lys Glu Lys Ala Arg Lys Leu Gln
305                 310                 315
```

<210> 37
<211> 867
<212> DNA
<213> Caenorhabditis elegans

<220>
<221> CDS
<222> (1)..(867)
<223> Delta-6-Elongase

<400> 37

```
atg gct cag cat ccg ctc gtt caa cgg ctt ctc gat gtc aaa ttc gac      48
Met Ala Gln His Pro Leu Val Gln Arg Leu Leu Asp Val Lys Phe Asp
1               5                   10                  15

acg aaa cga ttt gtg gct att gct act cat ggg cca aag aat ttc cct      96
Thr Lys Arg Phe Val Ala Ile Ala Thr His Gly Pro Lys Asn Phe Pro
            20                  25                  30

gac gca gaa ggt cgc aag ttc ttt gct gat cac ttt gat gtt act att      144
Asp Ala Glu Gly Arg Lys Phe Phe Ala Asp His Phe Asp Val Thr Ile
        35                  40                  45

cag gct tca atc ctg tac atg gtc gtt gtg ttc gga aca aaa tgg ttc      192
Gln Ala Ser Ile Leu Tyr Met Val Val Val Phe Gly Thr Lys Trp Phe
    50                  55                  60

atg cgt aat cgt caa cca ttc caa ttg act att cca ctc aac atc tgg      240
Met Arg Asn Arg Gln Pro Phe Gln Leu Thr Ile Pro Leu Asn Ile Trp
65                  70                  75                  80

aat ttc atc ctc gcc gca ttt tcc atc gca gga gct gtc aaa atg acc      288
Asn Phe Ile Leu Ala Ala Phe Ser Ile Ala Gly Ala Val Lys Met Thr
                85                  90                  95

cca gag ttc ttt gga acc att gcc aac aaa gga att gtc gca tcc tac      336
Pro Glu Phe Phe Gly Thr Ile Ala Asn Lys Gly Ile Val Ala Ser Tyr
            100                 105                 110

tgc aaa gtg ttt gat ttc acg aaa gga gag aat gga tac tgg gtg tgg      384
Cys Lys Val Phe Asp Phe Thr Lys Gly Glu Asn Gly Tyr Trp Val Trp
        115                 120                 125

ctc ttc atg gct tcc aaa ctt ttc gaa ctt gtt gac acc atc ttc ttg      432
```

Leu Phe Met Ala Ser Lys Leu Phe Glu Leu Val Asp Thr Ile Phe Leu
130 135 140

gtt ctc cgt aaa cgt cca ctc atg ttc ctt cac tgg tat cac cat att 480
Val Leu Arg Lys Arg Pro Leu Met Phe Leu His Trp Tyr His His Ile
145 150 155 160

ctc acc atg atc tac gcc tgg tac tct cat cca ttg acc cca gga ttc 528
Leu Thr Met Ile Tyr Ala Trp Tyr Ser His Pro Leu Thr Pro Gly Phe
165 170 175

aac aga tac gga att tat ctt aac ttt gtc gtc cac gcc ttc atg tac 576
Asn Arg Tyr Gly Ile Tyr Leu Asn Phe Val Val His Ala Phe Met Tyr
180 185 190

tct tac tac ttc ctt cgc tcg atg aag att cgc gtg cca gga ttc atc 624
Ser Tyr Tyr Phe Leu Arg Ser Met Lys Ile Arg Val Pro Gly Phe Ile
195 200 205

gcc caa gct atc aca tct ctt caa atc gtt caa ttc atc atc tct tgc 672
Ala Gln Ala Ile Thr Ser Leu Gln Ile Val Gln Phe Ile Ile Ser Cys
210 215 220

gcc gtt ctt gct cat ctt ggt tat ctc atg cac ttc acc aat gcc aac 720
Ala Val Leu Ala His Leu Gly Tyr Leu Met His Phe Thr Asn Ala Asn
225 230 235 240

tgt gat ttc gag cca tca gta ttc aag ctc gca gtt ttc atg gac aca 768
Cys Asp Phe Glu Pro Ser Val Phe Lys Leu Ala Val Phe Met Asp Thr
245 250 255

aca tac ttg gct ctt ttc gtc aac ttc ttc ctc caa tca tat gtt ctc 816
Thr Tyr Leu Ala Leu Phe Val Asn Phe Phe Leu Gln Ser Tyr Val Leu
260 265 270

cgc gga gga aaa gac aag tac aag gca gtg cca aag aag aag aac aac 864
Arg Gly Gly Lys Asp Lys Tyr Lys Ala Val Pro Lys Lys Lys Asn Asn
275 280 285

taa 867

<210> 38
<211> 288
<212> PRT
<213> Caenorhabditis elegans

<400> 38

Met Ala Gln His Pro Leu Val Gln Arg Leu Leu Asp Val Lys Phe Asp
1 5 10 15

Thr Lys Arg Phe Val Ala Ile Ala Thr His Gly Pro Lys Asn Phe Pro
20 25 30

Asp Ala Glu Gly Arg Lys Phe Phe Ala Asp His Phe Asp Val Thr Ile
35 40 45

Gln Ala Ser Ile Leu Tyr Met Val Val Val Phe Gly Thr Lys Trp Phe
50 55 60

Met Arg Asn Arg Gln Pro Phe Gln Leu Thr Ile Pro Leu Asn Ile Trp
65 70 75 80

Asn Phe Ile Leu Ala Ala Phe Ser Ile Ala Gly Ala Val Lys Met Thr
85 90 95

Pro Glu Phe Phe Gly Thr Ile Ala Asn Lys Gly Ile Val Ala Ser Tyr
100 105 110

Cys Lys Val Phe Asp Phe Thr Lys Gly Glu Asn Gly Tyr Trp Val Trp
115 120 125

Leu Phe Met Ala Ser Lys Leu Phe Glu Leu Val Asp Thr Ile Phe Leu
130 135 140

Val Leu Arg Lys Arg Pro Leu Met Phe Leu His Trp Tyr His His Ile
145 150 155 160

Leu Thr Met Ile Tyr Ala Trp Tyr Ser His Pro Leu Thr Pro Gly Phe
165 170 175

Asn Arg Tyr Gly Ile Tyr Leu Asn Phe Val Val His Ala Phe Met Tyr
180 185 190

Ser Tyr Tyr Phe Leu Arg Ser Met Lys Ile Arg Val Pro Gly Phe Ile
195 200 205

Ala Gln Ala Ile Thr Ser Leu Gln Ile Val Gln Phe Ile Ile Ser Cys
210 215 220

Ala Val Leu Ala His Leu Gly Tyr Leu Met His Phe Thr Asn Ala Asn
225 230 235 240

Cys Asp Phe Glu Pro Ser Val Phe Lys Leu Ala Val Phe Met Asp Thr
245 250 255

Thr Tyr Leu Ala Leu Phe Val Asn Phe Phe Leu Gln Ser Tyr Val Leu
260 265 270

Arg Gly Gly Lys Asp Lys Tyr Lys Ala Val Pro Lys Lys Lys Asn Asn
275 280 285

<210> 39
<211> 1626
<212> DNA
<213> Euglena gracilis

<220>
<221> CDS
<222> (1)..(1626)
<223> Delta-4-Desaturase

<400> 39

```
atg ttg gtg ctg ttt ggc aat ttc tat gtc aag caa tac tcc caa aag      48
Met Leu Val Leu Phe Gly Asn Phe Tyr Val Lys Gln Tyr Ser Gln Lys
1               5                   10                  15

aac ggc aag ccg gag aac gga gcc acc cct gag aac gga gcg aag ccg      96
Asn Gly Lys Pro Glu Asn Gly Ala Thr Pro Glu Asn Gly Ala Lys Pro
            20                  25                  30

caa cct tgc gag aac ggc acg gtg gaa aag cga gag aat gac acc gcc     144
Gln Pro Cys Glu Asn Gly Thr Val Glu Lys Arg Glu Asn Asp Thr Ala
        35                  40                  45

aac gtt cgg ccc acc cgt cca gct gga ccc ccg ccg gcc acg tac tac     192
Asn Val Arg Pro Thr Arg Pro Ala Gly Pro Pro Pro Ala Thr Tyr Tyr
    50                  55                  60

gac tcc ctg gca gtg tcg ggg cag ggc aag gag cgg ctg ttc acc acc     240
Asp Ser Leu Ala Val Ser Gly Gln Gly Lys Glu Arg Leu Phe Thr Thr
65                  70                  75                  80

gat gag gtg agg cgg cac atc ctc ccc acc gat ggc tgg ctg acg tgc     288
Asp Glu Val Arg Arg His Ile Leu Pro Thr Asp Gly Trp Leu Thr Cys
                85                  90                  95

cac gaa gga gtc tac gat gtc act gat ttc ctt gcc aag cac cct ggt     336
His Glu Gly Val Tyr Asp Val Thr Asp Phe Leu Ala Lys His Pro Gly
            100                 105                 110

ggc ggt gtc atc acg ctg ggc ctt gga agg gac tgc aca atc ctc atc     384
Gly Gly Val Ile Thr Leu Gly Leu Gly Arg Asp Cys Thr Ile Leu Ile
        115                 120                 125

gag tca tac cac cct gct ggg cgc ccg gac aag gtg atg gag aag tac     432
Glu Ser Tyr His Pro Ala Gly Arg Pro Asp Lys Val Met Glu Lys Tyr
    130                 135                 140

cgc att ggt acg ctg cag gac ccc aag acg ttc tat gct tgg gga gag     480
Arg Ile Gly Thr Leu Gln Asp Pro Lys Thr Phe Tyr Ala Trp Gly Glu
145                 150                 155                 160

tcc gat ttc tac cct gag ttg aag cgc cgg gcc ctt gca agg ctg aag     528
Ser Asp Phe Tyr Pro Glu Leu Lys Arg Arg Ala Leu Ala Arg Leu Lys
                165                 170                 175

gag gct ggt cag gcg cgg cgc ggc ggc ctt ggg gtg aag gcc ctc ctg     576
Glu Ala Gly Gln Ala Arg Arg Gly Gly Leu Gly Val Lys Ala Leu Leu
            180                 185                 190

gtg ctc acc ctc ttc ttc gtg tcg tgg tac atg tgg gtg gcc cac aag     624
Val Leu Thr Leu Phe Phe Val Ser Trp Tyr Met Trp Val Ala His Lys
        195                 200                 205

tcc ttc ctc tgg gcc gcc gtc tgg ggc ttc gcc ggc tcc cac gtc ggg     672
Ser Phe Leu Trp Ala Ala Val Trp Gly Phe Ala Gly Ser His Val Gly
    210                 215                 220

ctg agc atc cag cac gat ggc aac cac ggc gcg ttc agc cgc aac aca     720
```

```
Leu Ser Ile Gln His Asp Gly Asn His Gly Ala Phe Ser Arg Asn Thr
225                 230                 235                 240

ctg gtg aac cgc ctg gcg ggg tgg ggc atg gac ttg atc ggc gcg tcg      768
Leu Val Asn Arg Leu Ala Gly Trp Gly Met Asp Leu Ile Gly Ala Ser
                245                 250                 255

tcc acg gtg tgg gag tac cag cac gtc atc ggc cac cac cag tac acc      816
Ser Thr Val Trp Glu Tyr Gln His Val Ile Gly His His Gln Tyr Thr
            260                 265                 270

aac ctc gtg tcg gac acg cta ttc agt ctg cct gag aac gat ccg gac      864
Asn Leu Val Ser Asp Thr Leu Phe Ser Leu Pro Glu Asn Asp Pro Asp
        275                 280                 285

gtc ttc tcc agc tac ccg ctg atg cgc atg cac ccg gat acg gcg tgg      912
Val Phe Ser Ser Tyr Pro Leu Met Arg Met His Pro Asp Thr Ala Trp
    290                 295                 300

cag ccg cac cac cgc ttc cag cac ctg ttc gcg ttc cca ctg ttc gcc      960
Gln Pro His His Arg Phe Gln His Leu Phe Ala Phe Pro Leu Phe Ala
305                 310                 315                 320

ctg atg aca atc agc aag gtg ctg acc agc gat ttc gct gtc tgc ctc     1008
Leu Met Thr Ile Ser Lys Val Leu Thr Ser Asp Phe Ala Val Cys Leu
                325                 330                 335

agc atg aag aag ggg tcc atc gac tgc tcc tcc agg ctc gtc cca ctg     1056
Ser Met Lys Lys Gly Ser Ile Asp Cys Ser Ser Arg Leu Val Pro Leu
            340                 345                 350

gag ggg cag ctg ctg ttc tgg ggg gcc aag ctg gcg aac ttc ctg ttg     1104
Glu Gly Gln Leu Leu Phe Trp Gly Ala Lys Leu Ala Asn Phe Leu Leu
        355                 360                 365

cag att gtg ttg cca tgc tac ctc cac ggg aca gct atg ggc ctg gcc     1152
Gln Ile Val Leu Pro Cys Tyr Leu His Gly Thr Ala Met Gly Leu Ala
    370                 375                 380

ctc ttc tct gtt gct cac ctt gtg tcg ggg gag tac ctc gcg atc tgc     1200
Leu Phe Ser Val Ala His Leu Val Ser Gly Glu Tyr Leu Ala Ile Cys
385                 390                 395                 400

ttc atc atc aac cac atc agc gag tct tgt gag ttt atg aat aca agc     1248
Phe Ile Ile Asn His Ile Ser Glu Ser Cys Glu Phe Met Asn Thr Ser
                405                 410                 415

ttt caa acc gcc gcc cgg agg aca gag atg ctt cag gca gca cat cag     1296
Phe Gln Thr Ala Ala Arg Arg Thr Glu Met Leu Gln Ala Ala His Gln
            420                 425                 430

gca gcg gag gcc aag aag gtg aag ccc acc cct cca ccg aac gat tgg     1344
Ala Ala Glu Ala Lys Lys Val Lys Pro Thr Pro Pro Pro Asn Asp Trp
        435                 440                 445

gct gtg aca cag gtc caa tgc tgc gtg aat tgg aga tca ggt ggc gtg     1392
Ala Val Thr Gln Val Gln Cys Cys Val Asn Trp Arg Ser Gly Gly Val
    450                 455                 460

ttg gcc aat cac ctc tct gga ggc ttg aac cac cag atc gag cat cat     1440
Leu Ala Asn His Leu Ser Gly Gly Leu Asn His Gln Ile Glu His His
465                 470                 475                 480

ctg ttc ccc agc atc tcg cat gcc aac tac ccc acc atc gcc cct gtt     1488
Leu Phe Pro Ser Ile Ser His Ala Asn Tyr Pro Thr Ile Ala Pro Val
                485                 490                 495

gtg aag gag gtg tgc gag gag tac ggg ttg ccg tac aag aat tac gtc     1536
```

```
Val Lys Glu Val Cys Glu Glu Tyr Gly Leu Pro Tyr Lys Asn Tyr Val
            500                 505                 510

acg ttc tgg gat gca gtc tgt ggc atg gtt cag cac ctc cgg ttg atg      1584
Thr Phe Trp Asp Ala Val Cys Gly Met Val Gln His Leu Arg Leu Met
        515                 520                 525

ggt gct cca ccg gtg cca acg aac ggg gac aaa aag tca taa              1626
Gly Ala Pro Pro Val Pro Thr Asn Gly Asp Lys Lys Ser
    530                 535                 540
```

<210> 40
<211> 541
<212> PRT
<213> Euglena gracilis

<400> 40

```
Met Leu Val Leu Phe Gly Asn Phe Tyr Val Lys Gln Tyr Ser Gln Lys
1               5                   10                  15

Asn Gly Lys Pro Glu Asn Gly Ala Thr Pro Glu Asn Gly Ala Lys Pro
            20                  25                  30

Gln Pro Cys Glu Asn Gly Thr Val Glu Lys Arg Glu Asn Asp Thr Ala
        35                  40                  45

Asn Val Arg Pro Thr Arg Pro Ala Gly Pro Pro Pro Ala Thr Tyr Tyr
    50                  55                  60

Asp Ser Leu Ala Val Ser Gly Gln Gly Lys Glu Arg Leu Phe Thr Thr
65                  70                  75                  80

Asp Glu Val Arg Arg His Ile Leu Pro Thr Asp Gly Trp Leu Thr Cys
                85                  90                  95

His Glu Gly Val Tyr Asp Val Thr Asp Phe Leu Ala Lys His Pro Gly
            100                 105                 110

Gly Gly Val Ile Thr Leu Gly Leu Gly Arg Asp Cys Thr Ile Leu Ile
        115                 120                 125

Glu Ser Tyr His Pro Ala Gly Arg Pro Asp Lys Val Met Glu Lys Tyr
    130                 135                 140

Arg Ile Gly Thr Leu Gln Asp Pro Lys Thr Phe Tyr Ala Trp Gly Glu
145                 150                 155                 160

Ser Asp Phe Tyr Pro Glu Leu Lys Arg Arg Ala Leu Ala Arg Leu Lys
                165                 170                 175
```

Glu Ala Gly Gln Ala Arg Arg Gly Gly Leu Gly Val Lys Ala Leu Leu
180 185 190

Val Leu Thr Leu Phe Phe Val Ser Trp Tyr Met Trp Val Ala His Lys
195 200 205

Ser Phe Leu Trp Ala Ala Val Trp Gly Phe Ala Gly Ser His Val Gly
210 215 220

Leu Ser Ile Gln His Asp Gly Asn His Gly Ala Phe Ser Arg Asn Thr
225 230 235 240

Leu Val Asn Arg Leu Ala Gly Trp Gly Met Asp Leu Ile Gly Ala Ser
245 250 255

Ser Thr Val Trp Glu Tyr Gln His Val Ile Gly His His Gln Tyr Thr
260 265 270

Asn Leu Val Ser Asp Thr Leu Phe Ser Leu Pro Glu Asn Asp Pro Asp
275 280 285

Val Phe Ser Ser Tyr Pro Leu Met Arg Met His Pro Asp Thr Ala Trp
290 295 300

Gln Pro His His Arg Phe Gln His Leu Phe Ala Phe Pro Leu Phe Ala
305 310 315 320

Leu Met Thr Ile Ser Lys Val Leu Thr Ser Asp Phe Ala Val Cys Leu
325 330 335

Ser Met Lys Lys Gly Ser Ile Asp Cys Ser Ser Arg Leu Val Pro Leu
340 345 350

Glu Gly Gln Leu Leu Phe Trp Gly Ala Lys Leu Ala Asn Phe Leu Leu
355 360 365

Gln Ile Val Leu Pro Cys Tyr Leu His Gly Thr Ala Met Gly Leu Ala
370 375 380

Leu Phe Ser Val Ala His Leu Val Ser Gly Glu Tyr Leu Ala Ile Cys
385 390 395 400

Phe Ile Ile Asn His Ile Ser Glu Ser Cys Glu Phe Met Asn Thr Ser
405 410 415

Phe Gln Thr Ala Ala Arg Arg Thr Glu Met Leu Gln Ala Ala His Gln
420 425 430

Ala Ala Glu Ala Lys Lys Val Lys Pro Thr Pro Pro Pro Asn Asp Trp
435 440 445

Ala Val Thr Gln Val Gln Cys Cys Val Asn Trp Arg Ser Gly Gly Val
450 455 460

Leu Ala Asn His Leu Ser Gly Gly Leu Asn His Gln Ile Glu His His
465 470 475 480

Leu Phe Pro Ser Ile Ser His Ala Asn Tyr Pro Thr Ile Ala Pro Val
485 490 495

Val Lys Glu Val Cys Glu Glu Tyr Gly Leu Pro Tyr Lys Asn Tyr Val
500 505 510

Thr Phe Trp Asp Ala Val Cys Gly Met Val Gln His Leu Arg Leu Met
515 520 525

Gly Ala Pro Pro Val Pro Thr Asn Gly Asp Lys Lys Ser
530 535 540

<210> 41
<211> 1548
<212> DNA
<213> Thraustochytrium

<220>
<221> CDS
<222> (1)..(1548)
<223> Delta-4-Desaturase

<400> 41

atg acg gtc ggg ttt gac gaa acg gtg act atg gac acg gtc cgc aac 48
Met Thr Val Gly Phe Asp Glu Thr Val Thr Met Asp Thr Val Arg Asn
1 5 10 15

cac aac atg ccg gac gac gcc tgg tgc gcg atc cac ggc acc gtg tac 96
His Asn Met Pro Asp Asp Ala Trp Cys Ala Ile His Gly Thr Val Tyr
20 25 30

gac atc acc aag ttc agc aag gtg cac ccc ggc ggg gac atc atc atg 144
Asp Ile Thr Lys Phe Ser Lys Val His Pro Gly Gly Asp Ile Ile Met
35 40 45

ctg gcc gct ggc aag gag gcc acc atc ctg ttc gag acc tac cac atc 192
Leu Ala Ala Gly Lys Glu Ala Thr Ile Leu Phe Glu Thr Tyr His Ile
50 55 60

aag ggc gtc ccg gac gcg gtg ctg cgc aag tac aag gtc ggc aag ctc 240
Lys Gly Val Pro Asp Ala Val Leu Arg Lys Tyr Lys Val Gly Lys Leu
65 70 75 80

ccc cag ggc aag aag ggc gaa acg agc cac atg ccc acc ggg ctc gac 288
Pro Gln Gly Lys Lys Gly Glu Thr Ser His Met Pro Thr Gly Leu Asp
85 90 95

```
tcg gcc tcc tac tac tcg tgg gac agc gag ttt tac agg gtg ctc cgc      336
Ser Ala Ser Tyr Tyr Ser Trp Asp Ser Glu Phe Tyr Arg Val Leu Arg
            100                 105                 110

gag cgc gtc gcc aag aag ctg gcc gag ccc ggc ctc atg cag cgc gcg      384
Glu Arg Val Ala Lys Lys Leu Ala Glu Pro Gly Leu Met Gln Arg Ala
        115                 120                 125

cgc atg gag ctc tgg gcc aag gcg atc ttc ctc ctg gca ggt ttc tgg      432
Arg Met Glu Leu Trp Ala Lys Ala Ile Phe Leu Leu Ala Gly Phe Trp
    130                 135                 140

ggc tcc ctt tac gcc atg tgc gtg cta gac ccg cac ggc ggt gcc atg      480
Gly Ser Leu Tyr Ala Met Cys Val Leu Asp Pro His Gly Gly Ala Met
145                 150                 155                 160

gta gcc gcc gtt acg ctc ggc gtg ttc gct gcc ttt gtc gga act tgc      528
Val Ala Ala Val Thr Leu Gly Val Phe Ala Ala Phe Val Gly Thr Cys
                165                 170                 175

atc cag cac gac ggc agc cac ggc gcc ttc tcc aag tcg cga ttc atg      576
Ile Gln His Asp Gly Ser His Gly Ala Phe Ser Lys Ser Arg Phe Met
            180                 185                 190

aac aag gcg gcg ggc tgg acc ctc gac atg atc ggc gcg agt gcg atg      624
Asn Lys Ala Ala Gly Trp Thr Leu Asp Met Ile Gly Ala Ser Ala Met
        195                 200                 205

acc tgg gag atg cag cac gtt ctt ggc cac cac ccg tac acc aac ctc      672
Thr Trp Glu Met Gln His Val Leu Gly His His Pro Tyr Thr Asn Leu
    210                 215                 220

atc gag atg gag aac ggt ttg gcc aag gtc aag ggc gcc gac gtc gac      720
Ile Glu Met Glu Asn Gly Leu Ala Lys Val Lys Gly Ala Asp Val Asp
225                 230                 235                 240

ccg aag aag gtc gac cag gag agc gac ccg gac gtc ttc agt acg tac      768
Pro Lys Lys Val Asp Gln Glu Ser Asp Pro Asp Val Phe Ser Thr Tyr
                245                 250                 255

ccg atg ctt cgc ctg cac ccg tgg cac cgc cag cgg ttt tac cac aag      816
Pro Met Leu Arg Leu His Pro Trp His Arg Gln Arg Phe Tyr His Lys
            260                 265                 270

ttc cag cac ctg tac gcc ccg ttt atc ttt ggg tct atg acg att aac      864
Phe Gln His Leu Tyr Ala Pro Phe Ile Phe Gly Ser Met Thr Ile Asn
        275                 280                 285

aag gtg att tcc cag gat gtc ggg gtt gtg ctg cgc aag cgc ctg ttc      912
Lys Val Ile Ser Gln Asp Val Gly Val Val Leu Arg Lys Arg Leu Phe
    290                 295                 300

cag atc gac gcc aac tgc cgg tat ggc agc ccc tgg tac gtg gcc cgc      960
Gln Ile Asp Ala Asn Cys Arg Tyr Gly Ser Pro Trp Tyr Val Ala Arg
305                 310                 315                 320

ttc tgg atc atg aag ctc ctc acc acg ctc tac atg gtg gcg ctt ccc     1008
Phe Trp Ile Met Lys Leu Leu Thr Thr Leu Tyr Met Val Ala Leu Pro
                325                 330                 335

atg tac atg cag ggg cct gct cag ggc ttg aag ctt ttc ttc atg gcc     1056
Met Tyr Met Gln Gly Pro Ala Gln Gly Leu Lys Leu Phe Phe Met Ala
            340                 345                 350

cac ttc acc tgc gga gag gtc ctc gcc acc atg ttt att gtc aac cac     1104
His Phe Thr Cys Gly Glu Val Leu Ala Thr Met Phe Ile Val Asn His
        355                 360                 365
```

```
atc atc gag ggc gtc agc tac gct tcc aag gac gcg gtc aag ggc gtc      1152
Ile Ile Glu Gly Val Ser Tyr Ala Ser Lys Asp Ala Val Lys Gly Val
    370                 375                 380

atg gct ccg ccg cgc act gtg cac ggt gtc acc ccg atg cag gtg acg      1200
Met Ala Pro Pro Arg Thr Val His Gly Val Thr Pro Met Gln Val Thr
385                 390                 395                 400

caa aag gcg ctc agt gcg gcc gag tcg gcc aag tcg gac gcc gac aag      1248
Gln Lys Ala Leu Ser Ala Ala Glu Ser Ala Lys Ser Asp Ala Asp Lys
                405                 410                 415

acg acc atg atc ccc ctc aac gac tgg gcc gct gtg cag tgc cag acc      1296
Thr Thr Met Ile Pro Leu Asn Asp Trp Ala Ala Val Gln Cys Gln Thr
            420                 425                 430

tct gtg aac tgg gct gtc ggg tcg tgg ttt tgg aac cac ttt tcg ggc      1344
Ser Val Asn Trp Ala Val Gly Ser Trp Phe Trp Asn His Phe Ser Gly
        435                 440                 445

ggc ctc aac cac cag att gag cac cac tgc ttc ccc caa aac ccc cac      1392
Gly Leu Asn His Gln Ile Glu His His Cys Phe Pro Gln Asn Pro His
    450                 455                 460

acg gtc aac gtc tac atc tcg ggc atc gtc aag gag acc tgc gaa gaa      1440
Thr Val Asn Val Tyr Ile Ser Gly Ile Val Lys Glu Thr Cys Glu Glu
465                 470                 475                 480

tac ggc gtg ccg tac cag gct gag atc agc ctc ttc tct gcc tat ttc      1488
Tyr Gly Val Pro Tyr Gln Ala Glu Ile Ser Leu Phe Ser Ala Tyr Phe
                485                 490                 495

aag atg ctg tcg cac ctc cgc acg ctc ggc aac gag gac ctc acg gcc      1536
Lys Met Leu Ser His Leu Arg Thr Leu Gly Asn Glu Asp Leu Thr Ala
            500                 505                 510

tgg tcc acg tga                                                      1548
Trp Ser Thr
        515
```

<210> 42
<211> 515
<212> PRT
<213> Thraustochytrium

<400> 42

```
Met Thr Val Gly Phe Asp Glu Thr Val Thr Met Asp Thr Val Arg Asn
1               5                   10                  15

His Asn Met Pro Asp Asp Ala Trp Cys Ala Ile His Gly Thr Val Tyr
            20                  25                  30

Asp Ile Thr Lys Phe Ser Lys Val His Pro Gly Gly Asp Ile Ile Met
        35                  40                  45

Leu Ala Ala Gly Lys Glu Ala Thr Ile Leu Phe Glu Thr Tyr His Ile
    50                  55                  60
```

Lys Gly Val Pro Asp Ala Val Leu Arg Lys Tyr Lys Val Gly Lys Leu
65 70 75 80

Pro Gln Gly Lys Lys Gly Glu Thr Ser His Met Pro Thr Gly Leu Asp
85 90 95

Ser Ala Ser Tyr Tyr Ser Trp Asp Ser Glu Phe Tyr Arg Val Leu Arg
100 105 110

Glu Arg Val Ala Lys Lys Leu Ala Glu Pro Gly Leu Met Gln Arg Ala
115 120 125

Arg Met Glu Leu Trp Ala Lys Ala Ile Phe Leu Leu Ala Gly Phe Trp
130 135 140

Gly Ser Leu Tyr Ala Met Cys Val Leu Asp Pro His Gly Gly Ala Met
145 150 155 160

Val Ala Ala Val Thr Leu Gly Val Phe Ala Ala Phe Val Gly Thr Cys
165 170 175

Ile Gln His Asp Gly Ser His Gly Ala Phe Ser Lys Ser Arg Phe Met
180 185 190

Asn Lys Ala Ala Gly Trp Thr Leu Asp Met Ile Gly Ala Ser Ala Met
195 200 205

Thr Trp Glu Met Gln His Val Leu Gly His His Pro Tyr Thr Asn Leu
210 215 220

Ile Glu Met Glu Asn Gly Leu Ala Lys Val Lys Gly Ala Asp Val Asp
225 230 235 240

Pro Lys Lys Val Asp Gln Glu Ser Asp Pro Asp Val Phe Ser Thr Tyr
245 250 255

Pro Met Leu Arg Leu His Pro Trp His Arg Gln Arg Phe Tyr His Lys
260 265 270

Phe Gln His Leu Tyr Ala Pro Phe Ile Phe Gly Ser Met Thr Ile Asn
275 280 285

Lys Val Ile Ser Gln Asp Val Gly Val Val Leu Arg Lys Arg Leu Phe
290 295 300

Gln Ile Asp Ala Asn Cys Arg Tyr Gly Ser Pro Trp Tyr Val Ala Arg
305 310 315 320

Phe Trp Ile Met Lys Leu Leu Thr Thr Leu Tyr Met Val Ala Leu Pro
325 330 335

Met Tyr Met Gln Gly Pro Ala Gln Gly Leu Lys Leu Phe Phe Met Ala
340 345 350

His Phe Thr Cys Gly Glu Val Leu Ala Thr Met Phe Ile Val Asn His
355 360 365

Ile Ile Glu Gly Val Ser Tyr Ala Ser Lys Asp Ala Val Lys Gly Val
370 375 380

Met Ala Pro Pro Arg Thr Val His Gly Val Thr Pro Met Gln Val Thr
385 390 395 400

Gln Lys Ala Leu Ser Ala Ala Glu Ser Ala Lys Ser Asp Ala Asp Lys
405 410 415

Thr Thr Met Ile Pro Leu Asn Asp Trp Ala Ala Val Gln Cys Gln Thr
420 425 430

Ser Val Asn Trp Ala Val Gly Ser Trp Phe Trp Asn His Phe Ser Gly
435 440 445

Gly Leu Asn His Gln Ile Glu His His Cys Phe Pro Gln Asn Pro His
450 455 460

Thr Val Asn Val Tyr Ile Ser Gly Ile Val Lys Glu Thr Cys Glu Glu
465 470 475 480

Tyr Gly Val Pro Tyr Gln Ala Glu Ile Ser Leu Phe Ser Ala Tyr Phe
485 490 495

Lys Met Leu Ser His Leu Arg Thr Leu Gly Asn Glu Asp Leu Thr Ala
500 505 510

Trp Ser Thr
515

<210> 43
<211> 960
<212> DNA
<213> Thalassiosira pseudonana

<220>
<221> CDS
<222> (1)..(960)
<223> Delta-5-Elongase

<400> 43

```
atg gtg ttg tac aat gtg gcg caa gtg ctg ctc aat ggg tgg acg gtg      48
Met Val Leu Tyr Asn Val Ala Gln Val Leu Leu Asn Gly Trp Thr Val
1               5                   10                  15

tat gcg att gtg gat gcg gtg atg aat aga gac cat ccg ttt att gga      96
Tyr Ala Ile Val Asp Ala Val Met Asn Arg Asp His Pro Phe Ile Gly
            20                  25                  30

agt aga agt ttg gtt ggg gcg gcg ttg cat agt ggg agc tcg tat gcg      144
Ser Arg Ser Leu Val Gly Ala Ala Leu His Ser Gly Ser Ser Tyr Ala
        35                  40                  45

gtg tgg gtt cat tat tgt gat aag tat ttg gag ttc ttt gat acg tat      192
Val Trp Val His Tyr Cys Asp Lys Tyr Leu Glu Phe Phe Asp Thr Tyr
    50                  55                  60

ttt atg gtg ttg agg ggg aaa atg gac cag atg gta ctt ggt gaa gtt      240
Phe Met Val Leu Arg Gly Lys Met Asp Gln Met Val Leu Gly Glu Val
65                  70                  75                  80

ggt ggc agt gtg tgg tgt ggc gtt gga tat atg gat atg gag aag atg      288
Gly Gly Ser Val Trp Cys Gly Val Gly Tyr Met Asp Met Glu Lys Met
                85                  90                  95

ata cta ctc agc ttt gga gtg cat cgg tct gct cag gga acg ggg aag      336
Ile Leu Leu Ser Phe Gly Val His Arg Ser Ala Gln Gly Thr Gly Lys
            100                 105                 110

gct ttc acc aac aac gtt acc aat cca cat ctc acg ctt cca cct cat      384
Ala Phe Thr Asn Asn Val Thr Asn Pro His Leu Thr Leu Pro Pro His
        115                 120                 125

tct aca aaa aca aaa aaa cag gtc tcc ttc ctc cac atc tac cac cac      432
Ser Thr Lys Thr Lys Lys Gln Val Ser Phe Leu His Ile Tyr His His
    130                 135                 140

acg acc ata gcg tgg gca tgg tgg atc gcc ctc cgc ttc tcc ccc ggt      480
Thr Thr Ile Ala Trp Ala Trp Trp Ile Ala Leu Arg Phe Ser Pro Gly
145                 150                 155                 160

gga gac att tac ttc ggg gca ctc ctc aac tcc atc atc cac gtc ctc      528
Gly Asp Ile Tyr Phe Gly Ala Leu Leu Asn Ser Ile Ile His Val Leu
                165                 170                 175

atg tat tcc tac tac gcc ctt gcc cta ctc aag gtc agt tgt cca tgg      576
Met Tyr Ser Tyr Tyr Ala Leu Ala Leu Leu Lys Val Ser Cys Pro Trp
            180                 185                 190

aaa cga tac ctg act caa gct caa tta ttg caa ttc aca agt gtg gtg      624
Lys Arg Tyr Leu Thr Gln Ala Gln Leu Leu Gln Phe Thr Ser Val Val
        195                 200                 205

gtt tat acg ggg tgt acg ggt tat act cat tac tat cat acg aag cat      672
Val Tyr Thr Gly Cys Thr Gly Tyr Thr His Tyr Tyr His Thr Lys His
    210                 215                 220

gga gcg gat gag aca cag cct agt tta gga acg tat tat ttc tgt tgt      720
Gly Ala Asp Glu Thr Gln Pro Ser Leu Gly Thr Tyr Tyr Phe Cys Cys
225                 230                 235                 240

gga gtg cag gtg ttt gag atg gtt agt ttg ttt gta ctc ttt tcc atc      768
Gly Val Gln Val Phe Glu Met Val Ser Leu Phe Val Leu Phe Ser Ile
                245                 250                 255

ttt tat aaa cga tcc tat tcg aag aag aac aag tca gga gga aag gat      816
```

```
Phe Tyr Lys Arg Ser Tyr Ser Lys Lys Asn Lys Ser Gly Gly Lys Asp
            260                 265                 270

agc aag aag aat gat gat ggg aat aat gag gat caa tgt cac aag gct      864
Ser Lys Lys Asn Asp Asp Gly Asn Asn Glu Asp Gln Cys His Lys Ala
        275                 280                 285

atg aag gat ata tcg gag ggt gcg aag gag gtt gtg ggg cat gca gcg      912
Met Lys Asp Ile Ser Glu Gly Ala Lys Glu Val Val Gly His Ala Ala
    290                 295                 300

aag gat gct gga aag ttg gtg gct acg aga gta agg tgt aag gtg taa      960
Lys Asp Ala Gly Lys Leu Val Ala Thr Arg Val Arg Cys Lys Val
305                 310                 315
```

<210> 44
<211> 319
<212> PRT
<213> Thalassiosira pseudonana

<400> 44

```
Met Val Leu Tyr Asn Val Ala Gln Val Leu Leu Asn Gly Trp Thr Val
1               5                   10                  15

Tyr Ala Ile Val Asp Ala Val Met Asn Arg Asp His Pro Phe Ile Gly
            20                  25                  30

Ser Arg Ser Leu Val Gly Ala Ala Leu His Ser Gly Ser Ser Tyr Ala
        35                  40                  45

Val Trp Val His Tyr Cys Asp Lys Tyr Leu Glu Phe Phe Asp Thr Tyr
    50                  55                  60

Phe Met Val Leu Arg Gly Lys Met Asp Gln Met Val Leu Gly Glu Val
65                  70                  75                  80

Gly Gly Ser Val Trp Cys Gly Val Gly Tyr Met Asp Met Glu Lys Met
                85                  90                  95

Ile Leu Leu Ser Phe Gly Val His Arg Ser Ala Gln Gly Thr Gly Lys
            100                 105                 110

Ala Phe Thr Asn Asn Val Thr Asn Pro His Leu Thr Leu Pro Pro His
        115                 120                 125

Ser Thr Lys Thr Lys Lys Gln Val Ser Phe Leu His Ile Tyr His His
    130                 135                 140

Thr Thr Ile Ala Trp Ala Trp Trp Ile Ala Leu Arg Phe Ser Pro Gly
145                 150                 155                 160
```

```
Gly Asp Ile Tyr Phe Gly Ala Leu Leu Asn Ser Ile Ile His Val Leu
                165                 170                 175

Met Tyr Ser Tyr Tyr Ala Leu Ala Leu Leu Lys Val Ser Cys Pro Trp
            180                 185                 190

Lys Arg Tyr Leu Thr Gln Ala Gln Leu Leu Gln Phe Thr Ser Val Val
        195                 200                 205

Val Tyr Thr Gly Cys Thr Gly Tyr Thr His Tyr Tyr His Thr Lys His
    210                 215                 220

Gly Ala Asp Glu Thr Gln Pro Ser Leu Gly Thr Tyr Tyr Phe Cys Cys
225                 230                 235                 240

Gly Val Gln Val Phe Glu Met Val Ser Leu Phe Val Leu Phe Ser Ile
                245                 250                 255

Phe Tyr Lys Arg Ser Tyr Ser Lys Lys Asn Lys Ser Gly Gly Lys Asp
            260                 265                 270

Ser Lys Lys Asn Asp Asp Gly Asn Asn Glu Asp Gln Cys His Lys Ala
        275                 280                 285

Met Lys Asp Ile Ser Glu Gly Ala Lys Glu Val Val Gly His Ala Ala
    290                 295                 300

Lys Asp Ala Gly Lys Leu Val Ala Thr Arg Val Arg Cys Lys Val
305                 310                 315
```

<210> 45
<211> 819
<212> DNA
<213> Thalassiosira pseudonana

<220>
<221> CDS
<222> (1)..(819)
<223> Delta-5-Elongase

<400> 45

```
atg gac gcc tac aac gct gca atg gat aag atc ggt gcc gcc atc atc      48
Met Asp Ala Tyr Asn Ala Ala Met Asp Lys Ile Gly Ala Ala Ile Ile
1               5                   10                  15

gat tgg tct gat ccc gat gga aag ttc cgt gcc gat aga gag gac tgg      96
Asp Trp Ser Asp Pro Asp Gly Lys Phe Arg Ala Asp Arg Glu Asp Trp
            20                  25                  30
```

```
tgg ctc tgc gac ttc cgt agc gcc atc acc atc gcc ctc atc tac atc      144
Trp Leu Cys Asp Phe Arg Ser Ala Ile Thr Ile Ala Leu Ile Tyr Ile
        35                  40                  45

gcc ttc gtc atc ctc ggt tcc gcc gtc atg caa tcc ctc ccc gca atg      192
Ala Phe Val Ile Leu Gly Ser Ala Val Met Gln Ser Leu Pro Ala Met
    50                  55                  60

gat ccc tac ccc atc aaa ttc ctc tac aac gtc tcc caa atc ttc ctt      240
Asp Pro Tyr Pro Ile Lys Phe Leu Tyr Asn Val Ser Gln Ile Phe Leu
65                  70                  75                  80

tgt gcc tac atg act gtc gag gcg gga ttt ttg gcc tac cgc aat gga      288
Cys Ala Tyr Met Thr Val Glu Ala Gly Phe Leu Ala Tyr Arg Asn Gly
                85                  90                  95

tat acc gtc atg cct tgc aat cat ttc aat gtg aat gat cct ccc gtg      336
Tyr Thr Val Met Pro Cys Asn His Phe Asn Val Asn Asp Pro Pro Val
            100                 105                 110

gcg aat ctt ctt tgg ttg ttt tat att tcc aag gtg tgg gac ttt tgg      384
Ala Asn Leu Leu Trp Leu Phe Tyr Ile Ser Lys Val Trp Asp Phe Trp
        115                 120                 125

gat acc att ttc att gtg ttg ggg aag aag tgg cgt caa tta tct ttc      432
Asp Thr Ile Phe Ile Val Leu Gly Lys Lys Trp Arg Gln Leu Ser Phe
    130                 135                 140

ttg cat gta tac cat cac acc acc atc ttt cta ttc tat tgg ctg aat      480
Leu His Val Tyr His His Thr Thr Ile Phe Leu Phe Tyr Trp Leu Asn
145                 150                 155                 160

gcc aat gtc ttg tac gat ggt gac atc ttc ctt acc atc ttg ctc aat      528
Ala Asn Val Leu Tyr Asp Gly Asp Ile Phe Leu Thr Ile Leu Leu Asn
                165                 170                 175

gga ttc atc cac acg gtg atg tac acg tat tac ttc atc tgt atg cat      576
Gly Phe Ile His Thr Val Met Tyr Thr Tyr Tyr Phe Ile Cys Met His
            180                 185                 190

acc aaa gat tcc aag acg ggc aag agt ctt cct ata tgg tgg aag tcg      624
Thr Lys Asp Ser Lys Thr Gly Lys Ser Leu Pro Ile Trp Trp Lys Ser
        195                 200                 205

agt ttg acg gcg ttt cag ttg ttg caa ttc act atc atg atg agt cag      672
Ser Leu Thr Ala Phe Gln Leu Leu Gln Phe Thr Ile Met Met Ser Gln
    210                 215                 220

gct acc tac ctt gtc ttc cac ggg tgt gat aag gtg tcg ctt cgt atc      720
Ala Thr Tyr Leu Val Phe His Gly Cys Asp Lys Val Ser Leu Arg Ile
225                 230                 235                 240

acg att gtg tac ttt gtg tcc ctt ttg agt ttg ttc ttc ctt ttt gct      768
Thr Ile Val Tyr Phe Val Ser Leu Leu Ser Leu Phe Phe Leu Phe Ala
                245                 250                 255

cag ttc ttt gtg caa tca tac atg gca ccc aaa aag aag aag agt gct      816
Gln Phe Phe Val Gln Ser Tyr Met Ala Pro Lys Lys Lys Lys Ser Ala
            260                 265                 270

tag                                                                  819
```

<210> 46
<211> 272
<212> PRT
<213> Thalassiosira pseudonana

<400> 46

Met Asp Ala Tyr Asn Ala Ala Met Asp Lys Ile Gly Ala Ala Ile Ile
1               5                   10                  15

Asp Trp Ser Asp Pro Asp Gly Lys Phe Arg Ala Asp Arg Glu Asp Trp
            20                  25                  30

Trp Leu Cys Asp Phe Arg Ser Ala Ile Thr Ile Ala Leu Ile Tyr Ile
        35                  40                  45

Ala Phe Val Ile Leu Gly Ser Ala Val Met Gln Ser Leu Pro Ala Met
    50                  55                  60

Asp Pro Tyr Pro Ile Lys Phe Leu Tyr Asn Val Ser Gln Ile Phe Leu
65                  70                  75                  80

Cys Ala Tyr Met Thr Val Glu Ala Gly Phe Leu Ala Tyr Arg Asn Gly
                85                  90                  95

Tyr Thr Val Met Pro Cys Asn His Phe Asn Val Asn Asp Pro Pro Val
            100                 105                 110

Ala Asn Leu Leu Trp Leu Phe Tyr Ile Ser Lys Val Trp Asp Phe Trp
        115                 120                 125

Asp Thr Ile Phe Ile Val Leu Gly Lys Lys Trp Arg Gln Leu Ser Phe
    130                 135                 140

Leu His Val Tyr His His Thr Thr Ile Phe Leu Phe Tyr Trp Leu Asn
145                 150                 155                 160

Ala Asn Val Leu Tyr Asp Gly Asp Ile Phe Leu Thr Ile Leu Leu Asn
                165                 170                 175

Gly Phe Ile His Thr Val Met Tyr Thr Tyr Tyr Phe Ile Cys Met His
            180                 185                 190

Thr Lys Asp Ser Lys Thr Gly Lys Ser Leu Pro Ile Trp Trp Lys Ser
        195                 200                 205

Ser Leu Thr Ala Phe Gln Leu Leu Gln Phe Thr Ile Met Met Ser Gln
    210                 215                 220

Ala Thr Tyr Leu Val Phe His Gly Cys Asp Lys Val Ser Leu Arg Ile
225                 230                 235                 240

```
Thr Ile Val Tyr Phe Val Ser Leu Leu Ser Leu Phe Phe Leu Phe Ala
                245                 250                 255

Gln Phe Phe Val Gln Ser Tyr Met Ala Pro Lys Lys Lys Lys Ser Ala
            260                 265                 270
```

<210> 47
<211> 936
<212> DNA
<213> Crypthecodinium cohnii

<220>
<221> CDS
<222> (1)..(936)
<223> Delta-5-Elongase

<400> 47

```
atg tct gcc ttc atg act ctc cca cag gct ctc tcc gat gtg acc tcg       48
Met Ser Ala Phe Met Thr Leu Pro Gln Ala Leu Ser Asp Val Thr Ser
1               5                   10                  15

gcc ttg gtc acg ctg gga aag gat gtc tcc agc cct tca gct ttt caa       96
Ala Leu Val Thr Leu Gly Lys Asp Val Ser Ser Pro Ser Ala Phe Gln
            20                  25                  30

gct gtc act ggc ttc tgc agg gag cag tgg ggg att ccg aca gta ttc      144
Ala Val Thr Gly Phe Cys Arg Glu Gln Trp Gly Ile Pro Thr Val Phe
        35                  40                  45

tgc ctg ggc tac ttg gcc atg gtc tac gcg gcc aga aga ccc ctc ccg      192
Cys Leu Gly Tyr Leu Ala Met Val Tyr Ala Ala Arg Arg Pro Leu Pro
    50                  55                  60

cag cac ggc tac atg gtt gcg gtg gac cgt tgc ttc gct gct tgg aac      240
Gln His Gly Tyr Met Val Ala Val Asp Arg Cys Phe Ala Ala Trp Asn
65                  70                  75                  80

ttg gct ctc tct gtc ttc agc act tgg ggc ttc tac cac atg gct gtc      288
Leu Ala Leu Ser Val Phe Ser Thr Trp Gly Phe Tyr His Met Ala Val
                85                  90                  95

ggg ctc tac aac atg aca gag acg agg ggc ttg caa ttc acc atc tgc      336
Gly Leu Tyr Asn Met Thr Glu Thr Arg Gly Leu Gln Phe Thr Ile Cys
            100                 105                 110

ggt tcg act ggg gag ctc gtg cag aac ctt cag act ggc cca acc gct      384
Gly Ser Thr Gly Glu Leu Val Gln Asn Leu Gln Thr Gly Pro Thr Ala
        115                 120                 125

ctg gcg ctc tgc ctc ttc tgc ttc agc aag atc ccc gag ttg atg gac      432
Leu Ala Leu Cys Leu Phe Cys Phe Ser Lys Ile Pro Glu Leu Met Asp
    130                 135                 140

acg gtg ttt ctc atc ctg aag gcc aag aag gtc cgc ttc ttg cag tgg      480
Thr Val Phe Leu Ile Leu Lys Ala Lys Lys Val Arg Phe Leu Gln Trp
145                 150                 155                 160
```

```
tac cac cat gcc aca gtc atg ctc ttc tgt tgg ctc gcc ctc gcg acg      528
Tyr His His Ala Thr Val Met Leu Phe Cys Trp Leu Ala Leu Ala Thr
                165                 170                 175

gag tac act cct ggc ttg tgg ttt gcg gcg acg aac tac ttc gtg cac      576
Glu Tyr Thr Pro Gly Leu Trp Phe Ala Ala Thr Asn Tyr Phe Val His
            180                 185                 190

tcc atc atg tac atg tac ttc ttc ctc atg acc ttc aag tcg gcc gcg      624
Ser Ile Met Tyr Met Tyr Phe Phe Leu Met Thr Phe Lys Ser Ala Ala
        195                 200                 205

aag gtg gtg aag ccc atc gcc cct ctc atc aca gtt atc cag att gct      672
Lys Val Val Lys Pro Ile Ala Pro Leu Ile Thr Val Ile Gln Ile Ala
    210                 215                 220

cag atg gtc tgg ggc ctc atc gtc aac ggc atc gcc atc acc acc ttc      720
Gln Met Val Trp Gly Leu Ile Val Asn Gly Ile Ala Ile Thr Thr Phe
225                 230                 235                 240

ttc acg act ggt gcc tgc cag atc cag tct gtg act gtg tat tcg gcc      768
Phe Thr Thr Gly Ala Cys Gln Ile Gln Ser Val Thr Val Tyr Ser Ala
                245                 250                 255

atc atc atg tac gct tcg tac ttc tac ctg ttc tcc cag ctc ttc ttc      816
Ile Ile Met Tyr Ala Ser Tyr Phe Tyr Leu Phe Ser Gln Leu Phe Phe
            260                 265                 270

gag gcc cat ggt gcc gct ggc aag aac aag aag aag ttg acc cgc gag      864
Glu Ala His Gly Ala Ala Gly Lys Asn Lys Lys Lys Leu Thr Arg Glu
        275                 280                 285

ctc tct cga aaa atc tcg gag gct ctc ctg aac acc ggt gac gag gtt      912
Leu Ser Arg Lys Ile Ser Glu Ala Leu Leu Asn Thr Gly Asp Glu Val
    290                 295                 300

tcc aag cac ctg aag gtg aat tga                                      936
Ser Lys His Leu Lys Val Asn
305                 310
```

<210> 48
<211> 311
<212> PRT
<213> Crypthecodinium cohnii

<400> 48

```
Met Ser Ala Phe Met Thr Leu Pro Gln Ala Leu Ser Asp Val Thr Ser
1               5                   10                  15

Ala Leu Val Thr Leu Gly Lys Asp Val Ser Ser Pro Ser Ala Phe Gln
            20                  25                  30

Ala Val Thr Gly Phe Cys Arg Glu Gln Trp Gly Ile Pro Thr Val Phe
        35                  40                  45

Cys Leu Gly Tyr Leu Ala Met Val Tyr Ala Ala Arg Arg Pro Leu Pro
    50                  55                  60
```

Gln His Gly Tyr Met Val Ala Val Asp Arg Cys Phe Ala Ala Trp Asn
65 70 75 80

Leu Ala Leu Ser Val Phe Ser Thr Trp Gly Phe Tyr His Met Ala Val
85 90 95

Gly Leu Tyr Asn Met Thr Glu Thr Arg Gly Leu Gln Phe Thr Ile Cys
100 105 110

Gly Ser Thr Gly Glu Leu Val Gln Asn Leu Gln Thr Gly Pro Thr Ala
115 120 125

Leu Ala Leu Cys Leu Phe Cys Phe Ser Lys Ile Pro Glu Leu Met Asp
130 135 140

Thr Val Phe Leu Ile Leu Lys Ala Lys Lys Val Arg Phe Leu Gln Trp
145 150 155 160

Tyr His His Ala Thr Val Met Leu Phe Cys Trp Leu Ala Leu Ala Thr
165 170 175

Glu Tyr Thr Pro Gly Leu Trp Phe Ala Ala Thr Asn Tyr Phe Val His
180 185 190

Ser Ile Met Tyr Met Tyr Phe Phe Leu Met Thr Phe Lys Ser Ala Ala
195 200 205

Lys Val Val Lys Pro Ile Ala Pro Leu Ile Thr Val Ile Gln Ile Ala
210 215 220

Gln Met Val Trp Gly Leu Ile Val Asn Gly Ile Ala Ile Thr Thr Phe
225 230 235 240

Phe Thr Thr Gly Ala Cys Gln Ile Gln Ser Val Thr Val Tyr Ser Ala
245 250 255

Ile Ile Met Tyr Ala Ser Tyr Phe Tyr Leu Phe Ser Gln Leu Phe Phe
260 265 270

Glu Ala His Gly Ala Ala Gly Lys Asn Lys Lys Lys Leu Thr Arg Glu
275 280 285

Leu Ser Arg Lys Ile Ser Glu Ala Leu Leu Asn Thr Gly Asp Glu Val
290 295 300

Ser Lys His Leu Lys Val Asn
305 310

<210> 49
<211> 927
<212> DNA
<213> Crypthecodinium cohnii

<220>
<221> CDS
<222> (1)..(927)
<223> Delta-5-Elongase

<400> 49

```
atg gct tcc tac caa caa gca ttc tcc gaa ttg gct aga gct ttg tcc      48
Met Ala Ser Tyr Gln Gln Ala Phe Ser Glu Leu Ala Arg Ala Leu Ser
1               5                   10                  15

act ttg aac cac gac ttc tcc agc gtc gag cca ttc aaa gtc gtg acg      96
Thr Leu Asn His Asp Phe Ser Ser Val Glu Pro Phe Lys Val Val Thr
            20                  25                  30

cag ttc tgc agg gac cag tgg gcg atc ccg aca gtc ttt tgc atc ggt      144
Gln Phe Cys Arg Asp Gln Trp Ala Ile Pro Thr Val Phe Cys Ile Gly
        35                  40                  45

tac ttg gca atg gtc tac gcc acg cga aga cct atc gcg aag cac ccc      192
Tyr Leu Ala Met Val Tyr Ala Thr Arg Arg Pro Ile Ala Lys His Pro
    50                  55                  60

tac atg tct ctc gtg gat cgc tgc ttt gcg gcc tgg aac ttg ggc ctc      240
Tyr Met Ser Leu Val Asp Arg Cys Phe Ala Ala Trp Asn Leu Gly Leu
65                  70                  75                  80

tcg ctc ttc agt tgc tgg ggc ttc tac cac atg gca gtg gga ctc tcc      288
Ser Leu Phe Ser Cys Trp Gly Phe Tyr His Met Ala Val Gly Leu Ser
                85                  90                  95

cac acc act tgg aat ttc ggg ctc cag ttc acc atc tgc ggc agc acc      336
His Thr Thr Trp Asn Phe Gly Leu Gln Phe Thr Ile Cys Gly Ser Thr
            100                 105                 110

acg gag ctt gtg aat ggc ttc cag aag ggc ccg gcg gcc ctc gcc ctc      384
Thr Glu Leu Val Asn Gly Phe Gln Lys Gly Pro Ala Ala Leu Ala Leu
        115                 120                 125

atc ctg ttc tgc ttc tcc aag atc ccg gag ttg ggc gac acc gtc ttc      432
Ile Leu Phe Cys Phe Ser Lys Ile Pro Glu Leu Gly Asp Thr Val Phe
    130                 135                 140

ttg atc ttg aag gga aag aag gtc cgc ttc ttg cag tgg tac cac cac      480
Leu Ile Leu Lys Gly Lys Lys Val Arg Phe Leu Gln Trp Tyr His His
145                 150                 155                 160

acg acc gtg atg ctc ttc tgt tgg atg gcc ttg gcg act gag tac act      528
Thr Thr Val Met Leu Phe Cys Trp Met Ala Leu Ala Thr Glu Tyr Thr
                165                 170                 175

cct gga ttg tgg ttc gcg gcc acg aac tac ttc gtg cac tcc atc atg      576
Pro Gly Leu Trp Phe Ala Ala Thr Asn Tyr Phe Val His Ser Ile Met
            180                 185                 190

tac atg tac ttc ttc ctc atg acc ttc aag acg gcc gcc ggc atc atc      624
```

```
Tyr Met Tyr Phe Phe Leu Met Thr Phe Lys Thr Ala Ala Gly Ile Ile
        195                 200                 205

aag ccc atc gcg cct ctc atc acc atc atc cag atc tcc cag atg gtc      672
Lys Pro Ile Ala Pro Leu Ile Thr Ile Ile Gln Ile Ser Gln Met Val
    210                 215                 220

tgg ggc ttg gtc gtg aac gcc atc gcc gtc ggc acc ttc ttc acc aca      720
Trp Gly Leu Val Val Asn Ala Ile Ala Val Gly Thr Phe Phe Thr Thr
225                 230                 235                 240

ggc aac tgc cag atc cag gca gtg aca gtc tac tcc gcc atc gtg atg      768
Gly Asn Cys Gln Ile Gln Ala Val Thr Val Tyr Ser Ala Ile Val Met
                245                 250                 255

tac gcc tcc tac ttc tac ctc ttc ggc cag ctc ttc ttc gag gcc cag      816
Tyr Ala Ser Tyr Phe Tyr Leu Phe Gly Gln Leu Phe Phe Glu Ala Gln
            260                 265                 270

ggt tcg gct gga aag gac aag aag aag ttg gcc cga gag ctg agc cga      864
Gly Ser Ala Gly Lys Asp Lys Lys Lys Leu Ala Arg Glu Leu Ser Arg
        275                 280                 285

aag gtc tcg cgg gct ctc aca gca acg ggc gaa gag gtg tcg aag cac      912
Lys Val Ser Arg Ala Leu Thr Ala Thr Gly Glu Glu Val Ser Lys His
    290                 295                 300

atg aag gtg aat tga                                                  927
Met Lys Val Asn
305
```

<210> 50
<211> 308
<212> PRT
<213> Crypthecodinium cohnii

<400> 50

```
Met Ala Ser Tyr Gln Gln Ala Phe Ser Glu Leu Ala Arg Ala Leu Ser
1               5                   10                  15

Thr Leu Asn His Asp Phe Ser Ser Val Glu Pro Phe Lys Val Val Thr
            20                  25                  30

Gln Phe Cys Arg Asp Gln Trp Ala Ile Pro Thr Val Phe Cys Ile Gly
        35                  40                  45

Tyr Leu Ala Met Val Tyr Ala Thr Arg Arg Pro Ile Ala Lys His Pro
    50                  55                  60

Tyr Met Ser Leu Val Asp Arg Cys Phe Ala Ala Trp Asn Leu Gly Leu
65                  70                  75                  80

Ser Leu Phe Ser Cys Trp Gly Phe Tyr His Met Ala Val Gly Leu Ser
                85                  90                  95
```

His Thr Thr Trp Asn Phe Gly Leu Gln Phe Thr Ile Cys Gly Ser Thr
100 105 110

Thr Glu Leu Val Asn Gly Phe Gln Lys Gly Pro Ala Ala Leu Ala Leu
115 120 125

Ile Leu Phe Cys Phe Ser Lys Ile Pro Glu Leu Gly Asp Thr Val Phe
130 135 140

Leu Ile Leu Lys Gly Lys Lys Val Arg Phe Leu Gln Trp Tyr His His
145 150 155 160

Thr Thr Val Met Leu Phe Cys Trp Met Ala Leu Ala Thr Glu Tyr Thr
165 170 175

Pro Gly Leu Trp Phe Ala Ala Thr Asn Tyr Phe Val His Ser Ile Met
180 185 190

Tyr Met Tyr Phe Phe Leu Met Thr Phe Lys Thr Ala Ala Gly Ile Ile
195 200 205

Lys Pro Ile Ala Pro Leu Ile Thr Ile Ile Gln Ile Ser Gln Met Val
210 215 220

Trp Gly Leu Val Val Asn Ala Ile Ala Val Gly Thr Phe Phe Thr Thr
225 230 235 240

Gly Asn Cys Gln Ile Gln Ala Val Thr Val Tyr Ser Ala Ile Val Met
245 250 255

Tyr Ala Ser Tyr Phe Tyr Leu Phe Gly Gln Leu Phe Phe Glu Ala Gln
260 265 270

Gly Ser Ala Gly Lys Asp Lys Lys Lys Leu Ala Arg Glu Leu Ser Arg
275 280 285

Lys Val Ser Arg Ala Leu Thr Ala Thr Gly Glu Glu Val Ser Lys His
290 295 300

Met Lys Val Asn
305

<210> 51
<211> 795
<212> DNA
<213> Oncorhynchus mykiss

<220>
<221> CDS
<222> (1)..(795)
<223> Delta-5-Elongase

<400> 51

```
atg gct tca aca tgg caa agc gtt cag tcc atg cgc cag tgg att tta      48
Met Ala Ser Thr Trp Gln Ser Val Gln Ser Met Arg Gln Trp Ile Leu
1               5                   10                  15

gag aat gga gat aaa agg aca gac cca tgg cta ctg gtc tac tcc cct      96
Glu Asn Gly Asp Lys Arg Thr Asp Pro Trp Leu Leu Val Tyr Ser Pro
            20                  25                  30

atg cca gtg gcc att ata ttc ctc ctc tat ctt ggt gtg gtc tgg gct      144
Met Pro Val Ala Ile Ile Phe Leu Leu Tyr Leu Gly Val Val Trp Ala
        35                  40                  45

ggg ccc aag ctg atg aaa cgc agg gaa cca gtt gat ctc aag gct gta      192
Gly Pro Lys Leu Met Lys Arg Arg Glu Pro Val Asp Leu Lys Ala Val
    50                  55                  60

ctc att gtc tac aac ttc gcc atg gtc tgc ctg tct gtc tac atg ttc      240
Leu Ile Val Tyr Asn Phe Ala Met Val Cys Leu Ser Val Tyr Met Phe
65                  70                  75                  80

cat gag ttc ttg gtc acg tcc ttg ctg tct aac tac agt tac ctg tgt      288
His Glu Phe Leu Val Thr Ser Leu Leu Ser Asn Tyr Ser Tyr Leu Cys
                85                  90                  95

caa cct gtg gat tac agc act agt cca ctg gcg atg agg atg gcc aaa      336
Gln Pro Val Asp Tyr Ser Thr Ser Pro Leu Ala Met Arg Met Ala Lys
            100                 105                 110

gta tgc tgg tgg ttt ttc ttc tcc aag gtc ata gaa ttg gct gac acg      384
Val Cys Trp Trp Phe Phe Phe Ser Lys Val Ile Glu Leu Ala Asp Thr
        115                 120                 125

gtg ttc ttc atc ctg agg aag aag aac agt cag ctg act ttc ctg cat      432
Val Phe Phe Ile Leu Arg Lys Lys Asn Ser Gln Leu Thr Phe Leu His
    130                 135                 140

gtc tat cac cat ggc acc atg atc ttc aac tgg tgg gca ggg gtc aag      480
Val Tyr His His Gly Thr Met Ile Phe Asn Trp Trp Ala Gly Val Lys
145                 150                 155                 160

tat ctg gct gga ggc caa tcg ttc ttc atc ggc ctg ctc aat acc ttt      528
Tyr Leu Ala Gly Gly Gln Ser Phe Phe Ile Gly Leu Leu Asn Thr Phe
                165                 170                 175

gtg cac atc gtg atg tac tct tac tac gga ctg gct gcc ctg ggg cct      576
Val His Ile Val Met Tyr Ser Tyr Tyr Gly Leu Ala Ala Leu Gly Pro
            180                 185                 190

cac acg cag aag tac tta tgg tgg aag cgc tat ctg acc tca ctg cag      624
His Thr Gln Lys Tyr Leu Trp Trp Lys Arg Tyr Leu Thr Ser Leu Gln
        195                 200                 205

ctg ctc cag ttt gtc ctg ttg acc act cac act ggc tac aac ctc ttc      672
Leu Leu Gln Phe Val Leu Leu Thr Thr His Thr Gly Tyr Asn Leu Phe
    210                 215                 220

act gag tgt gac ttc ccg gac tcc atg aac gct gtg gtg ttt gcc tac      720
Thr Glu Cys Asp Phe Pro Asp Ser Met Asn Ala Val Val Phe Ala Tyr
225                 230                 235                 240

tgt gtc agt ctc att gct ctc ttc agc aac ttc tac tat cag agc tac      768
Cys Val Ser Leu Ile Ala Leu Phe Ser Asn Phe Tyr Tyr Gln Ser Tyr
                245                 250                 255

ctc aac agg aag agc aag aag aca taa                                  795
Leu Asn Arg Lys Ser Lys Lys Thr
            260
```

<210> 52
<211> 264
<212> PRT
<213> Oncorhynchus mykiss

<400> 52

```
Met Ala Ser Thr Trp Gln Ser Val Gln Ser Met Arg Gln Trp Ile Leu
1               5                   10                  15

Glu Asn Gly Asp Lys Arg Thr Asp Pro Trp Leu Leu Val Tyr Ser Pro
            20                  25                  30

Met Pro Val Ala Ile Ile Phe Leu Leu Tyr Leu Gly Val Val Trp Ala
        35                  40                  45

Gly Pro Lys Leu Met Lys Arg Arg Glu Pro Val Asp Leu Lys Ala Val
    50                  55                  60

Leu Ile Val Tyr Asn Phe Ala Met Val Cys Leu Ser Val Tyr Met Phe
65                  70                  75                  80

His Glu Phe Leu Val Thr Ser Leu Leu Ser Asn Tyr Ser Tyr Leu Cys
                85                  90                  95

Gln Pro Val Asp Tyr Ser Thr Ser Pro Leu Ala Met Arg Met Ala Lys
            100                 105                 110

Val Cys Trp Trp Phe Phe Phe Ser Lys Val Ile Glu Leu Ala Asp Thr
        115                 120                 125

Val Phe Phe Ile Leu Arg Lys Lys Asn Ser Gln Leu Thr Phe Leu His
    130                 135                 140

Val Tyr His His Gly Thr Met Ile Phe Asn Trp Trp Ala Gly Val Lys
145                 150                 155                 160

Tyr Leu Ala Gly Gly Gln Ser Phe Phe Ile Gly Leu Leu Asn Thr Phe
                165                 170                 175

Val His Ile Val Met Tyr Ser Tyr Tyr Gly Leu Ala Ala Leu Gly Pro
            180                 185                 190
```

```
His Thr Gln Lys Tyr Leu Trp Trp Lys Arg Tyr Leu Thr Ser Leu Gln
        195                 200                 205

Leu Leu Gln Phe Val Leu Leu Thr Thr His Thr Gly Tyr Asn Leu Phe
    210                 215                 220

Thr Glu Cys Asp Phe Pro Asp Ser Met Asn Ala Val Val Phe Ala Tyr
225                 230                 235                 240

Cys Val Ser Leu Ile Ala Leu Phe Ser Asn Phe Tyr Tyr Gln Ser Tyr
                245                 250                 255

Leu Asn Arg Lys Ser Lys Lys Thr
            260
```

<210> 53
<211> 885
<212> DNA
<213> Oncorhynchus mykiss

<220>
<221> CDS
<222> (1)..(885)
<223> Delta-5-Elongase

<400> 53

```
atg gag act ttt aat tat aaa cta aac atg tac ata gac tca tgg atg      48
Met Glu Thr Phe Asn Tyr Lys Leu Asn Met Tyr Ile Asp Ser Trp Met
1               5                   10                  15

ggt ccc aga gat gag cgg gta cag gga tgg ctg ctt ctg gac aac tac      96
Gly Pro Arg Asp Glu Arg Val Gln Gly Trp Leu Leu Leu Asp Asn Tyr
            20                  25                  30

cct cca acc ttt gca cta aca gtc atg tac ctg ctg atc gta tgg atg     144
Pro Pro Thr Phe Ala Leu Thr Val Met Tyr Leu Leu Ile Val Trp Met
        35                  40                  45

ggg ccc aag tac atg aga cac aga cag ccg gtg tct tgc cgg ggt ctc     192
Gly Pro Lys Tyr Met Arg His Arg Gln Pro Val Ser Cys Arg Gly Leu
    50                  55                  60

ctc ttg gtc tac aat ctg ggc ctc acg atc ttg tcc ttc tat atg ttc     240
Leu Leu Val Tyr Asn Leu Gly Leu Thr Ile Leu Ser Phe Tyr Met Phe
65                  70                  75                  80

tat gag atg gtg tct gct gtg tgg cac ggg gat tat aac ttc ttt tgc     288
Tyr Glu Met Val Ser Ala Val Trp His Gly Asp Tyr Asn Phe Phe Cys
                85                  90                  95

caa gac aca cac agt gca gga gaa acc gat acc aag atc ata aat gtg     336
```

```
Gln Asp Thr His Ser Ala Gly Glu Thr Asp Thr Lys Ile Ile Asn Val
            100                 105                 110

ctg tgg tgg tac tac ttc tcc aag ctc ata gag ttt atg gat acc ttc      384
Leu Trp Trp Tyr Tyr Phe Ser Lys Leu Ile Glu Phe Met Asp Thr Phe
        115                 120                 125

ttc ttc atc ctg cgg aag aac aac cat caa atc acg ttt ctg cac atc      432
Phe Phe Ile Leu Arg Lys Asn Asn His Gln Ile Thr Phe Leu His Ile
    130                 135                 140

tac cac cat gct agc atg ctc aac atc tgg tgg ttc gtc atg aac tgg      480
Tyr His His Ala Ser Met Leu Asn Ile Trp Trp Phe Val Met Asn Trp
145                 150                 155                 160

gtg ccc tgt ggt cac tcc tac ttt ggt gcc tcc ctg aac agc ttc atc      528
Val Pro Cys Gly His Ser Tyr Phe Gly Ala Ser Leu Asn Ser Phe Ile
                165                 170                 175

cat gtc ctg atg tac tct tac tat ggg ctc tct gct gtc ccg gcc ttg      576
His Val Leu Met Tyr Ser Tyr Tyr Gly Leu Ser Ala Val Pro Ala Leu
            180                 185                 190

cgg ccc tat cta tgg tgg aag aaa tac atc aca caa gta cag ctg att      624
Arg Pro Tyr Leu Trp Trp Lys Lys Tyr Ile Thr Gln Val Gln Leu Ile
        195                 200                 205

cag ttc ttt ttg acc atg tcc cag acg ata tgt gca gtc att tgg cca      672
Gln Phe Phe Leu Thr Met Ser Gln Thr Ile Cys Ala Val Ile Trp Pro
    210                 215                 220

tgt gat ttc ccc aga ggg tgg ctg tat ttc cag ata ttc tat gtc atc      720
Cys Asp Phe Pro Arg Gly Trp Leu Tyr Phe Gln Ile Phe Tyr Val Ile
225                 230                 235                 240

aca ctt att gcc ctt ttc tca aac ttc tac att cag act tac aag aaa      768
Thr Leu Ile Ala Leu Phe Ser Asn Phe Tyr Ile Gln Thr Tyr Lys Lys
                245                 250                 255

cac ctt gtt tca caa aag aag gag tat cat cag aat ggc tct gtt gct      816
His Leu Val Ser Gln Lys Lys Glu Tyr His Gln Asn Gly Ser Val Ala
            260                 265                 270

tca ttg aat ggc cat gtg aat ggg gtg aca ccc acg gaa acc att aca      864
Ser Leu Asn Gly His Val Asn Gly Val Thr Pro Thr Glu Thr Ile Thr
        275                 280                 285

cac agg aaa gtg agg ggg gac                                          885
His Arg Lys Val Arg Gly Asp
    290                 295
```

<210> 54
<211> 295
<212> PRT
<213> Oncorhynchus mykiss

<400> 54

```
Met Glu Thr Phe Asn Tyr Lys Leu Asn Met Tyr Ile Asp Ser Trp Met
1               5                   10                  15
```

Gly Pro Arg Asp Glu Arg Val Gln Gly Trp Leu Leu Leu Asp Asn Tyr
20 25 30

Pro Pro Thr Phe Ala Leu Thr Val Met Tyr Leu Leu Ile Val Trp Met
35 40 45

Gly Pro Lys Tyr Met Arg His Arg Gln Pro Val Ser Cys Arg Gly Leu
50 55 60

Leu Leu Val Tyr Asn Leu Gly Leu Thr Ile Leu Ser Phe Tyr Met Phe
65 70 75 80

Tyr Glu Met Val Ser Ala Val Trp His Gly Asp Tyr Asn Phe Phe Cys
85 90 95

Gln Asp Thr His Ser Ala Gly Glu Thr Asp Thr Lys Ile Ile Asn Val
100 105 110

Leu Trp Trp Tyr Tyr Phe Ser Lys Leu Ile Glu Phe Met Asp Thr Phe
115 120 125

Phe Phe Ile Leu Arg Lys Asn Asn His Gln Ile Thr Phe Leu His Ile
130 135 140

Tyr His His Ala Ser Met Leu Asn Ile Trp Trp Phe Val Met Asn Trp
145 150 155 160

Val Pro Cys Gly His Ser Tyr Phe Gly Ala Ser Leu Asn Ser Phe Ile
165 170 175

His Val Leu Met Tyr Ser Tyr Tyr Gly Leu Ser Ala Val Pro Ala Leu
180 185 190

Arg Pro Tyr Leu Trp Trp Lys Lys Tyr Ile Thr Gln Val Gln Leu Ile
195 200 205

Gln Phe Phe Leu Thr Met Ser Gln Thr Ile Cys Ala Val Ile Trp Pro
210 215 220

Cys Asp Phe Pro Arg Gly Trp Leu Tyr Phe Gln Ile Phe Tyr Val Ile
225 230 235 240

Thr Leu Ile Ala Leu Phe Ser Asn Phe Tyr Ile Gln Thr Tyr Lys Lys
245 250 255

His Leu Val Ser Gln Lys Lys Glu Tyr His Gln Asn Gly Ser Val Ala
260 265 270

Ser Leu Asn Gly His Val Asn Gly Val Thr Pro Thr Glu Thr Ile Thr
275 280 285

His Arg Lys Val Arg Gly Asp
290 295

<210> 55
<211> 6753
<212> DNA
<213> Oncorhynchus mykiss

<220>
<221> CDS
<222> (513)..(1397)
<223> Delta-5-Elongase

<400> 55

```
acggattaga agccgccgag cgggtgacag ccctccgaag gaagactctc ctccgtgcgt      60

cctcgtcctc accggtcgcg ttcctgaaac gcagatgtgc ctcgcgccgc actgctccga     120

acaataaaga ttctacaata ctagctttta tggttatgaa gaggaaaaat tggcagtaac     180

ctggccccac aaaccttcaa atgaacgaat caaattaaca accataggat gataatgcga     240

ttagtttttt agccttattt ctggggtaat taatcagcga agcgatgatt tttgatctat     300

taacagatat ataaatgcaa aaactgcatt aaccacttta actaatactt tcaacatttt     360

cggtttgtat tacttcttat tcaaatgtaa taaaagtatc aacaaaaaat tgttaatata     420

cctctatact ttaacgtcaa ggagaaaaaa ccccggatcg gactactagc agctgtaata     480

cgactcacta tagggaatat taagcttaca ta atg gag act ttt aat tat aaa       533
                                    Met Glu Thr Phe Asn Tyr Lys
                                    1               5

cta aac atg tac ata gac tca tgg atg ggt ccc aga gat gag cgg gta       581
Leu Asn Met Tyr Ile Asp Ser Trp Met Gly Pro Arg Asp Glu Arg Val
        10                  15                  20

cag gga tgg ctg ctt ctg gac aac tac cct cca acc ttt gca cta aca       629
Gln Gly Trp Leu Leu Leu Asp Asn Tyr Pro Pro Thr Phe Ala Leu Thr
    25                  30                  35

gtc atg tac ctg ctg atc gta tgg atg ggg ccc aag tac atg aga cac       677
Val Met Tyr Leu Leu Ile Val Trp Met Gly Pro Lys Tyr Met Arg His
40                  45                  50                  55

aga cag ccg gtg tct tgc cgg ggt ctc ctc ttg gtc tac aat ctg ggc       725
Arg Gln Pro Val Ser Cys Arg Gly Leu Leu Leu Val Tyr Asn Leu Gly
                60                  65                  70

ctc acg atc ttg tcc ttc tat atg ttc tat gag atg gtg tct gct gtg       773
Leu Thr Ile Leu Ser Phe Tyr Met Phe Tyr Glu Met Val Ser Ala Val
            75                  80                  85

tgg cac ggg gat tat aac ttc ttt tgc caa gac aca cac agt gca gga       821
Trp His Gly Asp Tyr Asn Phe Phe Cys Gln Asp Thr His Ser Ala Gly
        90                  95                  100
```

```
gaa acc gat acc aag atc ata aat gtg ctg tgg tgg tac tac ttc tcc      869
Glu Thr Asp Thr Lys Ile Ile Asn Val Leu Trp Trp Tyr Tyr Phe Ser
    105                 110                 115

aag ctc ata gag ttt atg gat acc ttc ttc ttc atc ctg cgg aag aac      917
Lys Leu Ile Glu Phe Met Asp Thr Phe Phe Phe Ile Leu Arg Lys Asn
120                 125                 130                 135

aac cat caa atc acg ttt ctg cac atc tac cac cat gct agc atg ctc      965
Asn His Gln Ile Thr Phe Leu His Ile Tyr His His Ala Ser Met Leu
                140                 145                 150

aac atc tgg tgg ttc gtc atg aac tgg gtg ccc tgt ggt cac tcc tac     1013
Asn Ile Trp Trp Phe Val Met Asn Trp Val Pro Cys Gly His Ser Tyr
            155                 160                 165

ttt ggt gcc tcc ctg aac agc ttc atc cat gtc ctg atg tac tct tac     1061
Phe Gly Ala Ser Leu Asn Ser Phe Ile His Val Leu Met Tyr Ser Tyr
        170                 175                 180

tat ggg ctc tct gct gtc ccg gcc ttg cgg ccc tat cta tgg tgg aag     1109
Tyr Gly Leu Ser Ala Val Pro Ala Leu Arg Pro Tyr Leu Trp Trp Lys
    185                 190                 195

aaa tac atc aca caa gta cag ctg att cag ttc ttt ttg acc atg tcc     1157
Lys Tyr Ile Thr Gln Val Gln Leu Ile Gln Phe Phe Leu Thr Met Ser
200                 205                 210                 215

cag acg ata tgt gca gtc att tgg cca tgt gat ttc ccc aga ggg tgg     1205
Gln Thr Ile Cys Ala Val Ile Trp Pro Cys Asp Phe Pro Arg Gly Trp
                220                 225                 230

ctg tat ttc cag ata ttc tat gtc atc aca ctt att gcc ctt ttc tca     1253
Leu Tyr Phe Gln Ile Phe Tyr Val Ile Thr Leu Ile Ala Leu Phe Ser
            235                 240                 245

aac ttc tac att cag act tac aag aaa cac ctt gtt tca caa aag aag     1301
Asn Phe Tyr Ile Gln Thr Tyr Lys Lys His Leu Val Ser Gln Lys Lys
        250                 255                 260

gag tat cat cag aat ggc tct gtt gct tca ttg aat ggc cat gtg aat     1349
Glu Tyr His Gln Asn Gly Ser Val Ala Ser Leu Asn Gly His Val Asn
    265                 270                 275

ggg gtg aca ccc acg gaa acc att aca cac agg aaa gtg agg ggg gac     1397
Gly Val Thr Pro Thr Glu Thr Ile Thr His Arg Lys Val Arg Gly Asp
280                 285                 290                 295

tgaaggatcc actagtaacg gccgccagtg tgctggaatt ctgcagatat ccagcacagt   1457

ggcggccgct cgagtctaga gggcccttcg aaggtaagcc tatccctaac cctctcctcg   1517

gtctcgattc tacgcgtacc ggtcatcatc accatcacca ttgagtttaa acccgctgat   1577

cctagagggc cgcatcatgt aattagttat gtcacgctta cattcacgcc ctccccccac   1637

atccgctcta accgaaaagg aaggagttag acaacctgaa gtctaggtcc ctatttattt   1697

ttttatagtt atgttagtat taagaacgtt atttatattt caaatttttc ttttttttct   1757

gtacagacgc gtgtacgcat gtaacattat actgaaaacc ttgcttgaga aggttttggg   1817

acgctcgaag gctttaattt gcaagctgcg gccctgcatt aatgaatcgg ccaacgcgcg   1877

gggagaggcg gtttgcgtat tgggcgctct tccgcttcct cgctcactga ctcgctgcgc   1937

tcggtcgttc ggctgcggcg agcggtatca gctcactcaa aggcggtaat acggttatcc   1997
```

acagaatcag gggataacgc aggaaagaac atgtgagcaa aaggccagca aaagcccagg 2057
aaccgtaaaa aggccgcgtt gctggcgttt ttccataggc tccgcccccc tgacgagcat 2117
cacaaaaatc gacgctcaag tcagaggtgg cgaaacccga caggactata aagataccag 2177
gcgtttcccc ctggaagctc cctcgtgcgc tctcctgttc cgaccctgcc gcttaccgga 2237
tacctgtccg cctttctccc ttcgggaagc gtggcgcttt ctcatagctc acgctgtagg 2297
tatctcagtt cggtgtaggt cgttcgctcc aagctgggct gtgtgcacga accccccgtt 2357
cagcccgacc gctgcgcctt atccggtaac tatcgtcttg agtccaaccc ggtaagacac 2417
gacttatcgc cactggcagc agccactggt aacaggatta gcagagcgag gtatgtaggc 2477
ggtgctacag agttcttgaa gtggtggcct aactacggct acactagaag gacagtattt 2537
ggtatctgcg ctctgctgaa gccagttacc ttcggaaaaa gagttggtag ctcttgatcc 2597
ggcaaacaaa ccaccgctgg tagcggtggt ttttttgttt gcaagcagca gattacgcgc 2657
agaaaaaaag gatctcaaga agatcctttg atcttttcta cggggtctga cgctcagtgg 2717
aacgaaaact cacgttaagg gattttggtc atgagattat caaaaaggat cttcacctag 2777
atccttttaa attaaaaatg aagttttaaa tcaatctaaa gtatatatga gtaaacttgg 2837
tctgacagtt accaatgctt aatcagtgag gcacctatct cagcgatctg tctatttcgt 2897
tcatccatag ttgcctgact ccccgtcgtg tagataacta cgatacggga gcgcttacca 2957
tctggcccca gtgctgcaat gataccgcga gacccacgct caccggctcc agatttatca 3017
gcaataaacc agccagccgg aagggccgag cgcagaagtg gtcctgcaac tttatccgcc 3077
tccatccagt ctattaattg ttgccgggaa gctagagtaa gtagttcgcc agttaatagt 3137
ttgcgcaacg ttgttgccat tgctacaggc atcgtggtgt cacgctcgtc gtttggtatg 3197
gcttcattca gctccggttc ccaacgatca aggcgagtta catgatcccc catgttgtgc 3257
aaaaaagcgg ttagctcctt cggtcctccg atcgttgtca gaagtaagtt ggccgcagtg 3317
ttatcactca tggttatggc agcactgcat aattctctta ctgtcatgcc atccgtaaga 3377
tgcttttctg tgactggtga gtactcaacc aagtcattct gagaatagtg tatgcggcga 3437
ccgagttgct cttgcccggc gtcaacacgg gataataccg cgccacatag cagaacttta 3497
aaagtgctca tcattggaaa acgttcttcg gggcgaaaac tctcaaggat cttaccgctg 3557
ttgagatcca gttcgatgta acccactcgt gcacccaact gatcttcagc atcttttact 3617
ttcaccagcg tttctgggtg agcaaaaaca ggaaggcaaa atgccgcaaa aaagggaata 3677
agggcgacac ggaaatgttg aatactcata ctcttccttt ttcaatatta ttgaagcatt 3737
tatcagggtt attgtctcat gagcggatac atatttgaat gtatttagaa aaataaacaa 3797
ataggggttc cgcgcacatt tccccgaaaa gtgccacctg acgtctaaga aaccattatt 3857
atcatgacat taacctataa aaataggcgt atcacgaggc cctttcgtct tcaagaaatt 3917
cggtcgaaaa aagaaaagga gagggccaag agggagggca ttggtgacta ttgagcacgt 3977
gagtatacgt gattaagcac acaaaggcag cttggagtat gtctgttatt aatttcacag 4037

gtagttctgg tccattggtg aaagtttgcg gcttgcagag cacagaggcc gcagaatgtg 4097
ctctagattc cgatgctgac ttgctgggta ttatatgtgt gcccaataga aagagaacaa 4157
ttgacccggt tattgcaagg aaaatttcaa gtcttgtaaa agcatataaa aatagttcag 4217
gcactccgaa atacttggtt ggcgtgtttc gtaatcaacc taaggaggat gttttggctc 4277
tggtcaatga ttacggcatt gatatcgtcc aactgcacgg agatgagtcg tggcaagaat 4337
accaagagtt cctcggtttg ccagttatta aaagactcgt atttccaaaa gactgcaaca 4397
tactactcag tgcagcttca cagaaacctc attcgtttat tcccttgttt gattcagaag 4457
caggtgggac aggtgaactt ttggattgga actcgatttc tgactgggtt ggaaggcaag 4517
agagccccga gagcttacat tttatgttag ctggtggact gacgccagaa aatgttggtg 4577
atgcgcttag attaaatggc gttattggtg ttgatgtaag cggaggtgtg gagacaaatg 4637
gtgtaaaaga ctctaacaaa atagcaaatt tcgtcaaaaa tgctaagaaa taggttatta 4697
ctgagtagta tttatttaag tattgtttgt gcacttgccc tagcttatcg atgataagct 4757
gtcaaagatg agaattaatt ccacggacta tagactatac tagatactcc gtctactgta 4817
cgatacactt ccgctcaggt ccttgtcctt taacgaggcc ttaccactct tttgttactc 4877
tattgatcca gctcagcaaa ggcagtgtga tctaagattc tatcttcgcg atgtagtaaa 4937
actagctaga ccgagaaaga gactagaaat gcaaaaggca cttctacaat ggctgccatc 4997
attattatcc gatgtgacgc tgcagcttct caatgatatt cgaatacgct tgaggagat 5057
acagcctaat atccgacaaa ctgttttaca gatttacgat cgtacttgtt acccatcatt 5117
gaattttgaa catccgaacc tgggagtttt ccctgaaaca gatagtatat ttgaacctgt 5177
ataataatat atagtctagc gctttacgga agacaatgta tgtatttcgg ttcctggaga 5237
aactattgca tctattgcat aggtaatctt gcacgtcgca tccccggttc attttctgcg 5297
tttccatctt gcacttcaat agcatatctt tgttaacgaa gcatctgtgc ttcattttgt 5357
agaacaaaaa tgcaacgcga gagcgctaat tttcaaaca aagaatctga gctgcatttt 5417
tacagaacag aaatgcaacg cgaaagcgct attttaccaa cgaagaatct gtgcttcatt 5477
tttgtaaaac aaaaatgcaa cgcgacgaga gcgctaattt ttcaaacaaa gaatctgagc 5537
tgcattttta cagaacagaa atgcaacgcg agagcgctat tttaccaaca aagaatctat 5597
acttcttttt tgttctacaa aaatgcatcc cgagagcgct atttttctaa caaagcatct 5657
tagattactt tttttctcct ttgtgcgctc tataatgcag tctcttgata acttttgca 5717
ctgtaggtcc gttaaggtta gaagaaggct actttggtgt ctattttctc ttccataaaa 5777
aaagcctgac tccacttccc gcgtttactg attactagcg aagctgcggg tgcatttttt 5837
caagataaag gcatccccga ttatattcta taccgatgtg gattgcgcat actttgtgaa 5897
cagaaagtga tagcgttgat gattcttcat tggtcagaaa attatgaacg gtttcttcta 5957
ttttgtctct atatactacg tataggaaat gtttacattt tcgtattgtt ttcgattcac 6017
tctatgaata gttcttacta caattttttt gtctaaagag taatactaga gataaacata 6077

```
aaaaatgtag aggtcgagtt tagatgcaag ttcaaggagc gaaaggtgga tgggtaggtt    6137
atatagggat atagcacaga gatatatagc aaagagatac ttttgagcaa tgtttgtgga    6197
agcggtattc gcaatgggaa gctccacccc ggttgataat cagaaaagcc ccaaaaacag    6257
gaagattgta taagcaaata tttaaattgt aaacgttaat attttgttaa aattcgcgtt    6317
aaatttttgt taaatcagct cattttttaa cgaatagccc gaaatcggca aaatccctta    6377
taaatcaaaa gaatagaccg agatagggtt gagtgttgtt ccagtttcca acaagagtcc    6437
actattaaag aacgtggact ccaacgtcaa agggcgaaaa agggtctatc agggcgatgg    6497
cccactacgt gaaccatcac cctaatcaag ttttttgggg tcgaggtgcc gtaaagcagt    6557
aaatcggaag ggtaaacgga tgcccccatt tagagcttga cggggaaagc cggcgaacgt    6617
ggcgagaaag gaagggaaga aagcgaaagg agcgggggct agggcggtgg gaagtgtagg    6677
ggtcacgctg ggcgtaacca ccacacccgc cgcgcttaat ggggcgctac agggcgcgtg    6737
gggatgatcc actagt                                                    6753
```

<210> 56
<211> 295
<212> PRT
<213> Oncorhynchus mykiss

<400> 56

```
Met Glu Thr Phe Asn Tyr Lys Leu Asn Met Tyr Ile Asp Ser Trp Met
1               5                   10                  15

Gly Pro Arg Asp Glu Arg Val Gln Gly Trp Leu Leu Leu Asp Asn Tyr
            20                  25                  30

Pro Pro Thr Phe Ala Leu Thr Val Met Tyr Leu Leu Ile Val Trp Met
        35                  40                  45

Gly Pro Lys Tyr Met Arg His Arg Gln Pro Val Ser Cys Arg Gly Leu
    50                  55                  60

Leu Leu Val Tyr Asn Leu Gly Leu Thr Ile Leu Ser Phe Tyr Met Phe
65                  70                  75                  80

Tyr Glu Met Val Ser Ala Val Trp His Gly Asp Tyr Asn Phe Phe Cys
                85                  90                  95

Gln Asp Thr His Ser Ala Gly Glu Thr Asp Thr Lys Ile Ile Asn Val
            100                 105                 110

Leu Trp Trp Tyr Tyr Phe Ser Lys Leu Ile Glu Phe Met Asp Thr Phe
        115                 120                 125
```

Phe Phe Ile Leu Arg Lys Asn Asn His Gln Ile Thr Phe Leu His Ile
130 135 140

Tyr His His Ala Ser Met Leu Asn Ile Trp Trp Phe Val Met Asn Trp
145 150 155 160

Val Pro Cys Gly His Ser Tyr Phe Gly Ala Ser Leu Asn Ser Phe Ile
165 170 175

His Val Leu Met Tyr Ser Tyr Tyr Gly Leu Ser Ala Val Pro Ala Leu
180 185 190

Arg Pro Tyr Leu Trp Trp Lys Lys Tyr Ile Thr Gln Val Gln Leu Ile
195 200 205

Gln Phe Phe Leu Thr Met Ser Gln Thr Ile Cys Ala Val Ile Trp Pro
210 215 220

Cys Asp Phe Pro Arg Gly Trp Leu Tyr Phe Gln Ile Phe Tyr Val Ile
225 230 235 240

Thr Leu Ile Ala Leu Phe Ser Asn Phe Tyr Ile Gln Thr Tyr Lys Lys
245 250 255

His Leu Val Ser Gln Lys Lys Glu Tyr His Gln Asn Gly Ser Val Ala
260 265 270

Ser Leu Asn Gly His Val Asn Gly Val Thr Pro Thr Glu Thr Ile Thr
275 280 285

His Arg Lys Val Arg Gly Asp
290 295

<210> 57
<211> 6645
<212> DNA
<213> Oncorhynchus mykiss

<220>
<221> CDS
<222> (513)..(1304)
<223> Delta-5-Elongase

<400> 57

acggattaga agccgccgag cgggtgacag ccctccgaag gaagactctc ctccgtgcgt 60

```
cctcgtcctc accggtcgcg ttcctgaaac gcagatgtgc ctcgcgccgc actgctccga    120

acaataaaga ttctacaata ctagctttta tggttatgaa gaggaaaaat tggcagtaac    180

ctggccccac aaaccttcaa atgaacgaat caaattaaca accataggat gataatgcga    240

ttagtttttt agccttattt ctggggtaat taatcagcga agcgatgatt tttgatctat    300

taacagatat ataaatgcaa aaactgcatt aaccacttta actaatactt tcaacatttt    360

cggtttgtat tacttcttat tcaaatgtaa taaaagtatc aacaaaaaat tgttaatata    420

cctctatact ttaacgtcaa ggagaaaaaa ccccggatcg gactactagc agctgtaata    480

cgactcacta tagggaatat taagcttaca ta atg gct tca aca tgg caa agc     533
                                    Met Ala Ser Thr Trp Gln Ser
                                    1               5

gtt cag tcc atg cgc cag tgg att tta gag aat gga gat aaa agg aca     581
Val Gln Ser Met Arg Gln Trp Ile Leu Glu Asn Gly Asp Lys Arg Thr
        10                  15                  20

gac cca tgg cta ctg gtc tac tcc cct atg cca gtg gcc att ata ttc     629
Asp Pro Trp Leu Leu Val Tyr Ser Pro Met Pro Val Ala Ile Ile Phe
    25                  30                  35

ctc ctc tat ctt ggt gtg gtc tgg gct ggg ccc aag ctg atg aaa cgc     677
Leu Leu Tyr Leu Gly Val Val Trp Ala Gly Pro Lys Leu Met Lys Arg
40                  45                  50                  55

agg gaa cca gtt gat ctc aag gct gta ctc att gtc tac aac ttc gcc     725
Arg Glu Pro Val Asp Leu Lys Ala Val Leu Ile Val Tyr Asn Phe Ala
                60                  65                  70

atg gtc tgc ctg tct gtc tac atg ttc cat gag ttc ttg gtc acg tcc     773
Met Val Cys Leu Ser Val Tyr Met Phe His Glu Phe Leu Val Thr Ser
            75                  80                  85

ttg ctg tct aac tac agt tac ctg tgt caa cct gtg gat tac agc act     821
Leu Leu Ser Asn Tyr Ser Tyr Leu Cys Gln Pro Val Asp Tyr Ser Thr
        90                  95                  100

agt cca ctg gcg atg agg atg gcc aaa gta tgc tgg tgg ttt ttc ttc     869
Ser Pro Leu Ala Met Arg Met Ala Lys Val Cys Trp Trp Phe Phe Phe
    105                 110                 115

tcc aag gtc ata gaa ttg gct gac acg gtg ttc ttc atc ctg agg aag     917
Ser Lys Val Ile Glu Leu Ala Asp Thr Val Phe Phe Ile Leu Arg Lys
120                 125                 130                 135

aag aac agt cag ctg act ttc ctg cat gtc tat cac cat ggc acc atg     965
Lys Asn Ser Gln Leu Thr Phe Leu His Val Tyr His His Gly Thr Met
                140                 145                 150

atc ttc aac tgg tgg gca ggg gtc aag tat ctg gct gga ggc caa tcg    1013
Ile Phe Asn Trp Trp Ala Gly Val Lys Tyr Leu Ala Gly Gly Gln Ser
            155                 160                 165

ttc ttc atc ggc ctg ctc aat acc ttt gtg cac atc gtg atg tac tct    1061
Phe Phe Ile Gly Leu Leu Asn Thr Phe Val His Ile Val Met Tyr Ser
        170                 175                 180

tac tac gga ctg gct gcc ctg ggg cct cac acg cag aag tac tta tgg    1109
Tyr Tyr Gly Leu Ala Ala Leu Gly Pro His Thr Gln Lys Tyr Leu Trp
    185                 190                 195

tgg aag cgc tat ctg acc tca ctg cag ctg ctc cag ttt gtc ctg ttg    1157
```

Trp Lys Arg Tyr Leu Thr Ser Leu Gln Leu Leu Gln Phe Val Leu Leu
200 205 210 215

acc act cac act ggc tac aac ctc ttc act gag tgt gac ttc ccg gac 1205
Thr Thr His Thr Gly Tyr Asn Leu Phe Thr Glu Cys Asp Phe Pro Asp
220 225 230

tcc atg aac gct gtg gtg ttt gcc tac tgt gtc agt ctc att gct ctc 1253
Ser Met Asn Ala Val Val Phe Ala Tyr Cys Val Ser Leu Ile Ala Leu
235 240 245

ttc agc aac ttc tac tat cag agc tac ctc aac agg aag agc aag aag 1301
Phe Ser Asn Phe Tyr Tyr Gln Ser Tyr Leu Asn Arg Lys Ser Lys Lys
250 255 260

aca taaggatcca ctagtaacgg ccgccagtgt gctggaattc tgcagatatc 1354
Thr

catcacactg gcggccgctc gagcatgcat ctagagggcc gcatcatgta attagttatg 1414

tcacgcttac attcacgccc tccccccaca tccgctctaa ccgaaaagga aggagttaga 1474

caacctgaag tctaggtccc tatttatttt tttatagtta tgttagtatt aagaacgtta 1534

tttatatttc aaatttttct tttttttctg tacagacgcg tgtacgcatg taacattata 1594

ctgaaaacct tgcttgagaa ggttttggga cgctcgaagg ctttaatttg cggccctgca 1654

ttaatgaatc ggccaacgcg cggggagagg cggtttgcgt attgggcgct cttccgcttc 1714

ctcgctcact gactcgctgc gctcggtcgt tcggctgcgg cgagcggtat cagctcactc 1774

aaaggcggta atacggttat ccacagaatc aggggataac gcaggaaaga acatgtgagc 1834

aaaaggccag caaaagccca ggaaccgtaa aaaggccgcg ttgctggcgt ttttccatag 1894

gctccgcccc cctgacgagc atcacaaaaa tcgacgctca agtcagaggt ggcgaaaccc 1954

gacaggacta taaagatacc aggcgtttcc ccctggaagc tccctcgtgc gctctcctgt 2014

tccgaccctg ccgcttaccg gatacctgtc cgcctttctc ccttcgggaa gcgtggcgct 2074

ttctcatagc tcacgctgta ggtatctcag ttcggtgtag gtcgttcgct ccaagctggg 2134

ctgtgtgcac gaaccccccg ttcagcccga ccgctgcgcc ttatccggta actatcgtct 2194

tgagtccaac ccggtaagac acgacttatc gccactggca gcagccactg gtaacaggat 2254

tagcagagcg aggtatgtag gcggtgctac agagttcttg aagtggtggc ctaactacgg 2314

ctacactaga aggacagtat ttggtatctg cgctctgctg aagccagtta ccttcggaaa 2374

aagagttggt agctcttgat ccggcaaaca aaccaccgct ggtagcggtg gtttttttgt 2434

ttgcaagcag cagattacgc gcagaaaaaa aggatctcaa gaagatcctt tgatcttttc 2494

tacggggtct gacgctcagt ggaacgaaaa ctcacgttaa gggattttgg tcatgagatt 2554

atcaaaaagg atcttcacct agatcctttt aaattaaaaa tgaagtttta aatcaatcta 2614

aagtatatat gagtaaactt ggtctgacag ttaccaatgc ttaatcagtg aggcacctat 2674

ctcagcgatc tgtctatttc gttcatccat agttgcctga ctccccgtcg tgtagataac 2734

tacgatacgg gagcgcttac catctggccc cagtgctgca atgataccgc gagacccacg 2794

ctcaccggct ccagatttat cagcaataaa ccagccagcc ggaagggccg agcgcagaag 2854

```
tggtcctgca actttatccg cctccattca gtctattaat tgttgccggg aagctagagt   2914
aagtagttcg ccagttaata gtttgcgcaa cgttgttggc attgctacag gcatcgtggt   2974
gtcactctcg tcgtttggta tggcttcatt cagctccggt tcccaacgat caaggcgagt   3034
tacatgatcc cccatgttgt gcaaaaaagc ggttagctcc ttcggtcctc cgatcgttgt   3094
cagaagtaag ttggccgcag tgttatcact catggttatg gcagcactgc ataattctct   3154
tactgtcatg ccatccgtaa gatgcttttc tgtgactggt gagtactcaa ccaagtcatt   3214
ctgagaatag tgtatgcggc gaccgagttg ctcttgcccg gcgtcaatac gggataatag   3274
tgtatcacat agcagaactt taaaagtgct catcattgga aaacgttctt cggggcgaaa   3334
actctcaagg atcttaccgc tgttgagatc cagttcgatg taacccactc gtgcacccaa   3394
ctgatcttca gcatctttta ctttcaccag cgtttctggg tgagcaaaaa caggaaggca   3454
aaatgccgca aaaaagggaa taagggcgac acggaaatgt tgaatactca tactcttcct   3514
ttttcaatgg gtaataactg atataattaa attgaagctc taatttgtga gtttagtata   3574
catgcattta cttataatac agttttttag ttttgctggc cgcatcttct caaatatgct   3634
tcccagcctg cttttctgta acgttcaccc tctaccttag catcccttcc ctttgcaaat   3694
agtcctcttc caacaataat aatgtcagat cctgtagaga ccacatcatc cacggttcta   3754
tactgttgac ccaatgcgtc tcccttgtca tctaaaccca caccgggtgt cataatcaac   3814
caatcgtaac cttcatctct tccacccatg tctctttgag caataaagcc gataacaaaa   3874
tctttgtcgc tcttcgcaat gtcaacagta ccctagtat attctccagt agatagggag    3934
cccttgcatg acaattctgc taacatcaaa aggcctctag gttcctttgt tacttcttct   3994
gccgcctgct tcaaaccgct aacaatacct gggcccacca caccgtgtgc attcgtaatg   4054
tctgcccatt ctgctattct gtatacaccc gcagagtact gcaatttgac tgtattacca   4114
atgtcagcaa attttctgtc ttcgaagagt aaaaaattgt acttggcgga taatgccttt   4174
agcggcttaa ctgtgccctc catggaaaaa tcagtcaaga tatccacatg tgtttttagt   4234
aaacaaattt tgggacctaa tgcttcaact aactccagta attccttggt ggtacgaaca   4294
tccaatgaag cacacaagtt tgtttgcttt tcgtgcatga tattaaatag cttggcagca   4354
acaggactag gatgagtagc agcacgttcc ttatatgtag ctttcgacat gatttatctt   4414
cgtttcctgc aggtttttgt tctgtgcagt tgggttaaga atactgggca atttcatgtt   4474
tcttcaacac tacatatgcg tatatatacc aatctaagtc tgtgctcctt ccttcgttct   4534
tccttctgtt cggagattac cgaatcaaaa aaatttcaaa gaaaccgaaa tcaaaaaaaa   4594
gaataaaaaa aaaatgatga attgaattga aaagctagct tatcgatgat aagctgtcaa   4654
agatgagaat taattccacg gactatagac tatactagat actccgtcta ctgtacgata   4714
cacttccgct caggtccttg tcctttaacg aggccttacc actcttttgt tactctattg   4774
atccagctca gcaaaggcag tgtgatctaa gattctatct tcgcgatgta gtaaaactag   4834
ctagaccgag aaagagacta gaaatgcaaa aggcacttct acaatggctg ccatcattat   4894
```

tatccgatgt gacgctgcag cttctcaatg atattcgaat acgctttgag gagatacagc 4954
ctaatatccg acaaactgtt ttacagattt acgatcgtac ttgttaccca tcattgaatt 5014
ttgaacatcc gaacctggga gttttccctg aaacagatag tatatttgaa cctgtataat 5074
aatatatagt ctagcgcttt acggaagaca atgtatgtat ttcggttcct ggagaaacta 5134
ttgcatctat tgcataggta atcttgcacg tcgcatcccc ggttcatttt ctgcgtttcc 5194
atcttgcact tcaatagcat atctttgtta acgaagcatc tgtgcttcat tttgtagaac 5254
aaaaatgcaa cgcgagagcg ctaatttttc aaacaaagaa tctgagctgc atttttacag 5314
aacagaaatg caacgcgaaa gcgctatttt accaacgaag aatctgtgct tcatttttgt 5374
aaaacaaaaa tgcaacgcga cgagagcgct aatttttcaa acaaagaatc tgagctgcat 5434
ttttacagaa cagaaatgca acgcgagagc gctattttac caacaaagaa tctatacttc 5494
tttttgttc tacaaaaatg catcccgaga gcgctatttt tctaacaaag catcttagat 5554
tactttttt ctcctttgtg cgctctataa tgcagtctct tgataacttt ttgcactgta 5614
ggtccgttaa ggttagaaga aggctacttt ggtgtctatt ttctcttcca taaaaaaagc 5674
ctgactccac ttcccgcgtt tactgattac tagcgaagct gcgggtgcat tttttcaaga 5734
taaaggcatc cccgattata ttctataccg atgtggattg cgcatacttt gtgaacagaa 5794
agtgatagcg ttgatgattc ttcattggtc agaaaattat gaacggtttc ttctattttg 5854
tctctatata ctacgtatag gaaatgttta cattttcgta ttgttttcga ttcactctat 5914
gaatagttct tactacaatt tttttgtcta aagagtaata ctagagataa acataaaaaa 5974
tgtagaggtc gagtttagat gcaagttcaa ggagcgaaag gtggatgggt aggttatata 6034
gggatatagc acagagatat atagcaaaga gatacttttg agcaatgttt gtggaagcgg 6094
tattcgcaat gggaagctcc accccggttg ataatcagaa aagccccaaa aacaggaaga 6154
ttgtataagc aaatatttaa attgtaaacg ttaatatttt gttaaaattc gcgttaaatt 6214
tttgttaaat cagctcattt tttaacgaat agcccgaaat cggcaaaatc ccttataaat 6274
caaaagaata gaccgagata gggttgagtg ttgttccagt ttccaacaag agtccactat 6334
taaagaacgt ggactccaac gtcaaagggc gaaaaagggt ctatcagggc gatggcccac 6394
tacgtgaacc atcaccctaa tcaagttttt tggggtcgag gtgccgtaaa gcagtaaatc 6454
ggaagggtaa acggatgccc ccatttagag cttgacgggg aaagccggcg aacgtggcga 6514
gaaaggaagg gaagaaagcg aaaggagcgg gggctagggc ggtgggaagt gtaggggtca 6574
cgctgggcgt aaccaccaca cccgccgcgc ttaatggggc gctacagggc gcgtggggat 6634
gatccactag t 6645

<210> 58
<211> 264
<212> PRT
<213> Oncorhynchus mykiss

<400> 58

Met Ala Ser Thr Trp Gln Ser Val Gln Ser Met Arg Gln Trp Ile Leu
1 5 10 15

Glu Asn Gly Asp Lys Arg Thr Asp Pro Trp Leu Leu Val Tyr Ser Pro
20 25 30

Met Pro Val Ala Ile Ile Phe Leu Leu Tyr Leu Gly Val Val Trp Ala
35 40 45

Gly Pro Lys Leu Met Lys Arg Arg Glu Pro Val Asp Leu Lys Ala Val
50 55 60

Leu Ile Val Tyr Asn Phe Ala Met Val Cys Leu Ser Val Tyr Met Phe
65 70 75 80

His Glu Phe Leu Val Thr Ser Leu Leu Ser Asn Tyr Ser Tyr Leu Cys
85 90 95

Gln Pro Val Asp Tyr Ser Thr Ser Pro Leu Ala Met Arg Met Ala Lys
100 105 110

Val Cys Trp Trp Phe Phe Phe Ser Lys Val Ile Glu Leu Ala Asp Thr
115 120 125

Val Phe Phe Ile Leu Arg Lys Lys Asn Ser Gln Leu Thr Phe Leu His
130 135 140

Val Tyr His His Gly Thr Met Ile Phe Asn Trp Trp Ala Gly Val Lys
145 150 155 160

Tyr Leu Ala Gly Gly Gln Ser Phe Phe Ile Gly Leu Leu Asn Thr Phe
165 170 175

Val His Ile Val Met Tyr Ser Tyr Tyr Gly Leu Ala Ala Leu Gly Pro
180 185 190

His Thr Gln Lys Tyr Leu Trp Trp Lys Arg Tyr Leu Thr Ser Leu Gln
195 200 205

Leu Leu Gln Phe Val Leu Leu Thr Thr His Thr Gly Tyr Asn Leu Phe
210 215 220

Thr Glu Cys Asp Phe Pro Asp Ser Met Asn Ala Val Val Phe Ala Tyr
225 230 235 240

Cys Val Ser Leu Ile Ala Leu Phe Ser Asn Phe Tyr Tyr Gln Ser Tyr
245 250 255

Leu Asn Arg Lys Ser Lys Lys Thr
260

<210> 59

<211> 1077
<212> DNA
<213> Thalassiosira pseudonana

<220>
<221> CDS
<222> (1)..(1077)
<223> Delta-5-Elongase

<400> 59

```
atg tgc tca tca ccg ccg tca caa tcc aaa aca aca tcc ctc cta gca      48
Met Cys Ser Ser Pro Pro Ser Gln Ser Lys Thr Thr Ser Leu Leu Ala
1               5                   10                  15

cgg tac acc acc gcc gcc ctc ctc ctc ctc acc ctc aca aca tgg tgc      96
Arg Tyr Thr Thr Ala Ala Leu Leu Leu Leu Thr Leu Thr Thr Trp Cys
            20                  25                  30

cac ttc gcc ttc cca gcc gcc acc gcc aca ccc ggc ctc acc gcc gaa      144
His Phe Ala Phe Pro Ala Ala Thr Ala Thr Pro Gly Leu Thr Ala Glu
        35                  40                  45

atg cac tcc tac aaa gtc cca ctc ggt ctc acc gta ttc tac ctg ctg      192
Met His Ser Tyr Lys Val Pro Leu Gly Leu Thr Val Phe Tyr Leu Leu
    50                  55                  60

agt cta ccg tca cta aag tac gtt acg gac aac tac ctt gcc aaa aag      240
Ser Leu Pro Ser Leu Lys Tyr Val Thr Asp Asn Tyr Leu Ala Lys Lys
65                  70                  75                  80

tat gat atg aag tca ctc cta acg gaa tca atg gtg ttg tac aat gtg      288
Tyr Asp Met Lys Ser Leu Leu Thr Glu Ser Met Val Leu Tyr Asn Val
                85                  90                  95

gcg caa gtg ctg ctc aat ggg tgg acg gtg tat gcg att gtg gat gcg      336
Ala Gln Val Leu Leu Asn Gly Trp Thr Val Tyr Ala Ile Val Asp Ala
            100                 105                 110

gtg atg aat aga gac cat ccg ttt att gga agt aga agt ttg gtt ggg      384
Val Met Asn Arg Asp His Pro Phe Ile Gly Ser Arg Ser Leu Val Gly
        115                 120                 125

gcg gcg ttg cat agt ggg agc tcg tat gcg gtg tgg gtt cat tat tgt      432
Ala Ala Leu His Ser Gly Ser Ser Tyr Ala Val Trp Val His Tyr Cys
    130                 135                 140

gat aag tat ttg gag ttc ttt gat acg tat ttt atg gtg ttg agg ggg      480
Asp Lys Tyr Leu Glu Phe Phe Asp Thr Tyr Phe Met Val Leu Arg Gly
145                 150                 155                 160

aaa atg gac cag gtc tcc ttc ctc cac atc tac cac cac acg acc ata      528
```

```
Lys Met Asp Gln Val Ser Phe Leu His Ile Tyr His His Thr Thr Ile
                165                 170                 175

gcg tgg gca tgg tgg atc gcc ctc cgc ttc tcc ccc ggt gga gac att      576
Ala Trp Ala Trp Trp Ile Ala Leu Arg Phe Ser Pro Gly Gly Asp Ile
            180                 185                 190

tac ttc ggg gca ctc ctc aac tcc atc atc cac gtc ctc atg tat tcc      624
Tyr Phe Gly Ala Leu Leu Asn Ser Ile Ile His Val Leu Met Tyr Ser
        195                 200                 205

tac tac gcc ctt gcc cta ctc aag gtc agt tgt cca tgg aaa cga tac      672
Tyr Tyr Ala Leu Ala Leu Leu Lys Val Ser Cys Pro Trp Lys Arg Tyr
    210                 215                 220

ctg act caa gct caa tta ttg caa ttc aca agt gtg gtg gtt tat acg      720
Leu Thr Gln Ala Gln Leu Leu Gln Phe Thr Ser Val Val Val Tyr Thr
225                 230                 235                 240

ggg tgt acg ggt tat act cat tac tat cat acg aag cat gga gcg gat      768
Gly Cys Thr Gly Tyr Thr His Tyr Tyr His Thr Lys His Gly Ala Asp
                245                 250                 255

gag aca cag cct agt tta gga acg tat tat ttc tgt tgt gga gtg cag      816
Glu Thr Gln Pro Ser Leu Gly Thr Tyr Tyr Phe Cys Cys Gly Val Gln
            260                 265                 270

gtg ttt gag atg gtt agt ttg ttt gta ctc ttt tcc atc ttt tat aaa      864
Val Phe Glu Met Val Ser Leu Phe Val Leu Phe Ser Ile Phe Tyr Lys
        275                 280                 285

cga tcc tat tcg aag aag aac aag tca gga gga aag gat agc aag aag      912
Arg Ser Tyr Ser Lys Lys Asn Lys Ser Gly Gly Lys Asp Ser Lys Lys
    290                 295                 300

aat gat gat ggg aat aat gag gat caa tgt cac aag gct atg aag gat      960
Asn Asp Asp Gly Asn Asn Glu Asp Gln Cys His Lys Ala Met Lys Asp
305                 310                 315                 320

ata tcg gag ggt gcg aag gag gtt gtg ggg cat gca gcg aag gat gct     1008
Ile Ser Glu Gly Ala Lys Glu Val Val Gly His Ala Ala Lys Asp Ala
                325                 330                 335

gga aag ttg gtg gct acg gcg agt aag gct gta aag agg aag gga act     1056
Gly Lys Leu Val Ala Thr Ala Ser Lys Ala Val Lys Arg Lys Gly Thr
            340                 345                 350

cgt gtt act ggt gcc atg tag                                         1077
Arg Val Thr Gly Ala Met
        355
```

<210> 60
<211> 358
<212> PRT
<213> Thalassiosira pseudonana

<400> 60

```
Met Cys Ser Ser Pro Pro Ser Gln Ser Lys Thr Thr Ser Leu Leu Ala
1               5                   10                  15
```

Arg Tyr Thr Thr Ala Ala Leu Leu Leu Leu Thr Leu Thr Thr Trp Cys
20 25 30

His Phe Ala Phe Pro Ala Ala Thr Ala Thr Pro Gly Leu Thr Ala Glu
35 40 45

Met His Ser Tyr Lys Val Pro Leu Gly Leu Thr Val Phe Tyr Leu Leu
50 55 60

Ser Leu Pro Ser Leu Lys Tyr Val Thr Asp Asn Tyr Leu Ala Lys Lys
65 70 75 80

Tyr Asp Met Lys Ser Leu Leu Thr Glu Ser Met Val Leu Tyr Asn Val
85 90 95

Ala Gln Val Leu Leu Asn Gly Trp Thr Val Tyr Ala Ile Val Asp Ala
100 105 110

Val Met Asn Arg Asp His Pro Phe Ile Gly Ser Arg Ser Leu Val Gly
115 120 125

Ala Ala Leu His Ser Gly Ser Ser Tyr Ala Val Trp Val His Tyr Cys
130 135 140

Asp Lys Tyr Leu Glu Phe Phe Asp Thr Tyr Phe Met Val Leu Arg Gly
145 150 155 160

Lys Met Asp Gln Val Ser Phe Leu His Ile Tyr His His Thr Thr Ile
165 170 175

Ala Trp Ala Trp Trp Ile Ala Leu Arg Phe Ser Pro Gly Gly Asp Ile
180 185 190

Tyr Phe Gly Ala Leu Leu Asn Ser Ile Ile His Val Leu Met Tyr Ser
195 200 205

Tyr Tyr Ala Leu Ala Leu Leu Lys Val Ser Cys Pro Trp Lys Arg Tyr
210 215 220

Leu Thr Gln Ala Gln Leu Leu Gln Phe Thr Ser Val Val Val Tyr Thr
225 230 235 240

Gly Cys Thr Gly Tyr Thr His Tyr Tyr His Thr Lys His Gly Ala Asp
245 250 255

Glu Thr Gln Pro Ser Leu Gly Thr Tyr Tyr Phe Cys Cys Gly Val Gln
260 265 270

Val Phe Glu Met Val Ser Leu Phe Val Leu Phe Ser Ile Phe Tyr Lys
275 280 285

Arg Ser Tyr Ser Lys Lys Asn Lys Ser Gly Gly Lys Asp Ser Lys Lys
290 295 300

Asn Asp Asp Gly Asn Asn Glu Asp Gln Cys His Lys Ala Met Lys Asp
305 310 315 320

Ile Ser Glu Gly Ala Lys Glu Val Val Gly His Ala Ala Lys Asp Ala
325 330 335

Gly Lys Leu Val Ala Thr Ala Ser Lys Ala Val Lys Arg Lys Gly Thr
340 345 350

Arg Val Thr Gly Ala Met
355

<210> 61
<211> 933
<212> DNA
<213> Thalassiosira pseudonana

<220>
<221> CDS
<222> (1)..(933)
<223> Delta-5-Elongase

<400> 61

atg cac tcc tac aaa gtc cca ctc ggt ctc acc gta ttc tac ctg ctg 48
Met His Ser Tyr Lys Val Pro Leu Gly Leu Thr Val Phe Tyr Leu Leu
1 5 10 15

agt cta ccg tca cta aag tac gtt acg gac aac tac ctt gcc aaa aag 96
Ser Leu Pro Ser Leu Lys Tyr Val Thr Asp Asn Tyr Leu Ala Lys Lys
20 25 30

tat gat atg aag tca ctc cta acg gaa tca atg gtg ttg tac aat gtg 144
Tyr Asp Met Lys Ser Leu Leu Thr Glu Ser Met Val Leu Tyr Asn Val
35 40 45

gcg caa gtg ctg ctc aat ggg tgg acg gtg tat gcg att gtg gat gcg 192
Ala Gln Val Leu Leu Asn Gly Trp Thr Val Tyr Ala Ile Val Asp Ala
50 55 60

gtg atg aat aga gac cat ccg ttt att gga agt aga agt ttg gtt ggg 240
Val Met Asn Arg Asp His Pro Phe Ile Gly Ser Arg Ser Leu Val Gly
65 70 75 80

gcg gcg ttg cat agt ggg agc tcg tat gcg gtg tgg gtt cat tat tgt 288
Ala Ala Leu His Ser Gly Ser Ser Tyr Ala Val Trp Val His Tyr Cys
85 90 95

gat aag tat ttg gag ttc ttt gat acg tat ttt atg gtg ttg agg ggg 336
Asp Lys Tyr Leu Glu Phe Phe Asp Thr Tyr Phe Met Val Leu Arg Gly
100 105 110

```
aaa atg gac cag gtc tcc ttc ctc cac atc tac cac cac acg acc ata       384
Lys Met Asp Gln Val Ser Phe Leu His Ile Tyr His His Thr Thr Ile
        115                 120                 125

gcg tgg gca tgg tgg atc gcc ctc cgc ttc tcc ccc ggt gga gac att       432
Ala Trp Ala Trp Trp Ile Ala Leu Arg Phe Ser Pro Gly Gly Asp Ile
    130                 135                 140

tac ttc ggg gca ctc ctc aac tcc atc atc cac gtc ctc atg tat tcc       480
Tyr Phe Gly Ala Leu Leu Asn Ser Ile Ile His Val Leu Met Tyr Ser
145                 150                 155                 160

tac tac gcc ctt gcc cta ctc aag gtc agt tgt cca tgg aaa cga tac       528
Tyr Tyr Ala Leu Ala Leu Leu Lys Val Ser Cys Pro Trp Lys Arg Tyr
                165                 170                 175

ctg act caa gct caa tta ttg caa ttc aca agt gtg gtg gtt tat acg       576
Leu Thr Gln Ala Gln Leu Leu Gln Phe Thr Ser Val Val Val Tyr Thr
            180                 185                 190

ggg tgt acg ggt tat act cat tac tat cat acg aag cat gga gcg gat       624
Gly Cys Thr Gly Tyr Thr His Tyr Tyr His Thr Lys His Gly Ala Asp
        195                 200                 205

gag aca cag cct agt tta gga acg tat tat ttc tgt tgt gga gtg cag       672
Glu Thr Gln Pro Ser Leu Gly Thr Tyr Tyr Phe Cys Cys Gly Val Gln
    210                 215                 220

gtg ttt gag atg gtt agt ttg ttt gta ctc ttt tcc atc ttt tat aaa       720
Val Phe Glu Met Val Ser Leu Phe Val Leu Phe Ser Ile Phe Tyr Lys
225                 230                 235                 240

cga tcc tat tcg aag aag aac aag tca gga gga aag gat agc aag aag       768
Arg Ser Tyr Ser Lys Lys Asn Lys Ser Gly Gly Lys Asp Ser Lys Lys
                245                 250                 255

aat gat gat ggg aat aat gag gat caa tgt cac aag gct atg aag gat       816
Asn Asp Asp Gly Asn Asn Glu Asp Gln Cys His Lys Ala Met Lys Asp
            260                 265                 270

ata tcg gag ggt gcg aag gag gtt gtg ggg cat gca gcg aag gat gct       864
Ile Ser Glu Gly Ala Lys Glu Val Val Gly His Ala Ala Lys Asp Ala
        275                 280                 285

gga aag ttg gtg gct acg gcg agt aag gct gta aag agg aag gga act       912
Gly Lys Leu Val Ala Thr Ala Ser Lys Ala Val Lys Arg Lys Gly Thr
    290                 295                 300

cgt gtt act ggt gcc atg tag                                           933
Arg Val Thr Gly Ala Met
305                 310
```

<210> 62
<211> 310
<212> PRT
<213> Thalassiosira pseudonana

<400> 62

```
Met His Ser Tyr Lys Val Pro Leu Gly Leu Thr Val Phe Tyr Leu Leu
1               5                   10                  15
```

```
Ser Leu Pro Ser Leu Lys Tyr Val Thr Asp Asn Tyr Leu Ala Lys Lys
            20                  25                  30

Tyr Asp Met Lys Ser Leu Leu Thr Glu Ser Met Val Leu Tyr Asn Val
        35                  40                  45

Ala Gln Val Leu Leu Asn Gly Trp Thr Val Tyr Ala Ile Val Asp Ala
    50                  55                  60

Val Met Asn Arg Asp His Pro Phe Ile Gly Ser Arg Ser Leu Val Gly
65                  70                  75                  80

Ala Ala Leu His Ser Gly Ser Ser Tyr Ala Val Trp Val His Tyr Cys
                85                  90                  95

Asp Lys Tyr Leu Glu Phe Phe Asp Thr Tyr Phe Met Val Leu Arg Gly
            100                 105                 110

Lys Met Asp Gln Val Ser Phe Leu His Ile Tyr His His Thr Thr Ile
        115                 120                 125

Ala Trp Ala Trp Trp Ile Ala Leu Arg Phe Ser Pro Gly Gly Asp Ile
    130                 135                 140

Tyr Phe Gly Ala Leu Leu Asn Ser Ile Ile His Val Leu Met Tyr Ser
145                 150                 155                 160

Tyr Tyr Ala Leu Ala Leu Leu Lys Val Ser Cys Pro Trp Lys Arg Tyr
                165                 170                 175

Leu Thr Gln Ala Gln Leu Leu Gln Phe Thr Ser Val Val Val Tyr Thr
            180                 185                 190

Gly Cys Thr Gly Tyr Thr His Tyr Tyr His Thr Lys His Gly Ala Asp
        195                 200                 205

Glu Thr Gln Pro Ser Leu Gly Thr Tyr Tyr Phe Cys Cys Gly Val Gln
    210                 215                 220

Val Phe Glu Met Val Ser Leu Phe Val Leu Phe Ser Ile Phe Tyr Lys
225                 230                 235                 240

Arg Ser Tyr Ser Lys Lys Asn Lys Ser Gly Gly Lys Asp Ser Lys Lys
                245                 250                 255

Asn Asp Asp Gly Asn Asn Glu Asp Gln Cys His Lys Ala Met Lys Asp
            260                 265                 270

Ile Ser Glu Gly Ala Lys Glu Val Val Gly His Ala Ala Lys Asp Ala
        275                 280                 285
```

```
Gly Lys Leu Val Ala Thr Ala Ser Lys Ala Val Lys Arg Lys Gly Thr
    290                 295                 300

Arg Val Thr Gly Ala Met
305                 310
```

<210> 63
<211> 933
<212> DNA
<213> Thalassiosira pseudonana

<220>
<221> CDS
<222> (1)..(933)
<223> Delta-5-Elongase

<400> 63

```
atg cac tcc tac aaa gtc cca ctc ggt ctc acc gta ttc tac ctg ctg      48
Met His Ser Tyr Lys Val Pro Leu Gly Leu Thr Val Phe Tyr Leu Leu
1               5                   10                  15

agt cta ccg tca cta aag tac gtt acg gac aac tac ctt gcc aaa aag      96
Ser Leu Pro Ser Leu Lys Tyr Val Thr Asp Asn Tyr Leu Ala Lys Lys
            20                  25                  30

tat gat atg aag tca ctc cta acg gaa tca atg gtg ttg tac aat gtg     144
Tyr Asp Met Lys Ser Leu Leu Thr Glu Ser Met Val Leu Tyr Asn Val
        35                  40                  45

gcg caa gtg ctg ctc aat ggg tgg acg gtg tat gcg att gtg gat gcg     192
Ala Gln Val Leu Leu Asn Gly Trp Thr Val Tyr Ala Ile Val Asp Ala
    50                  55                  60

gtg atg aat aga gac cat ccg ttt att gga agt aga agt ttg gtt ggg     240
Val Met Asn Arg Asp His Pro Phe Ile Gly Ser Arg Ser Leu Val Gly
65                  70                  75                  80

gcg gcg ttg cat agt ggg agc tcg tat gcg gtg tgg gtt cat tat tgt     288
Ala Ala Leu His Ser Gly Ser Ser Tyr Ala Val Trp Val His Tyr Cys
                85                  90                  95

gat aag tat ttg gag ttc ttt gat acg tat ttt atg gtg ttg agg ggg     336
Asp Lys Tyr Leu Glu Phe Phe Asp Thr Tyr Phe Met Val Leu Arg Gly
            100                 105                 110

aaa atg gac cag gtc tcc ttc ctc cac atc tac cac cac acg acc ata     384
Lys Met Asp Gln Val Ser Phe Leu His Ile Tyr His His Thr Thr Ile
        115                 120                 125

gcg tgg gca tgg tgg atc gcc ctc cgc ttc tcc ccc ggt gga gac att     432
Ala Trp Ala Trp Trp Ile Ala Leu Arg Phe Ser Pro Gly Gly Asp Ile
    130                 135                 140

tac ttc ggg gca ctc ctc aac tcc atc atc cac gtc ctc atg tat tcc     480
```

```
Tyr Phe Gly Ala Leu Leu Asn Ser Ile Ile His Val Leu Met Tyr Ser
145                 150                 155                 160

tac tac gcc ctt gcc cta ctc aag gtc agt tgt cca tgg aaa cga tac      528
Tyr Tyr Ala Leu Ala Leu Leu Lys Val Ser Cys Pro Trp Lys Arg Tyr
                165                 170                 175

ctg act caa gct caa tta ttg caa ttc aca agt gtg gtg gtt tat acg      576
Leu Thr Gln Ala Gln Leu Leu Gln Phe Thr Ser Val Val Val Tyr Thr
            180                 185                 190

ggg tgt acg ggt tat act cat tac tat cat acg aag cat gga gcg gat      624
Gly Cys Thr Gly Tyr Thr His Tyr Tyr His Thr Lys His Gly Ala Asp
        195                 200                 205

gag aca cag cct agt tta gga acg tat tat ttc tgt tgt gga gtg cag      672
Glu Thr Gln Pro Ser Leu Gly Thr Tyr Tyr Phe Cys Cys Gly Val Gln
    210                 215                 220

gtg ttt gag atg gtt agt ttg ttt gta ctc ttt tcc atc ttt tat aaa      720
Val Phe Glu Met Val Ser Leu Phe Val Leu Phe Ser Ile Phe Tyr Lys
225                 230                 235                 240

cga tcc tat tcg aag aag aac aag tca gga gga aag gat agc aag aag      768
Arg Ser Tyr Ser Lys Lys Asn Lys Ser Gly Gly Lys Asp Ser Lys Lys
                245                 250                 255

aat gat gat ggg aat aat gag gat caa tgt cac aag gct atg aag gat      816
Asn Asp Asp Gly Asn Asn Glu Asp Gln Cys His Lys Ala Met Lys Asp
            260                 265                 270

ata tcg gag ggt gcg aag gag gtt gtg ggg cat gca gcg aag gat gct      864
Ile Ser Glu Gly Ala Lys Glu Val Val Gly His Ala Ala Lys Asp Ala
        275                 280                 285

gga aag ttg gtg gct acg gcg agt aag gct gta aag agg aag gga act      912
Gly Lys Leu Val Ala Thr Ala Ser Lys Ala Val Lys Arg Lys Gly Thr
    290                 295                 300

cgt gtt act ggt gcc atg tag                                          933
Arg Val Thr Gly Ala Met
305                 310
```

<210> 64
<211> 310
<212> PRT
<213> Thalassiosira pseudonana

<400> 64

```
Met His Ser Tyr Lys Val Pro Leu Gly Leu Thr Val Phe Tyr Leu Leu
1               5                   10                  15

Ser Leu Pro Ser Leu Lys Tyr Val Thr Asp Asn Tyr Leu Ala Lys Lys
            20                  25                  30

Tyr Asp Met Lys Ser Leu Leu Thr Glu Ser Met Val Leu Tyr Asn Val
        35                  40                  45
```

```
Ala Gln Val Leu Leu Asn Gly Trp Thr Val Tyr Ala Ile Val Asp Ala
    50                  55                  60

Val Met Asn Arg Asp His Pro Phe Ile Gly Ser Arg Ser Leu Val Gly
65                  70                  75                  80

Ala Ala Leu His Ser Gly Ser Ser Tyr Ala Val Trp Val His Tyr Cys
                85                  90                  95

Asp Lys Tyr Leu Glu Phe Phe Asp Thr Tyr Phe Met Val Leu Arg Gly
            100                 105                 110

Lys Met Asp Gln Val Ser Phe Leu His Ile Tyr His His Thr Thr Ile
        115                 120                 125

Ala Trp Ala Trp Trp Ile Ala Leu Arg Phe Ser Pro Gly Gly Asp Ile
    130                 135                 140

Tyr Phe Gly Ala Leu Leu Asn Ser Ile Ile His Val Leu Met Tyr Ser
145                 150                 155                 160

Tyr Tyr Ala Leu Ala Leu Leu Lys Val Ser Cys Pro Trp Lys Arg Tyr
                165                 170                 175

Leu Thr Gln Ala Gln Leu Leu Gln Phe Thr Ser Val Val Val Tyr Thr
            180                 185                 190

Gly Cys Thr Gly Tyr Thr His Tyr Tyr His Thr Lys His Gly Ala Asp
        195                 200                 205

Glu Thr Gln Pro Ser Leu Gly Thr Tyr Tyr Phe Cys Cys Gly Val Gln
    210                 215                 220

Val Phe Glu Met Val Ser Leu Phe Val Leu Phe Ser Ile Phe Tyr Lys
225                 230                 235                 240

Arg Ser Tyr Ser Lys Lys Asn Lys Ser Gly Gly Lys Asp Ser Lys Lys
                245                 250                 255

Asn Asp Asp Gly Asn Asn Glu Asp Gln Cys His Lys Ala Met Lys Asp
            260                 265                 270

Ile Ser Glu Gly Ala Lys Glu Val Val Gly His Ala Ala Lys Asp Ala
        275                 280                 285

Gly Lys Leu Val Ala Thr Ala Ser Lys Ala Val Lys Arg Lys Gly Thr
    290                 295                 300

Arg Val Thr Gly Ala Met
305                 310
```

<210> 65
<211> 825

<212> DNA
<213> Thraustochytrium aureum

<220>
<221> CDS
<222> (1)..(825)
<223> Delta-5-Elongase

<400> 65

```
atg acg agc aac atg agc gcg tgg ggc gtc gcc gtc gac cag acg cag         48
Met Thr Ser Asn Met Ser Ala Trp Gly Val Ala Val Asp Gln Thr Gln
1               5                   10                  15

cag gtc gtc gac cag atc atg ggc ggc gcc gag ccg tac aag ctg aca         96
Gln Val Val Asp Gln Ile Met Gly Gly Ala Glu Pro Tyr Lys Leu Thr
            20                  25                  30

gaa ggg cgc atg acg aac gtc gag acg atg ctg gcg atc gag tgc ggc        144
Glu Gly Arg Met Thr Asn Val Glu Thr Met Leu Ala Ile Glu Cys Gly
        35                  40                  45

tac gcc gcc atg ctg ctg ttc ctg acc ccg atc atg aag cag gcc gag        192
Tyr Ala Ala Met Leu Leu Phe Leu Thr Pro Ile Met Lys Gln Ala Glu
    50                  55                  60

aag ccc ttc gag ctc aag tcc ttc aag ctc gcc cac aac ctg ttc ctg        240
Lys Pro Phe Glu Leu Lys Ser Phe Lys Leu Ala His Asn Leu Phe Leu
65                  70                  75                  80

ttc gtc ctg tcc gcc tac atg tgc ctc gag acc gtc cgc cag gcc tac        288
Phe Val Leu Ser Ala Tyr Met Cys Leu Glu Thr Val Arg Gln Ala Tyr
                85                  90                  95

ctt gcg ggc tac tcg gtg ttc ggc aac gac atg gag aag ggc agc gag        336
Leu Ala Gly Tyr Ser Val Phe Gly Asn Asp Met Glu Lys Gly Ser Glu
            100                 105                 110

ccg cac gcg cac ggc atg gcc caa atc gtg tgg atc ttt tac gtg tcc        384
Pro His Ala His Gly Met Ala Gln Ile Val Trp Ile Phe Tyr Val Ser
        115                 120                 125

aag gcg tac gag ttc gtg gac acg ctg atc atg atc ctg tgc aaa aag        432
Lys Ala Tyr Glu Phe Val Asp Thr Leu Ile Met Ile Leu Cys Lys Lys
    130                 135                 140

ttc aac cag gtc tcc gtc ctg cac gtg tac cac cac gcc acc atc ttt        480
Phe Asn Gln Val Ser Val Leu His Val Tyr His His Ala Thr Ile Phe
145                 150                 155                 160

gct atc tgg ttt atg atc gcc aag tac gcc ccg ggc ggc gac gca tac        528
Ala Ile Trp Phe Met Ile Ala Lys Tyr Ala Pro Gly Gly Asp Ala Tyr
                165                 170                 175

ttt agc gtc atc ctg aac tcg ttc gtg cac acc gtc atg tac gcg tac        576
Phe Ser Val Ile Leu Asn Ser Phe Val His Thr Val Met Tyr Ala Tyr
            180                 185                 190
```

```
tac ttc ttc tcg tcg cag ggc ttc ggg ttc gtc aag ccg atc aag ccg      624
Tyr Phe Phe Ser Ser Gln Gly Phe Gly Phe Val Lys Pro Ile Lys Pro
        195                 200                 205

tac atc acc tcg ctg cag atg acg cag ttc atg gcg atg ctc gtg cag      672
Tyr Ile Thr Ser Leu Gln Met Thr Gln Phe Met Ala Met Leu Val Gln
    210                 215                 220

tcg ctg tac gac tac ctt tac ccg tgc gac tac ccg cag ggg ctc gtc      720
Ser Leu Tyr Asp Tyr Leu Tyr Pro Cys Asp Tyr Pro Gln Gly Leu Val
225                 230                 235                 240

aag ctc ctc ggc gtg tac atg ctc acc ctg ctt gcg ctc ttc ggc aac      768
Lys Leu Leu Gly Val Tyr Met Leu Thr Leu Leu Ala Leu Phe Gly Asn
                245                 250                 255

ttt ttc gtg cag agc tac ctc aag aag tcg aac aag ccc aag gcc aag      816
Phe Phe Val Gln Ser Tyr Leu Lys Lys Ser Asn Lys Pro Lys Ala Lys
            260                 265                 270

tcg gcc taa                                                          825
Ser Ala
```

<210> 66
<211> 274
<212> PRT
<213> Thraustochytrium aureum

<400> 66

```
Met Thr Ser Asn Met Ser Ala Trp Gly Val Ala Val Asp Gln Thr Gln
1               5                   10                  15

Gln Val Val Asp Gln Ile Met Gly Gly Ala Glu Pro Tyr Lys Leu Thr
            20                  25                  30

Glu Gly Arg Met Thr Asn Val Glu Thr Met Leu Ala Ile Glu Cys Gly
        35                  40                  45

Tyr Ala Ala Met Leu Leu Phe Leu Thr Pro Ile Met Lys Gln Ala Glu
    50                  55                  60

Lys Pro Phe Glu Leu Lys Ser Phe Lys Leu Ala His Asn Leu Phe Leu
65                  70                  75                  80

Phe Val Leu Ser Ala Tyr Met Cys Leu Glu Thr Val Arg Gln Ala Tyr
                85                  90                  95

Leu Ala Gly Tyr Ser Val Phe Gly Asn Asp Met Glu Lys Gly Ser Glu
            100                 105                 110

Pro His Ala His Gly Met Ala Gln Ile Val Trp Ile Phe Tyr Val Ser
        115                 120                 125
```

```
Lys Ala Tyr Glu Phe Val Asp Thr Leu Ile Met Ile Leu Cys Lys Lys
    130                 135                 140

Phe Asn Gln Val Ser Val Leu His Val Tyr His His Ala Thr Ile Phe
145                 150                 155                 160

Ala Ile Trp Phe Met Ile Ala Lys Tyr Ala Pro Gly Gly Asp Ala Tyr
                165                 170                 175

Phe Ser Val Ile Leu Asn Ser Phe Val His Thr Val Met Tyr Ala Tyr
            180                 185                 190

Tyr Phe Phe Ser Ser Gln Gly Phe Gly Phe Val Lys Pro Ile Lys Pro
        195                 200                 205

Tyr Ile Thr Ser Leu Gln Met Thr Gln Phe Met Ala Met Leu Val Gln
    210                 215                 220

Ser Leu Tyr Asp Tyr Leu Tyr Pro Cys Asp Tyr Pro Gln Gly Leu Val
225                 230                 235                 240

Lys Leu Leu Gly Val Tyr Met Leu Thr Leu Leu Ala Leu Phe Gly Asn
                245                 250                 255

Phe Phe Val Gln Ser Tyr Leu Lys Lys Ser Asn Lys Pro Lys Ala Lys
            260                 265                 270

Ser Ala
```

<210> 67
<211> 903
<212> DNA
<213> Ostreococcus tauri

<220>
<221> CDS
<222> (1)..(903)
<223> Delta-5-Elongase

<400> 67

```
atg agc gcc tcc ggt gcg ctg ctg ccc gcg atc gcg ttc gcc gcg tac       48
Met Ser Ala Ser Gly Ala Leu Leu Pro Ala Ile Ala Phe Ala Ala Tyr
1               5                   10                  15

gcg tac gcg acg tac gcc tac gcc ttt gag tgg tcg cac gcg aat ggc       96
```

```
Ala Tyr Ala Thr Tyr Ala Tyr Ala Phe Glu Trp Ser His Ala Asn Gly
            20                  25                  30

atc gac aac gtc gac gcg cgc gag tgg atc ggt gcg ctg tcg ttg agg      144
Ile Asp Asn Val Asp Ala Arg Glu Trp Ile Gly Ala Leu Ser Leu Arg
        35                  40                  45

ctc ccg gcg atc gcg acg acg atg tac ctg ttg ttc tgc ctg gtc gga      192
Leu Pro Ala Ile Ala Thr Thr Met Tyr Leu Leu Phe Cys Leu Val Gly
    50                  55                  60

ccg agg ttg atg gcg aag cgc gag gcg ttc gac ccg aag ggg ttc atg      240
Pro Arg Leu Met Ala Lys Arg Glu Ala Phe Asp Pro Lys Gly Phe Met
65                  70                  75                  80

ctg gcg tac aat gcg tat cag acg gcg ttc aac gtc gtc gtg ctc ggg      288
Leu Ala Tyr Asn Ala Tyr Gln Thr Ala Phe Asn Val Val Val Leu Gly
                85                  90                  95

atg ttc gcg cga gag atc tcg ggg ctg ggg cag ccc gtg tgg ggg tca      336
Met Phe Ala Arg Glu Ile Ser Gly Leu Gly Gln Pro Val Trp Gly Ser
            100                 105                 110

acc atg ccg tgg agc gat aga aaa tcg ttt aag atc ctc ctc ggg gtg      384
Thr Met Pro Trp Ser Asp Arg Lys Ser Phe Lys Ile Leu Leu Gly Val
        115                 120                 125

tgg ttg cac tac aac aac caa tat ttg gag cta ttg gac act gtg ttc      432
Trp Leu His Tyr Asn Asn Gln Tyr Leu Glu Leu Leu Asp Thr Val Phe
    130                 135                 140

atg gtt gcg cgc aag aag acg aag cag ttg agc ttc ttg cac gtt tat      480
Met Val Ala Arg Lys Lys Thr Lys Gln Leu Ser Phe Leu His Val Tyr
145                 150                 155                 160

cat cac gcc ctg ttg atc tgg gcg tgg tgg ttg gtg tgt cac ttg atg      528
His His Ala Leu Leu Ile Trp Ala Trp Trp Leu Val Cys His Leu Met
                165                 170                 175

gcc acg aac gat tgt atc gat gcc tac ttc ggc gcg gcg tgc aac tcg      576
Ala Thr Asn Asp Cys Ile Asp Ala Tyr Phe Gly Ala Ala Cys Asn Ser
            180                 185                 190

ttc att cac atc gtg atg tac tcg tat tat ctc atg tcg gcg ctc ggc      624
Phe Ile His Ile Val Met Tyr Ser Tyr Tyr Leu Met Ser Ala Leu Gly
        195                 200                 205

att cga tgc ccg tgg aag cga tac atc acc cag gct caa atg ctc caa      672
Ile Arg Cys Pro Trp Lys Arg Tyr Ile Thr Gln Ala Gln Met Leu Gln
    210                 215                 220

ttc gtc att gtc ttc gcg cac gcc gtg ttc gtg ctg cgt cag aag cac      720
Phe Val Ile Val Phe Ala His Ala Val Phe Val Leu Arg Gln Lys His
225                 230                 235                 240

tgc ccg gtc acc ctt cct tgg gcg caa atg ttc gtc atg acg aac atg      768
Cys Pro Val Thr Leu Pro Trp Ala Gln Met Phe Val Met Thr Asn Met
                245                 250                 255

ctc gtg ctc ttc ggg aac ttc tac ctc aag gcg tac tcg aac aag tcg      816
Leu Val Leu Phe Gly Asn Phe Tyr Leu Lys Ala Tyr Ser Asn Lys Ser
            260                 265                 270

cgc ggc gac ggc gcg agt tcc gtg aaa cca gcc gag acc acg cgc gcg      864
Arg Gly Asp Gly Ala Ser Ser Val Lys Pro Ala Glu Thr Thr Arg Ala
        275                 280                 285

ccc agc gtg cga cgc acg cga tct cga aaa att gac taa                  903
```

Pro Ser Val Arg Arg Thr Arg Ser Arg Lys Ile Asp
290 295 300

<210> 68
<211> 300
<212> PRT
<213> Ostreococcus tauri

<400> 68

Met Ser Ala Ser Gly Ala Leu Leu Pro Ala Ile Ala Phe Ala Ala Tyr
1 5 10 15

Ala Tyr Ala Thr Tyr Ala Tyr Ala Phe Glu Trp Ser His Ala Asn Gly
20 25 30

Ile Asp Asn Val Asp Ala Arg Glu Trp Ile Gly Ala Leu Ser Leu Arg
35 40 45

Leu Pro Ala Ile Ala Thr Thr Met Tyr Leu Leu Phe Cys Leu Val Gly
50 55 60

Pro Arg Leu Met Ala Lys Arg Glu Ala Phe Asp Pro Lys Gly Phe Met
65 70 75 80

Leu Ala Tyr Asn Ala Tyr Gln Thr Ala Phe Asn Val Val Val Leu Gly
85 90 95

Met Phe Ala Arg Glu Ile Ser Gly Leu Gly Gln Pro Val Trp Gly Ser
100 105 110

Thr Met Pro Trp Ser Asp Arg Lys Ser Phe Lys Ile Leu Leu Gly Val
115 120 125

Trp Leu His Tyr Asn Asn Gln Tyr Leu Glu Leu Leu Asp Thr Val Phe
130 135 140

Met Val Ala Arg Lys Lys Thr Lys Gln Leu Ser Phe Leu His Val Tyr
145 150 155 160

His His Ala Leu Leu Ile Trp Ala Trp Trp Leu Val Cys His Leu Met
165 170 175

Ala Thr Asn Asp Cys Ile Asp Ala Tyr Phe Gly Ala Ala Cys Asn Ser
180 185 190

Phe Ile His Ile Val Met Tyr Ser Tyr Tyr Leu Met Ser Ala Leu Gly
195 200 205

```
Ile Arg Cys Pro Trp Lys Arg Tyr Ile Thr Gln Ala Gln Met Leu Gln
    210                 215                 220

Phe Val Ile Val Phe Ala His Ala Val Phe Val Leu Arg Gln Lys His
225                 230                 235                 240

Cys Pro Val Thr Leu Pro Trp Ala Gln Met Phe Val Met Thr Asn Met
                245                 250                 255

Leu Val Leu Phe Gly Asn Phe Tyr Leu Lys Ala Tyr Ser Asn Lys Ser
            260                 265                 270

Arg Gly Asp Gly Ala Ser Ser Val Lys Pro Ala Glu Thr Thr Arg Ala
        275                 280                 285

Pro Ser Val Arg Arg Thr Arg Ser Arg Lys Ile Asp
    290                 295                 300
```

<210> 69
<211> 879
<212> DNA
<213> Ostreococcus tauri

<220>
<221> CDS
<222> (1)..(879)
<223> Delta-6-Elongase

<400> 69

```
atg agt ggc tta cgt gca ccc aac ttt tta cac aga ttc tgg aca aag      48
Met Ser Gly Leu Arg Ala Pro Asn Phe Leu His Arg Phe Trp Thr Lys
1               5                   10                  15

tgg gac tac gcg att tcc aaa gtc gtc ttc acg tgt gcc gac agt ttt      96
Trp Asp Tyr Ala Ile Ser Lys Val Val Phe Thr Cys Ala Asp Ser Phe
            20                  25                  30

cag tgg gac atc ggg cca gtg agt tcg agt acg gcg cat tta ccc gcc     144
Gln Trp Asp Ile Gly Pro Val Ser Ser Ser Thr Ala His Leu Pro Ala
        35                  40                  45

att gaa tcc cct acc cca ctg gtg act agc ctc ttg ttc tac tta gtc     192
Ile Glu Ser Pro Thr Pro Leu Val Thr Ser Leu Leu Phe Tyr Leu Val
    50                  55                  60

aca gtt ttc ttg tgg tat ggt cgt tta acc agg agt tca gac aag aaa     240
Thr Val Phe Leu Trp Tyr Gly Arg Leu Thr Arg Ser Ser Asp Lys Lys
65                  70                  75                  80

att aga gag cct acg tgg tta aga aga ttc ata ata tgt cat aat gcg     288
Ile Arg Glu Pro Thr Trp Leu Arg Arg Phe Ile Ile Cys His Asn Ala
                85                  90                  95
```

```
ttc ttg ata gtc ctc agt ctt tac atg tgc ctt ggt tgt gtg gcc caa     336
Phe Leu Ile Val Leu Ser Leu Tyr Met Cys Leu Gly Cys Val Ala Gln
            100                 105                 110

gcg tat cag aat gga tat act tta tgg ggt aat gaa ttc aag gcc acg     384
Ala Tyr Gln Asn Gly Tyr Thr Leu Trp Gly Asn Glu Phe Lys Ala Thr
        115                 120                 125

gaa act cag ctt gct ctc tac att tac att ttt tac gta agt aaa ata     432
Glu Thr Gln Leu Ala Leu Tyr Ile Tyr Ile Phe Tyr Val Ser Lys Ile
    130                 135                 140

tac gag ttt gta gat act tac att atg ctt ctc aag aat aac ttg cgg     480
Tyr Glu Phe Val Asp Thr Tyr Ile Met Leu Leu Lys Asn Asn Leu Arg
145                 150                 155                 160

caa gta agt ttc cta cac att tat cac cac agc acg att tcc ttt att     528
Gln Val Ser Phe Leu His Ile Tyr His His Ser Thr Ile Ser Phe Ile
                165                 170                 175

tgg tgg atc att gct cgg agg gct ccg ggt ggt gat gct tac ttc agc     576
Trp Trp Ile Ile Ala Arg Arg Ala Pro Gly Gly Asp Ala Tyr Phe Ser
            180                 185                 190

gcg gcc ttg aac tca tgg gta cac gtg tgc atg tac acc tat tat cta     624
Ala Ala Leu Asn Ser Trp Val His Val Cys Met Tyr Thr Tyr Tyr Leu
        195                 200                 205

tta tca acc ctt att gga aaa gaa gat cct aag cgt tcc aac tac ctt     672
Leu Ser Thr Leu Ile Gly Lys Glu Asp Pro Lys Arg Ser Asn Tyr Leu
    210                 215                 220

tgg tgg ggt cgc cac cta acg caa atg cag atg ctt cag ttt ttc ttc     720
Trp Trp Gly Arg His Leu Thr Gln Met Gln Met Leu Gln Phe Phe Phe
225                 230                 235                 240

aac gta ctt caa gcg ttg tac tgc gct tcg ttc tct acg tat ccc aag     768
Asn Val Leu Gln Ala Leu Tyr Cys Ala Ser Phe Ser Thr Tyr Pro Lys
                245                 250                 255

ttt ttg tcc aaa att ctg ctc gtc tat atg atg agc ctt ctc ggc ttg     816
Phe Leu Ser Lys Ile Leu Leu Val Tyr Met Met Ser Leu Leu Gly Leu
            260                 265                 270

ttt ggg cat ttc tac tat tcc aag cac ata gca gca gct aag ctc cag     864
Phe Gly His Phe Tyr Tyr Ser Lys His Ile Ala Ala Ala Lys Leu Gln
        275                 280                 285

aaa aaa cag cag tga                                                 879
Lys Lys Gln Gln
    290
```

<210> 70
<211> 292
<212> PRT
<213> Ostreococcus tauri

<400> 70

```
Met Ser Gly Leu Arg Ala Pro Asn Phe Leu His Arg Phe Trp Thr Lys
1               5                   10                  15
```

```
Trp Asp Tyr Ala Ile Ser Lys Val Val Phe Thr Cys Ala Asp Ser Phe
            20                  25                  30

Gln Trp Asp Ile Gly Pro Val Ser Ser Ser Thr Ala His Leu Pro Ala
        35                  40                  45

Ile Glu Ser Pro Thr Pro Leu Val Thr Ser Leu Leu Phe Tyr Leu Val
    50                  55                  60

Thr Val Phe Leu Trp Tyr Gly Arg Leu Thr Arg Ser Ser Asp Lys Lys
65                  70                  75                  80

Ile Arg Glu Pro Thr Trp Leu Arg Arg Phe Ile Ile Cys His Asn Ala
                85                  90                  95

Phe Leu Ile Val Leu Ser Leu Tyr Met Cys Leu Gly Cys Val Ala Gln
            100                 105                 110

Ala Tyr Gln Asn Gly Tyr Thr Leu Trp Gly Asn Glu Phe Lys Ala Thr
        115                 120                 125

Glu Thr Gln Leu Ala Leu Tyr Ile Tyr Ile Phe Tyr Val Ser Lys Ile
    130                 135                 140

Tyr Glu Phe Val Asp Thr Tyr Ile Met Leu Leu Lys Asn Asn Leu Arg
145                 150                 155                 160

Gln Val Ser Phe Leu His Ile Tyr His His Ser Thr Ile Ser Phe Ile
                165                 170                 175

Trp Trp Ile Ile Ala Arg Arg Ala Pro Gly Gly Asp Ala Tyr Phe Ser
            180                 185                 190

Ala Ala Leu Asn Ser Trp Val His Val Cys Met Tyr Thr Tyr Tyr Leu
        195                 200                 205

Leu Ser Thr Leu Ile Gly Lys Glu Asp Pro Lys Arg Ser Asn Tyr Leu
    210                 215                 220

Trp Trp Gly Arg His Leu Thr Gln Met Gln Met Leu Gln Phe Phe Phe
225                 230                 235                 240

Asn Val Leu Gln Ala Leu Tyr Cys Ala Ser Phe Ser Thr Tyr Pro Lys
                245                 250                 255

Phe Leu Ser Lys Ile Leu Leu Val Tyr Met Met Ser Leu Leu Gly Leu
            260                 265                 270

Phe Gly His Phe Tyr Tyr Ser Lys His Ile Ala Ala Ala Lys Leu Gln
        275                 280                 285

Lys Lys Gln Gln
    290
```

<210> 71
<211> 1362
<212> DNA
<213> Primula farinosa

<220>
<221> CDS
<222> (1)..(1362)
<223> Delta-6-Desaturase

<400> 71

```
atg gct aac aaa tct cca cca aac ccc aaa aca ggt tac ata acc agc      48
Met Ala Asn Lys Ser Pro Pro Asn Pro Lys Thr Gly Tyr Ile Thr Ser
1               5                   10                  15

tca gac ctg aaa tcc cac aac aag gca ggt gac cta tgg ata tca atc      96
Ser Asp Leu Lys Ser His Asn Lys Ala Gly Asp Leu Trp Ile Ser Ile
            20                  25                  30

cac ggc caa gtc tac gac gtg tcc tct tgg gcc gcc ctt cat ccg ggg     144
His Gly Gln Val Tyr Asp Val Ser Ser Trp Ala Ala Leu His Pro Gly
        35                  40                  45

ggc act gcc cct ctc atg gcc ctt gca gga cac gac gtg acc gat gct     192
Gly Thr Ala Pro Leu Met Ala Leu Ala Gly His Asp Val Thr Asp Ala
    50                  55                  60

ttc ctc gcg tac cat ccc cct tcc act gcc cgt ctc ctc cct cct ctc     240
Phe Leu Ala Tyr His Pro Pro Ser Thr Ala Arg Leu Leu Pro Pro Leu
65                  70                  75                  80

tct acc aac ctc ctt ctt caa aac cac tcc gtc tcc ccc acc tcc tca     288
Ser Thr Asn Leu Leu Leu Gln Asn His Ser Val Ser Pro Thr Ser Ser
                85                  90                  95

gac tac cgc aaa ctc ctc gac aac ttc cat aaa cat ggc ctt ttc cgc     336
Asp Tyr Arg Lys Leu Leu Asp Asn Phe His Lys His Gly Leu Phe Arg
            100                 105                 110

gcc agg ggc cac act gct tac gcc acc ttc gtc ttc atg ata gcg atg     384
Ala Arg Gly His Thr Ala Tyr Ala Thr Phe Val Phe Met Ile Ala Met
        115                 120                 125

ttt cta atg agc gtg act gga gtc ctt tgc agc gac agt gcg tgg gtc     432
Phe Leu Met Ser Val Thr Gly Val Leu Cys Ser Asp Ser Ala Trp Val
    130                 135                 140

cat ttg gct agc ggc gga gca atg ggg ttc gcc tgg atc caa tgc gga     480
His Leu Ala Ser Gly Gly Ala Met Gly Phe Ala Trp Ile Gln Cys Gly
145                 150                 155                 160

tgg ata ggt cac gac tct ggg cat tac cgg att atg tct gac agg aaa     528
```

```
Trp Ile Gly His Asp Ser Gly His Tyr Arg Ile Met Ser Asp Arg Lys
                165                 170                 175

tgg aac tgg ttc gcg caa atc cta agc aca aac tgc ctc cag ggg att      576
Trp Asn Trp Phe Ala Gln Ile Leu Ser Thr Asn Cys Leu Gln Gly Ile
            180                 185                 190

agt atc ggg tgg tgg aag tgg aac cat aat gcg cac cac atc gct tgc      624
Ser Ile Gly Trp Trp Lys Trp Asn His Asn Ala His His Ile Ala Cys
        195                 200                 205

aat agc ctg gat tac gac ccc gac ctc cag tat atc cct ttg ctc gtc      672
Asn Ser Leu Asp Tyr Asp Pro Asp Leu Gln Tyr Ile Pro Leu Leu Val
    210                 215                 220

gtc tcc ccc aag ttc ttc aac tcc ctt act tct cgt ttc tac gac aag      720
Val Ser Pro Lys Phe Phe Asn Ser Leu Thr Ser Arg Phe Tyr Asp Lys
225                 230                 235                 240

aag ctg aac ttc gac ggc gtg tcg agg ttt ctg gtt tgc tac cag cac      768
Lys Leu Asn Phe Asp Gly Val Ser Arg Phe Leu Val Cys Tyr Gln His
                245                 250                 255

tgg acg ttt tat ccg gtc atg tgt gtc gct agg ctg aac atg ctc gcg      816
Trp Thr Phe Tyr Pro Val Met Cys Val Ala Arg Leu Asn Met Leu Ala
            260                 265                 270

cag tca ttt ata acg ctt ttc tcg agt agg gag gtg tgc cat agg gcg      864
Gln Ser Phe Ile Thr Leu Phe Ser Ser Arg Glu Val Cys His Arg Ala
        275                 280                 285

caa gag gtt ttc gga ctt gcc gtg ttt tgg gtt tgg ttt ccg ctt tta      912
Gln Glu Val Phe Gly Leu Ala Val Phe Trp Val Trp Phe Pro Leu Leu
    290                 295                 300

ctt tct tgt tta cct aat tgg ggc gag agg att atg ttt ttg ctt gcg      960
Leu Ser Cys Leu Pro Asn Trp Gly Glu Arg Ile Met Phe Leu Leu Ala
305                 310                 315                 320

agc tat tcc gtt acg ggg ata caa cac gtg cag ttc agc ttg aac cat     1008
Ser Tyr Ser Val Thr Gly Ile Gln His Val Gln Phe Ser Leu Asn His
                325                 330                 335

ttt tct tcg gac gtc tat gtg ggc ccg cca gta ggt aat gac tgg ttc     1056
Phe Ser Ser Asp Val Tyr Val Gly Pro Pro Val Gly Asn Asp Trp Phe
            340                 345                 350

aag aaa cag act gcc ggg aca ctt aac ata tcg tgc ccg gcg tgg atg     1104
Lys Lys Gln Thr Ala Gly Thr Leu Asn Ile Ser Cys Pro Ala Trp Met
        355                 360                 365

gat tgg ttc cat ggc ggg tta cag ttt cag gtc gag cac cac ttg ttt     1152
Asp Trp Phe His Gly Gly Leu Gln Phe Gln Val Glu His His Leu Phe
    370                 375                 380

ccg cgg atg cct agg ggt cag ttt agg aag att tct cct ttt gtg agg     1200
Pro Arg Met Pro Arg Gly Gln Phe Arg Lys Ile Ser Pro Phe Val Arg
385                 390                 395                 400

gat ttg tgt aag aaa cac aac ttg cct tac aat atc gcg tct ttt act     1248
Asp Leu Cys Lys Lys His Asn Leu Pro Tyr Asn Ile Ala Ser Phe Thr
                405                 410                 415

aaa gcg aat gtg ttt acg ctt aag acg ctg aga aat acg gcc att gag     1296
Lys Ala Asn Val Phe Thr Leu Lys Thr Leu Arg Asn Thr Ala Ile Glu
            420                 425                 430

gct cgg gac ctc tct aat ccg ctc cca aag aat atg gtg tgg gaa gct     1344
```

```
Ala Arg Asp Leu Ser Asn Pro Leu Pro Lys Asn Met Val Trp Glu Ala
        435                 440                 445

ctt aaa act ctc ggg tga                                          1362
Leu Lys Thr Leu Gly
    450
```

<210> 72
<211> 453
<212> PRT
<213> Primula farinosa

<400> 72

```
Met Ala Asn Lys Ser Pro Pro Asn Pro Lys Thr Gly Tyr Ile Thr Ser
1               5                   10                  15

Ser Asp Leu Lys Ser His Asn Lys Ala Gly Asp Leu Trp Ile Ser Ile
            20                  25                  30

His Gly Gln Val Tyr Asp Val Ser Ser Trp Ala Ala Leu His Pro Gly
        35                  40                  45

Gly Thr Ala Pro Leu Met Ala Leu Ala Gly His Asp Val Thr Asp Ala
    50                  55                  60

Phe Leu Ala Tyr His Pro Pro Ser Thr Ala Arg Leu Leu Pro Pro Leu
65                  70                  75                  80

Ser Thr Asn Leu Leu Leu Gln Asn His Ser Val Ser Pro Thr Ser Ser
                85                  90                  95

Asp Tyr Arg Lys Leu Leu Asp Asn Phe His Lys His Gly Leu Phe Arg
            100                 105                 110

Ala Arg Gly His Thr Ala Tyr Ala Thr Phe Val Phe Met Ile Ala Met
        115                 120                 125

Phe Leu Met Ser Val Thr Gly Val Leu Cys Ser Asp Ser Ala Trp Val
    130                 135                 140

His Leu Ala Ser Gly Gly Ala Met Gly Phe Ala Trp Ile Gln Cys Gly
145                 150                 155                 160

Trp Ile Gly His Asp Ser Gly His Tyr Arg Ile Met Ser Asp Arg Lys
                165                 170                 175

Trp Asn Trp Phe Ala Gln Ile Leu Ser Thr Asn Cys Leu Gln Gly Ile
            180                 185                 190
```

Ser Ile Gly Trp Trp Lys Trp Asn His Asn Ala His His Ile Ala Cys
195 200 205

Asn Ser Leu Asp Tyr Asp Pro Asp Leu Gln Tyr Ile Pro Leu Leu Val
210 215 220

Val Ser Pro Lys Phe Phe Asn Ser Leu Thr Ser Arg Phe Tyr Asp Lys
225 230 235 240

Lys Leu Asn Phe Asp Gly Val Ser Arg Phe Leu Val Cys Tyr Gln His
245 250 255

Trp Thr Phe Tyr Pro Val Met Cys Val Ala Arg Leu Asn Met Leu Ala
260 265 270

Gln Ser Phe Ile Thr Leu Phe Ser Ser Arg Glu Val Cys His Arg Ala
275 280 285

Gln Glu Val Phe Gly Leu Ala Val Phe Trp Val Trp Phe Pro Leu Leu
290 295 300

Leu Ser Cys Leu Pro Asn Trp Gly Glu Arg Ile Met Phe Leu Leu Ala
305 310 315 320

Ser Tyr Ser Val Thr Gly Ile Gln His Val Gln Phe Ser Leu Asn His
325 330 335

Phe Ser Ser Asp Val Tyr Val Gly Pro Pro Val Gly Asn Asp Trp Phe
340 345 350

Lys Lys Gln Thr Ala Gly Thr Leu Asn Ile Ser Cys Pro Ala Trp Met
355 360 365

Asp Trp Phe His Gly Gly Leu Gln Phe Gln Val Glu His His Leu Phe
370 375 380

Pro Arg Met Pro Arg Gly Gln Phe Arg Lys Ile Ser Pro Phe Val Arg
385 390 395 400

Asp Leu Cys Lys Lys His Asn Leu Pro Tyr Asn Ile Ala Ser Phe Thr
405 410 415

Lys Ala Asn Val Phe Thr Leu Lys Thr Leu Arg Asn Thr Ala Ile Glu
420 425 430

Ala Arg Asp Leu Ser Asn Pro Leu Pro Lys Asn Met Val Trp Glu Ala
435 440 445

Leu Lys Thr Leu Gly
450

<210> 73

<211> 1362
<212> DNA
<213> Primula vialii

<220>
<221> CDS
<222> (1)..(1362)
<223> Delta-6-Desaturase

<400> 73

```
atg gct aac aaa tct cca cca aac ccc aaa aca ggt tac att acc agc      48
Met Ala Asn Lys Ser Pro Pro Asn Pro Lys Thr Gly Tyr Ile Thr Ser
1               5                   10                  15

tca gac ctg aaa ggg cac aac aaa gca gga gac cta tgg ata tca atc      96
Ser Asp Leu Lys Gly His Asn Lys Ala Gly Asp Leu Trp Ile Ser Ile
            20                  25                  30

cac ggg gag gta tac gac gtg tcc tcg tgg gcc ggc ctt cac ccg ggg     144
His Gly Glu Val Tyr Asp Val Ser Ser Trp Ala Gly Leu His Pro Gly
        35                  40                  45

ggc agt gcc ccc ctc atg gcc ctc gca gga cac gac gta acc gac gct     192
Gly Ser Ala Pro Leu Met Ala Leu Ala Gly His Asp Val Thr Asp Ala
    50                  55                  60

ttt cta gcg tat cat cct cct tct acc gcc cgc ctc ctc cct ccc ctc     240
Phe Leu Ala Tyr His Pro Pro Ser Thr Ala Arg Leu Leu Pro Pro Leu
65                  70                  75                  80

tcc acc aac ctc ctc ctt caa aac cac tcc gtc tcc ccc acc tcc tct     288
Ser Thr Asn Leu Leu Leu Gln Asn His Ser Val Ser Pro Thr Ser Ser
                85                  90                  95

gac tac cgc aaa ctc ctc cac aac ttc cat aaa att ggt atg ttc cgc     336
Asp Tyr Arg Lys Leu Leu His Asn Phe His Lys Ile Gly Met Phe Arg
            100                 105                 110

gcc agg ggc cac act gct tac gcc acc ttc gtc atc atg ata gtg atg     384
Ala Arg Gly His Thr Ala Tyr Ala Thr Phe Val Ile Met Ile Val Met
        115                 120                 125

ttt cta acg agc gtg acc gga gtc ctt tgc agc gac agt gcg tgg gtc     432
Phe Leu Thr Ser Val Thr Gly Val Leu Cys Ser Asp Ser Ala Trp Val
    130                 135                 140

cat ctg gct agc ggc gca gca atg ggg ttc gcc tgg atc cag tgc gga     480
His Leu Ala Ser Gly Ala Ala Met Gly Phe Ala Trp Ile Gln Cys Gly
145                 150                 155                 160

tgg ata ggt cac gac tct ggg cat tac cgg att atg tct gac agg aaa     528
Trp Ile Gly His Asp Ser Gly His Tyr Arg Ile Met Ser Asp Arg Lys
                165                 170                 175

tgg aac tgg ttc gcg cag gtc ctg agc aca aac tgc ctc cag ggg atc     576
Trp Asn Trp Phe Ala Gln Val Leu Ser Thr Asn Cys Leu Gln Gly Ile
            180                 185                 190
```

```
agt atc ggg tgg tgg aag tgg aac cat aac gcc cac cac att gct tgc      624
Ser Ile Gly Trp Trp Lys Trp Asn His Asn Ala His His Ile Ala Cys
        195                 200                 205

aat agc ctg gac tac gac ccc gac ctc cag tat atc cct ttg ctc gtg      672
Asn Ser Leu Asp Tyr Asp Pro Asp Leu Gln Tyr Ile Pro Leu Leu Val
    210                 215                 220

gtc tcc ccc aag ttc ttc aac tcc ctt act tct cgt ttc tac gac aag      720
Val Ser Pro Lys Phe Phe Asn Ser Leu Thr Ser Arg Phe Tyr Asp Lys
225                 230                 235                 240

aag ctg aat ttc gac ggc gtg tca agg ttt ctg gtt tgc tac cag cac      768
Lys Leu Asn Phe Asp Gly Val Ser Arg Phe Leu Val Cys Tyr Gln His
                245                 250                 255

tgg acg ttt tat cca gtc atg tgt gtc gct agg cta aac atg atc gca      816
Trp Thr Phe Tyr Pro Val Met Cys Val Ala Arg Leu Asn Met Ile Ala
            260                 265                 270

cag tcg ttt ata acg ctt ttc tcg agc agg gag gtg ggt cat agg gcg      864
Gln Ser Phe Ile Thr Leu Phe Ser Ser Arg Glu Val Gly His Arg Ala
        275                 280                 285

caa gag att ttc gga ctt gct gtg ttt tgg gtt tgg ttt ccg ctc ctg      912
Gln Glu Ile Phe Gly Leu Ala Val Phe Trp Val Trp Phe Pro Leu Leu
    290                 295                 300

ctc tct tgc tta cct aat tgg agc gag agg att atg ttt ctg cta gcg      960
Leu Ser Cys Leu Pro Asn Trp Ser Glu Arg Ile Met Phe Leu Leu Ala
305                 310                 315                 320

agc tat tcc gtt acg ggg ata cag cac gtg cag ttc agc ttg aac cat     1008
Ser Tyr Ser Val Thr Gly Ile Gln His Val Gln Phe Ser Leu Asn His
                325                 330                 335

ttt tct tcg gac gtc tac gtg ggc ccg cca gta gct aac gac tgg ttc     1056
Phe Ser Ser Asp Val Tyr Val Gly Pro Pro Val Ala Asn Asp Trp Phe
            340                 345                 350

aag aaa cag act gct ggg aca ctt aac ata tcg tgc ccg gcg tgg atg     1104
Lys Lys Gln Thr Ala Gly Thr Leu Asn Ile Ser Cys Pro Ala Trp Met
        355                 360                 365

gac tgg ttc cat ggc ggg ttg cag ttt cag gtc gag cac cac ttg ttt     1152
Asp Trp Phe His Gly Gly Leu Gln Phe Gln Val Glu His His Leu Phe
    370                 375                 380

ccg cgg atg cct agg ggt cag ttt agg aag att tct cct ttt gtg agg     1200
Pro Arg Met Pro Arg Gly Gln Phe Arg Lys Ile Ser Pro Phe Val Arg
385                 390                 395                 400

gat ttg tgt aag aaa cac aac ttg cct tac aat atc gcg tct ttt act     1248
Asp Leu Cys Lys Lys His Asn Leu Pro Tyr Asn Ile Ala Ser Phe Thr
                405                 410                 415

aaa gca aac gtg ttg acg ctt aag acg ctg aga aat acg gcc att gag     1296
Lys Ala Asn Val Leu Thr Leu Lys Thr Leu Arg Asn Thr Ala Ile Glu
            420                 425                 430

gct cgg gac ctc tct aat ccg acc cca aag aat atg gtg tgg gaa gcc     1344
Ala Arg Asp Leu Ser Asn Pro Thr Pro Lys Asn Met Val Trp Glu Ala
        435                 440                 445

gtc cac aca cac ggc tag                                             1362
Val His Thr His Gly
    450
```

<210> 74

<211> 453
<212> PRT
<213> Primula vialii

<400> 74

```
Met Ala Asn Lys Ser Pro Pro Asn Pro Lys Thr Gly Tyr Ile Thr Ser
1               5                   10                  15

Ser Asp Leu Lys Gly His Asn Lys Ala Gly Asp Leu Trp Ile Ser Ile
            20                  25                  30

His Gly Glu Val Tyr Asp Val Ser Ser Trp Ala Gly Leu His Pro Gly
        35                  40                  45

Gly Ser Ala Pro Leu Met Ala Leu Ala Gly His Asp Val Thr Asp Ala
    50                  55                  60

Phe Leu Ala Tyr His Pro Pro Ser Thr Ala Arg Leu Leu Pro Pro Leu
65                  70                  75                  80

Ser Thr Asn Leu Leu Leu Gln Asn His Ser Val Ser Pro Thr Ser Ser
                85                  90                  95

Asp Tyr Arg Lys Leu Leu His Asn Phe His Lys Ile Gly Met Phe Arg
            100                 105                 110

Ala Arg Gly His Thr Ala Tyr Ala Thr Phe Val Ile Met Ile Val Met
        115                 120                 125

Phe Leu Thr Ser Val Thr Gly Val Leu Cys Ser Asp Ser Ala Trp Val
    130                 135                 140

His Leu Ala Ser Gly Ala Ala Met Gly Phe Ala Trp Ile Gln Cys Gly
145                 150                 155                 160

Trp Ile Gly His Asp Ser Gly His Tyr Arg Ile Met Ser Asp Arg Lys
                165                 170                 175

Trp Asn Trp Phe Ala Gln Val Leu Ser Thr Asn Cys Leu Gln Gly Ile
            180                 185                 190

Ser Ile Gly Trp Trp Lys Trp Asn His Asn Ala His His Ile Ala Cys
        195                 200                 205

Asn Ser Leu Asp Tyr Asp Pro Asp Leu Gln Tyr Ile Pro Leu Leu Val
    210                 215                 220
```

Val Ser Pro Lys Phe Phe Asn Ser Leu Thr Ser Arg Phe Tyr Asp Lys
225 230 235 240

Lys Leu Asn Phe Asp Gly Val Ser Arg Phe Leu Val Cys Tyr Gln His
245 250 255

Trp Thr Phe Tyr Pro Val Met Cys Val Ala Arg Leu Asn Met Ile Ala
260 265 270

Gln Ser Phe Ile Thr Leu Phe Ser Ser Arg Glu Val Gly His Arg Ala
275 280 285

Gln Glu Ile Phe Gly Leu Ala Val Phe Trp Val Trp Phe Pro Leu Leu
290 295 300

Leu Ser Cys Leu Pro Asn Trp Ser Glu Arg Ile Met Phe Leu Leu Ala
305 310 315 320

Ser Tyr Ser Val Thr Gly Ile Gln His Val Gln Phe Ser Leu Asn His
325 330 335

Phe Ser Ser Asp Val Tyr Val Gly Pro Pro Val Ala Asn Asp Trp Phe
340 345 350

Lys Lys Gln Thr Ala Gly Thr Leu Asn Ile Ser Cys Pro Ala Trp Met
355 360 365

Asp Trp Phe His Gly Gly Leu Gln Phe Gln Val Glu His His Leu Phe
370 375 380

Pro Arg Met Pro Arg Gly Gln Phe Arg Lys Ile Ser Pro Phe Val Arg
385 390 395 400

Asp Leu Cys Lys Lys His Asn Leu Pro Tyr Asn Ile Ala Ser Phe Thr
405 410 415

Lys Ala Asn Val Leu Thr Leu Lys Thr Leu Arg Asn Thr Ala Ile Glu
420 425 430

Ala Arg Asp Leu Ser Asn Pro Thr Pro Lys Asn Met Val Trp Glu Ala
435 440 445

Val His Thr His Gly
450

<210> 75
<211> 903
<212> DNA
<213> Ostreococcus tauri

<220>
<221> CDS
<222> (1)..(903)

<223> Delta-5-Elongase

<400> 75

```
atg agc gcc tcc ggt gcg ctg ctg ccc gcg atc gcg tcc gcc gcg tac        48
Met Ser Ala Ser Gly Ala Leu Leu Pro Ala Ile Ala Ser Ala Ala Tyr
1               5                   10                  15

gcg tac gcg acg tac gcc tac gcc ttt gag tgg tcg cac gcg aat ggc        96
Ala Tyr Ala Thr Tyr Ala Tyr Ala Phe Glu Trp Ser His Ala Asn Gly
            20                  25                  30

atc gac aac gtc gac gcg cgc gag tgg atc ggt gcg ctg tcg ttg agg       144
Ile Asp Asn Val Asp Ala Arg Glu Trp Ile Gly Ala Leu Ser Leu Arg
        35                  40                  45

ctc ccg gcg atc gcg acg acg atg tac ctg ttg ttc tgc ctg gtc gga       192
Leu Pro Ala Ile Ala Thr Thr Met Tyr Leu Leu Phe Cys Leu Val Gly
    50                  55                  60

ccg agg ttg atg gcg aag cgc gag gcg ttc gac ccg aag ggg ttc atg       240
Pro Arg Leu Met Ala Lys Arg Glu Ala Phe Asp Pro Lys Gly Phe Met
65                  70                  75                  80

ctg gcg tac aat gcg tat cag acg gcg ttc aac gtc gtc gtg ctc ggg       288
Leu Ala Tyr Asn Ala Tyr Gln Thr Ala Phe Asn Val Val Val Leu Gly
                85                  90                  95

atg ttc gcg cga gag atc tcg ggg ctg ggg cag ccc gtg tgg ggg tca       336
Met Phe Ala Arg Glu Ile Ser Gly Leu Gly Gln Pro Val Trp Gly Ser
            100                 105                 110

acc atg ccg tgg agc gat aga aaa tcg ttt aag atc ctc ctc ggg gtg       384
Thr Met Pro Trp Ser Asp Arg Lys Ser Phe Lys Ile Leu Leu Gly Val
        115                 120                 125

tgg ttg cac tac aac aac aaa tat ttg gag cta ttg gac act gtg ttc       432
Trp Leu His Tyr Asn Asn Lys Tyr Leu Glu Leu Leu Asp Thr Val Phe
    130                 135                 140

atg gtt gcg cgc aag aag acg aag cag ttg agc ttc ttg cac gtt tat       480
Met Val Ala Arg Lys Lys Thr Lys Gln Leu Ser Phe Leu His Val Tyr
145                 150                 155                 160

cat cac gcc ctg ttg atc tgg gcg tgg tgg ttg gtg tgt cac ttg atg       528
His His Ala Leu Leu Ile Trp Ala Trp Trp Leu Val Cys His Leu Met
                165                 170                 175

gcc acg aac gat tgt atc gat gcc tac ttc ggc gcg gcg tgc aac tcg       576
Ala Thr Asn Asp Cys Ile Asp Ala Tyr Phe Gly Ala Ala Cys Asn Ser
            180                 185                 190

ttc att cac atc gtg atg tac tcg tat tat ctc atg tcg gcg ctc ggc       624
Phe Ile His Ile Val Met Tyr Ser Tyr Tyr Leu Met Ser Ala Leu Gly
        195                 200                 205

att cga tgc ccg tgg aag cga tac atc acc cag gct caa atg ctc caa       672
```

```
Ile Arg Cys Pro Trp Lys Arg Tyr Ile Thr Gln Ala Gln Met Leu Gln
    210                 215                 220

ttc gtc att gtc ttc gcg cac gcc gtg ttc gtg ctg cgt cag aag cac      720
Phe Val Ile Val Phe Ala His Ala Val Phe Val Leu Arg Gln Lys His
225                 230                 235                 240

tgc ccg gtc acc ctt cct tgg gcg caa atg ttc gtc atg acg aac atg      768
Cys Pro Val Thr Leu Pro Trp Ala Gln Met Phe Val Met Thr Asn Met
                245                 250                 255

ctc gtg ctc ttc ggg aac ttc tac ctc aag gcg tac tcg aac aag tcg      816
Leu Val Leu Phe Gly Asn Phe Tyr Leu Lys Ala Tyr Ser Asn Lys Ser
            260                 265                 270

cgc ggc gac ggc gcg agt tcc gtg aaa cca gcc gag acc acg cgc gcg      864
Arg Gly Asp Gly Ala Ser Ser Val Lys Pro Ala Glu Thr Thr Arg Ala
        275                 280                 285

ccc agc gtg cga cgc acg cga tct cga aaa att gac taa                  903
Pro Ser Val Arg Arg Thr Arg Ser Arg Lys Ile Asp
    290                 295                 300
```

<210> 76
<211> 300
<212> PRT
<213> Ostreococcus tauri

<400> 76

```
Met Ser Ala Ser Gly Ala Leu Leu Pro Ala Ile Ala Ser Ala Ala Tyr
1               5                   10                  15

Ala Tyr Ala Thr Tyr Ala Tyr Ala Phe Glu Trp Ser His Ala Asn Gly
            20                  25                  30

Ile Asp Asn Val Asp Ala Arg Glu Trp Ile Gly Ala Leu Ser Leu Arg
        35                  40                  45

Leu Pro Ala Ile Ala Thr Thr Met Tyr Leu Leu Phe Cys Leu Val Gly
    50                  55                  60

Pro Arg Leu Met Ala Lys Arg Glu Ala Phe Asp Pro Lys Gly Phe Met
65                  70                  75                  80

Leu Ala Tyr Asn Ala Tyr Gln Thr Ala Phe Asn Val Val Val Leu Gly
                85                  90                  95

Met Phe Ala Arg Glu Ile Ser Gly Leu Gly Gln Pro Val Trp Gly Ser
            100                 105                 110

Thr Met Pro Trp Ser Asp Arg Lys Ser Phe Lys Ile Leu Leu Gly Val
        115                 120                 125
```

```
Trp Leu His Tyr Asn Asn Lys Tyr Leu Glu Leu Leu Asp Thr Val Phe
    130                 135                 140

Met Val Ala Arg Lys Lys Thr Lys Gln Leu Ser Phe Leu His Val Tyr
145                 150                 155                 160

His His Ala Leu Leu Ile Trp Ala Trp Trp Leu Val Cys His Leu Met
                165                 170                 175

Ala Thr Asn Asp Cys Ile Asp Ala Tyr Phe Gly Ala Ala Cys Asn Ser
            180                 185                 190

Phe Ile His Ile Val Met Tyr Ser Tyr Tyr Leu Met Ser Ala Leu Gly
        195                 200                 205

Ile Arg Cys Pro Trp Lys Arg Tyr Ile Thr Gln Ala Gln Met Leu Gln
    210                 215                 220

Phe Val Ile Val Phe Ala His Ala Val Phe Val Leu Arg Gln Lys His
225                 230                 235                 240

Cys Pro Val Thr Leu Pro Trp Ala Gln Met Phe Val Met Thr Asn Met
                245                 250                 255

Leu Val Leu Phe Gly Asn Phe Tyr Leu Lys Ala Tyr Ser Asn Lys Ser
            260                 265                 270

Arg Gly Asp Gly Ala Ser Ser Val Lys Pro Ala Glu Thr Thr Arg Ala
        275                 280                 285

Pro Ser Val Arg Arg Thr Arg Ser Arg Lys Ile Asp
    290                 295                 300
```

<210> 77
<211> 903
<212> DNA
<213> Ostreococcus tauri

<220>
<221> CDS
<222> (1)..(903)
<223> Delta-5-Elongase

<400> 77

```
atg agc gcc tcc ggt gcg ctg ctg ccc gcg atc gcg ttc gcc gcg tac      48
Met Ser Ala Ser Gly Ala Leu Leu Pro Ala Ile Ala Phe Ala Ala Tyr
1               5                   10                  15
```

```
gcg tac gcg acg tac gcc tac gcc ttt gag tgg tcg cac gcg aat ggc       96
Ala Tyr Ala Thr Tyr Ala Tyr Ala Phe Glu Trp Ser His Ala Asn Gly
            20                  25                  30

atc gac aac gtc gac gcg cgc gag tgg atc ggt gcg ctg tcg ttg agg      144
Ile Asp Asn Val Asp Ala Arg Glu Trp Ile Gly Ala Leu Ser Leu Arg
        35                  40                  45

ctc ccg gcg atc gcg acg acg atg tac ctg ttg ttc tgc ctg gtc gga      192
Leu Pro Ala Ile Ala Thr Thr Met Tyr Leu Leu Phe Cys Leu Val Gly
    50                  55                  60

ccg agg ttg atg gcg aag cgc gag gcg ttc gac ccg aag ggg ttc atg      240
Pro Arg Leu Met Ala Lys Arg Glu Ala Phe Asp Pro Lys Gly Phe Met
65                  70                  75                  80

ctg gcg tac aat gcg tat cag acg gcg ttc aac gtc gtc gtg ctc ggg      288
Leu Ala Tyr Asn Ala Tyr Gln Thr Ala Phe Asn Val Val Val Leu Gly
                85                  90                  95

atg ttc gcg cga gag atc tcg ggg ctg ggg cag ccc gtg tgg ggg tca      336
Met Phe Ala Arg Glu Ile Ser Gly Leu Gly Gln Pro Val Trp Gly Ser
            100                 105                 110

acc atg ccg tgg agc gat aga aaa tcg ttt aag atc ctc ctc ggg gtg      384
Thr Met Pro Trp Ser Asp Arg Lys Ser Phe Lys Ile Leu Leu Gly Val
        115                 120                 125

tgg ttg cac tac aac aac aaa tat ttg gag cta ttg gac act gtg ttc      432
Trp Leu His Tyr Asn Asn Lys Tyr Leu Glu Leu Leu Asp Thr Val Phe
    130                 135                 140

atg gtt gcg cgc aag aag acg aag cag ttg agc ttc ttg cac gtt tat      480
Met Val Ala Arg Lys Lys Thr Lys Gln Leu Ser Phe Leu His Val Tyr
145                 150                 155                 160

cat cac gcc ctg ttg atc tgg gcg tgg tgg ttg gtg tgt cac ttg atg      528
His His Ala Leu Leu Ile Trp Ala Trp Trp Leu Val Cys His Leu Met
                165                 170                 175

gcc acg aac gat tgt atc gat gcc tac ttc ggc gcg gcg tgc aac tcg      576
Ala Thr Asn Asp Cys Ile Asp Ala Tyr Phe Gly Ala Ala Cys Asn Ser
            180                 185                 190

ttc att cac atc gtg atg tac tcg tat tat ctc atg tcg gcg ctc ggc      624
Phe Ile His Ile Val Met Tyr Ser Tyr Tyr Leu Met Ser Ala Leu Gly
        195                 200                 205

att cga tgc ccg tgg aag cga tac atc acc cag gct caa atg ctc caa      672
Ile Arg Cys Pro Trp Lys Arg Tyr Ile Thr Gln Ala Gln Met Leu Gln
    210                 215                 220

ttc gtc att gtc ttc gcg cac gcc gtg ttc gtg ctg cgt cag aag cac      720
Phe Val Ile Val Phe Ala His Ala Val Phe Val Leu Arg Gln Lys His
225                 230                 235                 240

tgc ccg gtc acc ctt cct tgg gcg caa atg ttc gtc atg acg aac atg      768
Cys Pro Val Thr Leu Pro Trp Ala Gln Met Phe Val Met Thr Asn Met
                245                 250                 255

ctc gtg ctc ttc ggg aac ttc tac ctc aag gcg tac tcg aac aag tcg      816
Leu Val Leu Phe Gly Asn Phe Tyr Leu Lys Ala Tyr Ser Asn Lys Ser
            260                 265                 270

cgc ggc gac ggc gcg agt tcc gtg aaa cca gcc gag acc acg cgc gcg      864
Arg Gly Asp Gly Ala Ser Ser Val Lys Pro Ala Glu Thr Thr Arg Ala
        275                 280                 285
```

```
ccc agc gtg cga cgc acg cga tct cga aaa att gac taa                903
Pro Ser Val Arg Arg Thr Arg Ser Arg Lys Ile Asp
    290                 295                 300
```

<210> 78
<211> 300
<212> PRT
<213> Ostreococcus tauri

<400> 78

```
Met Ser Ala Ser Gly Ala Leu Leu Pro Ala Ile Ala Phe Ala Ala Tyr
1               5                   10                  15

Ala Tyr Ala Thr Tyr Ala Tyr Ala Phe Glu Trp Ser His Ala Asn Gly
            20                  25                  30

Ile Asp Asn Val Asp Ala Arg Glu Trp Ile Gly Ala Leu Ser Leu Arg
        35                  40                  45

Leu Pro Ala Ile Ala Thr Thr Met Tyr Leu Leu Phe Cys Leu Val Gly
    50                  55                  60

Pro Arg Leu Met Ala Lys Arg Glu Ala Phe Asp Pro Lys Gly Phe Met
65                  70                  75                  80

Leu Ala Tyr Asn Ala Tyr Gln Thr Ala Phe Asn Val Val Val Leu Gly
                85                  90                  95

Met Phe Ala Arg Glu Ile Ser Gly Leu Gly Gln Pro Val Trp Gly Ser
            100                 105                 110

Thr Met Pro Trp Ser Asp Arg Lys Ser Phe Lys Ile Leu Leu Gly Val
        115                 120                 125

Trp Leu His Tyr Asn Asn Lys Tyr Leu Glu Leu Leu Asp Thr Val Phe
    130                 135                 140

Met Val Ala Arg Lys Lys Thr Lys Gln Leu Ser Phe Leu His Val Tyr
145                 150                 155                 160

His His Ala Leu Leu Ile Trp Ala Trp Trp Leu Val Cys His Leu Met
                165                 170                 175

Ala Thr Asn Asp Cys Ile Asp Ala Tyr Phe Gly Ala Ala Cys Asn Ser
            180                 185                 190

Phe Ile His Ile Val Met Tyr Ser Tyr Tyr Leu Met Ser Ala Leu Gly
        195                 200                 205
```

```
Ile Arg Cys Pro Trp Lys Arg Tyr Ile Thr Gln Ala Gln Met Leu Gln
    210                 215                 220

Phe Val Ile Val Phe Ala His Ala Val Phe Val Leu Arg Gln Lys His
225                 230                 235                 240

Cys Pro Val Thr Leu Pro Trp Ala Gln Met Phe Val Met Thr Asn Met
                245                 250                 255

Leu Val Leu Phe Gly Asn Phe Tyr Leu Lys Ala Tyr Ser Asn Lys Ser
            260                 265                 270

Arg Gly Asp Gly Ala Ser Ser Val Lys Pro Ala Glu Thr Thr Arg Ala
        275                 280                 285

Pro Ser Val Arg Arg Thr Arg Ser Arg Lys Ile Asp
    290                 295                 300
```

<210> 79
<211> 903
<212> DNA
<213> Ostreococcus tauri

<220>
<221> CDS
<222> (1)..(903)
<223> Delta-5-Elongase

<400> 79

```
atg agc gcc tcc ggt gcg ctg ctg ccc gcg atc gcg tcc gcc gcg tac       48
Met Ser Ala Ser Gly Ala Leu Leu Pro Ala Ile Ala Ser Ala Ala Tyr
1               5                   10                  15

gcg tac gcg acg tac gcc tac gcc ttt gag tgg tcg cac gcg aat ggc       96
Ala Tyr Ala Thr Tyr Ala Tyr Ala Phe Glu Trp Ser His Ala Asn Gly
            20                  25                  30

atc gac aac gtc gac gcg cgc gag tgg atc ggt gcg ctg tcg ttg agg      144
Ile Asp Asn Val Asp Ala Arg Glu Trp Ile Gly Ala Leu Ser Leu Arg
        35                  40                  45

ctc ccg gcg atc gcg acg acg atg tac ctg ttg ttc tgc ctg gtc gga      192
Leu Pro Ala Ile Ala Thr Thr Met Tyr Leu Leu Phe Cys Leu Val Gly
    50                  55                  60

ccg agg ttg atg gcg aag cgc gag gcg ttc gac ccg aag ggg ttc atg      240
Pro Arg Leu Met Ala Lys Arg Glu Ala Phe Asp Pro Lys Gly Phe Met
65                  70                  75                  80

ctg gcg tac aat gcg tat cag acg gcg ttc aac gtc gtc gtg ctc ggg      288
```

```
Leu Ala Tyr Asn Ala Tyr Gln Thr Ala Phe Asn Val Val Val Leu Gly
                85                  90                  95

atg ttc gcg cga gag atc tcg ggg ctg ggg cag ccc gtg tgg ggg tca      336
Met Phe Ala Arg Glu Ile Ser Gly Leu Gly Gln Pro Val Trp Gly Ser
            100                 105                 110

acc atg ccg tgg agc gat aga aaa tcg ttt aag atc ctc ctc ggg gtg      384
Thr Met Pro Trp Ser Asp Arg Lys Ser Phe Lys Ile Leu Leu Gly Val
        115                 120                 125

tgg ttg cac tac aac aac caa tat ttg gag cta ttg gac act gtg ttc      432
Trp Leu His Tyr Asn Asn Gln Tyr Leu Glu Leu Leu Asp Thr Val Phe
    130                 135                 140

atg gtt gcg cgc aag aag acg aag cag ttg agc ttc ttg cac gtt tat      480
Met Val Ala Arg Lys Lys Thr Lys Gln Leu Ser Phe Leu His Val Tyr
145                 150                 155                 160

cat cac gcc ctg ttg atc tgg gcg tgg tgg ttg gtg tgt cac ttg atg      528
His His Ala Leu Leu Ile Trp Ala Trp Trp Leu Val Cys His Leu Met
                165                 170                 175

gcc acg aac gat tgt atc gat gcc tac ttc ggc gcg gcg tgc aac tcg      576
Ala Thr Asn Asp Cys Ile Asp Ala Tyr Phe Gly Ala Ala Cys Asn Ser
            180                 185                 190

ttc att cac atc gtg atg tac tcg tat tat ctc atg tcg gcg ctc ggc      624
Phe Ile His Ile Val Met Tyr Ser Tyr Tyr Leu Met Ser Ala Leu Gly
        195                 200                 205

att cga tgc ccg tgg aag cga tac atc acc cag gct caa atg ctc caa      672
Ile Arg Cys Pro Trp Lys Arg Tyr Ile Thr Gln Ala Gln Met Leu Gln
    210                 215                 220

ttc gtc att gtc ttc gcg cac gcc gtg ttc gtg ctg cgt cag aag cac      720
Phe Val Ile Val Phe Ala His Ala Val Phe Val Leu Arg Gln Lys His
225                 230                 235                 240

tgc ccg gtc acc ctt cct tgg gcg caa atg ttc gtc atg acg aac atg      768
Cys Pro Val Thr Leu Pro Trp Ala Gln Met Phe Val Met Thr Asn Met
                245                 250                 255

ctc gtg ctc ttc ggg aac ttc tac ctc aag gcg tac tcg aac aag tcg      816
Leu Val Leu Phe Gly Asn Phe Tyr Leu Lys Ala Tyr Ser Asn Lys Ser
            260                 265                 270

cgc ggc gac ggc gcg agt tcc gtg aaa cca gcc gag acc acg cgc gcg      864
Arg Gly Asp Gly Ala Ser Ser Val Lys Pro Ala Glu Thr Thr Arg Ala
        275                 280                 285

ccc agc gtg cga cgc acg cga tct cga aaa att gac taa                  903
Pro Ser Val Arg Arg Thr Arg Ser Arg Lys Ile Asp
    290                 295                 300
```

<210> 80
<211> 300
<212> PRT
<213> Ostreococcus tauri

<400> 80

Met Ser Ala Ser Gly Ala Leu Leu Pro Ala Ile Ala Ser Ala Ala Tyr
1 5 10 15

Ala Tyr Ala Thr Tyr Ala Tyr Ala Phe Glu Trp Ser His Ala Asn Gly
20 25 30

Ile Asp Asn Val Asp Ala Arg Glu Trp Ile Gly Ala Leu Ser Leu Arg
35 40 45

Leu Pro Ala Ile Ala Thr Thr Met Tyr Leu Leu Phe Cys Leu Val Gly
50 55 60

Pro Arg Leu Met Ala Lys Arg Glu Ala Phe Asp Pro Lys Gly Phe Met
65 70 75 80

Leu Ala Tyr Asn Ala Tyr Gln Thr Ala Phe Asn Val Val Val Leu Gly
85 90 95

Met Phe Ala Arg Glu Ile Ser Gly Leu Gly Gln Pro Val Trp Gly Ser
100 105 110

Thr Met Pro Trp Ser Asp Arg Lys Ser Phe Lys Ile Leu Leu Gly Val
115 120 125

Trp Leu His Tyr Asn Asn Gln Tyr Leu Glu Leu Leu Asp Thr Val Phe
130 135 140

Met Val Ala Arg Lys Lys Thr Lys Gln Leu Ser Phe Leu His Val Tyr
145 150 155 160

His His Ala Leu Leu Ile Trp Ala Trp Trp Leu Val Cys His Leu Met
165 170 175

Ala Thr Asn Asp Cys Ile Asp Ala Tyr Phe Gly Ala Ala Cys Asn Ser
180 185 190

Phe Ile His Ile Val Met Tyr Ser Tyr Tyr Leu Met Ser Ala Leu Gly
195 200 205

Ile Arg Cys Pro Trp Lys Arg Tyr Ile Thr Gln Ala Gln Met Leu Gln
210 215 220

Phe Val Ile Val Phe Ala His Ala Val Phe Val Leu Arg Gln Lys His
225 230 235 240

Cys Pro Val Thr Leu Pro Trp Ala Gln Met Phe Val Met Thr Asn Met
245 250 255

Leu Val Leu Phe Gly Asn Phe Tyr Leu Lys Ala Tyr Ser Asn Lys Ser
260 265 270

```
Arg Gly Asp Gly Ala Ser Ser Val Lys Pro Ala Glu Thr Thr Arg Ala
        275                 280                 285

Pro Ser Val Arg Arg Thr Arg Ser Arg Lys Ile Asp
    290                 295                 300
```

<210> 81
<211> 879
<212> DNA
<213> Ostreococcus tauri

<220>
<221> CDS
<222> (1)..(879)
<223> Delta-6-Elongase

<400> 81

```
atg agt ggc tta cgt gca ccc aac ttt tta cac aga ttc tgg aca aag        48
Met Ser Gly Leu Arg Ala Pro Asn Phe Leu His Arg Phe Trp Thr Lys
1               5                   10                  15

tgg gac tac gcg att tcc aaa gtc gtc ttc acg tgt gcc gac agt ttt        96
Trp Asp Tyr Ala Ile Ser Lys Val Val Phe Thr Cys Ala Asp Ser Phe
            20                  25                  30

cag tgg gac atc ggg cca gtg agt tcg agt acg gcg cat tta ccc gcc       144
Gln Trp Asp Ile Gly Pro Val Ser Ser Ser Thr Ala His Leu Pro Ala
        35                  40                  45

att gaa tcc cct acc cca ctg gtg act agc ctc ttg ttc tac tta gtc       192
Ile Glu Ser Pro Thr Pro Leu Val Thr Ser Leu Leu Phe Tyr Leu Val
    50                  55                  60

aca gtt ttc ttg tgg tat ggt cgt tta acc agg agt tca gac aag aaa       240
Thr Val Phe Leu Trp Tyr Gly Arg Leu Thr Arg Ser Ser Asp Lys Lys
65                  70                  75                  80

att aga gag cct acg tgg tta aga aga ttc ata ata tgt cat aat gcg       288
Ile Arg Glu Pro Thr Trp Leu Arg Arg Phe Ile Ile Cys His Asn Ala
                85                  90                  95

ttc ttg ata gtc ctc agt ctt tac atg tgc ctt ggt tgt gtg gcc caa       336
Phe Leu Ile Val Leu Ser Leu Tyr Met Cys Leu Gly Cys Val Ala Gln
            100                 105                 110

gcg tat cag aat gga tat act tta tgg ggt aat gaa ttc aag gcc acg       384
Ala Tyr Gln Asn Gly Tyr Thr Leu Trp Gly Asn Glu Phe Lys Ala Thr
        115                 120                 125

gaa act cag ctt gct ctc tac att tac att ttt tac gta agt aaa ata       432
Glu Thr Gln Leu Ala Leu Tyr Ile Tyr Ile Phe Tyr Val Ser Lys Ile
    130                 135                 140

tac gag ttt gta gat act tac att atg ctt ctc aag aat aac ttg cgg       480
Tyr Glu Phe Val Asp Thr Tyr Ile Met Leu Leu Lys Asn Asn Leu Arg
145                 150                 155                 160
```

```
caa gta aga ttc cta cac act tat cac cac agc acg att tcc ttt att      528
Gln Val Arg Phe Leu His Thr Tyr His His Ser Thr Ile Ser Phe Ile
                165                 170                 175

tgg tgg atc att gct cgg agg gct ccg ggt ggt gat gct tac ttc agc      576
Trp Trp Ile Ile Ala Arg Arg Ala Pro Gly Gly Asp Ala Tyr Phe Ser
            180                 185                 190

gcg gcc ttg aac tca tgg gta cac gtg tgc atg tac acc tat tat cta      624
Ala Ala Leu Asn Ser Trp Val His Val Cys Met Tyr Thr Tyr Tyr Leu
        195                 200                 205

tta tca acc ctt att gga aaa gaa gat cct aag cgt tcc aac tac ctt      672
Leu Ser Thr Leu Ile Gly Lys Glu Asp Pro Lys Arg Ser Asn Tyr Leu
    210                 215                 220

tgg tgg ggt cgc cac cta acg caa atg cag atg ctt cag ttt ttc ttc      720
Trp Trp Gly Arg His Leu Thr Gln Met Gln Met Leu Gln Phe Phe Phe
225                 230                 235                 240

aac gta ctt caa gcg ttg tac tgc gct tcg ttc tct acg tat ccc aag      768
Asn Val Leu Gln Ala Leu Tyr Cys Ala Ser Phe Ser Thr Tyr Pro Lys
                245                 250                 255

ttt ttg tcc aaa att ctg ctc gtc tat atg atg agc ctt ctc ggc ttg      816
Phe Leu Ser Lys Ile Leu Leu Val Tyr Met Met Ser Leu Leu Gly Leu
            260                 265                 270

ttt ggg cat ttc tac tat tcc aag cac ata gca gca gct aag ctc cag      864
Phe Gly His Phe Tyr Tyr Ser Lys His Ile Ala Ala Ala Lys Leu Gln
        275                 280                 285

aaa aaa cag cag tga                                                  879
Lys Lys Gln Gln
    290
```

<210> 82
<211> 292
<212> PRT
<213> Ostreococcus tauri

<400> 82

```
Met Ser Gly Leu Arg Ala Pro Asn Phe Leu His Arg Phe Trp Thr Lys
1               5                   10                  15

Trp Asp Tyr Ala Ile Ser Lys Val Val Phe Thr Cys Ala Asp Ser Phe
            20                  25                  30

Gln Trp Asp Ile Gly Pro Val Ser Ser Ser Thr Ala His Leu Pro Ala
        35                  40                  45

Ile Glu Ser Pro Thr Pro Leu Val Thr Ser Leu Leu Phe Tyr Leu Val
    50                  55                  60

Thr Val Phe Leu Trp Tyr Gly Arg Leu Thr Arg Ser Ser Asp Lys Lys
65                  70                  75                  80
```

```
Ile Arg Glu Pro Thr Trp Leu Arg Arg Phe Ile Ile Cys His Asn Ala
                85                  90                  95

Phe Leu Ile Val Leu Ser Leu Tyr Met Cys Leu Gly Cys Val Ala Gln
            100                 105                 110

Ala Tyr Gln Asn Gly Tyr Thr Leu Trp Gly Asn Glu Phe Lys Ala Thr
        115                 120                 125

Glu Thr Gln Leu Ala Leu Tyr Ile Tyr Ile Phe Tyr Val Ser Lys Ile
    130                 135                 140

Tyr Glu Phe Val Asp Thr Tyr Ile Met Leu Leu Lys Asn Asn Leu Arg
145                 150                 155                 160

Gln Val Arg Phe Leu His Thr Tyr His His Ser Thr Ile Ser Phe Ile
                165                 170                 175

Trp Trp Ile Ile Ala Arg Arg Ala Pro Gly Gly Asp Ala Tyr Phe Ser
            180                 185                 190

Ala Ala Leu Asn Ser Trp Val His Val Cys Met Tyr Thr Tyr Tyr Leu
        195                 200                 205

Leu Ser Thr Leu Ile Gly Lys Glu Asp Pro Lys Arg Ser Asn Tyr Leu
    210                 215                 220

Trp Trp Gly Arg His Leu Thr Gln Met Gln Met Leu Gln Phe Phe Phe
225                 230                 235                 240

Asn Val Leu Gln Ala Leu Tyr Cys Ala Ser Phe Ser Thr Tyr Pro Lys
                245                 250                 255

Phe Leu Ser Lys Ile Leu Leu Val Tyr Met Met Ser Leu Leu Gly Leu
            260                 265                 270

Phe Gly His Phe Tyr Tyr Ser Lys His Ile Ala Ala Ala Lys Leu Gln
        275                 280                 285

Lys Lys Gln Gln
    290
```

<210> 83
<211> 831
<212> DNA
<213> Thraustochytrium sp.

<220>
<221> CDS
<222> (1)..(831)
<223> Delta-5-Elongase

<400> 83

```
atg gac gtc gtc gag cag caa tgg cgc cgc ttc gtg gac gcc gtg gac      48
Met Asp Val Val Glu Gln Gln Trp Arg Arg Phe Val Asp Ala Val Asp
1               5                   10                  15

aac gga atc gtg gag ttc atg gag cat gag aag ccc aac aag ctg aac      96
Asn Gly Ile Val Glu Phe Met Glu His Glu Lys Pro Asn Lys Leu Asn
            20                  25                  30

gag ggc aag ctc ttc acc tcg acc gag gag atg atg gcg ctt atc gtc      144
Glu Gly Lys Leu Phe Thr Ser Thr Glu Glu Met Met Ala Leu Ile Val
        35                  40                  45

ggc tac ctg gcg ttc gtg gtc ctc ggg tcc gcc ttc atg aag gcc ttt      192
Gly Tyr Leu Ala Phe Val Val Leu Gly Ser Ala Phe Met Lys Ala Phe
    50                  55                  60

gtc gat aag cct ttc gag ctc aag ttc ctc aag ctc gtg cac aac atc      240
Val Asp Lys Pro Phe Glu Leu Lys Phe Leu Lys Leu Val His Asn Ile
65                  70                  75                  80

ttc ctc acc ggt ctg tcc atg tac atg gcc acc gag tgc gcg cgc cag      288
Phe Leu Thr Gly Leu Ser Met Tyr Met Ala Thr Glu Cys Ala Arg Gln
                85                  90                  95

gca tac ctc ggc ggc tac aag ctc ttt ggc aac ccg atg gag aag ggc      336
Ala Tyr Leu Gly Gly Tyr Lys Leu Phe Gly Asn Pro Met Glu Lys Gly
            100                 105                 110

acc gag tcg cac gcc ccg ggc atg gcc aac atc atc tac atc ttc tac      384
Thr Glu Ser His Ala Pro Gly Met Ala Asn Ile Ile Tyr Ile Phe Tyr
        115                 120                 125

gtg agc aag ttc ctc gaa ttc ctc gac acc gtc ttc atg atc ctc ggc      432
Val Ser Lys Phe Leu Glu Phe Leu Asp Thr Val Phe Met Ile Leu Gly
    130                 135                 140

aag aag tgg aag cag ctc agc ttt ctc cac gtc tac cac cac gcg agc      480
Lys Lys Trp Lys Gln Leu Ser Phe Leu His Val Tyr His His Ala Ser
145                 150                 155                 160

atc agc ttc atc tgg ggc atc atc gcc cgc ttc gcg ccc ggt ggc gac      528
Ile Ser Phe Ile Trp Gly Ile Ile Ala Arg Phe Ala Pro Gly Gly Asp
                165                 170                 175

gcc tac ttc tct acc atc ctc aac agc agc gtg cat gtc gtg ctc tac      576
Ala Tyr Phe Ser Thr Ile Leu Asn Ser Ser Val His Val Val Leu Tyr
            180                 185                 190

ggc tac tac gcc tcg acc acc ctc ggc tac acc ttc atg cgc ccg ctg      624
Gly Tyr Tyr Ala Ser Thr Thr Leu Gly Tyr Thr Phe Met Arg Pro Leu
        195                 200                 205

cgc ccg tac att acc acc att cag ctc acg cag ttc atg gcc atg gtc      672
Arg Pro Tyr Ile Thr Thr Ile Gln Leu Thr Gln Phe Met Ala Met Val
    210                 215                 220

gtc cag tcc gtc tat gac tac tac aac ccc tgc gac tac ccg cag ccc      720
```

```
Val Gln Ser Val Tyr Asp Tyr Tyr Asn Pro Cys Asp Tyr Pro Gln Pro
225                 230                 235                 240

ctc gtc aag ctg ctc ttc tgg tac atg ctc acc atg ctc ggc ctc ttc      768
Leu Val Lys Leu Leu Phe Trp Tyr Met Leu Thr Met Leu Gly Leu Phe
                245                 250                 255

ggc aac ttc ttc gtg cag cag tac ctc aag ccc aag gcg ccc aag aag      816
Gly Asn Phe Phe Val Gln Gln Tyr Leu Lys Pro Lys Ala Pro Lys Lys
            260                 265                 270

cag aag acc atc taa                                                  831
Gln Lys Thr Ile
        275
```

<210> 84
<211> 276
<212> PRT
<213> Thraustochytrium sp.

<400> 84

```
Met Asp Val Val Glu Gln Gln Trp Arg Arg Phe Val Asp Ala Val Asp
1               5                   10                  15

Asn Gly Ile Val Glu Phe Met Glu His Glu Lys Pro Asn Lys Leu Asn
            20                  25                  30

Glu Gly Lys Leu Phe Thr Ser Thr Glu Glu Met Met Ala Leu Ile Val
        35                  40                  45

Gly Tyr Leu Ala Phe Val Val Leu Gly Ser Ala Phe Met Lys Ala Phe
    50                  55                  60

Val Asp Lys Pro Phe Glu Leu Lys Phe Leu Lys Leu Val His Asn Ile
65                  70                  75                  80

Phe Leu Thr Gly Leu Ser Met Tyr Met Ala Thr Glu Cys Ala Arg Gln
                85                  90                  95

Ala Tyr Leu Gly Gly Tyr Lys Leu Phe Gly Asn Pro Met Glu Lys Gly
            100                 105                 110

Thr Glu Ser His Ala Pro Gly Met Ala Asn Ile Ile Tyr Ile Phe Tyr
        115                 120                 125

Val Ser Lys Phe Leu Glu Phe Leu Asp Thr Val Phe Met Ile Leu Gly
    130                 135                 140

Lys Lys Trp Lys Gln Leu Ser Phe Leu His Val Tyr His His Ala Ser
145                 150                 155                 160
```

```
Ile Ser Phe Ile Trp Gly Ile Ile Ala Arg Phe Ala Pro Gly Gly Asp
                165                 170                 175

Ala Tyr Phe Ser Thr Ile Leu Asn Ser Ser Val His Val Val Leu Tyr
            180                 185                 190

Gly Tyr Tyr Ala Ser Thr Thr Leu Gly Tyr Thr Phe Met Arg Pro Leu
        195                 200                 205

Arg Pro Tyr Ile Thr Thr Ile Gln Leu Thr Gln Phe Met Ala Met Val
    210                 215                 220

Val Gln Ser Val Tyr Asp Tyr Tyr Asn Pro Cys Asp Tyr Pro Gln Pro
225                 230                 235                 240

Leu Val Lys Leu Leu Phe Trp Tyr Met Leu Thr Met Leu Gly Leu Phe
                245                 250                 255

Gly Asn Phe Phe Val Gln Gln Tyr Leu Lys Pro Lys Ala Pro Lys Lys
            260                 265                 270

Gln Lys Thr Ile
        275
```

<210> 85
<211> 1077
<212> DNA
<213> Thalassiosira pseudonana

<220>
<221> CDS
<222> (1)..(1077)
<223> Delta-5-Elongase

<400> 85

```
atg tgc tca cca ccg ccg tca caa tcc aaa aca aca tcc ctc cta gca      48
Met Cys Ser Pro Pro Pro Ser Gln Ser Lys Thr Thr Ser Leu Leu Ala
1               5                   10                  15

cgg tac acc acc gcc gcc ctc ctc ctc ctc acc ctc aca acg tgg tgc      96
Arg Tyr Thr Thr Ala Ala Leu Leu Leu Leu Thr Leu Thr Thr Trp Cys
            20                  25                  30

cac ttc gcc ttc cca gcc gcc acc gcc aca ccc ggc ctc acc gcc gaa     144
His Phe Ala Phe Pro Ala Ala Thr Ala Thr Pro Gly Leu Thr Ala Glu
        35                  40                  45

atg cac tcc tac aaa gtc cca ctc ggt ctc acc gta ttc tac ctg ctg     192
Met His Ser Tyr Lys Val Pro Leu Gly Leu Thr Val Phe Tyr Leu Leu
    50                  55                  60
```

```
agt cta ccg tca cta aag tac gtt acg gac aac tac ctt gcc aaa aag      240
Ser Leu Pro Ser Leu Lys Tyr Val Thr Asp Asn Tyr Leu Ala Lys Lys
65                  70                  75                  80

tat gat atg aag tca ctc ctg acg gaa tca atg gtg ttg tac aat gtg      288
Tyr Asp Met Lys Ser Leu Leu Thr Glu Ser Met Val Leu Tyr Asn Val
                85                  90                  95

gcg caa gtg ctg ctc aat ggg tgg acg gtg tat gcg att gtg gat gcg      336
Ala Gln Val Leu Leu Asn Gly Trp Thr Val Tyr Ala Ile Val Asp Ala
            100                 105                 110

gtg atg aat aga gac cat cct ttt att gga agt aga agt ttg gtt ggg      384
Val Met Asn Arg Asp His Pro Phe Ile Gly Ser Arg Ser Leu Val Gly
        115                 120                 125

gcg gcg ttg cat agt ggg agc tcg tat gcg gtg tgg gtt cat tat tgt      432
Ala Ala Leu His Ser Gly Ser Ser Tyr Ala Val Trp Val His Tyr Cys
    130                 135                 140

gat aag tat ttg gag ttc ttt gat acg tat ttt atg gtg ttg agg ggg      480
Asp Lys Tyr Leu Glu Phe Phe Asp Thr Tyr Phe Met Val Leu Arg Gly
145                 150                 155                 160

aaa atg gac cag gtc tcc ttc ctc cac atc tac cac cac acg acc ata      528
Lys Met Asp Gln Val Ser Phe Leu His Ile Tyr His His Thr Thr Ile
                165                 170                 175

gcg tgg gca tgg tgg atc gcc ctc cgc ttc tcc ccc ggc gga gac att      576
Ala Trp Ala Trp Trp Ile Ala Leu Arg Phe Ser Pro Gly Gly Asp Ile
            180                 185                 190

tac ttc ggg gca ctc ctc aac tcc atc atc cac gtc ctc atg tat tcc      624
Tyr Phe Gly Ala Leu Leu Asn Ser Ile Ile His Val Leu Met Tyr Ser
        195                 200                 205

tac tac gcc ctt gcc cta ctc aag gtc agt tgt cca tgg aaa cga tac      672
Tyr Tyr Ala Leu Ala Leu Leu Lys Val Ser Cys Pro Trp Lys Arg Tyr
    210                 215                 220

ttg act caa gct caa tta ttg caa ttc aca agt gtg gtg gtt tat acg      720
Leu Thr Gln Ala Gln Leu Leu Gln Phe Thr Ser Val Val Val Tyr Thr
225                 230                 235                 240

ggg tgt acg ggt tat act cat tac tat cat acg aag cat gga gcg gat      768
Gly Cys Thr Gly Tyr Thr His Tyr Tyr His Thr Lys His Gly Ala Asp
                245                 250                 255

gag aca cag cct agt tta gga acg tat tat ttc tgt tgt gga gtg cag      816
Glu Thr Gln Pro Ser Leu Gly Thr Tyr Tyr Phe Cys Cys Gly Val Gln
            260                 265                 270

gtg ttt gag atg gtt agt ttg ttt gta ctc ttt tcc atc ttt tat aaa      864
Val Phe Glu Met Val Ser Leu Phe Val Leu Phe Ser Ile Phe Tyr Lys
        275                 280                 285

cga tcc tat tcg aag aag aac aag tca gga gga aag gat agc aag aag      912
Arg Ser Tyr Ser Lys Lys Asn Lys Ser Gly Gly Lys Asp Ser Lys Lys
    290                 295                 300

aat gat gat ggg aat aat gag gat caa tgt cac aag gct atg aag gat      960
Asn Asp Asp Gly Asn Asn Glu Asp Gln Cys His Lys Ala Met Lys Asp
305                 310                 315                 320

ata tcg gag ggt gcg aag gag gtt gtg ggg cat gca gcg aag gat gct     1008
Ile Ser Glu Gly Ala Lys Glu Val Val Gly His Ala Ala Lys Asp Ala
                325                 330                 335
```

```
gga aag ttg gtg gct acg gcg agt aag gct gta aag agg aag gga act      1056
Gly Lys Leu Val Ala Thr Ala Ser Lys Ala Val Lys Arg Lys Gly Thr
            340                 345                 350

cgt gtt act ggt gcc atg tag                                          1077
Arg Val Thr Gly Ala Met
        355
```

<210> 86
<211> 358
<212> PRT
<213> Thalassiosira pseudonana

<400> 86

```
Met Cys Ser Pro Pro Pro Ser Gln Ser Lys Thr Thr Ser Leu Leu Ala
1               5                   10                  15

Arg Tyr Thr Thr Ala Ala Leu Leu Leu Leu Thr Leu Thr Thr Trp Cys
            20                  25                  30

His Phe Ala Phe Pro Ala Ala Thr Ala Thr Pro Gly Leu Thr Ala Glu
        35                  40                  45

Met His Ser Tyr Lys Val Pro Leu Gly Leu Thr Val Phe Tyr Leu Leu
    50                  55                  60

Ser Leu Pro Ser Leu Lys Tyr Val Thr Asp Asn Tyr Leu Ala Lys Lys
65                  70                  75                  80

Tyr Asp Met Lys Ser Leu Leu Thr Glu Ser Met Val Leu Tyr Asn Val
                85                  90                  95

Ala Gln Val Leu Leu Asn Gly Trp Thr Val Tyr Ala Ile Val Asp Ala
            100                 105                 110

Val Met Asn Arg Asp His Pro Phe Ile Gly Ser Arg Ser Leu Val Gly
        115                 120                 125

Ala Ala Leu His Ser Gly Ser Ser Tyr Ala Val Trp Val His Tyr Cys
    130                 135                 140

Asp Lys Tyr Leu Glu Phe Phe Asp Thr Tyr Phe Met Val Leu Arg Gly
145                 150                 155                 160

Lys Met Asp Gln Val Ser Phe Leu His Ile Tyr His His Thr Thr Ile
                165                 170                 175

Ala Trp Ala Trp Trp Ile Ala Leu Arg Phe Ser Pro Gly Gly Asp Ile
            180                 185                 190
```

Tyr Phe Gly Ala Leu Leu Asn Ser Ile Ile His Val Leu Met Tyr Ser
195 200 205

Tyr Tyr Ala Leu Ala Leu Leu Lys Val Ser Cys Pro Trp Lys Arg Tyr
210 215 220

Leu Thr Gln Ala Gln Leu Leu Gln Phe Thr Ser Val Val Val Tyr Thr
225 230 235 240

Gly Cys Thr Gly Tyr Thr His Tyr Tyr His Thr Lys His Gly Ala Asp
245 250 255

Glu Thr Gln Pro Ser Leu Gly Thr Tyr Tyr Phe Cys Cys Gly Val Gln
260 265 270

Val Phe Glu Met Val Ser Leu Phe Val Leu Phe Ser Ile Phe Tyr Lys
275 280 285

Arg Ser Tyr Ser Lys Lys Asn Lys Ser Gly Gly Lys Asp Ser Lys Lys
290 295 300

Asn Asp Asp Gly Asn Asn Glu Asp Gln Cys His Lys Ala Met Lys Asp
305 310 315 320

Ile Ser Glu Gly Ala Lys Glu Val Val Gly His Ala Ala Lys Asp Ala
325 330 335

Gly Lys Leu Val Ala Thr Ala Ser Lys Ala Val Lys Arg Lys Gly Thr
340 345 350

Arg Val Thr Gly Ala Met
355

<210> 87
<211> 1086
<212> DNA
<213> Phytophthora infestans

<220>
<221> CDS
<222> (1)..(1086)
<223> Omega-3-Desaturase

<400> 87

atg gcg acg aag gag gcg tat gtg ttc ccc act ctg acg gag atc aag 48

```
Met Ala Thr Lys Glu Ala Tyr Val Phe Pro Thr Leu Thr Glu Ile Lys
1               5                   10                  15

cgg tcg cta cct aaa gac tgt ttc gag gct tcg gtg cct ctg tcg ctc        96
Arg Ser Leu Pro Lys Asp Cys Phe Glu Ala Ser Val Pro Leu Ser Leu
            20                  25                  30

tac tac acc gtg cgt tgt ctg gtg atc gcg gtg gct cta acc ttc ggt       144
Tyr Tyr Thr Val Arg Cys Leu Val Ile Ala Val Ala Leu Thr Phe Gly
        35                  40                  45

ctc aac tac gct cgc gct ctg ccc gag gtc gag agc ttc tgg gct ctg       192
Leu Asn Tyr Ala Arg Ala Leu Pro Glu Val Glu Ser Phe Trp Ala Leu
    50                  55                  60

gac gcc gca ctc tgc acg ggc tac atc ttg ctg cag ggc atc gtg ttc       240
Asp Ala Ala Leu Cys Thr Gly Tyr Ile Leu Leu Gln Gly Ile Val Phe
65                  70                  75                  80

tgg ggc ttc ttc acg gtg ggc cac gat gcc ggc cac ggc gcc ttc tcg       288
Trp Gly Phe Phe Thr Val Gly His Asp Ala Gly His Gly Ala Phe Ser
                85                  90                  95

cgc tac cac ctg ctt aac ttc gtg gtg ggc act ttc atg cac tcg ctc       336
Arg Tyr His Leu Leu Asn Phe Val Val Gly Thr Phe Met His Ser Leu
            100                 105                 110

atc ctc acg ccc ttc gag tcg tgg aag ctc acg cac cgt cac cac cac       384
Ile Leu Thr Pro Phe Glu Ser Trp Lys Leu Thr His Arg His His His
        115                 120                 125

aag aac acg ggc aac att gac cgt gac gag gtc ttc tac ccg caa cgc       432
Lys Asn Thr Gly Asn Ile Asp Arg Asp Glu Val Phe Tyr Pro Gln Arg
    130                 135                 140

aag gcc gac gac cac ccg ctg tct cgc aac ctg att ctg gcg ctc ggg       480
Lys Ala Asp Asp His Pro Leu Ser Arg Asn Leu Ile Leu Ala Leu Gly
145                 150                 155                 160

gca gcg tgg ctc gcc tat ttg gtc gag ggc ttc cct cct cgt aag gtc       528
Ala Ala Trp Leu Ala Tyr Leu Val Glu Gly Phe Pro Pro Arg Lys Val
                165                 170                 175

aac cac ttc aac ccg ttc gag cct ctg ttc gtg cgt cag gtg tca gct       576
Asn His Phe Asn Pro Phe Glu Pro Leu Phe Val Arg Gln Val Ser Ala
            180                 185                 190

gtg gta atc tct ctt ctc gcc cac ttc ttc gtg gcc gga ctc tcc atc       624
Val Val Ile Ser Leu Leu Ala His Phe Phe Val Ala Gly Leu Ser Ile
        195                 200                 205

tat ctg agc ctc cag ctg ggc ctt aag acg atg gca atc tac tac tat       672
Tyr Leu Ser Leu Gln Leu Gly Leu Lys Thr Met Ala Ile Tyr Tyr Tyr
    210                 215                 220

gga cct gtt ttt gtg ttc ggc agc atg ctg gtc att acc acc ttc cta       720
Gly Pro Val Phe Val Phe Gly Ser Met Leu Val Ile Thr Thr Phe Leu
225                 230                 235                 240

cac cac aat gat gag gag acc cca tgg tac gcc gac tcg gag tgg acg       768
His His Asn Asp Glu Glu Thr Pro Trp Tyr Ala Asp Ser Glu Trp Thr
                245                 250                 255

tac gtc aag ggc aac ctc tcg tcc gtg gac cga tcg tac ggc gcg ctc       816
Tyr Val Lys Gly Asn Leu Ser Ser Val Asp Arg Ser Tyr Gly Ala Leu
            260                 265                 270

att gac aac ctg agc cac aac atc ggc acg cac cag atc cac cac ctt       864
```

```
Ile Asp Asn Leu Ser His Asn Ile Gly Thr His Gln Ile His His Leu
        275                 280                 285

ttc cct atc att ccg cac tac aaa ctc aag aaa gcc act gcg gcc ttc      912
Phe Pro Ile Ile Pro His Tyr Lys Leu Lys Lys Ala Thr Ala Ala Phe
    290                 295                 300

cac cag gct ttc cct gag ctc gtg cgc aag agc gac gag cca att atc      960
His Gln Ala Phe Pro Glu Leu Val Arg Lys Ser Asp Glu Pro Ile Ile
305                 310                 315                     320

aag gct ttc ttc cgg gtt gga cgt ctc tac gca aac tac ggc gtt gtg     1008
Lys Ala Phe Phe Arg Val Gly Arg Leu Tyr Ala Asn Tyr Gly Val Val
                325                 330                 335

gac cag gag gcg aag ctc ttc acg cta aag gaa gcc aag gcg gcg acc     1056
Asp Gln Glu Ala Lys Leu Phe Thr Leu Lys Glu Ala Lys Ala Ala Thr
            340                 345                 350

gag gcg gcg gcc aag acc aag tcc acg taa                             1086
Glu Ala Ala Ala Lys Thr Lys Ser Thr
        355                 360
```

<210> 88
<211> 361
<212> PRT
<213> Phytophthora infestans

<400> 88

```
Met Ala Thr Lys Glu Ala Tyr Val Phe Pro Thr Leu Thr Glu Ile Lys
1               5                   10                  15

Arg Ser Leu Pro Lys Asp Cys Phe Glu Ala Ser Val Pro Leu Ser Leu
            20                  25                  30

Tyr Tyr Thr Val Arg Cys Leu Val Ile Ala Val Ala Leu Thr Phe Gly
        35                  40                  45

Leu Asn Tyr Ala Arg Ala Leu Pro Glu Val Glu Ser Phe Trp Ala Leu
    50                  55                  60

Asp Ala Ala Leu Cys Thr Gly Tyr Ile Leu Leu Gln Gly Ile Val Phe
65                  70                  75                  80

Trp Gly Phe Phe Thr Val Gly His Asp Ala Gly His Gly Ala Phe Ser
                85                  90                  95

Arg Tyr His Leu Leu Asn Phe Val Val Gly Thr Phe Met His Ser Leu
            100                 105                 110

Ile Leu Thr Pro Phe Glu Ser Trp Lys Leu Thr His Arg His His His
        115                 120                 125
```

```
Lys Asn Thr Gly Asn Ile Asp Arg Asp Glu Val Phe Tyr Pro Gln Arg
    130                 135                 140

Lys Ala Asp Asp His Pro Leu Ser Arg Asn Leu Ile Leu Ala Leu Gly
145                 150                 155                 160

Ala Ala Trp Leu Ala Tyr Leu Val Glu Gly Phe Pro Pro Arg Lys Val
                165                 170                 175

Asn His Phe Asn Pro Phe Glu Pro Leu Phe Val Arg Gln Val Ser Ala
            180                 185                 190

Val Val Ile Ser Leu Leu Ala His Phe Phe Val Ala Gly Leu Ser Ile
        195                 200                 205

Tyr Leu Ser Leu Gln Leu Gly Leu Lys Thr Met Ala Ile Tyr Tyr Tyr
    210                 215                 220

Gly Pro Val Phe Val Phe Gly Ser Met Leu Val Ile Thr Thr Phe Leu
225                 230                 235                 240

His His Asn Asp Glu Glu Thr Pro Trp Tyr Ala Asp Ser Glu Trp Thr
                245                 250                 255

Tyr Val Lys Gly Asn Leu Ser Ser Val Asp Arg Ser Tyr Gly Ala Leu
            260                 265                 270

Ile Asp Asn Leu Ser His Asn Ile Gly Thr His Gln Ile His His Leu
        275                 280                 285

Phe Pro Ile Ile Pro His Tyr Lys Leu Lys Lys Ala Thr Ala Ala Phe
    290                 295                 300

His Gln Ala Phe Pro Glu Leu Val Arg Lys Ser Asp Glu Pro Ile Ile
305                 310                 315                 320

Lys Ala Phe Phe Arg Val Gly Arg Leu Tyr Ala Asn Tyr Gly Val Val
                325                 330                 335

Asp Gln Glu Ala Lys Leu Phe Thr Leu Lys Glu Ala Lys Ala Ala Thr
            340                 345                 350

Glu Ala Ala Ala Lys Thr Lys Ser Thr
        355                 360
```

<210> 89
<211> 1371
<212> DNA
<213> Ostreococcus tauri

<220>

<221> CDS
<222> (1)..(1371)
<223> Delta-6-Desaturase

<400> 89

```
atg tgc gtg gag acg gaa aat aac gat ggg atc ccc acg gtg gag atc      48
Met Cys Val Glu Thr Glu Asn Asn Asp Gly Ile Pro Thr Val Glu Ile
1               5                   10                  15

gcg ttc gac ggt gag cgc gag cgg gcg gag gca aac gtg aag ctg tcc      96
Ala Phe Asp Gly Glu Arg Glu Arg Ala Glu Ala Asn Val Lys Leu Ser
            20                  25                  30

gcg gag aag atg gag ccg gcg gcg ctg gcg aag acg ttc gcg agg cgg     144
Ala Glu Lys Met Glu Pro Ala Ala Leu Ala Lys Thr Phe Ala Arg Arg
        35                  40                  45

tac gtc gtg atc gag ggg gtg gag tac gat gtg acg gat ttt aag cac     192
Tyr Val Val Ile Glu Gly Val Glu Tyr Asp Val Thr Asp Phe Lys His
    50                  55                  60

ccg gga gga acg gtt att ttc tat gcg ttg tca aac acc ggg gcg gac     240
Pro Gly Gly Thr Val Ile Phe Tyr Ala Leu Ser Asn Thr Gly Ala Asp
65                  70                  75                  80

gcg acg gaa gcg ttc aag gag ttt cat cat cgg tcg aga aag gcg agg     288
Ala Thr Glu Ala Phe Lys Glu Phe His His Arg Ser Arg Lys Ala Arg
                85                  90                  95

aaa gcc ttg gcg gcg ctc ccg tct cga ccg gcc aag acg gcc aag gtg     336
Lys Ala Leu Ala Ala Leu Pro Ser Arg Pro Ala Lys Thr Ala Lys Val
            100                 105                 110

gac gac gcg gag atg ctc caa gat ttc gcc aag tgg cgg aaa gaa ttg     384
Asp Asp Ala Glu Met Leu Gln Asp Phe Ala Lys Trp Arg Lys Glu Leu
        115                 120                 125

gag aga gat gga ttc ttc aag ccc tct ccg gcg cac gtg gcg tat cgc     432
Glu Arg Asp Gly Phe Phe Lys Pro Ser Pro Ala His Val Ala Tyr Arg
    130                 135                 140

ttc gcc gag ctc gcg gcg atg tac gct ctc ggg acg tac ctg atg tac     480
Phe Ala Glu Leu Ala Ala Met Tyr Ala Leu Gly Thr Tyr Leu Met Tyr
145                 150                 155                 160

gct cga tac gtc gtc tcc tcg gtg ctc gtg tac gct tgc ttt ttc ggc     528
Ala Arg Tyr Val Val Ser Ser Val Leu Val Tyr Ala Cys Phe Phe Gly
                165                 170                 175

gcc cga tgc ggt tgg gtg cag cac gag ggc gga cac agc tcg ctg acg     576
Ala Arg Cys Gly Trp Val Gln His Glu Gly Gly His Ser Ser Leu Thr
            180                 185                 190

ggc aac att tgg tgg gac aag cgc atc cag gcc ttc aca gcc ggg ttc     624
Gly Asn Ile Trp Trp Asp Lys Arg Ile Gln Ala Phe Thr Ala Gly Phe
        195                 200                 205

ggt ctc gcc ggt agc ggc gac atg tgg aac tcg atg cac aac aag cat     672
Gly Leu Ala Gly Ser Gly Asp Met Trp Asn Ser Met His Asn Lys His
    210                 215                 220
```

```
cac gcg acg cct caa aag gtt cgt cac gac atg gat ctg gac acc acc      720
His Ala Thr Pro Gln Lys Val Arg His Asp Met Asp Leu Asp Thr Thr
225                 230                 235                 240

ccc gcg gtg gcg ttc ttc aac acc gcg gtg gaa gac aat cgt ccc cgt      768
Pro Ala Val Ala Phe Phe Asn Thr Ala Val Glu Asp Asn Arg Pro Arg
                245                 250                 255

ggc ttt agc aag tac tgg ttg cgc ctt cag gcg tgg acc ttc atc ccc      816
Gly Phe Ser Lys Tyr Trp Leu Arg Leu Gln Ala Trp Thr Phe Ile Pro
            260                 265                 270

gtg acg tcc ggc ttg gtg ctc ctt ttc tgg atg ttt ttc ctc cac ccc      864
Val Thr Ser Gly Leu Val Leu Leu Phe Trp Met Phe Phe Leu His Pro
        275                 280                 285

tcc aag gct ttg aag ggt ggc aag tac gaa gag ttg gtg tgg atg ctc      912
Ser Lys Ala Leu Lys Gly Gly Lys Tyr Glu Glu Leu Val Trp Met Leu
    290                 295                 300

gcc gcg cac gtc atc cgc acg tgg acg atc aag gcg gtg acc gga ttc      960
Ala Ala His Val Ile Arg Thr Trp Thr Ile Lys Ala Val Thr Gly Phe
305                 310                 315                 320

acc gcg atg cag tcc tac ggc tta ttt ttg gcg acg agc tgg gtg agc      1008
Thr Ala Met Gln Ser Tyr Gly Leu Phe Leu Ala Thr Ser Trp Val Ser
                325                 330                 335

ggc tgc tat ctg ttt gca cac ttc tcc acg tcg cac acg cac ctg gat      1056
Gly Cys Tyr Leu Phe Ala His Phe Ser Thr Ser His Thr His Leu Asp
            340                 345                 350

gtg gtg ccc gcg gac gag cat ctc tcc tgg gtt cga tac gcc gtc gat      1104
Val Val Pro Ala Asp Glu His Leu Ser Trp Val Arg Tyr Ala Val Asp
        355                 360                 365

cac acg atc gac atc gat ccg agt caa ggt tgg gtg aac tgg ttg atg      1152
His Thr Ile Asp Ile Asp Pro Ser Gln Gly Trp Val Asn Trp Leu Met
    370                 375                 380

ggc tac ctc aac tgc caa gtc atc cac cac ctc ttt ccg agc atg ccg      1200
Gly Tyr Leu Asn Cys Gln Val Ile His His Leu Phe Pro Ser Met Pro
385                 390                 395                 400

cag ttc cgc cag ccc gag gta tct cgc cgc ttc gtc gcc ttt gcg aaa      1248
Gln Phe Arg Gln Pro Glu Val Ser Arg Arg Phe Val Ala Phe Ala Lys
                405                 410                 415

aag tgg aac ctc aac tac aag gtc atg acc tac gcc ggt gcg tgg aag      1296
Lys Trp Asn Leu Asn Tyr Lys Val Met Thr Tyr Ala Gly Ala Trp Lys
            420                 425                 430

gca acg ctc gga aac ctc gac aac gtg ggt aag cac tac tac gtg cac      1344
Ala Thr Leu Gly Asn Leu Asp Asn Val Gly Lys His Tyr Tyr Val His
        435                 440                 445

ggc caa cac tcc gga aag acg gcg taa                                  1371
Gly Gln His Ser Gly Lys Thr Ala
    450                 455
```

<210> 90
<211> 456
<212> PRT
<213> Ostreococcus tauri

<400> 90

Met Cys Val Glu Thr Glu Asn Asn Asp Gly Ile Pro Thr Val Glu Ile
1               5                   10                  15

Ala Phe Asp Gly Glu Arg Glu Arg Ala Glu Ala Asn Val Lys Leu Ser
            20                  25                  30

Ala Glu Lys Met Glu Pro Ala Ala Leu Ala Lys Thr Phe Ala Arg Arg
        35                  40                  45

Tyr Val Val Ile Glu Gly Val Glu Tyr Asp Val Thr Asp Phe Lys His
    50                  55                  60

Pro Gly Gly Thr Val Ile Phe Tyr Ala Leu Ser Asn Thr Gly Ala Asp
65                  70                  75                  80

Ala Thr Glu Ala Phe Lys Glu Phe His His Arg Ser Arg Lys Ala Arg
                85                  90                  95

Lys Ala Leu Ala Ala Leu Pro Ser Arg Pro Ala Lys Thr Ala Lys Val
            100                 105                 110

Asp Asp Ala Glu Met Leu Gln Asp Phe Ala Lys Trp Arg Lys Glu Leu
        115                 120                 125

Glu Arg Asp Gly Phe Phe Lys Pro Ser Pro Ala His Val Ala Tyr Arg
    130                 135                 140

Phe Ala Glu Leu Ala Ala Met Tyr Ala Leu Gly Thr Tyr Leu Met Tyr
145                 150                 155                 160

Ala Arg Tyr Val Val Ser Ser Val Leu Val Tyr Ala Cys Phe Phe Gly
                165                 170                 175

Ala Arg Cys Gly Trp Val Gln His Glu Gly Gly His Ser Ser Leu Thr
            180                 185                 190

Gly Asn Ile Trp Trp Asp Lys Arg Ile Gln Ala Phe Thr Ala Gly Phe
        195                 200                 205

Gly Leu Ala Gly Ser Gly Asp Met Trp Asn Ser Met His Asn Lys His
    210                 215                 220

His Ala Thr Pro Gln Lys Val Arg His Asp Met Asp Leu Asp Thr Thr
225                 230                 235                 240

Pro Ala Val Ala Phe Phe Asn Thr Ala Val Glu Asp Asn Arg Pro Arg
                245                 250                 255

```
Gly Phe Ser Lys Tyr Trp Leu Arg Leu Gln Ala Trp Thr Phe Ile Pro
            260                 265                 270

Val Thr Ser Gly Leu Val Leu Leu Phe Trp Met Phe Phe Leu His Pro
        275                 280                 285

Ser Lys Ala Leu Lys Gly Gly Lys Tyr Glu Glu Leu Val Trp Met Leu
    290                 295                 300

Ala Ala His Val Ile Arg Thr Trp Thr Ile Lys Ala Val Thr Gly Phe
305                 310                 315                 320

Thr Ala Met Gln Ser Tyr Gly Leu Phe Leu Ala Thr Ser Trp Val Ser
                325                 330                 335

Gly Cys Tyr Leu Phe Ala His Phe Ser Thr Ser His Thr His Leu Asp
            340                 345                 350

Val Val Pro Ala Asp Glu His Leu Ser Trp Val Arg Tyr Ala Val Asp
        355                 360                 365

His Thr Ile Asp Ile Asp Pro Ser Gln Gly Trp Val Asn Trp Leu Met
    370                 375                 380

Gly Tyr Leu Asn Cys Gln Val Ile His His Leu Phe Pro Ser Met Pro
385                 390                 395                 400

Gln Phe Arg Gln Pro Glu Val Ser Arg Arg Phe Val Ala Phe Ala Lys
                405                 410                 415

Lys Trp Asn Leu Asn Tyr Lys Val Met Thr Tyr Ala Gly Ala Trp Lys
            420                 425                 430

Ala Thr Leu Gly Asn Leu Asp Asn Val Gly Lys His Tyr Tyr Val His
        435                 440                 445

Gly Gln His Ser Gly Lys Thr Ala
    450                 455
```

<210> 91
<211> 606
<212> DNA
<213> Ostreococcus tauri

<220>
<221> CDS
<222> (1)..(606)
<223> Delta-5-Desaturase

<400> 91

```
atg tac ggt ttg cta tcg ctc aag tcg tgc ttc gtc gac gat ttc aac      48
Met Tyr Gly Leu Leu Ser Leu Lys Ser Cys Phe Val Asp Asp Phe Asn
1               5                   10                  15

gcc tac ttc tcc gga cgc atc ggc tgg gtc aag gtg atg aag ttc acc      96
Ala Tyr Phe Ser Gly Arg Ile Gly Trp Val Lys Val Met Lys Phe Thr
            20                  25                  30

cgc ggc gag gcg atc gca ttt tgg ggc acc aag ctc ttg tgg gcc gcg     144
Arg Gly Glu Ala Ile Ala Phe Trp Gly Thr Lys Leu Leu Trp Ala Ala
        35                  40                  45

tat tac ctc gcg ttg ccg cta aag atg tcg cat cgg ccg ctc gga gaa     192
Tyr Tyr Leu Ala Leu Pro Leu Lys Met Ser His Arg Pro Leu Gly Glu
    50                  55                  60

ctc ctc gca ctc tgg gcc gtc acc gag ttc gtc acc gga tgg ctg ttg     240
Leu Leu Ala Leu Trp Ala Val Thr Glu Phe Val Thr Gly Trp Leu Leu
65                  70                  75                  80

gcg ttc atg ttc caa gtc gcc cac gtc gtc ggc gag gtt cac ttc ttc     288
Ala Phe Met Phe Gln Val Ala His Val Val Gly Glu Val His Phe Phe
                85                  90                  95

acc ctc gac gcg aag aac cgc gtg aac ttg gga tgg gga gag gca cag     336
Thr Leu Asp Ala Lys Asn Arg Val Asn Leu Gly Trp Gly Glu Ala Gln
            100                 105                 110

ctc atg tcg agc gcg gat ttc gcc cac gga tcc aag ttt tgg acg cac     384
Leu Met Ser Ser Ala Asp Phe Ala His Gly Ser Lys Phe Trp Thr His
        115                 120                 125

ttc tcc gga ggc tta aac tac caa gtc gtc cac cat ctc ttc ccg ggc     432
Phe Ser Gly Gly Leu Asn Tyr Gln Val Val His His Leu Phe Pro Gly
    130                 135                 140

gtc tgc cac gtg cac tat ccc gcg ctc gcg cca att att aag gcg gca     480
Val Cys His Val His Tyr Pro Ala Leu Ala Pro Ile Ile Lys Ala Ala
145                 150                 155                 160

gct gag aag cac ggc ctc cac tac cag att tac ccc acg ttt tgg tcc     528
Ala Glu Lys His Gly Leu His Tyr Gln Ile Tyr Pro Thr Phe Trp Ser
                165                 170                 175

gcc ctg cgc gcg cac ttc cgg cac ctc gcc aac gtc ggc cgc gcc gcg     576
Ala Leu Arg Ala His Phe Arg His Leu Ala Asn Val Gly Arg Ala Ala
            180                 185                 190

tac gta ccg tcc ctc caa acc gtc gga tga                             606
Tyr Val Pro Ser Leu Gln Thr Val Gly
        195                 200
```

<210> 92
<211> 201
<212> PRT
<213> Ostreococcus tauri

<400> 92

```
Met Tyr Gly Leu Leu Ser Leu Lys Ser Cys Phe Val Asp Asp Phe Asn
1               5                   10                  15

Ala Tyr Phe Ser Gly Arg Ile Gly Trp Val Lys Val Met Lys Phe Thr
            20                  25                  30

Arg Gly Glu Ala Ile Ala Phe Trp Gly Thr Lys Leu Leu Trp Ala Ala
        35                  40                  45

Tyr Tyr Leu Ala Leu Pro Leu Lys Met Ser His Arg Pro Leu Gly Glu
    50                  55                  60

Leu Leu Ala Leu Trp Ala Val Thr Glu Phe Val Thr Gly Trp Leu Leu
65                  70                  75                  80

Ala Phe Met Phe Gln Val Ala His Val Val Gly Glu Val His Phe Phe
                85                  90                  95

Thr Leu Asp Ala Lys Asn Arg Val Asn Leu Gly Trp Gly Glu Ala Gln
            100                 105                 110

Leu Met Ser Ser Ala Asp Phe Ala His Gly Ser Lys Phe Trp Thr His
        115                 120                 125

Phe Ser Gly Gly Leu Asn Tyr Gln Val Val His His Leu Phe Pro Gly
    130                 135                 140

Val Cys His Val His Tyr Pro Ala Leu Ala Pro Ile Ile Lys Ala Ala
145                 150                 155                 160

Ala Glu Lys His Gly Leu His Tyr Gln Ile Tyr Pro Thr Phe Trp Ser
                165                 170                 175

Ala Leu Arg Ala His Phe Arg His Leu Ala Asn Val Gly Arg Ala Ala
            180                 185                 190

Tyr Val Pro Ser Leu Gln Thr Val Gly
        195                 200
```

<210> 93
<211> 714
<212> DNA
<213> Ostreococcus tauri

<220>
<221> CDS
<222> (1)..(714)
<223> Delta-5-Desaturase

<400> 93

```
atg gtg agc cat cac tcg tac tgt aac gac gcg gat ttg gat cag gat        48
Met Val Ser His His Ser Tyr Cys Asn Asp Ala Asp Leu Asp Gln Asp
1               5                   10                  15

gtg tac acc gca ctg ccg ctc ctg cgc ctg gac ccg tct cag gag ttg        96
Val Tyr Thr Ala Leu Pro Leu Leu Arg Leu Asp Pro Ser Gln Glu Leu
            20                  25                  30

aag tgg ttt cat cga tac cag gcg ttt tac gcc ccg ctc atg tgg ccg       144
Lys Trp Phe His Arg Tyr Gln Ala Phe Tyr Ala Pro Leu Met Trp Pro
        35                  40                  45

ttt ttg tgg ctc gcg gcg cag ttt ggc gac gcg cag aac atc ctg atc       192
Phe Leu Trp Leu Ala Ala Gln Phe Gly Asp Ala Gln Asn Ile Leu Ile
    50                  55                  60

gac cga gcg tcg ccg ggc gtc gcg tac aag gga ttg atg gcg aac gag       240
Asp Arg Ala Ser Pro Gly Val Ala Tyr Lys Gly Leu Met Ala Asn Glu
65                  70                  75                  80

gtc gcg ctg tac gtt ctc ggt aag gtt tta cac ttt ggt ctt ctc ctc       288
Val Ala Leu Tyr Val Leu Gly Lys Val Leu His Phe Gly Leu Leu Leu
                85                  90                  95

ggc gtt cct gcg tac ttg cac gga ttg tcc aac gcg atc gtt cca ttc       336
Gly Val Pro Ala Tyr Leu His Gly Leu Ser Asn Ala Ile Val Pro Phe
            100                 105                 110

ttg gcg tac ggc gca ttc ggc tcc ttc gtc ctg tgc tgg ttc ttc atc       384
Leu Ala Tyr Gly Ala Phe Gly Ser Phe Val Leu Cys Trp Phe Phe Ile
        115                 120                 125

gtc agc cat aac ctc gaa gcg ctg aca ccc gtt aac ctt aac aag tcc       432
Val Ser His Asn Leu Glu Ala Leu Thr Pro Val Asn Leu Asn Lys Ser
    130                 135                 140

acg aag aac gac tgg ggg gcg tgg cag atc gag aca tcg gcg tct tgg       480
Thr Lys Asn Asp Trp Gly Ala Trp Gln Ile Glu Thr Ser Ala Ser Trp
145                 150                 155                 160

ggc aac gcg ttc tgg agc ttc ttc tct gga ggt ctg aac ctg caa atc       528
Gly Asn Ala Phe Trp Ser Phe Phe Ser Gly Gly Leu Asn Leu Gln Ile
                165                 170                 175

gag cac cac ctc ttc ccg ggc atg gcg cac aac ctg tac ccg aag atg       576
Glu His His Leu Phe Pro Gly Met Ala His Asn Leu Tyr Pro Lys Met
            180                 185                 190

gtg ccg atc atc aag gac gag tgt gcg aaa gcg ggc gtt cgc tac acc       624
Val Pro Ile Ile Lys Asp Glu Cys Ala Lys Ala Gly Val Arg Tyr Thr
        195                 200                 205

ggt tac ggt ggc tac acc ggc ctg ctc ccg atc acc cgc gac atg ttc       672
Gly Tyr Gly Gly Tyr Thr Gly Leu Leu Pro Ile Thr Arg Asp Met Phe
    210                 215                 220

tcc tac ctc cat aag tgt ggc cga acg gcg aaa cta gcc taa               714
Ser Tyr Leu His Lys Cys Gly Arg Thr Ala Lys Leu Ala
225                 230                 235
```

<210> 94
<211> 237
<212> PRT
<213> Ostreococcus tauri

<400> 94

Met Val Ser His His Ser Tyr Cys Asn Asp Ala Asp Leu Asp Gln Asp
1 5 10 15

Val Tyr Thr Ala Leu Pro Leu Leu Arg Leu Asp Pro Ser Gln Glu Leu
20 25 30

Lys Trp Phe His Arg Tyr Gln Ala Phe Tyr Ala Pro Leu Met Trp Pro
35 40 45

Phe Leu Trp Leu Ala Ala Gln Phe Gly Asp Ala Gln Asn Ile Leu Ile
50 55 60

Asp Arg Ala Ser Pro Gly Val Ala Tyr Lys Gly Leu Met Ala Asn Glu
65 70 75 80

Val Ala Leu Tyr Val Leu Gly Lys Val Leu His Phe Gly Leu Leu Leu
85 90 95

Gly Val Pro Ala Tyr Leu His Gly Leu Ser Asn Ala Ile Val Pro Phe
100 105 110

Leu Ala Tyr Gly Ala Phe Gly Ser Phe Val Leu Cys Trp Phe Phe Ile
115 120 125

Val Ser His Asn Leu Glu Ala Leu Thr Pro Val Asn Leu Asn Lys Ser
130 135 140

Thr Lys Asn Asp Trp Gly Ala Trp Gln Ile Glu Thr Ser Ala Ser Trp
145 150 155 160

Gly Asn Ala Phe Trp Ser Phe Phe Ser Gly Gly Leu Asn Leu Gln Ile
165 170 175

Glu His His Leu Phe Pro Gly Met Ala His Asn Leu Tyr Pro Lys Met
180 185 190

Val Pro Ile Ile Lys Asp Glu Cys Ala Lys Ala Gly Val Arg Tyr Thr
195 200 205

Gly Tyr Gly Gly Tyr Thr Gly Leu Leu Pro Ile Thr Arg Asp Met Phe
210 215 220

Ser Tyr Leu His Lys Cys Gly Arg Thr Ala Lys Leu Ala
225 230 235

<210> 95
<211> 1611
<212> DNA
<213> Ostreococcus tauri

<220>
<221> CDS

<222> (1)..(1611)
<223> Delta-4-Desaturase

<400> 95

```
atg tac ctc gga cgc ggc cgt ctc gag agc ggg acg acg cga ggg atg       48
Met Tyr Leu Gly Arg Gly Arg Leu Glu Ser Gly Thr Thr Arg Gly Met
1               5                   10                  15

atg cgg acg cac gcg cgg cga ccg tcg acg acg tcg aat ccg tgc gcg       96
Met Arg Thr His Ala Arg Arg Pro Ser Thr Thr Ser Asn Pro Cys Ala
            20                  25                  30

cgg tca cgc gtg cgt aag acg acg gag cga tcg ctc gcg cga gtg cga      144
Arg Ser Arg Val Arg Lys Thr Thr Glu Arg Ser Leu Ala Arg Val Arg
        35                  40                  45

cga tcg acg agt gag aag gga agc gcg ctc gtg ctc gag cga gag agc      192
Arg Ser Thr Ser Glu Lys Gly Ser Ala Leu Val Leu Glu Arg Glu Ser
    50                  55                  60

gaa cgg gag aag gag gag gga ggg aaa gcg cga gcg gag gga ttg cga      240
Glu Arg Glu Lys Glu Glu Gly Gly Lys Ala Arg Ala Glu Gly Leu Arg
65                  70                  75                  80

ttc caa cgc ccg gac gtc gcc gcg ccg ggg gga gcg gat cct tgg aac      288
Phe Gln Arg Pro Asp Val Ala Ala Pro Gly Gly Ala Asp Pro Trp Asn
                85                  90                  95

gac gag aag tgg aca aag acc aag tgg acg gta ttc aga gac gtc gcg      336
Asp Glu Lys Trp Thr Lys Thr Lys Trp Thr Val Phe Arg Asp Val Ala
            100                 105                 110

tac gat ctc gat cct ttc ttc gct cga cac ccc gga gga gac tgg ctc      384
Tyr Asp Leu Asp Pro Phe Phe Ala Arg His Pro Gly Gly Asp Trp Leu
        115                 120                 125

ctg aac ttg gcc gtg gga cga gac tgc acc gcg ctc atc gaa tcc tat      432
Leu Asn Leu Ala Val Gly Arg Asp Cys Thr Ala Leu Ile Glu Ser Tyr
    130                 135                 140

cac ttg cga cca gag gtg gcg acg gct cgt ttc aga atg ctg ccc aaa      480
His Leu Arg Pro Glu Val Ala Thr Ala Arg Phe Arg Met Leu Pro Lys
145                 150                 155                 160

ctc gag gat ttt ccc gtc gag gcc gtg ccc aag tcc ccg aga ccg aac      528
```

```
Leu Glu Asp Phe Pro Val Glu Ala Val Pro Lys Ser Pro Arg Pro Asn
                165                 170                 175

gat tcg ccg tta tac aac aac att cgc aac cga gtc cgc gaa gag ctc      576
Asp Ser Pro Leu Tyr Asn Asn Ile Arg Asn Arg Val Arg Glu Glu Leu
            180                 185                 190

ttc cca gag gag gga aag aat atg cac aga cag ggc ggc gac cac ggc      624
Phe Pro Glu Glu Gly Lys Asn Met His Arg Gln Gly Gly Asp His Gly
        195                 200                 205

gac ggt gac gat tct ggg ttt cgc cgc ctt ttg ctt atg ccg tgt acc      672
Asp Gly Asp Asp Ser Gly Phe Arg Arg Leu Leu Leu Met Pro Cys Thr
    210                 215                 220

tat tcc ctt ccg ggg gtt cct ttc cgg ctg cct cct cgg gtc tcg cgg      720
Tyr Ser Leu Pro Gly Val Pro Phe Arg Leu Pro Pro Arg Val Ser Arg
225                 230                 235                 240

ggg cgt gga ttg gtc tca cga ttc agg cac tgc gcc aac cac ggc gcg      768
Gly Arg Gly Leu Val Ser Arg Phe Arg His Cys Ala Asn His Gly Ala
                245                 250                 255

atg tct cct tcg ccg gcc gtt aac ggc gtc ctc ggt ttg acg aac gat      816
Met Ser Pro Ser Pro Ala Val Asn Gly Val Leu Gly Leu Thr Asn Asp
            260                 265                 270

ctc atc ggc ggc tcg tcc ttg atg tgg aga tat cac cac caa gtc agc      864
Leu Ile Gly Gly Ser Ser Leu Met Trp Arg Tyr His His Gln Val Ser
        275                 280                 285

cac cac att cat tgc aac gac aac gcc atg gat caa gac gtg tac acg      912
His His Ile His Cys Asn Asp Asn Ala Met Asp Gln Asp Val Tyr Thr
    290                 295                 300

gcg atg cca tta ttg cgt ttc gac gct cgc cgg ccc aag tcc tgg tac      960
Ala Met Pro Leu Leu Arg Phe Asp Ala Arg Arg Pro Lys Ser Trp Tyr
305                 310                 315                 320

cat cgc ttc cag cag tgg tac atg ttt tta gcg ttc ccg ttg ttg cag     1008
His Arg Phe Gln Gln Trp Tyr Met Phe Leu Ala Phe Pro Leu Leu Gln
                325                 330                 335

gtt gcc ttc caa gtc gga gac att gcc gca ctg ttc acg cgt gat acc     1056
Val Ala Phe Gln Val Gly Asp Ile Ala Ala Leu Phe Thr Arg Asp Thr
            340                 345                 350

gaa ggc gct aag ctt cac ggg gcg acg acg tgg gag ctt acc acg gtt     1104
Glu Gly Ala Lys Leu His Gly Ala Thr Thr Trp Glu Leu Thr Thr Val
        355                 360                 365

gtc ctc ggt aag att gtg cac ttc ggt ctt ttg ttg ggg ccg ttg atg     1152
Val Leu Gly Lys Ile Val His Phe Gly Leu Leu Leu Gly Pro Leu Met
    370                 375                 380

aac cac gcg gtg agt tct gtt ttg ctg ggg atc gtc ggt ttc atg gcg     1200
Asn His Ala Val Ser Ser Val Leu Leu Gly Ile Val Gly Phe Met Ala
385                 390                 395                 400

tgc caa ggt ata gtt ctg gcg tgc acg ttt gct gtg agt cac aat gtc     1248
Cys Gln Gly Ile Val Leu Ala Cys Thr Phe Ala Val Ser His Asn Val
                405                 410                 415

gcg gag gcg aag ata cct gag gac acc gga gga gaa gcc tgg gag aga     1296
Ala Glu Ala Lys Ile Pro Glu Asp Thr Gly Gly Glu Ala Trp Glu Arg
            420                 425                 430

gat tgg ggt gtc cag cag ttg gtg act agc gcc gac tgg ggt gga aag     1344
```

```
Asp Trp Gly Val Gln Gln Leu Val Thr Ser Ala Asp Trp Gly Gly Lys
        435                 440                 445

ata ggt aac ttc ttc acg ggt ggc ctc aac ttg caa gtt gag cac cac      1392
Ile Gly Asn Phe Phe Thr Gly Gly Leu Asn Leu Gln Val Glu His His
    450                 455                 460

ttg ttt ccg gcg att tgc ttc gtc cac tac ccg gac atc gcg aag atc      1440
Leu Phe Pro Ala Ile Cys Phe Val His Tyr Pro Asp Ile Ala Lys Ile
465                 470                 475                 480

gtg aag gaa gaa gcg gcc aag ctc aac atc cct tac gcg tct tac agg      1488
Val Lys Glu Glu Ala Ala Lys Leu Asn Ile Pro Tyr Ala Ser Tyr Arg
                485                 490                 495

act ctt cct ggt att ttc gtc caa ttc tgg aga ttt atg aag gac atg      1536
Thr Leu Pro Gly Ile Phe Val Gln Phe Trp Arg Phe Met Lys Asp Met
            500                 505                 510

ggc acg gct gag caa att ggt gaa gtt cca ttg ccg aag att ccc aac      1584
Gly Thr Ala Glu Gln Ile Gly Glu Val Pro Leu Pro Lys Ile Pro Asn
        515                 520                 525

ccg cag ctc gcg ccg aag ctc gct tag                                  1611
Pro Gln Leu Ala Pro Lys Leu Ala
    530                 535
```

<210> 96
<211> 536
<212> PRT
<213> Ostreococcus tauri

<400> 96

```
Met Tyr Leu Gly Arg Gly Arg Leu Glu Ser Gly Thr Thr Arg Gly Met
1               5                   10                  15

Met Arg Thr His Ala Arg Arg Pro Ser Thr Thr Ser Asn Pro Cys Ala
            20                  25                  30

Arg Ser Arg Val Arg Lys Thr Thr Glu Arg Ser Leu Ala Arg Val Arg
        35                  40                  45

Arg Ser Thr Ser Glu Lys Gly Ser Ala Leu Val Leu Glu Arg Glu Ser
    50                  55                  60

Glu Arg Glu Lys Glu Glu Gly Gly Lys Ala Arg Ala Glu Gly Leu Arg
65                  70                  75                  80

Phe Gln Arg Pro Asp Val Ala Ala Pro Gly Gly Ala Asp Pro Trp Asn
                85                  90                  95

Asp Glu Lys Trp Thr Lys Thr Lys Trp Thr Val Phe Arg Asp Val Ala
            100                 105                 110
```

Tyr Asp Leu Asp Pro Phe Phe Ala Arg His Pro Gly Gly Asp Trp Leu
115 120 125

Leu Asn Leu Ala Val Gly Arg Asp Cys Thr Ala Leu Ile Glu Ser Tyr
130 135 140

His Leu Arg Pro Glu Val Ala Thr Ala Arg Phe Arg Met Leu Pro Lys
145 150 155 160

Leu Glu Asp Phe Pro Val Glu Ala Val Pro Lys Ser Pro Arg Pro Asn
165 170 175

Asp Ser Pro Leu Tyr Asn Asn Ile Arg Asn Arg Val Arg Glu Glu Leu
180 185 190

Phe Pro Glu Glu Gly Lys Asn Met His Arg Gln Gly Gly Asp His Gly
195 200 205

Asp Gly Asp Asp Ser Gly Phe Arg Arg Leu Leu Leu Met Pro Cys Thr
210 215 220

Tyr Ser Leu Pro Gly Val Pro Phe Arg Leu Pro Pro Arg Val Ser Arg
225 230 235 240

Gly Arg Gly Leu Val Ser Arg Phe Arg His Cys Ala Asn His Gly Ala
245 250 255

Met Ser Pro Ser Pro Ala Val Asn Gly Val Leu Gly Leu Thr Asn Asp
260 265 270

Leu Ile Gly Gly Ser Ser Leu Met Trp Arg Tyr His His Gln Val Ser
275 280 285

His His Ile His Cys Asn Asp Asn Ala Met Asp Gln Asp Val Tyr Thr
290 295 300

Ala Met Pro Leu Leu Arg Phe Asp Ala Arg Arg Pro Lys Ser Trp Tyr
305 310 315 320

His Arg Phe Gln Gln Trp Tyr Met Phe Leu Ala Phe Pro Leu Leu Gln
325 330 335

Val Ala Phe Gln Val Gly Asp Ile Ala Ala Leu Phe Thr Arg Asp Thr
340 345 350

Glu Gly Ala Lys Leu His Gly Ala Thr Thr Trp Glu Leu Thr Thr Val
355 360 365

Val Leu Gly Lys Ile Val His Phe Gly Leu Leu Leu Gly Pro Leu Met
370 375 380

```
Asn His Ala Val Ser Ser Val Leu Leu Gly Ile Val Gly Phe Met Ala
385                 390                 395                 400

Cys Gln Gly Ile Val Leu Ala Cys Thr Phe Ala Val Ser His Asn Val
                405                 410                 415

Ala Glu Ala Lys Ile Pro Glu Asp Thr Gly Gly Glu Ala Trp Glu Arg
            420                 425                 430

Asp Trp Gly Val Gln Gln Leu Val Thr Ser Ala Asp Trp Gly Gly Lys
        435                 440                 445

Ile Gly Asn Phe Phe Thr Gly Gly Leu Asn Leu Gln Val Glu His His
    450                 455                 460

Leu Phe Pro Ala Ile Cys Phe Val His Tyr Pro Asp Ile Ala Lys Ile
465                 470                 475                 480

Val Lys Glu Glu Ala Ala Lys Leu Asn Ile Pro Tyr Ala Ser Tyr Arg
                485                 490                 495

Thr Leu Pro Gly Ile Phe Val Gln Phe Trp Arg Phe Met Lys Asp Met
            500                 505                 510

Gly Thr Ala Glu Gln Ile Gly Glu Val Pro Leu Pro Lys Ile Pro Asn
        515                 520                 525

Pro Gln Leu Ala Pro Lys Leu Ala
    530                 535
```

<210> 97
<211> 1455
<212> DNA
<213> Thalassiosira pseudonana

<220>
<221> CDS
<222> (1)..(1455)
<223> Delta-6-Desaturase

<400> 97

```
atg gga aaa gga gga gac gca gcc gca gct acc aag cgt agt gga gca      48
Met Gly Lys Gly Gly Asp Ala Ala Ala Ala Thr Lys Arg Ser Gly Ala
1               5                   10                  15

ttg aaa ttg gcg gag aag ccg cag aag tac act tgg cag gag gtg aag      96
Leu Lys Leu Ala Glu Lys Pro Gln Lys Tyr Thr Trp Gln Glu Val Lys
            20                  25                  30
```

```
aag cac atc acc ccc gac gat gcc tgg gta gtc cac caa aac aaa gtc       144
Lys His Ile Thr Pro Asp Asp Ala Trp Val Val His Gln Asn Lys Val
        35                  40                  45

tac gac gtc tcc aac tgg tac gac cac ccc ggt gga gcc gtg gtg ttc       192
Tyr Asp Val Ser Asn Trp Tyr Asp His Pro Gly Gly Ala Val Val Phe
    50                  55                  60

acc cac gcc gga gac gac atg acg gac atc ttc gcc gcc ttc cac gcc       240
Thr His Ala Gly Asp Asp Met Thr Asp Ile Phe Ala Ala Phe His Ala
65                  70                  75                  80

caa ggc tct cag gcc atg atg aag aag ttt tac att gga gat ttg att       288
Gln Gly Ser Gln Ala Met Met Lys Lys Phe Tyr Ile Gly Asp Leu Ile
                85                  90                  95

ccg gag agt gtg gag cat aag gat caa aga cag ttg gat ttc gag aag       336
Pro Glu Ser Val Glu His Lys Asp Gln Arg Gln Leu Asp Phe Glu Lys
            100                 105                 110

gga tat cgt gat tta cgg gcc aag ctt gtc atg atg ggg atg ttc aag       384
Gly Tyr Arg Asp Leu Arg Ala Lys Leu Val Met Met Gly Met Phe Lys
        115                 120                 125

tcg agt aag atg tat tat gca tac aag tgc tcg ttc aat atg tgc atg       432
Ser Ser Lys Met Tyr Tyr Ala Tyr Lys Cys Ser Phe Asn Met Cys Met
    130                 135                 140

tgg ttg gtg gcg gtg gcc atg gtg tac tac tcg gac agt ttg gca atg       480
Trp Leu Val Ala Val Ala Met Val Tyr Tyr Ser Asp Ser Leu Ala Met
145                 150                 155                 160

cac att gga tcg gct ctc ttg ttg gga ttg ttc tgg cag cag tgt gga       528
His Ile Gly Ser Ala Leu Leu Leu Gly Leu Phe Trp Gln Gln Cys Gly
                165                 170                 175

tgg ctt gcg cac gac ttt ctt cac cac caa gtc ttt aag caa cga aag       576
Trp Leu Ala His Asp Phe Leu His His Gln Val Phe Lys Gln Arg Lys
            180                 185                 190

tac gga gat ctc gtt ggc atc ttt tgg gga gat ctc atg cag ggg ttc       624
Tyr Gly Asp Leu Val Gly Ile Phe Trp Gly Asp Leu Met Gln Gly Phe
        195                 200                 205

tcg atg cag tgg tgg aag aac aag cac aat ggc cac cat gct gtt ccc       672
Ser Met Gln Trp Trp Lys Asn Lys His Asn Gly His His Ala Val Pro
    210                 215                 220

aac ttg cac aac tct tcc ttg gac agt cag gat ggt gat ccc gat att       720
Asn Leu His Asn Ser Ser Leu Asp Ser Gln Asp Gly Asp Pro Asp Ile
225                 230                 235                 240

gat acc atg cca ctc ctt gct tgg agt ctc aag cag gct cag agt ttc       768
Asp Thr Met Pro Leu Leu Ala Trp Ser Leu Lys Gln Ala Gln Ser Phe
                245                 250                 255

aga gag atc aat aag gga aag gac agt acc ttc gtc aag tac gct atc       816
Arg Glu Ile Asn Lys Gly Lys Asp Ser Thr Phe Val Lys Tyr Ala Ile
            260                 265                 270

aaa ttc cag gca ttc aca tac ttc ccc atc ctc ctc ttg gct cgc atc       864
Lys Phe Gln Ala Phe Thr Tyr Phe Pro Ile Leu Leu Leu Ala Arg Ile
        275                 280                 285

tct tgg ttg aat gaa tcc ttc aaa act gca ttc gga ctc gga gct gcc       912
Ser Trp Leu Asn Glu Ser Phe Lys Thr Ala Phe Gly Leu Gly Ala Ala
    290                 295                 300
```

```
tcg gag aat gcc aag ttg gag ttg gag aag cgt gga ctt cag tac cca      960
Ser Glu Asn Ala Lys Leu Glu Leu Glu Lys Arg Gly Leu Gln Tyr Pro
305                 310                 315                 320

ctt ttg gag aag ctt gga atc acc ctt cat tac act tgg atg ttc gtc     1008
Leu Leu Glu Lys Leu Gly Ile Thr Leu His Tyr Thr Trp Met Phe Val
                325                 330                 335

ctc tct tcc gga ttt gga agg tgg tct ctt cca tat tcc atc atg tat     1056
Leu Ser Ser Gly Phe Gly Arg Trp Ser Leu Pro Tyr Ser Ile Met Tyr
            340                 345                 350

ttc ttc act gcc aca tgc tcc tcg gga ctt ttc ctc gca ttg gtc ttt     1104
Phe Phe Thr Ala Thr Cys Ser Ser Gly Leu Phe Leu Ala Leu Val Phe
        355                 360                 365

gga ttg gga cac aac ggt atg tca gtg tac gat gcc acc acc cga cct     1152
Gly Leu Gly His Asn Gly Met Ser Val Tyr Asp Ala Thr Thr Arg Pro
    370                 375                 380

gac ttc tgg caa ctc caa gtc acc act aca cgt aac atc att ggt gga     1200
Asp Phe Trp Gln Leu Gln Val Thr Thr Thr Arg Asn Ile Ile Gly Gly
385                 390                 395                 400

cac ggc att ccc caa ttc ttt gtg gat tgg ttc tgc ggt gga ttg caa     1248
His Gly Ile Pro Gln Phe Phe Val Asp Trp Phe Cys Gly Gly Leu Gln
                405                 410                 415

tac caa gtg gat cac cac ctc ttc ccc atg atg cct aga aac aat atc     1296
Tyr Gln Val Asp His His Leu Phe Pro Met Met Pro Arg Asn Asn Ile
            420                 425                 430

gcg aaa tgc cac aag ctt gtg gag tca ttc tgt aag gag tgg ggt gtg     1344
Ala Lys Cys His Lys Leu Val Glu Ser Phe Cys Lys Glu Trp Gly Val
        435                 440                 445

aag tac cat gag gcc gat atg tgg gat ggt acc gtg gaa gtg ttg caa     1392
Lys Tyr His Glu Ala Asp Met Trp Asp Gly Thr Val Glu Val Leu Gln
    450                 455                 460

cat ctc tcc aag gtg tcg gat gat ttc ctt gtg gag atg gtg aag gat     1440
His Leu Ser Lys Val Ser Asp Asp Phe Leu Val Glu Met Val Lys Asp
465                 470                 475                 480

ttc cct gcc atg taa                                                 1455
Phe Pro Ala Met
```

<210> 98
<211> 484
<212> PRT
<213> Thalassiosira pseudonana

<400> 98

```
Met Gly Lys Gly Gly Asp Ala Ala Ala Ala Thr Lys Arg Ser Gly Ala
1               5                   10                  15

Leu Lys Leu Ala Glu Lys Pro Gln Lys Tyr Thr Trp Gln Glu Val Lys
            20                  25                  30
```

```
Lys His Ile Thr Pro Asp Asp Ala Trp Val Val His Gln Asn Lys Val
        35                  40                  45

Tyr Asp Val Ser Asn Trp Tyr Asp His Pro Gly Gly Ala Val Val Phe
    50                  55                  60

Thr His Ala Gly Asp Asp Met Thr Asp Ile Phe Ala Ala Phe His Ala
65                  70                  75                  80

Gln Gly Ser Gln Ala Met Met Lys Lys Phe Tyr Ile Gly Asp Leu Ile
                85                  90                  95

Pro Glu Ser Val Glu His Lys Asp Gln Arg Gln Leu Asp Phe Glu Lys
            100                 105                 110

Gly Tyr Arg Asp Leu Arg Ala Lys Leu Val Met Met Gly Met Phe Lys
        115                 120                 125

Ser Ser Lys Met Tyr Tyr Ala Tyr Lys Cys Ser Phe Asn Met Cys Met
    130                 135                 140

Trp Leu Val Ala Val Ala Met Val Tyr Tyr Ser Asp Ser Leu Ala Met
145                 150                 155                 160

His Ile Gly Ser Ala Leu Leu Leu Gly Leu Phe Trp Gln Gln Cys Gly
                165                 170                 175

Trp Leu Ala His Asp Phe Leu His His Gln Val Phe Lys Gln Arg Lys
            180                 185                 190

Tyr Gly Asp Leu Val Gly Ile Phe Trp Gly Asp Leu Met Gln Gly Phe
        195                 200                 205

Ser Met Gln Trp Trp Lys Asn Lys His Asn Gly His His Ala Val Pro
    210                 215                 220

Asn Leu His Asn Ser Ser Leu Asp Ser Gln Asp Gly Asp Pro Asp Ile
225                 230                 235                 240

Asp Thr Met Pro Leu Leu Ala Trp Ser Leu Lys Gln Ala Gln Ser Phe
                245                 250                 255

Arg Glu Ile Asn Lys Gly Lys Asp Ser Thr Phe Val Lys Tyr Ala Ile
            260                 265                 270

Lys Phe Gln Ala Phe Thr Tyr Phe Pro Ile Leu Leu Leu Ala Arg Ile
        275                 280                 285

Ser Trp Leu Asn Glu Ser Phe Lys Thr Ala Phe Gly Leu Gly Ala Ala
    290                 295                 300
```

Ser Glu Asn Ala Lys Leu Glu Leu Glu Lys Arg Gly Leu Gln Tyr Pro
305 310 315 320

Leu Leu Glu Lys Leu Gly Ile Thr Leu His Tyr Thr Trp Met Phe Val
325 330 335

Leu Ser Ser Gly Phe Gly Arg Trp Ser Leu Pro Tyr Ser Ile Met Tyr
340 345 350

Phe Phe Thr Ala Thr Cys Ser Ser Gly Leu Phe Leu Ala Leu Val Phe
355 360 365

Gly Leu Gly His Asn Gly Met Ser Val Tyr Asp Ala Thr Thr Arg Pro
370 375 380

Asp Phe Trp Gln Leu Gln Val Thr Thr Thr Arg Asn Ile Ile Gly Gly
385 390 395 400

His Gly Ile Pro Gln Phe Phe Val Asp Trp Phe Cys Gly Gly Leu Gln
405 410 415

Tyr Gln Val Asp His His Leu Phe Pro Met Met Pro Arg Asn Asn Ile
420 425 430

Ala Lys Cys His Lys Leu Val Glu Ser Phe Cys Lys Glu Trp Gly Val
435 440 445

Lys Tyr His Glu Ala Asp Met Trp Asp Gly Thr Val Glu Val Leu Gln
450 455 460

His Leu Ser Lys Val Ser Asp Asp Phe Leu Val Glu Met Val Lys Asp
465 470 475 480

Phe Pro Ala Met

<210> 99
<211> 1431
<212> DNA
<213> Thalassiosira pseudonana

<220>
<221> CDS
<222> (1)..(1431)
<223> Delta-5-Desaturase

<400> 99

```
atg ccc ccc aac gcc gat atc tcc cgc atc cgc aac cgc atc ccc acc      48
Met Pro Pro Asn Ala Asp Ile Ser Arg Ile Arg Asn Arg Ile Pro Thr
1               5                   10                  15

aaa aca ggt acc gtt gcc tct gcc gac aac aac gac ccc gcc acc caa      96
Lys Thr Gly Thr Val Ala Ser Ala Asp Asn Asn Asp Pro Ala Thr Gln
            20                  25                  30

tcc gtc cga acc ctc aaa tct ctc aag ggc aac gag gtc gtc atc aac     144
Ser Val Arg Thr Leu Lys Ser Leu Lys Gly Asn Glu Val Val Ile Asn
        35                  40                  45

ggc aca att tat gac att gct gac ttt gtc cat cct gga gga gag gtt     192
Gly Thr Ile Tyr Asp Ile Ala Asp Phe Val His Pro Gly Gly Glu Val
    50                  55                  60

gtc aag ttc ttt ggt ggg aat gat gtt act att cag tat aat atg att     240
Val Lys Phe Phe Gly Gly Asn Asp Val Thr Ile Gln Tyr Asn Met Ile
65                  70                  75                  80

cat ccg tat cat acg ggg aaa cat ctg gag aag atg aag gct gtt gga     288
His Pro Tyr His Thr Gly Lys His Leu Glu Lys Met Lys Ala Val Gly
                85                  90                  95

aag gtt gta gat tgg cag tcg gac tac aag ttc gac acc ccc ttt gaa     336
Lys Val Val Asp Trp Gln Ser Asp Tyr Lys Phe Asp Thr Pro Phe Glu
            100                 105                 110

cga gag atc aaa tca gaa gtg ttc aag atc gta cgt cgc ggg cgt gag     384
Arg Glu Ile Lys Ser Glu Val Phe Lys Ile Val Arg Arg Gly Arg Glu
        115                 120                 125

ttc ggc aca aca ggc tac ttc ctc cgt gcc ttt ttc tac atc gct ctc     432
Phe Gly Thr Thr Gly Tyr Phe Leu Arg Ala Phe Phe Tyr Ile Ala Leu
    130                 135                 140

ttc ttc acc atg caa tac act ttc gcc aca tgc acc acc ttc acc acc     480
Phe Phe Thr Met Gln Tyr Thr Phe Ala Thr Cys Thr Thr Phe Thr Thr
145                 150                 155                 160

tac gat cac tgg tat cag agt ggt gta ttc atc gca att gtg ttt ggt     528
Tyr Asp His Trp Tyr Gln Ser Gly Val Phe Ile Ala Ile Val Phe Gly
                165                 170                 175

att tca cag gca ttc att ggg ttg aat gtc cag cac gat gcc aat cac     576
Ile Ser Gln Ala Phe Ile Gly Leu Asn Val Gln His Asp Ala Asn His
            180                 185                 190

gga gct gcc agt aag cgt ccc tgg gtg aat gac ttg ttg gga ttt gga     624
Gly Ala Ala Ser Lys Arg Pro Trp Val Asn Asp Leu Leu Gly Phe Gly
        195                 200                 205

acg gat ttg att gga tct aac aaa tgg aat tgg atg gca cag cat tgg     672
Thr Asp Leu Ile Gly Ser Asn Lys Trp Asn Trp Met Ala Gln His Trp
    210                 215                 220

act cat cac gct tac act aac cat agt gag aag gat ccc gat agc ttc     720
Thr His His Ala Tyr Thr Asn His Ser Glu Lys Asp Pro Asp Ser Phe
225                 230                 235                 240

agc tcg gaa cct atg ttt gca ttc aat gac tat ccc att gga cac ccg     768
Ser Ser Glu Pro Met Phe Ala Phe Asn Asp Tyr Pro Ile Gly His Pro
                245                 250                 255

aag aga aag tgg tgg cat agg ttc cag gga ggg tac ttc ctc ttc atg     816
```

```
Lys Arg Lys Trp Trp His Arg Phe Gln Gly Gly Tyr Phe Leu Phe Met
            260                 265                 270

ctt gga ctt tac tgg ctc tcg act gta ttc aat ccg caa ttc att gat      864
Leu Gly Leu Tyr Trp Leu Ser Thr Val Phe Asn Pro Gln Phe Ile Asp
        275                 280                 285

ctt cgt caa cgt ggg gct cag tac gtc gga att caa atg gag aat gat      912
Leu Arg Gln Arg Gly Ala Gln Tyr Val Gly Ile Gln Met Glu Asn Asp
    290                 295                 300

ttc att gtc aag agg agg aag tac gcc gtt gca ttg agg atg atg tac      960
Phe Ile Val Lys Arg Arg Lys Tyr Ala Val Ala Leu Arg Met Met Tyr
305                 310                 315                 320

att tac ttg aac att gtc agc ccc ttc atg aac aat ggt ttg agc tgg     1008
Ile Tyr Leu Asn Ile Val Ser Pro Phe Met Asn Asn Gly Leu Ser Trp
                325                 330                 335

tct acc ttt gga atc atc atg ttg atg gga atc agc gag agt ctc act     1056
Ser Thr Phe Gly Ile Ile Met Leu Met Gly Ile Ser Glu Ser Leu Thr
            340                 345                 350

ctc agt gtg ctc ttc tcg ttg tct cac aac ttc atc aat tcg gat cgt     1104
Leu Ser Val Leu Phe Ser Leu Ser His Asn Phe Ile Asn Ser Asp Arg
        355                 360                 365

gat cct acg gct gac ttc aaa aag acc gga gaa caa gtg tgc tgg ttc     1152
Asp Pro Thr Ala Asp Phe Lys Lys Thr Gly Glu Gln Val Cys Trp Phe
    370                 375                 380

aag tcg cag gtg gag act tcg tct acc tat ggg ggt ttt att tcc gga     1200
Lys Ser Gln Val Glu Thr Ser Ser Thr Tyr Gly Gly Phe Ile Ser Gly
385                 390                 395                 400

tgt ctt acg gga gga ctc aac ttt cag gtg gaa cat cat ctc ttt ccc     1248
Cys Leu Thr Gly Gly Leu Asn Phe Gln Val Glu His His Leu Phe Pro
                405                 410                 415

cgt atg agc agt gct tgg tat cct tac att gca cct acg gtt cgt gag     1296
Arg Met Ser Ser Ala Trp Tyr Pro Tyr Ile Ala Pro Thr Val Arg Glu
            420                 425                 430

gtt tgc aag aag cac ggg gtg aac tac gct tat tat cct tgg att ggg     1344
Val Cys Lys Lys His Gly Val Asn Tyr Ala Tyr Tyr Pro Trp Ile Gly
        435                 440                 445

cag aat ttg gta tca aca ttc aaa tac atg cat cgc gct ggt agt gga     1392
Gln Asn Leu Val Ser Thr Phe Lys Tyr Met His Arg Ala Gly Ser Gly
    450                 455                 460

gcc aac tgg gag ctc aag ccg ttg tct gga agt gcc taa                 1431
Ala Asn Trp Glu Leu Lys Pro Leu Ser Gly Ser Ala
465                 470                 475
```

<210> 100
<211> 476
<212> PRT
<213> Thalassiosira pseudonana

<400> 100

Met Pro Pro Asn Ala Asp Ile Ser Arg Ile Arg Asn Arg Ile Pro Thr
1 5 10 15

Lys Thr Gly Thr Val Ala Ser Ala Asp Asn Asn Asp Pro Ala Thr Gln
20 25 30

Ser Val Arg Thr Leu Lys Ser Leu Lys Gly Asn Glu Val Val Ile Asn
35 40 45

Gly Thr Ile Tyr Asp Ile Ala Asp Phe Val His Pro Gly Gly Glu Val
50 55 60

Val Lys Phe Phe Gly Gly Asn Asp Val Thr Ile Gln Tyr Asn Met Ile
65 70 75 80

His Pro Tyr His Thr Gly Lys His Leu Glu Lys Met Lys Ala Val Gly
85 90 95

Lys Val Val Asp Trp Gln Ser Asp Tyr Lys Phe Asp Thr Pro Phe Glu
100 105 110

Arg Glu Ile Lys Ser Glu Val Phe Lys Ile Val Arg Arg Gly Arg Glu
115 120 125

Phe Gly Thr Thr Gly Tyr Phe Leu Arg Ala Phe Phe Tyr Ile Ala Leu
130 135 140

Phe Phe Thr Met Gln Tyr Thr Phe Ala Thr Cys Thr Thr Phe Thr Thr
145 150 155 160

Tyr Asp His Trp Tyr Gln Ser Gly Val Phe Ile Ala Ile Val Phe Gly
165 170 175

Ile Ser Gln Ala Phe Ile Gly Leu Asn Val Gln His Asp Ala Asn His
180 185 190

Gly Ala Ala Ser Lys Arg Pro Trp Val Asn Asp Leu Leu Gly Phe Gly
195 200 205

Thr Asp Leu Ile Gly Ser Asn Lys Trp Asn Trp Met Ala Gln His Trp
210 215 220

Thr His His Ala Tyr Thr Asn His Ser Glu Lys Asp Pro Asp Ser Phe
225 230 235 240

Ser Ser Glu Pro Met Phe Ala Phe Asn Asp Tyr Pro Ile Gly His Pro
245 250 255

Lys Arg Lys Trp Trp His Arg Phe Gln Gly Gly Tyr Phe Leu Phe Met
260 265 270

Leu Gly Leu Tyr Trp Leu Ser Thr Val Phe Asn Pro Gln Phe Ile Asp
275 280 285

Leu Arg Gln Arg Gly Ala Gln Tyr Val Gly Ile Gln Met Glu Asn Asp
290 295 300

Phe Ile Val Lys Arg Arg Lys Tyr Ala Val Ala Leu Arg Met Met Tyr
305 310 315 320

Ile Tyr Leu Asn Ile Val Ser Pro Phe Met Asn Asn Gly Leu Ser Trp
325 330 335

Ser Thr Phe Gly Ile Ile Met Leu Met Gly Ile Ser Glu Ser Leu Thr
340 345 350

Leu Ser Val Leu Phe Ser Leu Ser His Asn Phe Ile Asn Ser Asp Arg
355 360 365

Asp Pro Thr Ala Asp Phe Lys Lys Thr Gly Glu Gln Val Cys Trp Phe
370 375 380

Lys Ser Gln Val Glu Thr Ser Ser Thr Tyr Gly Gly Phe Ile Ser Gly
385 390 395 400

Cys Leu Thr Gly Gly Leu Asn Phe Gln Val Glu His His Leu Phe Pro
405 410 415

Arg Met Ser Ser Ala Trp Tyr Pro Tyr Ile Ala Pro Thr Val Arg Glu
420 425 430

Val Cys Lys Lys His Gly Val Asn Tyr Ala Tyr Tyr Pro Trp Ile Gly
435 440 445

Gln Asn Leu Val Ser Thr Phe Lys Tyr Met His Arg Ala Gly Ser Gly
450 455 460

Ala Asn Trp Glu Leu Lys Pro Leu Ser Gly Ser Ala
465 470 475

<210> 101
<211> 1449
<212> DNA
<213> Thalassiosira pseudonana

<220>
<221> CDS
<222> (1)..(1449)
<223> Delta-5-Desaturase

<400> 101

```
atg cca ccc aac gcc gag gtc aaa aac ctc cgt tca cgt tcc atc cca      48
Met Pro Pro Asn Ala Glu Val Lys Asn Leu Arg Ser Arg Ser Ile Pro
1               5                   10                  15

acg aag aag tcc agt tca tcg tca tcc acc gcg aac gac gat ccg gct      96
Thr Lys Lys Ser Ser Ser Ser Ser Ser Thr Ala Asn Asp Asp Pro Ala
            20                  25                  30

acc caa tcc acc tca cct gtg aac cga acc ctc aag tct ttg aat gga     144
Thr Gln Ser Thr Ser Pro Val Asn Arg Thr Leu Lys Ser Leu Asn Gly
        35                  40                  45

aac gaa ata gct att gac ggt gtc atc tat gat att gat ggc ttt gtc     192
Asn Glu Ile Ala Ile Asp Gly Val Ile Tyr Asp Ile Asp Gly Phe Val
    50                  55                  60

cat cct gga gga gag gtt att agc ttc ttt gga ggc aac gat gtg act     240
His Pro Gly Gly Glu Val Ile Ser Phe Phe Gly Gly Asn Asp Val Thr
65                  70                  75                  80

gta cag tac aaa atg att cat ccg tat cat aat agt aag cat ctc gag     288
Val Gln Tyr Lys Met Ile His Pro Tyr His Asn Ser Lys His Leu Glu
                85                  90                  95

aag atg aga gcc gtt gga aag att gca gac tac tcc aca gag tac aag     336
Lys Met Arg Ala Val Gly Lys Ile Ala Asp Tyr Ser Thr Glu Tyr Lys
            100                 105                 110

ttc gac aca ccc ttt gaa cga gag atc aaa tcc gaa gtg ttc aaa atc     384
Phe Asp Thr Pro Phe Glu Arg Glu Ile Lys Ser Glu Val Phe Lys Ile
        115                 120                 125

gtc cgt cga gga cgt gaa ttc ggt aca aca gga tat ttc ctc cgt gcc     432
Val Arg Arg Gly Arg Glu Phe Gly Thr Thr Gly Tyr Phe Leu Arg Ala
    130                 135                 140

ttc ttc tac att gct ctc ttc ttc acc atg caa tac acc ttc gcc aca     480
Phe Phe Tyr Ile Ala Leu Phe Phe Thr Met Gln Tyr Thr Phe Ala Thr
145                 150                 155                 160

tgc act acc ttc acc acc tac gat cat tgg tat caa agt ggt gta ttc     528
Cys Thr Thr Phe Thr Thr Tyr Asp His Trp Tyr Gln Ser Gly Val Phe
                165                 170                 175

atc gcc att gtg ttt ggt atc tca caa gct ttc att ggg ttg aat gta     576
Ile Ala Ile Val Phe Gly Ile Ser Gln Ala Phe Ile Gly Leu Asn Val
            180                 185                 190

caa cat gat gcc aat cac gga gct gct agc aaa cga cct tgg gtg aat     624
Gln His Asp Ala Asn His Gly Ala Ala Ser Lys Arg Pro Trp Val Asn
        195                 200                 205

gat ctc ctt gga tct gga gct gat ctc atc ggt gga tgc aaa tgg aac     672
Asp Leu Leu Gly Ser Gly Ala Asp Leu Ile Gly Gly Cys Lys Trp Asn
    210                 215                 220

tgg ttg gct cag cat tgg act cat cat gcg tat acc aat cac gct gat     720
Trp Leu Ala Gln His Trp Thr His His Ala Tyr Thr Asn His Ala Asp
225                 230                 235                 240

aaa gat cct gat agc ttt agt tcc gag ccg gtc ttc aac ttt aac gat     768
Lys Asp Pro Asp Ser Phe Ser Ser Glu Pro Val Phe Asn Phe Asn Asp
                245                 250                 255
```

```
tat ccc att ggt cac ccc aaa aga aag tgg tgg cat agg ttc caa ggg      816
Tyr Pro Ile Gly His Pro Lys Arg Lys Trp Trp His Arg Phe Gln Gly
        260                 265                 270

ctc tac ttc cta atc atg ctg agt ttc tat tgg gta tcg atg gta ttc      864
Leu Tyr Phe Leu Ile Met Leu Ser Phe Tyr Trp Val Ser Met Val Phe
    275                 280                 285

aac cca caa gtt atc gac ctc cgt cat gct gga gct gcc tac gtt gga      912
Asn Pro Gln Val Ile Asp Leu Arg His Ala Gly Ala Ala Tyr Val Gly
    290                 295                 300

ttt cag atg gag aac gac ttt atc gtc aaa cgg aga aag tat gca atg      960
Phe Gln Met Glu Asn Asp Phe Ile Val Lys Arg Arg Lys Tyr Ala Met
305                 310                 315                 320

gca ctt cgt gca atg tac ttc tat ttc aac atc tat tgt ccg att gtc     1008
Ala Leu Arg Ala Met Tyr Phe Tyr Phe Asn Ile Tyr Cys Pro Ile Val
                325                 330                 335

aac aat gga ttg act tgg tcg aca gtt gga atc atc ctc tta atg gga     1056
Asn Asn Gly Leu Thr Trp Ser Thr Val Gly Ile Ile Leu Leu Met Gly
            340                 345                 350

gtt agc gaa agc ttc atg ctc tcc ggt cta ttc gta ctc tca cac aac     1104
Val Ser Glu Ser Phe Met Leu Ser Gly Leu Phe Val Leu Ser His Asn
        355                 360                 365

ttt gaa aat tcc gaa cgt gat cct acc tct gag tat cgc aag act ggt     1152
Phe Glu Asn Ser Glu Arg Asp Pro Thr Ser Glu Tyr Arg Lys Thr Gly
    370                 375                 380

gag caa gta tgt tgg ttc aag tct caa gtg gag act tct tct acc tac     1200
Glu Gln Val Cys Trp Phe Lys Ser Gln Val Glu Thr Ser Ser Thr Tyr
385                 390                 395                 400

gga ggt atc gtt gct ggg tgt ctc act ggt gga ctc aac ttt caa gtg     1248
Gly Gly Ile Val Ala Gly Cys Leu Thr Gly Gly Leu Asn Phe Gln Val
                405                 410                 415

gag cat cat ttg ttc ccg agg atg agc agt gct tgg tat cct ttc atc     1296
Glu His His Leu Phe Pro Arg Met Ser Ser Ala Trp Tyr Pro Phe Ile
            420                 425                 430

gcg ccg aag gtt aga gag att tgt aag aag cat gga gtt aga tac gct     1344
Ala Pro Lys Val Arg Glu Ile Cys Lys Lys His Gly Val Arg Tyr Ala
        435                 440                 445

tac tat ccg tac atc tgg cag aac ttg cat tct acc gtg agt tac atg     1392
Tyr Tyr Pro Tyr Ile Trp Gln Asn Leu His Ser Thr Val Ser Tyr Met
    450                 455                 460

cat ggg acg gga acg gga gct aga tgg gag ctt cag ccg ttg tct gga     1440
His Gly Thr Gly Thr Gly Ala Arg Trp Glu Leu Gln Pro Leu Ser Gly
465                 470                 475                 480

agg gcg tag                                                         1449
Arg Ala
```

<210> 102
<211> 482
<212> PRT
<213> Thalassiosira pseudonana

<400> 102

```
Met Pro Pro Asn Ala Glu Val Lys Asn Leu Arg Ser Arg Ser Ile Pro
1               5                   10                  15

Thr Lys Lys Ser Ser Ser Ser Ser Ser Thr Ala Asn Asp Asp Pro Ala
            20                  25                  30

Thr Gln Ser Thr Ser Pro Val Asn Arg Thr Leu Lys Ser Leu Asn Gly
        35                  40                  45

Asn Glu Ile Ala Ile Asp Gly Val Ile Tyr Asp Ile Asp Gly Phe Val
    50                  55                  60

His Pro Gly Gly Glu Val Ile Ser Phe Phe Gly Gly Asn Asp Val Thr
65                  70                  75                  80

Val Gln Tyr Lys Met Ile His Pro Tyr His Asn Ser Lys His Leu Glu
                85                  90                  95

Lys Met Arg Ala Val Gly Lys Ile Ala Asp Tyr Ser Thr Glu Tyr Lys
            100                 105                 110

Phe Asp Thr Pro Phe Glu Arg Glu Ile Lys Ser Glu Val Phe Lys Ile
        115                 120                 125

Val Arg Arg Gly Arg Glu Phe Gly Thr Thr Gly Tyr Phe Leu Arg Ala
    130                 135                 140

Phe Phe Tyr Ile Ala Leu Phe Phe Thr Met Gln Tyr Thr Phe Ala Thr
145                 150                 155                 160

Cys Thr Thr Phe Thr Thr Tyr Asp His Trp Tyr Gln Ser Gly Val Phe
                165                 170                 175

Ile Ala Ile Val Phe Gly Ile Ser Gln Ala Phe Ile Gly Leu Asn Val
            180                 185                 190

Gln His Asp Ala Asn His Gly Ala Ala Ser Lys Arg Pro Trp Val Asn
        195                 200                 205

Asp Leu Leu Gly Ser Gly Ala Asp Leu Ile Gly Gly Cys Lys Trp Asn
    210                 215                 220

Trp Leu Ala Gln His Trp Thr His His Ala Tyr Thr Asn His Ala Asp
225                 230                 235                 240

Lys Asp Pro Asp Ser Phe Ser Ser Glu Pro Val Phe Asn Phe Asn Asp
                245                 250                 255
```

```
Tyr Pro Ile Gly His Pro Lys Arg Lys Trp Trp His Arg Phe Gln Gly
            260                 265                 270

Leu Tyr Phe Leu Ile Met Leu Ser Phe Tyr Trp Val Ser Met Val Phe
        275                 280                 285

Asn Pro Gln Val Ile Asp Leu Arg His Ala Gly Ala Ala Tyr Val Gly
    290                 295                 300

Phe Gln Met Glu Asn Asp Phe Ile Val Lys Arg Arg Lys Tyr Ala Met
305                 310                 315                 320

Ala Leu Arg Ala Met Tyr Phe Tyr Phe Asn Ile Tyr Cys Pro Ile Val
                325                 330                 335

Asn Asn Gly Leu Thr Trp Ser Thr Val Gly Ile Ile Leu Leu Met Gly
            340                 345                 350

Val Ser Glu Ser Phe Met Leu Ser Gly Leu Phe Val Leu Ser His Asn
        355                 360                 365

Phe Glu Asn Ser Glu Arg Asp Pro Thr Ser Glu Tyr Arg Lys Thr Gly
    370                 375                 380

Glu Gln Val Cys Trp Phe Lys Ser Gln Val Glu Thr Ser Ser Thr Tyr
385                 390                 395                 400

Gly Gly Ile Val Ala Gly Cys Leu Thr Gly Gly Leu Asn Phe Gln Val
                405                 410                 415

Glu His His Leu Phe Pro Arg Met Ser Ser Ala Trp Tyr Pro Phe Ile
            420                 425                 430

Ala Pro Lys Val Arg Glu Ile Cys Lys Lys His Gly Val Arg Tyr Ala
        435                 440                 445

Tyr Tyr Pro Tyr Ile Trp Gln Asn Leu His Ser Thr Val Ser Tyr Met
    450                 455                 460

His Gly Thr Gly Thr Gly Ala Arg Trp Glu Leu Gln Pro Leu Ser Gly
465                 470                 475                 480

Arg Ala
```

<210> 103
<211> 1512
<212> DNA
<213> Thalassiosira pseudonana

<220>
<221> CDS
<222> (1)..(1512)
<223> Delta-4-Desaturase

<400> 103

```
atg tgc aac ggc aac ctc cca gca tcc acc gca cag ctc aag tcc acc      48
Met Cys Asn Gly Asn Leu Pro Ala Ser Thr Ala Gln Leu Lys Ser Thr
1               5                   10                  15

tcg aag ccc cag cag caa cat gag cat cgc acc atc tcc aag tcc gag      96
Ser Lys Pro Gln Gln Gln His Glu His Arg Thr Ile Ser Lys Ser Glu
            20                  25                  30

ctc gcc caa cac aac acg ccc aaa tca gca tgg tgt gcc gtc cac tcc      144
Leu Ala Gln His Asn Thr Pro Lys Ser Ala Trp Cys Ala Val His Ser
        35                  40                  45

act ccc gcc acc gac cca tcc cac tcc aac aac aaa caa cac gca cac      192
Thr Pro Ala Thr Asp Pro Ser His Ser Asn Asn Lys Gln His Ala His
    50                  55                  60

cta gtc ctc gac att acc gac ttt gcg tcc cgc cat cca ggg gga gac      240
Leu Val Leu Asp Ile Thr Asp Phe Ala Ser Arg His Pro Gly Gly Asp
65                  70                  75                  80

ctc atc ctc ctc gct tcc ggc aaa gac gcc tcg gtg ctg ttt gaa aca      288
Leu Ile Leu Leu Ala Ser Gly Lys Asp Ala Ser Val Leu Phe Glu Thr
                85                  90                  95

tac cat cca cgt gga gtt ccg acg tct ctc att caa aag ctg cag att      336
Tyr His Pro Arg Gly Val Pro Thr Ser Leu Ile Gln Lys Leu Gln Ile
            100                 105                 110

gga gtg atg gag gag gag gcg ttt cgg gat tcg ttt tac agt tgg act      384
Gly Val Met Glu Glu Glu Ala Phe Arg Asp Ser Phe Tyr Ser Trp Thr
        115                 120                 125

gat tct gac ttt tat act gtg ttg aag agg agg gtt gtg gag cgg ttg      432
Asp Ser Asp Phe Tyr Thr Val Leu Lys Arg Arg Val Val Glu Arg Leu
    130                 135                 140

gag gag agg ggg ttg gac agg agg gga tcg aaa gag att tgg atc aag      480
Glu Glu Arg Gly Leu Asp Arg Arg Gly Ser Lys Glu Ile Trp Ile Lys
145                 150                 155                 160

gct ttg ttc ttg ttg gtt gga ttt tgg tac tgt ttg tac aag atg tat      528
Ala Leu Phe Leu Leu Val Gly Phe Trp Tyr Cys Leu Tyr Lys Met Tyr
                165                 170                 175

act acg tcg gat atc gat cag tac ggt att gcc att gcc tat tct att      576
Thr Thr Ser Asp Ile Asp Gln Tyr Gly Ile Ala Ile Ala Tyr Ser Ile
            180                 185                 190

gga atg gga acc ttt gcg gca ttc atc ggc acg tgt att caa cac gat      624
Gly Met Gly Thr Phe Ala Ala Phe Ile Gly Thr Cys Ile Gln His Asp
        195                 200                 205

gga aat cac ggt gca ttc gct cag aac aag tta ctc aac aag ttg gct      672
```

```
Gly Asn His Gly Ala Phe Ala Gln Asn Lys Leu Leu Asn Lys Leu Ala
    210                 215                 220

ggg tgg acg ttg gat atg att ggt gcg agt gcg ttt acg tgg gag ctt      720
Gly Trp Thr Leu Asp Met Ile Gly Ala Ser Ala Phe Thr Trp Glu Leu
225                 230                 235                 240

cag cac atg ctg ggg cat cat cca tat acg aat gtg ttg gat ggg gtg      768
Gln His Met Leu Gly His His Pro Tyr Thr Asn Val Leu Asp Gly Val
                245                 250                 255

gag gag gag agg aag gag agg ggg gag gat gtt gct ttg gaa gaa aag      816
Glu Glu Glu Arg Lys Glu Arg Gly Glu Asp Val Ala Leu Glu Glu Lys
            260                 265                 270

gat cag gat ttt gaa gtt gcc aca tcc gga cga tta tat cat att gat      864
Asp Gln Asp Phe Glu Val Ala Thr Ser Gly Arg Leu Tyr His Ile Asp
        275                 280                 285

gcc aat gta cgt tat ggt tcg gta tgg aat gtc atg agg ttt tgg gct      912
Ala Asn Val Arg Tyr Gly Ser Val Trp Asn Val Met Arg Phe Trp Ala
    290                 295                 300

atg aag gtc att acg atg gga tat atg atg gga tta cca atc tac ttt      960
Met Lys Val Ile Thr Met Gly Tyr Met Met Gly Leu Pro Ile Tyr Phe
305                 310                 315                 320

cat gga gta ctg agg gga gtt gga ttg ttt gtt att ggg cat ttg gcg     1008
His Gly Val Leu Arg Gly Val Gly Leu Phe Val Ile Gly His Leu Ala
                325                 330                 335

tgt gga gag ttg ttg gcg acg atg ttt att gtg aat cac gtc att gag     1056
Cys Gly Glu Leu Leu Ala Thr Met Phe Ile Val Asn His Val Ile Glu
            340                 345                 350

ggt gtg agt tat gga acg aag gat ttg gtt ggt ggt gcg agt cat gta     1104
Gly Val Ser Tyr Gly Thr Lys Asp Leu Val Gly Gly Ala Ser His Val
        355                 360                 365

gat gag aag aag att gtc aag cca acg act gta ttg gga gat aca cca     1152
Asp Glu Lys Lys Ile Val Lys Pro Thr Thr Val Leu Gly Asp Thr Pro
    370                 375                 380

atg gta aag act cgc gag gag gca ttg aaa agc aac agc aat aac aac     1200
Met Val Lys Thr Arg Glu Glu Ala Leu Lys Ser Asn Ser Asn Asn Asn
385                 390                 395                 400

aag aag aag gga gag aag aac tcg gta cca tcc gtt cca ttc aac gac     1248
Lys Lys Lys Gly Glu Lys Asn Ser Val Pro Ser Val Pro Phe Asn Asp
                405                 410                 415

tgg gca gca gtc caa tgc cag acc tcc gtg aat tgg tct cca ggc tca     1296
Trp Ala Ala Val Gln Cys Gln Thr Ser Val Asn Trp Ser Pro Gly Ser
            420                 425                 430

tgg ttc tgg aat cac ttt tct ggg gga ctc tct cat cag att gag cat     1344
Trp Phe Trp Asn His Phe Ser Gly Gly Leu Ser His Gln Ile Glu His
        435                 440                 445

cac ttg ttc ccc agc att tgt cat aca aac tac tgt cat atc cag gat     1392
His Leu Phe Pro Ser Ile Cys His Thr Asn Tyr Cys His Ile Gln Asp
    450                 455                 460

gtt gtg gag agt acg tgt gct gag tac gga gtt ccg tat cag agt gag     1440
Val Val Glu Ser Thr Cys Ala Glu Tyr Gly Val Pro Tyr Gln Ser Glu
465                 470                 475                 480

agt aat ttg ttt gtt gct tat gga aag atg att agt cat ttg aag ttt     1488
```

```
Ser Asn Leu Phe Val Ala Tyr Gly Lys Met Ile Ser His Leu Lys Phe
                485                 490                 495

ttg ggt aaa gcc aag tgt gag tag                                        1512
Leu Gly Lys Ala Lys Cys Glu
            500
```

<210> 104
<211> 503
<212> PRT
<213> Thalassiosira pseudonana

<400> 104

```
Met Cys Asn Gly Asn Leu Pro Ala Ser Thr Ala Gln Leu Lys Ser Thr
1               5                   10                  15

Ser Lys Pro Gln Gln Gln His Glu His Arg Thr Ile Ser Lys Ser Glu
            20                  25                  30

Leu Ala Gln His Asn Thr Pro Lys Ser Ala Trp Cys Ala Val His Ser
        35                  40                  45

Thr Pro Ala Thr Asp Pro Ser His Ser Asn Asn Lys Gln His Ala His
    50                  55                  60

Leu Val Leu Asp Ile Thr Asp Phe Ala Ser Arg His Pro Gly Gly Asp
65                  70                  75                  80

Leu Ile Leu Leu Ala Ser Gly Lys Asp Ala Ser Val Leu Phe Glu Thr
                85                  90                  95

Tyr His Pro Arg Gly Val Pro Thr Ser Leu Ile Gln Lys Leu Gln Ile
            100                 105                 110

Gly Val Met Glu Glu Glu Ala Phe Arg Asp Ser Phe Tyr Ser Trp Thr
        115                 120                 125

Asp Ser Asp Phe Tyr Thr Val Leu Lys Arg Arg Val Val Glu Arg Leu
    130                 135                 140

Glu Glu Arg Gly Leu Asp Arg Arg Gly Ser Lys Glu Ile Trp Ile Lys
145                 150                 155                 160

Ala Leu Phe Leu Leu Val Gly Phe Trp Tyr Cys Leu Tyr Lys Met Tyr
                165                 170                 175

Thr Thr Ser Asp Ile Asp Gln Tyr Gly Ile Ala Ile Ala Tyr Ser Ile
            180                 185                 190
```

Gly Met Gly Thr Phe Ala Ala Phe Ile Gly Thr Cys Ile Gln His Asp
195 200 205

Gly Asn His Gly Ala Phe Ala Gln Asn Lys Leu Leu Asn Lys Leu Ala
210 215 220

Gly Trp Thr Leu Asp Met Ile Gly Ala Ser Ala Phe Thr Trp Glu Leu
225 230 235 240

Gln His Met Leu Gly His His Pro Tyr Thr Asn Val Leu Asp Gly Val
245 250 255

Glu Glu Glu Arg Lys Glu Arg Gly Glu Asp Val Ala Leu Glu Glu Lys
260 265 270

Asp Gln Asp Phe Glu Val Ala Thr Ser Gly Arg Leu Tyr His Ile Asp
275 280 285

Ala Asn Val Arg Tyr Gly Ser Val Trp Asn Val Met Arg Phe Trp Ala
290 295 300

Met Lys Val Ile Thr Met Gly Tyr Met Met Gly Leu Pro Ile Tyr Phe
305 310 315 320

His Gly Val Leu Arg Gly Val Gly Leu Phe Val Ile Gly His Leu Ala
325 330 335

Cys Gly Glu Leu Leu Ala Thr Met Phe Ile Val Asn His Val Ile Glu
340 345 350

Gly Val Ser Tyr Gly Thr Lys Asp Leu Val Gly Gly Ala Ser His Val
355 360 365

Asp Glu Lys Lys Ile Val Lys Pro Thr Thr Val Leu Gly Asp Thr Pro
370 375 380

Met Val Lys Thr Arg Glu Glu Ala Leu Lys Ser Asn Ser Asn Asn Asn
385 390 395 400

Lys Lys Lys Gly Glu Lys Asn Ser Val Pro Ser Val Pro Phe Asn Asp
405 410 415

Trp Ala Ala Val Gln Cys Gln Thr Ser Val Asn Trp Ser Pro Gly Ser
420 425 430

Trp Phe Trp Asn His Phe Ser Gly Gly Leu Ser His Gln Ile Glu His
435 440 445

His Leu Phe Pro Ser Ile Cys His Thr Asn Tyr Cys His Ile Gln Asp
450 455 460

Val Val Glu Ser Thr Cys Ala Glu Tyr Gly Val Pro Tyr Gln Ser Glu
465 470 475 480

Ser Asn Leu Phe Val Ala Tyr Gly Lys Met Ile Ser His Leu Lys Phe
485 490 495

Leu Gly Lys Ala Lys Cys Glu
500

<210> 105
<211> 1257
<212> DNA
<213> Thalassiosira pseudonana

<220>
<221> CDS
<222> (1)..(1257)
<223> Omega-3-Desaturase

<400> 105

atg tac aga tta aca tcc acc ttc ctc atc gca ttg gca ttc tcc tcc 48
Met Tyr Arg Leu Thr Ser Thr Phe Leu Ile Ala Leu Ala Phe Ser Ser
1 5 10 15

tcc atc aat gcc ttc tct cca caa cgg cca cca cgt act atc acc aaa 96
Ser Ile Asn Ala Phe Ser Pro Gln Arg Pro Pro Arg Thr Ile Thr Lys
20 25 30

agt aaa gtc caa agc acc gtg cta ccc ata ccg acc aag gat gat ctg 144
Ser Lys Val Gln Ser Thr Val Leu Pro Ile Pro Thr Lys Asp Asp Leu
35 40 45

aac ttt ctc caa cca caa ctc gat gag aat gat ctc tac ctc gac gat 192
Asn Phe Leu Gln Pro Gln Leu Asp Glu Asn Asp Leu Tyr Leu Asp Asp
50 55 60

gtc aac act cca cca aga gca ggt acc atc atg aag atg ttg ccg aag 240
Val Asn Thr Pro Pro Arg Ala Gly Thr Ile Met Lys Met Leu Pro Lys
65 70 75 80

gaa acg ttc aac att gat aca gca act tca ttg ggt tac ttt ggt atg 288
Glu Thr Phe Asn Ile Asp Thr Ala Thr Ser Leu Gly Tyr Phe Gly Met
85 90 95

gat atg gca gcg gtt gta tcg tcc atg acg ttg cta aat gct att gta 336
Asp Met Ala Ala Val Val Ser Ser Met Thr Leu Leu Asn Ala Ile Val
100 105 110

act tcg gat cag tac cat gct ctt cca ctt cct ctc caa gca gca aca 384
Thr Ser Asp Gln Tyr His Ala Leu Pro Leu Pro Leu Gln Ala Ala Thr
115 120 125

gtg att ccc ttt cag cta ttg gct ggg ttc gcc atg tgg tgt atg tgg 432
Val Ile Pro Phe Gln Leu Leu Ala Gly Phe Ala Met Trp Cys Met Trp
130 135 140

```
tgc att gga cac gat gct gga cat tct act gtt tcg aag aca aag tgg        480
Cys Ile Gly His Asp Ala Gly His Ser Thr Val Ser Lys Thr Lys Trp
145                 150                 155                 160

atc aac cga gtc gtt ggt gaa gtg gct cat tct gtt gtt tgt ctc acg        528
Ile Asn Arg Val Val Gly Glu Val Ala His Ser Val Val Cys Leu Thr
            165                 170                 175

ccg ttc gtg cct tgg cag atg tcg cat agg aaa cac cat ttg aat cac        576
Pro Phe Val Pro Trp Gln Met Ser His Arg Lys His His Leu Asn His
        180                 185                 190

aat cat att gaa aag gac tac tct cat aag tgg tac agt cgc gac gag        624
Asn His Ile Glu Lys Asp Tyr Ser His Lys Trp Tyr Ser Arg Asp Glu
    195                 200                 205

ttt gat gat atc cca caa ctc tat aag aca ttt ggc tac aac cca aga        672
Phe Asp Asp Ile Pro Gln Leu Tyr Lys Thr Phe Gly Tyr Asn Pro Arg
    210                 215                 220

atg atg caa ctt cca ttc ctc tac ttc atg tat ctt gca ttg gga att        720
Met Met Gln Leu Pro Phe Leu Tyr Phe Met Tyr Leu Ala Leu Gly Ile
225                 230                 235                 240

cca gat ggt ggg cat gtt gtg ttc tac gga aga atg tgg gaa gga gtg        768
Pro Asp Gly Gly His Val Val Phe Tyr Gly Arg Met Trp Glu Gly Val
            245                 250                 255

tca ttg cag aag aag ttt gat gct gct att tct gtg gcc gta tca tgt        816
Ser Leu Gln Lys Lys Phe Asp Ala Ala Ile Ser Val Ala Val Ser Cys
        260                 265                 270

gca act gct gga tcg ctt tgg atg aat atg ggt aca gca gac ttc acg        864
Ala Thr Ala Gly Ser Leu Trp Met Asn Met Gly Thr Ala Asp Phe Thr
    275                 280                 285

gtg gta tgc atg gtt cct tgg cta gtt cta tcg tgg tgg ctc ttc atg        912
Val Val Cys Met Val Pro Trp Leu Val Leu Ser Trp Trp Leu Phe Met
    290                 295                 300

gta aca tac ctt cag cat cat tca gaa gac gga aag cta tac act gat        960
Val Thr Tyr Leu Gln His His Ser Glu Asp Gly Lys Leu Tyr Thr Asp
305                 310                 315                 320

gaa acg ttt aca ttt gaa aag gga gcc ttc gag acc gtg gat cgt tcg       1008
Glu Thr Phe Thr Phe Glu Lys Gly Ala Phe Glu Thr Val Asp Arg Ser
            325                 330                 335

tac ggc aag ttg atc aac cga atg tcg cat cac atg atg gac ggt cac       1056
Tyr Gly Lys Leu Ile Asn Arg Met Ser His His Met Met Asp Gly His
        340                 345                 350

gtg gtg cac cac ttg ttc ttt gaa cgt gta cct cac tac aga tta gag       1104
Val Val His His Leu Phe Phe Glu Arg Val Pro His Tyr Arg Leu Glu
    355                 360                 365

gca gct acc gaa gct ctt gtg aaa gga atg gat gaa acg gga cag aaa       1152
Ala Ala Thr Glu Ala Leu Val Lys Gly Met Asp Glu Thr Gly Gln Lys
    370                 375                 380

cat ttg tac aaa tac att gat act cct gat ttc aat gcc gag att gtc       1200
His Leu Tyr Lys Tyr Ile Asp Thr Pro Asp Phe Asn Ala Glu Ile Val
385                 390                 395                 400

aac gga ttt cgc gac aat tgg ttc ctt gtt gaa gag gag aac atc aaa       1248
Asn Gly Phe Arg Asp Asn Trp Phe Leu Val Glu Glu Glu Asn Ile Lys
            405                 410                 415
```

```
agg gag tag                                                      1257
Arg Glu
```

<210> 106
<211> 418
<212> PRT
<213> Thalassiosira pseudonana

<400> 106

```
Met Tyr Arg Leu Thr Ser Thr Phe Leu Ile Ala Leu Ala Phe Ser Ser
1               5                   10                  15

Ser Ile Asn Ala Phe Ser Pro Gln Arg Pro Pro Arg Thr Ile Thr Lys
            20                  25                  30

Ser Lys Val Gln Ser Thr Val Leu Pro Ile Pro Thr Lys Asp Asp Leu
        35                  40                  45

Asn Phe Leu Gln Pro Gln Leu Asp Glu Asn Asp Leu Tyr Leu Asp Asp
    50                  55                  60

Val Asn Thr Pro Pro Arg Ala Gly Thr Ile Met Lys Met Leu Pro Lys
65                  70                  75                  80

Glu Thr Phe Asn Ile Asp Thr Ala Thr Ser Leu Gly Tyr Phe Gly Met
                85                  90                  95

Asp Met Ala Ala Val Val Ser Ser Met Thr Leu Leu Asn Ala Ile Val
            100                 105                 110

Thr Ser Asp Gln Tyr His Ala Leu Pro Leu Pro Leu Gln Ala Ala Thr
        115                 120                 125

Val Ile Pro Phe Gln Leu Leu Ala Gly Phe Ala Met Trp Cys Met Trp
    130                 135                 140

Cys Ile Gly His Asp Ala Gly His Ser Thr Val Ser Lys Thr Lys Trp
145                 150                 155                 160

Ile Asn Arg Val Val Gly Glu Val Ala His Ser Val Val Cys Leu Thr
                165                 170                 175

Pro Phe Val Pro Trp Gln Met Ser His Arg Lys His His Leu Asn His
            180                 185                 190

Asn His Ile Glu Lys Asp Tyr Ser His Lys Trp Tyr Ser Arg Asp Glu
        195                 200                 205
```

Phe Asp Asp Ile Pro Gln Leu Tyr Lys Thr Phe Gly Tyr Asn Pro Arg
210 215 220

Met Met Gln Leu Pro Phe Leu Tyr Phe Met Tyr Leu Ala Leu Gly Ile
225 230 235 240

Pro Asp Gly Gly His Val Val Phe Tyr Gly Arg Met Trp Glu Gly Val
245 250 255

Ser Leu Gln Lys Lys Phe Asp Ala Ala Ile Ser Val Ala Val Ser Cys
260 265 270

Ala Thr Ala Gly Ser Leu Trp Met Asn Met Gly Thr Ala Asp Phe Thr
275 280 285

Val Val Cys Met Val Pro Trp Leu Val Leu Ser Trp Trp Leu Phe Met
290 295 300

Val Thr Tyr Leu Gln His His Ser Glu Asp Gly Lys Leu Tyr Thr Asp
305 310 315 320

Glu Thr Phe Thr Phe Glu Lys Gly Ala Phe Glu Thr Val Asp Arg Ser
325 330 335

Tyr Gly Lys Leu Ile Asn Arg Met Ser His His Met Met Asp Gly His
340 345 350

Val Val His His Leu Phe Phe Glu Arg Val Pro His Tyr Arg Leu Glu
355 360 365

Ala Ala Thr Glu Ala Leu Val Lys Gly Met Asp Glu Thr Gly Gln Lys
370 375 380

His Leu Tyr Lys Tyr Ile Asp Thr Pro Asp Phe Asn Ala Glu Ile Val
385 390 395 400

Asn Gly Phe Arg Asp Asn Trp Phe Leu Val Glu Glu Glu Asn Ile Lys
405 410 415

Arg Glu

<210> 107
<211> 1086
<212> DNA
<213> Ostreococcus tauri

<220>
<221> CDS
<222> (1)..(1086)
<223> Delta-12-Desaturase

<400> 107

```
atg cag gag ggg gtg cga aac att ccg aac gag tgc ttt gag acg gga      48
Met Gln Glu Gly Val Arg Asn Ile Pro Asn Glu Cys Phe Glu Thr Gly
1               5                   10                  15

cat ctt gaa aga ccc tgg cgt tcc ggc cgg tgt ggg cgc gat ccc ggt      96
His Leu Glu Arg Pro Trp Arg Ser Gly Arg Cys Gly Arg Asp Pro Gly
            20                  25                  30

tcg aat tgg ggc gct ggc ttc cgc ttt ttt tcg ctc aag ggg ttt tgg      144
Ser Asn Trp Gly Ala Gly Phe Arg Phe Phe Ser Leu Lys Gly Phe Trp
        35                  40                  45

tgg ccg gcg tgg tgg gcg tac gcg ttc gtg acg ggg acg gcg gcc act      192
Trp Pro Ala Trp Trp Ala Tyr Ala Phe Val Thr Gly Thr Ala Ala Thr
    50                  55                  60

ggg tgt tgg gtc gcc gcg cac gag tgc ggg cac ggc gcg ttc agc gat      240
Gly Cys Trp Val Ala Ala His Glu Cys Gly His Gly Ala Phe Ser Asp
65                  70                  75                  80

aac aag acg ttg caa gat gcg gtt gga tac gtg ttg cac tcg ttg ctc      288
Asn Lys Thr Leu Gln Asp Ala Val Gly Tyr Val Leu His Ser Leu Leu
                85                  90                  95

ttg gtg ccg tac ttt tct tgg cag cga tca cac gcg gtg cat cac tcg      336
Leu Val Pro Tyr Phe Ser Trp Gln Arg Ser His Ala Val His His Ser
            100                 105                 110

agg acg aat cac gtt ctt gag ggc gag acg cac gtg ccg gcg cgc ttg      384
Arg Thr Asn His Val Leu Glu Gly Glu Thr His Val Pro Ala Arg Leu
        115                 120                 125

ggg acg gaa gac gcc aac gtc gtg ttc aag ctt cgc gaa ttg atc ggt      432
Gly Thr Glu Asp Ala Asn Val Val Phe Lys Leu Arg Glu Leu Ile Gly
    130                 135                 140

gaa ggg ccg ttc acg ttt ttc aac ctc gtc ggc gtc ttc gcg ctc gga      480
Glu Gly Pro Phe Thr Phe Phe Asn Leu Val Gly Val Phe Ala Leu Gly
145                 150                 155                 160

tgg ccg att tac ttg ctc acc ggc gcg agc ggc gga ccg gtg cgc ggt      528
Trp Pro Ile Tyr Leu Leu Thr Gly Ala Ser Gly Gly Pro Val Arg Gly
                165                 170                 175

aac acg aac cac ttc tta ccc ttc atg ggc gag aaa ggt aag cac gcg      576
Asn Thr Asn His Phe Leu Pro Phe Met Gly Glu Lys Gly Lys His Ala
            180                 185                 190

ctg ttc ccg ggt aag tgg gcg aag aag gtg tgg cag tct gac atc ggc      624
Leu Phe Pro Gly Lys Trp Ala Lys Lys Val Trp Gln Ser Asp Ile Gly
        195                 200                 205

gtt gtt gcc gtc ctg ggc gcg ctc gcg gct tgg gcg gcg cac agc ggg      672
Val Val Ala Val Leu Gly Ala Leu Ala Ala Trp Ala Ala His Ser Gly
    210                 215                 220

att gcc aca gtg atg gca ctc tac gtc ggc ccg tac atg gtg acc aac      720
```

```
Ile Ala Thr Val Met Ala Leu Tyr Val Gly Pro Tyr Met Val Thr Asn
225                 230                 235                 240

ttt tgg ctc gtc ttg tac acg tgg tta cag cac acc gac gtt gac gtg      768
Phe Trp Leu Val Leu Tyr Thr Trp Leu Gln His Thr Asp Val Asp Val
                245                 250                 255

ccg cac ttc gag ggc gac gat tgg aac ttg gtc aag ggg gca ttc atg      816
Pro His Phe Glu Gly Asp Asp Trp Asn Leu Val Lys Gly Ala Phe Met
            260                 265                 270

acg atc gat cgc ccg tac ggc cca gtt ttt gat ttc ttg cac cac cgc      864
Thr Ile Asp Arg Pro Tyr Gly Pro Val Phe Asp Phe Leu His His Arg
        275                 280                 285

atc ggc agc acg cac gtc gcg cac cac atc aac aca cca ttc ccg cat      912
Ile Gly Ser Thr His Val Ala His His Ile Asn Thr Pro Phe Pro His
    290                 295                 300

tac aag gct caa atg gcg acg gat gcg cta aag gag gcg tat ccc gac      960
Tyr Lys Ala Gln Met Ala Thr Asp Ala Leu Lys Glu Ala Tyr Pro Asp
305                 310                 315                 320

ctc tac ctt tac gat cca act ccg atc gcg acc gct acg tgg cgc gtg     1008
Leu Tyr Leu Tyr Asp Pro Thr Pro Ile Ala Thr Ala Thr Trp Arg Val
                325                 330                 335

ggg agc aag tgc atc gcc gtc gtg aag aag gga gac gaa tgg gtg ttc     1056
Gly Ser Lys Cys Ile Ala Val Val Lys Lys Gly Asp Glu Trp Val Phe
            340                 345                 350

acg gat aag caa ctc ccg gtc gcg gcg tga                             1086
Thr Asp Lys Gln Leu Pro Val Ala Ala
        355                 360
```

<210> 108
<211> 361
<212> PRT
<213> Ostreococcus tauri

<400> 108

```
Met Gln Glu Gly Val Arg Asn Ile Pro Asn Glu Cys Phe Glu Thr Gly
1               5                   10                  15

His Leu Glu Arg Pro Trp Arg Ser Gly Arg Cys Gly Arg Asp Pro Gly
            20                  25                  30

Ser Asn Trp Gly Ala Gly Phe Arg Phe Phe Ser Leu Lys Gly Phe Trp
        35                  40                  45

Trp Pro Ala Trp Trp Ala Tyr Ala Phe Val Thr Gly Thr Ala Ala Thr
    50                  55                  60

Gly Cys Trp Val Ala Ala His Glu Cys Gly His Gly Ala Phe Ser Asp
65                  70                  75                  80
```

Asn Lys Thr Leu Gln Asp Ala Val Gly Tyr Val Leu His Ser Leu Leu
85 90 95

Leu Val Pro Tyr Phe Ser Trp Gln Arg Ser His Ala Val His His Ser
100 105 110

Arg Thr Asn His Val Leu Glu Gly Glu Thr His Val Pro Ala Arg Leu
115 120 125

Gly Thr Glu Asp Ala Asn Val Val Phe Lys Leu Arg Glu Leu Ile Gly
130 135 140

Glu Gly Pro Phe Thr Phe Phe Asn Leu Val Gly Val Phe Ala Leu Gly
145 150 155 160

Trp Pro Ile Tyr Leu Leu Thr Gly Ala Ser Gly Gly Pro Val Arg Gly
165 170 175

Asn Thr Asn His Phe Leu Pro Phe Met Gly Glu Lys Gly Lys His Ala
180 185 190

Leu Phe Pro Gly Lys Trp Ala Lys Lys Val Trp Gln Ser Asp Ile Gly
195 200 205

Val Val Ala Val Leu Gly Ala Leu Ala Ala Trp Ala Ala His Ser Gly
210 215 220

Ile Ala Thr Val Met Ala Leu Tyr Val Gly Pro Tyr Met Val Thr Asn
225 230 235 240

Phe Trp Leu Val Leu Tyr Thr Trp Leu Gln His Thr Asp Val Asp Val
245 250 255

Pro His Phe Glu Gly Asp Asp Trp Asn Leu Val Lys Gly Ala Phe Met
260 265 270

Thr Ile Asp Arg Pro Tyr Gly Pro Val Phe Asp Phe Leu His His Arg
275 280 285

Ile Gly Ser Thr His Val Ala His His Ile Asn Thr Pro Phe Pro His
290 295 300

Tyr Lys Ala Gln Met Ala Thr Asp Ala Leu Lys Glu Ala Tyr Pro Asp
305 310 315 320

Leu Tyr Leu Tyr Asp Pro Thr Pro Ile Ala Thr Ala Thr Trp Arg Val
325 330 335

Gly Ser Lys Cys Ile Ala Val Val Lys Lys Gly Asp Glu Trp Val Phe
340 345 350

Thr Asp Lys Gln Leu Pro Val Ala Ala
355 360

<210> 109
<211> 1305
<212> DNA
<213> Thalassiosira pseudonana

<220>
<221> CDS
<222> (1)..(1305)
<223> Delta-12-Desaturase

<400> 109

```
atg gga aag gga gga aga tca gta acc cgc gct caa aca gca gaa aag      48
Met Gly Lys Gly Gly Arg Ser Val Thr Arg Ala Gln Thr Ala Glu Lys
1               5                   10                  15

tca gca cac acc atc caa acc ttc acc gac ggc cga tgg gtc tcc ccc      96
Ser Ala His Thr Ile Gln Thr Phe Thr Asp Gly Arg Trp Val Ser Pro
            20                  25                  30

tac aac ccc ctc gca aaa gat gca cct gaa ctc ccc tcc aag ggt gaa      144
Tyr Asn Pro Leu Ala Lys Asp Ala Pro Glu Leu Pro Ser Lys Gly Glu
        35                  40                  45

atc aag gcg gtc atc ccc aaa gag tgc ttc gaa cga agc tac ctc cac      192
Ile Lys Ala Val Ile Pro Lys Glu Cys Phe Glu Arg Ser Tyr Leu His
    50                  55                  60

tcc atg tac ttc gtc ctc cgt gac acc gtc atg gcc gtg gcc tgc gcc      240
Ser Met Tyr Phe Val Leu Arg Asp Thr Val Met Ala Val Ala Cys Ala
65                  70                  75                  80

tac atc gcc cac tca acg ctc tcc acc gat att ccc tcc gag tta ctg      288
Tyr Ile Ala His Ser Thr Leu Ser Thr Asp Ile Pro Ser Glu Leu Leu
                85                  90                  95

agc gtg gac gca ctc aaa tgg ttc ctc gga tgg aac acc tac gcc ttt      336
Ser Val Asp Ala Leu Lys Trp Phe Leu Gly Trp Asn Thr Tyr Ala Phe
            100                 105                 110

tgg atg ggg tgc att ctc acc gga cac tgg gtc cta gcc cat gaa tgt      384
Trp Met Gly Cys Ile Leu Thr Gly His Trp Val Leu Ala His Glu Cys
        115                 120                 125

gga cat ggt gca ttc tct ccc tct cag acg ttt aat gac ttt tgg ggg      432
Gly His Gly Ala Phe Ser Pro Ser Gln Thr Phe Asn Asp Phe Trp Gly
    130                 135                 140

ttc att atg cat cag gcg gtg ttg gtt ccg tat ttc gcc tgg cag tac      480
Phe Ile Met His Gln Ala Val Leu Val Pro Tyr Phe Ala Trp Gln Tyr
145                 150                 155                 160

tct cat gcg aag cat cat cga cgt acc aac aac att atg gat ggg gag      528
Ser His Ala Lys His His Arg Arg Thr Asn Asn Ile Met Asp Gly Glu
                165                 170                 175
```

```
agc cat gtg ccc aat atc gcc aag gaa atg gga ttg aac gag aag aat      576
Ser His Val Pro Asn Ile Ala Lys Glu Met Gly Leu Asn Glu Lys Asn
            180                 185                 190

gag cgc agt gga gga tat gcc gcc att cat gag gct att gga gat gga      624
Glu Arg Ser Gly Gly Tyr Ala Ala Ile His Glu Ala Ile Gly Asp Gly
        195                 200                 205

ccc ttt gcg atg ttt caa atc ttt gct cac ttg gtg atc ggg tgg cct      672
Pro Phe Ala Met Phe Gln Ile Phe Ala His Leu Val Ile Gly Trp Pro
    210                 215                 220

att tac ttg atg gga ttt gct tcc act gga cgt ctc ggt cag gat ggg      720
Ile Tyr Leu Met Gly Phe Ala Ser Thr Gly Arg Leu Gly Gln Asp Gly
225                 230                 235                 240

aag gaa ctt cag gct gga gag atc atc gac cat tac cgt cct tgg agt      768
Lys Glu Leu Gln Ala Gly Glu Ile Ile Asp His Tyr Arg Pro Trp Ser
                245                 250                 255

aag atg ttc ccc acc aag ttg cga ttc aaa att gct ctt tcg aca ctt      816
Lys Met Phe Pro Thr Lys Leu Arg Phe Lys Ile Ala Leu Ser Thr Leu
            260                 265                 270

gga gtg att gcc gcc tgg gtt ggg ttg tac ttt gct gca caa gag tat      864
Gly Val Ile Ala Ala Trp Val Gly Leu Tyr Phe Ala Ala Gln Glu Tyr
        275                 280                 285

gga gtc ttg ccc gtg gtt ctt tgg tac att ggc cca ctc atg tgg aat      912
Gly Val Leu Pro Val Val Leu Trp Tyr Ile Gly Pro Leu Met Trp Asn
    290                 295                 300

cag gcg tgg ctt gtg ctc tac act tgg ctt cag cac aat gat ccc tcc      960
Gln Ala Trp Leu Val Leu Tyr Thr Trp Leu Gln His Asn Asp Pro Ser
305                 310                 315                 320

gtg cct caa tat gga agt gac gaa tgg aca tgg gtc aag gga gct ttg     1008
Val Pro Gln Tyr Gly Ser Asp Glu Trp Thr Trp Val Lys Gly Ala Leu
                325                 330                 335

tcg acg att gat cgc ccg tat ggt atc ttt gac ttc ttc cat cac aag     1056
Ser Thr Ile Asp Arg Pro Tyr Gly Ile Phe Asp Phe Phe His His Lys
            340                 345                 350

att gga agc act cac gta gct cat cat ttg ttc cac gag atg cca ttt     1104
Ile Gly Ser Thr His Val Ala His His Leu Phe His Glu Met Pro Phe
        355                 360                 365

tac aag gcg gat gtg gct act gcg tcg atc aag ggt ttc ttg gag ccg     1152
Tyr Lys Ala Asp Val Ala Thr Ala Ser Ile Lys Gly Phe Leu Glu Pro
    370                 375                 380

aag gga ctt tac aac tat gat cca acg cct tgg tat gtg gcc atg tgg     1200
Lys Gly Leu Tyr Asn Tyr Asp Pro Thr Pro Trp Tyr Val Ala Met Trp
385                 390                 395                 400

agg gtg gcc aag act tgt cat tat att gag gat gtg gat gga gtt cag     1248
Arg Val Ala Lys Thr Cys His Tyr Ile Glu Asp Val Asp Gly Val Gln
                405                 410                 415

tat tat aag agt ttg gag gat gtg cct ttg aag aag gat gcc aag aag     1296
Tyr Tyr Lys Ser Leu Glu Asp Val Pro Leu Lys Lys Asp Ala Lys Lys
            420                 425                 430

tct gat tag                                                         1305
Ser Asp
```

<210> 110
<211> 434

<212> PRT
<213> Thalassiosira pseudonana

<400> 110

Met Gly Lys Gly Gly Arg Ser Val Thr Arg Ala Gln Thr Ala Glu Lys
1               5                   10                  15

Ser Ala His Thr Ile Gln Thr Phe Thr Asp Gly Arg Trp Val Ser Pro
            20                  25                  30

Tyr Asn Pro Leu Ala Lys Asp Ala Pro Glu Leu Pro Ser Lys Gly Glu
        35                  40                  45

Ile Lys Ala Val Ile Pro Lys Glu Cys Phe Glu Arg Ser Tyr Leu His
    50                  55                  60

Ser Met Tyr Phe Val Leu Arg Asp Thr Val Met Ala Val Ala Cys Ala
65                  70                  75                  80

Tyr Ile Ala His Ser Thr Leu Ser Thr Asp Ile Pro Ser Glu Leu Leu
                85                  90                  95

Ser Val Asp Ala Leu Lys Trp Phe Leu Gly Trp Asn Thr Tyr Ala Phe
            100                 105                 110

Trp Met Gly Cys Ile Leu Thr Gly His Trp Val Leu Ala His Glu Cys
        115                 120                 125

Gly His Gly Ala Phe Ser Pro Ser Gln Thr Phe Asn Asp Phe Trp Gly
    130                 135                 140

Phe Ile Met His Gln Ala Val Leu Val Pro Tyr Phe Ala Trp Gln Tyr
145                 150                 155                 160

Ser His Ala Lys His His Arg Arg Thr Asn Asn Ile Met Asp Gly Glu
                165                 170                 175

Ser His Val Pro Asn Ile Ala Lys Glu Met Gly Leu Asn Glu Lys Asn
            180                 185                 190

Glu Arg Ser Gly Gly Tyr Ala Ala Ile His Glu Ala Ile Gly Asp Gly
        195                 200                 205

Pro Phe Ala Met Phe Gln Ile Phe Ala His Leu Val Ile Gly Trp Pro
    210                 215                 220

Ile Tyr Leu Met Gly Phe Ala Ser Thr Gly Arg Leu Gly Gln Asp Gly
225 230 235 240

Lys Glu Leu Gln Ala Gly Glu Ile Ile Asp His Tyr Arg Pro Trp Ser
245 250 255

Lys Met Phe Pro Thr Lys Leu Arg Phe Lys Ile Ala Leu Ser Thr Leu
260 265 270

Gly Val Ile Ala Ala Trp Val Gly Leu Tyr Phe Ala Ala Gln Glu Tyr
275 280 285

Gly Val Leu Pro Val Val Leu Trp Tyr Ile Gly Pro Leu Met Trp Asn
290 295 300

Gln Ala Trp Leu Val Leu Tyr Thr Trp Leu Gln His Asn Asp Pro Ser
305 310 315 320

Val Pro Gln Tyr Gly Ser Asp Glu Trp Thr Trp Val Lys Gly Ala Leu
325 330 335

Ser Thr Ile Asp Arg Pro Tyr Gly Ile Phe Asp Phe Phe His His Lys
340 345 350

Ile Gly Ser Thr His Val Ala His His Leu Phe His Glu Met Pro Phe
355 360 365

Tyr Lys Ala Asp Val Ala Thr Ala Ser Ile Lys Gly Phe Leu Glu Pro
370 375 380

Lys Gly Leu Tyr Asn Tyr Asp Pro Thr Pro Trp Tyr Val Ala Met Trp
385 390 395 400

Arg Val Ala Lys Thr Cys His Tyr Ile Glu Asp Val Asp Gly Val Gln
405 410 415

Tyr Tyr Lys Ser Leu Glu Asp Val Pro Leu Lys Lys Asp Ala Lys Lys
420 425 430

Ser Asp

<210> 111
<211> 879
<212> DNA
<213> Ostreococcus tauri

<220>
<221> CDS
<222> (1)..(879)
<223> Delta-6-Elongase

<400> 111

```
atg agt ggc tta cgt gca ccc aac ttt tta cac aga ttc tgg aca aag      48
Met Ser Gly Leu Arg Ala Pro Asn Phe Leu His Arg Phe Trp Thr Lys
1               5                   10                  15

tgg gac tac gcg att tcc aaa gtc gtc ttc acg tgt gcc gac agt ttt      96
Trp Asp Tyr Ala Ile Ser Lys Val Val Phe Thr Cys Ala Asp Ser Phe
            20                  25                  30

cag tgg gac atc ggg cca gtg agt tcg agt acg gcg cat tta ccc gcc     144
Gln Trp Asp Ile Gly Pro Val Ser Ser Ser Thr Ala His Leu Pro Ala
        35                  40                  45

att gaa tcc cct acc cca ctg gtg act agc ctc ttg ttc tac tta gtc     192
Ile Glu Ser Pro Thr Pro Leu Val Thr Ser Leu Leu Phe Tyr Leu Val
    50                  55                  60

aca gtt ttc ttg tgg tat ggt cgt tta acc agg agt tca gac aag aaa     240
Thr Val Phe Leu Trp Tyr Gly Arg Leu Thr Arg Ser Ser Asp Lys Lys
65                  70                  75                  80

att aga gag cct acg tgg tta aga aga ttc ata ata tgt cat aat gcg     288
Ile Arg Glu Pro Thr Trp Leu Arg Arg Phe Ile Ile Cys His Asn Ala
                85                  90                  95

ttc ttg ata gtc ctc agt ctt tac atg tgc ctt ggt tgt gtg gcc caa     336
Phe Leu Ile Val Leu Ser Leu Tyr Met Cys Leu Gly Cys Val Ala Gln
            100                 105                 110

gcg tat cag aat gga tat act tta tgg ggt aat gaa ttc aag gcc acg     384
Ala Tyr Gln Asn Gly Tyr Thr Leu Trp Gly Asn Glu Phe Lys Ala Thr
        115                 120                 125

gaa act cag ctt gct ctc tac att tac att ttt tac gta agt aaa ata     432
Glu Thr Gln Leu Ala Leu Tyr Ile Tyr Ile Phe Tyr Val Ser Lys Ile
    130                 135                 140

tac gag ttt gta gat act tac att atg ctt ctc aag aat aac ttg cgg     480
Tyr Glu Phe Val Asp Thr Tyr Ile Met Leu Leu Lys Asn Asn Leu Arg
145                 150                 155                 160

caa gta agt ttc cta cac att tat cac cac agc acg att tcc ttt att     528
Gln Val Ser Phe Leu His Ile Tyr His His Ser Thr Ile Ser Phe Ile
                165                 170                 175

tgg tgg atc att gct cgg agg gct ccg ggt ggt gat gct tac ttc agc     576
Trp Trp Ile Ile Ala Arg Arg Ala Pro Gly Gly Asp Ala Tyr Phe Ser
            180                 185                 190

gcg gcc ttg aac tca tgg gta cac gtg tgc atg tac acc tat tat cta     624
Ala Ala Leu Asn Ser Trp Val His Val Cys Met Tyr Thr Tyr Tyr Leu
        195                 200                 205

tta tca acc ctt att gga aaa gaa gat cct aag cgt tcc aac tac ctt     672
Leu Ser Thr Leu Ile Gly Lys Glu Asp Pro Lys Arg Ser Asn Tyr Leu
    210                 215                 220

tgg tgg ggt cgc cac cta acg caa atg cag atg ctt cag ttt ttc ttc     720
```

```
Trp Trp Gly Arg His Leu Thr Gln Met Gln Met Leu Gln Phe Phe Phe
225                 230                 235                 240

aac gta ctt caa gcg ttg tac tgc gct tcg ttc tct acg tat ccc aag      768
Asn Val Leu Gln Ala Leu Tyr Cys Ala Ser Phe Ser Thr Tyr Pro Lys
                245                 250                 255

ttt ttg tcc aaa att ctg ctc gtc tat atg atg agc ctt ctc ggc ttg      816
Phe Leu Ser Lys Ile Leu Leu Val Tyr Met Met Ser Leu Leu Gly Leu
            260                 265                 270

ttt ggg cat ttc tac tat tcc aag cac ata gca gca gct aag ctc cag      864
Phe Gly His Phe Tyr Tyr Ser Lys His Ile Ala Ala Ala Lys Leu Gln
        275                 280                 285

aaa aaa cag cag tga                                                  879
Lys Lys Gln Gln
    290
```

<210> 112
<211> 292
<212> PRT
<213> Ostreococcus tauri

<400> 112

```
Met Ser Gly Leu Arg Ala Pro Asn Phe Leu His Arg Phe Trp Thr Lys
1               5                   10                  15

Trp Asp Tyr Ala Ile Ser Lys Val Val Phe Thr Cys Ala Asp Ser Phe
            20                  25                  30

Gln Trp Asp Ile Gly Pro Val Ser Ser Ser Thr Ala His Leu Pro Ala
        35                  40                  45

Ile Glu Ser Pro Thr Pro Leu Val Thr Ser Leu Leu Phe Tyr Leu Val
    50                  55                  60

Thr Val Phe Leu Trp Tyr Gly Arg Leu Thr Arg Ser Ser Asp Lys Lys
65                  70                  75                  80

Ile Arg Glu Pro Thr Trp Leu Arg Arg Phe Ile Ile Cys His Asn Ala
                85                  90                  95

Phe Leu Ile Val Leu Ser Leu Tyr Met Cys Leu Gly Cys Val Ala Gln
            100                 105                 110

Ala Tyr Gln Asn Gly Tyr Thr Leu Trp Gly Asn Glu Phe Lys Ala Thr
        115                 120                 125

Glu Thr Gln Leu Ala Leu Tyr Ile Tyr Ile Phe Tyr Val Ser Lys Ile
    130                 135                 140
```

```
Tyr Glu Phe Val Asp Thr Tyr Ile Met Leu Leu Lys Asn Asn Leu Arg
145                 150                 155                 160

Gln Val Ser Phe Leu His Ile Tyr His His Ser Thr Ile Ser Phe Ile
                165                 170                 175

Trp Trp Ile Ile Ala Arg Arg Ala Pro Gly Gly Asp Ala Tyr Phe Ser
            180                 185                 190

Ala Ala Leu Asn Ser Trp Val His Val Cys Met Tyr Thr Tyr Tyr Leu
        195                 200                 205

Leu Ser Thr Leu Ile Gly Lys Glu Asp Pro Lys Arg Ser Asn Tyr Leu
    210                 215                 220

Trp Trp Gly Arg His Leu Thr Gln Met Gln Met Leu Gln Phe Phe Phe
225                 230                 235                 240

Asn Val Leu Gln Ala Leu Tyr Cys Ala Ser Phe Ser Thr Tyr Pro Lys
                245                 250                 255

Phe Leu Ser Lys Ile Leu Leu Val Tyr Met Met Ser Leu Leu Gly Leu
            260                 265                 270

Phe Gly His Phe Tyr Tyr Ser Lys His Ile Ala Ala Ala Lys Leu Gln
        275                 280                 285

Lys Lys Gln Gln
    290
```

<210> 113
<211> 903
<212> DNA
<213> Ostreococcus tauri

<220>
<221> CDS
<222> (1)..(903)
<223> Delta-5-Elongase

<400> 113

```
atg agc gcc tcc ggt gcg ctg ctg ccc gcg atc gcg ttc gcc gcg tac        48
Met Ser Ala Ser Gly Ala Leu Leu Pro Ala Ile Ala Phe Ala Ala Tyr
1               5                   10                  15

gcg tac gcg acg tac gcc tac gcc ttt gag tgg tcg cac gcg aat ggc        96
Ala Tyr Ala Thr Tyr Ala Tyr Ala Phe Glu Trp Ser His Ala Asn Gly
            20                  25                  30
```

```
atc gac aac gtc gac gcg cgc gag tgg atc ggt gcg ctg tcg ttg agg      144
Ile Asp Asn Val Asp Ala Arg Glu Trp Ile Gly Ala Leu Ser Leu Arg
        35                  40                  45

ctc ccg gcg atc gcg acg acg atg tac ctg ttg ttc tgc ctg gtc gga      192
Leu Pro Ala Ile Ala Thr Thr Met Tyr Leu Leu Phe Cys Leu Val Gly
    50                  55                  60

ccg agg ttg atg gcg aag cgc gag gcg ttc gac ccg aag ggg ttc atg      240
Pro Arg Leu Met Ala Lys Arg Glu Ala Phe Asp Pro Lys Gly Phe Met
65                  70                  75                  80

ctg gcg tac aat gcg tat cag acg gcg ttc aac gtc gtc gtg ctc ggg      288
Leu Ala Tyr Asn Ala Tyr Gln Thr Ala Phe Asn Val Val Val Leu Gly
                85                  90                  95

atg ttc gcg cga gag atc tcg ggg ctg ggg cag ccc gtg tgg ggg tca      336
Met Phe Ala Arg Glu Ile Ser Gly Leu Gly Gln Pro Val Trp Gly Ser
            100                 105                 110

acc atg ccg tgg agc gat aga aaa tcg ttt aag atc ctc ctc ggg gtg      384
Thr Met Pro Trp Ser Asp Arg Lys Ser Phe Lys Ile Leu Leu Gly Val
        115                 120                 125

tgg ttg cac tac aac aac aaa tat ttg gag cta ttg gac act gtg ttc      432
Trp Leu His Tyr Asn Asn Lys Tyr Leu Glu Leu Leu Asp Thr Val Phe
    130                 135                 140

atg gtt gcg cgc aag aag acg aag cag ttg agc ttc ttg cac gtt tat      480
Met Val Ala Arg Lys Lys Thr Lys Gln Leu Ser Phe Leu His Val Tyr
145                 150                 155                 160

cat cac gcc ctg ttg atc tgg gcg tgg tgg ttg gtg tgt cac ttg atg      528
His His Ala Leu Leu Ile Trp Ala Trp Trp Leu Val Cys His Leu Met
                165                 170                 175

gcc acg aac gat tgt atc gat gcc tac ttc ggc gcg gcg tgc aac tcg      576
Ala Thr Asn Asp Cys Ile Asp Ala Tyr Phe Gly Ala Ala Cys Asn Ser
            180                 185                 190

ttc att cac atc gtg atg tac tcg tat tat ctc atg tcg gcg ctc ggc      624
Phe Ile His Ile Val Met Tyr Ser Tyr Tyr Leu Met Ser Ala Leu Gly
        195                 200                 205

att cga tgc ccg tgg aag cga tac atc acc cag gct caa atg ctc caa      672
Ile Arg Cys Pro Trp Lys Arg Tyr Ile Thr Gln Ala Gln Met Leu Gln
    210                 215                 220

ttc gtc att gtc ttc gcg cac gcc gtg ttc gtg ctg cgt cag aag cac      720
Phe Val Ile Val Phe Ala His Ala Val Phe Val Leu Arg Gln Lys His
225                 230                 235                 240

tgc ccg gtc acc ctt cct tgg gcg caa atg ttc gtc atg acg aac atg      768
Cys Pro Val Thr Leu Pro Trp Ala Gln Met Phe Val Met Thr Asn Met
                245                 250                 255

ctc gtg ctc ttc ggg aac ttc tac ctc aag gcg tac tcg aac aag tcg      816
Leu Val Leu Phe Gly Asn Phe Tyr Leu Lys Ala Tyr Ser Asn Lys Ser
            260                 265                 270

cgc ggc gac ggc gcg agt tcc gtg aaa cca gcc gag acc acg cgc gcg      864
Arg Gly Asp Gly Ala Ser Ser Val Lys Pro Ala Glu Thr Thr Arg Ala
        275                 280                 285

ccc agc gtg cga cgc acg cga tct cga aaa att gac taa                  903
Pro Ser Val Arg Arg Thr Arg Ser Arg Lys Ile Asp
    290                 295                 300
```

<210> 114
<211> 300
<212> PRT
<213> Ostreococcus tauri

<400> 114

```
Met Ser Ala Ser Gly Ala Leu Leu Pro Ala Ile Ala Phe Ala Ala Tyr
1               5                   10                  15

Ala Tyr Ala Thr Tyr Ala Tyr Ala Phe Glu Trp Ser His Ala Asn Gly
            20                  25                  30

Ile Asp Asn Val Asp Ala Arg Glu Trp Ile Gly Ala Leu Ser Leu Arg
        35                  40                  45

Leu Pro Ala Ile Ala Thr Thr Met Tyr Leu Leu Phe Cys Leu Val Gly
    50                  55                  60

Pro Arg Leu Met Ala Lys Arg Glu Ala Phe Asp Pro Lys Gly Phe Met
65                  70                  75                  80

Leu Ala Tyr Asn Ala Tyr Gln Thr Ala Phe Asn Val Val Val Leu Gly
                85                  90                  95

Met Phe Ala Arg Glu Ile Ser Gly Leu Gly Gln Pro Val Trp Gly Ser
            100                 105                 110

Thr Met Pro Trp Ser Asp Arg Lys Ser Phe Lys Ile Leu Leu Gly Val
        115                 120                 125

Trp Leu His Tyr Asn Asn Lys Tyr Leu Glu Leu Leu Asp Thr Val Phe
    130                 135                 140

Met Val Ala Arg Lys Lys Thr Lys Gln Leu Ser Phe Leu His Val Tyr
145                 150                 155                 160

His His Ala Leu Leu Ile Trp Ala Trp Trp Leu Val Cys His Leu Met
                165                 170                 175

Ala Thr Asn Asp Cys Ile Asp Ala Tyr Phe Gly Ala Ala Cys Asn Ser
            180                 185                 190

Phe Ile His Ile Val Met Tyr Ser Tyr Tyr Leu Met Ser Ala Leu Gly
        195                 200                 205

Ile Arg Cys Pro Trp Lys Arg Tyr Ile Thr Gln Ala Gln Met Leu Gln
    210                 215                 220
```

Phe Val Ile Val Phe Ala His Ala Val Phe Val Leu Arg Gln Lys His
225 230 235 240

Cys Pro Val Thr Leu Pro Trp Ala Gln Met Phe Val Met Thr Asn Met
245 250 255

Leu Val Leu Phe Gly Asn Phe Tyr Leu Lys Ala Tyr Ser Asn Lys Ser
260 265 270

Arg Gly Asp Gly Ala Ser Ser Val Lys Pro Ala Glu Thr Thr Arg Ala
275 280 285

Pro Ser Val Arg Arg Thr Arg Ser Arg Lys Ile Asp
290 295 300

<210> 115
<211> 13
<212> PRT
<213> Konsensus

<220>
<221> MISC_FEATURE
<222> (1)..(13)
<223> Xaa in der Sequenz an der Position 2, 3, 4, 6, 7, 8 und 9 hat die in Tabelle A wiedergegebene Bedeutung.

<400> 115

Asn Xaa Xaa Xaa His Xaa Xaa Met Tyr Xaa Tyr Tyr Xaa
1 5 10

<210> 116
<211> 10
<212> PRT
<213> Konsensus

<220>
<221> MISC_FEATURE
<222> (1)..(10)
<223> Xaa an der Position 3, 4, 5 und 6 in der Sequenz hat die in Tabel le A wiedergegebene Bedeutung.

<400> 116

His His Xaa Xaa Xaa Xaa Trp Ala Trp Trp
1 5 10

<210> 117
<211> 909
<212> DNA
<213> Xenopus laevis

<220>
<221> CDS
<222> (1)..(909)
<223> Delta-5-Elongase

<400> 117

```
atg gcc ttc aag gag ctc aca tca agg gca gtg ctc ctg tat gat gaa       48
Met Ala Phe Lys Glu Leu Thr Ser Arg Ala Val Leu Leu Tyr Asp Glu
1               5                   10                  15

tgg att aaa gat gct gat cct agg gtt gaa gac tgg cca ctc atg tcc       96
Trp Ile Lys Asp Ala Asp Pro Arg Val Glu Asp Trp Pro Leu Met Ser
            20                  25                  30

tct cct atc cta caa acc atc atc atc ggc gct tac atc tac ttt gtc      144
Ser Pro Ile Leu Gln Thr Ile Ile Ile Gly Ala Tyr Ile Tyr Phe Val
        35                  40                  45

aca tca ttg ggc cca agg atc atg gag aac agg aag ccg ttt gct ctg      192
Thr Ser Leu Gly Pro Arg Ile Met Glu Asn Arg Lys Pro Phe Ala Leu
    50                  55                  60

aag gag atc atg gca tgt tac aac tta ttc atg gtt ctg ttt tct gtg      240
Lys Glu Ile Met Ala Cys Tyr Asn Leu Phe Met Val Leu Phe Ser Val
65                  70                  75                  80

tac atg tgc tat gag ttt ctc atg tcg ggc tgg gct act gga tat tcc      288
Tyr Met Cys Tyr Glu Phe Leu Met Ser Gly Trp Ala Thr Gly Tyr Ser
                85                  90                  95

ttt aga tgt gac att gtt gac tac tct cag tca cct cag gcg tta cgg      336
Phe Arg Cys Asp Ile Val Asp Tyr Ser Gln Ser Pro Gln Ala Leu Arg
            100                 105                 110

atg gcc tgg acc tgc tgg ctc ttc tat ttt tca aag ttc att gaa tta      384
Met Ala Trp Thr Cys Trp Leu Phe Tyr Phe Ser Lys Phe Ile Glu Leu
        115                 120                 125

tta gac act gtt ttc ttt gtg ctg cgt aag aag aac agc cag att aca      432
Leu Asp Thr Val Phe Phe Val Leu Arg Lys Lys Asn Ser Gln Ile Thr
    130                 135                 140

ttc ctg cac gtc tat cac cac tcc att atg cct tgg acg tgg tgg ttt      480
Phe Leu His Val Tyr His His Ser Ile Met Pro Trp Thr Trp Trp Phe
145                 150                 155                 160
```

```
gga gtc aaa ttt gct cca ggt ggt ttg ggc aca ttc cat gca ctg gtg      528
Gly Val Lys Phe Ala Pro Gly Gly Leu Gly Thr Phe His Ala Leu Val
                165                 170                 175

aac tgt gtg gtc cat gtt atc atg tac agc tac tac ggc ctg tca gcc      576
Asn Cys Val Val His Val Ile Met Tyr Ser Tyr Tyr Gly Leu Ser Ala
            180                 185                 190

ttg ggg cct gcc tac cag aag tac ctg tgg tgg aaa aag tac atg acg      624
Leu Gly Pro Ala Tyr Gln Lys Tyr Leu Trp Trp Lys Lys Tyr Met Thr
        195                 200                 205

tct atc caa ctg acc cag ttc ttg atg gtt act ttt cac atc ggc cag      672
Ser Ile Gln Leu Thr Gln Phe Leu Met Val Thr Phe His Ile Gly Gln
    210                 215                 220

ttc ttc ttc atg gag aat tgc ccg tac cag tat ccc gtc ttc ttg tat      720
Phe Phe Phe Met Glu Asn Cys Pro Tyr Gln Tyr Pro Val Phe Leu Tyr
225                 230                 235                 240

gtc att tgg ctg tac ggg ttc gtt ttc tta atc ttg ttc ctc aac ttc      768
Val Ile Trp Leu Tyr Gly Phe Val Phe Leu Ile Leu Phe Leu Asn Phe
                245                 250                 255

tgg ttc cac gct tac atc aaa gga cag agg ctg ccg aaa gcc gtc caa      816
Trp Phe His Ala Tyr Ile Lys Gly Gln Arg Leu Pro Lys Ala Val Gln
            260                 265                 270

aat ggc cac tgc aag aac aac aac aac caa gaa aac act tgg tgc aag      864
Asn Gly His Cys Lys Asn Asn Asn Asn Gln Glu Asn Thr Trp Cys Lys
        275                 280                 285

aac aaa aac cag aaa aac ggt gca ttg aaa agc aaa aac cat tga          909
Asn Lys Asn Gln Lys Asn Gly Ala Leu Lys Ser Lys Asn His
    290                 295                 300
```

<210> 118
<211> 302
<212> PRT
<213> Xenopus laevis

<400> 118

```
Met Ala Phe Lys Glu Leu Thr Ser Arg Ala Val Leu Leu Tyr Asp Glu
1               5                   10                  15

Trp Ile Lys Asp Ala Asp Pro Arg Val Glu Asp Trp Pro Leu Met Ser
            20                  25                  30

Ser Pro Ile Leu Gln Thr Ile Ile Ile Gly Ala Tyr Ile Tyr Phe Val
        35                  40                  45

Thr Ser Leu Gly Pro Arg Ile Met Glu Asn Arg Lys Pro Phe Ala Leu
    50                  55                  60

Lys Glu Ile Met Ala Cys Tyr Asn Leu Phe Met Val Leu Phe Ser Val
65                  70                  75                  80
```

Tyr Met Cys Tyr Glu Phe Leu Met Ser Gly Trp Ala Thr Gly Tyr Ser
85 90 95

Phe Arg Cys Asp Ile Val Asp Tyr Ser Gln Ser Pro Gln Ala Leu Arg
100 105 110

Met Ala Trp Thr Cys Trp Leu Phe Tyr Phe Ser Lys Phe Ile Glu Leu
115 120 125

Leu Asp Thr Val Phe Phe Val Leu Arg Lys Lys Asn Ser Gln Ile Thr
130 135 140

Phe Leu His Val Tyr His His Ser Ile Met Pro Trp Thr Trp Trp Phe
145 150 155 160

Gly Val Lys Phe Ala Pro Gly Gly Leu Gly Thr Phe His Ala Leu Val
165 170 175

Asn Cys Val Val His Val Ile Met Tyr Ser Tyr Tyr Gly Leu Ser Ala
180 185 190

Leu Gly Pro Ala Tyr Gln Lys Tyr Leu Trp Trp Lys Lys Tyr Met Thr
195 200 205

Ser Ile Gln Leu Thr Gln Phe Leu Met Val Thr Phe His Ile Gly Gln
210 215 220

Phe Phe Phe Met Glu Asn Cys Pro Tyr Gln Tyr Pro Val Phe Leu Tyr
225 230 235 240

Val Ile Trp Leu Tyr Gly Phe Val Phe Leu Ile Leu Phe Leu Asn Phe
245 250 255

Trp Phe His Ala Tyr Ile Lys Gly Gln Arg Leu Pro Lys Ala Val Gln
260 265 270

Asn Gly His Cys Lys Asn Asn Asn Asn Gln Glu Asn Thr Trp Cys Lys
275 280 285

Asn Lys Asn Gln Lys Asn Gly Ala Leu Lys Ser Lys Asn His
290 295 300

<210> 119
<211> 870
<212> DNA
<213> Ciona intestinalis

<220>
<221> CDS
<222> (1)..(870)
<223> Delta-5-Elongase

<400> 119

```
atg gac gta ctt cat cgt ttc tta gga ttc tac gaa tgg acg ctg act      48
Met Asp Val Leu His Arg Phe Leu Gly Phe Tyr Glu Trp Thr Leu Thr
1               5                   10                  15

ttc gcg gac ccc cga gtg gca aaa tgg cct tta ata gaa aac ccc ctt      96
Phe Ala Asp Pro Arg Val Ala Lys Trp Pro Leu Ile Glu Asn Pro Leu
            20                  25                  30

cct aca att gct att gtg ttg ctg tac ctg gcg ttt gtt ctg tat att      144
Pro Thr Ile Ala Ile Val Leu Leu Tyr Leu Ala Phe Val Leu Tyr Ile
        35                  40                  45

ggg ccg cgt ttt atg cga aaa aga gca cca gtt gac ttt ggt tta ttc      192
Gly Pro Arg Phe Met Arg Lys Arg Ala Pro Val Asp Phe Gly Leu Phe
    50                  55                  60

ctc cct gga tat aac ttt gct ttg gtt gca tta aat tat tat atc ctg      240
Leu Pro Gly Tyr Asn Phe Ala Leu Val Ala Leu Asn Tyr Tyr Ile Leu
65                  70                  75                  80

caa gaa gtg gtc act ggg agt tat ggg gct ggg tat gat ttg gtt tgc      288
Gln Glu Val Val Thr Gly Ser Tyr Gly Ala Gly Tyr Asp Leu Val Cys
                85                  90                  95

aca cca ctt cga agt gat tcc tac gat ccc aat gaa atg aag gtt gca      336
Thr Pro Leu Arg Ser Asp Ser Tyr Asp Pro Asn Glu Met Lys Val Ala
            100                 105                 110

aac gct gta tgg tgg tat tat gta tcc aag ata ata gag ttg ttt gat      384
Asn Ala Val Trp Trp Tyr Tyr Val Ser Lys Ile Ile Glu Leu Phe Asp
        115                 120                 125

act gtg ttg ttc act cta cgc aaa cga gac cga caa gta act ttc ctt      432
Thr Val Leu Phe Thr Leu Arg Lys Arg Asp Arg Gln Val Thr Phe Leu
    130                 135                 140

cat gtt tat cac cat tct acc atg ccc ctg ttg tgg tgg att ggg gca      480
His Val Tyr His His Ser Thr Met Pro Leu Leu Trp Trp Ile Gly Ala
145                 150                 155                 160

aag tgg gtg cct ggt ggg caa tca ttt gtt ggc atc ata ctg aac tcc      528
Lys Trp Val Pro Gly Gly Gln Ser Phe Val Gly Ile Ile Leu Asn Ser
                165                 170                 175

agt gtt cat gtt atc atg tat acg tac tat gga ttg tca gcc ttg ggg      576
Ser Val His Val Ile Met Tyr Thr Tyr Tyr Gly Leu Ser Ala Leu Gly
            180                 185                 190

cct cac atg cag aag ttt cta tgg tgg aag aaa tat atc aca atg ttg      624
Pro His Met Gln Lys Phe Leu Trp Trp Lys Lys Tyr Ile Thr Met Leu
        195                 200                 205

caa ctg gtt caa ttt gtt ctt gcc atc tac cat act gct cga tca ttg      672
Gln Leu Val Gln Phe Val Leu Ala Ile Tyr His Thr Ala Arg Ser Leu
    210                 215                 220

tac gtt aaa tgt ccc tcg cct gtt tgg atg cac tgg gca ctt atc ttg      720
```

```
Tyr Val Lys Cys Pro Ser Pro Val Trp Met His Trp Ala Leu Ile Leu
225                 230                 235                 240

tac gct ttc tca ttc att ttg ctt ttc tca aac ttc tac atg cat gcc      768
Tyr Ala Phe Ser Phe Ile Leu Leu Phe Ser Asn Phe Tyr Met His Ala
            245                 250                 255

tat atc aag aaa tca aga aaa ggg aaa gag aat ggc agt cga gga aaa      816
Tyr Ile Lys Lys Ser Arg Lys Gly Lys Glu Asn Gly Ser Arg Gly Lys
        260                 265                 270

ggt ggt gta agt aat gga aag gaa aag ctg cac gct aat ggt aaa acc      864
Gly Gly Val Ser Asn Gly Lys Glu Lys Leu His Ala Asn Gly Lys Thr
    275                 280                 285

gat taa                                                              870
Asp
```

<210> 120
<211> 289
<212> PRT
<213> Ciona intestinalis

<400> 120

```
Met Asp Val Leu His Arg Phe Leu Gly Phe Tyr Glu Trp Thr Leu Thr
1               5                   10                  15

Phe Ala Asp Pro Arg Val Ala Lys Trp Pro Leu Ile Glu Asn Pro Leu
            20                  25                  30

Pro Thr Ile Ala Ile Val Leu Leu Tyr Leu Ala Phe Val Leu Tyr Ile
        35                  40                  45

Gly Pro Arg Phe Met Arg Lys Arg Ala Pro Val Asp Phe Gly Leu Phe
    50                  55                  60

Leu Pro Gly Tyr Asn Phe Ala Leu Val Ala Leu Asn Tyr Tyr Ile Leu
65                  70                  75                  80

Gln Glu Val Val Thr Gly Ser Tyr Gly Ala Gly Tyr Asp Leu Val Cys
                85                  90                  95

Thr Pro Leu Arg Ser Asp Ser Tyr Asp Pro Asn Glu Met Lys Val Ala
            100                 105                 110

Asn Ala Val Trp Trp Tyr Tyr Val Ser Lys Ile Ile Glu Leu Phe Asp
        115                 120                 125

Thr Val Leu Phe Thr Leu Arg Lys Arg Asp Arg Gln Val Thr Phe Leu
    130                 135                 140
```

His Val Tyr His His Ser Thr Met Pro Leu Leu Trp Trp Ile Gly Ala
145 150 155 160

Lys Trp Val Pro Gly Gly Gln Ser Phe Val Gly Ile Ile Leu Asn Ser
165 170 175

Ser Val His Val Ile Met Tyr Thr Tyr Tyr Gly Leu Ser Ala Leu Gly
180 185 190

Pro His Met Gln Lys Phe Leu Trp Trp Lys Lys Tyr Ile Thr Met Leu
195 200 205

Gln Leu Val Gln Phe Val Leu Ala Ile Tyr His Thr Ala Arg Ser Leu
210 215 220

Tyr Val Lys Cys Pro Ser Pro Val Trp Met His Trp Ala Leu Ile Leu
225 230 235 240

Tyr Ala Phe Ser Phe Ile Leu Leu Phe Ser Asn Phe Tyr Met His Ala
245 250 255

Tyr Ile Lys Lys Ser Arg Lys Gly Lys Glu Asn Gly Ser Arg Gly Lys
260 265 270

Gly Gly Val Ser Asn Gly Lys Glu Lys Leu His Ala Asn Gly Lys Thr
275 280 285

Asp

<210> 121
<211> 30
<212> DNA
<213> Primer

<220>
<221> misc_feature
<222> (1)..(30)
<223>

<400> 121
aggatccatg gccttcaagg agctcacatc 30

<210> 122
<211> 35
<212> DNA
<213> Primer

<220>
<221> misc_feature
<222> (1)..(35)
<223>

<400> 122
cctcgagtca atggtttttg cttttcaatg caccg 35

<210> 123
<211> 25
<212> DNA
<213> Primer

<220>
<221> misc_feature
<222> (1)..(25)
<223>

<400> 123
taagcttatg gacgtacttc atcgt 25

<210> 124
<211> 26
<212> DNA
<213> Primer

<220>
<221> misc_feature
<222> (1)..(26)
<223>

<400> 124
tcagatcttt aatcggtttt accatt 26

<210> 125
<211> 34
<212> DNA
<213> Primer

<220>
<221> misc_feature
<222> (1)..(34)
<223>

<400> 125
gcggccgcac catggccttc aaggagctca catc 34

<210> 126
<211> 38
<212> DNA
<213> Primer

<220>
<221> misc_feature
<222> (1)..(38)
<223>

<400> 126
gcggccgcct tcaatggttt ttgcttttca atgcaccg 38

<210> 127
<211> 29
<212> DNA
<213> Primer

<220>

<221> misc_feature
<222> (1)..(29)
<223>

<400> 127
gcggccgcac catggacgta cttcatcgt 29

<210> 128
<211> 27
<212> DNA
<213> Primer

<220>
<221> misc_feature
<222> (1)..(27)
<223>

<400> 128
gcggccgctt taatcggttt taccatt 27

<210> 129
<211> 60
<212> DNA
<213> Primer

<220>
<221> misc_feature
<222> (1)..(60)
<223>

<400> 129
gtcgacccgc ggactagtgg gccctctaga cccgggggat ccggatctgc tggctatgaa 60

<210> 130
<211> 60
<212> DNA
<213> Primer

<220>
<221> misc_feature
<222> (1)..(60)
<223>

<400> 130
gtcgacccgc ggactagtgg gccctctaga cccgggggat ccggatctgc tggctatgaa 60

<210> 131
<211> 789
<212> DNA
<213> Euglena gracilis

<220>
<221> CDS
<222> (1)..(789)
<223> Delta-5-Elongase

<400> 131

```
atg ctg ggg gcc atc gcg gac gtc gtg ctc cgg ggg ccc gcc gca ttc      48
Met Leu Gly Ala Ile Ala Asp Val Val Leu Arg Gly Pro Ala Ala Phe
1               5                   10                  15

cac tgg gac cct gcc acc acc ccg ctc gca tcg atc gtc agc ccc tgt      96
His Trp Asp Pro Ala Thr Thr Pro Leu Ala Ser Ile Val Ser Pro Cys
            20                  25                  30

gtg gcc tcc gtg gcg tac ctg ggg gcc atc ggg ctg ctg aag cgc cgc     144
Val Ala Ser Val Ala Tyr Leu Gly Ala Ile Gly Leu Leu Lys Arg Arg
        35                  40                  45

act gga ccg gag gtc cgc tcc aag ccc ttc gag ctg cta cac aac ggg     192
Thr Gly Pro Glu Val Arg Ser Lys Pro Phe Glu Leu Leu His Asn Gly
    50                  55                  60

ctg ctg gtg ggc tgg tcc ctc gtg gtg ctg ctc ggg acg ctg tac ggc     240
Leu Leu Val Gly Trp Ser Leu Val Val Leu Leu Gly Thr Leu Tyr Gly
65                  70                  75                  80

gcg ttc cag cgc gtg cag gag gac ggc cgg ggg gtg cag gcc ctc ctg     288
Ala Phe Gln Arg Val Gln Glu Asp Gly Arg Gly Val Gln Ala Leu Leu
                85                  90                  95

tgc acc cag cgg cca cca tct cag atc tgg gac ggc ccg gtg ggg tac     336
Cys Thr Gln Arg Pro Pro Ser Gln Ile Trp Asp Gly Pro Val Gly Tyr
            100                 105                 110

ttc acg tac ctc ttc tac ctc gcg aag tac tgg gag ctg gcg gac act     384
Phe Thr Tyr Leu Phe Tyr Leu Ala Lys Tyr Trp Glu Leu Ala Asp Thr
        115                 120                 125

gtc atc ctc gcc ctc cgc cag aag ccc acc atc ccc ctc cac gtc tac     432
Val Ile Leu Ala Leu Arg Gln Lys Pro Thr Ile Pro Leu His Val Tyr
    130                 135                 140

cat cac gcc gtc atg ctg ttc atc gtg tgg tcg tgg ttc gcg cac ccc     480
His His Ala Val Met Leu Phe Ile Val Trp Ser Trp Phe Ala His Pro
145                 150                 155                 160

tgg ctc gag ggg agc tgg tgg tgc tcc ctg gtc aac tct ttc atc cac     528
Trp Leu Glu Gly Ser Trp Trp Cys Ser Leu Val Asn Ser Phe Ile His
                165                 170                 175

acg gtg atg tac tcg tac tac acc ctg acg gtg gtt ggc atc aac cct     576
Thr Val Met Tyr Ser Tyr Tyr Thr Leu Thr Val Val Gly Ile Asn Pro
            180                 185                 190

tgg tgg aag aag tgg atg acc acc atg cag atc atc cag ttc atc acg     624
Trp Trp Lys Lys Trp Met Thr Thr Met Gln Ile Ile Gln Phe Ile Thr
        195                 200                 205

ggc tgc gtg tac gtc atg gcg ttc ttc ggc cta tat tat gcc ggg gcg     672
Gly Cys Val Tyr Val Met Ala Phe Phe Gly Leu Tyr Tyr Ala Gly Ala
    210                 215                 220

ggc tgc acc tcc aac gtg tac act gcc tgg ttc tcg atg ggg gtc aac     720
Gly Cys Thr Ser Asn Val Tyr Thr Ala Trp Phe Ser Met Gly Val Asn
225                 230                 235                 240

ctc agc ttt ctg tgg ctc ttc gct ctt ttc ttc cgc cgg tca tac agc     768
Leu Ser Phe Leu Trp Leu Phe Ala Leu Phe Phe Arg Arg Ser Tyr Ser
                245                 250                 255

aaa cct agc cgg aag gag tag                                         789
Lys Pro Ser Arg Lys Glu
            260
```

<210> 132
<211> 262
<212> PRT
<213> Euglena gracilis

<400> 132

```
Met Leu Gly Ala Ile Ala Asp Val Val Leu Arg Gly Pro Ala Ala Phe
1               5                   10                  15

His Trp Asp Pro Ala Thr Thr Pro Leu Ala Ser Ile Val Ser Pro Cys
            20                  25                  30

Val Ala Ser Val Ala Tyr Leu Gly Ala Ile Gly Leu Leu Lys Arg Arg
        35                  40                  45

Thr Gly Pro Glu Val Arg Ser Lys Pro Phe Glu Leu Leu His Asn Gly
    50                  55                  60

Leu Leu Val Gly Trp Ser Leu Val Val Leu Leu Gly Thr Leu Tyr Gly
65                  70                  75                  80

Ala Phe Gln Arg Val Gln Glu Asp Gly Arg Gly Val Gln Ala Leu Leu
                85                  90                  95

Cys Thr Gln Arg Pro Pro Ser Gln Ile Trp Asp Gly Pro Val Gly Tyr
            100                 105                 110
```

```
Phe Thr Tyr Leu Phe Tyr Leu Ala Lys Tyr Trp Glu Leu Ala Asp Thr
        115                 120                 125

Val Ile Leu Ala Leu Arg Gln Lys Pro Thr Ile Pro Leu His Val Tyr
    130                 135                 140

His His Ala Val Met Leu Phe Ile Val Trp Ser Trp Phe Ala His Pro
145                 150                 155                 160

Trp Leu Glu Gly Ser Trp Trp Cys Ser Leu Val Asn Ser Phe Ile His
                165                 170                 175

Thr Val Met Tyr Ser Tyr Tyr Thr Leu Thr Val Val Gly Ile Asn Pro
            180                 185                 190

Trp Trp Lys Lys Trp Met Thr Thr Met Gln Ile Ile Gln Phe Ile Thr
        195                 200                 205

Gly Cys Val Tyr Val Met Ala Phe Phe Gly Leu Tyr Tyr Ala Gly Ala
    210                 215                 220

Gly Cys Thr Ser Asn Val Tyr Thr Ala Trp Phe Ser Met Gly Val Asn
225                 230                 235                 240

Leu Ser Phe Leu Trp Leu Phe Ala Leu Phe Phe Arg Arg Ser Tyr Ser
                245                 250                 255

Lys Pro Ser Arg Lys Glu
            260
```

<210> 133
<211> 789
<212> DNA
<213> Euglena gracilis

<220>
<221> CDS
<222> (1)..(789)
<223> Delta-5-Elongase

<400> 133

```
atg ctg ggg gcc atc gcg gac gtc gtg ctc cgg ggg ccc gcc gca ttc      48
Met Leu Gly Ala Ile Ala Asp Val Val Leu Arg Gly Pro Ala Ala Phe
1               5                   10                  15

cac tgg gac cct gcc acc acc ccg ctc gca tcg atc gtc agc ccc tgt      96
```

```
His Trp Asp Pro Ala Thr Thr Pro Leu Ala Ser Ile Val Ser Pro Cys
            20                  25                  30

gtg gcc tcc gtg gcg tac ctg ggg gcc atc ggg ctg ctg aag cgc cgc      144
Val Ala Ser Val Ala Tyr Leu Gly Ala Ile Gly Leu Leu Lys Arg Arg
        35                  40                  45

act gga ccg gag gtc cgc tcc aag ccc ttc gag ctg cta cac aac ggg      192
Thr Gly Pro Glu Val Arg Ser Lys Pro Phe Glu Leu Leu His Asn Gly
    50                  55                  60

ctg ctg gtg ggc tgg tcc ctc gtg gtg ctg ctc ggg acg ctg tac ggc      240
Leu Leu Val Gly Trp Ser Leu Val Val Leu Leu Gly Thr Leu Tyr Gly
65                  70                  75                  80

gcg tac cag cgc gtg cag gag gac ggc cgg ggg gtg cag gcc ctg ctg      288
Ala Tyr Gln Arg Val Gln Glu Asp Gly Arg Gly Val Gln Ala Leu Leu
                85                  90                  95

tgc acc cag cgg cca cca tct cag atc tgg gac ggc ccg gtg ggg tac      336
Cys Thr Gln Arg Pro Pro Ser Gln Ile Trp Asp Gly Pro Val Gly Tyr
            100                 105                 110

ttc acg tac ctt ttc tac ctc gcg aag tac tgg gag ctg gtg gac act      384
Phe Thr Tyr Leu Phe Tyr Leu Ala Lys Tyr Trp Glu Leu Val Asp Thr
        115                 120                 125

gtc atc ctc gcc ctc cgc cag aag ccc acc atc ccc ctc cac gtc tac      432
Val Ile Leu Ala Leu Arg Gln Lys Pro Thr Ile Pro Leu His Val Tyr
    130                 135                 140

cat cac gcc gtc atg ctg ttc att gtg tgg tcg tgg ttc gcg cac ccc      480
His His Ala Val Met Leu Phe Ile Val Trp Ser Trp Phe Ala His Pro
145                 150                 155                 160

tgg ctc gag ggg agc tgg tgg tgc tcc ctg gtc aac tct ttc atc cac      528
Trp Leu Glu Gly Ser Trp Trp Cys Ser Leu Val Asn Ser Phe Ile His
                165                 170                 175

acg gtg atg tac tcg tat tac acc ctg acg gtg gtt ggc atc aac cct      576
Thr Val Met Tyr Ser Tyr Tyr Thr Leu Thr Val Val Gly Ile Asn Pro
            180                 185                 190

tgg tgg aag aag tgg atg acc acc atg cag atc atc cag ttc atc acg      624
Trp Trp Lys Lys Trp Met Thr Thr Met Gln Ile Ile Gln Phe Ile Thr
        195                 200                 205

ggc tgc gtg tac gtc acg gcg ttc ttc ggc cta tac tat gcc ggg gcg      672
Gly Cys Val Tyr Val Thr Ala Phe Phe Gly Leu Tyr Tyr Ala Gly Ala
    210                 215                 220

ggc tgc acc tcc aac gtg tac act gcc tgg ttc tcg atg ggg gtc aac      720
Gly Cys Thr Ser Asn Val Tyr Thr Ala Trp Phe Ser Met Gly Val Asn
225                 230                 235                 240

ctc agc ttt ctg tgg ctc ttc gct ctt ttc ttc cgc cgg tcg tac agc      768
Leu Ser Phe Leu Trp Leu Phe Ala Leu Phe Phe Arg Arg Ser Tyr Ser
                245                 250                 255

aaa cct agc cgg aag gag tag                                          789
Lys Pro Ser Arg Lys Glu
            260
```

<210> 134
<211> 262
<212> PRT
<213> Euglena gracilis

<400> 134

```
Met Leu Gly Ala Ile Ala Asp Val Val Leu Arg Gly Pro Ala Ala Phe
1               5                   10                  15

His Trp Asp Pro Ala Thr Thr Pro Leu Ala Ser Ile Val Ser Pro Cys
            20                  25                  30

Val Ala Ser Val Ala Tyr Leu Gly Ala Ile Gly Leu Leu Lys Arg Arg
        35                  40                  45

Thr Gly Pro Glu Val Arg Ser Lys Pro Phe Glu Leu Leu His Asn Gly
    50                  55                  60

Leu Leu Val Gly Trp Ser Leu Val Val Leu Leu Gly Thr Leu Tyr Gly
65                  70                  75                  80

Ala Tyr Gln Arg Val Gln Glu Asp Gly Arg Gly Val Gln Ala Leu Leu
                85                  90                  95

Cys Thr Gln Arg Pro Pro Ser Gln Ile Trp Asp Gly Pro Val Gly Tyr
            100                 105                 110

Phe Thr Tyr Leu Phe Tyr Leu Ala Lys Tyr Trp Glu Leu Val Asp Thr
        115                 120                 125

Val Ile Leu Ala Leu Arg Gln Lys Pro Thr Ile Pro Leu His Val Tyr
    130                 135                 140

His His Ala Val Met Leu Phe Ile Val Trp Ser Trp Phe Ala His Pro
145                 150                 155                 160

Trp Leu Glu Gly Ser Trp Trp Cys Ser Leu Val Asn Ser Phe Ile His
                165                 170                 175

Thr Val Met Tyr Ser Tyr Tyr Thr Leu Thr Val Val Gly Ile Asn Pro
            180                 185                 190

Trp Trp Lys Lys Trp Met Thr Thr Met Gln Ile Ile Gln Phe Ile Thr
        195                 200                 205

Gly Cys Val Tyr Val Thr Ala Phe Phe Gly Leu Tyr Tyr Ala Gly Ala
    210                 215                 220

Gly Cys Thr Ser Asn Val Tyr Thr Ala Trp Phe Ser Met Gly Val Asn
225                 230                 235                 240

Leu Ser Phe Leu Trp Leu Phe Ala Leu Phe Phe Arg Arg Ser Tyr Ser
                245                 250                 255

Lys Pro Ser Arg Lys Glu
            260
```

<210> 135
<211> 897
<212> DNA
<213> Arabidopsis thaliana

<220>
<221> CDS
<222> (1)..(897)
<223> Delta-5-Elongase

<400> 135

```
atg gca tct gtt tac tcc acc cta acc tac tgg ctc gtc cac cac ccc      48
Met Ala Ser Val Tyr Ser Thr Leu Thr Tyr Trp Leu Val His His Pro
1               5                   10                  15

tac att gcc aac ttc acg tgg acc gaa ggt gaa aca cta ggc tcc acc      96
Tyr Ile Ala Asn Phe Thr Trp Thr Glu Gly Glu Thr Leu Gly Ser Thr
            20                  25                  30

gtt ttc ttt gtc ttt gtc gtc gtc tcc ctt tac ctc tcc gcc aca ttc     144
Val Phe Phe Val Phe Val Val Val Ser Leu Tyr Leu Ser Ala Thr Phe
        35                  40                  45

ctc ctc cga tac acc gtc gat tca ctc ccc aca ctc ggt ccc cgc att     192
Leu Leu Arg Tyr Thr Val Asp Ser Leu Pro Thr Leu Gly Pro Arg Ile
    50                  55                  60

ctc aaa cca atc aca gcc gtt cac agc ctc att ctc ttc ctc ctc tcc     240
Leu Lys Pro Ile Thr Ala Val His Ser Leu Ile Leu Phe Leu Leu Ser
65                  70                  75                  80

tta acc atg gcc gtt ggt tgc act ctc tcc cta atc tct tcc tcg gac     288
Leu Thr Met Ala Val Gly Cys Thr Leu Ser Leu Ile Ser Ser Ser Asp
                85                  90                  95

ccg aag gcg cgt ctc ttc gac gcc gtt tgt ttc ccc ctc gac gtg aaa     336
Pro Lys Ala Arg Leu Phe Asp Ala Val Cys Phe Pro Leu Asp Val Lys
            100                 105                 110

cct aag gga ccg ctt ttc ttt tgg gct caa gtc ttt tac ctc tcg aag     384
Pro Lys Gly Pro Leu Phe Phe Trp Ala Gln Val Phe Tyr Leu Ser Lys
        115                 120                 125

atc ctt gag ttc gta gac aca ctt ctc atc ata ctc aac aaa tca atc     432
Ile Leu Glu Phe Val Asp Thr Leu Leu Ile Ile Leu Asn Lys Ser Ile
    130                 135                 140

caa cgg ctc tcg ttc ctc cac gtc tac cac cac gca acg gtt gtg att     480
Gln Arg Leu Ser Phe Leu His Val Tyr His His Ala Thr Val Val Ile
145                 150                 155                 160
```

```
ttg tgc tac ctc tgg tta cga aca cgt caa tcg atg ttt cct gtt ggg       528
Leu Cys Tyr Leu Trp Leu Arg Thr Arg Gln Ser Met Phe Pro Val Gly
                165                 170                 175

ctc gtg ttg aac tcg acg gtc cat gtg att atg tac ggg tac tat ttc       576
Leu Val Leu Asn Ser Thr Val His Val Ile Met Tyr Gly Tyr Tyr Phe
            180                 185                 190

ctc tgc gct atc gga tcg agg ccc aag tgg aag aag ttg gtg acg aat       624
Leu Cys Ala Ile Gly Ser Arg Pro Lys Trp Lys Lys Leu Val Thr Asn
        195                 200                 205

ttt caa atg gtt cag ttt gct ttc ggc atg ggg tta gga gcc gct tgg       672
Phe Gln Met Val Gln Phe Ala Phe Gly Met Gly Leu Gly Ala Ala Trp
    210                 215                 220

atg ctc cca gag cat tat ttc ggg tcg ggt tgc gcc ggg att tgg aca       720
Met Leu Pro Glu His Tyr Phe Gly Ser Gly Cys Ala Gly Ile Trp Thr
225                 230                 235                 240

gtt tat ttc aat ggt gtg ttt act gct tct cta ttg gct ctc ttc tac       768
Val Tyr Phe Asn Gly Val Phe Thr Ala Ser Leu Leu Ala Leu Phe Tyr
                245                 250                 255

aac ttc cac tcc aag aac tat gag aag act aca acg tcg cct ttg tat       816
Asn Phe His Ser Lys Asn Tyr Glu Lys Thr Thr Thr Ser Pro Leu Tyr
            260                 265                 270

aag atc gaa tcc ttt ata ttt att cac gga gag agg tgg gca aat aaa       864
Lys Ile Glu Ser Phe Ile Phe Ile His Gly Glu Arg Trp Ala Asn Lys
        275                 280                 285

gcg att aca tta ttt tcc aag aaa aac gat taa                           897
Ala Ile Thr Leu Phe Ser Lys Lys Asn Asp
    290                 295
```

<210> 136
<211> 298
<212> PRT
<213> Arabidopsis thaliana

<400> 136

```
Met Ala Ser Val Tyr Ser Thr Leu Thr Tyr Trp Leu Val His His Pro
1               5                   10                  15

Tyr Ile Ala Asn Phe Thr Trp Thr Glu Gly Glu Thr Leu Gly Ser Thr
            20                  25                  30

Val Phe Phe Val Phe Val Val Val Ser Leu Tyr Leu Ser Ala Thr Phe
        35                  40                  45

Leu Leu Arg Tyr Thr Val Asp Ser Leu Pro Thr Leu Gly Pro Arg Ile
    50                  55                  60

Leu Lys Pro Ile Thr Ala Val His Ser Leu Ile Leu Phe Leu Leu Ser
65                  70                  75                  80
```

Leu Thr Met Ala Val Gly Cys Thr Leu Ser Leu Ile Ser Ser Ser Asp
85 90 95

Pro Lys Ala Arg Leu Phe Asp Ala Val Cys Phe Pro Leu Asp Val Lys
100 105 110

Pro Lys Gly Pro Leu Phe Phe Trp Ala Gln Val Phe Tyr Leu Ser Lys
115 120 125

Ile Leu Glu Phe Val Asp Thr Leu Leu Ile Ile Leu Asn Lys Ser Ile
130 135 140

Gln Arg Leu Ser Phe Leu His Val Tyr His His Ala Thr Val Val Ile
145 150 155 160

Leu Cys Tyr Leu Trp Leu Arg Thr Arg Gln Ser Met Phe Pro Val Gly
165 170 175

Leu Val Leu Asn Ser Thr Val His Val Ile Met Tyr Gly Tyr Tyr Phe
180 185 190

Leu Cys Ala Ile Gly Ser Arg Pro Lys Trp Lys Lys Leu Val Thr Asn
195 200 205

Phe Gln Met Val Gln Phe Ala Phe Gly Met Gly Leu Gly Ala Ala Trp
210 215 220

Met Leu Pro Glu His Tyr Phe Gly Ser Gly Cys Ala Gly Ile Trp Thr
225 230 235 240

Val Tyr Phe Asn Gly Val Phe Thr Ala Ser Leu Leu Ala Leu Phe Tyr
245 250 255

Asn Phe His Ser Lys Asn Tyr Glu Lys Thr Thr Thr Ser Pro Leu Tyr
260 265 270

Lys Ile Glu Ser Phe Ile Phe Ile His Gly Glu Arg Trp Ala Asn Lys
275 280 285

Ala Ile Thr Leu Phe Ser Lys Lys Asn Asp
290 295

<210> 137
<211> 837
<212> DNA
<213> Arabidopsis thaliana

<220>
<221> CDS
<222> (1)..(837)
<223> Delta-5-Elongase

<400> 137

```
atg gca tca att tac tcc tct tta acc tac tgg ctc gtt aac cac ccc      48
Met Ala Ser Ile Tyr Ser Ser Leu Thr Tyr Trp Leu Val Asn His Pro
1               5                   10                  15

tac atc tcc aat ttt act tgg atc gaa ggt gaa acc cta ggc tcc acc      96
Tyr Ile Ser Asn Phe Thr Trp Ile Glu Gly Glu Thr Leu Gly Ser Thr
            20                  25                  30

gtc ttt ttc gta tcc gtc gta gtc tcc gtt tac ctc tcc gcc acg ttc      144
Val Phe Phe Val Ser Val Val Val Ser Val Tyr Leu Ser Ala Thr Phe
        35                  40                  45

ctc ctc cga tcc gcc atc gat tca ctc cca tca ctc agt cca cgt atc      192
Leu Leu Arg Ser Ala Ile Asp Ser Leu Pro Ser Leu Ser Pro Arg Ile
    50                  55                  60

ctc aaa ccg atc aca gcc gtc cac agc cta atc ctc tgt ctc ctc tcc      240
Leu Lys Pro Ile Thr Ala Val His Ser Leu Ile Leu Cys Leu Leu Ser
65                  70                  75                  80

tta gtc atg gcc gtc ggt tgc act ctc tca ata acc tca tct cac gcg      288
Leu Val Met Ala Val Gly Cys Thr Leu Ser Ile Thr Ser Ser His Ala
                85                  90                  95

tct tca gat ccg atg gcg cgt ttc ctt cac gcg att tgc ttt ccc gtc      336
Ser Ser Asp Pro Met Ala Arg Phe Leu His Ala Ile Cys Phe Pro Val
            100                 105                 110

gac gtt aaa cct aac gga ccg ctt ttc ttc tgg gct caa gtc ttc tac      384
Asp Val Lys Pro Asn Gly Pro Leu Phe Phe Trp Ala Gln Val Phe Tyr
        115                 120                 125

ctc tcg aag atc ctc gag ttc gga gac acg atc ctc atc ata ctc ggc      432
Leu Ser Lys Ile Leu Glu Phe Gly Asp Thr Ile Leu Ile Ile Leu Gly
    130                 135                 140

aaa tca atc caa cgg cta tcc ttc ctc cac gtg tac cac cac gcg acg      480
Lys Ser Ile Gln Arg Leu Ser Phe Leu His Val Tyr His His Ala Thr
145                 150                 155                 160

gtt gtg gtc atg tgt tat ctc tgg ctc cga act cgc caa tcg atg ttt      528
Val Val Val Met Cys Tyr Leu Trp Leu Arg Thr Arg Gln Ser Met Phe
                165                 170                 175

ccg att gcg ctc gtg acg aat tcg acg gta cac gtc atc atg tac ggt      576
Pro Ile Ala Leu Val Thr Asn Ser Thr Val His Val Ile Met Tyr Gly
            180                 185                 190

tac tac ttc ctc tgc gcc gtt gga tcg agg ccc aag tgg aag aga ttg      624
Tyr Tyr Phe Leu Cys Ala Val Gly Ser Arg Pro Lys Trp Lys Arg Leu
        195                 200                 205

gtg acg gat tgt cag att gtt cag ttt gtt ttc agt ttc ggg tta tcc      672
Val Thr Asp Cys Gln Ile Val Gln Phe Val Phe Ser Phe Gly Leu Ser
    210                 215                 220

ggt tgg atg ctc cga gag cac tta ttc ggg tcg ggt tgc acc ggg att      720
```

```
Gly Trp Met Leu Arg Glu His Leu Phe Gly Ser Gly Cys Thr Gly Ile
225                 230                 235                 240

tgg gga tgg tgt ttc aac gct gca ttt aat gct tct ctt ttg gct ctc       768
Trp Gly Trp Cys Phe Asn Ala Ala Phe Asn Ala Ser Leu Leu Ala Leu
                245                 250                 255

ttt tcc aac ttc cat tca aag aat tat gtc aag aag cca acg aga gag       816
Phe Ser Asn Phe His Ser Lys Asn Tyr Val Lys Lys Pro Thr Arg Glu
            260                 265                 270

gat ggc aaa aaa agc gat tag                                           837
Asp Gly Lys Lys Ser Asp
        275
```

<210> 138
<211> 278
<212> PRT
<213> Arabidopsis thaliana

<400> 138

```
Met Ala Ser Ile Tyr Ser Ser Leu Thr Tyr Trp Leu Val Asn His Pro
1               5                   10                  15

Tyr Ile Ser Asn Phe Thr Trp Ile Glu Gly Glu Thr Leu Gly Ser Thr
            20                  25                  30

Val Phe Phe Val Ser Val Val Val Ser Val Tyr Leu Ser Ala Thr Phe
        35                  40                  45

Leu Leu Arg Ser Ala Ile Asp Ser Leu Pro Ser Leu Ser Pro Arg Ile
    50                  55                  60

Leu Lys Pro Ile Thr Ala Val His Ser Leu Ile Leu Cys Leu Leu Ser
65                  70                  75                  80

Leu Val Met Ala Val Gly Cys Thr Leu Ser Ile Thr Ser Ser His Ala
                85                  90                  95

Ser Ser Asp Pro Met Ala Arg Phe Leu His Ala Ile Cys Phe Pro Val
            100                 105                 110

Asp Val Lys Pro Asn Gly Pro Leu Phe Phe Trp Ala Gln Val Phe Tyr
        115                 120                 125

Leu Ser Lys Ile Leu Glu Phe Gly Asp Thr Ile Leu Ile Ile Leu Gly
    130                 135                 140

Lys Ser Ile Gln Arg Leu Ser Phe Leu His Val Tyr His His Ala Thr
145                 150                 155                 160
```

Val Val Val Met Cys Tyr Leu Trp Leu Arg Thr Arg Gln Ser Met Phe
165 170 175

Pro Ile Ala Leu Val Thr Asn Ser Thr Val His Val Ile Met Tyr Gly
180 185 190

Tyr Tyr Phe Leu Cys Ala Val Gly Ser Arg Pro Lys Trp Lys Arg Leu
195 200 205

Val Thr Asp Cys Gln Ile Val Gln Phe Val Phe Ser Phe Gly Leu Ser
210 215 220

Gly Trp Met Leu Arg Glu His Leu Phe Gly Ser Gly Cys Thr Gly Ile
225 230 235 240

Trp Gly Trp Cys Phe Asn Ala Ala Phe Asn Ala Ser Leu Leu Ala Leu
245 250 255

Phe Ser Asn Phe His Ser Lys Asn Tyr Val Lys Lys Pro Thr Arg Glu
260 265 270

Asp Gly Lys Lys Ser Asp
275

<210> 139
<211> 6
<212> PRT
<213> Konsensus

<220>
<221> MISC_FEATURE
<222> (1)..(6)
<223> Xaa in der Position 3 und 4 in der Sequenz hat die in Tabelle A w iedergegebene Bedeutung.

<400> 139

Leu His Xaa Xaa His His
1 5

<210> 140
<211> 8
<212> PRT
<213> Konsensus

<220>
<221> MISC_FEATURE
<222> (1)..(8)
<223> Xaa an der Position 2, 3, 5 und 6 in der Sequenz hat die in Tabel le A wiedergegebene Bedeutung.

<400> 140

Thr Xaa Xaa Gln Xaa Xaa Gln Phe
1 5

<210> 141

<211> 6
<212> PRT
<213> Konsensus

<220>
<221> MISC_FEATURE
<222> (1)..(6)
<223> Xaa an Postion 3 in der Sequenz hat die in Tabelle A wiedergegebe ne Bedeutung.

<400> 141

Asp Thr Xaa Phe Met Val
1 5

<210> 142
<211> 8
<212> PRT
<213> Konsensus

<220>
<221> MISC_FEATURE
<222> (1)..(8)
<223> Xaa an Postion 5 und 6 in der Sequenz hat die in Tabelle A wieder gegebene Bedeutung.

<400> 142

Thr Gln Ala Gln Xaa Xaa Gln Phe
1 5

<210> 143
<211> 60
<212> DNA
<213> Primer

<220>
<221> misc_feature
<222> (1)..(60)
<223>

<400> 143
gtcgacccgc ggactagtgg gccctctaga cccgggggat ccggatctgc tggctatgaa 60

<210> 144
<211> 60
<212> DNA
<213> Primer

<220>
<221> misc_feature
<222> (1)..(60)
<223>

<400> 144
gtcgacccgc ggactagtgg gccctctaga cccgggggat ccggatctgc tggctatgaa 60

<210> 145
<211> 36

<212> DNA
<213> Primer

<220>
<221> misc_feature
<222> (1)..(36)
<223>

<400> 145
ggtaccacat aatgtgcgtg gagacggaaa ataacg 36

<210> 146
<211> 33
<212> DNA
<213> Primer

<220>
<221> misc_feature
<222> (1)..(33)
<223>

<400> 146
ctcgagttac gccgtctttc cggagtgttg gcc 33

<210> 147
<211> 24
<212> DNA
<213> Primer

<220>
<221> misc_feature
<222> (1)..(24)
<223>

<400> 147
gcggccgctt acgtggactt ggtc 24

<210> 148
<211> 24
<212> DNA
<213> Primer

<220>
<221> misc_feature
<222> (1)..(24)
<223>

<400> 148
gcggccgcat ggcgacgaag gagg 24

<210> 149
<211> 25
<212> DNA
<213> Primer

<220>
<221> misc_feature
<222> (1)..(25)

<223>

<400> 149
taagcttaca tggcgacgaa ggagg 25

<210> 150
<211> 24
<212> DNA
<213> Primer

<220>
<221> misc_feature
<222> (1)..(24)
<223>

<400> 150
tggatccact tacgtggact tggt 24

<210> 151
<211> 60
<212> DNA
<213> Primer

<220>
<221> misc_feature
<222> (1)..(60)
<223>

<400> 151
gtcgacccgc ggactagtgg gccctctaga cccgggggat ccggatctgc tggctatgaa 60

<210> 152
<211> 31
<212> DNA
<213> Primer

<220>
<221> misc_feature
<222> (1)..(31)
<223>

<400> 152
gcggccgcac catgtgctca ccaccgccgt c 31

<210> 153
<211> 26
<212> DNA
<213> Primer

<220>
<221> misc_feature
<222> (1)..(26)
<223>

<400> 153
gcggccgcct acatggcacc agtaac 26

<210> 154

<211> 31
<212> DNA
<213> Primer

<220>
<221> misc_feature
<222> (1)..(31)
<223>

<400> 154
gcggccgcac catgtgctca tcaccgccgt c 31

<210> 155
<211> 26
<212> DNA
<213> Primer

<220>
<221> misc_feature
<222> (1)..(26)
<223>

<400> 155
gcggccgcct acatggcacc agtaac 26

<210> 156
<211> 31
<212> DNA
<213> Primer

<220>
<221> misc_feature
<222> (1)..(31)
<223>

<400> 156
gcggccgcac catggacgcc tacaacgctg c 31

<210> 157
<211> 27
<212> DNA
<213> Primer

<220>
<221> misc_feature
<222> (1)..(27)
<223>

<400> 157
gcggccgcct aagcactctt cttcttt 27

<210> 158
<211> 23
<212> DNA
<213> Primer

<220>
<221> misc_feature

<222> (1)..(23)
<223>

<400> 158
accatgtgct caccaccgcc gtc 23

<210> 159
<211> 18
<212> DNA
<213> Primer

<220>
<221> misc_feature
<222> (1)..(18)
<223>

<400> 159
ctacatggca ccagtaac 18

<210> 160
<211> 23
<212> DNA
<213> Primer

<220>
<221> misc_feature
<222> (1).. (23)
<223>

<400> 160
accatgtgct catcaccgcc gtc 23

<210> 161
<211> 18
<212> DNA
<213> Primer

<220>
<221> misc_feature
<222> (1)..(18)
<223>

<400> 161
ctacatggca ccagtaac 18

<210> 162
<211> 23
<212> DNA
<213> Primer

<220>
<221> misc_feature
<222> (1)..(23)
<223>

<400> 162
accatggacg cctacaacgc tgc 23

<210> 163
<211> 19
<212> DNA
<213> Primer

<220>
<221> misc_feature
<222> (1)..(19)
<223>

<400> 163
ctaagcactc ttcttcttt 19

<210> 164
<211> 60
<212> DNA
<213> Primer

<220>
<221> misc_feature
<222> (1)..(60)
<223>

<400> 164
gtcgacccgc ggactagtgg gccctctaga cccgggggat ccggatctgc tggctatgaa 60

<210> 165
<211> 60
<212> DNA
<213> Primer

<220>
<221> misc_feature
<222> (1)..(60)
<223>

<400> 165
gtcgacccgc ggactagtgg gccctctaga cccgggggat ccggatctgc tggctatgaa 60

<210> 166
<211> 29
<212> DNA
<213> Primer

<220>
<221> misc_feature
<222> (1)..(29)
<223>

<400> 166
gcggccgcat aatgacgagc aacatgagc 29

<210> 167
<211> 29
<212> DNA
<213> Primer

<220>

<221> misc_feature
<222> (1)..(29)
<223>

<400> 167
gcggccgctt aggccgactt ggccttggg 29

<210> 168
<211> 34
<212> DNA
<213> Primer

<220>
<221> misc_feature
<222> (1)..(34)
<223>

<400> 168
gcggccgcac catggacgtc gtcgagcagc aatg 34

<210> 169
<211> 36
<212> DNA
<213> Primer

<220>
<221> misc_feature
<222> (1)..(36)
<223>

<400> 169
gcggccgctt agatggtctt ctgcttcttg ggcgcc 36

<210> 170
<211> 23
<212> DNA
<213> Primer

<220>
<221> misc_feature
<222> (1)..(23)
<223>

<400> 170
gacataatga cgagcaacat gag 23

<210> 171
<211> 25
<212> DNA
<213> Primer

<220>
<221> misc_feature
<222> (1)..(25)
<223>

<400> 171
cggcttaggc cgacttggcc ttggg 25

<210> 172
<211> 30
<212> DNA
<213> Primer

<220>
<221> misc_feature
<222> (1)..(30)
<223>

<400> 172
agacataatg gacgtcgtcg agcagcaatg 30

<210> 173
<211> 28
<212> DNA
<213> Primer

<220>
<221> misc_feature
<222> (1)..(28)
<223>

<400> 173
ttagatggtc ttctgcttct tgggcgcc 28

<210> 174
<211> 60
<212> DNA
<213> Primer

<220>
<221> misc_feature
<222> (1)..(60)
<223>

<400> 174
gtcgacccgc ggactagtgg gccctctaga cccgggggat ccggatctgc tggctatgaa 60

<210> 175
<211> 29
<212> DNA
<213> Primer

<220>
<221> misc_feature
<222> (1)..(29)
<223>

<400> 175
gcggccgcat aatggcttca acatggcaa 29

<210> 176
<211> 32
<212> DNA
<213> Primer

<220>

<221> misc_feature
<222> (1)..(32)
<223>

<400> 176
gcggccgctt atgtcttctt gctcttcctg tt 32

<210> 177
<211> 26
<212> DNA
<213> Primer

<220>
<221> misc_feature
<222> (1)..(26)
<223>

<400> 177
gcggccgcat aatggagact tttaat 26

<210> 178
<211> 28
<212> DNA
<213> Primer

<220>
<221> misc_feature
<222> (1)..(28)
<223>

<400> 178
gcggccgctc agtcccccct cactttcc 28

<210> 179
<211> 29
<212> DNA
<213> Primer

<220>
<221> misc_feature
<222> (1)..(29)
<223>

<400> 179
aagcttacat aatggcttca acatggcaa 29

<210> 180
<211> 30
<212> DNA
<213> Primer

<220>
<221> misc_feature
<222> (1)..(30)
<223>

<400> 180
ggatccttat gtcttcttgc tcttcctgtt 30

<210> 181
<211> 26
<212> DNA
<213> Primer

<220>
<221> misc_feature
<222> (1)..(26)
<223>

<400> 181
aagcttacat aatggagact tttaat 26

<210> 182
<211> 27
<212> DNA
<213> Primer

<220>
<221> misc_feature
<222> (1)..(27)
<223>

<400> 182
ggatccttca gtcccccctc actttcc 27

<210> 183
<211> 993
<212> DNA
<213> Phaeodactylum tricornutum

<220>
<221> CDS
<222> (103)..(939)
<223> Delta-6-Elongase

<400> 183

```
ggtcttttgt ggtagctatc gtcatcacac gcaggtcgtt gctcactatc gtgatccgta     60

tattgaccgt gcacttgtgt aaaacagaga tatttcaaga gt atg atg gta cct       114
                                               Met Met Val Pro
                                               1

tca agt tat gac gag tat atc gtc atg gtc aac gac ctt ggc gac tct      162
Ser Ser Tyr Asp Glu Tyr Ile Val Met Val Asn Asp Leu Gly Asp Ser
5                   10                  15                  20

att ctg agc tgg gcc gac cct gat cac tat cgt gga cat acc gag gga      210
Ile Leu Ser Trp Ala Asp Pro Asp His Tyr Arg Gly His Thr Glu Gly
                25                  30                  35

tgg gag ttc act gac ttt tct gct gct ttt agc att gcc gtc gcg tac      258
Trp Glu Phe Thr Asp Phe Ser Ala Ala Phe Ser Ile Ala Val Ala Tyr
            40                  45                  50

ctc ctg ttt gtc ttt gtt gga tct ctc att atg agt atg gga gtc ccc      306
Leu Leu Phe Val Phe Val Gly Ser Leu Ile Met Ser Met Gly Val Pro
        55                  60                  65

gca att gac cct tat ccg ctc aag ttt gtc tac aat gtt tca cag att      354
Ala Ile Asp Pro Tyr Pro Leu Lys Phe Val Tyr Asn Val Ser Gln Ile
    70                  75                  80

atg ctt tgt gct tac atg acc att gaa gcc agt ctt cta gct tat cgt      402
Met Leu Cys Ala Tyr Met Thr Ile Glu Ala Ser Leu Leu Ala Tyr Arg
85                  90                  95                  100

aac ggc tac aca ttc tgg cct tgc aac gat tgg gac ttt gaa aag ccg      450
Asn Gly Tyr Thr Phe Trp Pro Cys Asn Asp Trp Asp Phe Glu Lys Pro
                105                 110                 115
```

```
cct atc gct aag ctc ctc tgg ctc ttt tac gtt tcc aaa att tgg gat       498
Pro Ile Ala Lys Leu Leu Trp Leu Phe Tyr Val Ser Lys Ile Trp Asp
            120                 125                 130

ttt tgg gac acc atc ttt att gtt ctc ggg aag aag tgg cgt caa ctt       546
Phe Trp Asp Thr Ile Phe Ile Val Leu Gly Lys Lys Trp Arg Gln Leu
        135                 140                 145

tcc ttc ctg cac gtc tac cat cac acc acc atc ttt ctc ttc tac tgg       594
Ser Phe Leu His Val Tyr His His Thr Thr Ile Phe Leu Phe Tyr Trp
    150                 155                 160

ttg aat gca cat gta aac ttt gat ggt gat att ttc ctc acc atc gtc       642
Leu Asn Ala His Val Asn Phe Asp Gly Asp Ile Phe Leu Thr Ile Val
165                 170                 175                 180

ttg aac ggt ttc atc cac acc gtc atg tac acg tac tac ttc att tgc       690
Leu Asn Gly Phe Ile His Thr Val Met Tyr Thr Tyr Tyr Phe Ile Cys
                185                 190                 195

atg cac acc aag gtc cca gag acc ggc aaa tcc ttg ccc att tgg tgg       738
Met His Thr Lys Val Pro Glu Thr Gly Lys Ser Leu Pro Ile Trp Trp
            200                 205                 210

aaa tct agt ttg aca agc atg cag ctg gtg cag ttc atc acg atg atg       786
Lys Ser Ser Leu Thr Ser Met Gln Leu Val Gln Phe Ile Thr Met Met
        215                 220                 225

acg cag gct atc atg atc ttg tac aag ggc tgt gct gct ccc cat agc       834
Thr Gln Ala Ile Met Ile Leu Tyr Lys Gly Cys Ala Ala Pro His Ser
    230                 235                 240

cgg gtg gtg aca tcg tac ttg gtt tac att ttg tcg ctc ttt att ttg       882
Arg Val Val Thr Ser Tyr Leu Val Tyr Ile Leu Ser Leu Phe Ile Leu
245                 250                 255                 260

ttc gcc cag ttc ttt gtc agc tca tac ctc aag ccg aag aag aag aag       930
Phe Ala Gln Phe Phe Val Ser Ser Tyr Leu Lys Pro Lys Lys Lys Lys
                265                 270                 275

aca gct taa gcgaaatttg ggtctacgtt aaaacaatta cgttacaaaa               979
Thr Ala

aaaaaaaaaa aaaa                                                       993
```

<210> 184
<211> 278
<212> PRT
<213> Phaeodactylum tricornutum

<400> 184

```
Met Met Val Pro Ser Ser Tyr Asp Glu Tyr Ile Val Met Val Asn Asp
1               5                   10                  15

Leu Gly Asp Ser Ile Leu Ser Trp Ala Asp Pro Asp His Tyr Arg Gly
            20                  25                  30
```

```
His Thr Glu Gly Trp Glu Phe Thr Asp Phe Ser Ala Ala Phe Ser Ile
        35                  40                  45

Ala Val Ala Tyr Leu Leu Phe Val Phe Val Gly Ser Leu Ile Met Ser
    50                  55                  60

Met Gly Val Pro Ala Ile Asp Pro Tyr Pro Leu Lys Phe Val Tyr Asn
65                  70                  75                  80

Val Ser Gln Ile Met Leu Cys Ala Tyr Met Thr Ile Glu Ala Ser Leu
                85                  90                  95

Leu Ala Tyr Arg Asn Gly Tyr Thr Phe Trp Pro Cys Asn Asp Trp Asp
            100                 105                 110

Phe Glu Lys Pro Pro Ile Ala Lys Leu Leu Trp Leu Phe Tyr Val Ser
        115                 120                 125

Lys Ile Trp Asp Phe Trp Asp Thr Ile Phe Ile Val Leu Gly Lys Lys
    130                 135                 140

Trp Arg Gln Leu Ser Phe Leu His Val Tyr His His Thr Thr Ile Phe
145                 150                 155                 160

Leu Phe Tyr Trp Leu Asn Ala His Val Asn Phe Asp Gly Asp Ile Phe
                165                 170                 175

Leu Thr Ile Val Leu Asn Gly Phe Ile His Thr Val Met Tyr Thr Tyr
            180                 185                 190

Tyr Phe Ile Cys Met His Thr Lys Val Pro Glu Thr Gly Lys Ser Leu
        195                 200                 205

Pro Ile Trp Trp Lys Ser Ser Leu Thr Ser Met Gln Leu Val Gln Phe
    210                 215                 220

Ile Thr Met Met Thr Gln Ala Ile Met Ile Leu Tyr Lys Gly Cys Ala
225                 230                 235                 240

Ala Pro His Ser Arg Val Val Thr Ser Tyr Leu Val Tyr Ile Leu Ser
                245                 250                 255

Leu Phe Ile Leu Phe Ala Gln Phe Phe Val Ser Ser Tyr Leu Lys Pro
            260                 265                 270

Lys Lys Lys Lys Thr Ala
        275
```

<210> 185
<211> 20
<212> DNA
<213> Primer

<220>
<221> misc_feature
<222> (1)..(20)
<223> N in den Positionen 3 und 18 bedeutet C oder T.

<400> 185
aanctuctut ggctuttnta 20

<210> 186
<211> 23
<212> DNA
<213> Primer

<220>
<221> misc_feature
<222> (1)..(23)
<223> N in den Positionen 3 und 15 bedeutet C oder T. N in den Position en 9, 12 und 21 bedeutet A oder G.

<400> 186
gantguacna anaantgugc naa 23

<210> 187
<211> 446
<212> DNA
<213> PCR-Fragment

<220>
<221> misc_feature
<222> (1)..(446)
<223> PCR-Fragment

<400> 187
aagctcctct ggctctttta cgtttccaaa atttgggatt tttgggacac catctttatt 60

gttctcggga agaagtggcg tcaactttcc ttcctgcacg tctaccatca caccaccatc 120

tttctcttct actggttgaa tgcacatgta aactttgatg gtgatatttt cctcaccatc 180

gtcttgaacg gtttcatcca caccgtcatg tacacgtact acttcatttg catgcacacc 240

aaggtcccag agaccggcaa atccttgccc atttggtgga aatctagttt gacaagcatg 300

cagctggtgc agttcatcac gatgatgacg caggctatca tgatcttgta caagggctgt 360

gctgctcccc atagccgggt ggtgacatcg tacttggttt acattttgtc gctctttatt 420

ttgttcgccc agttctttgt cagctc 446

<210> 188
<211> 30
<212> DNA
<213> Primer

<220>
<221> misc_feature
<222> (1)..(30)
<223>

<400> 188

gcggccgcac ataatgatgg taccttcaag 30

<210> 189
<211> 22
<212> DNA
<213> Primer

<220>
<221> misc_feature
<222> (1)..(22)
<223>

<400> 189
gaagacagct taatagacta gt 22

<210> 190
<211> 31
<212> DNA
<213> Primer

<220>
<221> misc_feature
<222> (1)..(31)
<223>

<400> 190
gcggccgcac catgatggta ccttcaagtt a 31

<210> 191
<211> 24
<212> DNA
<213> Primer

<220>
<221> misc_feature
<222> (1)..(24)
<223>

<400> 191
gaagacagct taataggcgg ccgc 24

<210> 192
<211> 859
<212> DNA
<213> PCR-Produkt

<400> 192

```
gcggccgcac ataatgatgg taccttcaag ttatgacgag tatatcgtca tggtcaacga    60
ccttggcgac tctattctga gctgggccga ccctgatcac tatcgtggac ataccgaggg   120
atgggagttc actgactttt ctgctgcttt tagcattgcc gtcgcgtacc tcctgtttgt   180
ctttgttgga tctctcatta tgagtatggg agtccccgca attgaccctt atccgctcaa   240
gtttgtctac aatgtttcac agattatgct ttgtgcttac atgaccattg aagccagtct   300
tctagcttat cgtaacggct acacattctg gccttgcaac gattgggact ttgaaaagcc   360
gcctatcgct aagctcctct ggctctttta cgtttccaaa atttgggatt tttgggacac   420

catctttatt gttctcggga agaagtggcg tcaactttcc ttcctgcacg tctaccatca   480
caccaccatc tttctcttct actggttgaa tgcacatgta aactttgatg gtgatatttt   540
cctcaccatc gtcttgaacg gtttcatcca caccgtcatg tacacgtact acttcatttg   600
catgcacacc aaggtcccag agaccggcaa atccttgccc atttggtgga aatctagttt   660
gacaagcatg cagctggtgc agttcatcac gatgatgacg caggctatca tgatcttgta   720
caagggctgt gctgctcccc atagccgggt ggtgacatcg tacttggttt acattttgtc   780
gctctttatt ttgttcgccc agttctttgt cagctcatac ctcaagccga agaagaagaa   840
gacagcttaa tagactagt                                                859
```

<210> 193
<211> 1380
<212> DNA
<213> Phytium irregulare

<220>
<221> CDS
<222> (1)..(1380)
<223> Delta-6-Desaturase

<400> 193

```
atg gtg gac ctc aag cct gga gtg aag cgc ctg gtg agc tgg aag gag      48
Met Val Asp Leu Lys Pro Gly Val Lys Arg Leu Val Ser Trp Lys Glu
1               5                   10                  15

atc cgc gag cac gcg acg ccc gcg acc gcg tgg atc gtg att cac cac      96
Ile Arg Glu His Ala Thr Pro Ala Thr Ala Trp Ile Val Ile His His
            20                  25                  30

aag gtc tac gac atc tcc aag tgg gac tcg cac ccg ggt ggc tcc gtg     144
Lys Val Tyr Asp Ile Ser Lys Trp Asp Ser His Pro Gly Gly Ser Val
        35                  40                  45

atg ctc acg cag gcc ggc gag gac gcc acg gac gcc ttc gcg gtc ttc     192
Met Leu Thr Gln Ala Gly Glu Asp Ala Thr Asp Ala Phe Ala Val Phe
    50                  55                  60

cac ccg tcc tcg gcg ctc aag ctg ctc gag cag ttc tac gtc ggc gac     240
His Pro Ser Ser Ala Leu Lys Leu Leu Glu Gln Phe Tyr Val Gly Asp
65                  70                  75                  80

gtg gac gaa acc tcc aag gcc gag atc gag ggg gag ccg gcg agc gac     288
Val Asp Glu Thr Ser Lys Ala Glu Ile Glu Gly Glu Pro Ala Ser Asp
                85                  90                  95

gag gag cgc gcg cgc cgc gag cgc atc aac gag ttc atc gcg tcc tac     336
Glu Glu Arg Ala Arg Arg Glu Arg Ile Asn Glu Phe Ile Ala Ser Tyr
            100                 105                 110

cgc cgt ctg cgc gtc aag gtc aag ggc atg ggg ctc tac gac gcc agc     384
```

```
Arg Arg Leu Arg Val Lys Val Lys Gly Met Gly Leu Tyr Asp Ala Ser
        115                 120                 125

gcg ctc tac tac gcg tgg aag ctc gtg agc acg ttc ggc atc gcg gtg      432
Ala Leu Tyr Tyr Ala Trp Lys Leu Val Ser Thr Phe Gly Ile Ala Val
    130                 135                 140

ctc tcg atg gcg atc tgc ttc ttc ttc aac agt ttc gcc atg tac atg      480
Leu Ser Met Ala Ile Cys Phe Phe Phe Asn Ser Phe Ala Met Tyr Met
145                 150                 155                 160

gtc gcc ggc gtg att atg ggg ctc ttc tac cag cag tcc gga tgg ctg      528
Val Ala Gly Val Ile Met Gly Leu Phe Tyr Gln Gln Ser Gly Trp Leu
                165                 170                 175

gcg cac gac ttc ttg cac aac cag gtg tgc gag aac cgc acg ctc ggc      576
Ala His Asp Phe Leu His Asn Gln Val Cys Glu Asn Arg Thr Leu Gly
            180                 185                 190

aac ctt atc ggc tgc ctc gtg ggc aac gcc tgg cag ggc ttc agc atg      624
Asn Leu Ile Gly Cys Leu Val Gly Asn Ala Trp Gln Gly Phe Ser Met
        195                 200                 205

cag tgg tgg aag aac aag cac aac ctg cac cac gcg gtg ccg aac ctg      672
Gln Trp Trp Lys Asn Lys His Asn Leu His His Ala Val Pro Asn Leu
    210                 215                 220

cac agc gcc aag gac gag ggc ttc atc ggc gac ccg gac atc gac acc      720
His Ser Ala Lys Asp Glu Gly Phe Ile Gly Asp Pro Asp Ile Asp Thr
225                 230                 235                 240

atg ccg ctg ctg gcg tgg tct aag gag atg gcg cgc aag gcg ttc gag      768
Met Pro Leu Leu Ala Trp Ser Lys Glu Met Ala Arg Lys Ala Phe Glu
                245                 250                 255

tcg gcg cac ggc ccg ttc ttc atc cgc aac cag gcg ttc cta tac ttc      816
Ser Ala His Gly Pro Phe Phe Ile Arg Asn Gln Ala Phe Leu Tyr Phe
            260                 265                 270

ccg ctg ctg ctg ctc gcg cgc ctg agc tgg ctc gcg cag tcg ttc ttc      864
Pro Leu Leu Leu Leu Ala Arg Leu Ser Trp Leu Ala Gln Ser Phe Phe
        275                 280                 285

tac gtg ttc acc gag ttc tcg ttc ggc atc ttc gac aag gtc gag ttc      912
Tyr Val Phe Thr Glu Phe Ser Phe Gly Ile Phe Asp Lys Val Glu Phe
    290                 295                 300

gac gga ccg gag aag gcg ggt ctg atc gtg cac tac atc tgg cag ctc      960
Asp Gly Pro Glu Lys Ala Gly Leu Ile Val His Tyr Ile Trp Gln Leu
305                 310                 315                 320

gcg atc ccg tac ttc tgc aac atg agc ctg ttt gag ggc gtg gca tac     1008
Ala Ile Pro Tyr Phe Cys Asn Met Ser Leu Phe Glu Gly Val Ala Tyr
                325                 330                 335

ttc ctc atg ggc cag gcg tcc tgc ggc ttg ctc ctg gcg ctg gtg ttc     1056
Phe Leu Met Gly Gln Ala Ser Cys Gly Leu Leu Leu Ala Leu Val Phe
            340                 345                 350

agt att ggc cac aac ggc atg tcg gtg tac gag cgc gaa acc aag ccg     1104
Ser Ile Gly His Asn Gly Met Ser Val Tyr Glu Arg Glu Thr Lys Pro
        355                 360                 365

gac ttc tgg cag ctg cag gtg acc acg acg cgc aac atc cgc gcg tcg     1152
Asp Phe Trp Gln Leu Gln Val Thr Thr Thr Arg Asn Ile Arg Ala Ser
    370                 375                 380

gta ttc atg gac tgg ttc acc ggt ggc ttg aac tac cag atc gac cat     1200
```

```
Val Phe Met Asp Trp Phe Thr Gly Gly Leu Asn Tyr Gln Ile Asp His
385                 390                 395                 400

cac ctg ttc ccg ctc gtg ccg cgc cac aac ttg cca aag gtc aac gtg      1248
His Leu Phe Pro Leu Val Pro Arg His Asn Leu Pro Lys Val Asn Val
                405                 410                 415

ctc atc aag tcg cta tgc aag gag ttc gac atc ccg ttc cac gag acc      1296
Leu Ile Lys Ser Leu Cys Lys Glu Phe Asp Ile Pro Phe His Glu Thr
            420                 425                 430

ggc ttc tgg gag ggc atc tac gag gtc gtg gac cac ctg gcg gac atc      1344
Gly Phe Trp Glu Gly Ile Tyr Glu Val Val Asp His Leu Ala Asp Ile
        435                 440                 445

agc aag gaa ttt atc acc gag ttc cca gcg atg taa                      1380
Ser Lys Glu Phe Ile Thr Glu Phe Pro Ala Met
    450                 455
```

<210> 194
<211> 459
<212> PRT
<213> Phytium irreguläre

<400> 194

```
Met Val Asp Leu Lys Pro Gly Val Lys Arg Leu Val Ser Trp Lys Glu
1               5                   10                  15

Ile Arg Glu His Ala Thr Pro Ala Thr Ala Trp Ile Val Ile His His
            20                  25                  30

Lys Val Tyr Asp Ile Ser Lys Trp Asp Ser His Pro Gly Gly Ser Val
        35                  40                  45

Met Leu Thr Gln Ala Gly Glu Asp Ala Thr Asp Ala Phe Ala Val Phe
    50                  55                  60

His Pro Ser Ser Ala Leu Lys Leu Leu Glu Gln Phe Tyr Val Gly Asp
65                  70                  75                  80

Val Asp Glu Thr Ser Lys Ala Glu Ile Glu Gly Glu Pro Ala Ser Asp
                85                  90                  95

Glu Glu Arg Ala Arg Arg Glu Arg Ile Asn Glu Phe Ile Ala Ser Tyr
            100                 105                 110

Arg Arg Leu Arg Val Lys Val Lys Gly Met Gly Leu Tyr Asp Ala Ser
        115                 120                 125

Ala Leu Tyr Tyr Ala Trp Lys Leu Val Ser Thr Phe Gly Ile Ala Val
    130                 135                 140
```

```
Leu Ser Met Ala Ile Cys Phe Phe Phe Asn Ser Phe Ala Met Tyr Met
145                 150                 155                 160

Val Ala Gly Val Ile Met Gly Leu Phe Tyr Gln Gln Ser Gly Trp Leu
                165                 170                 175

Ala His Asp Phe Leu His Asn Gln Val Cys Glu Asn Arg Thr Leu Gly
            180                 185                 190

Asn Leu Ile Gly Cys Leu Val Gly Asn Ala Trp Gln Gly Phe Ser Met
        195                 200                 205

Gln Trp Trp Lys Asn Lys His Asn Leu His His Ala Val Pro Asn Leu
    210                 215                 220

His Ser Ala Lys Asp Glu Gly Phe Ile Gly Asp Pro Asp Ile Asp Thr
225                 230                 235                 240

Met Pro Leu Leu Ala Trp Ser Lys Glu Met Ala Arg Lys Ala Phe Glu
                245                 250                 255

Ser Ala His Gly Pro Phe Phe Ile Arg Asn Gln Ala Phe Leu Tyr Phe
            260                 265                 270

Pro Leu Leu Leu Leu Ala Arg Leu Ser Trp Leu Ala Gln Ser Phe Phe
        275                 280                 285

Tyr Val Phe Thr Glu Phe Ser Phe Gly Ile Phe Asp Lys Val Glu Phe
    290                 295                 300

Asp Gly Pro Glu Lys Ala Gly Leu Ile Val His Tyr Ile Trp Gln Leu
305                 310                 315                 320

Ala Ile Pro Tyr Phe Cys Asn Met Ser Leu Phe Glu Gly Val Ala Tyr
                325                 330                 335

Phe Leu Met Gly Gln Ala Ser Cys Gly Leu Leu Leu Ala Leu Val Phe
            340                 345                 350

Ser Ile Gly His Asn Gly Met Ser Val Tyr Glu Arg Glu Thr Lys Pro
        355                 360                 365

Asp Phe Trp Gln Leu Gln Val Thr Thr Thr Arg Asn Ile Arg Ala Ser
    370                 375                 380

Val Phe Met Asp Trp Phe Thr Gly Gly Leu Asn Tyr Gln Ile Asp His
385                 390                 395                 400

His Leu Phe Pro Leu Val Pro Arg His Asn Leu Pro Lys Val Asn Val
                405                 410                 415
```

```
Leu Ile Lys Ser Leu Cys Lys Glu Phe Asp Ile Pro Phe His Glu Thr
            420                     425                     430

Gly Phe Trp Glu Gly Ile Tyr Glu Val Val Asp His Leu Ala Asp Ile
        435                     440                     445

Ser Lys Glu Phe Ile Thr Glu Phe Pro Ala Met
    450                     455
```

<210> 195
<211> 1152
<212> DNA
<213> Calendula officinalis

<220>
<221> CDS
<222> (1)..(1152)
<223> Delta-12-Desaturase

<400> 195

```
atg ggt gca ggc ggt cga atg caa gat ccc acc aac ggt ggc aac aaa       48
Met Gly Ala Gly Gly Arg Met Gln Asp Pro Thr Asn Gly Gly Asn Lys
1               5                   10                  15

acc gag ccc gaa cca atc caa cgg gtc cca cat gaa aaa ccc cca ttc       96
Thr Glu Pro Glu Pro Ile Gln Arg Val Pro His Glu Lys Pro Pro Phe
            20                  25                  30

aca gtt gga gac atc aag aaa gcg atc cca cct cat tgt ttc aac cga      144
Thr Val Gly Asp Ile Lys Lys Ala Ile Pro Pro His Cys Phe Asn Arg
        35                  40                  45

tcg gta att cgt tca ttt tca tac gtc ttt tac gac ctc aca atc gcg      192
Ser Val Ile Arg Ser Phe Ser Tyr Val Phe Tyr Asp Leu Thr Ile Ala
    50                  55                  60

tca atc ttg tac tac att gcc aac aat tac atc tct acc ctc cct agc      240
Ser Ile Leu Tyr Tyr Ile Ala Asn Asn Tyr Ile Ser Thr Leu Pro Ser
65                  70                  75                  80

ccg ctc gcc tac gtg gca tgg ccc gtt tac tgg gcc gtc caa ggg tgc      288
Pro Leu Ala Tyr Val Ala Trp Pro Val Tyr Trp Ala Val Gln Gly Cys
                85                  90                  95

gtc tta acc ggg gtg tgg gtc ata gcc cac gaa tgt ggc cat cat gct      336
Val Leu Thr Gly Val Trp Val Ile Ala His Glu Cys Gly His His Ala
            100                 105                 110

ttt agc gac cac caa tgg ctc gat gac acc gtg ggt ctc gtc ttg cac      384
Phe Ser Asp His Gln Trp Leu Asp Asp Thr Val Gly Leu Val Leu His
        115                 120                 125

tcg ttc cta ctc gtg ccc tac ttt tcg tgg aaa tat agc cac cgt agg      432
Ser Phe Leu Leu Val Pro Tyr Phe Ser Trp Lys Tyr Ser His Arg Arg
    130                 135                 140
```

```
cac cac tcg aac acg ggc tcg atc gag cac gat gag gtt ttc gtc ccg      480
His His Ser Asn Thr Gly Ser Ile Glu His Asp Glu Val Phe Val Pro
145                 150                 155                 160

aag ttg aaa tcg ggc gtc cgg tca acc gcc cgg tac cta aac aac cca      528
Lys Leu Lys Ser Gly Val Arg Ser Thr Ala Arg Tyr Leu Asn Asn Pro
                165                 170                 175

ccg ggc cga atc ttg acc cta ctc gta acc cta acc ctc ggt tgg cct      576
Pro Gly Arg Ile Leu Thr Leu Leu Val Thr Leu Thr Leu Gly Trp Pro
            180                 185                 190

cta tac ctc acg ttc aac gtt tcg ggc cgt tac tac gac cgg ttc gcg      624
Leu Tyr Leu Thr Phe Asn Val Ser Gly Arg Tyr Tyr Asp Arg Phe Ala
        195                 200                 205

tgc cat ttc gac ccg aat agc ccg atc tac tcg aag cgc gaa cgg gct      672
Cys His Phe Asp Pro Asn Ser Pro Ile Tyr Ser Lys Arg Glu Arg Ala
    210                 215                 220

caa atc ttc ata tcc gac gcc ggg atc tta gcc gta gtc ttc gta ctc      720
Gln Ile Phe Ile Ser Asp Ala Gly Ile Leu Ala Val Val Phe Val Leu
225                 230                 235                 240

ttc cga ctc gca atg acc aaa ggg ctc acg tgg gtc cta acc atg tac      768
Phe Arg Leu Ala Met Thr Lys Gly Leu Thr Trp Val Leu Thr Met Tyr
                245                 250                 255

ggt ggc ccg tta ctc gtg gtc aac ggt ttc cta gtc ttg atc aca ttc      816
Gly Gly Pro Leu Leu Val Val Asn Gly Phe Leu Val Leu Ile Thr Phe
            260                 265                 270

cta caa cac act cac cct tcg ctc ccg cac tat gac tca acc gaa tgg      864
Leu Gln His Thr His Pro Ser Leu Pro His Tyr Asp Ser Thr Glu Trp
        275                 280                 285

gat tgg tta cgt ggg gcc ctc acc aca atc gac cgt gat tac ggg atc      912
Asp Trp Leu Arg Gly Ala Leu Thr Thr Ile Asp Arg Asp Tyr Gly Ile
    290                 295                 300

cta aac aaa gtg ttc cat aac ata acc gac act cac gtg gcc cac cat      960
Leu Asn Lys Val Phe His Asn Ile Thr Asp Thr His Val Ala His His
305                 310                 315                 320

ttg ttc tct aca atg cct cat tac cat gca atg gaa gcc acg aag gtg     1008
Leu Phe Ser Thr Met Pro His Tyr His Ala Met Glu Ala Thr Lys Val
                325                 330                 335

atc aaa ccg att ttg ggc gat tat tat cag ttt gac ggg acc tcg att     1056
Ile Lys Pro Ile Leu Gly Asp Tyr Tyr Gln Phe Asp Gly Thr Ser Ile
            340                 345                 350

ttt aag gcg atg tat cgg gaa aca aag gag tgc att tat gtt gat aag     1104
Phe Lys Ala Met Tyr Arg Glu Thr Lys Glu Cys Ile Tyr Val Asp Lys
        355                 360                 365

gat gag gag gtg aaa gat ggt gtt tat tgg tat cgt aat aag att taa     1152
Asp Glu Glu Val Lys Asp Gly Val Tyr Trp Tyr Arg Asn Lys Ile
    370                 375                 380
```

<210> 196
<211> 383
<212> PRT
<213> Calendula officinalis

<400> 196

```
Met Gly Ala Gly Gly Arg Met Gln Asp Pro Thr Asn Gly Gly Asn Lys
1               5                   10                  15

Thr Glu Pro Glu Pro Ile Gln Arg Val Pro His Glu Lys Pro Pro Phe
            20                  25                  30

Thr Val Gly Asp Ile Lys Lys Ala Ile Pro Pro His Cys Phe Asn Arg
        35                  40                  45

Ser Val Ile Arg Ser Phe Ser Tyr Val Phe Tyr Asp Leu Thr Ile Ala
    50                  55                  60

Ser Ile Leu Tyr Tyr Ile Ala Asn Asn Tyr Ile Ser Thr Leu Pro Ser
65                  70                  75                  80

Pro Leu Ala Tyr Val Ala Trp Pro Val Tyr Trp Ala Val Gln Gly Cys
                85                  90                  95

Val Leu Thr Gly Val Trp Val Ile Ala His Glu Cys Gly His His Ala
            100                 105                 110

Phe Ser Asp His Gln Trp Leu Asp Asp Thr Val Gly Leu Val Leu His
        115                 120                 125

Ser Phe Leu Leu Val Pro Tyr Phe Ser Trp Lys Tyr Ser His Arg Arg
    130                 135                 140

His His Ser Asn Thr Gly Ser Ile Glu His Asp Glu Val Phe Val Pro
145                 150                 155                 160

Lys Leu Lys Ser Gly Val Arg Ser Thr Ala Arg Tyr Leu Asn Asn Pro
                165                 170                 175

Pro Gly Arg Ile Leu Thr Leu Leu Val Thr Leu Thr Leu Gly Trp Pro
            180                 185                 190

Leu Tyr Leu Thr Phe Asn Val Ser Gly Arg Tyr Tyr Asp Arg Phe Ala
        195                 200                 205

Cys His Phe Asp Pro Asn Ser Pro Ile Tyr Ser Lys Arg Glu Arg Ala
    210                 215                 220

Gln Ile Phe Ile Ser Asp Ala Gly Ile Leu Ala Val Val Phe Val Leu
225                 230                 235                 240

Phe Arg Leu Ala Met Thr Lys Gly Leu Thr Trp Val Leu Thr Met Tyr
                245                 250                 255
```

```
Gly Gly Pro Leu Leu Val Val Asn Gly Phe Leu Val Leu Ile Thr Phe
            260                 265                 270

Leu Gln His Thr His Pro Ser Leu Pro His Tyr Asp Ser Thr Glu Trp
        275                 280                 285

Asp Trp Leu Arg Gly Ala Leu Thr Thr Ile Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr His Ala Met Glu Ala Thr Lys Val
                325                 330                 335

Ile Lys Pro Ile Leu Gly Asp Tyr Tyr Gln Phe Asp Gly Thr Ser Ile
            340                 345                 350

Phe Lys Ala Met Tyr Arg Glu Thr Lys Glu Cys Ile Tyr Val Asp Lys
        355                 360                 365

Asp Glu Glu Val Lys Asp Gly Val Tyr Trp Tyr Arg Asn Lys Ile
    370                 375                 380
```

<210> 197
<211> 903
<212> DNA
<213> Ostreococcus tauri

<220>
<221> CDS
<222> (1)..(903)
<223> Delta-5-Elongase

<400> 197

```
atg tct gct tct gga gct ttg ttg cct gct att gct ttc gct gct tac        48
Met Ser Ala Ser Gly Ala Leu Leu Pro Ala Ile Ala Phe Ala Ala Tyr
1               5                   10                  15

gct tac gct acc tac gct tat gct ttc gag tgg tct cat gct aac gga        96
Ala Tyr Ala Thr Tyr Ala Tyr Ala Phe Glu Trp Ser His Ala Asn Gly
            20                  25                  30

atc gat aac gtg gat gct aga gag tgg att gga gct ttg tct ttg aga       144
Ile Asp Asn Val Asp Ala Arg Glu Trp Ile Gly Ala Leu Ser Leu Arg
        35                  40                  45

ctc cct gca att gct acc acc atg tac ctc ttg ttc tgc ctt gtg gga       192
```

```
Leu Pro Ala Ile Ala Thr Thr Met Tyr Leu Leu Phe Cys Leu Val Gly
    50                  55                  60

cct aga ttg atg gct aag agg gag gct ttt gat cct aag gga ttc atg      240
Pro Arg Leu Met Ala Lys Arg Glu Ala Phe Asp Pro Lys Gly Phe Met
65                  70                  75                  80

ctc gct tac aac gct tac caa acc gct ttc aac gtt gtg gtg ctc gga      288
Leu Ala Tyr Asn Ala Tyr Gln Thr Ala Phe Asn Val Val Val Leu Gly
                85                  90                  95

atg ttc gct aga gag atc tct gga ttg gga caa cct gtt tgg gga tct      336
Met Phe Ala Arg Glu Ile Ser Gly Leu Gly Gln Pro Val Trp Gly Ser
            100                 105                 110

act atg cct tgg agc gat agg aag tcc ttc aag att ttg ttg gga gtg      384
Thr Met Pro Trp Ser Asp Arg Lys Ser Phe Lys Ile Leu Leu Gly Val
        115                 120                 125

tgg ctc cat tac aac aat aag tac ctc gag ttg ttg gat act gtg ttc      432
Trp Leu His Tyr Asn Asn Lys Tyr Leu Glu Leu Leu Asp Thr Val Phe
    130                 135                 140

atg gtg gct agg aaa aag acc aag cag ctc tct ttc ttg cat gtg tac      480
Met Val Ala Arg Lys Lys Thr Lys Gln Leu Ser Phe Leu His Val Tyr
145                 150                 155                 160

cat cat gct ttg ttg att tgg gct tgg tgg ctt gtt tgt cat ctc atg      528
His His Ala Leu Leu Ile Trp Ala Trp Trp Leu Val Cys His Leu Met
                165                 170                 175

gct acc aac gat tgc atc gat gct tat ttc gga gct gct tgc aac tct      576
Ala Thr Asn Asp Cys Ile Asp Ala Tyr Phe Gly Ala Ala Cys Asn Ser
            180                 185                 190

ttc atc cac atc gtg atg tac tcc tac tac ctc atg tct gct ttg gga      624
Phe Ile His Ile Val Met Tyr Ser Tyr Tyr Leu Met Ser Ala Leu Gly
        195                 200                 205

att aga tgc cct tgg aag aga tat atc acc cag gct cag atg ttg caa      672
Ile Arg Cys Pro Trp Lys Arg Tyr Ile Thr Gln Ala Gln Met Leu Gln
    210                 215                 220

ttc gtg atc gtg ttc gct cat gct gtt ttc gtg ctc aga caa aag cac      720
Phe Val Ile Val Phe Ala His Ala Val Phe Val Leu Arg Gln Lys His
225                 230                 235                 240

tgc cct gtt act ttg cct tgg gca caa atg ttc gtg atg aca aat atg      768
Cys Pro Val Thr Leu Pro Trp Ala Gln Met Phe Val Met Thr Asn Met
                245                 250                 255

ttg gtg ctc ttc gga aac ttc tac ctc aag gct tac tct aac aag tct      816
Leu Val Leu Phe Gly Asn Phe Tyr Leu Lys Ala Tyr Ser Asn Lys Ser
            260                 265                 270

agg gga gat gga gct tct tct gtt aag cct gct gag act act aga gca      864
Arg Gly Asp Gly Ala Ser Ser Val Lys Pro Ala Glu Thr Thr Arg Ala
        275                 280                 285

cct tct gtg aga aga acc agg tcc agg aag atc gat tga                  903
Pro Ser Val Arg Arg Thr Arg Ser Arg Lys Ile Asp
    290                 295                 300
```

<210> 198
<211> 300
<212> PRT
<213> Ostreococcus tauri

<400> 198

```
Met Ser Ala Ser Gly Ala Leu Leu Pro Ala Ile Ala Phe Ala Ala Tyr
1               5                   10                  15

Ala Tyr Ala Thr Tyr Ala Tyr Ala Phe Glu Trp Ser His Ala Asn Gly
            20                  25                  30

Ile Asp Asn Val Asp Ala Arg Glu Trp Ile Gly Ala Leu Ser Leu Arg
        35                  40                  45

Leu Pro Ala Ile Ala Thr Thr Met Tyr Leu Leu Phe Cys Leu Val Gly
    50                  55                  60

Pro Arg Leu Met Ala Lys Arg Glu Ala Phe Asp Pro Lys Gly Phe Met
65                  70                  75                  80

Leu Ala Tyr Asn Ala Tyr Gln Thr Ala Phe Asn Val Val Val Leu Gly
                85                  90                  95

Met Phe Ala Arg Glu Ile Ser Gly Leu Gly Gln Pro Val Trp Gly Ser
            100                 105                 110

Thr Met Pro Trp Ser Asp Arg Lys Ser Phe Lys Ile Leu Leu Gly Val
        115                 120                 125

Trp Leu His Tyr Asn Asn Lys Tyr Leu Glu Leu Leu Asp Thr Val Phe
    130                 135                 140

Met Val Ala Arg Lys Lys Thr Lys Gln Leu Ser Phe Leu His Val Tyr
145                 150                 155                 160

His His Ala Leu Leu Ile Trp Ala Trp Trp Leu Val Cys His Leu Met
                165                 170                 175

Ala Thr Asn Asp Cys Ile Asp Ala Tyr Phe Gly Ala Ala Cys Asn Ser
            180                 185                 190

Phe Ile His Ile Val Met Tyr Ser Tyr Tyr Leu Met Ser Ala Leu Gly
        195                 200                 205

Ile Arg Cys Pro Trp Lys Arg Tyr Ile Thr Gln Ala Gln Met Leu Gln
    210                 215                 220

Phe Val Ile Val Phe Ala His Ala Val Phe Val Leu Arg Gln Lys His
225                 230                 235                 240
```

```
Cys Pro Val Thr Leu Pro Trp Ala Gln Met Phe Val Met Thr Asn Met
                245                 250                 255

Leu Val Leu Phe Gly Asn Phe Tyr Leu Lys Ala Tyr Ser Asn Lys Ser
            260                 265                 270

Arg Gly Asp Gly Ala Ser Ser Val Lys Pro Ala Glu Thr Thr Arg Ala
        275                 280                 285

Pro Ser Val Arg Arg Thr Arg Ser Arg Lys Ile Asp
    290                 295                 300
```

<210> 199
<211> 879
<212> DNA
<213> Ostreococcus tauri

<220>
<221> CDS
<222> (1)..(879)
<223> Delta-6-Elongase

<400> 199

```
atg tct gga ttg agg gct cct aac ttc ttg cat agg ttc tgg acc aag      48
Met Ser Gly Leu Arg Ala Pro Asn Phe Leu His Arg Phe Trp Thr Lys
1               5                   10                  15

tgg gat tac gct atc tct aag gtg gtg ttc act tgc gct gat tct ttc      96
Trp Asp Tyr Ala Ile Ser Lys Val Val Phe Thr Cys Ala Asp Ser Phe
            20                  25                  30

cag tgg gat atc gga cct gtt tct tct tct acc gct cat ttg cct gct     144
Gln Trp Asp Ile Gly Pro Val Ser Ser Ser Thr Ala His Leu Pro Ala
        35                  40                  45

att gag tct cct act cct ttg gtg acc tct ttg ctc ttc tac ttg gtg     192
Ile Glu Ser Pro Thr Pro Leu Val Thr Ser Leu Leu Phe Tyr Leu Val
    50                  55                  60

act gtg ttc ttg tgg tac gga aga ttg acc aga tcc tcc gat aag aag     240
Thr Val Phe Leu Trp Tyr Gly Arg Leu Thr Arg Ser Ser Asp Lys Lys
65                  70                  75                  80

atc aga gag cct acc tgg ttg agg aga ttc atc atc tgc cac aac gct     288
Ile Arg Glu Pro Thr Trp Leu Arg Arg Phe Ile Ile Cys His Asn Ala
                85                  90                  95

ttc ttg att gtg ctc tcc ttg tac atg tgt ttg gga tgc gtt gct caa     336
Phe Leu Ile Val Leu Ser Leu Tyr Met Cys Leu Gly Cys Val Ala Gln
            100                 105                 110

gct tac caa aac gga tac acc ttg tgg gga aac gag ttc aag gct act     384
Ala Tyr Gln Asn Gly Tyr Thr Leu Trp Gly Asn Glu Phe Lys Ala Thr
        115                 120                 125
```

```
gag acc caa ttg gct ctc tac atc tac atc ttc tac gtg tcc aag atc      432
Glu Thr Gln Leu Ala Leu Tyr Ile Tyr Ile Phe Tyr Val Ser Lys Ile
    130                 135                 140

tac gag ttc gtg gat acc tac atc atg ctc ctc aag aac aac ctc agg      480
Tyr Glu Phe Val Asp Thr Tyr Ile Met Leu Leu Lys Asn Asn Leu Arg
145                 150                 155                 160

caa gtg tct ttc ttg cac atc tac cac cac tct acc atc tct ttc atc      528
Gln Val Ser Phe Leu His Ile Tyr His His Ser Thr Ile Ser Phe Ile
                165                 170                 175

tgg tgg atc atc gct aga aga gca cct gga gga gat gct tat ttc tcc      576
Trp Trp Ile Ile Ala Arg Arg Ala Pro Gly Gly Asp Ala Tyr Phe Ser
            180                 185                 190

gct gct ctc aac tct tgg gtt cat gtg tgc atg tac act tac tac ctc      624
Ala Ala Leu Asn Ser Trp Val His Val Cys Met Tyr Thr Tyr Tyr Leu
        195                 200                 205

ctc tct acc ttg att gga aag gaa gat cct aag agg tct aac tac ctc      672
Leu Ser Thr Leu Ile Gly Lys Glu Asp Pro Lys Arg Ser Asn Tyr Leu
    210                 215                 220

tgg tgg gga agg cat ttg acc caa atg caa atg ctc cag ttc ttc ttc      720
Trp Trp Gly Arg His Leu Thr Gln Met Gln Met Leu Gln Phe Phe Phe
225                 230                 235                 240

aac gtg ctc caa gct ctt tat tgc gct tcc ttc tcc act tac cct aag      768
Asn Val Leu Gln Ala Leu Tyr Cys Ala Ser Phe Ser Thr Tyr Pro Lys
                245                 250                 255

ttc ctc tcc aag atc ttg ctc gtg tac atg atg tct ttg ctc gga ctt      816
Phe Leu Ser Lys Ile Leu Leu Val Tyr Met Met Ser Leu Leu Gly Leu
            260                 265                 270

ttc gga cac ttc tac tac tct aag cac atc gct gct gct aag ttg caa      864
Phe Gly His Phe Tyr Tyr Ser Lys His Ile Ala Ala Ala Lys Leu Gln
        275                 280                 285

aag aag cag cag tga                                                  879
Lys Lys Gln Gln
    290
```

<210> 200
<211> 292
<212> PRT
<213> Ostreococcus tauri

<400> 200

```
Met Ser Gly Leu Arg Ala Pro Asn Phe Leu His Arg Phe Trp Thr Lys
1               5                   10                  15

Trp Asp Tyr Ala Ile Ser Lys Val Val Phe Thr Cys Ala Asp Ser Phe
            20                  25                  30

Gln Trp Asp Ile Gly Pro Val Ser Ser Ser Thr Ala His Leu Pro Ala
        35                  40                  45
```

Ile Glu Ser Pro Thr Pro Leu Val Thr Ser Leu Leu Phe Tyr Leu Val
50 55 60

Thr Val Phe Leu Trp Tyr Gly Arg Leu Thr Arg Ser Ser Asp Lys Lys
65 70 75 80

Ile Arg Glu Pro Thr Trp Leu Arg Arg Phe Ile Ile Cys His Asn Ala
85 90 95

Phe Leu Ile Val Leu Ser Leu Tyr Met Cys Leu Gly Cys Val Ala Gln
100 105 110

Ala Tyr Gln Asn Gly Tyr Thr Leu Trp Gly Asn Glu Phe Lys Ala Thr
115 120 125

Glu Thr Gln Leu Ala Leu Tyr Ile Tyr Ile Phe Tyr Val Ser Lys Ile
130 135 140

Tyr Glu Phe Val Asp Thr Tyr Ile Met Leu Leu Lys Asn Asn Leu Arg
145 150 155 160

Gln Val Ser Phe Leu His Ile Tyr His His Ser Thr Ile Ser Phe Ile
165 170 175

Trp Trp Ile Ile Ala Arg Arg Ala Pro Gly Gly Asp Ala Tyr Phe Ser
180 185 190

Ala Ala Leu Asn Ser Trp Val His Val Cys Met Tyr Thr Tyr Tyr Leu
195 200 205

Leu Ser Thr Leu Ile Gly Lys Glu Asp Pro Lys Arg Ser Asn Tyr Leu
210 215 220

Trp Trp Gly Arg His Leu Thr Gln Met Gln Met Leu Gln Phe Phe Phe
225 230 235 240

Asn Val Leu Gln Ala Leu Tyr Cys Ala Ser Phe Ser Thr Tyr Pro Lys
245 250 255

Phe Leu Ser Lys Ile Leu Leu Val Tyr Met Met Ser Leu Leu Gly Leu
260 265 270

Phe Gly His Phe Tyr Tyr Ser Lys His Ile Ala Ala Ala Lys Leu Gln
275 280 285

Lys Lys Gln Gln
290

<210> 201
<211> 1421
<212> DNA
<213> Ostreococcus tauri

<220>
<221> CDS
<222> (26)..(1399)
<223> Delta-6-Desaturase

<400> 201

```
ggatccttaa ttaaggcgcg ccaaa atg tgt gtt gag acc gag aac aac gat       52
                            Met Cys Val Glu Thr Glu Asn Asn Asp
                            1               5

gga atc cct act gtg gag atc gct ttc gat gga gag aga gaa aga gct      100
Gly Ile Pro Thr Val Glu Ile Ala Phe Asp Gly Glu Arg Glu Arg Ala
10                  15                  20                  25

gag gct aac gtg aag ttg tct gct gag aag atg gaa cct gct gct ttg      148
Glu Ala Asn Val Lys Leu Ser Ala Glu Lys Met Glu Pro Ala Ala Leu
                30                  35                  40

gct aag acc ttc gct aga aga tac gtg gtt atc gag gga gtt gag tac      196
Ala Lys Thr Phe Ala Arg Arg Tyr Val Val Ile Glu Gly Val Glu Tyr
            45                  50                  55

gat gtg acc gat ttc aaa cat cct gga gga acc gtg att ttc tac gct      244
Asp Val Thr Asp Phe Lys His Pro Gly Gly Thr Val Ile Phe Tyr Ala
        60                  65                  70

ctc tct aac act gga gct gat gct act gag gct ttc aag gag ttc cac      292
Leu Ser Asn Thr Gly Ala Asp Ala Thr Glu Ala Phe Lys Glu Phe His
    75                  80                  85

cac aga tct aga aag gct agg aag gct ttg gct gct ttg cct tct aga      340
His Arg Ser Arg Lys Ala Arg Lys Ala Leu Ala Ala Leu Pro Ser Arg
90                  95                  100                 105

cct gct aag acc gct aaa gtg gat gat gct gag atg ctc cag gat ttc      388
Pro Ala Lys Thr Ala Lys Val Asp Asp Ala Glu Met Leu Gln Asp Phe
                110                 115                 120

gct aag tgg aga aag gag ttg gag agg gac gga ttc ttc aag cct tct      436
Ala Lys Trp Arg Lys Glu Leu Glu Arg Asp Gly Phe Phe Lys Pro Ser
            125                 130                 135

cct gct cat gtt gct tac aga ttc gct gag ttg gct gct atg tac gct      484
Pro Ala His Val Ala Tyr Arg Phe Ala Glu Leu Ala Ala Met Tyr Ala
        140                 145                 150

ttg gga acc tac ttg atg tac gct aga tac gtt gtg tcc tct gtg ttg      532
Leu Gly Thr Tyr Leu Met Tyr Ala Arg Tyr Val Val Ser Ser Val Leu
    155                 160                 165

gtt tac gct tgc ttc ttc gga gct aga tgt gga tgg gtt caa cat gag      580
Val Tyr Ala Cys Phe Phe Gly Ala Arg Cys Gly Trp Val Gln His Glu
170                 175                 180                 185

gga gga cat tct tct ttg acc gga aac atc tgg tgg gat aag aga atc      628
```

```
Gly Gly His Ser Ser Leu Thr Gly Asn Ile Trp Trp Asp Lys Arg Ile
                190                 195                 200

caa gct ttc act gct gga ttc gga ttg gct gga tct gga gat atg tgg       676
Gln Ala Phe Thr Ala Gly Phe Gly Leu Ala Gly Ser Gly Asp Met Trp
            205                 210                 215

aac tcc atg cac aac aag cac cat gct act cct caa aaa gtg agg cac       724
Asn Ser Met His Asn Lys His His Ala Thr Pro Gln Lys Val Arg His
        220                 225                 230

gat atg gat ttg gat acc act cct gct gtt gct ttc ttc aac acc gct       772
Asp Met Asp Leu Asp Thr Thr Pro Ala Val Ala Phe Phe Asn Thr Ala
    235                 240                 245

gtg gag gat aat aga cct agg gga ttc tct aag tac tgg ctc aga ttg       820
Val Glu Asp Asn Arg Pro Arg Gly Phe Ser Lys Tyr Trp Leu Arg Leu
250                 255                 260                 265

caa gct tgg acc ttc att cct gtg act tct gga ttg gtg ttg ctc ttc       868
Gln Ala Trp Thr Phe Ile Pro Val Thr Ser Gly Leu Val Leu Leu Phe
                270                 275                 280

tgg atg ttc ttc ctc cat cct tct aag gct ttg aag gga gga aag tac       916
Trp Met Phe Phe Leu His Pro Ser Lys Ala Leu Lys Gly Gly Lys Tyr
            285                 290                 295

gag gag ctt gtg tgg atg ttg gct gct cat gtg att aga acc tgg acc       964
Glu Glu Leu Val Trp Met Leu Ala Ala His Val Ile Arg Thr Trp Thr
        300                 305                 310

att aag gct gtt act gga ttc acc gct atg caa tcc tac gga ctc ttc      1012
Ile Lys Ala Val Thr Gly Phe Thr Ala Met Gln Ser Tyr Gly Leu Phe
    315                 320                 325

ttg gct act tct tgg gtt tcc gga tgc tac ttg ttc gct cac ttc tct      1060
Leu Ala Thr Ser Trp Val Ser Gly Cys Tyr Leu Phe Ala His Phe Ser
330                 335                 340                 345

act tct cac acc cat ttg gat gtt gtt cct gct gat gag cat ttg tct      1108
Thr Ser His Thr His Leu Asp Val Val Pro Ala Asp Glu His Leu Ser
                350                 355                 360

tgg gtt agg tac gct gtg gat cac acc att gat atc gat cct tct cag      1156
Trp Val Arg Tyr Ala Val Asp His Thr Ile Asp Ile Asp Pro Ser Gln
            365                 370                 375

gga tgg gtt aac tgg ttg atg gga tac ttg aac tgc caa gtg att cat      1204
Gly Trp Val Asn Trp Leu Met Gly Tyr Leu Asn Cys Gln Val Ile His
        380                 385                 390

cac ctc ttc cct tct atg cct caa ttc aga caa cct gag gtg tcc aga      1252
His Leu Phe Pro Ser Met Pro Gln Phe Arg Gln Pro Glu Val Ser Arg
    395                 400                 405

aga ttc gtt gct ttc gct aag aag tgg aac ctc aac tac aag gtg atg      1300
Arg Phe Val Ala Phe Ala Lys Lys Trp Asn Leu Asn Tyr Lys Val Met
410                 415                 420                 425

act tat gct gga gct tgg aag gct act ttg gga aac ctc gat aat gtg      1348
Thr Tyr Ala Gly Ala Trp Lys Ala Thr Leu Gly Asn Leu Asp Asn Val
                430                 435                 440

gga aag cac tac tac gtg cac gga caa cat tct gga aag acc gct tga      1396
Gly Lys His Tyr Tyr Val His Gly Gln His Ser Gly Lys Thr Ala
            445                 450                 455

taa ttaattaagg cgcgccgaat tc                                         1421
```

<210> 202
<211> 456
<212> PRT
<213> Ostreococcus tauri

<400> 202

```
Met Cys Val Glu Thr Glu Asn Asn Asp Gly Ile Pro Thr Val Glu Ile
1               5                   10                  15

Ala Phe Asp Gly Glu Arg Glu Arg Ala Glu Ala Asn Val Lys Leu Ser
            20                  25                  30

Ala Glu Lys Met Glu Pro Ala Ala Leu Ala Lys Thr Phe Ala Arg Arg
        35                  40                  45

Tyr Val Val Ile Glu Gly Val Glu Tyr Asp Val Thr Asp Phe Lys His
    50                  55                  60

Pro Gly Gly Thr Val Ile Phe Tyr Ala Leu Ser Asn Thr Gly Ala Asp
65                  70                  75                  80

Ala Thr Glu Ala Phe Lys Glu Phe His His Arg Ser Arg Lys Ala Arg
                85                  90                  95

Lys Ala Leu Ala Ala Leu Pro Ser Arg Pro Ala Lys Thr Ala Lys Val
            100                 105                 110

Asp Asp Ala Glu Met Leu Gln Asp Phe Ala Lys Trp Arg Lys Glu Leu
        115                 120                 125

Glu Arg Asp Gly Phe Phe Lys Pro Ser Pro Ala His Val Ala Tyr Arg
    130                 135                 140

Phe Ala Glu Leu Ala Ala Met Tyr Ala Leu Gly Thr Tyr Leu Met Tyr
145                 150                 155                 160

Ala Arg Tyr Val Val Ser Ser Val Leu Val Tyr Ala Cys Phe Phe Gly
                165                 170                 175

Ala Arg Cys Gly Trp Val Gln His Glu Gly Gly His Ser Ser Leu Thr
            180                 185                 190

Gly Asn Ile Trp Trp Asp Lys Arg Ile Gln Ala Phe Thr Ala Gly Phe
        195                 200                 205

Gly Leu Ala Gly Ser Gly Asp Met Trp Asn Ser Met His Asn Lys His
    210                 215                 220
```

His Ala Thr Pro Gln Lys Val Arg His Asp Met Asp Leu Asp Thr Thr
225 230 235 240

Pro Ala Val Ala Phe Phe Asn Thr Ala Val Glu Asp Asn Arg Pro Arg
245 250 255

Gly Phe Ser Lys Tyr Trp Leu Arg Leu Gln Ala Trp Thr Phe Ile Pro
260 265 270

Val Thr Ser Gly Leu Val Leu Leu Phe Trp Met Phe Phe Leu His Pro
275 280 285

Ser Lys Ala Leu Lys Gly Gly Lys Tyr Glu Glu Leu Val Trp Met Leu
290 295 300

Ala Ala His Val Ile Arg Thr Trp Thr Ile Lys Ala Val Thr Gly Phe
305 310 315 320

Thr Ala Met Gln Ser Tyr Gly Leu Phe Leu Ala Thr Ser Trp Val Ser
325 330 335

Gly Cys Tyr Leu Phe Ala His Phe Ser Thr Ser His Thr His Leu Asp
340 345 350

Val Val Pro Ala Asp Glu His Leu Ser Trp Val Arg Tyr Ala Val Asp
355 360 365

His Thr Ile Asp Ile Asp Pro Ser Gln Gly Trp Val Asn Trp Leu Met
370 375 380

Gly Tyr Leu Asn Cys Gln Val Ile His His Leu Phe Pro Ser Met Pro
385 390 395 400

Gln Phe Arg Gln Pro Glu Val Ser Arg Arg Phe Val Ala Phe Ala Lys
405 410 415

Lys Trp Asn Leu Asn Tyr Lys Val Met Thr Tyr Ala Gly Ala Trp Lys
420 425 430

Ala Thr Leu Gly Asn Leu Asp Asn Val Gly Lys His Tyr Tyr Val His
435 440 445

Gly Gln His Ser Gly Lys Thr Ala
450 455

## Beschreibung

Die vorliegende Erfindung betrifft ein Verfahren zur Herstellung von mehrfach ungesättigten Fettsäuren im Samen transgener Pflanzen, indem Nukleinsäuren in den Organismus eingebracht werden, die für Polypeptide mit ω-3-Desaturase-, Δ-12-Desaturase-, Δ-6-Desaturase-, Δ-6-Elongase-, Δ-5-Desaturase-, Δ-5-Elongase- und/oder Δ-4-Desaturaseaktivität bevorzugt für Polypeptide mit Δ-6-Desaturase-, Δ-6-Elongase- und Δ-5-Desaturaseaktivität codieren.

Bei den Nukleinsäuresequenzen handelt es sich um die in SEQ ID NO: 11, SEQ ID NO: 27, SEQ ID NO: 193, SEQ ID NO: 197, SEQ ID NO: 199 und SEQ ID NO: 201 dargestellten Sequenzen. Bevorzugt wird neben diesen Nukleinsäuresequenzen eine weitere Nukleinsäuresequenz, die für ein Polypeptid mit einer Δ-12-Desaturaseaktivität kodiert, in die Pflanze eingebracht und ebenfalls gleichzeitig exprimiert. Besonders bevorzugt handelt es sich dabei um die in SEQ ID NO: 195 dargestellte Nukleinsäuresequenz.

Vorteilhaft können diese Nukleinsäuresequenzen gegebenenfalls zusammen mit weiteren Nukleinsäuresequenzen, die für Polypeptide der Biosynthese des Fettsäure- oder Lipidstoffwechels codieren, in dem Organismus exprimiert werden. Besonders vorteilhaft sind Nukleinsäuresequenzen, die für eine Δ-6-Desaturase-, eine Δ-5-Desaturase-, Δ-4-Desaturase-, Δ-12-Desaturase- und/oder Δ-6-Elongaseaktivität codieren. Vorteilhaft stammen diese Desaturasen und Elongasen aus Thalassiosira, Euglena oder Ostreococcus. Weiterhin betrifft die Erfindung ein Verfahren zur Herstellung von Ölen und/oder Triacylglyceriden mit einem erhöhten Gehalt an langkettigen mehrfach ungesättigten Fettsäuren.

Die Erfindung betrifft in einer bevorzugten Ausführungsform außerdem ein Verfahren zur Herstellung von Arachidonsäure, Eicosapentaensäure oder Docosahexaensäure sowie ein Verfahren zur Herstellung von Triglyceriden mit einem erhöhten Gehalt an ungesättigten Fettsäuren, insbesondere Arachidonsäure, Eicosapentaensäure und/oder Docosahexaensäure, in transgenen Pflanzen vorteilhaft im Samen der transgenen Pflanze. Die Erfindung betrifft die Herstellung einer transgenen Pflanze mit erhöhtem Gehalt an mehrfach ungesättigten Fettsäuren, insbesondere Arachidonsäure, Eicosapentaensäure und/oder Docosahexaensäure, aufgrund der Expression der im erfindungsgemäßen Verfahren verwendeten Elongasen und Desaturasen.

Die Erfindung betrifft weiterhin rekombinante Nukleinsäuremoleküle, die die Nukleinsäuresequenzen, die für die Polypeptide mit Δ-6-Desaturase-, Δ-6-Elongase-, Δ-5-Desaturase- und Δ-5-Elongaseaktivität kodieren, gemeinsam oder einzeln enthalten, sowie transgene Pflanzen, die die vorgenannten rekombinanten Nukleinsäuremoleküle enthalten.

Ein weiterer Teil der Erfindung betrifft Öle, Lipide und/oder Fettsäuren hergestellt nach dem erfindungsgemäßen Verfahren und deren Verwendung. Außerdem betrifft die Erfindung ungesättigte Fettsäuren sowie Triglyceride mit einem erhöhten Gehalt an ungesättigten Fettsäuren und deren Verwendung.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend für die weiteren Elongationen aus den Phospholipiden wieder in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und - Assemblierung einschließlich der Literaturstellen darin siehe in den folgenden Artikeln: Kinney, 1997, Genetic Engineering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engineering, Hrsgb.: JK Setlow, 18:111-13; Gerhardt, 1992, Prog. Lipid R. 31:397-417; Gühnemann-Schäfer & Kindl, 1995, Biochim. Biophys

Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA,** mehrfach ungesättigte Fettsäuren; **l**ong **c**hain **p**oly **u**nsaturated **f**atty **a**cids, **LCPUFA,** langkettige mehrfach ungesättigte Fettsäuren).

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfachungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar. So werden z.B. in der humanen Ernährung Lipide mit ungesättigten Fettsäuren, speziell mehrfach ungesättigten, Fettsäuren bevorzugt. Den mehrfach ungesättigten ω-3-Fettsäuren wird dabei ein positiver Effekt auf den Cholesterinspiegel im Blut und damit auf die Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden (Shimikawa 2001, World Rev. Nutr. Diet. 88, 100-108).

Auch entzündliche, speziell chronisch entzündliche, Prozesse im Rahmen immunologischer Erkrankungen wie rheumatoider Arthritis lassen sich durch ω-3-Fettsäuren positiv beeinflussen (Calder 2002, Proc. Nutr. Soc. 61, 345-358; Cleland und James 2000, J. Rheumatol. 27, 2305-2307). Sie werden deshalb Lebensmitteln, speziell diätetischen Lebensmitteln, zugegeben oder finden in Medikamenten Anwendung. ω-6-Fettsäuren wie Arachidonsäure üben bei diesen rheumatischen Erkrankungen eher einen negativen Effekt aus.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, und den Thromboxanen und Leukotrienen, die sich von der Arachidonsäure und der Eicosapentaensäure ableiten. Eicosanoide (sog. $PG_2$-Serie), die aus ω-6-Fettsäuren gebildet werden, fördern in der Regel Entzündungsreaktionen, während Eicosanoide (sog. $PG_3$-Serie) aus ω-3-Fettsäuren geringe oder keine entzündungsfördernde Wirkung haben.

Mehrfach ungesättigte langkettige ω-3-Fettsäuren wie Eicosapentaensäure (= EPA, $C20{:}5^{\Delta5,8,11,14,17}$) oder Docosahexaensäure (= DHA, $C22{:}6^{\Delta4,7,10,13,16,19}$) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, $C22{:}6^{\Delta4,7,10,13,16,19}$) oder Eisosapentaensäure (= EPA, $C20{:}5^{\Delta5,8,11,14,17}$) Babynahrung zur Erhöhung des Nährwertes zugesetzt. Der ungesättigten Fettsäure DHA wird dabei ein positiver Effekt auf die Entwicklung und Aufrechterhaltung von Gehirnfunktionen zugeschrieben. Es besteht aus diesem Grund ein Bedarf an der Produktion mehrfach ungesättigter langkettiger Fettsäuren.

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, $C20{:}4^{\Delta5,8,11,14}$), Dihomo-γ-linolensäure ($C20{:}3^{\Delta8,11,14}$) oder Docosapentaensäure (DPA, $C22{:}5^{\Delta7,10,13,16,19}$) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck werden Öle mit gesättigten oder ungesättigten Fettsäuren bevorzugt. So werden z.B. in der humanen Ernährung Lipide mit ungesättigten Fettsäuren speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch ω-3-Fettsäuren positiv beeinflussen. Sie werden deshalb Lebensmitteln speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen

negativen Effekt auf diese Krankheiten.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben. Die Anwendung zur Produktion in transgenen Organismen wird in WO98/46763 WO98/46764, WO9846765 beschrieben. Die Expression verschiedener Desaturasen wird in WO99/64616 oder WO98/46776 beschrieben und beansprucht. Bzgl. der Effektivität der Expression von Desaturasen und ihrem Einfluss auf die Bildung mehrfach ungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden.

In der Vergangenheit wurden zahlreiche Versuche unternommen, Elongase-Gene zu erhalten. Millar and Kunst, 1997 (Plant Journal 12:121-131) und Millar et al., 1999 (Plant Cell 11:825-838) beschreiben die Charakterisierung von pflanzlichen Elongasen zur Synthese von einfach ungesättigten langkettigen Fettsäuren (C22:1) bzw. zur Synthese von sehr langkettigen Fettsäuren für die Wachsbildung in Pflanzen ($C_{28}$-$C_{32}$). Beschreibungen zur Synthese von Arachidonsäure und EPA finden sich beispielsweise in WO 01/59128, WO 00/12720, WO 02/077213 und WO 02/08401. Die Synthese von mehrfach ungesättigter C24-Fettsäuren ist beispielsweise in Tvrdik et al. 2000, J. Cell Biol. 149:707-718 oder WO 02/44320 beschrieben.

Besonders geeignete Mikroorganismen zur Herstellung von PUFAs sind Mikroorganismen wie Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moosen wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und ARA anfallen.

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu werden vorteilhafterweise über gentechnische Methoden Gene, die für Enzyme der Biosynthese von LCPUFAs kodieren, in Ölsaaten eingeführt und exprimiert, vorteilhaft im Samen exprimiert. Dies sind Gene, die beispielsweise für Δ-6-Desaturasen, Δ-6-Elongasen, Δ-5-Desaturasen, Δ-5-Elongasen oder Δ-4-Desaturasen kodieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert werden.

Für die Synthese von Arachidonsäure, Eicosapentaensäure (EPA) und Docosahexaensäure (DHA) werden verschiedene Synthesewege diskutiert (Figur. 1). So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des beschriebenen Weges über Δ6-Desaturase, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein

weiterer Elongationsschritt auf $C_{24}$, eine weitere Δ6-Desaturierung und abschliessend eine β-Oxidation auf die $C_{22}$-Kettenlänge. Für die Herstellung in Pflanzen und Mikroorganismen ist der sogenannte Sprecher-Syntheseweg (siehe Figur 1) allerdings nicht geeignet, da die Regulationsmechanismen nicht bekannt sind.

Die polyungesättigten Fettsäuren können entsprechend ihrem Desaturierungsmuster in zwei große Klassen, in ω-6- oder ω-3-Fettsäuren eingeteilt werden, die metabolisch und funktionell unterschiedlich Aktivitäten haben (Fig. 1).

Als Ausgangsprodukt für den ω-6-Stoffwechselweg fungiert die Fettsäure Linolsäure ($18{:}2^{\Delta 9,12}$), während der ω-3-Weg über Linolensäure ($18{:}3^{\Delta 9,12,15}$) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113).

Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, $20{:}4^{\Delta 5,8,11,14}$), eine ω-6-Fettsäure und die beiden ω-3-Fettsäuren Eicosapentaen- (= EPA, $20{:}5^{\Delta 5,8,11,14,17}$) und Docosahexaensäure (DHA, $22{:}6^{\Delta 4,7,10,13,17,19}$) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthridis (Cleland und James 2000, J. Rheumatol. 27, 2305-2307).

Aus ernährungsphysiologischer Sicht ist es deshalb günstig eine Verschiebung zwischen dem ω-6-Syntheseweg und dem ω-3-Syntheseweg (siehe Figur 1) zu erreichen, so dass mehr ω-3-Fettsäuren hergestellt werden. In der Literatur wurden die enzymatischen Aktivitäten verschiedener ω-3-Desaturasen beschrieben, die $C_{18:2}$-, $C_{22:4}$- oder $C_{22:5}$-Fettsäuren desaturieren (siehe Figur 1). Keine der biochemisch beschriebenen Desaturasen setzt jedoch ein breites Substratspektrum des ω-6-Synthesewegs zu den entsprechenden Fettsäuren des ω-3-Syntheseweg um.

Die Verlängerung von Fettsäuren durch Elongasen um 2 bzw. 4 C-Atome ist für die Produktion von $C_{20}$- bzw. $C_{22}$-PUFAs von entscheidender Bedeutung. Dieser Prozess verläuft über 4 Stufen. Der erste Schritt stellt die Kondensation von Malonyl-CoA an das Fettsäure-Acyl-CoA durch die Ketoacyl-CoA-Synthase (KCS, im weiteren Text als Elongase bezeichnet). Es folgt dann ein Reduktionschritt (Ketoacyl-CoA-Reduktase, KCR), ein Dehydratationsschritt (Dehydratase) und ein abschliessender Reduktionsschritt (enoyl-CoA-Reduktase). Es wurde postuliert, dass die Aktivität der Elongase die Spezifität und Geschwindigkeit des gesamten Prozesses beeinflussen (Millar and Kunst, 1997 Plant Journal 12:121-131).

Zur Herstellung von DHA (C22:6 n-3) in Organismen, die diese Fettsäure natürlicherweise nicht produzieren, wurde bisher keine spezifische Elongase beschrieben. Bisher wurden nur Elongasen beschrieben, die $C_{20}$- bzw. $C_{24}$-Fettsäuren bereitstellen. Eine Δ-5-Elongase-Aktivität wurde bisher noch nicht beschrieben.

Erste transgene Pflanzen, die für Enzyme der LCPUFA-Biosynthese kodierende Gene enthalten und exprimieren und als Folge dessen LCPUFAs produzieren, wurden beispielsweise in DE 102 19 203 (Verfahren zur Herstellung mehrfach ungesättigter Fettsäuren in Pflanzen) oder WO 2004/071467 beschrieben. Diese Pflanzen produzieren allerdings LCPUFAs in Mengen, die für eine Aufarbeitung der in den Pflanzen enthaltenen Öle noch weiter optimiert werden müssen. So beträgt der Gehalt von ARA in den in DE-A-102 19 203 beschriebenen Pflanzen lediglich 0,4 bis 2% und der Gehalt von EPA lediglich 0,5 bis 1%, jeweils bezogen auf den Gesamtlipidgehalt der Pflanze. In WO 2004/071467 werden höhere Gehalte an mehrfach ungesättigten $C_{20}$- und $C_{22}$-Fettsäuren, wie ARA, EPA oder DHA offenbart. Jedoch weist das offenbarte Verfahren einige gravierende Nachteile auf. DHA lässt sich im offenbarten Verfahren offenbar überhaupt nicht im Samen nachweisen. Für eine Herstellung von PUFAs ist Soja aufgrund des geringen Ölgehalts von ca. nur 20 Gew.-% weniger geeignet. Soja ist eine vorteilhafte Proteinquelle und wird deshalb in großem Umfang angebaut. Der Ölgehalt von Soja ist jedoch eher gering. Weiterhin ist der im Herstellungsverfahren erzielte Gehalt an Dihomo-γ-linolensäure (=DGHL oder HGLA) viel zu hoch. In Fisch- oder Algenölen oder mikrobiellen Ölen ist HGLA kaum nachweisbar. Ein weiterer Nachteil ist, dass die in WO 2004/071467 offenbarten Pflanzen durch Cotransformation erzeugt wurden, dies führt zur Aufspaltung der Eigenschaften in den folgenden Generationen und damit zu einem erhöhten Selektionsaufwand.

Um eine Anreicherung der Nahrung und/oder des Futters mit diesen mehrfach ungesättigten Fettsäuren zu ermöglichen, besteht daher nachwievor ein großer Bedarf an einem einfachen, kostengünstigen Verfahren zur Herstellung dieser mehrfach ungesättigten Fettsäuren in pflanzlichen Systemen speziell im Samen von transgenen Pflanzen.

Daher bestand die Aufgabe der Erfindung darin, ein Verfahren zur Herstellung großer Mengen von mehrfach ungesättigten Fettsäuren, speziell ARA, EPA und DHA, im Samen einer transgenen Pflanze zu entwickeln.

Diese Aufgabe wurde gelöst durch durch das erfindungsgemäße zur Verfügungstellen einer Öl-produzierende Pflanze exprimierend unter Kontrolle von Samen-spezifischen Promotoren oder Promotoren aktiv in Pflanzenembryo oder Endosperm

a) eine delta-6 Desaturase,
b) eine delta-6 Elongase,
c) eine delta-5 Desaturase,

d) eine delta-5 Elongase und
e) eine delta-12 Desaturase,

wobei die Pflanze in ihrem Samen unabhängig voneinander ungesättigte C18-, C20-, oder C22-Alkylcarbonylsäuren mit mindestens drei, vier, fünf oder sechs Doppelbindungen herstellt.
Herin beschrieben ist auch ein Verfahren zur Herstellung von Verbindungen der allgemeinen Formel I

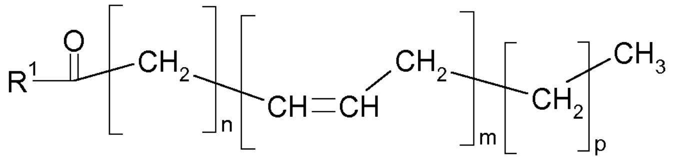
(I)

im Samen von transgenen Pflanzen mit einem Gehalt von mindestens 20 Gew.-% bezogen auf den Gesamtlipidgehalt, dass es folgende Verfahrensschritte umfasst:

a) Einbringen mindestens einer Nukleinsäuresequenz in den Organismus, welche für eine Δ-9-Elongase- und/oder eine Δ-6-Desaturase-Aktivität codiert, und

b) Einbringen mindestens einer Nukleinsäuresequenz in den Organismus, welche für eine Δ-8-Desaturase- und/oder eine Δ-6-Elongase-Aktivität codiert, und

c) Einbringen mindestens einer Nukleinsäuresequenz in den Organismus, welche für eine Δ-5-Desaturase-Aktivität codiert, und

d) Einbringen mindestens einer Nukleinsäuresequenz in den Organismus, welche für eine Δ-5-Elongase-Aktivität codiert, und

e) Einbringen mindestens einer Nukleinsäuresequenz in den Organismus, welche für eine Δ-4-Desaturase-Aktivität codiert, und

wobei die Variablen und Substituenten in der Formel I die folgende Bedeutung haben:

$R^1$ = Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

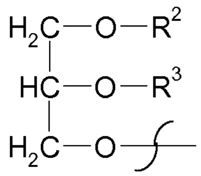
(II)

$R^2$ = Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes $C_2$-$C_{24}$-Alkylcarbonyl-,
$R^3$ = Wasserstoff-, gesättigtes oder ungesättigtes $C_2$-$C_{24}$-Alkylcarbonyl-, oder $R^2$ oder $R^3$ unabhängig voneinander einen Rest der allgemeinen Formel Ia:

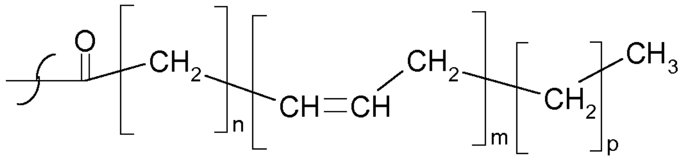
(Ia)

n = 2, 3, 4, 5, 6, 7 oder 9, m = 2, 3, 4, 5 oder 6 und p = 0 oder 3, gelöst. Vorteilhaft bedeuten die Variablen n, m und p in den vorgenannten Formel I und la folgendes: n = 2, 3 oder 5, m = 4, 5 oder 6 und p = 0 oder 3. In einer besonders vorteilhaften Ausführung des Verfahrens bedeuten die Variable n, m und p in den Formeln I und la das folgende: m = 4, n = 3, p = 3 und die Verbindungen der allgemeinen Formel I und la bedeuten damit Arachidonsäure und/oder m = 5, n = 3, p = 0 und die Verbindungen der allgemeinen Formel I und la bedeuten damit Eicosapentaensäure und/oder m = 5, n = 5, p = 0 und die Verbindungen der allgemeinen Formel I und la bedeuten damit Docosapentaensäure ist und/oder m = 6, n = 3, p = 0 und die Verbindungen der allgemeinen Formel I und la bedeuten damit Docosahexaensäure ist.

$R^1$ bedeutet in der allgemeinen Formel I Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

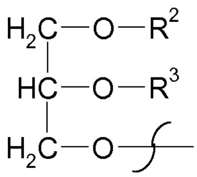

(II)

Die oben genannten Reste von $R^1$ sind immer in Form ihrer Thioester an die Verbindungen der allgemeinen Formel I gebunden.

$R^2$ bedeutet in der allgemeinen Formel II Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes $C_2$-$C_{24}$-Alkylcarbonyl-,

Als Alkylreste seien substituiert oder unsubstituiert, gesättigt oder ungesättigte $C_2$-$C_{24}$-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- or n-Tetracosanylcarbonyl- genannt, die ein oder mehrere Doppelbindungen enthalten. Gesättigte oder ungesättigte $C_{10}$-$C_{22}$-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-., die ein oder mehrere Doppelbindungen enthalten, sind bevorzugt. Besonders bevorzugt sind gesättigte und/oder ungesättigte $C_{10}$-$C_{22}$-Alkylcarbonylreste wie $C_{10}$-Alkylcarbonyl-, $C_{11}$-Alkylcarbonyl-, $C_{12}$-Alkylcarbonyl-, $C_{13}$-Alkylcarbonyl-, $C_{14}$-Alkylcarbonyl-, $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Ganz besonders bevorzugt sind gesättigte oder ungesättigte $C_{16}$-$C_{22}$-Alkylcarbonylreste wie $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt vier, fünf oder sechs Doppelbindungen, ganz besonders bevorzugt fünf oder sechs. Alle genannten Reste leiten sich von den entsprechenden Fettsäuren ab.

$R^3$ bedeutet in der allgemeinen Formel II Wasserstoff-, gesättigtes oder ungesättigtes $C_2$-$C_{24}$-Alkylcarbonyl.

Als Alkylreste seien substituiert oder unsubstituiert, gesättigt oder ungesättigte $C_2$-$C_{24}$-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- or n-Tetracosanylcarbonyl- genannt, die ein oder mehrere Doppelbindungen enthalten. Gesättigte oder ungesättigte $C_{10}$-$C_{22}$-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-, die ein oder mehrere Doppelbindungen enthalten, sind bevorzugt. Besonders bevorzugt sind gesättigte und/oder ungesättigte $C_{10}$-$C_{22}$-Alkylcarbonylreste wie $C_{10}$-Alkylcarbonyl-, $C_{11}$-Alkylcarbonyl-, $C_{12}$-Alkylcarbonyl-, $C_{13}$-Alkylcarbonyl-, $C_{14}$-Alkylcarbonyl-, $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Ganz besonders bevorzugt sind gesättigte oder unge sättigte $C_{16}$-$C_{22}$-Alkylcarbonylreste wie $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen

in der Fettsäurekette enthalten bis zu sechs Doppelbindungen, vorteilhaft drei, vier, fünf oder sechs Doppelbindungen, besonders bevorzugt vier, fünf oder sechs Doppelbindungen, ganz besonders bevorzugt fünf oder sechs.. Alle genannten Reste leiten sich von den entsprechenden Fettsäuren ab.

Die oben genannten Reste von $R^1$, $R^2$ and $R^3$ können mit Hydroxyl- und/oder Epoxygruppen substituierte sein und/oder können Dreifachbindungen enthalten.

Vorteilhaft werden die im erfindungsgemäßen Verfahren ungesättigte C18. C20-, oder C22-Alkylcarbonylsäuren mit mindestens drei, vier, fünf oder sechs Doppelbindungen, mit einem Gehalt von mindestens 30 Gew-% bezogen auf die gesamten Fettsäuren im Samen der Pflanze hergestellt.

Die in dem hierin beschriebenen Verfahren hergestellten mehrfach ungesättigten Fettsäuren mindestens zwei vorteilhaft drei, vier, fünf oder sechs Doppelbindungen. Besonders vorteilhaft enthalten die Fettsäuren vier fünf oder sechs Doppelbindungen. Im Verfahren hergestellte Fettsäuren haben vorteilhaft 18-, 20- oder 22-C-Atome in der Fettsäurekette, bevorzugt enthalten die Fettsäuren 20 oder 22 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen. Bei den im erfindungsgemäßen Verfahren verwendeten Nukleinsäuresequenzen handelt es sich um isolierte Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- und/oder Δ-4-Desaturaseaktivität codieren.

Vorteilhaft werden im erfindungsgemäßen Verfahren Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität codieren, verwendet ausgewählt aus der Gruppe bestehend aus:

a) einer Nukleinsäuresequenz mit der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellten Sequenz, oder

b) Nukleinsäuresequenzen, die sich als Ergebnis des degenerierten genetischen Codes von den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellten Aminosäuresequenzen ableiten lassen, oder

c) Derivate der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellten Nukleinsäuresequenz, die für Polypeptide mit mindestens 40 % Identität auf Aminosäureebene mit SEQ ID NO: 2, SEQ ID NO: 4, SEQ ID NO:6, SEQ ID NO: 8,

SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 codieren und eine Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität aufweisen.

Vorteilhaft bedeuten die Substituenten $R^2$ oder $R^3$ in den allgemeinen Formeln I und II unabhängig voneinander gesättigtes oder ungesättigtes $C_{18}$-$C_{22}$-Alkylcarbonyl-, besonders vorteilhaft bedeuten sie unabhängig voneinander ungesättigtes $C_{18}$-, $C_{20}$- oder $C_{22}$-Alkylcarbonyl- mit mindestens zwei Doppelbindungen, vorteilhaft mit mindestens drei, vier, fünf oder sechs Doppelbindungen, besonders vorteilhaft mit mindestens vier, fünf oder sechs Doppelbindungen.

Eine bevorzugte Ausführungsform des Verfahrens ist dadurch gekennzeichnet, dass eine Nukleinsäuresequenz zusätzlich in die transgene Pflanze eingebracht wird, die für Polypeptide mit ω-3-Desaturase-Aktivität codiert, ausgewählt aus der Gruppe bestehend aus:

a) einer Nukleinsäuresequenz mit der in SEQ ID NO: 87 oder SEQ ID NO: 105 dargestellten Sequenz, oder

b) Nukleinsäuresequenzen, die sich als Ergebnis des degenerierten genetischen Codes von der in SEQ ID NO: 88 oder SEQ ID NO: 106 dargestellten Aminosäuresequenz ableiten lassen, oder

c) Derivate der in SEQ ID NO: 87 oder SEQ ID NO: 105 dargestellten Nukleinsäuresequenz, die für Polypeptide mit mindestens 60 % Identität auf Aminosäureebene mit SEQ ID NO: 88 oder SEQ ID NO: 106 codieren und eine ω3-Desaturaseaktivität aufweisen.

In einer weiteren bevorzugten Ausführungsform ist das Verfahren dadurch gekennzeichnet, dass eine Nukleinsäuresequenz zusätzlich in die transgene Pflanze eingebracht wird, die für Polypeptide mit Δ-12-Desaturaseaktivität codiert, ausgewählt aus der Gruppe bestehend aus:

a) einer Nukleinsäuresequenz mit der in SEQ ID NO: 107, SEQ ID NO: 109 oder SEQ ID NO: 195 dargestellten Sequenz, oder

b) Nukleinsäuresequenzen, die sich als Ergebnis des degenerierten genetischen Codes von der in SEQ ID NO: 108, SEQ ID NO: 110 oder SEQ ID NO: 196 dargestellten Aminosäuresequenz ableiten lassen, oder

c) Derivate der in SEQ ID NO: 107, SEQ ID NO: 109 oder SEQ ID NO: 195 dargestellten Nukleinsäuresequenz, die für Polypeptide mit mindestens 60 % Identität auf Aminosäureebene mit SEQ ID NO: 108, SEQ ID NO: 110 oder SEQ ID NO: 196 codieren und eine Δ-12-Desaturaseaktivität aufweisen.

Diese vorgenannten Δ-12-Desaturasesequenzen können allein oder in Kombination mit den ω3-Desaturasesequenzen mit den im Verfahren verwendeten Nukleinsäuresequenzen, die für Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen codieren verwendet werden.

Tabelle 1 gibt die Nukleinsäuresequenzen, den Herkunftsorganismus und die Sequenz-ID-Nummer wieder.

| Nr. | Organismus | Aktivität | Sequenznummer |
|---|---|---|---|
| 1. | Euglena gracilis | Δ-8-Desaturase | SEQ ID NO: 1 |
| 2. | Isochrysis galbana | Δ-9-Elongase | SEQ ID NO: 3 |
| 3. | Phaeodactylum tricornutum | Δ-5-Desaturase | SEQ ID NO: 5 |
| 4. | Ceratodon purpureus | Δ-5-Desaturase | SEQ ID NO: 7 |
| 5. | Physcomitrella patens | Δ-5-Desaturase | SEQ ID NO: 9 |

(fortgesetzt)

| Nr. | Organismus | Aktivität | Sequenznummer |
|---|---|---|---|
| 6. | Thraustrochytrium sp. | Δ-5-Desaturase | SEQ ID NO: 11 |
| 7. | Mortierella alpina | Δ-5-Desaturase | SEQ ID NO: 13 |
| 8. | Caenorhabditis elegans | Δ-5-Desaturase | SEQ ID NO: 15 |
| 9. | Borago officinalis | Δ-6-Desaturase | SEQ ID NO: 17 |
| 10. | Ceratodon purpureus | Δ-6-Desaturase | SEQ ID NO: 19 |
| 11. | Phaeodactylum tricornutum | Δ-6-Desaturase | SEQ ID NO: 21 |
| 12. | Physcomitrella patens | Δ-6-Desaturase | SEQ ID NO: 23 |
| 13. | Caenorhabditis elegans | Δ-6-Desaturase | SEQ ID NO: 25 |
| 14. | Physcomitrella patens | Δ-6-Elongase | SEQ ID NO: 27 |
| 15. | Thraustrochytrium sp. | Δ-6-Elongase | SEQ ID NO: 29 |
| 16. | Phytophtora infestans | Δ-6-Elongase | SEQ ID NO: 31 |
| 17. | Mortierella alpina | Δ-6-Elongase | SEQ ID NO: 33 |
| 18. | Mortierella alpina | Δ-6-Elongase | SEQ ID NO: 35 |
| 19. | Caenorhabditis elegans | Δ-6-Elongase | SEQ ID NO: 37 |
| 20. | Euglena gracilis | Δ-4-Desaturase | SEQ ID NO: 39 |
| 21. | Thraustrochytrium sp. | Δ-4-Desaturase | SEQ ID NO: 41 |
| 22. | Thalassiosira pseudonana | Δ-5-Elongase | SEQ ID NO: 43 |
| 23. | Thalassiosira pseudonana | Δ-6-Elongase | SEQ ID NO: 45 |
| 24. | Crypthecodinium cohnii | Δ-5-Elongase | SEQ ID NO: 47 |
| 25. | Crypthecodinium cohnii | Δ-5-Elongase | SEQ ID NO: 49 |
| 26. | Oncorhynchus mykiss | Δ-5-Elongase | SEQ ID NO: 51 |
| 27. | Oncorhynchus mykiss | Δ-5-Elongase | SEQ ID NO: 53 |
| 28. | Thalassiosira pseudonana | Δ-5-Elongase | SEQ ID NO: 59 |
| 29. | Thalassiosira pseudonana | Δ-5-Elongase | SEQ ID NO: 61 |
| 30. | Thalassiosira pseudonana | Δ-5-Elongase | SEQ ID NO: 63 |
| 31. | Thraustrochytrium aureum | Δ-5-Elongase | SEQ ID NO: 65 |
| 32. | Ostreococcus tauri | Δ-5-Elongase | SEQ ID NO: 67 |
| 33. | Ostreococcus tauri | Δ-6-Elongase | SEQ ID NO: 69 |
| 34. | Primula farinosa | Δ-6-Desaturase | SEQ ID NO: 71 |
| 35. | Primula vialii | Δ-6-Desaturase | SEQ ID NO: 73 |
| 36. | Ostreococcus tauri | Δ-5-Elongase | SEQ ID NO: 75 |
| 37. | Ostreococcus tauri | Δ-5-Elongase | SEQ ID NO: 77 |
| 38. | Ostreococcus tauri | Δ-5-Elongase | SEQ ID NO: 79 |
| 39. | Ostreococcus tauri | Δ-6-Elongase | SEQ ID NO: 81 |
| 40. | Thraustrochytrium sp. | Δ-5-Elongase | SEQ ID NO: 83 |
| 41. | Thalassiosira pseudonana | Δ-5-Elongase | SEQ ID NO: 85 |
| 42. | Phytophtora infestans | ω-3-Desaturase | SEQ ID NO: 87 |
| 43. | Ostreococcus tauri | Δ-6-Desaturase | SEQ ID NO: 89 |

(fortgesetzt)

| Nr. | Organismus | Aktivität | Sequenznummer |
|---|---|---|---|
| 44. | Ostreococcus tauri | Δ-5-Desaturase | SEQ ID NO: 91 |
| 45. | Ostreococcus tauri | Δ-5-Desaturase | SEQ ID NO: 93 |
| 46. | Ostreococcus tauri | Δ-4-Desaturase | SEQ ID NO: 95 |
| 47. | Thalassiosira pseudonana | Δ-6-Desaturase | SEQ ID NO: 97 |
| 48. | Thalassiosira pseudonana | Δ-5-Desaturase | SEQ ID NO: 99 |
| 49. | Thalassiosira pseudonana | Δ-5-Desaturase | SEQ ID NO: 101 |
| 50. | Thalassiosira pseudonana | Δ-4-Desaturase | SEQ ID NO: 103 |
| 51. | Thalassiosira pseudonana | ω-3-Desaturase | SEQ ID NO: 105 |
| 52. | Ostreococcus tauri | Δ-12-Desaturase | SEQ ID NO: 107 |
| 53. | Thalassiosira pseudonana | Δ-12-Desaturase | SEQ ID NO: 109 |
| 54. | Ostreococcus tauri | Δ-6-Elongase | SEQ ID NO: 111 |
| 55. | Ostreococcus tauri | Δ-5-Elongase | SEQ ID NO: 113 |
| 56. | Xenopus laevis (BC044967) | Δ-5-Elongase | SEQ ID NO: 117 |
| 57. | Ciona intestinalis (AK112719) | Δ-5-Elongase | SEQ ID NO: 119 |
| 58. | Euglena gracilis | Δ-5-Elongase | SEQ ID NO: 131 |
| 59. | Euglena gracilis | Δ-5-Elongase | SEQ ID NO: 133 |
| 60. | Arabidopsis thaliana | Δ-5-Elongase | SEQ ID NO: 135 |
| 61. | Arabidopsis thaliana | Δ-5-Elongase | SEQ ID NO: 137 |
| 62. | Phaeodactylum tricornutum | Δ-6-Elongase | SEQ ID NO: 183 |
| 63. | Phytium irregulare | Δ-6-Desaturase | SEQ ID NO: 193 |
| 64. | Calendula officinalis | Δ-12-Desaturase | SEQ ID NO: 195 |
| 65. | Ostreococcus tauri | Δ-5-Elongase | SEQ ID NO: 197 |
| 66. | Ostreococcus tauri | Δ-6-Elongase | SEQ ID NO: 199 |
| 67. | Ostreococcus tauri | Δ-6-Desaturase | SEQ ID NO: 201 |

Beschrieben wird auch ein Verfahren zur Herstellung großer Mengen von mehrfach ungesättigten Fettsäuren, speziell ARA und EPA, in einer transgenen Pflanze zu entwickeln. Dieses Verfahren ist ebenfalls zur Herstellung von DHA geeignet. So lassen sich im Verfahren ARA, EPA, DHA oder deren Mischungen hergestellen.

Ebenfalls beschrieben wird ist ein Verfahren zur Herstellung von Verbindungen der allgemeinen Formel I

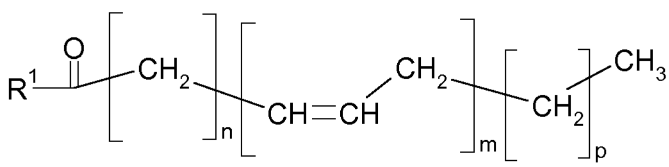

(I)

in transgenen Pflanzen gelöst, wobei das Verfahren umfasst:

a) Einbringen mindestens einer Nukleinsäuresequenz in eine Pflanze, welche für ein Polypeptid mit der Aktivität einer Δ-6-Desaturase-Aktivität kodiert, und ausgewählt ist aus der Gruppe bestehend aus:

i) einer Nukleinsäuresequenz mit der in SEQ ID NO: 193 oder SEQ ID NO: 201 dargestellten Sequenz,
ii) Nukleinsäuresequenzen, die für die in SEQ ID NO: 194 oder SEQ ID NO: 202 angegebene Aminosäuresequenz kodieren,

iii) Nukleinsäuresequenzen, die mit dem komplementären Strang der in SEQ ID NO: 193 oder SEQ ID NO: 201 angegebenen Nukleinsäuresequenz unter stringenten Bedingungen hybridisieren, und

iv) Nukleinsäuresequenzen, die zu der in SEQ ID NO: 193 oder SEQ ID NO: 201 angegebenen Sequenz zu mindestens 60% identisch sind, und

b) Einbringen mindestens einer Nukleinsäuresequenz in eine Pflanze, welche für ein Polypeptid mit einer Δ-6-Elongase-Aktivität kodiert, und ausgewählt ist aus der Gruppe bestehend aus:

i) einer Nukleinsäuresequenz mit der in SEQ ID NO: 27 oder SEQ ID NO: 199 dargestellten Sequenz,

ii) Nukleinsäuresequenzen, die für die in SEQ ID NO: 28 oder SEQ ID NO: 200 angegebene Aminosäuresequenz kodieren,

iii) Nukleinsäuresequenzen, die mit dem komplementären Strang der in SEQ ID NO: 27 oder SEQ ID NO: 199 angegebenen Nukleinsäuresequenz unter stringenten Bedingungen hybridisieren, und

iv) Nukleinsäuresequenzen, die zu der in SEQ ID NO: 27 oder SEQ ID NO: 199 angegebenen Sequenz zu mindestens 60% identisch sind,

c) Einbringen mindestens einer Nukleinsäuresequenz in eine Pflanze, welche für ein Polypeptid mit einer Δ-5-Desaturase-Aktivität kodiert, und ausgewählt ist aus der Gruppe bestehend aus:

i) einer Nukleinsäuresequenz mit der in SEQ ID NO: 11 dargestellten Sequenz,

ii) Nukleinsäuresequenzen, die für die in SEQ ID NO: 12 angegebene Aminosäuresequenz kodieren,

iii) Nukleinsäuresequenzen, die mit dem komplementären Strang der in SEQ ID NO: 11 angegebenen Nukleinsäuresequenz unter stringenten Bedingungen hybridisieren, und

iv) Nukleinsäuresequenzen, die zu der in SEQ ID NO: 11 angegebenen Sequenz zu mindestens 60% identisch sind,

wobei die Variablen und Substituenten in der Formel I die oben genannte Bedeutung haben.

Die im erfindungsgemäßen Verfahren verwendbaren Nukleinsäuresequenzen sind beschrieben in WO 02/26946 (Δ-5-Desaturase aus Thraustochytrium ssp., SEQ ID NO: 11 und Δ-6-Desaturase aus Phytium irregulare, SEQ ID NO: 193) sowie in WO 01/59128 (Δ-6-Elongase aus Physcomitrella patens, SEQ ID NO: 27), auf die hier ausdrücklich Bezug genommen wird. Allerdings wurde in diesen Fällen die Bildung von ARA und EPA entweder nicht in transgenen Pflanzen, sondern lediglich in Mikroorganismen untersucht, oder es konnte keine Steigerung der ARA- und EPA-Synthese in den transgenen Pflanzen nachgewiesen werden. Darüber hinaus wurden in diesen Anmeldungen die erfindungsgemäßen Nukleinsäuren nicht mit Nukleinsäuren, die für andere Enzyme des Fettsäuresynthesewegs kodieren, kombiniert.

Es wurde nun überraschend gefunden, dass die Co-Expression der Nukleinsäuren mit den in SEQ ID NO: 11, 27, 193, 199 und 201 angegebenen Sequenzen in transgenen Pflanzen zu einer starken Erhöhung des ARA-Gehalts auf bis zu mehr als 8%, vorteilhaft bis zu mehr als 10%, 11%, 12%, 13%, 14%, 15%, 16%, 17%, 18%, 19% oder 20%, besonders vorteilhaft auf mehr als 21 %, 22%, 23%, 24% oder 25%, bezogen auf den gesamten Lipidgehalt der Pflanze, führt (vgl. Tabelle 2, Tabelle 3, Tabelle 4 und Figur 31). Bei den vorgenannten Prozentwerten handelt es sich um Gewichtsprozentangaben.

Zur weiteren Steigerung der Ausbeute im beschriebenen Verfahren zur Herstellung von Ölen und/oder Triglyceriden mit einem vorteilhaft gegenüber Ölen und/oder Triglyceriden aus Wildtyp-Pflanzen erhöhten Gehalt an mehrfach ungesättigten Fettsäuren, vor allem von ARA, EPA oder DHA oder deren Mischungen, kann es vorteilhaft sein, die Menge des Ausgangsstoffs für die Fettsäuresynthese zu steigern. Dies kann beispielsweise durch das Einbringen einer Nukleinsäure, die für ein Polypeptid mit der Aktivität einer Δ-12-Desaturase kodiert, und deren Co-Expression in dem Organismus erreicht werden.

Dies ist besonders vorteilhaft in Öl-produzierenden Organismen wie der Familie der Brassicaceae wie der Gattung Brassica, z.B. Raps, Rübsen oder Sareptasenf; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max,* die einen hohen Ölsäuregehalt, aber nur einen geringen Gehalt an Linolsäure aufweisen (Mikoklajczak et al., Journal of the American Oil Chemical Society, 38, 1961, 678 - 681).

Daher wird in einer bevorzugten Ausführungsform der vorliegenden Erfindung eine Nukleinsäuresequenz in die transgene Pflanze eingebracht, die für ein Polypeptid mit Δ-12-Desaturaseaktivität kodiert.

Besonders bevorzugt ist diese Nukleinsäuresequenz ausgewählt aus der Gruppe bestehend aus:

a) einer Nukleinsäuresequenz mit der in SEQ ID NO: 195 dargestellten Sequenz,

b) Nukleinsäuresequenzen, die für die in SEQ ID NO: 196 dargestellte Aminosäuresequenz kodieren,

c) Nukleinsäuresequenzen, die mit dem komplementären Strang der in SEQ ID NO: 195 angegebenen Nukleinsäuresequenz unter stringenten Bedingungen hybridisieren, und

d) Nukleinsäuresequenzen, die zu der in SEQ ID NO: 195 angegebenen Sequenz zu mindestens 60% identisch sind.

Die Nukleinsäuresequenz mit der SEQ ID NO: 195 stammt aus Calendula officinalis und ist beschrieben in WO 01/85968, deren Offenbarung hier ebenfalls durch Bezugnahme in die vorliegende Anmeldung mit aufgenommen ist.

Vorteilhaft setzen die im erfingungsgemäßen Verfahren verwendeten Δ-12-Desaturasen Ölsaure (C18:$1^{\Delta 9}$) zu Linolsäure (C18:$2^{\Delta 9,12}$) oder C18:$2^{\Delta 6,9}$ zu C18:$3^{\Delta 6,9,12}$ (Gammalinolensäure = GLA), den Ausgangssubstanzen für die Synthese von ARA, EPA und DHA um. Vorteilhaft setzen die verwendeten Δ-12-Desaturasen Fettsäuren gebunden an Phospholipide oder CoA-Fettsäureester, vorteilhaft gebunden an CoA-Fettsäureester, um. Dies führt, wenn vorher ein Elongationsschritt stattgefunden hat, vorteilhaft zu höheren Ausbeuten an Syntheseprodukten, da die Elongation in der Regel an CoA-Fettsäureestern erfolgt, während die Desaturierung überwiegend an den Phospholipiden oder an den Triglyceriden erfolgt. Ein Ausstausch, der eine weitere möglicherweise limitierende Enzymreaktion erfoderlich machen würde, zwischen den CoA-Fettsäureestern und den Phospholipiden oder Triglyceriden ist somit nicht erforderlich.

Die zusätzliche Expression der Δ-12-Desaturase in den transgenen Pflanzen führt zu einer weiteren Steigerung des ARA-Gehalts auf bis zu mehr als 10%, 11%, 12%, 13%, 14%, 15%, 16%, 17%, 18%, 19% oder 20%, besonders vorteilhaft auf mehr als 21 %, 22%, 23%, 24% oder 25%, bezogen auf den gesamten Lipidgehalt der Pflanze (vgl. Tabelle 3 und 4 und Figur 32). Bei den vorgenannten Prozentwerten handelt es sich um Gewichtsprozentangaben.

Vorteilhaft können im erfindungsgemäßen Verfahren weitere Nukleinsäuresequenzen in die Pflanzen eingebracht werden, die für ein Polypeptid mit einer Δ-5-Elongase-Aktivität kodieren.

Bevorzugt werden derartige Nukleinsäuresequenzen, die für Δ-5-Elongaseaktivität kodieren, ausgewählt ist aus der Gruppe bestehend aus:

a) einer Nukleinsäuresequenz mit der in SEQ ID NO: 43, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 197 dargestellten Sequenz,

b) Nukleinsäuresequenzen, die für die in SEQ ID NO: 44, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 76, SEQ ID NO: 78, SEQ ID NO: 80, SEQ ID NO: 81, SEQ ID NO: 86, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 198 angegebene Aminosäuresequenz kodieren,

c) Nukleinsäuresequenzen, die mit dem komplementären Strang der in SEQ ID NO: 43, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 197 angegebenen Nukleinsäuresequenz unter stringenten Bedingungen hybridisieren, und

d) Nukleinsäuresequenzen, die zu der in SEQ ID NO: 43, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 197 angegebenen Sequenz zu mindestens 60% identisch sind.

In einer bevorzugten Ausführungsform des Verfahrens werden die Δ-5-Elongase-Gene unter der Kontrolle eines samenspezifischen Promotors exprimiert.

In einer weiteren vorteilhaften Ausführungsform des Verfahrens werden alle Nukleinsäuresequenzen auf einem gemeinsamen rekombinanten Nukleinsäuremolekül in die Pflanzen eingebracht werden, wobei jede Nukleinsäuresequenz unter Kontrolle eines eigenen Promotors steht kann und es sich bei diesem eigenen Promotor um einen samenspezifischen Promotor handelt kann.

Die Erfindung kann aber nicht nur mit den im Sequenzprotokoll angegebenen Nukleinsäuren erfolgreich umgesetzt werden, vielmehr können auch von diesen Sequenzen bis zu einem gewissen Grad abweichende Sequenzen, die für Proteine mit der im Wesentlichen gleichen enzymatischen Aktivität kodieren, eingesetzt werden. Hierbei handelt es sich um Nukleinsäuren, die zu den im Sequenzprotokoll spezifizierten Sequenzen einen bestimmten Identitäts- oder

Homologiegrad aufweisen. Unter im wesentlichen gleiche enzymatische Aktivität sind Proteine zu verstehen, die mindestens 20%, 30%, 40%, 50% oder 60%, vorteilhaft mindestens 70%, 80%, 90% oder 95%, besonders vorteilhaft mindestens 96%, 97%, 98% oder 99% der enzymatischen Aktivität der Wildtyp-Enzyme aufweisen.

Zur Bestimmung der prozentualen Homologie (= Identität) von zwei Aminosäuresequenzen oder von zwei Nukleinsäuren werden die Sequenzen untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an Positionen, die den Sequenzen gemeinsam sind (d.h. % Homologie = Anzahl der identischen Positionen/Gesamtanzahl der Positionen x 100). Die Begriffe Homologie und Identität sind damit als synonym anzusehen.

Die Homologie wurde über den gesamten Aminosäure- bzw. Nukleinsäuresequenzbereich berechnet. Für den Vergleich verschiedener Sequenzen stehen dem Fachmann eine Reihe von Programmen, die auf verschiedenen Algorithmen beruhen, zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet.

Der Fachmann erkennt, dass innerhalb einer Population DNA-Sequenzpolymorphismen, die zu Änderungen der Aminosäuresequenz der SEQ ID NO: 12, 28, 194, 196, 198, 200 und/oder 202 führen, auftreten können. Diese natürlichen Varianten bewirken üblicherweise eine Varianz von 1 bis 5 % in der Nukleotidsequenz des Δ-12-Desaturase-, Δ-6-Desaturase-, Δ-5-Desaturase-, Δ-5-Elongase- und/oder Δ-6-Elongase-Gens. Sämtliche und alle dieser Nukleotidvariationen und daraus resultierende Aminosäurepolymorphismen in der Δ-12-Desaturase, Δ-6-Desaturase, Δ-5-Desaturase, Δ-5-Elongase und/oder Δ-6-Elongase, die das Ergebnis natürlicher Variation sind und die die enzymatische Aktivität nicht wesentlich verändern, sollen im Umfang der Erfindung enthalten sein.

Unter wesentlicher enzymatischer Aktivität der im erfindungsgemäßen Verfahren verwendeten Δ-12-Desaturase-, Δ-6-Desaturase, Δ-5-Elongase, Δ-6-Elongase oder Δ-5-Desaturase ist zu verstehen, dass sie gegenüber den durch die Sequenz und deren Derivate kodierten Proteinen/Enzymen im Vergleich noch eine enzymatische Aktivität von mindestens 10 %, bevorzugt von mindestens 20 %, besonders bevorzugt von mindestens 30 %, 40 %, 50 % oder mind. 60 % und am meisten bevorzugt von mindestens 70 %, 80 %, 90 %, 95 %, 96 %, 97 %, 98 % oder 99 % aufweisen und damit am Stoffwechsel von Verbindungen, die zum Aufbau von Fettsäuren, Fettsäureestern wie Diacylglyceriden und/oder Triacylglyceriden in einer Pflanze oder Pflanzenzelle benötigt werden oder am Transport von Molekülen über Membranen teilnehmen können, wobei $C_{18}$-, $C_{20}$- oder $C_{22}$-Kohlenstoffketten im Fettsäuremolekül mit Doppelbindungen an mindestens zwei, vorteilhaft drei, vier oder fünf Stellen gemeint sind.

Ebenfalls im Umfang der Erfindung enthalten sind Nukleinsäuremoleküle, die unter stringenten Bedingungen mit dem komplementären Strang der hier verwendeten Δ-12-Desaturase-, Δ-6-Desaturase-, Δ-5-Desaturase-, Δ-5-Elongase- und/oder Δ-6-Elongase-Nukleinsäuren hybridisieren. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, 70 %, 80 % oder 90 %, bevorzugt mindestens etwa 91 %, 92 %, 93 %, 94 % oder 95 % und besonders bevorzugt mindestens etwa 96 %, 97 %, 98 %, 99 % oder stärker zueinander homolog sind, gewöhnlich aneinander hybridisiert bleiben. Diese stringenten Bedingungen sind dem Fachmann bekannt und z.B. in Current Protocols in Molecular Biology, John Wiley & Sons, N. Y. (1989), 6.3.1-6.3.6, beschrieben.

Ein bevorzugtes, nicht einschränkendes Beispiel für stringente Hybridisierungsbedingungen sind Hybridisierungen in 6 x Natriumchlorid/Natriumcitrat (sodium chloride/sodium citrate = SSC) bei etwa 45°C, gefolgt von einem oder mehreren Waschschritten in 0,2 x SSC, 0,1 % SDS bei 50 bis 65°C. Dem Fachmann ist bekannt, dass sich diese Hybridisierungsbedingungen je nach dem Typ der Nukleinsäure und, wenn beispielsweise organische Lösungsmittel vorliegen, hinsichtlich der Temperatur und der Konzentration des Puffers unterscheiden. Die Hybridisierungstemperatur liegt beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel, zum Beispiel 50 % Formamid, im obengenannten Puffer vorliegt, beträgt die Temperatur unter Standardbedingungen etwa 42°C. Vorzugsweise sind die Hybridisierungsbedingungen für DNA:DNA-Hybride zum Beispiel 0,1 x SSC und 20°C

bis 45°C, vorzugsweise 30°C bis 45°C. Vorzugsweise sind die Hybridisierungsbedingungen für DNA:RNA-Hybride zum Beispiel 0,1 x SSC und 30°C bis 55°C, vorzugsweise 45°C bis 55°C. Die vorstehend genannten Hybridisierungstemperaturen sind für eine Nukleinsäure mit etwa 100 bp (= Basenpaare) Länge und einem G + C-Gehalt von 50 % in Abwesenheit von Formamid bestimmt. Der Fachmann weiß, wie die für eine bestimmte Nukleinsäure erforderlichen Hybridisierungsbedingungen anhand von Lehrbüchern, wie etwa Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Durch Einbringen einer oder mehrerer Nukleotidsubstitutionen, -additionen oder - deletionen in eine Nukleotidsequenz kann ein isoliertes Nukleinsäuremolekül erzeugt werden, das für eine Δ-12-Desaturase, Δ-6-Desaturase, Δ-5-Desaturase, Δ-5-Elongase und/oder Δ-6-Elongase mit einer oder mehreren Aminosäuresubstitutionen, -additionen oder -deletionen kodiert. Mutationen können in eine der Sequenzen durch Standardtechniken, wie stellenspezifische Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Δ-12-Desaturase, Δ-6-Desaturase, Δ-5-Desaturase, Δ-5-Elongase oder Δ-6-Elongase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der für die Δ-12-Desaturase, Δ-6-Desaturase, Δ-5-Desaturase, Δ-5-Elongase oder Δ-6-Elongase kodierenden Sequenz eingebracht werden, z.B. durch Sättigungsmutagenese, und die resultierenden Mutanten können durch rekombinante Expression nach der hier beschriebenen Δ-12-Desaturase-, Δ-6-Desaturase-, Δ-5-Desaturase-, Δ-5-Elongase- oder Δ-6-Elongase-Aktivität durchmustert werden, um Mutanten zu identifizieren, die die Δ-12-Desaturase-, Δ-6-Desaturase-, Δ-5-Desaturase-, Δ-5-Elongase- oder Δ-6-Elongase-Aktivität beibehalten haben.

In einer Ausfürungsform betrifft die Erfindung auch ein Verfahren zu Herstellung einer Öl-produzierende Pflanze exprimierend unter Kontrolle von Samen-spezifischen Promotoren oder Promotoren aktiv in Pflanzenembryo oder Endosperm

a) eine delta-6 Desaturase,
b) eine delta-6 Elongase,
c) eine delta-5 Desaturase,
d) eine delta-5 Elongase und
e) eine delta-12 Desaturase,

wobei die Pflanze in ihrem Samen unabhängig voneinander ungesättigte C18-, C20-, oder C22-Alkylcarbonylsäuren mit mindestens drei, vier, fünf oder sechs Doppelbindungen, mit einem Gehalt von mindestens 30 Gew-% bezogen auf die gesamten Fettsäuren im Samen der Pflanze herstellt, umfassend das Einbringen der genannten Enzyme in eine Öl-produzierende Pflanze.

Besonders bevorzugt enthalten die Fettsäuren vier, fünf oder sechs Doppelbindungen. Im Verfahren hergestellte Fettsäuren weisen bevorzugt eine Länge von 20C- oder 22C-Atomen auf.

Vorteilhaft werden gesättigte Fettsäuren mit den im Verfahren verwendeten Nukleinsäuren wenig oder gar nicht umgesetzt. Unter wenig ist zu verstehen, dass im Vergleich zu mehrfach ungesättigten Fettsäuren die gesättigten Fettsäuren mit weniger als 5 %, bevorzugt mit weniger als 3 %, besonders bevorzugt mit weniger als 2 %, am meisten bevorzugt mit weniger als 1; 0,5; 0,25 oder 0,125 % der Aktivität umgesetzt werden. Die hergestellten Fettsäuren können das einzige Produkt des Verfahrens darstellen oder in einem Fettsäuregemisch vorliegen.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschieden Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren besonders bevorzugt sind die langkettigen Fettsäuren LCPUFAs von $C_{18}$-, $C_{20}$- und/oder $C_{22}$-Fettsäuren, ganz besonders bevorzugt sind die langkettigen Fettsäuren LCPUFAs von $C_{20}$- und/oder $C_{22}$-Fettsäuren

wie ARA, EPA, DHA oder deren Kombination.

Im erfindungsgemäßen Verfahren werden vorteilhaft Fettsäureester mit mehrfach ungesättigten $C_{18}$-, $C_{20}$- und/oder $C_{22}$-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft von mindestens vier, fünf oder sechs Doppelbindungen im Fettsäureester, ganz besonders vorteilhaft von mindestens fünf oder sechs Doppelbindungen im Fettsäureester hergestellt. Dies führt vorteilhaft zur Synthese von Linolsäure (=LA, C18:$2^{\Delta 9,12}$), γ-Linolensäure (= GLA, C18:$3^{\Delta 6,9,12}$), Stearidonsäure (= SDA, C18:$4^{\Delta 6,9,12,15}$), Dihomo-γ-Linolensäure (= DGLA, 20:$3^{\Delta 8,11,14}$), ω-3-Eicosatetraensäure (= ETA, C20:$4^{\Delta 5,8,11,14}$), Arachidonsäure (ARA, C20:$4^{\Delta 5,8,11,14}$), Eicosapentaensäure (EPA, C20:$5^{\Delta 5,8,11,14,17}$), oder deren Mischungen synthetisiert, bevorzugt werden ω-3-Eicosatetraensäure (= ETA, C20:$4^{\Delta 5,8,11,14}$), Arachidonsäure (ARA, C20:$4^{\Delta 5,8,11,14}$), Eicosapentaensäure (EPA, C20:$5^{\Delta 5,8,11,14,17}$), ω-6-Docosapentaensäure (C22:$5^{\Delta 4,7,10,13,16}$), ω-6-Docosatetraensäure (C22:$4^{\Delta,7,10,13,16}$), ω-3-Docosapentaensäure (= DPA, C22:$5^{\Delta 7,10,13,16,19}$), Docosahexaensäure (= DHA, C22:$6^{\Delta 4,7,10,13,16,19}$) oder deren Mischungen, ganz besonders bevorzugt ARA, EPA und/oder DHA hergestellt. Vorteilhaft werden ω-3-Fettsäuren wie EPA und/oder DHA, bevorzugt DHA hergestellt.

Die Fettsäureester mit mehrfach ungesättigten $C_{18}$-, $C_{20}$- und/oder $C_{22}$-Fettsäuremolekülen vorteilhaft mit mehrfach ungesättigten $C_{20}$- und/oder $C_{22}$-Fettsäuremolekülen können aus den Pflanzen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei, drei, vier, fünf oder sechs, bevorzugt vier, fünf oder sechs, besonders bevorzugt fünf oder sechs Doppelbindungen enthalten, isoliert werden. Vorteilhaft werden sie in der Form ihrer Diacylglyceride, Triacylglyceride und/oder in Form des Phosphatidylcholin isoliert, besonders bevorzugt in der Form der Triacylglyceride isoliert. Neben diesen Estern sind die mehrfach ungesättigten Fettsäuren auch als freie Fettsäuren oder gebunden an andere Verbindungen in den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren bzw. in den erfindungsgemäßen Verfahren (der singular soll im Sinne der Erfindung und der hier dargestellten Offenbarung den plural umfassen und umgekehrt) werden die hergestellten LCPUFAs mit einem Gehalt von mindestens 3, 5, 6, 7 oder 8 Gew.-%, vorteilhaft von mindestens 9, 10, 11, 12, 13, 14 oder 15 Gew.-%, bevorzugt von mindestens 16, 17, 18, 19 oder 20 Gew.-%, besonders bevorzugt von mindestens 21, 22, 23, 24 oder 25 Gew.-%, ganz besonders bevorzugt von mindestens 26, 27, 28, 29 oder 30 Gew.-% bezogen auf die gesamten Fettsäuren in den transgenen Organismen vorteilhaft im Samen der transgenen Pflanzen hergestellt. Dabei werden vorteilhaft $C_{18}$- und/oder $C_{20}$-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie ARA, EPA, DPA oder DHA, um nur einige beispielhaft zu nennen, umgesetzt. Vorteilhaft werden die Fettsäuren in gebundener Form hergestellt.

Vorteilhaft werden dabei im Verfahren mehrfach ungesättigte $C_{20}$-Fettsäuren mit vier oder fünf Doppelbindungen im Molekül mit einem Gehalt von zusammen allen derartigen Fettsäuren von mindestens 15, 16, 17, 18, 19 oder 20 Gew.-%, vorteilhaft zu mindestens 21, 22, 23, 24 oder 25 Gew.-%, besonders vorteilhaft von mindestens 26, 27, 28, 29 oder 30 Gew.-% bezogen auf die gesamten Fettsäuren in den Samen der transgenen Pflanzen hergestellt.

Vorteilhaft werden dabei im Verfahren mehrfach ungesättigte $C_{20}$- und/oder $C_{22}$-Fettsäuren mit vier, fünf oder sechs Doppelbindungen im Molekül mit einem Gehalt von zusammen allen derartigen Fettsäuren von mindestens 15, 16, 17, 18, 19 oder 20 Gew.-%, vorteilhaft zu mindestens 21, 22, 23, 24 oder 25 Gew.-%, besonders vorteilhaft von mindestens 26, 27, 28, 29 oder 30 Gew.-%, ganz besonders vorteilhaft von mindestens 31, 32, 33, 34 oder 35 Gew.-% bezogen auf die gesamten Fettsäuren in den Samen der transgenen Pflanze hergestellt.

Im erfindungsgemäßen Verfahren wird ARA mit einem Gehalt von mindestens 3, 5, 6, 7, 8, 9 oder 10 Gew.-%, vorteilhaft von mindestens 11, 12, 13, 14 oder 15 Gew.-%, bevorzugt von mindestens 16, 17, 18, 19 oder 20 Gew.-%, besonders bevorzugt von mindestens 21, 22, 23, 24 oder 25 Gew.-%, am meisten bevorzugt von mindestens 26 Gew.-%, bezogen auf den gesamten Lipidgehalt in den Samen der transgenen Pflanzen, hergestellt.

EPA wird im erfindungsgemäßen Verfahren mit einem Gehalt von mindestens 0,2; 0,3; 0,4; 0,5; 0,6; 0,7; 0,8; 0,9 oder 1 Gew.-%, vorteilhaft von mindestens 2, 3, 4 oder 5 Gew.-%, bevorzugt von mindestens 6, 7, 8, 9 oder 10 Gew.-%, besonders bevorzugt von mindestens 11, 12, 13, 14 oder 15 Gew.-% und am meisten bevorzugt von mindestens 16 Gew.-%, bezogen auf den gesamten Lipidgehalt in den Samen der transgenen Pflanzen, hergestellt.

DHA wird im erfindungsgemäßen Verfahren mit einem Gehalt von mindestens 0,01 oder 0,02 Gew.-%, vorteilhaft von mindestens 0,03 oder 0,05 Gew.-%, bevorzugt von mindestens 0,09 oder 0,1 Gew.-%, besonders bevorzugt von

mindestens 0,2 oder 0,3 Gew.-% und am meisten bevorzugt von mindestens 0,35 Gew.-%, bezogen auf den gesamten Lipidgehalt in den Samen der transgenen Pflanzen, hergestellt.

Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA als Mischungen vor. Vorteilhaft sollten in den Endprodukten ARA oder DHA nur geringe Mengen, der jeweils anderen Endprodukte vorhanden sein. In einem DHA haltigen Lipid und/oder Öl sollten deshalb weniger als 15, 14, 13, 12 oder 11 Gew.-%, vorteilhaft weniger als10, 9, 8, 7, 6 oder 5 Gew.-%, besonders vorteilhaft weniger als 4, 3, 2 oder 1 Gew.-% EPA und/oder ARA enthalten sein. In einem EPA haltigen Lipid und/oder Öl sollten deshalb weniger als 15, 14, 13, 12 oder 11 Gew.-%, vorteilhaft weniger als10, 9, 8, 7, 6 oder 5 Gew.-%, besonders vorteilhaft weniger als 4, 3, 2 oder 1 Gew.-% ARA enthalten sein. Auch in einem ARA haltigen Lipid und/oder Öl sollten deshalb weniger als 15, 14, 13, 12 oder 11 Gew.-%, vorteilhaft weniger als10, 9, 8, 7, 6 oder 5 Gew.-%, besonders vorteilhaft weniger als 4, 3, 2 oder 1 Gew.-% EPA und/oder DHA enthalten sein.

Es können aber auch Mischungen von verschiedenen mehrfach ungesättigten $C_{20}$- und/oder $C_{22}$-Fettsäuren in einem Produkt wünschenswert sein. In solchen Fällen können DHA haltige Lipide und/oder Öle mindestens 1, 2, 3, 4 oder 5 Gew.-% ARA und/oder EPA, vorteilhaft mindestens 6, 7 oder 8 Gew.-%, besonders vorteilhaft mindestens 9, 10, 11, 12, 13, 14 oder 15 Gew.-%, ganz besonders vorteilhaft mindestens 16, 17, 18, 19, 20, 21, 22, 23, 24 oder 25 Gew.-% bezogen auf den gesamten Lipidgehalt in den Samen der transgenen Pflanzen enthalten.

Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endprodukts betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt. Werden die Verbindungen ARA und EPA gleichzeitig hergestellt, werden sie vorteilhaft in einem Verhältnis von mindestens 1:6 (EPA:ARA), vorteilhaft von mindestens 1:8, bevorzugt von mindestens 1:10, besonders bevorzugt von mindestens 1:12 in der Pflanze hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen.

Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin, keine Clupanodonsäure (= Docosapentaensäure, $C22:5^{\Delta 4,8,12,15,21}$) sowie keine Nisinsäure (Tetracosahexaensäure, $C23:6^{\Delta 3,8,12,15,18,21}$).

Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren, vor allem an ARA

und EPA aber auch DHA, von mindestens 50, 80 oder 100 %, vorteilhaft von mindestens 150, 200 oder 250 %, besonders vorteilhaft von mindestens 300, 400, 500, 600, 700, 800 oder 900 %, ganz besonders vorteilhaft von mindestens 1000, 1100, 1200, 1300, 1400 oder 1500 % gegenüber der nicht transgenen Ausgangspflanze beispielsweise einer Pflanze wie Brassica juncea, Brassica napus, Camelina sativa, Arabidopsis thanliana oder Linum usitatissimum beim Vergleich in der GC-Analyse siehe Beispiele erreicht werden.

Vorteilhaft werden, wie oben beschrieben, die im Verfahren hergestellten mehrfach ungesättigten $C_{20}$- und/oder $C_{22}$-Fettsäuren mit vier, fünf oder sechs Doppelbindungen im Molekül im Samen von Pflanzen, die keine oder nur sehr geringe Mengen an C12:0-bzw. C14:0-Fettsäuren enthalten. Auch noch kürzere gesättigte Fettsäuren wie die Fettsäuren C4:0, C6:0, C8:0 oder C10:0 sollten nicht oder nur in geringen Mengen im Lipid und/oder Öl vorhanden sein. Unter nur sehr geringen Mengen sind vorteilhaft Mengen zu verstehen, die in der GC-Analyse vorteilhaft unter 5, 4, 3, 2 oder 1 %, vorteilhaft unter 0,9; 0,8; 0,7; 0,6 oder 0,5 %, besonders vorteilhaft unter 0,4; 0,3; 0,2 ider 0,1 %, ganz besonders bevorzugt unter 0,09; 0,08; 0,07; 0,06; 0,05; 0,04; 0,03; 0,02 oder 0,01 Flächeneinheiten in der GC liegen. Die Fettsäure C16:0 sollte vorteilhaft in einem Bereich von 1 bis 28 % GC-Flächeneinheiten liegen. Vorteilhaft sollte die Fettsäure C16:0 in GC-Flächeneinheiten von weniger als 25%, 20%, 15% oder 10%, vorteilhaft von weniger als 9%, 8%, 7%, 6% oder 5%, besonders vorteilhaft von weniger als 4%, 3%, 2% oder 1% oder gar nicht in den Lipiden, Ölen und/oder freien Fettsäuren vorhanden sein. Die Fettsäure C16:1 sollte vorteilhaft weniger als 1; 0,5; 0,4; 0,3; 0,2 oder 0,1 %, besonders vorteilhaft 0,09; 0,08; 0,07; 0,06; 0,05; 0,04; 0,03; 0,02 oder 0,01 Flächeneinheiten in der GC betragen. Ganz besonders bevorzugt sollte die Fettsäure C16:1 nicht in den nach dem Verfahren hergestellten Ölen und/oder Lipiden vorhanden sein. Gleiches gilt für die Fettsäuren C15:0, C17:0, C16:1 $^{\Delta3}$trans, C16:4$^{\Delta4,7,10,13}$ und C18:5$^{\Delta3,6,9,12,15}$. Neben Ölsäure (C18:1$^{\Delta9}$) können auch die Isomere (C18:1$^{\Delta7}$, C18:1$^{\Delta11}$) in den Lipiden, Ölen oder freien Fettsäuren vorhanden sein. Vorteilhaft in Mengen, gemessen als GC-Flächeneinheiten, von weniger als 5%, 4%, 3%, 2% oder 1%. Die Fettsäuren C20:0, C20:1, C24:0 und C24:1 sollten jeweils in einem Bereich von 0 bis 1 %, 0 bis 3% bzw. 0 bis 5 % Flächeneinheiten in der GC liegen. Weiterhin sollte in der GC-Analyse wenig Dihomo-$\gamma$-linolensäure (= DGLA) im Samenöl und/oder -lipid in GC-Flächeneinheiten detektierbar sein. Unter wenig sind weniger als 2; 1,9; 1,8; 1,7; 1,6 oder 1,5 %, vorteilhaft weniger als 1,4; 1,3; 1,2; 1,1 oder 1 %, besonders vorteilhaft weniger als 0,9; 0,8; 0,7; 0,6; 0,5 oder 0,4 % in GC-Flächeneinheiten zu verstehen.

In einer bevorzugten Ausführungsform des Verfahrens sollte DGLA und ARA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

In weiteren bevorzugten Ausführungsform des Verfahrens sollte DGLA und EPA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

Vorteilhaft sollten die im erfindungsgemäßen Verfahren hergestellten Lipide und/oder Öle einen hohen Anteil von ungesättigten Fettsäuren vorteilhaft von mehrfach ungesättigten Fettsäuren von mindestens 30, 40 oder 50 Gew.-%, vorteilhaft von mindestens 60, 70 oder 80 Gew.-% bezogen auf den Gesamtfettsäuregehalt in den Samen der transgenen Pflanzen betragen.

Alle gesättigten Fettsäuren zusammen sollten vorteilhaft in den für das erfindungsgemäße Verfahren bevorzugt verwendeten Pflanzen nur einen geringen Anteil ausmachen. Unter geringen Anteil ist in diesem Zusammenhang ein Anteil in GC-Flächeneinheiten von weniger als 15%, 14%, 13%, 12%, 11% oder 10%, bevorzugt von weniger als 9%, 8%, 7% oder 6% zu verstehen.

Weiterhin sollten die im Verfahren vorteilhaft als Wirtspflanzen, die die über verschiedene Methoden eingebrachten im Verfahren verwendeten Gene zur Synthese der mehrfach ungesättigten Fettsäuren enthalten, vorteilhaft einen höheren Ölanteil als Proteinanteil im Samen haben, vorteilhafte Pflanzen haben einen Öl-/Proteingehaltverhältnis von 5 zu 1, 4 zu 1, 3 zu 1, 2 zu 1 oder 1 zu 1. Dabei sollte der Ölgehalt bezogen auf das Gesamtgewicht des Samens in einem Bereich von 15 - 55%, vorteilhaft zwischen 25 - 50%, besonders vorteilhaft zwischen 35 - 50% liegen.

Vorteilhafte im Verfahren verwendete Wirtspflanzen sollten am Triglycerid in sn1-, sn2- und sn3-Position eine Verteilung der ungesättigten Fettsäuren wie Ölsäure, Linolsäure und Linolensäure, die die Ausgangsverbindungen im erfindungsgemäßen Verfahren zur Synthese mehrfach ungesättigter Fettsäuren sind, wie in der folgenden Tabelle 5 dargestellt haben, wobei die Zeilen Nr. 1 - 7 verschiedene vorteilhafte Alternativen derartiger Verteilungen wiedergeben. Die Bezeichnung n.v. bedeutet nicht vorhanden.

Tabelle 5: Pflanzen mit vorteilhafter Fettsäureverteilung in sn1-, sn2- und sn3-Postion am Triglycerid

| Nr. | Ölsäure | | | Linolsäure | | | $\alpha$-Linolensäure | | |
|---|---|---|---|---|---|---|---|---|---|
| | sn1 | sn2 | sn3 | sn1 | sn2 | sn3 | sn1 | sn2 | sn3 |
| 1. | 1 | 1 | 1 | 2 | 4 | 1 | n.v. | n.v. | n.v. |
| 2. | 1,4 | 2,2 | 1 | 2,8 | 9 | 1 | 2 | 6,7 | 1 |

(fortgesetzt)

| Nr. | Ölsäure | | | Linolsäure | | | α-Linolensäure | | |
|---|---|---|---|---|---|---|---|---|---|
| | sn1 | sn2 | sn3 | sn1 | sn2 | sn3 | sn1 | sn2 | sn3 |
| 3. | 0,8 | 0,8 | 1 | 1,1 | 1,6 | 1 | 1 | 0,8 | 1 |
| 4. | 0,9 | 0,9 | 1 | 1,2 | 1,6 | 1 | 0,9 | 1 | 1 |
| 5. | 0,9 | 0,9 | 1 | 1 | 1,3 | 1 | 1 | 1 | 1 |
| 6. | 1 | 1,1 | 1 | 2 | 2,8 | 1 | 1 | 1 | n.v. |
| 7. | 1,3 | 9,7 | 1 | 1 | 9 | Spuren | 1 | n.v. | n.v. |

Die Zeilen geben die Verhältnisse der folgenden Pflanzen wieder: Zeile 1 = Arachis hypogaea, Zeile 2 = Brassica napus, Zeile 3 = Glycine max, Zeile 4 = Linum usitatissimum, Zeile 5 = Zea mays, Zeile 6 = Olea europaea und Zeile 7 = Theobroma cacao.

Für das Verfahren vorteilhafte Wirtspflanzen sind solche, die einen hohen Anteil an Ölsäure, das heißt von mindestens 40, 50, 60 oder 70 Gew.-% bezogen auf den gesamten Fettsäuregehalt der Pflanze haben, im Vergleich zu Linolsäure und/oder Linolensäure in den Lipiden und/oder Ölen besonders im Triglycerid haben wie z.B. Anarcardium occidentale, Argania spinosa, Bombax malabaricum, Brassica napus, Butyrospermum parkii, hoch Ölsäure Distel (Carthamus tinctorius), Citrullus colocythis, Corylus avellana, Curcurbita foetidissima, Curcurbita pepo, Guizotia abyssinica, hoch Ölsäure Sonneblume (Helianthus annus), Macadamia intergrifolia, Nigella sativa, Olea europaea, Papaver somniferium, Passiflora edulis, Persea americana, Prunus amygdalis, Prunus armeniaca, Prunus dulcis, Prunus communis, Sesamum indicum, Simarouba glauca, Thea sasumgua, oder Theobroma cacao. Weitere vorteilhafte Pflanzen haben einen höheren Anteil der ungesättigten Fettsäuren Ölsäure, Linolsäure und α-Linolensäure in sn2-Position im Vergleich zu den anderen Positionen sn1 und sn3. Unter höheren Anteil sind Verhältnisse von (sn1:sn2:sn3) 1:1,1:1; 1:1,5:1 bis 1:3:1 zu verstehen. Vorteilhafte Pflanzen wie Actinidia chinensis, Aleurites moluccana, Arnebia griffithii, Brassica alba, Brassica hirta, Brassica nigra, Brassica juncea, Brassica carinata, Camelina sativa, Cannabis sativa, Echium rubrum, Echium vulgare, Humulus lupulus, Juglans regia, Linum usitatissimum, Ocimum spp., Perilla frutescens, Portulaca oleracea, Prunus cerasus, Salicornia bigelovii, Salvia hispanica sind auch solche die einen hohen Anteil an α-Linolensäure im Lipid und/oder Öl der Pflanze aufweisen, das heißt eine Anteil an α-Linolensäure von mindestens 10, 15 oder 20 Gew.-%, vorteilhaft von mindestens 25, 30, 35, 40, 45 oder 50 Gew.-% bezogen auf den gesamten Fettsäuregehalt der Pflanze aufweisen. Ganz besonders vorteilhafte Pflanzen zeigen für die im Verfahren hergestellte Arachidonsäure, Eicosapentaensäure oder Docosahexaensäure ebenfalls eine Präferenz für die sn2-Position im Triglycerid gegenüber den Positionen sn1 und sn3 von vorteilhaft 1:1,1:1; 1:1,5:1 bis 1:3:1.

Für das Verfahren verwendete Pflanzen sollten vorteilhaft einen Gehalt an Erucasäure von weniger als 2 Gew.-% bezogen auf den Gesamtfettsäuregehalt der Pflanze haben. Auch sollte der Gehalt an gesättigten Fettsäuren C16:0 und/oder C18:0 vorteilhaft geringer als 19, 18, 17, 16, 15, 14, 13, 12, 11, oder 10 Gew.-%, vorteilhaft weniger als 9, 8, 7, 6 oder 5 Gew.-% bezogen auf den gesamten Fettsäuregehalt der Pflanze sein. Vorteilhaft sollten auch längere Fettsäuren wie C20:0 oder C22:1 gar nicht oder in nur geringen Mengen vorteilhaft geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Pflanze in den im Verfahren verwendeten Pflanzen vorhanden sein. Typischerweise ist in den für das erfindungsgemäße Verfahren verwendeten Pflanzen kein oder nur in geringen Mengen C16:1 als Fettsäure enthalten. Unter geringen Mengen sind vorteilhaft Gehalte an Fettsäuren zu verstehen, die geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Pflanze sind.

Aus wirtschaftlichen Gründen, das heißt aufgrund der Anbaufläche und Ölerträge werden Pflanzen bevorzugt, die auf großen Flächen angebaut werden, wie Soja, Raps, Senf, Camelina, Lein, Sonnenblume, Ölpalme, Baumwolle, Sesam, Mais, Olive bevorzugt Raps, Camelina, Lein, Sonnenblume im Verfahren als Wirtspflanze gern genommen.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus den Pflanzen vorteilhaft den Pflanzensamen in bekannter Weise beispielsweise über aufbrechen der Samen wie Mahlen und anschließender Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Pflanzen für das erfindungsgemäße Verfahren kommen prinzipiell alle Pflanzen in Frage, die in der Lage sind Fettsäuren zu synthetisieren wie alle dicotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Compositae, Convolvulaceae, Cruciferae, Cucurbitaceae, Elaeagnaceae, Ericaceae, Euphorbiaceae, Fabaceae, Geraniaceae, Gramineae, Juglandaceae, Lauraceae, Leguminosae, Linaceae, Malvaceae, Moringaceae, Marchantiaceae, Onagraceae, Olacaceae, Oleaceae, Papaveraceae, Piperaceae, Pedaliaceae, Poaceae, Rosaceae oder Solanaceae, vorteilhaft Anacardiaceae, Asteraceae, Boraginaceae, Brassicaceae, Cannabaceae, Compositae, Cruciferae, Cucurbitaceae, Elaeagnaceae, Euphorbiaceae, Fabaceae, Geraniaceae, Gramineae, Leguminosae, Linaceae, Malvaceae, Moringaceae, Marchantiaceae, Onagraceae, Olacaceae, Oleaceae, Papaveraceae, Piperaceae, Pedaliaceae, Poaceae oder Solanaceae. Aber auch Gemüsepflanzen oder Zierpflanzen wie Tagetes kommen für das Verfahren in Betracht.

Beispielhaft seien die folgenden Pflanzen genannt ausgewählt aus der Gruppe: Anacardiaceae wie die Gattungen Pistacia, Mangifera, Anacardium z.B. die Gattung und Arten *Pistacia vera* [Pistazie], *Mangifer indica* [Mango] oder *Anacardium occidentale* [Cashew], Asteraceae wie die Gattungen Artemisia, Calendula, Carthamus, Centaurea, Cichorium, Cynara, Helianthus, Lactuca, Locusta, Tagetes, Valeriana z.B. die Gattung und Arten Artemisia sphaerocephala, Calendula officinalis [Garten-Ringelblume], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana*, *Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Adelocaryum, Alkanna, Anchusa, Borago, Brunnera, Cerinthe, Cynoglossum, Echium, Gastrocatyle, Lithospermum, Moltkia, Nonea, Onosma, Onosmodium, Paracaryum, Pectocarya, Symphytum z.B. die Gattung und Art Adelocaryum coelestinum, Alkanna orientalis, Anchusa anzurea, Anchusa capensis, Anchusa hybrida, *Borago officinalis* [Borretsch], Brunnera orientalis, Cerinthe minor, Cynoglossum amabile, Cynoglossum lanceolatum, Echium rubrum, Echium vulgare, Gastrocatyle hispida, Lithospermum arvense, Lithospermum purpureocaeruleum, Moltkia aurea, Moltkia coerules, Nonea macrosperma, Onosma sericeum, Onosmodium molle, Onosmodium occidentale, Paracaryum caelestinum, Pectocarya platycarpa, Symphytum officinale, Brassicaceae wie die Gattungen Brassica, Camelina, Melanosinapis, Sinapis, Arabadopsis z.B. die Gattungen und Arten *Brassica alba, Brassica carinata, Brassica hirta, Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima*, *Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii,* Cucurbitaceae wie die Gattung Cucubita z.B. die Gattungen und Arten *Cucurbita maxima, Cucurbita mixta, Cucurbita pepo* oder *Cucurbita moscha*ta [Kürbis], Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euphorbiaceae wie die Gattungen Manihot, Janipha, Jatropha, Ricinus z.B. die Gattungen und Arten *Manihot utilissima, Janipha manihot,, Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicago, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max, Dolichos soja, Glycine gracilis, Glycine hispida, Phaseolus max, Soja hispida* oder *Soja max* [Sojabohne], Geraniaceae wie die Gattungen Pelargonium, Cocos, Oleum z.B. die Gattungen und Arten *Cocos nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea*

*gratissima* oder *Persea persea* [Avocado], Leguminosae wie die Gattung Arachis z.B. die Gattung und Art *Arachis hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroana, Marchantia foliacea, Marchantia macropora,* Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis, Oenothera grandiflora* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Rosaceae wie die Gattung Prunus z.B. die Gattung und Art Prunus armeniaca, Prunus amygdalus, Prunus avilum, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Scrophularia, Verbascum z.B. die Gattungen und Arten Scrophularia *marilandica, Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus* [*Königskerze*], Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersicum* [Tomate], Sterculiaceae wie die Gattung Theobroma z.B. die Gattung und Art *Theobroma cacao* [Kakao] oder Theaceae wie die Gattung Camellia z.B. die Gattung und Art *Camellia sinensis* [Tee]. Als weitere Pflanzen seien die Gattung und Art Argania spinosa, Arnebia griffithii, Adansonia digitata, Orbignya martiana, Carum carvi, Bertholletia excelsa, Aleurites moluccana, Hydnocarpus kurzii, Salvia hispanica, Vitis vinifera, Corvlus avellana, Humulus lupus, Hyptis spicigera und Shorea stenoptera genannt.

Vorteilhaft werden im erfindungsgemäßen Verfahren transgene Pflanzen wie zweikeimblättrige oder einkeimblättrige Pflanzen verwendet. Besonders vorteilhaft werden transgene Pflanzen im erfindungsgemäßen Verfahren verwendet, die zu den Öl-produzierenden Pflanzen gehören, das heißt die für die Herstellung von Ölen verwendet werden, wie bevorzugt Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte.

Bevorzugte erfindungsgemäße Pflanzen sind Ölsamen- oder Ölfruchtpflanzen, wie Erdnuss, Raps, Canola, Sonnenblume, Saflor, Mohn, Saeptasenf. Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche

Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein, Sareptasenf, Camelina oder Hanf.

Für die erfindungsgemäßen beschriebenen Verfahren ist es vorteilhaft in die Pflanze zusätzlich zu den unter Verfahrensschritt (a) bis (e) bzw. (a) bis (c) eingebrachten Nukleinsäuren sowie den ggf. eingebrachten Nukleinsäuresequenzen, die für die ω-3-Desaturasen und/oder die für die Δ-12-Desaturasen codieren, zusätzlich weitere Nukleinsäuren einzubringen, die für Enzyme des Fettsäure- oder Lipidstoffwechsels codieren.

Im Prinzip können alle Gene des Fettsäure- oder Lipidstoffwechsels vorteilhaft in Kombination mit der(den) erfinderischen Δ-5-Elongase(n), Δ-6-Elongase(n) und/oder ω-3-Desaturase(n) [im Sinne dieser Anmeldung soll der Plural den Singular und umgekehrt beinhalten] im Verfahren zur Herstellung mehrfach ungesättigter Fettsäuren verwendet werden vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Δ-5-Elongase, Δ-6-Elongase und/oder ω-3-Desaturase verwendet.

Besonders bevorzugt werden Gene ausgewählt aus der Gruppe der Δ-4-Desaturasen, Δ-5-Desaturasen, Δ-6-Desaturasen, Δ-8-Desatuasen, Δ-9-Desaturasen, Δ-12-Desaturasen, Δ-6-Elongasen oder Δ-9-Elongasen in Kombination mit den vorgenannten Genen für die Δ-5-Elongase, Δ-6-Elongase und/oder ω-3-Desaturase verwendet, wobei einzelne Gene oder mehrere Gene in Kombination verwendet werden können.Vorteilhaft werden die vorgenannten Geni in Kombination mit der erfindungsgemäß verwendeten Δ-6-Elongase, Δ-5-Elongase, Δ-5-Desaturase, Δ-6-Desaturase und/oder Δ-12-Desaturase verwendet

Besonders bevorzugt werden Gene ausgewählt aus der Gruppe der Δ-8-Desaturasen, Δ-9-Desaturasen, Δ-5-Elongase oder Δ-9-Elongasen in Kombination mit den vorgenannten Genen verwendet.

Durch die enzymatische Aktivität der im erfindungsgemäßen Verfahren verwendeten Nukleinsäuren, die für Polypeptide mit Δ-6-Elongase-, Δ-6-Desaturase, Δ-5-Desaturase- und/oder Δ-12-Desaturaseaktivität kodieren, vorteilhaft in Kombination mit Nukleinsäuresequenzen, die für Polypeptide des Fettsäure- oder Lipidstoffwechsels wie Polypeptide mit Δ-8-Desaturase- oder Δ-5- oder Δ-9-Elongaseaktivität kodieren, können im erfindungsgemäßen Verfahren unterschiedlichste mehrfach ungesättigte Fettsäuren hergestellt werden. Je nach Auswahl der für das erfindungsgemäße Verfahren verwendeten Pflanzen lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA, oder Fettsäuren, die sich von C18:3-Fettsäuren ableiten, wie SDA, ETA oder EPA. Liegt in der für das Verfahren verwendeten Pflanze als ungesättigte Fettsäure nur Linolsäure (= LA, $C18:2^{\Delta 9,12}$) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, $C18:3^{\Delta 9,12,15}$) vorhanden, wie beispielsweise in Lein, so können als Produkte des Verfahrens nur SDA, ETA oder EPA entstehen, die wie oben beschrieben als freie Fettsäuren oder gebunden vorliegen können.

Durch die Aktivität der Δ-6-Desaturase und Δ-6-Elongase entstehen beispielsweise GLA und DGLA bzw. SDA und ETA, je nach Ausgangspflanze und darin enthaltener ungesättigter Fettsäure. Bevorzugt entstehen DGLA bzw. ETA oder Mischungen daraus. Wird zusätzlich die Δ-5-Desaturase in die Pflanze eingebracht, so entstehen auch ARA und/oder EPA. Werden darüber hinaus noch Gene eingebracht, die für eine Δ-5-Elongase- und/oder Δ-4-Desaturaseaktivität codieren, so lassen sich die Fettsäuren DPA und/oder DHA im erfindungsgemäßen Verfahren herstellen. Vorteilhaft werden nur ARA, EPA und/oder DHA oder eine Mischung davon synthetisiert, abhängig von der in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, bevorzugt weniger als 15 Gew.-%, besonders bevorzugt weniger als 10 Gew.-%, am meisten bevorzugt weniger als 5, 4, 3, 2 oder 1 Gew.-%, bezogen auf die Endprodukte DGLA, ETA oder deren Mischungen bzw. ARA, EPA oder deren Mischungen bzw. ARA, EPA, DHA oder deren Mischungen.

Neben der Produktion der Ausgangsfettsäuren für die erfindungsgemäß verwendeten Enzyme direkt in der Pflanze können die Fettsäuren auch von außen gefüttert werden. Aus Kostengründen ist die Produktion in der Pflanze bevorzugt. Bevorzugte Substrate für die Produktion von ARA sind die Linolsäure ($C18:2^{\Delta 9,12}$), die γ-Linolensäure ($C18:3^{\Delta 6,9,12}$) und die Dihomo-γ-linolensäure ($C20:3^{\Delta 8,11,14}$). Bevorzugte Substrate für die Produktion von EPA sind die Linolensäure ($C18:3^{\Delta 9,12,15}$), die Stearidonsäure ($C18:4^{\Delta 6,9,12,15}$) und die Eicosatetraensäure ($C20:4^{\Delta 8,11,14,17}$). Bevorzugte Substrate für die Produktion von DHA sind die Linolensäure ($C18:3^{\Delta 9,12,15}$), die Stearidonsäure ($C18:4^{\Delta 6,9,12,15}$), die Eicosatetraensäure ($C20:4^{\Delta 8,11,14,17}$), EPA und DPA.

Die erfindungsgemäßen Δ-5-Elongasen haben gegenüber den humanen Elongasen oder Elongasen aus nicht-humanen Tieren wie denen aus Oncorhynchus, Xenopus oder Ciona die vorteilhafte Eigenschaft, dass sie $C_{22}$-Fettsäuren nicht zu den entsprechenden $C_{24}$-Fettsäuren elongieren. Weiterhin setzen sie vorteilhaft keine Fettsäuren mit einer Doppelbindung in Δ-6-Position um, wie sie von den humanen Elongasen oder den Elongasen aus nicht-humanen Tieren umgesetzt werden. Besonders vorteilhafte Δ-5-Elongasen setzen bevorzugt nur ungesättigte $C_{20}$-Fettsäuren um. Diese vorteilhaften Δ-5-Elongasen weisen einige putative Transmembran-Helixes (5 - 7) auf. Vorteilhaft werden nur $C_{20}$-Fettsäuren mit einer Doppelbindung in Δ-5-Position umgesetzt, wobei ω-3-$C_{20}$ Fettsäuren bevorzugt werden (EPA). Weiterhin haben sie in einer bevorzugten Ausführungsform der Erfindung die Eigenschaft, dass sie neben der Δ-5-Elongaseaktivität vorteilhaft keine oder nur eine relativ geringe Δ-6-Elongaseaktivität aufweisen. Im Gegensatz dazu weisen die humanen Elongasen oder nicht-humanen Tier-Elongasen eine annäherend gleiche Aktivität gegenüber Fettsäuren mit einer Δ-6- oder Δ-5-Doppelbindung auf. Diese vorteilhaften Elongasen werden als sogenannte monofunktionelle Elongasen bezeichnet. Die humanen Elongasen oder die nicht-humanen Tierelongasen werden dem gegenüber als multifunktionelle Elongasen bezeichnet, die neben den vorgenannten Substraten auch monoungesättigte $C_{16}$- und $C_{18}$-Fettsäuren beispielsweise mit Δ-9- oder Δ-11-Doppelbindung umsetzen. Vorteilhaft setzen die monofunktionellen Elongasen in einem Hefefütterungstext, in dem als Substrat EPA den Hefen zugesetzt wurde, mindestens 15 Gew.-% des zugesetzten EPAs zu Docosapentaensäure (DPA, C22:5$^{\Delta 7,10,13,16,19}$), vorteilhaft mindestens 20 Gew.-%, besonders vorteilhaft mindestens 25 Gew.-% um. Wird als Substrat γ-Linolensäure (= GLA, C18:3$^{\Delta 6,9,12}$) gegeben, so wird diese vorteilhaft gar nicht elongiert. Ebenfalls wird auch C18:3$^{\Delta 5,9,12}$ nicht elongiert. In einer anderen vorteilhaften Ausführungsform werden weniger als 60 Gew.-% des zugesetzten GLA zu Dihomo-γ-linolensäure (= C20:3$^{\Delta 8,11,14}$) umgesetzt, vorteilhaft weniger als 55 Gew.-%, bevorzugt weniger als 50 Gew.-%, besonders vorteilhaft weniger als 45 Gew.-%, ganz besonders vorteilhaft weniger als 40 Gew.-%. In einer weiteren ganz bevorzugten Ausführungsform der erfindungsgemäßen Δ-5-Elongaseaktivität wird GLA nicht umgesetzt.

Die Figuren 27 und 28 geben die gemessenen Substratspezifitäten der verschiedenen Elongasen wieder. In Figur 27 sind die Spezifitäten der multifunktnonellen Elongasen von Xenopus laevis (Fig. 27 A), Ciona intestinalis (Fig. 27 B) und Oncorhynchus mykiss (Fig. 27 C) wiedergegeben. Alle diese Elongasen setzen ein breites Spektrum an Substraten um. Dies kann im erfindungsgemäßen Verfahren zu Nebenprodukten führen, die durch weitere enzymatische Aktivitäten umgesetzt werden müssen. Diese Enzyme sind deshalb im erfindungsgemäßen Verfahren weniger bevorzugt. Die bevorzugten monofunktionellen Elongasen und ihre Substratspezifität werden in Figur 28 wiedergegeben. Figur 28 A zeigt die Spezifität der Ostreococcus tauri Δ-5-Elongase. Dies setzt nur Fettsäuren mit einer Doppelbindung in Δ-5-Position um. Vorteilhaft werden nur C20-Fettsäuren umgesetzt. Eine ähnlich hohe Substratspezifität weist die Δ-5-Elongase von Thalassiosira pseudonana (Fig. 28. C) auf. Sowohl die Δ-6-Elongase von Ostreococcus tauri (Fig. 28 B) als auch die von Thalassiosira pseudonana (Fig. 28 D) setzen vorteilhaft nur Fettsäuren mit einer Doppelbindung in Δ-6-Position um. Vorteilhaft werden nur C18-Fettsäuren umgesetzt. Auch die Δ-5-Elongasen aus Arabidopsis thaliana und Euglena gracilis zeichnen sich durch ihre Spezifität aus.

Vorteilhafte erfindungsgemäße Δ-6-Elongasen zeichnen sich ebenfalls durch eine hohe Spezifität aus, das heißt bevorzugt werden $C_{18}$-Fettsäuren elongiert. Vorteilhaft setzen sie Fettsäuren mit einer Doppelbindung in Δ-6-Position um. Besonders vorteilhafte Δ-6-Elongasen setzen vorteilhaft $C_{18}$-Fettsäuren mit drei oder vier Doppelbindungen im Molekül um, wobei diese eine Doppelbindung in Δ-6-Position enthalten müssen. Weiterhin haben sie in einer bevorzugten Ausführungsform der Erfindung die Eigenschaft, dass sie neben der Δ-6-Elongaseaktivität vorteilhaft keine oder nur eine relativ geringe Δ-5-Elongaseaktivität aufweisen. Im Gegensatz dazu weisen die humanen Elongasen oder nicht-humanen Tier-Elongasen eine annäherend gleiche Aktivität gegenüber Fettsäuren mit einer Δ-6- oder Δ-5-Doppelbindung auf. Diese vorteilhaften Elongasen werden als sogenannte monofunktionelle Elongasen bezeichnet. Die humanen Elongasen oder die nicht-humanen Tierelongasen werden, wie oben beschrieben, dem gegenüber als multifunktionelle Elongasen bezeichnet, die neben den vorgenannten Substraten auch monoungesättigte $C_{16}$- und $C_{18}$-Fettsäuren beispielsweise mit Δ-9- oder Δ-11-Doppelbindung umsetzen. Vorteilhaft setzen die monofunktionellen Elongasen in einem Hefefütterungstext, in dem als Substrat EPA den Hefen zugesetzt wurde, mindestens 10 Gew.-% der zugesetzten α-Linolensäure (= ALA, C18:3$^{\Delta 9,12,15}$) bzw. mindestens 40 Gew.-% der zugesetzten γ-Linolensäure (= GLA, C18:3$^{\Delta 6,9,12}$), vorteilhaft mindestens 20 Gew.-% bzw. 50 Gew.-%, besonders vorteilhaft mindestens 25 Gew.-% bzw. 60 Gew.-% um. Besonders vorteilhaft wird auch C18:4$^{\Delta 6,9,12,15}$ (Stearidonsäure) elongiert. SDA wird dabei zu mindestens 40 Gew.-%, vorteilhaft zu mindestens 50 Gew.-%, besonders vorteilhaft zu mindestens 60 Gew.-%, ganz besonders vorteihaft zu mindestens 70 Gew.-% umgesetzt. Besonders vorteilhafte Δ-6-Elongasen zeigen keine oder nur eine sehr geringe Aktivität (weniger als 0,1 Gew-% Umsatz) gegenüber den folgenden Substraten: C18:1$^{\Delta 6}$, C18:1$^{\Delta 9}$, C18:1$^{\Delta 11}$, C20:2$^{\Delta 11,14}$, C20:3$^{\Delta 11,14,17}$, C20:3$^{\Delta 8,11,14}$, C20:4$^{\Delta 5,8,11,14}$, C20:5$^{\Delta 5,8,11,14,17}$ oder C22:4$^{\Delta 7,10,13,16}$.

Die Figuren 29 und 30 sowie die Tabelle 21 gibt die gemessenen Substratspezifitäten der verschiedenen Elongasen wieder.

Die im erfindungsgemäßen Verfahren verwendete ω-3-Desaturase hat gegenüber den bekannten ω-3-Desaturase die vorteilhafte Eigenschaft, dass sie ein breites Spektrum an ω-6-Fettsäuren desaturieren kann, bevorzugt werden $C_{20}$- und $C_{22}$-Fettsäuren wie $C_{20:2}$-, $C_{20:3}$-, $C_{20:4}$-, $C_{22:4}$- oder $C_{22:5}$-Fettsäuren desaturiert. Aber auch die kür-

zeren $C_{18}$-Fettsäuren wie $C_{18:2}$- oder $C_{18:3}$-Fettsäuren werden vorteilhaft desaturiert. Durch diese Eigenschaften der ω-3-Desaturase ist es vorteilhaft möglich das Fettsäurespektrum innerhalb eines Organismus vorteilhaft innerhalb einer Pflanze oder einem Pilz von den ω-6-Fettsäuren zu den ω-3-Fettsäuren hin zu verschieben. Bevorzugt werden von der erfindungsgemäßen ω-3-Desaturase $C_{20}$-Fettsäuren desaturiert. Innerhalb des Organismus werden diese Fettsäuren aus dem vorhandenen Fettsäurepool zu mindestens 10%, 15%, 20%, 25% oder 30% zu den entsprechenden ω-3-Fettsäuren umgesetzt. Gegenüber den $C_{18}$-Fettsäuren weist die ω-3-Desaturase eine um den Faktor 10 geringere Aktivität auf, das heißt es werden nur ca. 1,5 bis 3% der im Fettsäurepool vorhandenen Fettsäuren zu den entsprechenden ω-3-Fettsäuren umgesetzt. Bevorzugtes Substrat der erfindungsgemäßen ω-3-Desaturase sind die in Phospholipiden gebundenen ω-6-Fettsäuren. Figur 19 zeigt deutlich am Beispiel der Desaturierung von Dihomo-γ-linolensäure [$C_{20:4}{}^{\Delta 8,11,14}$], dass die ω-3-Desaturase bei der Desaturierung vorteilhaft nicht zwischen an sn1- oder sn2-Position gebundenen Fettsäuren unterscheidet. Sowohl an sn1- oder sn2-Position in den Phospholipide gebundene Fettsäuren werden desaturiert. Weiterhin ist vorteilhaft, dass die ω-3-Desaturase eine breite Palette von Phospholipiden wie Phosphatidylcholin (= PC), Phosphatidylinositol (= PIS) oder Phosphatidylethanolamin (= PE) umsetzt. Schließlich lassen sich auch Desaturierungsprodukte in den Neutrallipiden (= NL), das heißt in den Triglyceriden finden.

Die im erfingungsgemäßen Verfahren verwendeten Δ-4-Desaturasen, Δ-5-Desaturasen und Δ-6-Desaturasen haben gegenüber den bekannten Δ-4-Desaturasen, Δ-5-Desaturasen und Δ-6-Desaturasen den Vorteil, dass sie Fettsäuren gebunden an Phospholipide oder CoA-Fettsäureester, vorteilhaft CoA-Fettsäureester umsetzen können.

Vorteilhaft setzen die im erfingungsgemäßen Verfahren verwendeten Δ-12-Desaturasen Ölsäure ($C18:1^{\Delta 9}$) zu Linolsäure ($C18:2^{\Delta 9,12}$) oder $C18:2^{\Delta 6,9}$ zu $C18:3^{\Delta 6,9,12}$ (= GLA) um. Vorteilhaft setzen die verwendeten Δ-12-Desaturasen Fettsäuren gebunden an Phospholipide oder CoA-Fettsäureester, vorteilhaft gebunden an CoA-Fettsäureester um.

Durch die enzymatische Aktivität der im erfindungsgemäßen Verfahren verwendeten Nukleinsäuren, die für Polypeptide mit Δ-5-Elongase-, Δ-6-Elongase- und/oder ω-3-Desaturaseaktivität codieren, vorteilhaft in Kombination mit Nukleinsäuresequenzen, die für Polypeptide des Fettsäure- oder Lipidstoffwechsels wie weiteren Polypeptiden mit Δ-4-, Δ-5-, Δ-6-, Δ-8-, Δ-12-Desaturase- oder Δ-5-, Δ-6-oder Δ-9-Elongaseaktivität codieren, können unterschiedlichste mehrfach ungesättigte Fettsäuren im erfindungsgemäßen Verfahren hergestellt werden. Je nach Auswahl der für das erfindungsgemäße Verfahren verwendeten vorteilhaften Pflanze lassen sich Mischungen der verschiedenen mehrfach ungesättigten Fettsäuren oder einzelne mehrfach ungesättigte Fettsäuren wie EPA, ARA oder DHA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA, EPA oder DHA. Liegt in der für das Verfahren verwendeten Pflanze als ungesättigte Fettsäure nur Linolsäure (= LA, $C18:2^{\Delta 9,12}$) vor, so können als Produkte des Verfahrens nur GLA, DGLA und ARA entstehen, die als freie Fettsäuren oder gebunden vorliegen können. Durch Expression der zusätzlichen ω-3-Desaturase in diesen Pflanzen kann das Fettsäurespektrum auch hin zu α-Linolensäure, DPA und DHA hin verschoben werden. Allerdings ist diese Verschiebung des Fettsäurespektrums nur relativ eingeschränkt möglich. Vorteilhafter ist eine solche Verschiebung in Pflanzen, die, wie im folgenden beschrieben, schon einen hohen Anteil an α-Linolensäure enthalten. Ist in der im Verfahren verwendeten Pflanze als ungesättigte Fettsäure nur α-Linolensäure (= ALA, $C18:3^{\Delta 9,12,15}$) beispielsweise wie in Lein, so können als Produkte des Verfahrens nur SDA, ETA, EPA und/oder DHA entstehen, die wie oben beschrieben als freie Fettsäuren oder gebunden vorliegen können. Durch Modifikation der Aktivität des an der Synthese beteiligten Enzyms Δ-5-Elongase vorteilhaft in Kombination mit der Δ-4-, Δ-5-, Δ-6-, Δ-12-Desaturase und/oder Δ-6-Elongase, oder der Δ-4-, Δ-5-, Δ-8-, Δ-12-Desaturase, und/oder Δ-9-Elongase lassen sich gezielt in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturase, die Δ-5-Elongase und die Δ-4-Desaturase zusätzlich in die Organismen vorteilhaft in die Pflanze eingebracht, so entstehen zusätzlich ARA, EPA und/oder DHA. Dies gilt auch für Organismen in die vorher die Δ-8-Desaturase und Δ-9-Elongase eingebracht wurde. Vorteilhaft werden nur ARA, EPA oder DHA oder deren Mischungen synthetisiert, abhängig von der in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA, DHA oder deren Mischungen vorteilhaft EPA oder DHA oder deren Mischungen.

Die im erfindungsgemäßen Verfahren verwendbare aus Forelle stammende Nukleinsäure mit der SEQ ID NO: 53 kodiert für ein Protein, das eine hohe Spezifität für die beiden $C18:4^{\Delta 6,9,12,15}$- und $C20:5^{\Delta 5,8,11,14,17}$-Fettsäuren zeigt, diese sind Vorstufen zur Synthese von DHA (Vorstufen und Synthese von DHA siehe Figur 1). Aber auch andere Fettsäuren werden durch das Enzym elongiert. Das von SEQ NO: 53 kodierte Protein hat damit eine Spezifität für Δ6- und Δ5-Fettsäuren mit zusätzlich einer ω3-Doppelbindung (Figur 2). Die Δ-5-Elongase hat eine keto-Acyl-CoA-Synthase-Aktivität, die vorteilhaft Fettsäurereste von Acyl-CoA-Estern um 2 Kohlenstoffatome verlängert.

Durch das Genprodukt des vorgenannten Fisch-Δ-5-Elongase-Gens und weiterer Δ-5-Elongasen, der Δ5-Desaturase aus Phaeodacylum sowie der Δ4-Desaturase aus Euglena konnte die Synthese von DHA in Hefe (Saccharomyces cerevisiae) nachgewiesen werden (Figur 3).

Neben der Produktion der Ausgangsfettsäuren für die im erfindungsgemäßen Verfahren vorteilhaft verwendeten Δ-5-Elongasen, Δ-6-Elongasen, Δ-9-Elongasen, Δ-4-Desaturasen, Δ-5-Desaturasen, Δ-6-Desaturasen, Δ-12-Desaturasen und/oder ω-3-Desaturasen direkt im transgenen Organismus vorteilhaft in der transgenen Pflanze können die Fettsäuren auch von außen gefüttert werden. Aus Kostengründen ist die Produktion im Organismus bevorzugt. Bevorzugt Substrate der ω-3-Desaturase sind die Linolsäure ($C18:2^{\Delta 9,12}$), die γ-Linolensäure ($C18:3^{\Delta 6,9,12}$), die Eicosadiensäure ($C20:2^{\Delta 11,14}$), die Dihomo-γ-linolensäure ($C20:3^{\Delta 8,11,14}$), die Arachidonsäure ($C20:4^{\Delta 5,8,11,14}$), die Docosatetraensäure ($C22:4^{\Delta 7,10,13,16}$) und die Docosapentaensäure ($C22:5^{\Delta 4,7,10,13,15}$).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max*, die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Im erfindungsgemäßen Verfahren verwendeten Nukleinsäuren stammen vorteilhaft aus Pflanzen wie Algen beispielsweise Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. rubens oder Tetraselmis sp. oder aus Algen der Familie Euglenaceae wie aus den Gattungen Ascoglena, Astasia, Colacium, Cyclidiopsis, Euglena, Euglenopsis, Hyalophacus, Khawkinea, Lepocinclis, Phacus, Strombomonas oder Trachelomonas wie die Gattungen und Art Euglena acus, Euglena geniculata, Euglena gracilis, Euglena mixocylindracea, Euglena rostrifera, Euglena viridis, Colacium stentorium, Trachelomonas cylindrica oder Trachelomonas volvocina. Auch aus Algen wie der Alge Porphyridium cruentum, Isochrysis galbana oder Chlorella minutissima, Chlorella vulgaris, Thraustochytrium aureum oder Nannochloropsis oculata können vorteilhaft die im Verfahren verwendeten Nukleinsäuresequenzen stammen. Vorteilhaft stammen die verwendeten Nukleinsäuren aus Algen der Gattungen Euglena, Mantoniella oder Ostreococcus.

Weitere vorteilhafte Pflanzen als Quellen für die im erfindungsgemäße Verfahren verwendeten Nukleinsäuresequenzen sind Algen wie Isochrysis oder Crypthecodinium, Algen/Diatomeen wie Thalassiosira oder Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Mucor oder Mortierella, Bakterien wie Shewanella, Hefen oder Tieren wie Nematoden wie Caenorhabditis, Insekten, Fröschen, Seegurken oder Fischen. Vorteilhaft stammen die im erfindungsgemäßen Verfahren isolierten, verwendeten Nukleinsäuresequenzen aus einem Tier aus der Ordnung der Vertebraten. Bevorzugt stammen die Nukleinsäuresequenzen aus der Klasse der Vertebrata; Euteleostomi, Actinopterygii; Neopterygii; Teleostei; Euteleostei, Protacanthopterygii, Salmoniformes; Salmonidae bzw. Oncorhynchus oder Vertebrata, Amphibia, Anura, Pipidae, Xenopus oder Evertebrata wie Protochordata, Tunicata, Holothuroidea, Cionidae wie Amaroucium constellatum, Botryllus schlosseri, Ciona intestinalis, Molgula citrina, Molgula manhattensis, Perophora viridis oder Styela partita. Besonders vorteilhaft stammen die Nukleinsäuren aus Pilzen, Tieren oder aus Pflanzen wie Algen oder Moosen, bevorzugt aus der Ordnung der Salmoniformes wie der Familie der Salmonidae wie der Gattung Salmo beispielsweise aus den Gattungen und Arten Oncorhynchus mykiss, Trutta trutta oder Salmo trutta fario, aus Algen wie den Gattungen Mantoniella oder Ostreococcus oder aus den Diatomeen wie den Gattungen Thalassiosira oder Phaeodactylum oder aus Algen wie Crypthecodinium.

Auch aus Mikroorganismen wie Pilze wie der Gattung Mortierella, Phytium z.B. der Gattung und Art Mortierella alpina, Mortierella elongata, Phytium irregulare, Phytium ultimum oder Bakterien wie der Gattung Shewanella z.B. der Gattung und Art Shewanella hanedai können vorteilhafte im erfindungsgemäßen Verfahren verwendete Nukleinsäure

stammen.

Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit den für die Δ-12-Desaturase, Δ-4-Desaturase, Δ-5-Desaturase, Δ-6-Desaturase, Δ-5-Elongase, Δ-6-Elongase und/oder ω-3-Desaturase codierenden Nukleinsäuresquenzen in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer transgenen Pflanze, die die im Verfahren verwendeten Nukleinsäuresequenzen enthält, wobei die Pflanze mit einer erfindungsgemäßen Nukleinsäuresequenz, die für die Δ-12-Desaturase, Δ-4-Desaturase, Δ-5-Desaturase, Δ-6-Desaturase, Δ-5-Elongase, Δ-6-Elongase und/oder ω-3-Desaturase codiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus dem Samen der Pflanze wie aus dem Samen einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

Ein weiterer Erfindungsgegenstand sind Genkonstrukte, die die erfindungsgemäßen Nukleinsäuresequenzen, die für eine Δ-5-Desaturase, Δ-6-Desaturase, Δ-5-Elongase oder Δ-6-Elongase codieren, enthalten, wobei die Nukleinsäure funktionsfähig mit einem oder mehreren Regulationssignalen verbunden ist. Zusätzlich können weitere Biosynthesegene des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) im Genkonstrukt enthalten sein. Vorteilhaft sind zusätzlich Biosynthesegene des Fettsäure- oder Lipidstoffwechsels ausgewählt aus der Gruppe der Δ-8-Desaturase, Δ-9-Desaturase, Δ-9-Elongase oder ω-3-Desaturase enthalten.

Die im Verfahren verwendeten Nukleinsäuresequenzen, die für Proteine mit Δ-5-Desaturase-, Δ-6-Desaturase-, Δ-12-Desaturase-, Δ-5-Elongase- oder Δ-6-Elongase-Aktivität kodieren, werden vorteilhaft allein oder bevorzugt in Kombination in einer Expressionskassette (= Nukleinsäurekonstrukt), die die Expression der Nukleinsäuren in einer Pflanze ermöglicht, in die Pflanze eingebracht. Es kann im Nukleinsäurekonstrukt mehr als eine Nukleinsäuresequenz einer enzymatischen Aktivität wie z.B. einer Δ-12-Desaturase, Δ-5-Desaturase, Δ-6-Desaturase, Δ-5-Elongase und/oder Δ-6-Elongase enthalten sein.

Zum Einbringen der Nukleinsäuren in die Genkonstrukte werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden unter Berücksichtigung der zu amplifizierenden Sequenz ausgewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann nach gelelektrophoretischer Auftrennung eine quantitative und qualitative Analyse erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilzen gewährleisten, und die die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cisregulatorische Regionen wie Promotoren und Terminatorsequenzen und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in *E. coli* als auch in Agrobacterium zu replizieren. Zu diesen binären Vektoren gehören Vektoren der Serien pBIB-HYG, pPZP, pBecks, pGreen. Erfindungsgemäß werden bevorzugt Bin19, pBI101, pBinAR, pGPTV und pCAMBIA verwendet.

Eine Übersicht über binäre Vektoren und ihre Verwendung gibt Hellens et al, Trends in Plant Science (2000) 5, 446-451.

Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittene und erforderlichenfalls gereinigte Amplifikat mit ähnlich präparierten Vektorfragmenten unter Einsatz von Ligase verbunden. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie Promotoren und Terminatorsequenzen. Die Konstrukte lassen sich vorteilhafterweise in Mikroorganismen, insbesondere in *E. coli* und *Agrobacterium tumefaciens*, unter Selektionsbedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren in Pflanzen eingebracht werden und damit bei der Transformation von Pflanzen verwendet werden, wie denjenigen, die veröffentlicht und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225. Die im Verfahren verwendeten Nukleinsäuren und/oder Vektoren lassen sich damit zur gentechnologischen Veränderung eines breiten Spektrums an Pflanzen verwenden, so dass diese bessere und/oder effizientere Produzenten von PUFAs werden.

Es gibt eine Reihe von Mechanismen, durch die eine Veränderung des Δ-12-Desaturase-, Δ-5-Elongase-, Δ-6-Elongase-, Δ-5-Desaturase- und/oder Δ-6-Desaturase-Proteins möglich ist, so dass die Ausbeute, Produktion und/oder Effizienz der Produktion der mehrfach ungesättigten Fettsäuren in einer Pflanze, bevorzugt in einer Ölsamen- oder Ölfruchtpflanze, aufgrund dieses veränderten Proteins direkt beeinflusst werden kann. Die Anzahl oder Aktivität der Δ-12-Desaturase-, Δ-6-Desaturase-, Δ-5-Elongase-, Δ-6-Elongase- oder Δ-5-Desaturase-Proteine oder -Gene kann erhöht werden, so dass größere Mengen der Genprodukte und damit letztlich größere Mengen der Verbindungen der allgemeinen Formel I hergestellt werden. Auch eine de novo Synthese in einer Pflanze, der die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren, die eine andere zeitliche Genexpression z.B. abhängig vom Reifegrad eines Samens oder Öl-speichernden Gewebes ermöglichen.

Durch das Einbringen einer Kombination von Δ-12-Desaturase-, Δ-6-Desaturase-, Δ-5-Elongase-, Δ-6-Elongase- und/oder Δ-5-Desaturase-Genen in die Pflanze allein oder in Kombination mit anderen Genen kann nicht nur der Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs weiter gesteigert wird. Durch Optimierung der Aktivität oder Erhöhung der Anzahl eines oder mehrerer Δ-12-Desaturase-, Δ-6-Desaturase-, Δ-5-Elongase-, Δ-6-Elongase- oder Δ-5-Desaturase-Gene, die an der Biosynthese dieser Verbindungen beteiligt sind, oder durch Zerstören der Aktivität einer oder mehrerer Gene, die am Abbau dieser Verbindungen beteiligt sind, wird die Steigerung der Ausbeute, Produktion und/oder Effizienz der Produktion von Fettsäure- und Lipidmolekülen in Pflanzen ermöglicht.

Die im Verfahren verwendeten Nukleinsäuresequenzen werden vorteilhaft in einer Expressionskassette, die die Expression der Nukleinsäuren in Pflanzen ermöglicht, eingebracht.

Dabei werden die Nukleinsäuresequenzen, die für die Δ-12-Desaturase, Δ-6-Desaturase, Δ-5-Elongase, Δ-6-Elongase oder Δ-5-Desaturase kodieren, mit einem oder mehreren Regulationssignalen vorteilhafterweise zur Erhöhung der Genexpression funktionell verknüpft. Diese regulatorischen Sequenzen sollen die gezielte Expression der Gene und Proteine ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen, an die Induktoren oder Repressoren binden und dadurch die Expression der Nukleinsäure regulieren. Zusätzlich zu diesen neuen Regulationssequenzen oder anstelle dieser Sequenzen können die natürlichen Regulationselemente dieser Sequenzen vor den eigentlichen Strukturgenen noch vorhanden sein und gegebenenfalls genetisch so verändert worden sein, dass ihre natürliche Regulation ausgeschaltet und die Expression der Gene erhöht wird. Diese veränderten Promotoren können in Form von Teilsequenzen (= Promotor mit Teilen der erfindungsgemäß verwendeten Nukleinsäuresequenzen) auch allein vor das natürliche Gen zur Steigerung der Aktivität gebracht werden. Das Genkonstrukt kann außerdem vorteilhafterweiser auch

eine oder mehrere sogenannte "Enhancer-Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatorsequenzen.

Die Δ-12-Desaturase-, Δ-5-Desaturase-, Δ-6-Desaturase-, Δ-5-Elongase- und/oder Δ-6-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen in der Wirtspflanze exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Eine weitere Ausführungsform der Erfindung sind ein oder mehrere Genkonstrukte, die eine oder mehrere Sequenzen enthalten, die durch SEQ ID NO: 11, SEQ ID NO: 27, SEQ ID NO: 193, SEQ ID NO: 195, SEQ ID NO: 197, SEQ ID NO: 199, SEQ ID NO: 201 oder deren Derivate definiert sind und für Polypeptide gemäß SEQ ID NO: 12, SEQ ID NO: 28, SEQ ID NO: 194, SEQ ID NO: 196, SEQ ID NO: 198, SEQ ID NO: 200, SEQ ID NO: 202 kodieren. Die genannten Δ-12-Desaturase-, Δ-6-Desaturase-, Δ-5-Elongase-, Δ-6-Elongase- oder Δ-5-Desaturase-Proteine führen dabei vorteilhaft zu einer Desaturierung oder Elongierung von Fettsäuren, wobei das Substrat vorteilhaft ein, zwei, drei oder vier Doppelbindungen und vorteilhaft 18, 20 oder 22 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. die in WO 99/16890 beschriebenen.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren, die im Embryo und/oder im Endosperm aktiv sind. Samenspezifische Promotoren können prinzipiell sowohl aus dikotyledonen als auch aus monokotyledonen Pflanzen isoliert werden. Im folgenden sind bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Conlinin (Lein) [WO 02/102970], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und lpt1 (Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die Δ-12-Desaturase, Δ-6-Desaturase, Δ-5-Elongase, Δ-6-Elongase und/oder Δ-5-Desaturase kodieren, unter der Kontrolle eines eigenen, bevorzugt eines von den anderen Promotoren verschiedenen, Promotors exprimiert werden, da sich wiederholende Sequenzmotive zur Instabilität der T-DNA bzw. zu Rekombinationsereignissen führen können. Die Expressionskassette ist dabei vorteilhaft so aufgebaut, dass einem Promotor eine geeignete Schnittstelle, vorteilhaft in einem Polylinker, zur Insertion der zu exprimierenden Nukleinsäure folgt und gegebenenfalls eine Terminatorsequenz hinter dem Polylinker liegt. Diese Abfolge wiederholt sich mehrfach, bevorzugt drei-, vier-, fünf-, sechs- oder siebenmal, so dass bis zu sieben Gene in einem Konstrukt zusammengeführt werden und zur Expression in die transgene Pflanze eingebracht werden können. Vorteilhaft wiederholt sich die Abfolge bis zu viermal. Die Nukleinsäuresequenzen werden zur Expression über eine geeignete Schnittstelle beispielsweise im Polylinker hinter den Promotor inseriert. Vorteilhaft hat jede Nukleinsäuresequenz ihren eigenen Promotor und gegebenenfalls ihre eigene Terminatorsequenz. Derartige vorteilhafte Konstrukte sind beispielsweise in DE 101 02 337 oder DE 101 02 338 offenbart. Es ist aber auch möglich, mehrere Nukleinsäuresequenzen

hinter einem gemeinsamen Promotor und ggf. vor einer gemeinsamen Terminatorsequenz zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch ihre Expression wesentlich beeinflusst wird. Es können in der Expressionskassette vorteilhaft unterschiedliche Promotoren wie beispielsweise der USP-, LegB4 oder DC3-Promotor und unterschiedliche Terminatorsequenzen verwendet werden. Es ist aber auch möglich, nur einen Promotortyp in der Kassette zu verwenden, was jedoch zu unerwünschten Rekombinationsereignissen führen kann.

Wie oben beschrieben sollte die Transkription der eingebrachten Gene vorteilhaft durch geeignete Terminatorsequenzen am 3'-Ende der eingebrachten Biosynthesegene (hinter dem Stopcodon) abgebrochen werden. Verwendet werden kann hier z.B. die OCS1-Terminatorsequenz. Wie auch für die Promotoren, so sollten für jedes Gen unterschiedliche Terminatorsequenzen verwendet werden.

Das Genkonstrukt kann, wie oben beschrieben, auch weitere Gene umfassen, die in die Pflanzen eingebracht werden sollen. Es ist möglich und vorteilhaft, in die Wirtspflanzen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und zu exprimieren. Diese Gene können heterologen oder homologen Ursprungs sein.

Weiterhin können vorteilhaft im Nukleinsäurekonstrukt bzw. Genkonstrukt weitere Biosynthesegene des Fettsäure- oder Lipidstoffwechsels enthalten sein, diese Gene können aber auch auf einem oder mehreren weiteren Nukleinsäurekonstrukten liegen. Vorteilhaft werden als Biosynthesegen des Fettsäure- oder Lipidstoffwechsels ein Gen ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder Kombinationen davon verwendet.

Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, ω-3-Desaturase, Δ-8-Desaturase, Δ-4-Desaturase, Δ-9-Desaturase, Δ-5-Elongase und/oder Δ-9-Elongase.

Dabei können die vorgenannten Nukleinsäuren bzw. Gene in Kombination mit anderen Elongasen und Desaturasen in Expressionskassetten, wie den vorgenannten, kloniert werden und zur Transformation von Pflanzen mit Hilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektor eingebracht werden.

Diese vorteilhaften Vektoren, vorzugsweise Expressionsvektoren, enthalten die im Verfahren verwendeten Nukleinsäuren, die für die Δ-12-Desaturasen, Δ-6-Desaturasen, Δ-5-Elongasen, Δ-6-Elongasen oder Δ-5-Desaturasen kodieren, oder ein Nukleinsäurekonstrukt, das die verwendete Nukleinsäure allein oder in Kombination mit weiteren Biosynthesegenen des Fettsäure- oder Lipidstoffwechsels wie den Acyl-CoA:Lysophospholipid-Acyltransferasen, ω-3-Desaturasen, Δ-8-Desaturasen, Δ-9-Desaturasen, ω3-Desaturasen, Δ-4-Desaturasen, Δ-5-Elongasen und/oder Δ-9-Elongasen enthält.

Wie hier verwendet, betrifft der Begriff "Vektor" ein Nukleinsäuremolekül, das eine andere Nukleinsäure transportieren kann, die an es gebunden ist. Ein Vektortyp ist ein "Plasmid", eine zirkuläre doppelsträngige DNA-Schleife, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch auch andere Expressionsvektorformen, wie virale Vektoren, die ähnliche Funktionen ausüben, umfassen. Ferner soll der Begriff "Vektor" auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die erfindungsgemäß verwendeten Nukleinsäuren oder das beschriebene Genkonstrukt in einer Form, die sich zur Expression der verwendeten Nukleinsäuren in einer Wirtszelle eignet, was bedeutet, dass die rekombinanten Expressionsvektoren eine oder mehrere

Regulationssequenzen, ausgewählt auf der Basis der zur Expression verwendeten Wirtszellen, die mit der zu exprimierenden Nukleinsäuresequenz funktionsfähig verbunden ist, umfassen. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird).

Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, die die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, der gewünschten Expressionsstärke des Proteins usw., abhängen kann.

Bei einer weiteren Ausführungsform des Verfahrens können die Δ-12-Desaturasen, Δ-6-Desaturasen, Δ-5-Elongasen, Δ-6-Elongasen und/oder Δ-5-Desaturasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und die funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus *Agrobacterium tumefaciens*-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktive Terminatorsequenzen sind geeignet.

Da die Regulation der Pflanzengenexpression sehr oft nicht auf Transkriptionsebene beschränkt ist, enthält eine Pflanzen-Expressionskassette vorzugsweise andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Das zu exprimierende Gen muss, wie oben beschrieben, funktionsfähig mit einem geeigneten Promotor verbunden sein, der die Genexpression auf rechtzeitige, zell- oder gewebespezifische Weise auslöst. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder konstitutive Pflanzenpromotoren, wie der in US 4,962,028 beschriebene der kleinen Untereinheit der Rubisco.

Die Pflanzengenexpression lässt sich auch wie oben beschrieben über einen chemisch induzierbaren Promotor erreichen (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignet, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napin-Promotor aus Raps (US 5,608,152), der Conlinin-Promotor aus Lein (WO 02/102970), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, die die samenspezifische Expression in monokotyledonen Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen Promotoren aus dem Gersten-Hordein-Gen, dem Reis-Glutelin-Gen, dem Reis-Oryzin-Gen,

dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren sind der virale RNA-Polymerase-Promotor, beschrieben in WO 95/16783 und WO 97/06250, und der clpP-Promotor aus Arabidopsis, beschrieben in WO 99/46394.

Insbesondere kann die multiparallele Expression der im Verfahren verwendeten Δ-12-Desaturasen, Δ-6-Desaturasen, Δ-5-Elongasen, Δ-6-Elongasen und/oder Δ-5-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment, beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen notwendig sind (siehe eine Übersicht in Kermode, Crit. Rev. Plant Sci. 15, 4 (1996) 285-423 und darin zitierte Literaturstellen).

Im erfindungsgemäßen Verfahren werden die Nukleinsäuresequenzen mit den SEQ ID NO: 11, SEQ ID NO: 27, SEQ ID NO: 193, SEQ ID NO: 195, SEQ ID NO:197, SEQ ID NO: 199, SEQ ID NO: 201 oder deren Derivate oder Homologe, die für Polypeptide kodieren, die noch die enzymatische Aktivität der durch Nukleinsäuresequenzen kodierten Proteine besitzen, verwendet. Diese Sequenzen werden einzeln oder in Kombination mit den Nukleinsäuresquenzen, die für die anderen verwendeten Enzyme kodieren, in Expressionskonstrukte kloniert und zur Transformation und Expression in Pflanzen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder einer ganzen Pflanze, die die im Verfahren verwendeten Nukleinsäuresequenzen enthält, wobei die Zelle und/oder die Pflanze mit einer Nukleinsäuresequenz, die für ein Polypeptid mit einer Δ-12-Desaturase-, Δ-5-Desaturase-, Δ-6-Desaturase-, Δ-5-Elongase- und/oder Δ-6-Elongase-Aktivität kodiert, einem Genkonstrukt oder einem Vektor wie vorstehend beschrieben, allein oder in Kombination mit weiteren Nukleinsäuresequenzen, die für Proteine des Fettsäure- oder Lipidstoffwechsels kodieren, transformiert wird. Die so hergestellte Zelle ist vorteilhaft eine Zelle eines Öl-produzierenden Organismus wie einer Ölfruchtpflanze wie beispielsweise Erdnuss, Raps, Canola, Lein, Hanf, Erdnuss, Soja, Färbersaflor, Hanf, Senf, Sonnenblumen oder Borretsch.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassette oder Vektor alle solche durch gentechnische Methoden zustandegekommenen Konstruktionen, in denen sich entweder

a) die erfindungsgemäße Nukleinsäuresequenz, oder

b) eine mit der erfindungsgemäßen Nukleinsäuresequenz funktionell verknüpfte genetische Kontrollsequenz, zum Beispiel ein Promotor, oder

c) (a) und (b)

sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der im erfindungsgemäßen Verfahren verwendeten Nukleinsäuresequenzen mit den entsprechenden Δ-12-Desaturase-, Δ-4-Desaturase-, Δ-5-Desaturase-, Δ-6-Desaturase-, Δ-8-Desaturase-, ω-3-Desaturase-, Δ-9-Elongase-, Δ-6-Elongase- und/oder Δ-5-Elongasegenen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter transgenen Pflanzen im Sinne der Erfindung ist daher zu verstehen, dass sich die im Verfahren verwendeten Nukleinsäuren nicht an ihrer natürlichen Stelle im Genom der Pflanze befinden, wobei die Nukleinsäuren homolog oder heterolog exprimiert werden können. Transgen bedeutet aber auch, dass die erfindungsgemäßen Nukleinsäuren an ihrem natürlichen Platz im Genom der Pflanze sind, dass jedoch die Sequenz gegenüber der natürlichen Sequenz verändert wurde und/oder das die Regulationssequenzen, der natürlichen Sequenz verändert wurden. Bevorzugt ist unter transgen die Expression der erfindungsgemäßen Nukleinsäuren oder der im erfindungsgemäßen Verfahren verwendeten Nukleinsäuresequenzen an nicht natürlicher Stelle im Genom zu verstehen, das heißt eine homologe oder bevorzugt heterologe Expression der Nukleinsäuren liegt vor. Bevorzugte transgene Pflanzen sind Ölsamen- oder Ölfruchtpflanzen.

Als Pflanzen zur Verwendung im erfindungsgemäßen Verfahren eignen sich prinzipiell vorteilhaft alle Pflanzen, die in der Lage sind Fettsäuren, speziell ungesättigte Fettsäuren wie ARA, EPA und/oder DHA, zu synthetisieren und die für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne genannt. Bevorzugt werden Pflanzen, die natürlicherweise Öle in größeren Mengen synthetisieren können wie Soja, Raps, Camelina, Sareptasenf, Kokosnuss, Ölpalme, Färbersaflor (Carthamus tinctorius), Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, FärberSaflor, Sonnenblume, Camelina, Sareptasenf oder Calendula.

Weitere für die Klonierung der im erfindungsgemäßen Verfahren verwendeten Nukleinsäuresequenzen nutzbare Wirtszellen sind weiterhin genannt in: Goeddel, Gene Expression Technology: Methods in Enzymology 185, Academic Press, San Diego, CA (1990).

Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128.

Hierzu gehören auch Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen bzw. vorteilhaft deren Samen, die die im erfindungsgemäßen Verfahren synthetisierten mehrfach ungesättigten Fettsäuren, insbesondere ARA, EPA und/oder DHA enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe.

Grundsätzlich eignet sich das erfindungsgemäße Verfahren auch zur Herstellung mehrfach ungesättigter Fettsäuren, insbesondere von ARA, EPA und/oder DHA in pflanzlichen Zellkulturen und anschließender Gewinnung der Fettsäuren aus den Kulturen. Dabei kann es sich insbesondere um Suspensions- oder Kalluskulturen handeln.

Die im erfindungsgemäßen Verfahren hergestellten Verbindungen können aber auch aus den Pflanzen vorteilhaft aus den Pflanzensamen in Form ihrer Öle, Fett, Lipide und/oder freien Fettsäuren isoliert werden. Durch dieses Verfahren hergestellte mehrfach ungesättigten Fettsäuren, insbesondere ARA, EPA und/oder DHA, lassen sich durch Ernten der Pflanzen bzw. Pflanzensamen entweder aus der Kultur, in der sie wachsen, oder vom Feld ernten.

Bei einer weiteren bevorzugten Ausführungsform umfasst dieses Verfahren ferner den Schritt des Gewinnens der Öle, Lipide oder freien Fettsäuren aus der Pflanze oder aus der Kultur. Bei der Kultur kann es sich beispielsweise um eine Treibhaus- oder Feldkultur einer Pflanze handeln.

Das Isolieren der Öle, Lipide oder freien Fettsäuren kann über Pressen oder Extraktion der Pflanzenteile bevorzugt der Pflanzensamen, erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt.

Danach werden die so erhaltenen Produkte, die die mehrfach ungesättigten Fettsäuren enthalten, weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer

Bleichung mit beispielsweise Bleicherde oder Aktivkohle unterzogen. Zum Schluss wird das Produkt noch beispielsweise mit Wasserdampf desodoriert.

Vorzugsweise sind die durch dieses Verfahren produzierten PUFAs bzw. LCPUFAs $C_{18}$-, $C_{20}$- oder $C_{22}$-Fettsäuremoleküle vorteilhaft $C_{20}$- oder $C_{22}$-Fettsäuremoleküle mit mindestens drei Doppelbindungen im Fettsäuremolekül, vorzugsweise, vier, fünf oder sechs Doppelbindungen, besonders bevorzugt mit vier, fünf oder sechs Doppelbindungen. Diese $C_{18}$-, $C_{20}$- oder $C_{22}$-Fettsäuremoleküle lassen sich aus der Pflanze in Form eines Öls, Lipids oder einer freien Fettsäure isolieren. Geeignete Pflanzen sind beispielsweise die vorstehend erwähnten. Bevorzugte Organismen sind transgene Pflanzen.

Eine Ausführungsform der Erfindung sind deshalb Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Diese Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Butterbuttersäure, kein Cholesterin, keine Clupanodonsäure (= Docosapentaensäure, C22:5$^{\Delta4,8,12,15,21}$) sowie keine Nisinsäure (Tetracosahexaensäure, C23:6$^{\Delta3,8,12,15,18,21}$).

Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin, keine Clupanodonsäure (= Docosapentaensäure, C22:5$^{\Delta4,8,12,15,21}$) sowie keine Nisinsäure (Tetracosahexaensäure, C23:6$^{\Delta3,8,12,15,18,21}$).

Vorteilhaft enthalten die beschriebenen Öle, Lipide oder Fettsäuren mindestens 0,5%, 1%, 2%, 3%, 4%, 5%, 6%, 7%, 8%, 9% oder 10%, vorteilhaft mindestens 11%, 12%, 13%, 14%, 15%, 16% oder 17%, besonders vorteilhaft mindestens 18%, 19%, 20%, 21%, 22%, 23%, 24% oder 25% ARA oder mindestens 0,5%, 1%, 2%, 3%, 4%, 5% oder 6%, vorteilhaft mindestens 7%, 8%, 9%, 10% oder 11% besonders vorteilhaft mindestens 12%, 13%, 14%, 15%, 16%, 17'%, 18%, 19% oder 20% EPA oder mindestens 0,01%, 0,02%, 0,03%, 0,04% oder 0,05% oder 0,06%, vorteilhaft mindestens 0,07%, 0,08%, 0,09 oder 0,1%, besonders vorteilhaft mindestens 0,2%, 0,3% oder 0,4% DHA bezogen auf den Gesamtfettsäuregehalt des Produktionsorganismus vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen. Alle Prozentangaben beziehen sich auf Gewichtsprozente.

Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren, vor allem an ARA und EPA aber auch DHA, von mindestens 50, 80 oder 100 %, vorteilhaft von mindestens 150, 200 oder 250 %, besonders vorteilhaft von mindestens 300, 400, 500, 600, 700, 800 oder 900 %, ganz besonders vorteilhaft von mindestens 1000, 1100, 1200, 1300, 1400 oder 1500 % gegenüber der nicht transgenen Ausgangspflanze beispielsweise einer Pflanze wie Brassica juncea, Brassica napus, Camelina sativa, Arabidopsis thanliana oder Linum usitatissimum beim Vergleich in der GC-Analyse siehe Beispiele erreicht werden.

Die im erfindungsgemäßen Verfahren hergestellten Lipide und/oder Öle haben einen höheren Anteil der ungesättigten Fettsäuren Ölsäure, Linolsäure und $\alpha$-Linolensäure in sn2-Position im Vergleich zu den anderen Positionen sn1 und sn3. Unter höheren Anteil sind Verhältnisse von (sn1:sn2:sn3) 1:1,1:1; 1:1,5:1 bis 1:3:1 zu verstehen. Auch die im Verfahren hergestellte Arachidonsäure, Eicosapentaensäure oder Docosahexaensäure zeigen in den Lipiden und/oder Ölen ebenfalls eine Präferenz für die sn2-Position im Triglycerid gegenüber den Positionen sn1 und sn3 von vorteilhaft 1:1,1:1; 1:1,5:1 bis 1:3:1.

Vorteilhaft werden, wie oben beschrieben, die im Verfahren hergestellten mehrfach ungesättigten $C_{20}$- und/oder $C_{22}$-Fettsäuren mit vier, fünf oder sechs Doppelbindungen im Molekül im Samen von Pflanzen, die keine oder nur sehr geringe Mengen an C12:0-bzw. C14:0-Fettsäuren enthalten. Auch noch kürzere gesättigte Fettsäuren wie die Fettsäuren C4:0, C6:0, C8:0 oder C10:0 sollten nicht oder nur in geringen Mengen im Lipid und/oder Öl vorhanden sein. Unter nur sehr geringen Mengen sind vorteilhaft Mengen zu verstehen, die in der GC-Analyse vorteilhaft unter 5, 4, 3, 2 oder 1 %, vorteilhaft unter 0,9; 0,8; 0,7; 0,6 oder 0,5 %, besonders vorteilhaft unter 0,4; 0,3; 0,2 ider 0,1 %, ganz besonders bevorzugt unter 0,09; 0,08; 0,07; 0,06; 0,05; 0,04; 0,03; 0,02 oder 0,01 Flächeneinheiten in der GC liegen. Die Fettsäure C16:0 sollte vorteilhaft in einem Bereich von 1 bis 28 % GC-Flächeneinheiten liegen. Vorteilhaft sollte die Fettsäure C16:0 in GC-Flächeneinheiten von weniger als 25%, 20%, 15% oder 10%, vorteilhaft von weniger als 9%, 8%, 7%, 6% oder 5%, besonders vorteilhaft von weniger als 4%, 3%, 2% oder 1% oder gar nicht in den Lipiden, Ölen und/oder freien Fettsäuren vorhanden sein. Die Fettsäure C16:1 sollte vorteilhaft weniger als 1; 0,5; 0,4; 0,3; 0,2 oder 0,1 %, besonders vorteilhaft 0,09; 0,08; 0,07; 0,06; 0,05; 0,04; 0,03; 0,02 oder 0,01 Flächeneinheiten in der GC betragen. Ganz besonders bevorzugt sollte die Fettsäure C16:1 nicht in den nach dem Verfahren hergestellten Ölen und/oder Lipiden vorhanden sein. Gleiches gilt für die Fettsäuren C15:0, C17:0, C16:1 $^{\Delta 3}$trans, C16:4$^{\Delta 4,7,10,13}$ und C18:5$^{\Delta 3,6,9,12,15}$. Neben Ölsäure (C18:1$^{\Delta 9}$) können auch die Isomere (C18:1$^{\Delta 7}$, C18:1$^{\Delta 11}$) in den Lipiden, Ölen oder freien Fettsäuren vorhanden sein. Vorteilhaft in Mengen, gemessen als GC-Flächeneinheiten, von weniger als 5%, 4%, 3%, 2% oder 1%. Die Fettsäuren C20:0, C20:1, C24:0 und C24:1 sollten jeweils in einem Bereich von 0 bis 1 %, 0 bis 3% bzw. 0 bis 5 % Flächeneinheiten in der GC liegen. Weiterhin sollte in der GC-Analyse wenig Dihomo-$\gamma$-linolensäure (= DGLA) im Samenöl und/oder -lipid in GC-Flächeneinheiten detektierbar sein. Unter wenig sind weniger als 2; 1,9; 1,8; 1,7; 1,6 oder 1,5 %, vorteilhaft weniger als 1,4; 1,3; 1,2; 1,1 oder 1 %, besonders vorteilhaft weniger als 0,9; 0,8; 0,7; 0,6; 0,5 oder 0,4 % in GC-Flächeneinheiten zu verstehen.

In einer bevorzugten Ausführungsform des Verfahrens sollte DGLA und ARA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

In weiteren bevorzugten Ausführungsform des Verfahrens sollte DGLA und EPA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

Vorteilhaft sollten die im erfindungsgemäßen Verfahren hergestellten Lipide, Öle und/oder freien Fettsäuren einen hohen Anteil von ungesättigten Fettsäuren vorteilhaft von mehrfach ungesättigten Fettsäuren von mindestens 30, 40 oder 50 Gew.-%, vorteilhaft von mindestens 60, 70 oder 80 Gew.-% bezogen auf den Gesamtfettsäuregehalt in den Samen der transgenen Pflanzen betragen.

Alle gesättigten Fettsäuren zusammen sollten vorteilhaft in den Lipiden, Ölen und/oder freien Fettsäuren bevorzugt verwendeten Pflanzen nur einen geringen Anteil ausmachen. Unter geringen Anteil ist in diesem Zusammenhang ein Anteil in GC-Flächeneinheiten von weniger als 15%, 14%, 13%, 12%, 11% oder 10%, bevorzugt von weniger als 9%, 8%, 7% oder 6% zu verstehen.

Im Verfahren hergestellte Lipide, Öle und/oder freie Fettsäuren sollten vorteilhaft einen Gehalt an Erucasäure von weniger als 2 Gew.-% bezogen auf den Gesamtfettsäuregehalt der Pflanze haben. Vorteilhaft sollte keine Erucasäure in den Lipiden und/oder Ölen vorhanden sein. Auch sollte der Gehalt an gesättigten Fettsäuren C16:0 und/oder C18:0 vorteilhaft geringer als 19, 18, 17, 16, 15, 14, 13, 12, 11, oder 10 Gew.-%, vorteilhaft weniger als 9, 8, 7, 6 oder 5 Gew.-% bezogen auf den gesamten Fettsäuregehalt der Lipide und/oder Öle sein. Vorteilhaft sollten auch längere Fettsäuren wie C20:0 oder C22:1 gar nicht oder in nur geringen Mengen vorteilhaft geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Lipde

und/oder Öle sein. Typischerweise ist in den Lipden und/oder Ölen, die nach dem erfindungsgemäßen Verfahren hergestellt wurden, kein oder nur in geringen Mengen C16:1 als Fettsäure enthalten. Unter geringen Mengen sind vorteilhaft Gehalte an Fettsäuren zu verstehen, die geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Lipide und/oder Öle.

Die nach dem Pressen erhaltenen hierin beschriebenen Öle, Lipide, Fettsäuren oder Fettsäuregemische werden als sogenannte Rohöle bezeichnet. Diese enthalten noch die gesamten Öl- und/oder Lipidkomponenten, sowie Verbindungen, die in diesen löslich sind. Derartige Verbindunge sind die verschiedenen Tocopherole wie $\alpha$-Tocopherol, $\beta$-Tocopherol, $\gamma$-Tocopherol und/oder $\delta$-Tocopherol oder Phytosterole wie Brassicasterol, Campesterol, Stigmasterol, $\beta$-Sitosterol, Sitostanol, $\Delta^5$-Avenasterol, $\Delta^5$,24-Stigmastadienol, $\Delta^7$-Stigmastenol oder $\Delta^7$-Avenasterol. Diese Verbindungen sind in einem Bereich von 1 bis 1000 mg/100 g vorteilhaft von 10 bis 800 mg/100 g Lipid oder Öl enthalten. Auch Triterpene wie Germaniol, Amyrin, Cycloartanol und andere können in diesen Lipiden und Ölen enthalten sein. Diese Lipide und/oder Öle enthalten die im Verfahren hergestellten mehrfach ungesättigten Fettsäuren wie ARA, EPA und/oder DHA gebunden in polaren und unpolaren Lipiden wie Phospholipiden z.B. Phosphatidylcholin, Phosphatidylethanolamin, Phosphatidylinositol, Phosphatidylserin, Phosphatidylglycerin, Galactolipiden, Monoglyceride, Diglyceride oder Triglyceride um nur einige zu nennen. Auch Lysophospholipide können in den Lipiden und/oder Ölen vorkommen. Diese Komponenten der Lipide und/oder Öle können durch geeignete Methoden voneinander getrennt werden. Nicht enthalten in diesen Rohölen ist Cholesterol.

Eine weitere erfindungsgemäße Ausführungsform ist die Verwendung des Öls, Lipids, der Fettsäuren und/oder der Fettsäurezusammensetzung in Futtermitteln, Nahrungsmitteln, Kosmetika oder Pharmazeutika. Die erfindungsgemäßen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Typisch für derartige Fischöle kurzkettige Fettsäuren wie C12:0, C14:0, C14:1, verzweigtkettiges C15:0, C15:0, C16:0 oder C16:1. Auch mehrfach ungesättige C16-Fettsäuren wie C16:2, C16:3 oder C16:4, verzweigtkettiges C17:0, C17:1, verzweigtkettiges C18:0 und C19:0 sowie C19:0 und C19:1 kommen im Fischöl vor. Derartige Fettsäuren sind typisch für Fischöle und werden nur selten oder gar nicht in pflanzlichen Ölen gefunden. Wirtschaftlich relevante Fischöle sind z.B. Anchovissöl, Menhadneöl, Tunfischöl, Sardinenöl, Heringsöl, Markrelenöl, Walöl und Lachsöl. Diese Lipide und/oder Öle tierischen Ursprungs können zum Abmischen mit den erfindungsgemäßen Ölen in Form von Rohölen, das heißt in Form von Lipiden und/oder Ölen, die noch nicht aufgereinigt wurden, verwendet werden oder aber es können verschieden aufgereinigte Fraktionen zum Abmischen verwendet werden.

Eine weitere erfindungsgemäße Ausführungsform ist die Verwendung des Öls, Lipids, Fettsäuren und/oder der Fettsäurezusammensetzung in Futtermitteln, Nahrungsmitteln, Kosmetika oder Pharmazeutika.

Die hierin beschriebenen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, $\gamma$-Linolensäure, Dihomo-$\gamma$-linolensäure, Arachidonsäure, $\alpha$-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 %, 85% oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangspflanze der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen enthalten, handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. $H_2SO_4$. Die Freisetzung der Fettsäuren kann auch direkt ohne die vorhergehend beschriebene Aufarbeitung erfolgen.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige

Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Die im Verfahren verwendeten Nukleinsäuren können nach Einbringung in eine Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Die Co-Expression mehrerer Gene kann natürlich nicht nur durch Einbringen der Gene auf einem gemeinsamen rekombinanten Nukleinsäurekonstrukt erfolgen. Vielmehr können einzelne Gene auch separat - gleichzeitig oder nacheinander - auf verschiedenen Konstrukten eingebracht werden. Hier wird z.B. durch die Verwendung verschiedener Selektionsmarker die gleichzeitige Anwesenheit in der alle Gene coexprimierenden Pflanze sichergestellt. Diese Pflanze kann das Produkt eines oder mehrerer Transformationsvorgänge sein, oder aber auch ein Kreuzungsprodukt von Pflanzen, die eines oder mehrere der Gene enthalten.

Als Substrate der im erfindungsgemäßen Verfahren verwendeten Nukleinsäuren, die für Polypeptide mit ω-3-Desaturase-, Δ-4-Desaturase-, Δ-5-Desaturase-, Δ-6-Desaturase-, Δ-8-Desaturase-, Δ-12-Desaturase-, Δ-5-Elongase-, Δ-6-Elongase- und/oder Δ-9-Elongase-Aktivität kodieren, und/oder den weiteren verwendeten Nukleinsäuren wie den Nukleinsäuren, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) kodieren, eignen sich vorteilhaft $C_{16}$-, $C_{18}$-, $C_{20}$- oder $C_{22}$-Fettsäuren. Bevorzugt werden die im Verfahren als Substrate umgesetzten Fettsäuren in Form ihrer Acyl-CoA-Ester und/oder ihrer Phospholipid-Ester umgesetzt. Vorteilhaft werden im Verfahren Desaturasen verwendet, die eine Spezifität für die Acyl-CoA-Ester haben. Dies hat den Vorteil, dass kein Ausstausch zwischen den Phospholipid-Estern, die in der Regel das Substrat der Desaturierung sind, und den Acyl-CoA-Estern stattfinden muss. Dadurch entfällt ein weiterer Enzymschritt, der, wie sich gezeigt hat, in einigen Fällen ein limitierender Schritt ist.

Zur Herstellung der hierin beschriebenen langkettigen PUFAs müssen die mehrfach ungesättigten $C_{16}$- oder $C_{18}$-Fettsäuren zunächst durch die enzymatische Aktivität einer Desaturase desaturiert und anschließend über eine Elongase um mindestens zwei Kohlenstoffatome verlängert werden. Nach einer Elongationsrunde führt diese Enzymaktivität zu $C_{18}$- oder $C_{20}$-Fettsäuren und nach zwei Elongationsrunden zu $C_{20}$- oder $C_{22}$-Fettsäuren. Die Aktivität der im erfindungsgemäßen Verfahren verwendeten Desaturasen und Elongasen führt vorzugsweise zu $C_{18}$-, $C_{20}$- und/oder $C_{22}$-Fettsäuren vorteilhaft mit mindestens zwei Doppelbindungen im Fettsäuremolekül, vorzugsweise mit drei, vier, fünf oder sechs Doppelbindungen, besonders bevorzugt zu $C_{20}$- oder $C_{22}$-Fettsäuren mit mindestens zwei Doppelbindungen im Fettsäuremolekül, vorzugsweise mit drei, vier, fünf oder sechs Doppelbindungen, am meisten bevorzugt mit vier, fünf oder sechs Doppelbindungen im Molekül. Besonders bevorzugte Produkte des erfindungsgemäßen Verfahrens sind Arachidonsäure, Eicosapentaensäure und/oder Docosahexaensäure. Die $C_{18}$-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Durch die Verwendung der erfindungsgemäßen Nukleinsäuren, die für eine Δ-5-Elongase codieren, können im Verfahren die hergestellten mehrfach ungesättigten Fettsäuren mindestens um 5 % , bevorzugt mindestens um 10 %, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Pflanzen prinzipiell auf zwei Arten erhöht werden. Es kann entweder der Pool an freien

mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Ein weiterer erfindungsgemäßer Gegenstand sind isolierte Nukleinsäuresequenzen, die für Polypeptide mit Δ-5-Elongase codieren, wobei die durch die Nukleinsäuresequenzen codierten Δ-5-Elongasen $C_{20}$-Fettsäuren mit mindestens vier Doppelbindungen im Fettsäuremolekül umsetzen; die vorteilhaft letztlich in Diacylglyceride und/oder Triacylglyceride eingebaut werden.

Ein weiterer Erfindungsgegenstand ist somit eine isolierte Nukleinsäuresequenz, die für Polypeptide mit Δ-5-Elongase codiert und die in SEQ ID NO: 197 dargestellte Sequenz hat.

Ein weiterer Erfindungsgegenstand ist eine isolierte Nukleinsäuresequenz, die für Polypeptide mit Δ-6-Elongaseaktivität codiert und die in SEQ ID NO: 199 dargestellte Sequenz hat.

Noch ein weiterer Erfindungsgegenstand ist eine isolierte Nukleinsäuresequenz, die für Polypeptide mit Δ-6-Desaturaseaktivität codiert und die in SEQ ID NO: 201 dargestellte Sequenz hat.

Ebenfalls zu den Erfindungsgegenständen gehört ein rekombinantes Nukleinsäuremolekül, umfassend:

a) eine oder mehrere Kopien eines in Pflanzenzellen, bevorzugt in Samenzellen, aktiven Promotors,
b) mindestens eine Nukleinsäuresequenz mit der in SEQ ID NO: 193 oder SEQ ID NO: 201 dargestellten Sequenz, die für eine Δ-6-Desaturase-Aktivität kodiert,
c) mindestens eine Nukleinsäuresequenz mit der in SEQ ID NO: 11 dargestellten Sequenz, die für eine Δ-5-Desaturase-Aktivität kodiert,
d) mindestens eine Nukleinsäuresequenz mit der in SEQ ID NO: 27 oder SEQ ID NO: 199 dargestellten Sequenz, die für eine Δ-6-Elongase-Aktivität kodiert, und
e) eine oder mehrere Kopien einer Terminatorsequenz.

Vorteilhaft kann in dem rekombinanten vorgenannten Nukleinsäuremolekül noch zusätzlich eine Nukleinsäuresequenz mit der in SEQ ID NO: 195 dargestellten Sequenz, die für eine Δ-12-Desaturase kodiert, enthalten sein.

In einer weiteren vorteilhaften Ausführungsform kann in dem rekombinanten Nukleinsäuremolekül vorteilhaft noch zusätzlich eine Nukleinsäuresequenz mit der in SEQ ID NO: 197 dargestellten Sequenz, die für eine Δ-5-Elongase kodiert, enthalten sein.

Neben diesen genannten Sequenzen können in das rekombinanten Nukleinsäuremolekül noch weitere Biosynthesegene des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen und Fettsäure-Elongase(n) eingebracht werden.

Bevorzugt sind dies Gene des Fettsäure- oder Lipidstoffwechsels ausgewählt aus der Gruppe bestehend aus Δ-4-Desaturase-, Δ-8-Desaturase-, Δ-9-Desaturase- oder Δ-9-Elongase.

Ein weiterer Erfindungsgegenstand sind Genkonstrukte, die die erfindungsgemäßen Nukleinsäuresequenzen SEQ ID NO: 11, SEQ ID NO: 27, SEQ ID NO: 193, SEQ ID NO: 195, SEQ ID NO: 197, SEQ ID NO: 199 oder SEQ ID NO: 201 enthalten, wobei die Nukleinsäure funktionsfähig mit einem oder mehreren Regulationssignalen verbunden ist.

Vorteilhaft stammen alle die im erfindungsgemäßen Verfahren verwendeten Nukleinsäuresequenzen aus einem eukaryontischen Organismus wie einer Pflanze, einem Mikroorganismus wie einer Alge oder einem Tier. Bevorzugt stammen die Nukleinsäuresequenzen aus der Ordnung Salmoniformes, Xenopus oder Ciona, Algen wie Mantoniella, Crypthecodinium, Euglena oder Ostreococcus, Pilzen wie der Gattung Phytophtora oder von Diatomeen wie den Gattungen Thalassiosira oder Phaeodactylum.

Die im Verfahren verwendeten Nukleinsäuresequenzen, die für Proteine mit ω-3-Desaturase-, Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongase-Aktivität codieren, werden vorteilhaft allein oder bevorzugt in Kombination in einer Expressionskassette (= Nukleinsäurekonstrukt), die die Expression der Nukleinsäuren in einer Pflanze, eingebracht. Es kann im Nukleinsäurekonstrukt mehr als eine Nukleinsäuresequenz einer enzymatischen Aktivität wie z.B. einer Δ-12-Desaturase, Δ-4-Desaturase, Δ-5-Desaturase, Δ-6-Desaturase, Δ-5-Elongase, Δ-6-Elongase und/oder ω-3-Desaturase enthalten sein.

Zum Einbringen in die Pflanze werden die im Verfahren verwendeten Nukleinsäuren vorteilhaft einer Amplifikation und Ligation in bekannter Weise, wie oben beschrieben, unterworfen.

Es gibt eine Reihe von Mechanismen, durch die eine Veränderung des erfindungsgemäßen Δ-12-Desaturase-, Δ-5-Elongase-, Δ-6-Elongase, Δ-5-Desaturase-, Δ-4-Desaturase-, Δ-6-Desaturase- und/oder ω-3-Desaturase-Proteins sowie der weiteren im Verfahren verwendeten Proteine wie die Δ-12-Desaturase-, Δ-9-Elongase-, Δ-6-Desaturase-, Δ-8-Desaturase-, Δ-6-Elongase-, Δ-5-Desaturase- oder Δ-4-Desaturase-Proteine möglich ist, so dass die Ausbeute, Produktion und/oder Effizienz der Produktion der vorteilhaft mehrfach ungesättigten Fettsäuren in einer Pflanze bevorzugt in einer Ölfruchtpflanze aufgrund dieses veränderten Proteins direkt beeinflusst werden kann. Die Anzahl oder Aktivität der Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Proteine oder -Gene kann erhöht werden, so dass größere Mengen der Genprodukte und damit letztlich größere Mengen der Verbindungen der allgemeinen Formel I hergestellt werden. Auch eine de novo Synthese in einer Pflanze, der die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Genes in eine Pflanze allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Durch Optimierung der Aktivität oder Erhöhung der Anzahl einer oder mehrerer Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten isolierten Nukleinsäuremoleküle codieren für Proteine oder Teile von diesen, wobei die Proteine oder das einzelne Protein oder Teile davon eine Aminosäuresequenz enthält, die ausreichend homolog zu einer Aminosäuresequenz ist, die in den Sequenzen SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellt ist, so dass die Proteine oder Teile davon noch eine Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Aktivität aufweisen. Vorzugsweise haben die Proteine oder Teile davon, die von dem Nukleinsäuremolekül/den Nukleinsäuremolekülen kodiert wird/werden, noch seine wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen. Vorteilhaft sind die von den Nukleinsäuremolekülen kodierten Proteine zu mindestens etwa 50 %, vorzugsweise mindestens etwa 60 % und stärker bevorzugt mindestens etwa 70 %, 80 % oder 90 % und am stärksten bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr identisch zu den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellten Aminosäuresequenzen. Im Sinne der Erfindung ist unter Homologie oder homolog, Identität oder identisch zu verstehen.

Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet wurden.

Unter wesentlicher enzymatischer Aktivität der im erfindungsgemäßen Verfahren verwendeten Δ-12-Desaturase-, ω-3-Desaturase-, Δ-9-Elongase, Δ-6-Desaturase, Δ-8-Desaturase, Δ-6-Elongase, Δ-5-Desaturase, Δ-5-Elongase oder Δ-4-Desaturase ist zu verstehen, dass sie gegenüber den durch die Sequenz mit SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren, Fettsäureester wie Diacylglyceride und/oder Triacylglyceride in einem Organismus vorteilhaft einer Pflanze oder Pflanzenzelle notwendigen Verbindungen oder am Transport von Molekülen über Membranen teilnehmen können, wobei $C_{18}$-, $C_{20}$- oder $C_{22}$-Kohlenstoffketten im Fettsäuremolekül mit Doppelbindungen an mindestens zwei, vorteilhaft drei, vier, fünf oder sechs Stellen gemeint sind.

Vorteilhaft im Verfahren verwendbare Nukleinsäuren stammen aus Bakterien, Pilzen, Diatomeen, Tieren wie Caenorhabditis oder Oncorhynchus oder Pflanzen wie Algen oder Moosen wie den Gattungen Shewanella, Physcomitrella, Thraustochytrium, Fusarium, Phytophthora, Ceratodon, Mantoniella, Ostreococcus, Isochrysis, Aleurita, Muscarioides, Mortierella, Borago, Phaeodactylum, Crypthecodinium, speziell aus den Gattungen und Arten Oncorhynchus mykiss, Xenopus laevis, Ciona intestinalis, Thalassiosira pseudonona, Mantoniella squamata, Ostreococcus sp., Ostreococcus tauri, Euglena gracilis, Physcomitrella patens, Phytophtora infestans, Fusarium graminaeum, Cryptocodinium cohnii, Ceratodon purpureus, Isochrysis galbana, Aleurita farinosa, Thraustochytrium sp., Muscarioides viallii, Mortierella alpina, Borago officinalis, Phaeodactylum tricornutum, Caenorhabditis elegans oder besonders vorteilhaft aus Oncorhynchus mykiss, Euglena gracilis, Thalassiosira pseudonana oder Crypthecodinium cohnii.

Alternativ können im erfindungsgemäßen Verfahren Nukleotidsequenzen verwendet werden, die für eine Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase codieren und die an eine Nukleotidsequenz, wie in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellt, vorteilhaft unter stringenten Bedingungen hybridisieren.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, eingebracht.

Dabei werden die Nukleinsäuresequenzen, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase codieren, mit einem oder mehreren Regulationssignalen vorteilhafterweise zur Erhöhung der Genexpression funktionell verknüpft. Diese regulatorischen Sequenzen sollen die gezielte Expression der Gene und der Proteinexpression ermöglichen. Dies kann beispielsweise je nach Wirtspflanze bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche

Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Δ-12-Desaturase-, ω-3-Desaturase-, Δ-4-Desaturase-, Δ5-Desaturase-, Δ-6-Desaturase-, Δ-8-Desaturase-, Δ-5-Elongase-, Δ-6-Elongase- und/oder Δ-9-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Vorteilhafte Regulationssequenzen für das neue Verfahren liegen beispielsweise in Promotoren vor, wie den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclininduzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2- oder lpt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Derartige vorteilhafte Promotoren sind weiter oben aufgeführt z.B. der USP-, Vicilin-, Napin-, Oleosin-, Phaseolin-, Bce4-, LegB4-, Lpt2-, lpt1-, Amy32b-, Amy 6-6-, Aleurain- oder Bce4-Promotor.

Darüber hinaus sind auch chemisch induzierbaren Promotor vorteilhaft im erfindungsgemäßen Verfahren nutzbar.

Weitere vorteilhafte Promotoren, die vorteilhaft zur Expression in Soya geeignet sind, sind die Promotoren der β-Conglycinin-α-Untereinheit, der β-Conglycinin-β-Untereinheit, des Kunitz-Trypsininhibitors, des Annexin, des Glysinin, des Albumin 2S, des Legumin A1, des Legumin A2 und der des BD30.

Besonders vorteilhafte Promotoren sind der USP-, LegB4-, Fad3-, SBP-, DC-3- oder Cruciferin820 Promotor.

Vorteilhafte Regulationssequenzen, die für die Expression der im erfindungsgemäßen Verfahren verwendeten

Nukleinsäuresequenzen benutzt werden, sind Terminatoren für die Expression vorteilhaft in Soya sind der Leg2A3', Kti3', Phas3', BD30 3' oder der AIS3'.

Besonders vorteilhafte Terminatoren sind der A7T-, OCS-, LeB3T- oder cat-Terminator.

Um eine stabile Integration der Biosynthesegene in die transgene Pflanze über mehrere Generation sicherzustellen, sollte, wie oben beschrieben, jede der im Verfahren verwendeten Nukleinsäuren, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase und/oder Δ-4-Desaturase codieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Das Genkonstrukt kann, wie oben beschrieben, auch weitere Gene umfassen, die in die Pflanze eingebracht werden sollen.

Die zur Expression der im erfindungsgemäßen Verfahren verwendeten Nukleinsäuren vorteilhaft genutzten regulatorischen Sequenzen bzw. Faktoren können dabei wie oben beschrieben vorzugsweise die Genexpression der eingeführten Gene positiv beeinflussen und dadurch erhöhen.

Diese vorteilhaften Vektoren, vorzugsweise Expressionsvektoren, enthalten die im Verfahren verwendeten Nukleinsäuren, die für die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen oder Δ-4-Desaturasen codieren, oder ein Nukleinsäurekonstrukt, die die verwendeten Nukleinsäure allein oder in Kombination mit weiteren Biosynthesegenen des Fettsäure- oder Lipidstoffwechsels wie den Acyl-CoA:Lysophospholipid-Acyltransferasen, ω-3-Desaturasen, Δ-4-Desaturasen, Δ-5-Desaturasen, Δ-6-Desaturasen, Δ-8-Desatuasen, Δ-9-Desaturasen, Δ-12-Desaturasen, ω3-Desaturasen, Δ-5-Elongasen, Δ-6-Elongasen und/oder Δ-9-Elongasen.

Wie hier verwendet und beschrieben, betrifft der Begriff "Vektor" ein Nukleinsäuremolekül, das eine andere Nukleinsäure transportieren kann, an welche es gebunden ist.

Die verwendeten rekombinanten Expressionsvektoren können zur Expression von Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in prokaryotischen oder eukaryotischen Zellen gestaltet sein. Dies ist vorteilhaft, da häufig Zwischenschritte der Vektorkonstruktion der Einfachheithalber in Mikroorganismen durchgeführt werden. Beispielsweise können die Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten, vorteilhaft zu einfachen Detektion der enzymatischen Aktivität z.B. zum Nachweis der Desaturase- oder Elongaseaktivität, erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174

(DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCI, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Zum Nachweis der Enzymaktivität können die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor

erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Die vorteilhafterweise verwendeten Wirtsorganismen sind Pflanzenzellen vorzugsweise Pflanzen oder Teile davon. Besonders bevorzugt sind Pflanzen, wie Ölsamen- oder Ölfruchtpflanzen, die große Mengen an Lipidverbindungen enthalten, wie Raps, Nachtkerze, Hanf, Diestel, Erdnuss, Canola, Lein, Soja, Saflor, Sareptasenf, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte erfindungsgemäße Pflanzen sind Ölfruchtpflanzen, wie Soja, Erdnuss, Raps, Canola, Lein, Hanf, Nachtkerze, Sonnenblume, Saflor, Bäume (Ölpalme, Kokosnuss).

Ein weiterer erfindungsgemäßer Gegenstand ist, wie oben beschrieben, eine isolierte Nukleinsäuresequenz, die für Polypeptide mit Δ-5-Elongase-Aktivität codiert und die die in in SEQ ID NO: 197 dargestellte Sequenz hat, wobei die durch die Nukleinsäuresequenz codierte Elongase $C_{16}$- und $C_{13}$- Fettsäuren mit einer Doppelbindung nicht elongiert. Auch mehrfach ungesättigte $C_{13}$-Fettsäuren mit einer Δ6-Doppelbindung oder $C_{22}$-Fettsäuren werden nicht umgesetzt. Durch die enzymatische Aktivität werden vorteilhaft nur mehrfach ungesättigte $C_{20}$-Fettsäuren mit einer Δ5-Doppelbindung elongiert. Weitere Erfindungsgegenstände sind, wie oben beschrieben, eine Δ-6-Elongase, Δ-6-Desaturase und eine Δ-12-Desaturase.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und

mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle, z.B. ein Nukleinsäuremolekül mit einer Nukleotidsequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 oder eines Teils davon, kann unter Verwendung molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 gezeigten Sequenzen oder Mithilfe der in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ

ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellten Aminosäuresequenzen erstellen. Eine der vorgenannten Nukleinsäuren kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Nukleinsäuresequenzen mit der Sequenz SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 bedeutet beispielsweise allelische Varianten mit mindestens etwa 50 oder 60 %, vorzugsweise mindestens etwa 60 oder 70 %, stärker bevorzugt mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu einer in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 gezeigten Nukleotidsequenzen oder einen Teil davon hybridisieren, z.B. unter stringenten Bedingungen hybridisiert. Unter einem Teil gemäß der Erfindung ist dabei zu verstehen, dass mindestens 25 Basenpaare (= bp), 50 bp, 75 bp, 100 bp, 125 bp oder 150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus/in der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellten Sequenz erhalten lassen, wobei aber die Absicht ist, dass die

Enzymaktivität der davon herrührenden synthetisierten Proteine für die Insertion eines oder mehrerer Gene vorteilhafterweise beibehalten wird. Proteine, die noch die enzymatische Aktivität der Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 kodierten Protein. Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Standardeinstellungen immer für Sequenzvergleiche verwendet wurden.

Homologen der vorgenannten Nukleinsäuresequenzen bedeuten beispielsweise auch bakterielle, Pilz- und Pflanzenhomologen, verkürzte Sequenzen, einzelsträngige DNA oder RNA der kodierenden und nicht-kodierenden DNA-Sequenz oder auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Aktivität, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola, Sareptasenf und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Besonders zur Herstellung von PUFAs, bevorzugt von Arachidonsäure, Eicosapentaensäure oder Docosahexaensäure, eignen sich Brassicaceen, Boraginaceen, Primulaceen, oder Linaceen. Besonders geeignet zur Herstellung von PUFAs mit den erfindungsgemäßen Nukleinsäuresequenzen, vorteilhaft, wie beschrieben, in Kombination mit weiteren Desaturasen und Elongasen, sind Sareptasenf (*Brassica juncea*), Raps und Camelina sativa.

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Besonders zur Herstellung von PUFAs, beispielsweise Stearidonsäure , Eicosapentaensäure und Docosahexaensäure eignen sich Brasicaceae, Boraginaceen, Primulaceen, oder Linaceen. Besonders vorteilhaft eignet sich Lein (Linum usitatissimum), Brassica juncea und Camelina sativa zur Herstellung von PUFAS mit dem erfindungsgemäßen

Nukleinsäuresequenzen vorteilhaft, wie beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese $C_{18}$-Kohlenstoff-Fettsäuren müssen auf $C_{20}$ und $C_{22}$ verlängert werden, damit Fettsäuren vom Eicosa- und Docosa-Kettentyp erhalten werden. Mithilfe der im Verfahren verwendeten Desaturasen wie der $\Delta$-12-, $\omega$3-, $\Delta$-4-, $\Delta$-5-, $\Delta$-6- und $\Delta$-8-Desaturasen und/oder der $\Delta$-5-, $\Delta$-6-, $\Delta$-9-Elongasen können Arachidonsäure, Eicosapentaensäure, Docosapentaensäure oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können $C_{20}$- und/oder $C_{22}$-Fettsäuren mit mindestens zwei vorteilhaft mindestens drei, vier, fünf oder sechs Doppelbindungen im Fettsäuremolekül, vorzugsweise $C_{20}$- oder $C_{22}$-Fettsäuren mit vorteilhaft vier, fünf oder sechs Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von $C_{20}$ zu $C_{22}$-Fettsäuren,zu Fettsäuren wie $\gamma$-Linolensäure, Dihomo-$\gamma$-linolensäure, Arachidonsäure, Stearidonsäure, Eicosatetraensäure oder Eicosapentaensäure. Substrate der verwendeten Desaturasen und Elongasen im erfindungsgemäßen Verfahren sind $C_{16}$-, $C_{18}$- oder $C_{20}$-Fettsäuren wie zum Beispiel Linolsäure, $\gamma$-Linolensäure, $\alpha$-Linolensäure, Dihomo-$\gamma$-linolensäure, Eicosatetraensäure oder Stearidonsäure. Bevorzugte Substrate sind Linolsäure, $\gamma$-Linolensäure und/oder $\alpha$-Linolensäure, Dihomo-$\gamma$-linolensäure bzw. Arachidonsäure, Eicosatetraensäure oder Eicosapentaensäure. Die synthetisierten $C_{20}$- oder $C_{22}$-Fettsäuren mit mindestens zwei, drei, vier, fünf oder sechs, vorteilhaft mit mindestens vier, fünf oder sechs Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin.

Die Begriffe "Produktion" oder "Produktivität" sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Sie umfassen auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff "Ausbeute" oder "Produkt/Kohlenstoff-Ausbeute" ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht.

Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess.

Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess.

Der Begriff "Stoffwechsel" ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Diese Erfindung wird durch die nachstehenden Beispiele weiter veranschaulicht, die nicht als beschränkend aufgefasst werden sollten. Der Inhalt sämtlicher in dieser Patentanmeldung zitierten Literaturstellen, Patentanmeldungen, Patente und veröffentlichten Patentanmeldungen ist hier durch Bezugnahme aufgenommen.

Beispiele

Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

Beispiel 3: Klonierung von Genen aus Oncorhynchus mykiss

Durch Suche nach konservierten Bereichen in den Proteinsequenzen entsprechend der in der Anmeldung aufgeführten Elongase-Gene wurden zwei Sequenzen mit entsprechenden Motiven in der Sequenzdatenbank von Genbank identifiziert.

| Gen-Name | Genbank No | Aminosäuren |
|---|---|---|
| OmELO2 | CA385234, CA364848, CA366480 | 264 |
| OmELO3 | CA360014, CA350786 | 295 |

Gesamt-RNA von Oncoryhnchus mykiss wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend Herstellerangaben wurde die cDNA zur Plasmid-DNA entpackt. Die cDNA-Plasmid-Bank wurde dann für die PCR zur Klonierung von Expressionsplasmiden verwendet.

Beispiel 4: Klonierung von Expressionsplasmiden zur heterologen Expression in Hefen

Für die Klonierung der zwei Sequenzen zur heterologen Expression in Hefen wurden folgende Oligonukleotide für die PCR-Reaktion verwendet:

| Primer | Nukleotidsequenz |
|---|---|
| 5' f* OmELO2 | 5' aagcttacataatggcttcaacatggcaa (SEQ ID NO: 179) |
| 3' r* OmELO2 | 5' ggatccttatgtcttcttgctcttcctgtt (SEQ ID NO: 180) |
| 5'f OmELO3 | 5' aagcttacataatggagacttttaat (SEQ ID NO: 181) |
| 3' r OmELO3 | 5' ggatccttcagtcccccctcactttcc (SEQ ID NO: 182) |
| * f: forward, r: reverse | |

Zusammensetzung des PCR-Ansatzes (50 μL):

5,00 μL Template cDNA

5,00 μL 10x Puffer (Advantage-Polymerase)+ 25mM MgCl2

5,00 μL 2mM dNTP

1,25 μL je Primer (10 pmol/μL)

0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 2 h bei 37 °C mit den Restriktionsenzymen HindIII und BamHI inkubiert. Der Hefe-Expressionsvektor pYES3 (Invitrogen) wurde in gleicherweise inkubiert. Anschliessend wurde das 812 bp bzw. 905 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und Elongase cDNA ligiert. Dazu wurde das Rapid Ligation Kit von Roche verwendet. Die entstandenen Plasmide pYES3-OmELO2 und pYES3-OmELO3 wurden durch Sequenzierung verifiziert und in den Saccharomyces Stamm INVSc1 (Invitrogen) durch Elektroporation (1500 V) transformiert. Zur Kontrolle wurde pYES3 parallel transformiert. Anschliessend wurden die Hefen auf Komplett-Minimalmedium ohne Tryptophan mit 2 % Glucose ausplattiert. Zellen, die auf ohne Tryptophan im Medium wachstumsfähig waren, enthalten damit die entsprechenden Plasmide pYES3, pYES3-OmELO2 (SEQ ID NO: 51) und pYES3-OmELO3 (SEQ ID NO: 53). Nach der Selektion wurden je zwei Transformaten zur weiteren funktionellen Expression ausgewählt.

Beispiel 5: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende des kodierenden Sequenz eingefügt:

PSUN-OmELO2

Forward: 5'-GCGGCCGCATAATGGCTTCAACATGGCAA (SEQ ID NO: 175)
Reverse: 3'-GCGGCCGCTTATGTCTTCTTGCTCTTCCTGTT (SEQ ID NO: 176)

PSUN-OMELO3

Forward: 5'-GCGGCCGCataatggagacttttaat (SEQ ID NO: 177)
Reverse: 3'-GCGGCCGCtcagtcccccctcactttcc (SEQ ID NO: 178)

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und PCR-Produkte ligiert. Dazu wurde das Rapid Ligation Kit von Roche verwendet. Die entstandenen Plasmide pSUN-OmELO2 und pSUN-OmELO3 wurde durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standard-primer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 174). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.

Beispiel 6: Lipidextraktion aus Hefen und Samen:

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter,

P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Hefen, die wie unter Beispiel 4 mit den Plasmiden pYES3, pYES3-OmELO2 und pYES3-OmELO3 transformiert wurden, wurden folgendermaßen analysiert:

Die Hefezellen aus den Hauptkulturen wurden durch Zentrifugation (100 x g, 10 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1N methonolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma).

Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 7: Funktionelle Charakterisierung von OmELO2 und OmELO3:

OmELO2 zeigt keine Elongase-Aktivität, während für OmELO3 eine deutliche Aktivität mit verschiedenen Substraten nachgewiesen werden konnte. Die Substratspezifität der OmElo3 konnte nach Expression und Fütterung verschiedener Fettsäuren ermittelt werden (Figur 2). Die gefütterten Substrate sind in großen Mengen in allen transgenen Hefen nachzuweisen. Alle transgene Hefen zeigen die Synthese neuer Fettsäuren, den Produkten der OmElo3-Reaktion. Dies bedeutet, dass das Gen OmElo3 funktional exprimiert werden konnte.

Figur 2 zeigt, dass die OmElo3 eine Substratspezifität aufweist, die mit hoher Spezifität zur Verlängerung von Δ5- und Δ6-Fettsäuren mit einer ω3-Doppelbindung führt. Es konnte in geringerer Spezifität des weiteren auch ω6-Fettsäuren (C18 und C20) elongiert werden. Stearidonsäure (C18:4 ω3) und Eicosapentaensäure (C20:5 ω3) stellen die besten Substrate für die OmElo3 dar (bis zu 66 % Elongation).

Beispiel 8: Rekonstitution der Synthese von DHA in Hefe

Die Rekonstitution der Biosynthese von DHA (22:6 ω3) wurde ausgehend von EPA (20:5 ω3) bzw. Stearidonsäure (18:4 ω3) durch die Coexpression der OmElo3 mit der Δ-4-Desaturase aus *Euglena gracilis* bzw. der Δ-5-Desaturase aus *Phaeodactylum tricornutum* und der Δ-4-Desaturase aus *Euglena gracilis* durchgeführt. Dazu wurden weiterhin die Expressionsvektoren pYes2-EgD4 und pESCLeu-PtD5 konstruiert. Der o.g. Hefestamm, der bereits mit dem pYes3-OmElo3 (SEQ ID NO: 55) transformiert ist, wurde weiter mit dem pYes2-EgD4 bzw. gleichzeitig mit pYes2-EgD4 und pESCLeu-PtD5 transformiert. Die Selektion der transformierten Hefen erfolgte auf Komplett-Minimalmedium-Agarplatten mit 2% Glucose, aber ohne Tryptophan und Uracil im Falle des pYes3-OmELO/pYes2-EgD4-Stammes und ohne Tryptophan, Uracil und Leucin im Falle des pYes3-OmELO/pYes2-EgD4+pESCLeu-PtD5-Stammes. Die Expression wurde wie oben angegeben durch die Zugabe von 2% (w/v) Galactose induziert. Die Kulturen wurden für weitere 120 h bei 15°C inkubiert.

Figur 3 zeigt die Fettsäureprofile von transgenen Hefen, die mit 20:5 ω3 gefüttert wurden. In der Kontroll-Hefe (A), die mit dem pYes3-OmElo3-Vektor und dem leeren Vektor pYes2 transformiert wurden, wurde 20:5 ω3 sehr effizient zu 22:5 ω3 elongiert (65% Elongation). Die zusätzliche Einführung der EgΔ-4-Desaturase führte zu der Umsetzung von 22:5 ω3 zu 22:6 ω3 (DHA). Die Fettsäure-Zusammensetzung der transgenen Hefen ist in Figur 5 wiedergegeben. Nach der Co-Expression von OmElo3 und EgD4 konnte bis zu 3% DHA in Hefen nachgewiesen werden.

In einem weiteren Co-Expressionsexperiment wurden OmElo3, EgD4 und eine□ Δ5-Desaturase aus *P. tricornutum* (PtD5) zusammen exprimiert. Die transgenen Hefen wurden mit Stearidonsäure (18:4 ω3) gefüttert und analysiert (Figur 4). Die Fettsäure-Zusammensetzung dieser Hefen ist in Figur 5 aufgeführt. Durch OmElo3 wurde die gefütterte Fettsäure 18:4 ω3 zu 20:4 ω3 elongiert (60% Elongation). Letztere wurde durch die PtD5 zu 20:5 ω3 desaturiert. Die Aktivität der PtD5 betrug 15%. 20:5 ω3 konnte weiterhin durch die OmElo3 zu 22:5 ω3 elongiert werden. Im Anschluß wurde die neu synthetisierte 22:5 ω3 zu 22:6 ω3 (DHA) desaturiert. In diesen Experimenten konnte bis zu 0,7% DHA erzielt werden.

Aus diesen Experimenten geht hervor, dass die in dieser Erfindung verwendeten Sequenzen OmElo3, EgD4 und PtD5 für die Produktion von DHA in eukaryotischen Zellen geeignet sind.

Beispiel 9: Erzeugung von transgenen Pflanzen

a) Erzeugung transgener Rapspflanzen (verändert nach Moloney et al., 1992, Plant Cell Reports, 8:238-242)

Zur Erzeugung transgener Rapspflanzen können binäre Vektoren in Agrobacterium tumefaciens C58C1:pGV2260 oder Escherichia coli genutzt (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 $cm^2$) werden in einer Petrischale mit einer 1:50 Agrobakterienverdünnung für 5-10 Minuten inkubiert. Es folgt eine 3-tägige CoInkubation in Dunkelheit bei 25°C auf 3MS-Medium mit 0,8 % Bacto-Agar. Die Kultivierung wird nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse werden auf MS-Medium mit

2 % Saccharose, 250 mg/l Claforan und 0,8 % Bacto-Agar überführt. Bilden sich nach drei Wochen keine Wurzeln, so wurde als Wachstumshormon 2-Indolbuttersäure zum Bewurzeln zum Medium gegeben.

Regenerierte Sprosse werden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Elongase-Expression wie Δ-5-Elongase- oder Δ-6-Elongaseaktivität oder ω-3-Desaturaseaktivität mittels Lipidanalysen untersucht. Linien mit erhöhten Gehalten an C20- und C22 mehrfachungesättigten Fettsäuren können so identifiziert werden.

b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. In der Regel wurde eine Agrobakterien-vermittelte Transformation zum Beispiel nach Mlynarova et al. (1994), Plant Cell Report 13: 282-285 zur Leintransformation verwendet.

Beispiel 10: Klonierung von Δ5-Elongase-Genen aus Thraustochytrium aureum ATCC34304 und Thraustochytrium ssp.

Durch Vergleiche der verschiedenen in dieser Anmeldung gefundenen Elongase-Proteinsequenzen konnten konservierte Nukleinsäurebereiche definiert werden (Histidin-Box: His-Val-X-His-His, Tyrosin-Box: Met-Tyr-X-Tyr-Tyr). Mit Hilfe dieser Sequenzen wurde eine EST-Datenbank von T. aureum ATCC34304 und Thraustochytrium ssp. nach weiteren Δ-5-Elongasen durchsucht. Folgende neue Sequenzen konnten gefunden werden:

| Gen-Name | Nukleotide | Aminosäuren |
|---|---|---|
| BioTaurELO1 | 828 bp | 275 |
| TL16y2 | 831 | 276 |

Gesamt-RNA von T. aureum ATCC34304 und Thraustochytrium ssp. wurde mit Hilfe des RNAeasy Kits der Firma Qiagen (Valencia, CA, US) isoliert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) reverse transkribiert und entsprechend der Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Beispiel 11: Klonierung von Expressionsplasmiden zur heterologen Expression in Hefen

Für die Klonierung der Sequenz zur heterologen Expression in Hefen wurden folgende Oligonukleotide für die PCR-Reaktion verwendet:

| Primer | Nukleotidsequenz |
|---|---|
| 5' f* BioTaurELO1 | 5' gacataatgacgagcaacatgag (SEQ ID NO: 170) |
| 3' r* BioTaurELO1 | 5' cggcttaggccgacttggccttggg (SEQ ID NO: 171) |
| 5'f*TL16y2 | 5' agacataatggacgtcgtcgagcagcaatg (SEQ ID NO: 172) |
| 3'r*TL16y2 | 5' ttagatggtcttctgcttcttgggcgcc (SEQ ID NO: 173) |
| * f: forward, r: reverse | |

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL pfu-Polymerase

Die Advantage-Polymerase von Clontech wurde eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte BioTaurELO1 (siehe SEQ ID NO: 65) und TL16y2 (siehe SEQ ID NO: 83) wurde für 30 min bei 21 °C mit dem Hefe-Expressionsvektor pYES2.1-TOPO (Invitrogen) inkubiert gemäss Herstellerangaben. Das PCR-Produkt wird dabei durch einen T-Überhang und Aktivität einer Topoisomerase (Invitrogen) in den Vektor ligiert. Nach der Inkubation erfolgte dann die Transformation von E. coli DH5a Zellen. Entsprechende Klone wurden durch PCR identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-BioTaurELO1 und pYES2.1-TL16y2. Nach der Selektion wurden je zwei Transformaten zur weiteren funktionellen Expression ausgewählt.

Beispiel 12: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende des kodierenden Sequenz eingefügt:

PSUN-BioTaurEL01

Forward: 5'-GCGGCCGCATAATGACGAGCAACATGAGC (SEQ ID NO: 166)
Reverse: 3'-GCGGCCGCTTAGGCCGACTTGGCCTTGGG (SEQ ID NO: 167)

PSUN-TL16y2

Forward: 5'-GCGGCCGCACCATGGACGTCGTCGAGCAGCAATG (SEQ ID NO: 168)
Reverse: 5'-GCGGCCGCTTAGATGGTCTTCTGCTTCTTGGGCGCC
(SEQ ID NO: 169)

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel Purification Kit gemäss Herstellerangaben. Anschließend wurden Vektor und PCR-Produkte ligiert. Dazu wurde das Rapid Ligation Kit von Roche verwendet. Die entstandenen Plasmide pSUN-BioTaurELO1 und pSUN-TL16y2 wurden durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal

ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standard-primer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 165). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Die Lipidextraktion aus Hefen und Samen erfolgte identisch zu Beispiel 6.

Beispiel 13: Funktionelle Charakterisierung von BioTaurELO1 und TL16y2:

Die Substratspezifität der BioTaurELO1 konnte nach Expression und Fütterung verschiedener Fettsäuren ermittelt werden (Figur 6). Figur 6 zeigt die Fütterungsexperimente zur Bestimmung der Funktionalität und Substratspezifität mit Hefestämmen, die entweder den Vektor pYes2.1 (Kontrolle = Control) oder den Vektor pYes2.1-BioTaurELO1 (= BioTaur) mit der Δ-5-Elongase enthalten. In beiden Ansätzen wurde 200 uM γ-Linolensäure und Eicosapentaensäure dem Hefeinkubationsmedium zugesetzt und 24 h inkubiert. Nach Extraktion der Fettsäuren aus den Hefen wurden diese transmethyliert und gaschromatographisch aufgetrennt. Die aus den beiden gefütterten Fettsäuren entstandenen Elongationsprodukte sind durch Pfeile markiert.

Die gefütterten Substrate sind in großen Mengen in allen transgenen Hefen nachzuweisen. Alle transgene Hefen zeigen die Synthese neuer Fettsäuren, den Produkten der BioTaurELO1-Reaktion. Dies bedeutet, dass das Gen BioTaurELO1 funktional exprimiert werden konnte.

Figur 6 zeigt, dass die BioTaurELO1 eine Substratspezifität aufweist, die mit hoher Spezifität zur Verlängerung von Δ5- und Δ6-Fettsäuren mit einer ω3-Doppelbindung führt. Des weiteren konnten auch ω6-Fettsäuren (C18 und C20) elongiert werden. Es werden γ-Linolensäure (C18:3 ω6) mit 65,28 %, Stearidonsäure (C18:4 ω3) mit 65.66 % und Eicosapentaensäure (C20:5 ω3) mit 22,01 % Konversion umgesetzt. Die Substratspezifitäten der verschiedenen Fütterungsexperimente sind in Tabelle 6 dargestellt (siehe am Ende der Beschreibung).

Die Konversionsrate von GLA bei Fütterung von GLA und EPA betrug 65,28 %. Die Konversionsrate von EPA bei gleicher Fütterung von GLA und EPA betrug 9,99 %. Wurde nur EPA gefüttert, so betrug die Konversionsrate von EPA 22,01 %. Auch Arachidonsäure (= ARA) wurde bei Fütterung umgesetzt. Die Konversionsrate betrug 14,47 %. Auch Stearidonsäure (= SDA) wurde umgesetzt. In diesem Fall betrug die Konversionsrate 65,66 %.

Die Funktionalität und Substratspezifität von TL16y2 konnte nach Expression und Fütterung verschiedener Fettsäuren ermittelt werden. Tabelle 7 zeigt die Fütterungsexperimente. Die Fütterungsversuche wurden in gleicherweise durchgeführt wie für BioTaurELO1 beschrieben. Die gefütterten Substrate sind in großen Mengen in allen transgenen Hefen nachzuweisen. Die transgenen Hefen zeigten die Synthese neuer Fettsäuren, den Produkten der TL16y2-Reaktion (Fig. 11). Dies bedeutet, dass das Gen TL16y2 funktional exprimiert werden konnte.

Tabelle 7: Expression von TL16y2 in Hefe.

| Flächen der gaschromatographischen Analyse in % | | | | | | | | | |
|---|---|---|---|---|---|---|---|---|---|
| Plasmid | Fettsäure | C18:3 (n-6) | C18:4 (n-3) | C20:3 (n-6) | C20:4 (n-6) | C20:4 (n-3) | C20:5 (n-3) | C22:4 (n-6) | C22:5 (n-3) |
| pYES | 250 uM EPA | | | | | | 13,79 | | |
| TL16y2 | 250 uM EPA | | | | | | 25,81 | | **2,25** |
| pYES | 50 uM EPA | | | | | | 5,07 | | |
| TL16y2 | 50 uM EPA | | | | | | 2,48 | | **1,73** |
| pYES | 250 uMGLA | 8,31 | | | | | | | |

(fortgesetzt)

| Flächen der gaschromatographischen Analyse in % | | | | | | | | | |
|---|---|---|---|---|---|---|---|---|---|
| Plasmid | Fettsäure | C18:3 (n-6) | C18:4 (n-3) | C20:3 (n-6) | C20:4 (n-6) | C20:4 (n-3) | C20:5 (n-3) | C22:4 (n-6) | C22:5 (n-3) |
| TL16y2 | 250 uM GLA | 3,59 | | **10,71** | | | | | |
| pYES | 250 uM ARA | | | | 16,03 | | | | |
| TL16y2 | 250 uM ARA | | | | 15,2 | | **3,87** | | |
| pYES | 250 uM SDA | | 26,79 | | | 0,35 | | | |
| TL16y2 | 250 uM SDA | | 7,74 | | | **29,17** | | | |

Die in Tabelle 7 wiedergegebenen Ergebnisse zeigen mit TL16y2 gegenüber der Kontrolle folgende prozentuale Umsätze: a) % Umsatz EPA (250 uM): 8 %, b) % Umsatz EPA (50 uM): 41 %, c) % Umsatz ARA: 20,3 %, d) % Umsatz SDA: 79, 4% und e) % Umsatz GLA: 74,9 %.

TL16y2 zeigt damit Δ5-, Δ6- und Δ8-Elongaseaktivität. Dabei ist die Aktivität für C18-Fettsäuren mit Δ6-Doppelbindung am höchsten. Abhängig von der Konzentration an gefütterten Fettsäuren werden dann C20-Fettsäuren mit einer Δ5- bzw. Δ8-Doppelbindung verlängert.

Beispiel 14: Klonierung von Genen aus Ostreococcus tauri

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten zwei Sequenzen mit entsprechenden Motiven in einer Ostreococcus tauri Sequenzdatenbank (genomische Sequenzen) identifiziert werden.

Es handelt sich dabei um die folgenden Sequenzen:

| Gen-Name | SEQ ID | Aminosäuren |
|---|---|---|
| OtELO1, (Δ-5-Elongase) | SEQ ID NO: 67 | 300 |
| OtELO2, (Δ-6-Elongase) | SEQ ID NO: 69 | 292 |

OtElo1 weist die höchste Ähnlichkeit zu einer Elongase aus Danio rerio auf (GenBank AAN77156; ca. 26 % Identität), während OtElo2 die größte Ähnlichkeit zur Physcomitrella Elo (PSE) [ca. 36 % Identität] aufweist (Alignments wurden mit dem tBLASTn-Aalgorithmus (Altschul et al., J. Mol. Biol. 1990, 215: 403 - 410) durchgeführt.

Die Klonierung wurde wie folgt durchgeführt:

40 ml einer *Ostreococcus tauri* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtElo-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel 15: Klonierung von Expressionsplasmiden zur heterologen Expression in Hefen:

Zur Charakterisierung der Funktion der Elongasen aus *Ostreococcus tauri* wurden die offenen Leserahmen der jeweiligen DNAs stromabwärts des Galactose-induzierbaren GAL1-Promotors von pYES2.1/V5-His-TOPO (Invitrogen) kloniert, wobei pOTE1 und pOTE2 erhalten wurden.

Der *Saccharomyces cerevisiae*-Stamm 334 wurde durch Elektroporation (1500 V) mit dem Vektor pOTE1 bzw.

pOTE2 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 16: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurden mittels PCR Notl-Schnittstellen am 5' und 3'-Ende der kodierenden Sequenzen eingefügt. Die entsprechenden Primersequenzen wurden von den 5'- und 3-Bereich von OtElo1 und OtElo2 abgeleitet.

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym Notl inkubiert. Der Pflanzen-Expressionsvektor pSUN300-USP wurde in gleicherweise inkubiert. Anschliessend wurde die PCR Produkte sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäss Herstellerangaben. Anschliessend wurden Vektor und PCR-Produkte ligiert. Dazu wurde das Rapid Ligation Kit von Roche verwendet. Die entstandenen Plasmide pSUN-OtELO1 und pSUN-OtELO2 wurde durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz:
5'–GTCGACCCGCGGACTAGTGGGCCCTCTAGACCCGGGGGATCC
GGATCTGCTGGCTATGAA–3', SEQ ID NO: 164).

Das PCR-Fragment wurde mit EcoRI/Sall nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel 17: Expression von OtELO1 und OtELO2 in Hefen

Hefen, die wie unter Beispiel 15 mit den Plasmiden pYES3, pYES3-OtELO1 und pYES3-OtELO2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 18: Funktionelle Charakterisierung von OtELO1 und OtELO2:

Die Substratspezifität der OtElo1 konnte nach Expression und Fütterung verschiedener Fettsäuren ermittelt werden (Tab.8). Die gefütterten Substrate sind in großen Mengen in allen transgenen Hefen nachzuweisen. Die transgenen Hefen zeigten die Synthese neuer Fettsäuren, den Produkten der OtElo1-Reaktion. Dies bedeutet, dass das Gen OtElo1 funktional exprimiert werden konnte.

Tabelle 7 zeigt, dass die OtElo1 eine enge Substratspezifität aufweist. Die OtElo1 konnte nur die C20-Fettsäuren Eicosapentaensäure (Figur 7) und Arachidonsäure (Figur 8) elongieren, bevorzugte aber die ω-3-desaturierte Eicosapentaensäure.

Tabelle 8:

| Fettsäuresubstrat | Umsatz (in %) |
|---|---|
| 16:0□ | - |
| 16:1$^{□\Delta9}$ | - |
| 18:0□ | - |
| 18:1$^{□\Delta9}$ | - |
| 18;1$^{\Delta11}$ | - |
| 18:2$^{\Delta9,12}$ | - |
| 18:3$^{□\Delta6,9,12}$ | - |
| 1 8;3$^{□\Delta5,9,12}$ | - |
| 20:3 $^{\Delta8,11,14}$ | - |
| 20:4 $^{\Delta5,□8,11,14}$ | **10,8 ± 0,6** |
| 20:5 $^{\Delta5,□8,11,14,17}$ | **46,8 ± 3,6** |
| 22:4 $^{\Delta7,□10,13,16}$ | - |
| 22:6$^{□\Delta4,7,10,13,16,19}$ | - |

Tabelle 8 zeigt die Substratspezifität der Elongase OtElo1 für C20 polyungesättigte Fettsäuren mit einer Doppelbindung in Δ5 Position gegenüber verschiedenen Fettsäuren.

Die Hefen, die mit dem Vektor pOTE1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder.

Die Substratspezifität der OtElo2 (SEQ ID NO: 81) konnte nach Expression und Fütterung verschiedener Fettsäuren ermittelt werden (Tab. 9). Die gefütterten Substrate sind in großen Mengen in allen transgenen Hefen nachzuweisen. Die transgenen Hefen zeigten die Synthese neuer Fettsäuren, den Produkten der OtElo2-Reaktion. Dies bedeutet, dass das Gen OtElo2 funktional exprimiert werden konnte.

Tabelle 9:

| Fettsäuresubstrat | Umsatz (in %) |
|---|---|
| 16:0 | - |
| 16:1$^{\square\Delta 9}$ | - |
| 16:3$^{\square\Delta 7,10,13}$ | - |
| 18:0$^{\square}$ | - |
| 18:1 $^{\square\Delta 6}$ | - |
| 18:1$^{\square A9}$ | - |
| 18:1$^{\square\Delta 11}$ | - |
| 18:2$^{\square\Delta 9,12}$ | - |
| 18:3$^{\square\Delta 6,9,12}$ | **15,3**± |
| 18:3$^{\square\ \Delta 5,9,12}$ | - |
| 18:4$^{\square\Delta 6,9,12,15}$ | **21,1**± |
| 20:2 $^{\Delta 11,14}$ | - |
| 20:3 $^{\Delta 8,11,14}$ | - |
| 20:4 $^{\Delta 5,\square 8,11,14}$ | - |
| 20:5 $^{\Delta 5,\square 8,11,14,17}$ | - |
| 22:4 $^{\Delta 7,\square 10,13,16}$ | - |
| 22:5 $^{\Delta 7,\square 10,13,16,19}$ | - |
| 22:6$^{\square\Delta 4,7,10,13,16,19}$ | - |

Tabelle 9 zeigt die Substratspezifität der Elongase OtElo2 gegenüber verschiedenen Fettsäuren.

Die Hefen, die mit dem Vektor pOTE2 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder.

Die enzymatische Aktivität, die in Tabelle 9 wiedergegeben wird, zeigt klar, dass OTELO2 eine Δ-6-Elongase ist.

Beispiel 19: Klonierung von Genen aus Thalassiosira pseudonana

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten zwei Sequenzen mit entsprechenden Motiven in einer Thalassiosira pseudonana Sequenzdatenbank (genomische Sequenzen) identifiziert werden. Es handelt sich dabei um die folgenden Sequenzen:

| Gen-Name | SEQ ID | Aminosäuren |
|---|---|---|
| TpELO1 (Δ5-Elongase) | 43 | 358 |
| TpELO2 (Δ5-Elongase) | 59 | 358 |
| TpELO3 (Δ6-Elongase) | 45 | 272 |

Eine 2 L Kultur von T. pseudonana wurde in f/2 Medium (Guillard, R.R.L. 1975. Culture of phytoplankton for feeding marine invertebrates. In Culture of Marine Invertebrate Animals (Eds. Smith, W.L. and Chanley, M.H.), Plenum

Press, New York, pp 29-60.) für 14 d (= Tage) bei einer Lichtstärke von 80 E/cm$^2$ angezogen. Nach Zentrifugation der Zellen wurde RNA mit Hilfe des RNAeasy Kits der Firma Quiagen (Valencia, CA, US) nach Herstellerangaben isoliert. Die mRNA wurde mit dem Marathon cDNA Amplification-Kit (BD Biosciences) reverse transkribiert und entsprechend den Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Beispiel 20: Klonierung von Expressionsplasmiden zur heterologen Expression in - Hefen

Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der TpElo-DNAs wurde jeweils mit 1 µL cDNA, 200 µM dNTPs, 2,5 U Advantage-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name und SEQ ID NO: | Primersequenz |
|---|---|
| TpELO1 (Δ5-Elongase), SEQ ID NO: 59 | F:5'-accatgtgctcaccaccgccgtc (SEQ ID NO: 158)<br>R:5'- ctacatggcaccagtaac (SEQ ID NO: 159) |
| TpELO2 (Δ5-Elongase), SEQ ID NO: 85 | F:5'-accatgtgctcatcaccgccgtc (SEQ ID NO: 160)<br>R:5'-ctacatggcaccagtaac (SEQ ID NO: 161) |
| TpELO3 (Δ6-Elongase), SEQ ID NO:45 | F:5'-accatggacgcctacaacgctgc (SEQ ID NO: 162)<br>R:5'- ctaagcactcttcttcttt (SEQ ID NO: 163) |
| *F=forward primer, R=reverse primer | |

Die PCR Produkte wurde für 30 min bei 21 °C mit dem Hefe-Expressionsvektor - pYES2.1-TOPO (Invitrogen) gemäß Herstellerangaben inkubiert. Das PCR-Produkt wird dabei durch einen T-Überhang und Aktivität einer Topoisomerase (Invitrogen) in den Vektor ligiert. Nach der Inkubation erfolgte dann die Transformation von E. coli DH5a Zellen. Entsprechende Klone wurden durch PCR identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-TpELO1, pYES2.1-TpELO2 und pYES2.1-TpELO3. Nach der Selektion wurden je zwei Transformaten zur weiteren funktionellen Expression ausgewählt.

Beispiel 21: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu wird mit folgendem Primerpaar NotI-Schnittstellen am 5' und 3'-Ende der kodierenden Sequenz eingefügt:.

PSUN-TPELO1

Forward: 5'-GCGGCCGCACCATGTGCTCACCACCGCCGTC (SEQ ID NO: 152)
Reverse: 3'-GCGGCCGCCTACATGGCACCAGTAAC (SEQ ID NO: 153)

PSUN-TPELO2

Forward: 5'-GCGGCCGCACCATGTGCTCATCACCGCCGTC (SEQ ID NO: 154)
Reverse: 3'-GCGGCCGCCTACATGGCACCAGTAAC (SEQ ID NO: 155)

PSUN-TPELO3

Forward: 5'-GCGGCCGCaccatggacgcctacaacgctgc (SEQ ID NO: 156)

Reverse: 3'-GCGGCCGCCTAAGCACTCTTCTTCTTT (SEQ ID NO: 157)

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschließend werden Vektor und PCR-Produkte ligiert. Dazu wird das Rapid Ligation Kit von Roche verwendet. Die entstandenen Plasmide pSUN-TPEL01, pSUN-TPELO2 und pSUN-TPELO3 werden durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–
GTCGACCCGCGGACTAGTGGGCCCTCTAGACCCGGGGGATCC
GGATCTGCTGGCTATGAA–3'; SEQ ID NO: 151).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 22: Expression von TpELO1, TpELO2 und TpELO3 in Hefen

Hefen, die wie unter Beispiel 4 mit den Plasmiden pYES2, pYES2-TpELO1, pYES2-TpELO2 und pYES2-TpELO3 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen

mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 23: Funktionelle Charakterisierung von TpELO1 und TpELO3:

Die Substratspezifität der TpElo1 konnte nach Expression und Fütterung verschiedener Fettsäuren ermittelt werden (Fig. 9). Die gefütterten Substrate sind in großen Mengen in allen transgenen Hefen nachzuweisen. Die transgenen Hefen zeigten die Synthese neuer Fettsäuren, den Produkten der TpElo1-Reaktion. Dies bedeutet, dass das Gen TpElo1 funktional exprimiert werden konnte.

Tabelle 10 zeigt, dass die TpElo1 eine enge Substratspezifität aufweist. Die TpElo1 konnte nur die C20-Fettsäuren Eicosapentaensäure und Arachidonsäure elongieren, bevorzugte aber die ω-3-desaturierte Eicosapentaensäure.

Die Hefen, die mit dem Vektor pYES2-TpELO1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 10: Expression von TpELO1 in Hefe. In den Spalten 1 und 3 sind die Kontrolreaktionen für die Spalten 2 (gefüttert 250 μM 20:4 Δ5,8,11,14) und 4 (gefüttert 250 μM 20:5 Δ5,8,11,14,17) wiedergegeben.

| | Expression | Expression | Expression | Expression |
|---|---|---|---|---|
| Fettsäuren | 1 | 2 | 3 | 4 |
| 16:0 | 18.8 | 17.8 | 25.4 | 25.2 |
| $16:1^{\Delta 9}$ | 28.0 | 29.8 | 36.6 | 36.6 |
| 18:0 | 5.2 | 5.0 | 6.8 | 6.9 |
| $18:1^{\Delta 9}$ | 25.5 | 23.6 | 24.6 | 23.9 |
| $20:4^{\Delta 5,8,11,14}$ | **22.5** | **23.4** | - | - |
| $22:4^{\Delta 7,10,13,16}$ | **-** | **0.4** | - | - |
| $20:5^{\Delta 5,8,11,14,17}$ | - | - | **6.6** | **6.5** |
| $22:5^{\Delta 7,10,13,16,19}$ | - | - | - | **0.9** |
| **% Umsatz** | **0** | **1.7** | **0** | **12.2** |

Die Substratspezifität der TpElo3 konnte nach Expression und Fütterung verschiedener Fettsäuren ermittelt werden (Fig. 10). Die gefütterten Substrate sind in großen Mengen in allen transgenen Hefen nachzuweisen. Die transgenen Hefen zeigten die Synthese neuer Fettsäuren, den Produkten der TpElo3-Reaktion. Dies bedeutet, dass das Gen TpElo3 funktional exprimiert werden konnte.

Tabelle 11 zeigt, dass die TpElo3 eine enge Substratspezifität aufweist. Die TpElo3 konnte nur die C18-Fettsäuren γ-Linolensäure und Stearidonsäure elongieren, bevorzugte aber die ω-3-desaturierte Stearidonsäure.

Die Hefen, die mit dem Vektor pYES2-TpELO3 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 11: Expression von TpELO3 in Hefe. Spalte 1 zeigt das Fettsäureprofil von Hefe ohne Fütterung. Spalte 2 zeigt die Kontrollreaktion. In den Spalten 3 bis 6 wurden γ-Linolensäure, Stearidonsäure, Arachidonsäure und Eicosapentaensäure gefüttert (250 μM jeder Fettsäure).

| Fettsäuren | 1 | 2 | 3 | 4 | 5 | 6 |
|---|---|---|---|---|---|---|
| 16:0 | 17.9 | 20.6 | 17.8 | 16.7 | 18.8 | 18.8 |
| $16:1^{\Delta 9}$ | 41.7 | 18.7 | 27.0 | 33.2 | 24.0 | 31.3 |
| 18:0 | 7.0 | 7.7 | 6.4 | 6.6 | 5.2 | 6,0 |

(fortgesetzt)

| Fettsäuren | 1 | 2 | 3 | 4 | 5 | 6 |
|---|---|---|---|---|---|---|
| $18:1^{\Delta9}$ | 33.3 | 16.8 | 24.2 | 31.8 | 25.5 | 26.4 |
| $18:2^{\Delta9,12}$ | - | **36.1** | - | - | - | - |
| $18:3^{\Delta6,9,12}$ | - | - | **6.1** | - | - | |
| $18:4^{\Delta6,9,12,15}$ | - | - | - | **1.7** | - | |
| $20:2^{\Delta11,14}$ | - | **0** | - | - | - | |
| $20:3^{\Delta8,11,14}$ | - | - | **18.5** | - | - | |
| $20:4^{\Delta8,11,14,17}$ | - | - | - | **10.0** | - | |
| $20:4^{\Delta5,8,11,14}$ | - | - | - | - | **22.5** | |
| $22:4^{\Delta7,10,13,16}$ | - | - | - | - | **0** | |
| $20:5^{\Delta5,8,11,14,17}$ | - | - | - | - | - | **17.4** |
| $22:5^{\Delta7,10,13,16,19}$ | - | - | - | - | - | **0** |
| **% Umsatz** | **0** | **0** | **75** | **85** | **0** | **0** |

Beispiel 24: Klonierung eines Expressionsplasmides zur heterologen Expression der Pi-omega3Des in Hefen

Der Pi-omega3Des Klon wurde für die heterologe Expression in Hefen über PCR mit entsprechenden Pi-omega3Des spezifischen Primern in den Hefe-Expressionsvektor pYES3 kloniert. Dabei wurde ausschließlich der für das Pi-omega3Des Protein kodierende offene Leseraster des Gens amplifiziert und mit zwei Schnittstellen für die Klonierung in den pYES3 Expressionsvektor versehen:

Forward Primer: 5'-TAAGCTTACATGGCGACGAAGGAGG (SEQ ID NO: 149)
Reverse Primer: 5'-TGGATCCACTTACGTGGACTTGGT (SEQ ID NO: 150)

Zusammensetzung des PCR-Ansatzes (50 $\mu$L):

5,00 $\mu$L Template cDNA
5,00 $\mu$L 10x Puffer (Advantage-Polymerase)+ 25mM $MgCl_2$
5,00 $\mu$L 2mM dNTP
1,25 $\mu$L je Primer (10 pmol/$\mu$L des 5'-ATG sowie des 3'-Stopp Primers)
0,50 $\mu$L Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 2 h bei 37 °C mit den Restriktionsenzymen HindIII und BamHI inkubiert. Der Hefe-Expressionsvektor pYES3 (Invitrogen) wurde in gleicherweise inkubiert. Anschließend wurde das 1104 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschließend wurden Vektor und Desaturase-cDNA ligiert. Dazu wurde das Rapid Ligation Kit von Roche verwendet. Das entstandene Plasmid pYES3-Pi-omega3Des wurde durch Sequenzierung überprüftt und in den Saccharomyces Stamm INVSc1 (Invitrogen) durch Elektroporation (1500 V) transformiert. Zur Kontrolle wurde pYES3 parallel transformiert. Anschliessend wurden die Hefen auf Komplett-Minimalmedium ohne Tryptophan mit 2 % Glucose ausplattiert. Zellen, die auf ohne Tryptophan im Medium wachstumsfähig waren, enthalten damit die entsprechenden Plasmide pYES3, pYES3-Pi-omega3Des. Nach der Selektion wurden je zwei Transformaten zur weiteren funktionellen Expression ausgewählt.

Beispiel 25: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende der kodierenden Sequenz eingefügt:.
PSUN-Pi-omega3Des

Reverse: 3'-GCGGCCGCTTACGTGGACTTGGTC (SEQ ID NO: 147)
Forward: 5'-GCGGCCGCatGGCGACGAAGGAGG (SEQ ID NO: 148)

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 4 h bei 37 °C mit dem Restriktionsenzym NotI inkubiert. Der Pflanzen-Expressionsvektor pSUN300-USP wurde in gleicherweise inkubiert. Anschließend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und PCR-Produkte ligiert. Dazu wurde das Rapid Ligation Kit von Roche verwendet. Das entstandene Plasmid pSUN-Piomega3Des wurde durch Sequenzierung verifiziert.

Beispiel 26: Expression von Pi-omega3Des in Hefen

Hefen, die wie unter Beispiel 24 mit dem Plasmid pYES3 oder pYES3- Pi-omega3Des transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert. Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 27: Funktionelle Charakterisierung von Pi-omega3Des:

Die Substratspezifität der Pi-omega3Des konnte nach Expression und Fütterung verschiedener Fettsäuren ermittelt werden (Figur 12 bis 18). Die gefütterten Substrate liegen in großen Mengen in allen transgenen Hefen vor,

wodurch die Aufnahme dieser Fettsäuren in die Hefen bewiesen ist. Die transgenen Hefen zeigen die Synthese neuer Fettsäuren, den Produkten der Pi-omega3Des-Reaktion. Dies bedeutet, dass das Gen Pi-omega3Des funktional exprimiert werden konnte.

Figur 12 gibt die Desaturierung von Linolsäure (18:2 ω-6-Fettsäure) zu α-Linolensäure (18:3 ω-3-Fettsäure) durch die Pi-omega3Des wieder. Die Synthese der Fettsäuremethylester erfolgte durch saure Methanolyse intakter Zellen, die mit dem Leervektor pYES2 (Figur 12 A) oder dem Vektor pYes3-Pi-omega3Des (Figur 12 B) transformiert worden waren. Die Hefen wurden in Minimalmedium in Gegenwart von C18:$2^{\Delta 9,12}$-Fettsäure (300 μM) kultiviert. Anschließend wurden die FAMEs über GLC analysiert.

In Figur 13 ist die Desaturierung von γ-Linolensäure (18:3 ω-6-Fettsäure) zu Stearidonsäure (18:4 ω-3-Fettsäure) durch Pi-omega3Des wiedergegeben.

Die Synthese der Fettsäuremethylester erfolgte durch saure Methanolyse intakter Zellen, die mit dem Leervektor pYES2 (Figur 13 A) oder dem Vektor pYes3-Pi-omega3Des (Figur 13 B) transformiert worden waren. Die Hefen wurden in Minimalmedium in Gegenwart von γ-C18:$3^{\Delta 6,9,12}$-Fettsäure (300 μM) kultiviert. Anschließend wurden die FAMEs über GLC analysiert.

Figur 14 gibt die Desaturierung von C20:2-ω-6-Fettsäure zu C20:3-ω-3-Fettsäure durch Pi-omega3Des wieder. Die Synthese der Fettsäuremethylester erfolgte durch saure Methanolyse intakter Zellen, die mit dem Leervektor pYES2 (Figur 14 A) oder dem Vektor pYes3-Pi-omega3Des (Figur 14 B) transformiert worden waren. Die Hefen wurden in Minimalmedium in Gegenwart von C20:$2^{\Delta 11,14}$-Fettsäure (300 μM) kultiviert. Anschließend wurden die FAMEs über GLC analysiert.

Figur 15 gibt die Desaturierung von C20:3-ω-6-Fettsäure zu C20:4-ω-3-Fettsäure durch Pi-omega3Des wieder. Die Synthese der Fettsäuremethylester erfolgte durch saure Methanolyse intakter Zellen, die mit dem Leervektor pYES2 (Figur 15 A) oder dem Vektor pYes3-Pi-omega3Des (Figur 15 B) transformiert worden waren. Die Hefen wurden in Minimalmedium in Gegenwart von C20:$3^{\Delta 8,11,14}$-Fettsäure (300 μM) kultiviert. Anschließend wurden die FAMEs über GLC analysiert.

In Figur 16 wird die Desaturierung von Arachidonsäure (C20:4-ω-6-Fettsäure) zu Eicosapentaensäure (C20:5-ω-3-Fettsäure) durch die Pi-omega3Des gezeigt.

Die Synthese der Fettsäuremethylester erfolgte durch saure Methanolyse intakter Zellen, die mit dem Leervektor pYES2 (Figur 16 A) oder dem Vektor pYes3-Pi-omega3Des (Figur 16 B) transformiert worden waren. Die Hefen wurden in Minimalmedium in Gegenwart von C20:$4^{\Delta 5,8,11,14-}$Fettsäure (300 μM) kultiviert. Anschließend wurden die FAMEs über GLC analysiert.

Figur 17 gibt die Desaturierung von Docosatetraensäure (C22:4-ω-6-Fettsäure) zu Docosapentaensäure (C22:5-ω-3-Fettsäure) durch Pi-omega3Des wieder. Die Synthese der Fettsäuremethylester erfolgte durch saure Methanolyse intakter Zellen, die mit dem Leervektor pYES2 (Figur 17 A) oder dem Vektor pYes3-Pi-omega3Des (Figur 17 B) transformiert worden waren. Die Hefen wurden in Minimalmedium in Gegenwart von C22:$4^{\Delta 7,10,13,16}$-Fettsäure (300 μM) kultiviert. Anschließend wurden die FAMEs über GLC analysiert.

Die Substratspezifität der Pi-omega3Des gegenüber verschiedenen Fettsäuren ist Figur 18 zu entnehmen. Die Hefen, die mit dem Vektor pYes3-Pi-omega3Des transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt einen Mittelwert aus drei Messungen wieder. Die Umsetzungsraten (% Desaturation) wurden mit der Formel:

$$[Produkt]/[Produkt]+[Substrat]*100 \text{ errechnet.}$$

Wie unter Beispiel 9 beschrieben kann auch die Pi-omega3Des zur Erzeugung transgener Pflanzen verwendet werden. Aus den Samen dieser Pflanzen kann dann die Lipidextraktion wie unter Beispiel 6 beschrieben erfolgen.

Beispiel 28: Klonierung von Desaturasegenen aus Ostreococcus tauri

Durch Suche nach konservierten Bereichen in den Proteinsequenzen mit Hilfe von konservierten Motiven (His-Boxen, Domergue et al. 2002, Eur. J. Biochem. 269, 4105-4113) konnten fünf Sequenzen mit entsprechenden Motiven in einer Ostreococcus tauri Sequenzdatenbank (genomische Sequenzen) identifiziert werden. Es handelt sich dabei um die folgenden Sequenzen:

| Gen-Name | SEQ ID | Aminosäuren | Homologie |
|---|---|---|---|
| OtD4 | SEQ ID NO: 95 | 536 | Δ-4-Desaturase |

(fortgesetzt)

| Gen-Name | SEQ ID | Aminosäuren | Homologie |
|---|---|---|---|
| OtD5.1 | SEQ ID NO: 91 | 201 | Δ-5-Desaturase |
| OtD5.2 | SEQ ID NO: 93 | 237 | Δ-5-Desaturase |
| OtD6.1 | SEQ ID NO: 89 | 456 | Δ-6-Desaturase |
| OtFad2 | SEQ ID NO: 107 | 361 | Δ-12-Desaturase |

Die Alignments zur Auffindung von Homologien der einzelnen Gene wurden mit dem tBLASTn-Aalgorithmus (Altschul et al., J. Mol. Biol. 1990, 215: 403 - 410) durchgeführt.

Die Klonierung erfolgte wie folgt:
40 ml einer *Ostreococcus tauri* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtDes-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Folgende Primer wurden für die PCR eingesetzt:

OtDes6.1 Forward: 5'ggtaccacataatgtgcgtggagacggaaaataacg3' (SEQ ID NO: 145)

OtDes6.1 Reverse: 5'ctcgagttacgccgtctttccggagtgttggcc3' (SEQ ID NO: 146)

Beispiel: 29 Klonierung von Expressionsplasmiden zur heterologen Expression in Hefen:

Zur Charakterisierung der Funktion der Desaturase OtDes6.1 (= Δ-6-Desaturase) aus *Ostreococcus tauri* wurde der offenen Leserahmen der DNA stromabwärts des Galactose-induzierbaren GAL1-Promotors von pYES2.1/V5-His-TOPO (Invitrogen) kloniert, wobei der entsprechenden pYES2.1-OtDes6.1 Klon erhalten wurde. In entsprechender Art und Weise können weitere Desaturase-Gene aus Ostreococcus kloniert werden.

Der *Saccharomyces cerevisiae*-Stamm 334 wurde durch Elektroporation (1500 V) mit dem Vektor pYES2.1-OtDes6.1 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der OtDes6.1 Desaturase wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel: 30 Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mittels PCR NotI-Schnittstellen am 5' und 3'-Ende der kodierenden Sequenzen eingefügt. Die entsprechenden Primersequenzen werden von den 5'- und 3-Bereich der Desaturasen abgeleitet.

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μLje Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wurde das Rapid Ligation Kit von Roche verwendet. Die entstandenen Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–
GTCGACCCGCGGACTAGTGGGCCCTCTAGACCCGGGGGATCC
GGATCTGCTGGCTATGAA–3', SEQ ID NO: 144).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel: 31 Expression von OtDes6.1 in Hefen

Hefen, die wie unter Beispiel 4 mit den Plasmiden pYES2 und pYES2-OtDes6.2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel: 32 Funktionelle Charakterisierung von Desaturasen aus Ostreococcus:

Die Substratspezifität von Desaturasen kann nach Expression in Hefe (siehe Beispiele Klonierung von Desaturase-Genen, Hefeexpression) durch die Fütterung mittels verschiedener Hefen ermittelt werden. Beschreibungen für die Bestimmung der einzelnen Aktivitäten finden sich in WO 93/11245 für Δ15-Desaturasen, WO 94/11516 für Δ12-Desaturasen, WO 93/06712, US 5,614,393, US5614393, WO 96/21022, WO0021557 und WO 99/27111 für Δ6-Desaturasen, Qiu et al. 2001, J. Biol. Chem. 276, 31561-31566 für Δ4-Desaturasen, Hong et al. 2002, Lipids 37,863-868 für

Δ5-Desaturasen.

Tabelle 12 gibt die Substratspezifität der Desaturase OtDes6.1 gegenüber verschiedenen Fettsäuren wieder. Die Substratspezifität der OtDes6.1 konnte nach Expression und Fütterung verschiedener Fettsäuren ermittelt werden. Die gefütterten Substrate sind in großen Mengen in allen transgenen Hefen nachzuweisen. Die transgenen Hefen zeigten die Synthese neuer Fettsäuren, den Produkten der OtDes6.2-Reaktion (Fig. 20). Dies bedeutet, dass das Gen OtDes6.1 funktional exprimiert werden konnte.

Die Hefen, die mit dem Vektor pYES2-OtDes6.1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder. Die Aktivität entspricht der Konversionsrate errechnet nach [Substrat/(Substrat+Produkt)*100].

Tabelle 12 zeigt, dass die OtDes6.1 eine Substratspezifität für Linol- und Linolensäure (18:2 und 18:3) aufweist, da mit diesen Fettsäuren die höchsten Aktivitäten erreicht werden. Die Aktivität für Ölsäure (18:1) und Palmitoleinsäure (16:1) ist dagegen deutlich geringer. Die bevorzugte Umsetzung von Linol- und Linolensäure zeigt die Eignung dieser Desaturase für die Herstellung von polyungesättigten Fettsäuren.

| Substrate | Aktivität in % |
|---|---|
| $16:1^{\Delta 9}$ | 5,6 |
| $18:1^{\Delta 9}$ | 13,1 |
| $18:2^{\Delta 9,12}$ | 68,7 |
| $18:3^{\Delta 9,12,15}$ | 64,6 |

Figur 20 zeigt die Umsetzung von Linolsäure durch OtDes6.1. Die Analyse der FAMEs erfolgte über Gaschromatographie. Das gefütterte Substrat (C18:2) wird zu γ-C18:3 umgesetzt. Sowohl Edukt als auch das entstandene Produkt sind durch Pfeile markiert.

In Figur 21 wird die Umsetzung von Linolsäure (= LA) und α-Linolensäure (= ALA) in Gegenwart von OtDes6.1 zu γ-Linolensäure (= GLA) bzw. Stearidonsäure (= STA) wiedergegeben (Figur 21 A und C). Weiterhin zeigt Figur 21 die Umsetzung von Linolsäure (= LA) und α-Linolensäure (= ALA) in Gegenwart der Δ-6-Desaturase OtDes6.1 zusammen mit der Δ-6-Elongase PSE1 aus Physcomitrella patens (Zank et al. 2002, Plant J. 31:255-268) und der Δ-5-Desaturase PtD5 aus Phaeodactylum tricornutum (Domergue et al. 2002, Eur. J. Biochem. 269, 4105-4113) zu Dihomo-γ-linolensäure (= DHGLA) und Arachidonsäure (= ARA, Figur 21 B) bzw. zu Dihomostearidonsäure (= DHSTA) bzw. Eicosapentaensäure (= EPA, Figur 21 D). Figur 21 zeigt deutlich, dass die Reaktionsprodukte GLA und STA der Δ-6-Desaturase OtDes6.1 in Gegenwart der Δ-6-Elongase PSE1 fast quantitativ zu DHGLA bzw. DHSTA elongiert wird. Die nachfolgende Desaturierung durch die Δ-5-Desaturase PtD5 erfolgt ebenfalls reibungslos zu ARA bzw. EPA. Es werden ca. 25 - 30% des Elongaseprodukts desaturiert (Figur 21 B und D).

Die folgenden Tabelle 13 gibt eine Übersiche über die klonierten Ostreococcus Desaturasen wieder:

| Ostreococcus tauri Desaturasen | | | | | | | |
|---|---|---|---|---|---|---|---|
| | | | | | | | |
| **Name** | **bp** | **aa** | **Homologie** | **Cyt. B5** | **His-Box1** | **His-Box2** | **His-Box3** |
| | | | | | | | |
| OtD4 | 1611 | 536 | Δ-4- Desaturase | HPGG | HCANH | WRYHHQVSHH | QVEHHLFP |
| OtD5.1 | 606 | 201 | Δ-5-Desaturase | - | - | - | QVVHHLFP |
| OtD5.2 | 714 | 237 | Δ-5-Desaturase | - | - | WRYHHMVSHH | QIEHHLPF |
| OtD6.1 | 1443 | 480 | Δ-6-Desaturase | HPGG | HEGGH | WNSMHNKHH | QVIHHLFP |
| OtFAD2 | 1086 | 361 | Δ-12-Desaturase | - | HECGH | WQRSHAVHH | HVAHH |

Beispiel: 33 Klonierung von Desaturasegenen aus Thalassiosira pseudonana

Durch Suche nach konservierten Bereichen in den Proteinsequenzen mit Hilfe von konservierten Motiven (His-Boxen, siehe Motive) konnten sechs Sequenzen mit entsprechenden Motiven in einer Thalassiosira pseudonana Sequenzdatenbank (genomische Sequenzen) identifiziert werden. Es handelt sich dabei um die folgenden Sequenzen:

| Gen-Name | SEQ ID | Aminosäuren | Homologie |
|---|---|---|---|
| TpD4 | SEQ ID NO: 103 | 503 | Δ-4-Desaturase |
| TpD5-1 | SEQ ID NO: 99 | 476 | Δ-5-Desaturase |
| TpD5-2 | SEQ ID NO: 101 | 482 | Δ-5-Desaturase |
| TpD6 | SEQ ID NO: 97 | 484 | Δ-6-Desaturase |
| TpFAD2 | SEQ ID NO: 109 | 434 | Δ-12-Desaturase |
| TpO3 | SEQ ID NO: 105 | 418 | ω-3-Desaturase |

Die Klonierung erfolgte wie folgt:
40 ml einer *Thalassiosira pseudonana* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der TpDes-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel: 34 Klonierung von Expressionsplasmiden zur heterologen Expression in Hefen:

Zur Charakterisierung der Funktion der Desaturasen aus *Thalassiosira pseudonana* wird der offenen Leserahmen der jeweiligen DNA stromabwärts des Galactose-induzierbaren GAL1-Promotors von pYES2.1/V5-His-TOPO (Invitrogen) kloniert, wobei der entsprechenden pYES2.1-Klone erhalten werden.
Der *Saccharomyces cerevisiae*-Stamm 334 wird durch Elektroporation (1500 V) mit den Vektoren pYES2.1-TpDesaturasen transformiert. Als Kontrolle wird eine Hefe verwendet, die mit dem leeren Vektor pYES2 transformiert wird. Die Selektion der transformierten Hefen erfolgt auf Komplett-Minimalmedium (CMdum)-Agarplatten mit 2% Glucose, aber ohne Uracil. Nach der Selektion werden je drei Transformanten zur weiteren funktionellen Expression ausgewählt.
Für die Expresssion der Tp-Desaturasen werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.
5 ml CMdum-Flüssigmedium (ohne Uracil) mit 2% Raffinose und 300 μM verschiedener Fettsäuren werden dann mit den Vorkulturen auf eine $OD_{600}$ von 0,05 angeimpft. Die Expression wird durch die Zugabe von 2% (w/v) Galactose induziert. Die Kulturen werden für weitere 96 h bei 20°C inkubiert.

Beispiel: 35 Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mittels PCR NotI-Schnittstellen am 5' und 3'-Ende der kodierenden Sequenzen eingefügt. Die entsprechenden Primersequenzen werden von den 5'- und 3-Bereich der Desaturasen abgeleitet.
Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C

Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wurde das Rapid Ligation Kit von Roche verwendet. Die entstandenen Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des OCS-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–
GTCGACCCGCGGACTAGTGGGCCCTCTAGACCCGGGGGATCC
GGATCTGCTGGCTATGAA–3'; SEQ ID NO: 143)

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel: 36 Expression von Tp-Desaturasen in Hefen

Hefen, die wie unter Beispiel 4 mit den Plasmiden pYES2 und pYES2-TpDesaturasen transformiert werden, werden folgendermaßen analysiert:
Die Hefezellen aus den Hauptkulturen werden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren werden die organischen Phasen je einmal mit 2 ml 100 mM $NaHCO_3$, pH 8,0 und 2 ml Aqua dest. gewaschen. Anschließend werden die PE-Phasen mit $Na_2SO_4$ getrocknet, unter Argon eingedampft und in 100 $\mu$l PE aufgenommen. Die Proben werden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse sind wie folgt: Die Ofentemperatur wird von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel: 37 Funktionelle Charakterisierung von Desaturasen aus Thalassiosira pseudonana:

Die Substratspezifität von Desaturasen kann nach Expression in Hefe (siehe Beispiele Klonierung von Desaturase-Genen, Hefeexpression) durch die Fütterung mittels verschiedener Hefen ermittelt werden. Beschreibungen für die Bestimmung der einzelnen Aktivitäten finden sich in WO 93/11245 für $\Delta$15-Desaturasen, WO 94/11516 für $\Delta$12-Desaturasen, WO 93/06712, US 5,614,393, US5614393, WO 96/21022, WO0021557 und WO 99/27111 für $\Delta$6-Desaturasen, Qiu et al. 2001, J. Biol. Chem. 276, 31561-31566 für $\Delta$4-Desaturasen, Hong et al. 2002, Lipids 37,863-868 für $\Delta$5-Desaturasen.

Die Aktivität der einzelnen Desaturasen wird aus der Konversionsrate errechnet nach der Formel [Substrat/(Substrat+Produkt)*100].

Die folgenden Tabellen 11 und 12 geben eine Übersicht über die clonierten Thalassiosira pseudonana Desaturasen wieder.

Tabelle 14: Länge und charakteristische Merkmale der clonierten Thalassiosira Desaturasen.

| **Desaturase** | **cDNA (bp)** | **Protein (aa)** | **Cyt. B5** | **His-Box1** | **His-Box2** | **His-Box3** |
|---|---|---|---|---|---|---|
| | | | | | | |
| TpD4 | 1512 | 503 | HPGG | HDGNH | WELQHMLGHH | QIEHHLFP |
| TpD5-1 | 1431 | 476 | HPGG | HDANH | WMAQHWTHH | QVEHHLFP |
| TpD5-2 | 1443 | 482 | HPGG | HDANH | WLAQHWTHH | QVEHHLFP |
| TpD6 | 1449 | 484 | HPGG | HDFLH | WKNKHNGHH | QVDHHLFP |
| TpFAD2 (d12) | 1305 | 434 | - | HECGH | HAKHH | HVAHHLFH |
| TpO3 | 1257 | 419 | - | HDAGH | WLFMVTYLQH H | HVVHHLF |

Tabelle 15: Länge, Exons, Homolgie und Identitäten der clonierten Desaturasen.

| **Des.** | **GDNA (bp)** | **Exon 1** | **Exon 2** | **First Blast Hit** | **Hom./Iden.** |
|---|---|---|---|---|---|
| | | | | | |
| TpD4 | 2633 | 496-1314 | 1571-2260 | Thrautochitrium D4-des | 56% / 43% |
| TpD5-1 | 2630 | 490-800 | 900-2019 | Phaeodactylum D5-des | 74% / 62% |
| TpD5-2 | 2643 | 532-765 | 854-2068 | Phaeodactylum D5-des | 72% / 61% |
| TpD6 | 2371 | 379-480 | 630-1982 | Phaeodactylum D6-des | 83% / 69% |
| TpFAD2 | 2667 | 728-2032 | - | Phaeodactylum FAD2 | 76% / 61% |
| TpO3 | 2402 | 403-988 | 1073-1743 | Chaenorhabdidis Fad2 | 49% / 28% |

Analog zu den vorgenannten Beispielen lassen sich auch die Δ-12-Desaturasegene aus Ostreococcus und Thalassiosira clonieren.

Beispiel 38 Klonierung von Elongase Genen aus Xenopus laevis und Ciona intestinalis

Durch Suche nach konservierten Bereichen (siehe Konsensus-Sequenzen, SEQ ID NO: 115 und SEQ ID NO: 116) in den Proteinsequenzen in Gendatenbanken (Genbank) mit Hilfe der in der Anmeldung aufgeführten Elongase-Gene mit Δ-5-Elongaseaktivität oder Δ-6-Elongaseaktivität konnten weitere Elongasesequenzen aus anderen Organismen identifiziert und isoliert werden. Aus X. laevis bzw. aus C. intestinalis konnten mit entsprechenden Motiven jeweils weitere Sequenzen identifiziert werden. Es handelt sich dabei um die folgenden Sequenzen:

| Gen-Name | Organismus | Genbank-Nr. | SEQ ID NO: | Aminosäuren |
|---|---|---|---|---|
| ELO(XI) | Xenopus laevis | BC044967 | 117 | 303 |
| ELO(Ci) | Ciona intestinalis | AK112719 | 119 | 290 |

Der cDNA Klon von X. laevis wurde vom NIH (National Institut of Health) bezogen [Genetic and genomic tools for Xenopus research: The NIH Xenopus initiative, Dev. Dyn. 225 (4), 384-391 (2002)].

Der cDNA Klon von C. inetstinalis wurde von der Universität von Kyto bezogen [Satou,Y., Yamada,L., Mochizuki,Y., Takatori,N., Kawashima,T., Sasaki,A., Hamaguchi,M., Awazu,S., Yagi,K., Sasakura,Y., Nakayama,A., Ishikawa,H., Inaba,K. and Satoh,N. "A cDNA resource from the basal chordate Ciona intestinalis" JOURNAL Genesis 33 (4), 153-154 (2002)].

Beispiel 39: Klonierung von Expressionsplasmiden zur heterologen Expression in - Hefen

Die Amplifizierung der Elongase-DNAs wurde jeweils mit 1 µL cDNA, 200 µM dNTPs, 2,5 U Advantage-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name und SEQ ID NO: | | Primersequenz |
|---|---|---|
| ELO(XI) | SEQ ID NO: 121<br>SEQ ID NO: 122 | F:5'- AGGATCCATGGCCTTCAAGGAGCTCACATC<br>R:5'- CCTCGAGTCAATGGTTTTTGCTTTTCAAT-GCACCG |
| ELO(Ci), | SEQ ID NO: 123<br>SEQ ID NO: 124 | F:5'-TAAGCTTATGGACGTACTTCATCGT<br>R:5'-TCAGATCTTTAATCGGTTTTACCATT |
| *F=forward primer, R=reverse primer | | |

Die PCR Produkte wurde für 30 min bei 21 °C mit dem Hefe-Expressionsvektor - pYES2.1-TOPO (Invitrogen) gemäß Herstellerangaben inkubiert. Das PCR-Produkt wird dabei durch einen T-Überhang und Aktivität einer Topoisomerase (Invitrogen) nach Herstellerangaben in den Vektor ligiert. Nach der Inkubation erfolgte dann die Transformation von E. coli DH5a Zellen. Entsprechende Klone wurden durch PCR - identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-ELO(XI) und pYES2.1-ELO(Ci). Nach der Selektion wurden je zwei Transformaten zur weiteren funktionellen Expression ausgewählt.

Beispiel 40: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mit folgendem Primerpaar NotI-Schnittstellen am 5' und 3'-Ende der kodierenden Sequenz eingefügt:.

pSUN-ELO(XI)

Forward: 5'-GCGGCCGCACCATGGCCTTCAAGGAGCTCACATC
(SEQ ID NO: 125)
Reverse: 3'-GCGGCCGCCTTCAATGGTTTTTGCTTTTCAATGCACCG
(SEQ ID NO: 126)

pSUN-ELO(Ci)

Forward: 5'-GCGGCCGCACCATGGACGTACTTCATCGT
(SEQ ID NO: 127)
Reverse: 3'-GCGGCCGCTTTAATCGGTTTTACCATT
(SEQ ID NO: 128)

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkte ligiert. Dazu wurde das Rapid Ligation Kit von Roche verwendet. Die entstandenen Plasmide pSUN-ELO(Xl) und pSUN-ELO(Ci) wurden durch Sequenzierung verifiziert.
pSUN300 ist ein Derivat des Plasmides pPZP [Hajdukiewicz,P, Svab, Z, Maliga, P., (1994) The small versatile pPZP family of Agrobacterium binary vectors forplant transformation. Plant Mol Biol 25:989-994]. pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

Primersequenz: 5'–
GTCGACCCGCGGACTAGTGGGCCCTCTAGACCCGGGGGATCC
GGATCTGCTGGCTATGAA–3' (SEQ ID NO: 129).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 41: Expression von ELO(Xl) und ELO(Ci) in Hefen

Hefen, die wie unter Beispiel 4 mit den Plasmiden pYES2, pYES2-ELO(Xl) und pYES2-ELO(Ci) transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8): 761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 42: Funktionelle Charakterisierung von ELO(Xl) und ELO(Ci):

Die Substratspezifität der ELO(Xl) konnte nach Expression und Fütterung verschiedener Fettsäuren ermittelt

werden (Fig. 22). Die gefütterten Substrate sind in großen Mengen in allen transgenen Hefen nachzuweisen. Die transgenen Hefen zeigten die Synthese neuer Fettsäuren, den Produkten der ELO(XI)-Reaktion. Dies bedeutet, dass das Gen ELO(XI) funktional exprimiert werden konnte.

Tabelle 16 zeigt, dass die ELO(XI) eine breite Substratspezifität aufweist. Es werden sowohl C18 als auch C20 Fettsäuren verlängert, wobei ein Bevorzugung von Δ5- und Δ6-desaturierten Fettsäuren zu beobachten ist.

Die Hefen, die mit dem Vektor pYES2-ELO(XI) transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 16: Expression von ELO(XI) in Hefe. Beschrieben ist die Umsetzungsrate (Konversionsrate) verschiedener Edukte (gefüttert jeweils 250 μM).

| Edukte | Konversion der Edukte durch ELO(XI) in % |
|---|---|
| 16:0 | 3 |
| $16{:}1^{\Delta 9}$ | 0 |
| 18:0 | 2 |
| $18{:}1^{\Delta 9}$ | 0 |
| $18{:}2^{\Delta 9,12}$ | 3 |
| $18{:}3^{\Delta 6,9,12}$ | 12 |
| $18{:}3^{\Delta 5,9,12}$ | 13 |
| $18{:}3^{\Delta 9,12,15}$ | 3 |
| $18{:}4^{\Delta 6,9,12,15}$ | 20 |
| $20{:}3^{\Delta 8,11,14}$ | 5 |
| $20{:}3^{\Delta 11,14,17}$ | 13 |
| $20{:}4^{\Delta 5,8,11,14}$ | 15 |
| $20{:}5^{\Delta 5,8,11,14,17}$ | 10 |
| $22{:}4^{\Delta 7,10,13,16}$ | 0 |
| $22{:}6^{\Delta 4,7,10,13,16,19}$ | 0 |

Die Substratspezifität der ELO(Ci) konnte nach Expression und Fütterung verschiedener Fettsäuren ermittelt werden (Fig. 23). Die gefütterten Substrate sind in großen Mengen in allen transgenen Hefen nachzuweisen. Die transgenen Hefen zeigten die Synthese neuer Fettsäuren, den Produkten der ELO(Ci)-Reaktion. Dies bedeutet, dass das Gen ELO(Ci) funktional exprimiert werden konnte.

Tabelle 17: Expression von ELO(Ci) in Hefe. Beschrieben ist die Umsetzungsrate (Konversionsrate) verschiedener Edukte (gefüttert jeweils 250 μM).

| Edukte | Konversion der Edukte durch ELO(Ci) in % |
|---|---|
| 16:0 | 0 |
| $16{:}1^{\Delta 9}$ | 0 |
| 18:0 | 0 |
| $18{:}1^{\Delta 9}$ | 0 |
| $18{:}^{2\Delta 9,12}$ | 23 |
| $18{:}3^{\Delta 6,9,12}$ | 10 |
| $18{:}3^{\Delta 5,9,12}$ | 38 |
| $18{:}3^{\Delta 9,12,15}$ | 25 |
| $18{:}4^{\Delta 6,9,12,15}$ | 3 |

(fortgesetzt)

| Edukte | Konversion der Edukte durch ELO(Ci) in % |
|---|---|
| 20:3$^{\Delta 8,11,14}$ | 10 |
| 20:3$^{\Delta 11,14,17}$ | 8 |
| 20:4Δ5,8,11,14 | 10 |
| 20:5Δ5,8,11,14,17 | 15 |
| 22:4Δ7,10,13,16 | 0 |
| 22:6Δ4,7,10,13,16,19 | 0 |

Tabelle 17 zeigt, dass die ELO(Ci) eine breite Substratspezifität aufweist. Es werden sowohl C18 als auch C20 Fettsäuren verlängert, wobei ein Bevorzugung von Δ5- und Δ6-desaturierten Fettsäuren zu beobachten ist.

Die Hefen, die mit dem Vektor pYES2-ELO(Ci) transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Beispiel 43: Klonierung von Genen aus Ostreococcus tauri

Durch Suche nach konservierten Bereichen in den Proteinsequenzen mit Hilfe der hierin beschriebenen Elongase-Gene mit Δ-5-Elongaseaktivität oder Δ-6-Elongaseaktivität konnten je zwei Sequenzen mit entsprechenden Motiven in einer Ostreococcus tauri Sequenzdatenbank (genomische Sequenzen) identifiziert werden. Es handelt sich dabei um die folgenden Sequenzen:

| Gen-Name | SEQ ID | Aminosäuren |
|---|---|---|
| OtELO1, (Δ-5-Elongase) | SEQ ID NO: 67 | 300 |
| OtELO1.2, (Δ-5-Elongase) | SEQ ID NO: 113 | 300 |
| OtELO2, (Δ-6-Elongase) | SEQ ID NO: 69 | 292 |
| OtELQ2.1, (Δ-6-Elongase) | SEQ ID NO: 111 | 292 |

OtElo1 und OtElo1.2 weisen die höchste Ähnlichkeit zu einer Elongase aus Danio rerio auf (GenBank AAN77156; ca. 26 % Identität), während OtElo2 und OtElo2.1 die größte Ähnlichkeit zur Physcomitrella Elo (PSE) [ca. 36 % Identität] aufweisen (Alignments wurden mit dem tBLASTn-Aalgorithmus (Altschul et al., J. Mol. Biol. 1990, 215: 403 - 410) durchgeführt.

Die Klonierung der Elongasen wurde wie folgt durchgeführt:
40 ml einer *Ostreococcus tauri* Kultur in der stationären Phase wurden abzentrifugiert und in 100 µl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtElo-DNAs wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel 44: Klonierung von Expressionsplasmiden zur heterologen Expression in Hefen:

Zur Charakterisierung der Funktion der Elongasen aus *Ostreococcus tauri* wurden die offenen Leserahmen der jeweiligen DNAs stromabwärts des Galactose-induzierbaren GAL1-Promotors von pYES2.1/V5-His-TOPO (Invitrogen) kloniert, wobei pOTE1, pOTE1.2, pOTE2 und pOTE2.1 erhalten wurden.

Der *Saccharomyces cerevisiae*-Stamm 334 wurde durch Elektroporation (1500 V) mit dem Vektor pOTE1, pOTE1.2, pOTE2 bzw. pOTE2.1 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren

funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.
5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 45: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurden mittels PCR NotI-Schnittstellen am 5' und 3'-Ende der kodierenden Sequenzen eingefügt. Die entsprechenden Primersequenzen wurden von den 5'- und 3-Bereich von OtElo1, OtElo1.2, OtElo2 und OtElo2.1 abgeleitet.

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μLje Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschließend wurden die PCR Produkte sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschliessend wurden Vektor und PCR-Produkte ligiert. Dazu wurde das Rapid Ligation Kit von Roche verwendet. Die entstandenen Plasmide pSUN-OtELO1, pSUN-OtELO1.2, pSUN-OtELO2 und pSUN-OtELO2.2 wurden durch Sequenzierung verifiziert.

pSUN300 ist ein Derivat des Plasmides pPZP [Hajdukiewicz,P, Svab, Z, Maliga, P., (1994) The small versatile pPZP family of Agrobacterium binary vectors for plant transformation. Plant Mol Biol 25:989-994]. pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

Primersequenz:

5'–GTCGACCCGCGGACTAGTGGGCCCTCTAGACCCGGGGGATCC
GGATCTGCTGGCTATGAA–3'). (SEQ ID NO: 130)

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.

Beispiel 46: Expression von OtElo1, OtElo1.2, OtElo2 und OtELO2.2 in Hefen

Hefen, die wie unter Beispiel 15 mit den Plasmiden pYES3, pYES3-OtELO1, pYES3-OtELO1.2, pYES3-OtELO2 und pYES3-OtELO2.2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C (halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 47: Funktionelle Charakterisierung von OtElo1, OtElo1.2, OtElo2 und OtElo2.1:

Die Substratspezifität der OtElo1 konnte nach Expression und Fütterung verschiedener Fettsäuren ermittelt werden (Tab. 18). Die gefütterten Substrate sind in großen Mengen in allen transgenen Hefen nachzuweisen. Die transgenen Hefen zeigten die Synthese neuer Fettsäuren, den Produkten der OtElo1-Reaktion. Dies bedeutet, dass das Gen OtElo1 funktional exprimiert werden konnte.

Tabelle 18 zeigt, dass OtElo1 bzw. OtElo1.2 eine enge Substratspezifität aufweist. OtElo1 bzw. OtElo1.2 konnte nur die C20-Fettsäuren Eicosapentaensäure (Figur 24A, 24B) und Arachidonsäure (Figur 25A, 25B) elongieren, bevorzugte aber die ω-3-desaturierte Eicosapentaensäure.

Tabelle 18 zeigt die Substratspezifität der Elongase OtElo1 und OtElo1.2 für C20 polyungesättigte Fettsäuren mit einer Doppelbindung in Δ5 Position gegenüber verschiedenen Fettsäuren.

Die Hefen, die mit dem Vektor pOTE1 bzw. pOTE1.2 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Die Substratspezifität der OtElo2 (SEQ ID NO: 81) OtElo2.1 (SEQ ID NO: 111) konnte nach Expression und Fütterung verschiedener Fettsäuren ermittelt werden (Tab. 19). Die gefütterten Substrate sind in großen Mengen in allen transgenen Hefen nachzuweisen. Die transgenen Hefen zeigten die Synthese neuer Fettsäuren, den Produkten der OtElo2-Reaktion. Dies bedeutet, dass die Gene OtElo2 und OtElo2.1 funktional exprimiert werden konnte.

Tabelle 18:

| Fettsäuresubstrat | Umsatz (in %) OtElo1 | Umsatz (in %) OtElo1.2 |
|---|---|---|
| 16:0□ | - | - |
| 16:1$^{\square\Delta 9}$ | - | - |
| 18:0□ | - | - |
| 18:1$^{\square\Delta 9}$ | - | - |
| 18:1$^{\Delta 11}$ | - | - |
| 18:2$^{\Delta 9,12}$ | - | - |
| 18:3$^{\square\Delta 6,9,12}$ | - | - |
| 18:3$^{\square\Delta 5,9,12}$ | - | - |
| 20:3 $^{\Delta 8,11,14}$ | - | - |
| 20:4 $^{\Delta 5,\square 8,11,14}$ | **10,8 ± 0,6** | **38,0** |
| 20:5 $^{\Delta 5,\square 8,11,14,17}$ | **46,8 ± 3,6** | **68,6** |
| 22:4 $^{\Delta 7,\square 10,13,16}$ | - | - |

(fortgesetzt)

| Fettsäuresubstrat | Umsatz (in %) OtElo1 | Umsatz (in %) OtElo1.2 |
|---|---|---|
| 22:6□$^{\Delta 7,10,13,16,19}$ | - | - |

Tabelle 19 zeigt die Substratspezifität der Elongase OtElo2 und OtElo2.1 gegenüber verschiedenen Fettsäuren. OtElo2.1 zeigt eine deutlich höhere Aktivität.

Die Hefen, die mit dem Vektor pOTE2 bzw. pOTE2.1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Die enzymatische Aktivität, die in Tabelle 19 wiedergegeben wird, zeigt klar, dass OtElo2 bzw. OtElo2.1 eine Δ-6-Elongase ist.

Tabelle 19:

| Fettsäuresubstrat | Umsatz (in %) OtElo2 | Umsatz (in %)O tELO2.2 |
|---|---|---|
| 16:0 | - | - |
| 16:1□$^{\Delta 9}$ | - | - |
| 16:3□$^{\Delta 7,10,13}$ | - | - |
| 18:0□ | - | - |
| 18:1□$^{\Delta 6}$ | - | - |
| 18:1□$^{\Delta 9}$ | - | - |
| 18:1□$^{\Delta 11}$ | - | - |
| 18:2□$^{\Delta 9,12}$ | - | - |
| 18:3$^{\Delta 6,9,12}$ | **15,3** | **55,7** |
| 18:3□ $^{\Delta 5,9,12}$ | - | - |
| 18:4□$^{\Delta 6,9,12,15}$ | **21,1** | **70,4** |
| 20:2 $^{\Delta 11,14}$ | - | - |
| 20:3 $^{\Delta 8,11,14}$ | - | - |
| 20:4 $^{\Delta 5,}$□$^{8,11,14}$ | - | - |
| 20:5 $^{\Delta 5,}$□$^{8,11,14,17}$ | - | - |
| 22:4 $^{\Delta 7,}$□$^{10,13,16}$ | - | - |
| 22:5 $^{\Delta 7,}$□$^{10,13,16,19}$ | - | - |
| 22:6□$^{\Delta 4,7,10,13,16,19}$ | - | - |

Figur 24 A - D zeigt die Elongation von Eicosapentaensäure durch OtElo1 (B) bzw. OtElo1.2 (D). Die Kontrollen (A, C) zeigen nicht das Produkt der Elongation (22:5ω3).

Figur 25 A - D zeigt die Elongation von Arachidonsäure durch OtElo1 (B) bzw. OtElo1.2 (D). Die Kontrollen (A, C) zeigen nicht das Produkt der Elongation (22:4ω6).

Beispiel 48: Klonierung von Elongase-Genen aus Euglena gracilis und Arabidopsis thaliana

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten Sequenzen aus Arabidopsis thaliana bzw. Euglena gracilis mit entsprechenden Motiven in Sequenzdatenbanken (Genbank, Euglena EST Bank) identifiziert werden. Es handelt sich dabei um die folgenden Sequenzen:

| Gen-Name | SEQ ID | Aminosäuren |
|---|---|---|
| EGY1019 (E. gracilis) | SEQ ID NO: 131 | 262 |
| EGY2019 (E. gracilis) | SEQ ID NO: 133 | 262 |
| At3g06460 (A. thaliana) | SEQ ID NO: 135 | 298 |
| At3g06470 (A. thaliana) | SEQ ID NO: 137 | 278 |

Die Klonierung der Elongasen aus Euglena gracilis wurden wie folgt durchgeführt:
Der Euglena gracilis Stamm 1224-5/25 wurde erhalten von der Sammlung für Algenkulturen Göttingen (SAG). Zur Isolierung wurde der Stamm in Medium II (Calvayrac R and Douce R, FEBS Letters 7:259-262, 1970) für 4 Tage bei 23 °C unter einem Licht-/ Dunkelintervall von 8 h / 16 h (35 mol s-1 m-2 Lichtstärke) angezogen.

Gesamt-RNA von einer viertägigen Euglena Kultur wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend der Herstellerangaben wurde die cDNA zur Plasmid-DNA entpackt und Klone wurden zur Zufallssequenzierung ansequenziert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) reverse transkribiert und entsprechend der Herstellerangaben wurden die Adaptoren ligiert. Die cDNA-Bank wurde dann für die PCR zur Klonierung von Expressionsplasmiden mittels 5'- und 3'-RACE (rapid amplification of cDNA ends) verwendet.

Die Klonierung der Elongasen aus Arabidopsis thaliana wurde wie folgt durchgeführt:
Ausgehend von der genomischen DNA wurden für die beiden Gene Primer entsprechend am 5'- und 3'-Ende des offenen Leserahmens abgeleitet.

Zur Isolierung von Gesamt-RNA aus *A. thaliana* wurde nach Chrigwin *et al.,* (1979) verfahren. Blätter von 21 Tage alten Pflanzen wurden in flüssigem Stickstoff zermörsert, mit Aufschlusspuffer versetzt und für 15 min bei 37 °C inkubiert. Nach Zentrifugation (10 min, 4 oC, 12000xg) wurde die RNA im Überstand mit 0,02 Volumen 3 M Natriumacetat pH 5,0 und 0,75 Volumen Ethanol bei -20 °C für 5 h präzipitiert. Die RNA wurde dann nach einem weiteren Zentrifugationsschritt in 1 mL TES pro g Ausgangsmaterial aufgenommen, einmal mit einem Volumen Phenol-Chloroform und einmal mit einem Volumen Chloroform extrahiert und die RNA mit 2,5 M LiCl gefällt. Nach anschliessendem Zentrifugieren und Waschen mit 80 %igem Ethanol wurde die RNA in Wasser resuspendiert. Entsprechend Sambrook et al. 1989 wurde die cDNA synthetisiert und RT-PCR mit den abgeleiteten Primer durchgeführt. Die PCR-Produkte wurden nach Herstellerangaben in den Vektor pYES2.1-TOPO (Invitrogen) kloniert.

Beispiel 49: Klonierung von Expressionsplasmiden zur heterologen Expression in Hefen:

Zur Charakterisierung der Funktion der Elongasen aus *A. thalina* wurden die offenen Leserahmen der jeweiligen DNAs stromabwärts des Galactose-induzierbaren GAL1-Promotors von pYES2.1/V5-His-TOPO (Invitrogen) kloniert, wobei pAt60 und pAt70 erhalten wurden.

Der *Saccharomyces cerevisiae*-Stamm 334 wurde durch Elektroporation (1500 V) mit dem Vektor pAt60 bzw. pAt70 transformiert. Als Kontrolle wurde eine Hefe verwendet, die mit dem leeren Vektor pYES2.1 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der At-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 50: Expression von pAt60 und pAt70 in Hefen

Hefen, die wie unter Beispiel 5 mit den Plasmiden pYES2.1, pAt60 bzw. pAt70 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N

methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 51: Funktionelle Charakterisierung von pAt60 und pAt70

Die Substratspezifität der Elongasen At3g06460 bzw. At3g06470 konnte nach Expression und Fütterung verschiedener Fettsäuren ermittelt werden (Tab. 20, Fig. 26). Die gefütterten Substrate sind in allen transgenen Hefen nachzuweisen. Die transgenen Hefen zeigten die Synthese neuer Fettsäuren, den Produkten der Gene At3g06460 bzw. At3g06470. Dies bedeutet, dass diese Gene funktional exprimiert werden konnte.

Tabelle 20: Elongation von EPA durch die Elongasen At3g06460 bzw. At3g06470. Messung der Hefeextrakte nach Fütterung mit 250 uM EPA.

| Gen | Gefütterte Fettsäure | Gehalt anC20:5n-3 | Gehalt an C22: 5n-3 |
|---|---|---|---|
| At3g06460 | EPA (C20:5n-3) | 20.8 | 0,6 |
| At3g06460 | EPA (C20:5n-3) | 25,4 | 1,1 |
| Konversionsrate von EPA | At3g06460: 3,0 % | At3g06470: 4,1 % | |

Figur 26 gibt die Elongation von 20:5n-3 durch die Elongasen At3g06470 wieder.

Beispiel 52: Klonierung einer Elongase aus Phaeodactylum tricornutum

Ausgehend von konservierten Bereichen in den Proteinsequenzen mit Hilfe der in der Anmeldung aufgeführten Elongase-Gene mit Δ-6-Elongaseaktivität wurden degenerierte Primer hergestellt und mit diesen eine *Phaeodactylum* cDNA Bank mittels PCR durchsucht. Folgende Primer-Sequenzen wurden eingesetzt:

| Primer-Name | Sequenz 5'-3' Orientierung | Korrespondierende Aminosäuren |
|---|---|---|
| Phaelo forward1 | AA(C/T)CTUCTUTGGCTUTT(C/T)TA (SEQ ID NO. 185) | NLLWLFY |
| Phaelo reverse1 | GA(C/T)TGUAC(A/G)AA(A/G)AA(C/T)TGUG C(A/G)AA (SEQ ID NO. 186) | FAQFFVQS |

Nukleotidbasen in Klammern bedeuten, dass eine Mischung von Oligonukleotiden mit jeweils der einen oder anderen Nukleotidbase vorliegen.

Herstellung der *Phaeodactylum* cDNA Bank:

Eine 2 L Kultur von P. tricornutum UTEX 646 wurde in f/2 Medium (Guillard, R.R.L. 1975. Culture of phytoplankton for feeding marine invertebrates. In Culture of Marine Invertebrate Animals (Eds. Smith, W.L. and Chanley, M.H.), Plenum Press, New York, pp 29-60.) für 14 d (= Tage) bei einer Lichtstärke von 35 E/$cm^2$ angezogen. Gefrorene Zellen wurden nach Zentrifugation in der Gegenwart von flüssigem Stickstoff zu einem feinen Pulver gemahlen und mit 2 mL Homogenisierungspuffer (0,33 M Sorbitol, 0,3 M NaCl, 10 mM EDTA, 10 mM EGTA, 2% SDS, 2% Mercaptoethanol in 0,2 M Tris-Cl ph 8,5) resuspendiert. Nach Zugabe von 4 mL Phenol und 2 mL Chloroform wurde 15 min kräftig bei 40-50 °C geschüttelt. Anschliessend wurde zentrifugiert (10 min x 10000g) und die wässerige Phase schrittweise mit Chloroform

extrahiert. Nukleinsäuren wurden dann durch Zugabe von 1/20 Volumen 4 M Natriumhydrogencarbonatlösung gefällt und zentrifugiert. Das Pellet wurde in 80 mM Tris-borat pH 7,0 und 1 mM EDTA aufgenommen und die RNA mit 8 M Lithiumclorid gefällt. Nach Zentrifugation und Waschen mit 70%igem Ethanol wurde das RNA-Pellet mit Rnase-freiem Wasser aufgenommen. Poly(A)-RNA wurde mit Dynabeads (Dynal, Oslo, Norwegen) nach Herstellerangaben isoliert und die Erst-Strang-cDNA-Synthese mit MLV-Rtase von Roche (Mannheim) durchgeführt. Die Zweit-Strang-Synthese erfolgte dann mittels DNA Polymerase I und Klenow Fragment, gefolgt von einem RnaseH Verdau. Die cDNA wurde mit T4 DNA Polymerase behandelt und anschliessend EcoRI/XhoI Adaptoren (Pharmacia, Freiburg) mittels T4 Ligase angehängt. Nach XhoI Verdau, Phosphorylierung und Geltrennung wurden Fragmente grösser als 300 bp entsprechend der Herstellerangaben in den lambda ZAP Express Phagen ligiert (Stratagene, Amsterdam, Niederlande). Nach Massenexcision der cDNA-Bank und Plasmid-Rückgewinnung wurde die Plasmid-Bank in E. coli DH10B Zellen transformiert und zur PCR-Sichtung eingesetzt.

Mittels den oben genannten degenerierten Primern konnte das PCR-Fragment mit der Sequenznummer SEQ ID NO: 187 generiert werden.

Dieses Fragment wurde mit Digoxigenin markiert (Roche, Mannheim) und als Sonde für die Sichtung der Phagen-Bank verwendet.

Mit Hilfe der Sequenz SEQ ID NO: 187 konnte die Gensequenz SEQ ID NO: 183 erhalten werden, die das Volllängen-RNA-Molekül der Δ6-Elongase von Phaeodactylum darstellt:

Beispiel 53: Klonierung von Expressionsplasmiden zur heterologen Expression in - Hefen

Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der PtELO6-DNA wurde jeweils mit 1 μL cDNA, 200 μM dNTPs, 2,5 U Advantage-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name und SEQ ID NO: | Primersequenz |
|---|---|
| PtELO6 (SEQ ID NO: 183) | F:5'-GCGGCCGCACATAATGATGGTACCTTCAAG (SEQ ID NO: 188)<br>R:3'- GAAGACAGCTTAATAGACTAGT (SEQ ID NO: 189) |
| *F=forward primer, R=reverse primer | |

Die PCR Produkte wurden für 30 min bei 21 °C mit dem Hefe-Expressionsvektor - pYES2.1-TOPO (Invitrogen) gemäß Herstellerangaben inkubiert. Das PCR-Produkt (siehe SEQ ID NO: 192) wurde dabei durch einen T-Überhang und Aktivität einer Topoisomerase (Invitrogen) in den Vektor ligiert. Nach der Inkubation erfolgte dann die Transformation von E. coli DH5a Zellen. Entsprechende Klone wurden durch PCR - identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1 und pYES2.1-PtELO6. Nach der Selektion wurden je zwei Transformaten zur weiteren funktionellen Expression ausgewählt.

Beispiel 54: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu wird mit folgendem Primerpaar NotI-Schnittstellen am 5' und 3'-Ende der kodierenden Sequenz eingefügt:.
PSUN-PtELO6

Forward: 5'-GCGGCCGCACCATGATGGTACCTTCAAGTTA (SEQ ID NO: 190)
Reverse: 3'-GAAGACAGCTTAATAGGCGGCCGC (SEQ ID NO: 191)

Zusammensetzung des PCR-Ansatzes (50 μL):

5,00 μL Template cDNA

5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschließend werden Vektor und PCR-Produkte ligiert. Dazu wird das Rapid Ligation Kit von Roche verwendet. Das entstandene Plasmide pSUN-PtELO wird durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–
GTCGACCCGCGGACTAGTGGGCCCTCTAGACCCGGGGGATCC
GGATCTGCTGGCTATGAA–3'; SEQ ID NO: 151).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 55: Expression von PtElo in Hefen

Hefen, die wie unter Beispiel 4 mit den Plasmiden pYES2 und pYES2-PtELO6 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys.

388(2):293-298 und Michaelson et al., 1998, FEBS Letters. 439(3):215-218.

Beispiel 56: Funktionelle Charakterisierung von PtELO6:

In Figur 29 ist die Umsetzung von C18:$3^{\Delta 6,9,12}$ und C18:$4^{\Delta 6,9,12,15}$ wiedergegeben. Die Substrate werden um je zwei Kohlenstoffatome elongiert es entstehen jeweils die Fettsäuren C20:$3^{\Delta 8,11,14}$ bzw. C20:$4^{\Delta 8,11,14,17}$. Die Substratspezifität von PtELO6 konnte nach Expression und Fütterung verschiedener Fettsäuren ermittelt werden (Fig. 30). Die gefütterten Substrate sind in großen Mengen in allen transgenen Hefen nachzuweisen. Die transgenen Hefen zeigten die Synthese neuer Fettsäuren, den Produkten der PtElo6-Reaktion. Dies bedeutet, dass das Gen PtELO6 funktional exprimiert werden konnte.

Tabelle 21 zeigt, dass die PtElo6 eine enge Substratspezifität aufweist. PtELO6 konnte nur die C18-Fettsäuren Linolsäure, Linolensäure, γ-Linolensäure und Stearidonsäure elongieren, bevorzugte aber die ω-3-desaturierte Stearidonsäure (siehe auch Figur 30).

Fütterungsexperiment: Fettsäuren (fett) wurden jeweils mit 250 μM zugegeben. Die unterstrichenen Fettsäuren wurden neu gebildet.

Tabelle 21: Substratspezifität der PtElo6

| **gefütterte Fettsäure:** | | **+ 18:2** | **+ 18:3** | **+ 18:3** | **+ 18:4** |
|---|---|---|---|---|---|
| 16:0 | 16,2 | 18,2 | 15,2 | 20 | 04:48 |
| 16:1 | 50,6 | 20,5 | 22,8 | 33,5 | 34,2 |
| 18:0 | 5,4 | 6,3 | 6,2 | 5,2 | 12,4 |
| 18:1 | 27,7 | 14,6 | 19,6 | 19,3 | 16,7 |
| 18:2 | | 40 | | | |
| 18:3 | | | 32,9 | | |
| 18:3 | | | | 12,3 | |
| 18:4 | | | | | 4,5 |
| 20:2 | | <u>0,4</u> | | | |
| 20:3 | | | <u>3,4</u> | | |
| 20:3 | | | | <u>9,7</u> | |
| 20:4 | | | | | <u>14,5</u> |
| **% Elongation** | **0,0** | **0,99** | **9,37** | **44,09** | **76,32** |

Folgende Fettsäuren wurden gefüttert, aber nicht umgesetzt:

- 18:$1^{\Delta 6}$, 18:$1^{\Delta 9}$, 18:$1^{\Delta 11}$
- 20:$2^{\Delta 11,14}$, 20:$3^{\Delta 11,14,17}$, 20:$3^{\Delta 8,11,14}$, 20:$4^{\Delta 5,8,11,14}$, 20:$5^{\Delta 5,8,11,14,17}$
- 22:$4^{\Delta 7,10,13,16}$

Die Hefen, die mit dem Vektor pYES2-PtELO6 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. So wurden die Ergebnisse, die in den Figuren 29 und 30 sowie in der Tabelle 19 dargestellt wurden, ermittelt.

Beispiel 57: Klonierung von Expressionsplasmiden zur Samen-spezifischen Expression in Pflanzen

Die folgenden beschriebenen allgemeinen Bedingungen gelten für alle nachfolgenden Versuche, wenn nicht anders beschrieben.

Erfindungsgemäß bevorzugt verwendet werden für die folgenden Beispiele Bin19, pBI101, pBinAR, pGPTV und pCAMBIA. Eine Übersicht über binäre Vektoren und ihre Verwendung gibt Hellens et al, Trends in Plant Science (2000) 5, 446-451. Verwendet wurde ein pGPTV-Derivat wie in DE10205607 beschrieben. Dieser Vektor unterscheidet sich von pGPTV durch eine zusätzlich eingefügte Ascl-Restriktionsschnittstelle.

Ausgangspunkt der Klonierung war der Klonierungsvektor pUC19 (Maniatis et al.). Im ersten Schritt wurde das Conlinin-Promotor-Fragment mit folgenden Primern amplifiziert:

CnI1 C 5': gaattcggcgcgccgagctcctcgagcaacggttccggcggtatagagttgggtaattcga
CnI1 C 3': cccgggatcgatgccggcagatctccaccatttttggtggtgat

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Eco*RI und dann für 12 h bei 25°C mit dem Restriktionsenzym *Sma*I inkubiert. Der Klonierungsvektor pUC19 wurde in gleicher Weise inkubiert. Anschließend wurden das PCR-Produkt und der 2668 bp große, geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1-C wurde durch Sequenzierung verifiziert.

Im nächsten Schritt wurde der OCS-Terminator (Genbank Accession V00088; De Greve, H., Dhaese, P., Seurinck, J., Lemmers, M., Van Montagu, M. and Schell, J. Nucleotide sequence and transcript map of the Agrobacterium tumefaciens Ti plasmid-encoded octopine synthase gene J. Mol. Appl. Genet. 1 (6), 499-511 (1982)) aus dem Vektor pGPVT-USP/OCS
(DE 102 05 607) mit den folgenden Primern amplifiziert:

OCS_C 5': aggcctccatggcctgctttaatgagatatgcgagacgcc
OCS_C 3': cccgggccggacaatcagtaaattgaacggag

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 12 h bei 25°C mit dem Restriktionsenzym *Sma*I inkubiert. Der Vektor pUC19-CnI1-C wurde 12 h bei 25°C mit dem Restriktionsenzym *Sma*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1C_OCS wurde durch Sequenzierung verifiziert.

Im nächsten Schritt wurde der CnI1-B Promotor durch PCR mittels folgender Primer amplifiziert:

CnI1-B 5': aggcctcaacggttccggcggtatag
CnI1-B 3': cccggggttaacgctagcgggcccgatatcggatcccattttttggtggtgattggttct

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 12 h bei 25°C mit dem Restriktionsenzym *Sma*I inkubiert. Der Vektor pUC19-CnI1-C wurde 12 h bei 25°C mit dem Restriktionsenzym *Sma*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_C_CnI1B_OCS wurde durch Sequenzierung verifiziert.

In einem weiteren Schritt wurde der OCS-Terminator für CnI1B eingefügt. Dazu wurde die PCR mit folgenden Primer durchgeführt:

OCS2 5': aggcctcctgctttaatgagatatgcgagac
OCS2 3': cccgggcggacaatcagtaaattgaacggag

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 12 h bei 25°C mit dem Restriktionsenzym *Sma*I inkubiert. Der Vektor pUC19-CnI1C_CnI1B_OCS wurde für 12 h bei 25°C mit dem Restriktionsenzym *Sma*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1C_CnI1B_OCS2 wurde durch Sequenzierung verifiziert.

Im nächsten Schritt wurde der CnI1-A Promotor durch PCR mittels folgender Primer amplifiziert:

CnI1-B 5': aggcctcaacggttccggcggtatagag

CnI1-B 3': aggccttctagactgcaggcggccgcccgcatttttggtggtgattggt

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym *Stu*I inkubiert. Der Vektor pUC19-CnI1-C wurde für 12 h bei 25°C mit dem Restriktionsenzym *Sma*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1C_CnI1B_CnI1A_OCS2 wurde durch Sequenzierung verifiziert.

In einem weiteren Schritt wurde der OCS-Terminator für CnI1A eingefügt. Dazu wurde die PCR mit folgenden Primer durchgeführt:

OCS2 5': ggcctcctgctttaatgagatatgcga
OCS2 3': aagcttggcgcgccgagctcgtcgacggacaatcagtaaattgaacggaga

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 2 h bei 37°C mit dem Restriktionsenzym *Hin*dIII inkubiert. Der Vektor pUC19-CnI1C_CnI1B_CnI1A_OCS2 wurde für 2 h bei 37°C mit dem Restriktionsenzym *Stu*I und für 2 h bei 37°C mit dem Restriktionsenzym *Hin*dIII inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1C_CnI1B_CnI1A_OCS3 wurde durch Sequenzierung verifiziert.

Das Plasmid pUC19-CnI1C_CnI1B_CnI1A_OCS3 wurde im nächsten Schritt zur Klonierung der Δ6-, Δ5-Desaturase und Δ6-Elongase verwendet. Dazu wurde die Δ6-Desaturase aus Phytium irregulare (WO02/26946) mit folgenden PCR-Primern amplifiziert:

D6Des(Pir) 5': agatctatggtggacctcaagcctggagtg
D6Des(Pir) 3': ccatggcccgggttacatcgctgggaactcggtgat

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Bgl*II und dann für 2 h bei 37°C mit dem Restriktionsenzym *Nco*I inkubiert. Der Vektor pUC19-CnI1C_CnI1B_CnI1A_OCS3 wurde für 2 h bei 37°C mit dem Restriktionsenzym *Bgl*II und für 2 h bei 37°C mit dem Restriktionsenzym *Nco*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_d6Des(Pir) wurde durch Sequenzierung verifiziert.

Das Plasmid pUC19-CnI1_d6Des(Pir) wurde im nächsten Schritt zur Klonierung der Δ5-Desaturase aus Thraustochytrium ssp. (WO02/26946) verwendet. Dazu wurde die Δ5-Desaturase aus Thraustochytrium ssp. mit folgenden PCR-Primern amplifiziert:

D5Des(Tc) 5': gggatccatgggcaagggcagcgagggccg
D5Des(Tc) 3': ggcgccgacaccaagaagcaggactgagatatc

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Bam*HI und dann für 2 h bei 37°C mit dem Restriktionsenzym EcoRV inkubiert. Der Vektor pUC19-CnI1_d6Des(Pir) wurde für 2 h bei 37°C mit dem Restriktionsenzym *Bam*HI und für 2 h bei 37°C mit dem Restriktionsenzym EcoRV inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_d6Des(Pir)_d5Des(Tc) wurde durch Sequenzierung verifiziert.

Das Plasmid pUC19-CnI1_d6Des(Pir)_d5Des(Tc) wurde im nächsten Schritt zur Klonierung der Δ6-Elongase aus Physcomitrella patens (WO01/59128) verwendet, wozu diese mit folgenden PCR-Primern amplifiziert wurde:

D6Elo(Pp) 5': gcggccgcatggaggtcgtggagagattctacggtg
D6Elo(Pp) 3': gcaaaagggagctaaaactgagtgatctaga

Zusammensetzung des PCR-Ansatzes (50 μl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Not*I und dann für 2 h bei 37°C mit dem Restriktionsenzym *Xba*I inkubiert. Der Vektor pUC19-CnI1_d6Des(Pir)_d5Des(Tc) wurde für 2 h bei 37°C mit dem Restriktionsenzym *Not*I und für 2 h bei 37°C mit dem Restriktionsenzym *Xba*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_d6Des(Pir)_d5Des(Tc)_D6Elo(Pp) wurde durch Sequenzierung verifiziert.

Ausgehend von pUC19-CnI1_d6Des(Pir)_d5Des(Tc)_D6Elo(Pp) wurde der binäre Vektor für die Pflanzentransformation hergestellt. Dazu wurde pUC19-CnI1_d6Des(Pir)_d5Des(Tc)_D6Elo(Pp) für 2 h bei 37°C mit dem Restriktionsenzym AscI inkubiert. Der Vektor pGPTV wurde in gleicher Weise behandelt. Anschließend wurden das Fragment aus pUC19-CnI1_d6Des(Pir)_d5Des(Tc)_D6Elo(Pp) und der geschnittene pGPTV-Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pGPTV-CnI1_d6Des(Pir)_d5Des(Tc)_D6Elo(Pp) wurde durch Sequenzierung verifiziert.

Ein weiteres Konstrukt, pGPTV- CnI1_d6Des(Pir)_d5Des(Tc)_D6Elo(Pp)_D12Des(Co), fand Verwendung. Dazu wurde ausgehend von pUC19-CnI1C_OCS mit folgenden Primern amplifiziert:

CnI1_OCS 5': gtcgatcaacggttccggcggtatagagttg
CnI1_OCS 3': gtcgatcggacaatcagtaaattgaacggaga

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym *Sal*I inkubiert. Der Vektor pUC19 wurde für 2 h bei 37°C mit dem Restriktionsenzym *Sal*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_OCS wurde durch Sequenzierung verifiziert.

In einem weiteren Schritt wurde das Δ12-Desaturase-Gen aus Calendula officinalis (WO01/85968) in pUC19-CnI1_OCS kloniert. Dazu wurde d12Des(Co) mit folgenden Primern amplifiziert:

D12Des(Co) 5': agatctatgggtgcaggcggtcgaatgc
D12Des(Co) 3': ccatggttaaatcttattacgatacc

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym *Bgl*II und anschließend für 2 h bei gleicher Temperatur mit *Nco*I inkubiert. Der Vektor pUC19-CnI1_OCS wurde in gleicher Weise inkubiert. Anschließend wurden das PCR-Fragment und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_D12Des(Co) wurde durch Sequenzierung verifiziert.

Das Plasmid pUC19-CnI1_D12Des(Co), sowie das Plasmid pUC19-CnI1_d6Des(Pir)_d5Des(Tc)_D6Elo(Pp) wurden für 2 h bei 37°C mit dem Restriktionsenzym inkubiert. Anschließend wurde das Vektor-Fragment sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und Vektor-Fragment ligiert. Dazu wurde das Rapid Ligation Kit von Roche verwendet. Das entstandene Plasmid pUC19-CnI1_d6Des(Pir)_d5Des(Tc)_D6Elo(Pp)_D12Des(Co) wurde durch Sequenzierung verifiziert.

Ausgehend von pUC19-CnI1_d6Des(Pir)_d5Des(Tc)_D6Elo(Pp)_D12Des(Co) wurde der binäre Vektor für die Pflanzentransformation hergestellt. Dazu wurde pUC19-CnI1_d6Des(Pir)_d5Des(Tc)_D6Elo(Pp)_D12Des(Co) für 2 h bei 37°C mit dem Restriktionsenzym AscI inkubiert. Der Vektor pGPTV wurde in gleicher Weise behandelt. Anschließend wurden das Fragment aus pUC19-CnI1_d6Des(Pir)_d5Des(Tc)_D6Elo(Pp)_D12Des(Co) und der geschnittene pGPTV-Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pGPTV- CnI1_d6Des(Pir)_d5Des(Tc)_D6Elo(Pp)_D12Des(Co) wurde durch Sequenzierung verifiziert.

Ein weiterer für die Pflanzentransformation geeigneter Vektor ist pSUN2. Um die Zahl der im Vektor enthaltenen Expressionskassetten auf mehr als vier zu erhöhen wurde dieser Vektor in Kombination mit dem Gateway-System (Invitrogen, Karlsruhe) verwendet. Dazu wurde in den Vektor pSUN2 gemäss Herstellerangaben die Gateway-Kassette A wie folgendermassen beschrieben, eingefügt:
Der pSUN2 Vektor (1 μg) wurde 1 h mit dem Restriktionsenzym EcoRV bei 37° inkubiert. Anschliessend wurde die Gateway-Kassette A (Invitrogen, Karlsruhe) in den geschnittene Vektor ligiert mittels des Rapid Ligation Kits von Roche, Mannheim. Das entstandene Plasmid wurde in E. coli DB3.1 Zellen (Invitrogen) transformiert. Das insolierte Plasmid pSUN-GW wurde anschliessend durch Sequenzierung verifiziert.

Im zweiten Schritt wurde die Expressionskassette aus pUC19-CnI1_ d6Des(Pir)_d5Des(Tc)_D6Elo(Pp)_D12Des(Co) mittels AscI ausgeschnitten und in den in gleicherweise behandelten Vektor pSUN-GW ligiert. Das so entstandene Plasmid pSUN-4G wurde für weitere Genkonstrukte verwendet.

Dazu wurde zuerst gemäss Herstellerangaben (Invitrogen) ein pENTR-Klon modifiziert. Das Plasmid pENTR1A (Invitrogen) wurde 1 h bei 37° mit dem Restriktionsenzym EcorI inkubiert, anschliessend für 30 min mit Klenow-Enzym, sowie einem 1 μM dNTP-Mix behandelt und dann der AscI-Adapter (5'-ggcgcgcc; am 5'-Ende phosphoryliert, doppelsträngig) in den pENTR1A-Vektor liegiert. In diesen modifierzerten wurde wie oben beschrieben schrittweise Gene in die CnI-Kassette eingefügt und über AscI in den pENTR-Vektor übertragen.

In dieser beschriebenen Art und Weise wurde das Gen TL16y2 aus Thraustochytrium ssp. (SEQ ID No. 83) in den pSUN-4G Vektor übertragen:
Das Plasmid pUC19-CnI1C_CnI1B_CnI1A_OCS3 wurde im nächsten Schritt zur Klonierung der Δ5-Elongase TL16y2 verwendet. Dazu wurde die Δ5-Elongase aus Thraustochytrium ssp. mit folgenden PCR-Primern amplifiziert:

TL16y2 5': agatct atggacgtcgtcgagcagca
TL16y2 3': ccatggcccggg agaagcagaagaccatctaa

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Bgl*II und dann für 2 h bei 37°C mit dem Restriktionsenzym *Nco*I inkubiert. Der Vektor pUC19-CnI1C_CnI1B_CnI1A_OCS3 wurde für 2 h bei 37°C mit dem Restriktionsenzym *Bgl*II und für 2 h bei 37°C mit dem Restriktionsenzym *Nco*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_TL16y2 wurde durch Sequenzierung verifiziert. Anschliessend wurde die Kassette mit AscI ausgeschnitten und in einen mit AscI vorbehandelten pENTR-Vektor ligiert. Das entstandene Plasmid pENTR-CnI1_TL16y2 wurde dann gemäss Herstellerangaben (Invitrogen) in einer Rekombinationsreaktion mit dem Vektor pSUN-4G inkubiert. Das Produkt ergab den Vektor pSUN-5G, der für die Pflanzentransformation eingesetzt wurde.
In einem weiteren Schritt wurde das Konstrukt pSUN-8G mittels derselben beschriebenen Methodik erstellt. Dazu wurden 5'- und 3'-Primer für die Gene SEQ ID 41, 53, 87 und 113 mit den oben beschriebenen Restriktionsschnittstellen sowie den ersten und jeweils letzten 20 Nukleotiden des offenen Leserahmens erstellt und mit den Standardbedingungen (siehe oben) amplifiziert und in den pENTR-CnI-Vektor ligiert.
Durch Rekombinationsreaktion mit dem Vektor pSUN-4G konnte so das Konstrukt pSUN-8G erstellt werden. Auch dieser Vektor wurde für die Pflanzentransformation eingesetzt.

Beispiel 58: Erzeugung von transgenen Pflanzen

a) Erzeugung transgener Sareptasenfpflanzen. Es wurde das Protokoll zur Transformation von Rapspflanzen verwendet (verändert nach Moloney et al., 1992, Plant Cell Reports, 8:238-242)

Zur Erzeugung transgener Pflanzen wurden die erzeugten binäre Vektoren pGPTV-CnI1_d6Des(Pir)_d5Des(Tc)_D6Elo(Pp)_D12Des(Co), pSUN-5G und pSUN-8G in Agrobacterium tumefaciens C58C1:pGV2260 transformiert (Deblaere et al, 1984, Nucl. Acids. Res. 13, 4777-4788). Zur Transformation von Sareptasenfpflanzen wurde eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) verwendet. Petiolen oder Hypokotyledonen frisch gekeimter steriler Pflanzen (zu je ca. 1 $cm^2$) wurden in einer Petrischale mit einer 1:50 Agrobakterienverdünnung für 5-10 Minuten inkubiert. Es folgt eine 3-tägige Coinkubation in Dunkelheit bei 25°C auf 3MS-Medium mit 0,8 % Bacto-Agar. Die Kultivierung wurde anschließend mit 16 Stunden Licht / 8 Stunden Dunkelheit und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln dem Medium zugegeben.
Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Elongase-Expression wie Δ-6-Elongaseaktivität oder Δ-5- oder Δ-6-Desaturaseaktivität mittels Lipidanalysen untersucht. Linien mit erhöhten Gehalten an C20- und C22 mehrfach ungesättigten Fettsäuren wurden so identifiziert.
Mit diesem Protokoll wurden auch transgene Rapspflanzen erfolgreich hergestellt.

b) Herstellung von transgenen Leinpflanzen

Die transgenen Leinpflanzen können zum Beispiel nach der Methode von Bell et al., 1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 durchgeführt werden.

Beispiel 59: Lipidextraktion aus Samen:

Die Auswirkung der genetischen Modifikation in Pflanzen auf die Produktion einer gewünschten Verbindung (wie einer Fettsäure) kann bestimmt werden, indem die modifizierte Pflanze unter geeigneten Bedingungen (wie den vorstehend beschriebenen) gezüchtet wird und das Medium und/oder die zellulären Komponenten auf die erhöhte Produktion des gewünschten Produktes (d.h. der Lipide oder einer Fettsäure) untersucht werden. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22): 12935-12940, und Browse et al. (1986) Analytic Biochemistry 152:141-145 beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan für 1h bei 90°C hydrolysiert und die Lipide transmethyliert. Die resultierenden Fettsäuremethylester (FAME) werden in Petrolether extrahiert. Die extrahierten FAME

werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Beispiel 60: Analyse der Samen von den erzeugten transgenen Pflanzen

Entsprechend Beispiel 59, wurden die Samen der Pflanzen, die mit den Konstrukten pGPTV-CnI1_d6Des(Pir)_d5Des(Tc)_D6Elo(Pp)_D12Des(Co), pSUN-5G und pSUN-8G transformiert wurden, analysiert. FigurXX zeigt dabei das Fettsäurespektrum von Samen mit dem Konstrukt pGPTV-CnI1_d6Des(Pir)_d5Des(Tc)_D6Elo(Pp)_D12Des(Co). Im Vergleich zu KontrollPflanzen, die nicht transformiert wurden (Wildtyp-Kontrolle, WT) konnte eine deutliche Veränderung im Fettsäurespektrum festgestellt werden. Damit konnte gezeigt werden, dass die transformierten Gene funktionell sind. Tabelle 22 fasst die Ergebnisse aus Figur 32 zusammen.

Tabelle 22:

| Linien | Fettsäuren | | | | | | | | |
|---|---|---|---|---|---|---|---|---|---|
| | 16:0 | 18:0 | 18:1 | 18:2 | GLA | 18:3 | SDA | ARA | EPA |
| WT Kontrolle | 5,6 | 6,5 | 31,7 | 41,7 | nd | 12,1 | nd | nd | nd |
| 1424_Ko82_4 | 6,6 | 1,5 | 8,9 | 10,5 | 42,2 | 3,1 | 2,8 | 17,2 | 0,2 |
| 1424_Ko82_5 | 6,1 | 1,5 | 11,0 | 9,0 | 40,6 | 2,9 | 4,0 | 15,0 | 1,5 |
| 1424_Ko82_6 | 5,7 | 1,6 | 15,5 | 10,6 | 37,1 | 3,0 | 3,2 | 14,6 | 0,2 |
| 1424_Ko82_7 | 5,4 | 2,0 | 20,4 | 10,7 | 32,6 | 3,5 | 3,2 | 12,1 | 1,0 |
| 1424_Ko82_8 | 5,4 | 1,4 | 15,1 | 12,5 | 39,9 | 2,6 | 2,4 | 12,2 | 0,7 |
| 1424_Ko82_9 | 6,0 | 1,8 | 25,0 | 9,9 | 29,7 | 2,2 | 2,5 | 10,2 | 0,8 |
| 1424_Ko82_10 | 5,7 | 1,3 | 10,1 | 10,3 | 42,5 | 2,6 | 3,5 | 13,9 | 1,1 |
| 1424_Ko82_11 | 5,4 | 1,4 | 15,7 | 11,3 | 38,2 | 2,6 | 2,8 | 14,1 | 1,0 |

Die Analyse der Samen mit dem Konstrukt pSUN-5G zeigt dabei Linien, die eine deutliche Erhöhung des Gehaltes an Arachidonsäure verglichen mit dem Konstrukt pGPTV-CnI1_d6Des(Pir)_d5Des(Tc)_D6Elo(Pp)_D12Des(Co) haben. Dabei konnten Linien mit bis zu 25 % ARA erhalten werden. Die zusätzliche Elongase (TL16y2) muss für diesen Effekt vorantwortlich sein (Figur31, pSUN-5G). Die Ergebnisse dieser Linie sind in Tabelle 23 zusammengefasst.

Tab. 23: Fettsäureanalytik von transgenen Samen, die mit dem Konstrukt pSUN-5G transformiert wurden.

| Linien | Fettsäuren | | | | | | | | |
|---|---|---|---|---|---|---|---|---|---|
| | 16:0 | 18:0 | 18:1 | 18:2 LA | 18:3 GLA | 18:3 ALA | 18:4 SDA | 20:3 HGLA | ARA | EPA |
| WT | 5,2 | 2,3 | 34,2 | 37,9 | 0,0 | 11,6 | 0,0 | 0,0 | 0,0 | 0,0 |
| 16-1-2 | 4,2 | 1,6 | 20,1 | 21,5 | 25,9 | 4,1 | 1,8 | 1,7 | 8,9 | 0,8 |
| 16-1-3 | 5,8 | 2,3 | 9,9 | 14,6 | 33,6 | 3,1 | 2,2 | 2,2 | 16,0 | 1,4 |
| 16-1-8 | 5,0 | 2,8 | 11,1 | 12,6 | 34,9 | 2,2 | 1,8 | 2,6 | 16,3 | 1,2 |
| 16-2-1 | 4,9 | 1,6 | 14,5 | 17,4 | 32,9 | 3,5 | 2,0 | 1,6 | 12,3 | 1,0 |
| 16-2-5 | 5,5 | 3,3 | 12,9 | 13,8 | 32,9 | 2,9 | 2,2 | 1,4 | 15,4 | 1,4 |
| 16-4-2 | 5,8 | 2,5 | 18,8 | 14,7 | 32,0 | 3,5 | 2,3 | 1,2 | 12,0 | 1,2 |
| 16-4-3 | 5,9 | 2,0 | 19,7 | 15,0 | 32,0 | 3,8 | 2,4 | 1,1 | 11,4 | 1,2 |
| 16-7-2 | 6,2 | 4,4 | 14,3 | 10,2 | 30,7 | 2,0 | 2,1 | 1,7 | 19,4 | 1,9 |
| 16-7-3 | 5,0 | 2,5 | 21,6 | 13,6 | 30,7 | 2,1 | 1,8 | 1,5 | 12,6 | 1,1 |

(fortgesetzt)

| Linien | Fettsäuren | | | | | | | | | |
|---|---|---|---|---|---|---|---|---|---|---|
| | 16:0 | 18:0 | 18:1 | 18:2 LA | 18:3 GLA | 18:3 ALA | 18:4 SDA | 20:3 HGLA | ARA | EPA |
| 16-7-4 | 5,3 | 4,1 | 18,8 | 19,5 | 23,1 | 4,2 | 2,2 | 2,9 | 11,3 | 1,4 |
| 16-7-5 | 7,4 | 1,8 | 4,2 | 6,8 | 33,7 | 1,8 | 2,7 | 2,6 | 25,8 | 2,6 |

Beispiel 61: Nachweis von DHA in Samen von transgenen Sareptasenf-Pflanzen.

Samen von Pflanzen, die mit dem Konstrukt pSUN-8G wie unter Beispiel 58 beschrieben hergestellt wurden, wurden wie in Beispiel 59 beschrieben, analysiert. Neben den LCPUFA Arachidonsäure und Eicosapentaensäure konnte in diesen Samen auch Docosahexaensäure nachgewiesen werden, das Produkt nach Umsetzung durch die Δ4-Desaturase aus Thraustochytrium und den Δ5-Elongasen aus Onchorynchis mykiss und Ostreococcus tauri. Figur 32 zeigt das Chromatogramm mit dem geänderten Fettsäurespektrum im Vergleich zu einer nicht-transformierten Kontrollpflanze. In Tabelle 24 sind die Ergebnisse mehrerer Messungen zusammengefasst.

Tabelle. 24 gibt die Fettsäureanalytik von transgenen Samen, die mit dem Konstrukt pSUN-8G transformiert wurden.

Mit diesem Experiment konnte zum ersten Mal die Synthese von Docosahexaensäure in Samen demonstriert werden. Z.B. in WO 2004/071467 wird zwar die Synthese von DHA in höheren Pflanzen beschrieben, allerdings konnte die Synthese nicht für Samen gezeigt werden, nur für eine embryogene Zellkultur.

## Patentansprüche

1. Öl-produzierende Pflanze exprimierend unter Kontrolle von Samen-spezifischen Promotoren oder Promotoren aktiv in Pflanzenembryo oder Endosperm, mindestens

   a) eine delta-6 Desaturase,
   b) eine delta-6 Elongase,

c) eine delta-5 Desaturase,
d) eine delta-12 Desaturase, und
e) eine delta-5 Elongase exprimiert

wobei die delta-12-Desaturase-, delta -6-Desaturase-, delta -5-Elongase-, delta -6-Elongase- und delta -5-Desaturase-Nukleinsäuresequenzen in einem Genkonstrukt in der Pflanze vorliegen, und wobei die Pflanze in ihrem Samen unabhängig voneinander ungesättigte C18-, C20-, oder C22-Alkylcarbonylsäuren mit mindestens drei, vier, fünf oder sechs Doppelbindungen herstellt.

**2.** Die Pflanze nach Anspruch 1, wobei die Pflanze in ihrem Samen unabhängig voneinander ungesättigte C18-, C20-, oder C22-Alkylcarbonylsäuren mit mindestens drei, vier, fünf oder sechs Doppelbindungen mit einem Gehalt von mindestens 30 Gew-% bezogen auf die gesamten Fettsäuren im Samen der Pflanze herstellt.

**3.** Pflanze nach Anspruch 1 oder 2, wobei die Pflanze unter Kontrolle von Samen-spezifischen Promotoren oder Promotoren aktiv in Pflanzenembryo oder Endosperm weiterhin delta-4-Desaturase; und/oder omega-3-Desaturase exprimiert.

**4.** Pflanze nach Anspruch 1 oder 2, wobei die Pflanze unter Kontrolle von Samen-spezifischen Promotoren oder Promotoren aktiv in Pflanzenembryo oder Endosperm weiterhin eine delta-4-Desaturase exprimiert und DHA und/oder EPA produziert.

**5.** Pflanze nach Anspruch 4, wobei die Pflanze unter Kontrolle von Samen-spezifischen Promotoren oder Promotoren aktiv in Pflanzenembryo oder Endosperm weiterhin eine omega-3-desaturase exprimiert und wobei der EPA Gehalt mindestens 5% und/oder der Docosahexaensäure-Gehalt mindestens 0,2 Gew-% bezogen auf den gesamten Lipidgehalt in den Samen der transgenen Pflanze beträgt und die Pflanze zu den Brassicaceen gehört.

**6.** Pflanze nach einem der Ansprüche 1 bis 5, wobei die Pflanze zu den Brassicaceen, Boraginaceen, Primulaceen, oder Linaceen gehören.

**7.** Transgene Pflanze gemäß einem der Ansprüche 1 bis 6, enthaltend eine Nukleinsäuresequenz, die für Polypeptide mit omega-3-Desaturaseaktivität codiert, ausgewählt aus der Gruppe bestehend aus:

a) einer Nukleinsäuresequenz mit der in SEQ ID NO: 87 oder SEQ ID NO: 105 dargestellten Sequenz,oder
b) Nukleinsäuresequenzen, die sich als Ergebnis des degenerierten genetischen Codes von der in SEQ ID NO: 88 oder SEQ ID NO: 106 dargestellten Aminosäuresequenz ableiten lassen, oder
c) Nukleinsäuresequenz, die für Polypeptide mit mindestens 60 % Identität auf Aminosäureebene mit SEQ ID NO: 88 oder SEQ ID NO: 106 codieren und eine omega-3-Desaturaseaktivität aufweisen.

**8.** Transgene Pflanze gemäß einem der Ansprüche 1 bis 7, ferner enthaltend eine Nukleinsäuresequenz, die für Polypeptide mit delta-12-Desaturaseaktivität codiert, ausgewählt aus der Gruppe bestehend aus:

a) einer Nukleinsäuresequenz mit der in SEQ ID NO: 107, SEQ ID NO: 109 oder SEQ ID NO: 195 dargestellten Sequenz,oder
b) Nukleinsäuresequenzen, die sich als Ergebnis des degenerierten genetischen Codes von der in SEQ ID NO: 108, SEQ ID NO: 110 oder SEQ ID NO: 196 dargestellten Aminosäuresequenz ableiten lassen, oder
c) Nukleinsäuresequenzen, die für Polypeptide mit mindestens 60 % Identität auf Aminosäureebene mit SEQ ID NO: 108, SEQ ID NO: 110 oder SEQ ID NO: 196 codieren und eine delta-12-Desaturaseaktivität aufweisen.

**9.** Pflanze nach einem der Ansprüche 1 bis 8, ferner enthaltend eine Nukleinsäuresequenz, die für Polypeptide mit delta-6-Desaturaseaktivität codiert, ausgewählt aus der Gruppe bestehend aus

a) Nukleinsäuresequenz codierend für eine Aminosäuresequenz gemäß SEQ ID NO 202 oder mindestens mit 40% Sequenzidentität zu der Aminosäuresequenz gemäß SEQ ID NO 202 und eine delta-6-Desaturaseaktivität aufweisend;
b) Nukleinsäuresequenzen, die sich als Ergebnis des degenerierten genetischen Codes von der in SEQ ID NO: 202 dargestellten Aminosäuresequenz ableiten lassen und eine delta-6-Desaturaseaktivität aufweisend; und
c) einer Nukleinsäuresequenz mit der in SEQ ID NO: 201 dargestellten Sequenz oder mindestens 40% Sequenzidentität zu der Nukleinsäuresequenz gemäß SEQ ID NO 201.

**10.** Same exprimierend unter Kontrolle von Samen-spezifischen Promotoren oder Promotoren aktiv in Pflanzenembryo oder Endosperm, mindestens

a) eine delta-6 Desaturase,
b) eine delta-6 Elongase,
c) eine delta-5 Desaturase,
d) eine delta-12 Desaturase, und
e) eine delta-5 Elongase exprimiert

wobei die delta-12-Desaturase-, delta -6-Desaturase-, delta -5-Elongase-, delta -6-Elongase- und delta -5-Desaturase-Nukleinsäuresequenzen in einem Genkonstrukt in der Pflanze vorliegen, und wobei die Pflanze in ihrem Samen unabhängig voneinander ungesättigte C18-, C20-, oder C22-Alkylcarbonylsäuren mit mindestens drei, vier, fünf oder sechs Doppelbindungen herstellt.

**11.** Same gemäß Anspruch 10, **dadurch gekennzeichnet, dass** im Samen der Pflanze der Gehalt an EPA mindestens 5 Gew.-% bezogen auf den Gesamtlipidgehalt beträgt.

**12.** Same gemäß einem der Ansprüche 10 bis 12, **dadurch gekennzeichnet, dass** im Samen der transgenen Pflanze der Gehalt an Docosahexaensäure mindestens 0,5 Gew.-% bezogen auf den Gesamtlipidgehalt beträgt.

**13.** Same gemäß einem der Ansprüche 5 bis 9, **dadurch gekennzeichnet, dass** die transgene Pflanze ausgewählt aus der Gruppe der Pflanzenfamilien:
Anacardiaceae, Asteraceae, Boraginaceae, Brassicaceae, Cannabaceae, Compositae, Cruciferae, Cucurbitaceae, Elaeagnaceae, Euphorbiaceae, Fabaceae, Geraniaceae, Gramineae, Leguminosae, Linaceae, Malvaceae, Moringaceae, Marchantiaceae, Onagraceae, Olacaceae, Oleaceae, Papaveraceae, Piperaceae, Pedaliaceae, Poaceae oder Solanaceae.

**14.** Verfahren zu Herstellung einer Öl-produzierende Pflanzenzelle exprimierend unter Kontrolle von Samen-spezifischen Promotoren oder Promotoren aktiv in Pflanzenembryo oder Endosperm

a) eine delta-6 Desaturase,
b) eine delta-6 Elongase,
c) eine delta-5 Desaturase,
d) eine delta-5 Elongase und
e) eine delta-12 Desaturase,

wobei die Pflanze in ihrem Samen unabhängig voneinander ungesättigte C18-, C20-, oder C22-Alkylcarbonylsäuren mit mindestens drei, vier, fünf oder sechs Doppelbindungen, mit einem Gehalt von mindestens 30 Gew-% bezogen auf die gesamten Fettsäuren im Samen der Pflanze herstellt, umfassend das Einbringen der genannten Gene in eine Öl-produzierende Pflanze.

**15.** Verfahren zu Herstellung einer C20- und/oder C22 Fettsäure oder eines Esters davon mit mindestens drei, vier, fünf oder sechs Doppelbindungen in einer Öl-produzierende Pflanze exprimierend unter Kontrolle von Samen-spezifischen Promotoren oder Promotoren aktiv in Pflanzenembryo oder Endosperm,

a) eine delta-6 Desaturase,
b) eine delta-6 Elongase,
c) eine delta-5 Desaturase,
d) eine delta-5 Elongase und
e) eine delta-12 Desaturase,

wobei die Pflanze in ihrem Samen unabhängig voneinander ungesättigte C18-, C20-, oder C22-Alkylcarbonylsäuren mit mindestens drei, vier, fünf oder sechs Doppelbindungen, mit einem Gehalt von mindestens 30 Gew-% bezogen auf die gesamten Fettsäuren im Samen der Pflanze herstellt, umfassend das Einbringen der genannten Gene in eine Öl-produzierende Pflanze.

**16.** Verfahren zur Herstellung eines Öls, Lipids oder einer freien Fettsäure umfassend das Gewinnen der Öle, Lipide oder freien Fettsäuren aus der Pflanze oder dem Samen nach einem der vorgenannten Ansprüche

**17.** Verfahren nach Anspruch 15 oder 16, wobei Arachidonsäure, Eicosapentaensäure, Docosapentaensäure oder Docosahexaensäure, Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden.

**18.** Verwendung einer Nukleinsäuresequenz codierend für eine Aminosäuresequenz gemäß SEQ ID NO 202 oder mindestens mit 40% Sequenzidentität zu der Aminosäuresequenz gemäß SEQ ID NO 202 und eine delta-6-Desaturaseaktivität aufweisend oder der codierten Aminosäuresequenz zur Herstellung von Öle, Lipide oder freien Fettsäuren, Pharmaka, Nahrungsmittel, Tierfutter oder Kosmetika.

**Claims**

**1.** An oil-producing plant expressing, under control of seed-specific promoters or promoters active in plant embryo or endosperm, at least

a) a delta-6 desaturase
b) a delta-6 elongase,
c) a delta-5 desaturase,
d) a delta-12 desaturase, and
e) a delta-5 elongase,

the delta-12 desaturase, delta-6 desaturase, delta-5 elongase, delta-6 elongase and delta-5 desaturase nucleic acid sequences being present in a gene construct in the plant, and the plant producing in its seed independently of one another unsaturated C18-, C20-, or C22-alkylcarbonyl acids having at least three, four, five or six double bonds.

**2.** The plant according to Claim 1, the plant producing in its seed independently of one another unsaturated C18-, C20-, or C22-alkylcarbonyl acids having at least three, four, five or six double bonds with a content of at least 30% by weight, based on the total fatty acids in the seed of the plant.

**3.** The plant according to claim 1 or 2, the plant further expressing, under control of seed-specific promoters or promoters active in plant embryo or endosperm, delta-4 desaturase; and/or omega-3 desaturase.

**4.** The plant according to claim 1 or 2, the plant further expressing, under control of seed-specific promoters or promoters active in plant embryo or endosperm, a delta-4 desaturase and producing DHA and/or EPA.

**5.** The plant according to claim 4, the plant further expressing, under control of seed-specific promoters or promoters active in plant embryo or endosperm, an omega-3 desaturase, and the EPA content being at least 5% and/or the docosahexaenoic acid content being at least 0.2% by weight based on the total lipid content in the seeds of the transgenic plant, and the plant belonging to the Brassicaceae.

**6.** The plant according to any of claims 1 to 5, the plant belonging to the Brassicaceae, Boraginaceae, Primulaceae, or Linaceae.

**7.** The transgenic plant according to one of claims 1 to 6, comprising a nucleic acid sequence which codes for polypeptides having omega-3-desaturase activity, selected from the group consisting of:

a) a nucleic acid sequence having the sequence shown in SEQ ID NO: 87 or SEQ ID NO: 105, or
b) nucleic acid sequences which can be derived as a result of the degenerate genetic code from the amino acid sequence shown in SEQ ID NO: 88 or SEQ ID NO: 106, or
c) nucleic acid sequences which code for polypeptides having at least 60% identity at amino acid level with SEQ ID NO: 88 or SEQ ID NO: 106, and which have an omega-3 desaturase activity.

**8.** The transgenic plant according to any of claims 1 to 7, further comprising a nucleic acid sequence which codes for polypeptides with delta-12 desaturase activity, selected from the group consisting of:

a) a nucleic acid sequence having the sequence shown in SEQ ID NO: 107, SEQ ID NO: 109 or SEQ ID NO: 195, or

b) nucleic acid sequences which can be derived as a result of the degenerate genetic code from the amino acid sequence shown in SEQ ID NO: 108, SEQ ID NO: 110 or SEQ ID NO: 196, or
c) nucleic acid sequences which code for polypeptides having at least 60% identity at amino acid level with SEQ ID NO: 108, SEQ ID NO: 110 or SEQ ID NO: 196, and which have a delta-12 desaturase activity.

**9.** The plant according to one of claims 1 to 8, further comprising a nucleic acid sequence which codes for polypeptides having delta-6 desaturase activity, selected from the group consisting of

a) nucleic acid sequence coding for an amino acid sequence according to SEQ ID NO: 202, or at least with 40% sequence identity with the amino acid sequence according to SEQ ID NO: 202, and having a delta-6 desaturase activity;
b) nucleic acid sequences which can be derived as a result of the degenerate genetic code from the amino acid seuqence shown in SEQ ID NO: 202, and having a dleta-6 desaturase activity; and
c) a nucleic acid sequence having the sequence shown in SEQ ID NO: 201 or at least 40% sequence identity with the nucleic acid sequence according to SEQ ID NO: 201.

**10.** A seed expressing, under control of seed-specific promoters or promoters active in plant embryo or endosperm, at least

a) a delta-6 desaturase,
b) a delta-6 elongase,
c) a delta-5 desaturase,
d) a delta-12 desaturase, and
e) a delta-5 elongase,

the delta-12 desaturase, delta-6 desaturase, delta-5 elongase, delta-6 elongase and delta-5 desaturase nucleic acid sequences being present in a gene construct in the plant, and the plant producing in its seed independently of one another unsaturated C18-, C20-, or C22-alkylcarbonyl acids having at least three, four, five or six double bonds.

**11.** The seed according to claim 10, wherein the content of EPA in the seed of the plant is at least 5% by weight, based on the total lipid content.

**12.** The seed according to one of claims 10 to 12, wherein the content of docosahexaenoic acid in the seed of the transgenic plant is at least 0.5% by weight, based on the total lipid content.

**13.** The seed according to one of claims 5 to 9, **characterized in that** the transgenic plant is selected from the group of the following plant families:
Anacardiaceae, Asteraceae, Boraginaceae, Brassicaceae, Cannabaceae, Compositae, Cruciferae, Cucurbitaceae, Elaeagnaceae, Euphorbiaceae, Fabaceae, Geraniaceae, Gramineae, Leguminosae, Linaceae, Malvaceae, Moringaceae, Marchantiaceae, Onagraceae, Olacaceae, Oleaceae, Papaveraceae, Piperaceae, Pedaliaceae, Poaceae or Solanaceae.

**14.** A process for producing an oil-producing plant cell expressing, under control of seed-specific promoters or promoters active in plant embryo or endosperm,

a) a delta-6 desaturase,
b) a delta-6 elongase,
c) a delta-5 desaturase,
d) a delta-5 elongase, and
e) a delta-12 desaturase,

where the plant produces in its seed, independently of one another, unsaturated C18-, C20-, or C22-alkylcarbonyl acids having at least three, four, five or six double bonds, with a content of at least 30% by weight, based on the total fatty acids in the seed of the plant, comprising the introduction of the stated genes into an oil-producing plant.

**15.** A process for producing a C20 and/or C22 fatty acid or an ester thereof having at least three, four, five or six double bonds in oil-producing plant expressing, under control of seed-specific promoters or promoters active in plant embryo or endosperm,

a) a delta-6 desaturase,
b) a delta-6 elongase
c) a delta-5 desaturase,
d) a delta-5 elongase, and
e) a delta-12 desaturase,

where the plant produces in its seed, independently of one another, unsaturated C18-,-C20-, or C22-alkylcarbonyl acids having at least three, four, five or six double bonds, with a content of at least 30% by weight, based on the total fatty acids in the seed of the plant, comprising the introduction of the stated genes into an oil-producing plant.

**16.** A process for producing an oil, lipid or a free fatty acid, comprising recovering the oils, lipids or free fatty acids from the plant or the seed according to one of the preceding claims.

**17.** The process according to claim 15 or 16, where arachidonic acid, eicosapentaenoic acid, docosapen-taenoic acid or docosahexaenoic acid, eicosapentaenoic acid and/or docosahexaenoic acid are produced and are subsequently used for various purposes in food, feed, cosmetic or pharmaceutical applications.

**18.** The use of a nucleic acid sequence coding for an amino acid sequence according to SEQ ID NO: 202 or at least having 40% sequence identity with the amino acid sequence according to SEQ ID NO: 202, and having a delta-6-desaturase activity, or the encoded amino acid sequence for producing oils, lipids or free fatty acids, pharmaceuticals, foodstuffs, animal feed or cosmetics.

**Revendications**

**1.** Plante produisant de l'huile exprimant sous le contrôle de promoteurs spécifiques aux graines ou de promoteurs actifs dans l'embryon de la plante ou l'endosperme, au moins :

a) une delta-6 désaturase,
b) une delta-6 élongase,
c) une delta-5 désaturase,
d) une delta-12 désaturase et
e) une delta-5 élongase,

les séquences d'acides nucléiques de la delta-12 désaturase, de la delta-6 désaturase, de la delta-5 élongase, de la delta-6 élongase et de la delta-5 désaturase se présentant dans une construction génique dans la plante, et la plante fabriquant dans ses graines indépendamment les uns des autres des acides alkylcarbonyliques en C18, C20 ou C22 insaturés contenant au moins trois, quatre, cinq ou six doubles liaisons.

**2.** Plante selon la revendication 1, la plante fabriquant dans ses graines indépendamment les uns des autres des acides alkylcarbonyliques en C18, C20 ou C22 insaturés contenant au moins trois, quatre, cinq ou six doubles liaisons en une teneur d'au moins 30 % en poids, par rapport à la totalité des acides gras dans les graines de la plante.

**3.** Plante selon la revendication 1 ou 2, la plante exprimant sous le contrôle de promoteurs spécifiques aux graines ou de promoteurs actifs dans l'embryon de la plante ou l'endosperme également une delta-4 désaturase et/ou une oméga-3 désaturase.

**4.** Plante selon la revendication 1 ou 2, la plante exprimant sous le contrôle de promoteurs spécifiques aux graines ou de promoteurs actifs dans l'embryon de la plante ou l'endosperme également une delta-4 désaturase, et produisant du DHA et/ou de l'EPA.

**5.** Plante selon la revendication 4, la plante exprimant sous le contrôle de promoteurs spécifiques aux graines ou de promoteurs actifs dans l'embryon de la plante ou l'endosperme également une oméga-3 désaturase, et la teneur en EPA étant d'au moins 5 % et/ou la teneur en acide docosahexaénoïque étant d'au moins 0,2 % en poids, par rapport à la teneur totale en lipides dans les graines de la plante transgénique, et la plante appartenant aux brassicacées.

**6.** Plante selon l'une quelconque des revendications 1 à 5, la plante appartenant aux brassicacées, aux boraginacées,

aux primulacées ou aux linacées.

7. Plante transgénique selon l'une quelconque des revendications 1 à 6, contenant une séquence d'acides nucléiques, qui code pour des polypeptides à activité oméga-3 désaturase, choisie dans le groupe constitué par :

a) une séquence d'acides nucléiques ayant la séquence indiquée dans SEQ ID NO : 87 ou SEQ ID NO : 105, ou
b) les séquences d'acides nucléiques qui peuvent être dérivées en tant que résultat du code génétique dégénéré de la séquence d'acides aminés indiquée dans SEQ ID NO : 88 ou SEQ ID NO : 106, ou
c) une séquence d'acides nucléiques qui code pour des polypeptides ayant au moins 60 % d'identité sur le plan des acides aminés avec SEQ ID NO : 88 ou SEQ ID NO : 106, et présentant une activité oméga-3 désaturase.

8. Plante transgénique selon l'une quelconque des revendications 1 à 7, contenant en outre une séquence d'acides nucléiques qui code pour des polypeptides à activité delta-12 désaturase, choisie dans le groupe constitué par :

a) une séquence d'acides nucléiques ayant la séquence indiquée dans SEQ ID NO : 107, SEQ ID NO : 109 ou SEQ ID NO : 195, ou
b) les séquences d'acides nucléiques qui peuvent être dérivées en tant que résultat du code génétique dégénéré de la séquence d'acides aminés indiquée dans SEQ ID NO : 108, SEQ ID NO : 110 ou SEQ ID NO : 196, ou
c) les séquences d'acides nucléiques qui codent pour des polypeptides ayant au moins 60 % d'identité sur le plan des acides aminés avec SEQ ID NO : 108, SEQ ID NO : 110 ou SEQ ID NO : 196, et présentant une activité delta-12 désaturase.

9. Plante selon l'une quelconque des revendications 1 à 8, contenant en outre une séquence d'acides nucléiques qui code pour des polypeptides à activité delta-6 désaturase, choisie dans le groupe constitué par :

a) une séquence d'acides nucléiques codant pour une séquence d'acides aminés selon SEQ ID NO 202 ou ayant au moins 40 % d'identité de séquence avec la séquence d'acides aminés selon SEQ ID NO 202 et présentant une activité delta-6-désaturase ;
b) les séquences d'acides nucléiques qui peuvent être dérivées en tant que résultat du code génétique dégénéré de la séquence d'acides aminés indiquée dans SEQ ID NO : 202, et présentant une activité delta-6 désaturase ; et
c) une séquence d'acides nucléiques ayant la séquence indiquée dans SEQ ID NO : 201 ou ayant au moins 40 % d'identité de séquence avec la séquence d'acides nucléiques selon SEQ ID NO 201.

10. Graine exprimant sous le contrôle de promoteurs spécifiques aux graines ou de promoteurs actifs dans l'embryon de la plante ou l'endosperme, au moins :

a) une delta-6 désaturase,
b) une delta-6 élongase,
c) une delta-5 désaturase,
d) une delta-12 désaturase et
e) une delta-5 élongase,

les séquences d'acides nucléiques de la delta-12 désaturase, de la delta-6 désaturase, de la delta-5 élongase, de la delta-6 élongase et de la delta-5 désaturase se présentant dans une construction génique dans la plante, et la plante fabriquant dans ses graines indépendamment les uns des autres des acides alkylcarbonyliques en C18, C20 ou C22 insaturés contenant au moins trois, quatre, cinq ou six doubles liaisons.

11. Graine selon la revendication 10, **caractérisée en ce que** la teneur en EPA dans les graines de la plante est d'au moins 5 % en poids, par rapport la teneur totale en lipides.

12. Graine selon l'une quelconque des revendications 10 à 12, **caractérisée en ce que** la teneur en acide docosahexaénoïque dans les graines de la plante transgénique est d'au moins 0,5 % en poids, par rapport à la teneur totale en lipides.

13. Graine selon l'une quelconque des revendications 5 à 9, **caractérisée en ce que** la plante transgénique est choisie dans le groupe des familles de plantes suivantes : anacardiacées, astéracées, boraginacées, brassicacées, cannabacées, compositées, crucifères, cucurbitacées, éléagnacées, euphorbiacées, fabacées, géraniacées, graminées, légumineuses, linacées, malvacées, moringacées, marchantiacées, onagracées, olacacées, oléacées, papavéracées, pipéracées, pédaliacées, poacées ou solanacées.

**14.** Procédé de fabrication d'une cellule de plante produisant de l'huile exprimant sous le contrôle de promoteurs spécifiques aux graines ou de promoteurs actifs dans l'embryon de la plante ou l'endosperme :

a) une delta-6 désaturase,
b) une delta-6 élongase,
c) une delta-5 désaturase,
d) une delta-5 élongase et
e) une delta-12 désaturase,

la plante fabriquant dans ses graines indépendamment les uns des autres des acides alkylcarbonyliques en C18, C20 ou C22 insaturés contenant au moins trois, quatre, cinq ou six doubles liaisons en une teneur d'au moins 30 % en poids, par rapport à la totalité des acides gras dans les graines de la plante, comprenant l'introduction des gènes mentionnés dans une plante produisant de l'huile.

**15.** Procédé de fabrication d'un acide gras en C20 et/ou en C22 ou d'un ester de celui-ci contenant au moins trois, quatre, cinq ou six doubles liaisons dans une plante produisant de l'huile exprimant sous le contrôle de promoteurs spécifiques aux graines ou de promoteurs actifs dans l'embryon de la plante ou l'endosperme :

a) une delta-6 désaturase,
b) une delta-6 élongase,
c) une delta-5 désaturase,
d) une delta-5 élongase et
e) une delta-12 désaturase,

la plante fabriquant dans ses graines indépendamment les uns des autres des acides alkylcarbonyliques en C18, C20 ou C22 insaturés contenant au moins trois, quatre, cinq ou six doubles liaisons en une teneur d'au moins 30 % en poids, par rapport à la totalité des acides gras dans les graines de la plante, comprenant l'introduction des gènes mentionnés dans une plante produisant de l'huile.

**16.** Procédé de fabrication d'une huile, d'un lipide ou d'un acide gras libre, comprenant l'obtention des huiles, des lipides ou des acides gras libres à partir de la plante ou de la graine selon l'une quelconque des revendications précédentes.

**17.** Procédé selon la revendication 15 ou 16, dans lequel de l'acide arachidonique, de l'acide eicosapentaénoïque, de l'acide docosapentaénoïque ou de l'acide docosahexaénoïque, de l'acide eicosapentaénoïque et/ou de l'acide docosahexaénoïque sont fabriqués, puis utilisés à diverses fins dans des applications alimentaires, fourragères, cosmétiques ou pharmaceutiques.

**18.** Utilisation d'une séquence d'acides nucléiques codant pour une séquence d'acides aminés selon SEQ ID NO 202 ou ayant au moins 40 % d'identité de séquence avec la séquence d'acides aminés selon SEQ ID NO 202 et présentant une activité delta-6 désaturase, ou de la séquence d'acides aminés codée, pour la fabrication d'huiles, de lipides ou d'acides gras libres, de produits pharmaceutiques, de produits alimentaires, de fourrages pour animaux ou de produits cosmétiques.

Figur 1: Verschiedene Synthese-Wege zur Biosynthese von DHA (Docosahexaensäure)

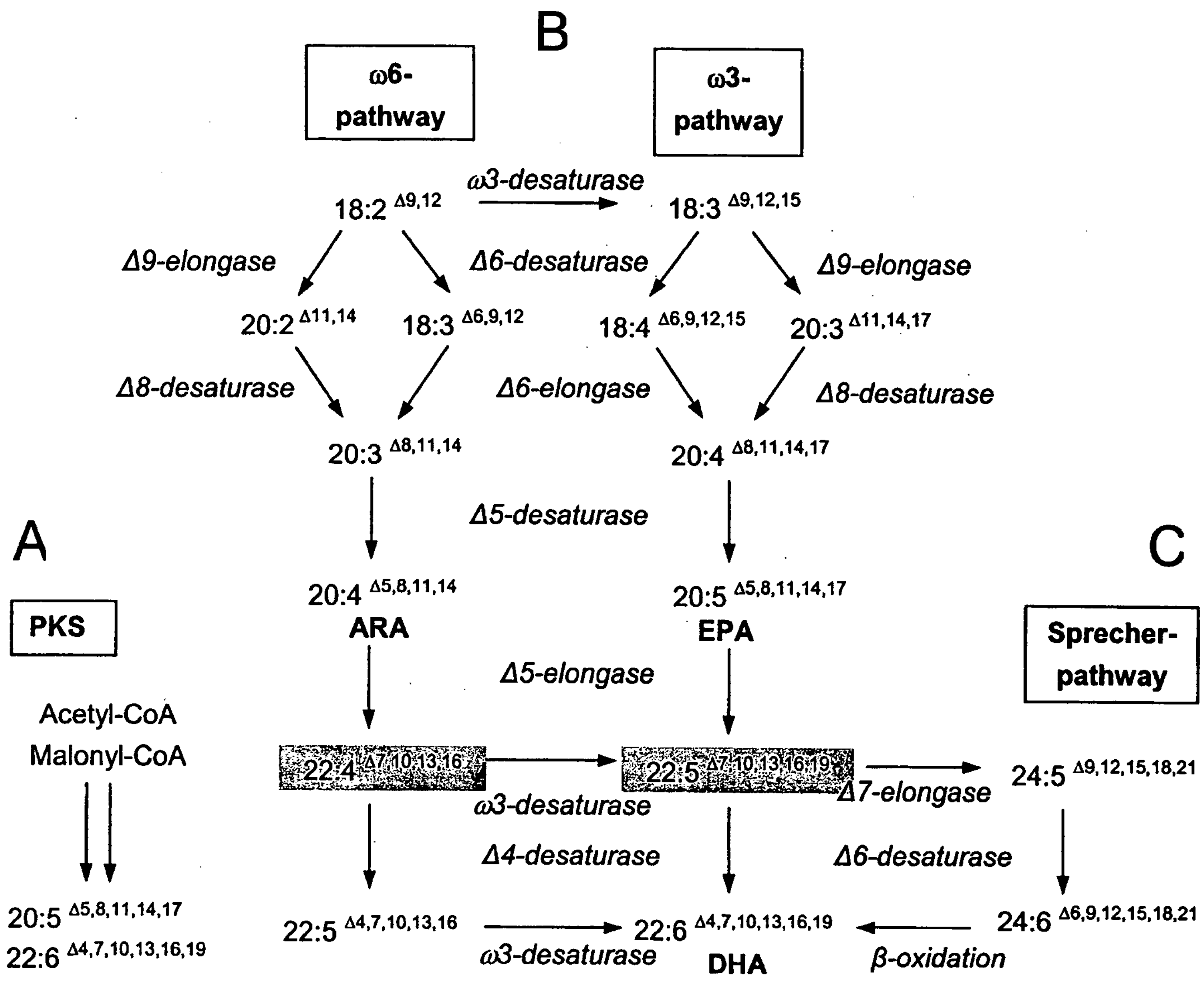

Figur 2: Substratspezifität der Δ-5-Elongase (SEQ ID NO: 53) gegenüber verschiedenen Fettsäuren

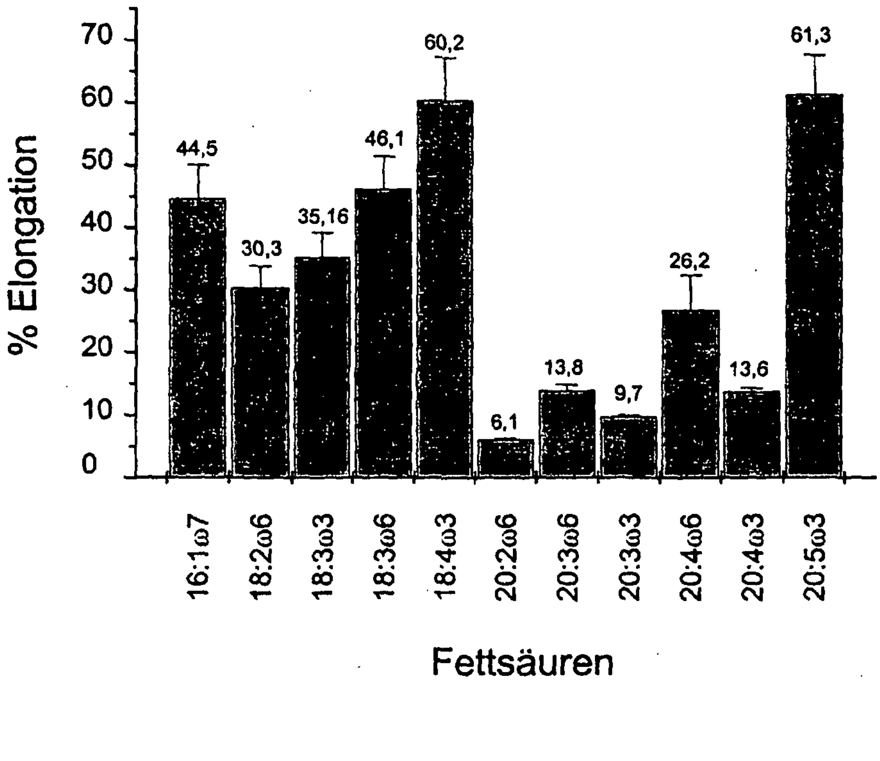

Figur 3: Rekonstitution der DHA-Biosynthese in Hefe ausgehend von 20:5ω3.

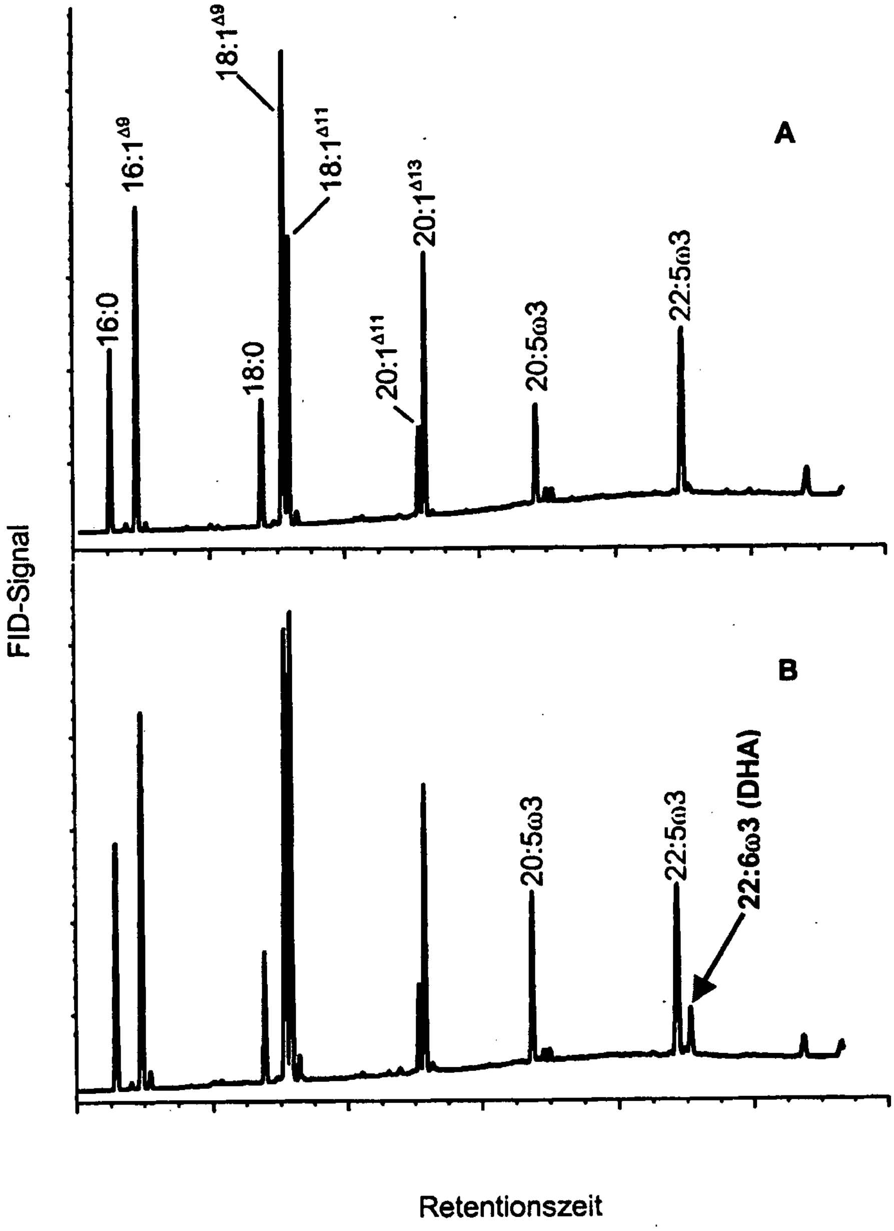

Figur 4: Rekonstitution der DHA-Biosynthese in Hefe ausgehend von 18:4ω3.

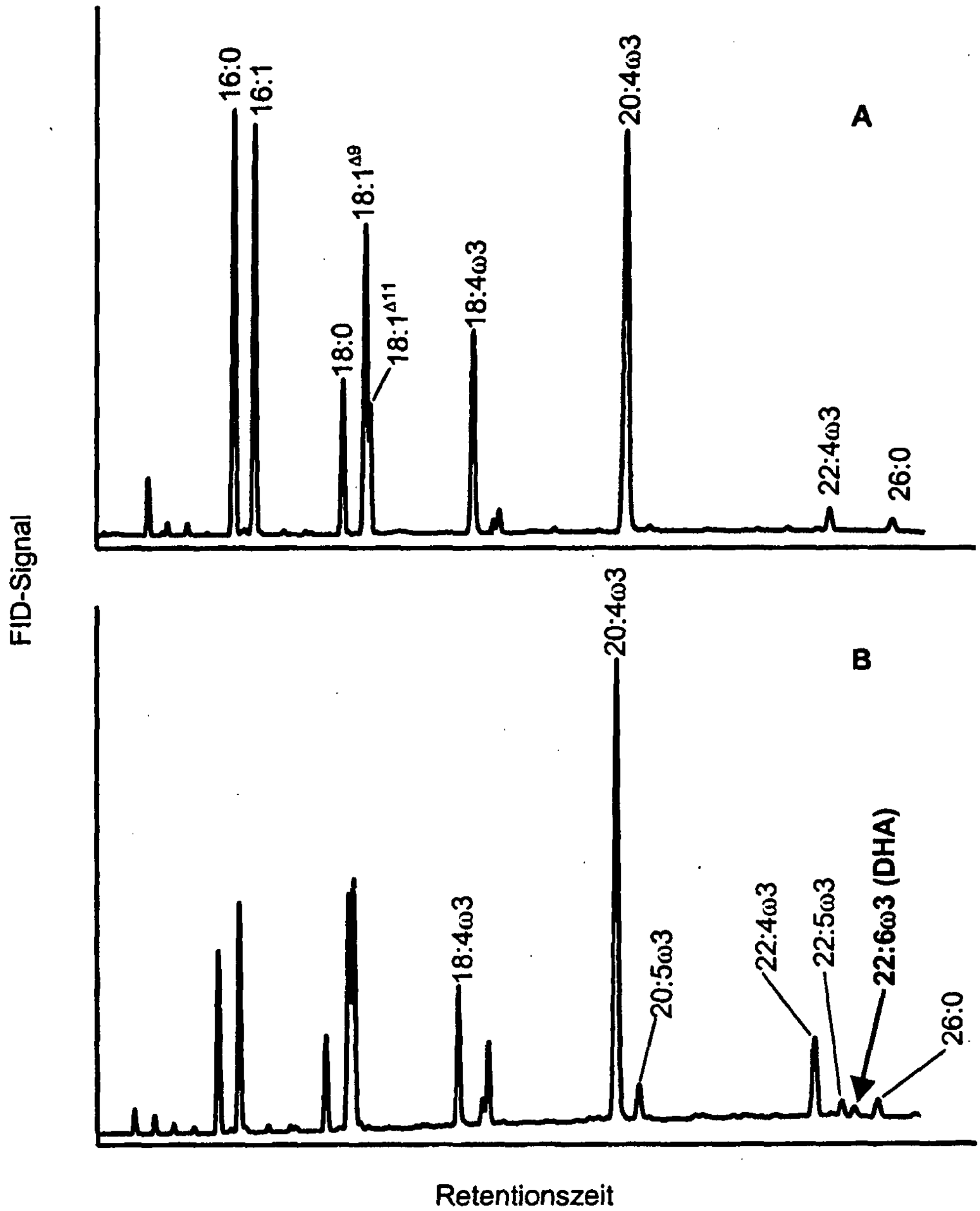

Figur 5: Fettsäure-Zusammensetzung (in Mol %) transgener Hefen, die mit den Vektoren pYes3-OmELO3/pYes2-EgD4 oder pYes3-OmELO3/pYes2-EgD4+pESCLeu-PtD5 transformiert worden waren. Die Hefezellen wurden in Minimalmedium ohne Tryptophan und Uracil / und Leucin in Gegenwart von 250 $\mu$M $20{:}5^{\Delta 5,8,11,14,17}$ bzw. $18{:}4^{\Delta 6,9,12,15}$ kultiviert. Die Fettsäuremethylester wurden durch saure Methanolyse aus Zellsedimenten gewonnen und über GLC analysiert. Jeder Wert gibt den Mittelwert (n=4) ± Standardabweichung wieder.

| | pYes3-OmELO/pYes2-EgD4 | pYes3-OmELO/pYes2-EgD4 EgD4 + pESCLeu-PtD5 |
|---|---|---|
| Fettsäuren | Fütterung mit $20{:}5^{\Delta 5,8,11,14,17}$ | Fütterung mit $18{:}4^{\Delta 6,9,12,15}$ |
| 16:0 | 9,35 ± 1,61 | 7,35 ± 1,37 |
| $16{:}1^{\Delta 9}$ | 14,70 ± 2,72 | 10,02 ± 1,81 |
| 18:0 | 5,11 ± 1,09 | 4,27 ± 1,21 |
| $18{:}1^{\Delta 9}$ | 19,49 ± 3,01 | 10,81 ± 1,95 |
| **$18{:}1^{\Delta 11}$** | **18,93 ± 2,71** | **11,61 ± 1,48** |
| **$18{:}4^{\Delta 6,9,12,15}$** | **-** | **7,79 ± 1,29** |
| **$20{:}1^{\Delta 11}$** | **3,24 ± 0,41** | **1,56 ± 0,23** |
| **$20{:}1^{\Delta 13}$** | **11,13± 2,07** | **4,40 ± 0,78** |
| **$20{:}4^{\Delta 8,11,14,17}$** | **-** | **30,05 ± 3,16** |
| **$20{:}5^{\Delta 5,8,11,14,17}$** | **6,91± 1,10** | **3,72 ± 0,59** |
| **$22{:}4^{\Delta 10,13,16,17}$** | **-** | **5,71 ± 1,30** |
| **$22{:}5^{\Delta 7,10,13,16,19}$** | **8,77 ± 1,32** | **1,10 ± 0,27** |
| **$22{:}6^{\Delta 4,7,10,13,16,19}$** | **2,73 ± 0,39** | **0,58 ± 0,10** |

Figur 6: Fütterungsexperiment zur Bestimmung der Funktionalität und Substratspezifität mit Hefestämmen

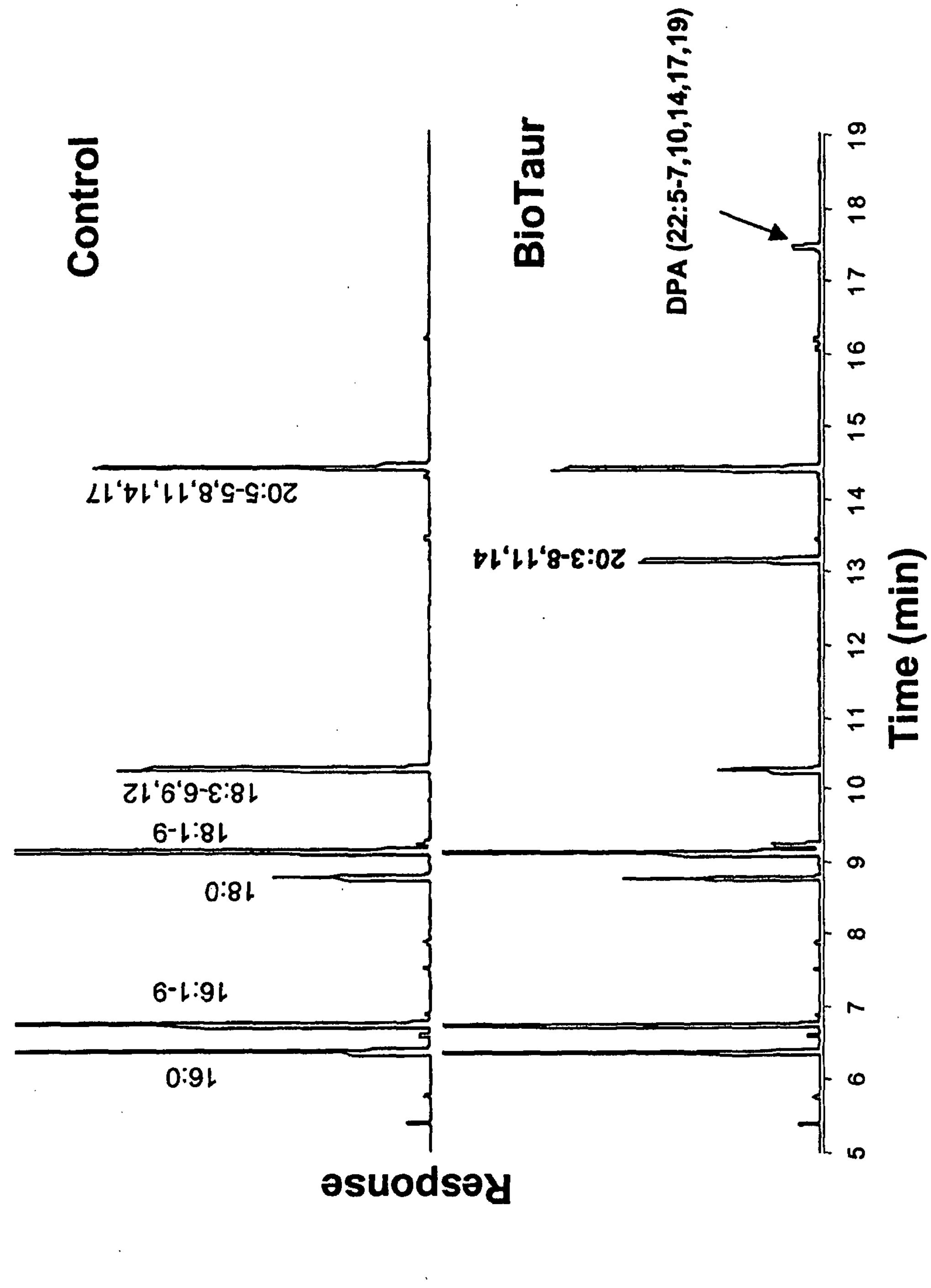

Figur 7: Elongation von Eicosapentaensäure durch OtElo1

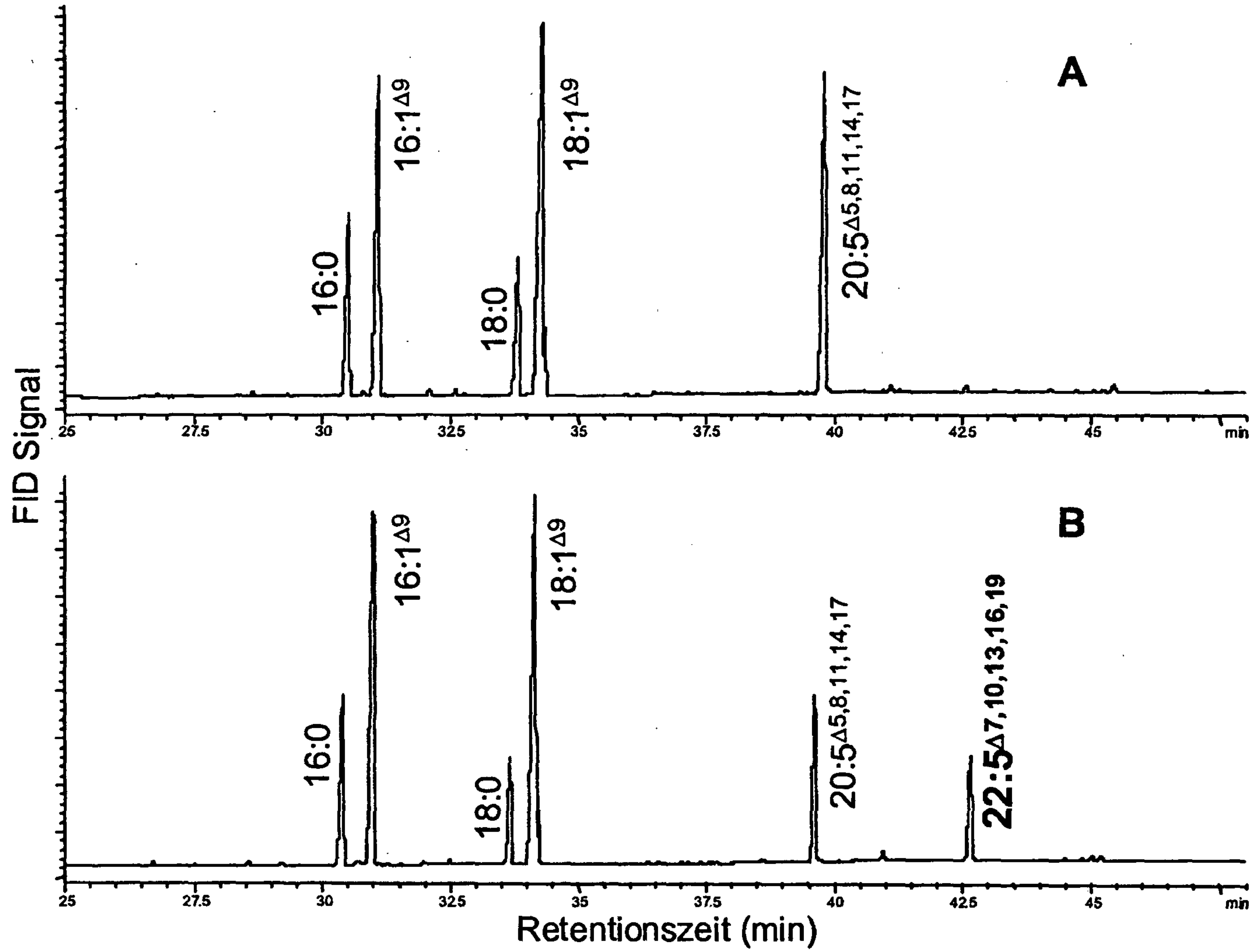

Figur 8: Elongation von Arachidonsäure durch OtElo1

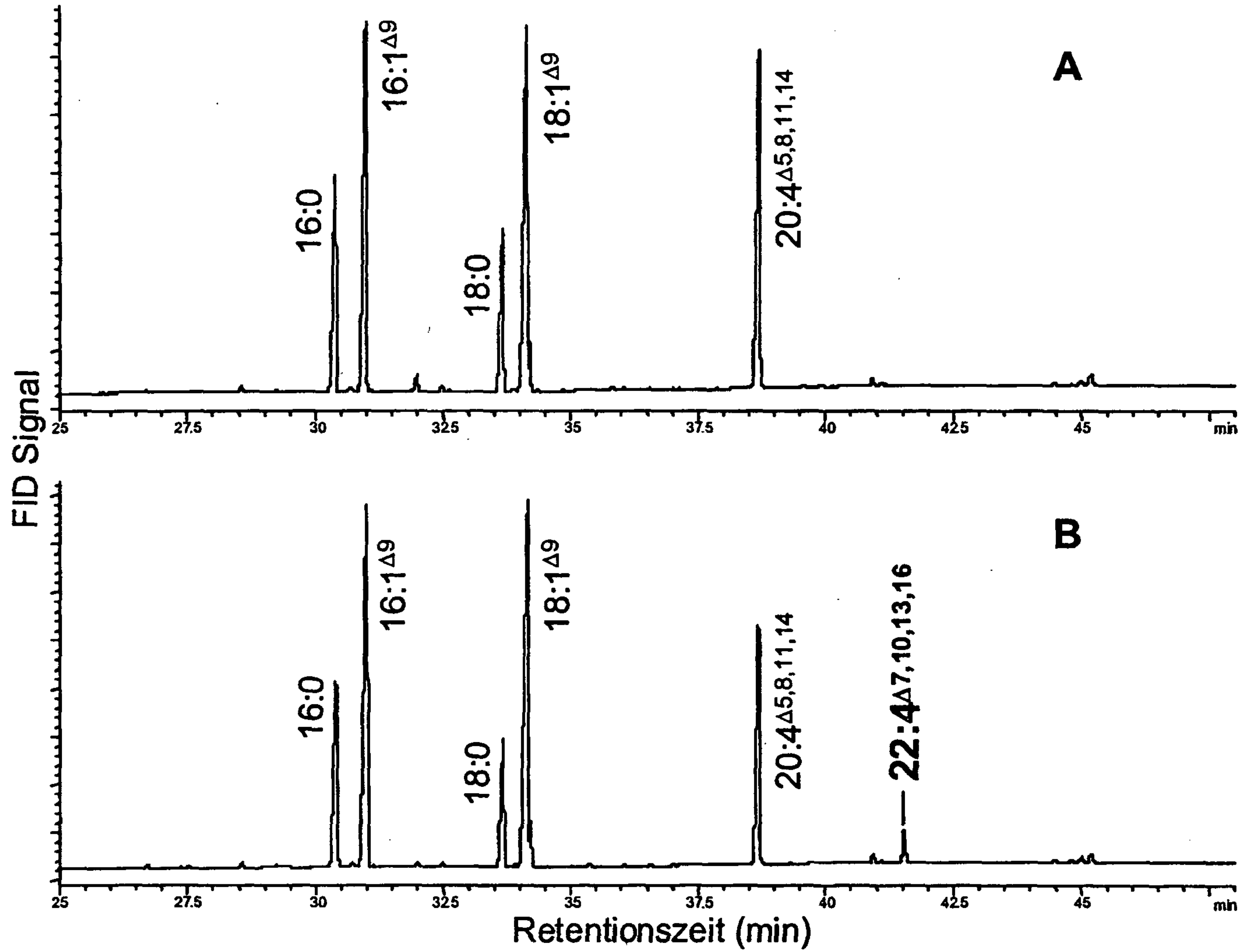

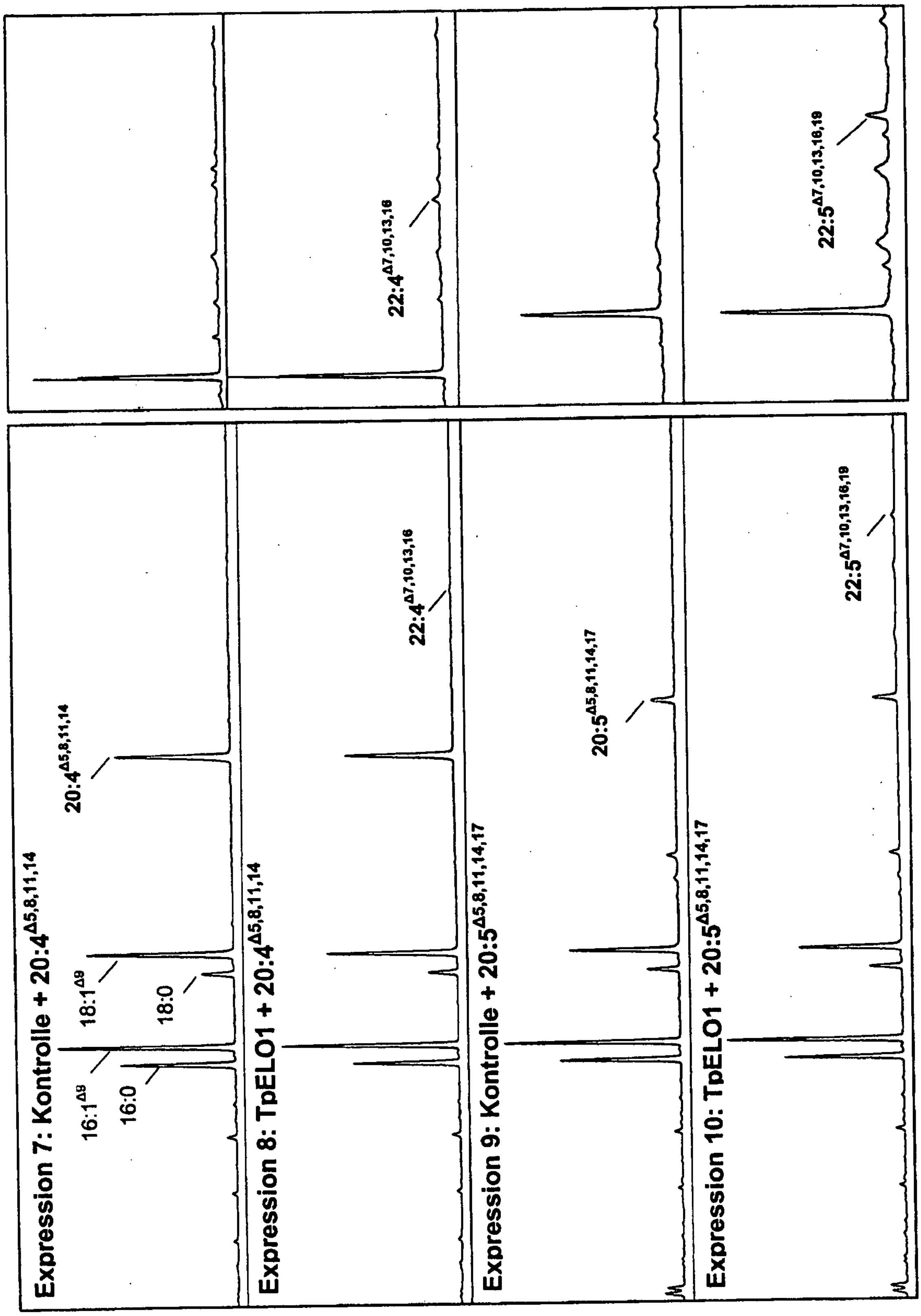


Figur 9: Expression von TpELO1 in Hefe

Figur 10: Expression von TpELO3 in Hefe.

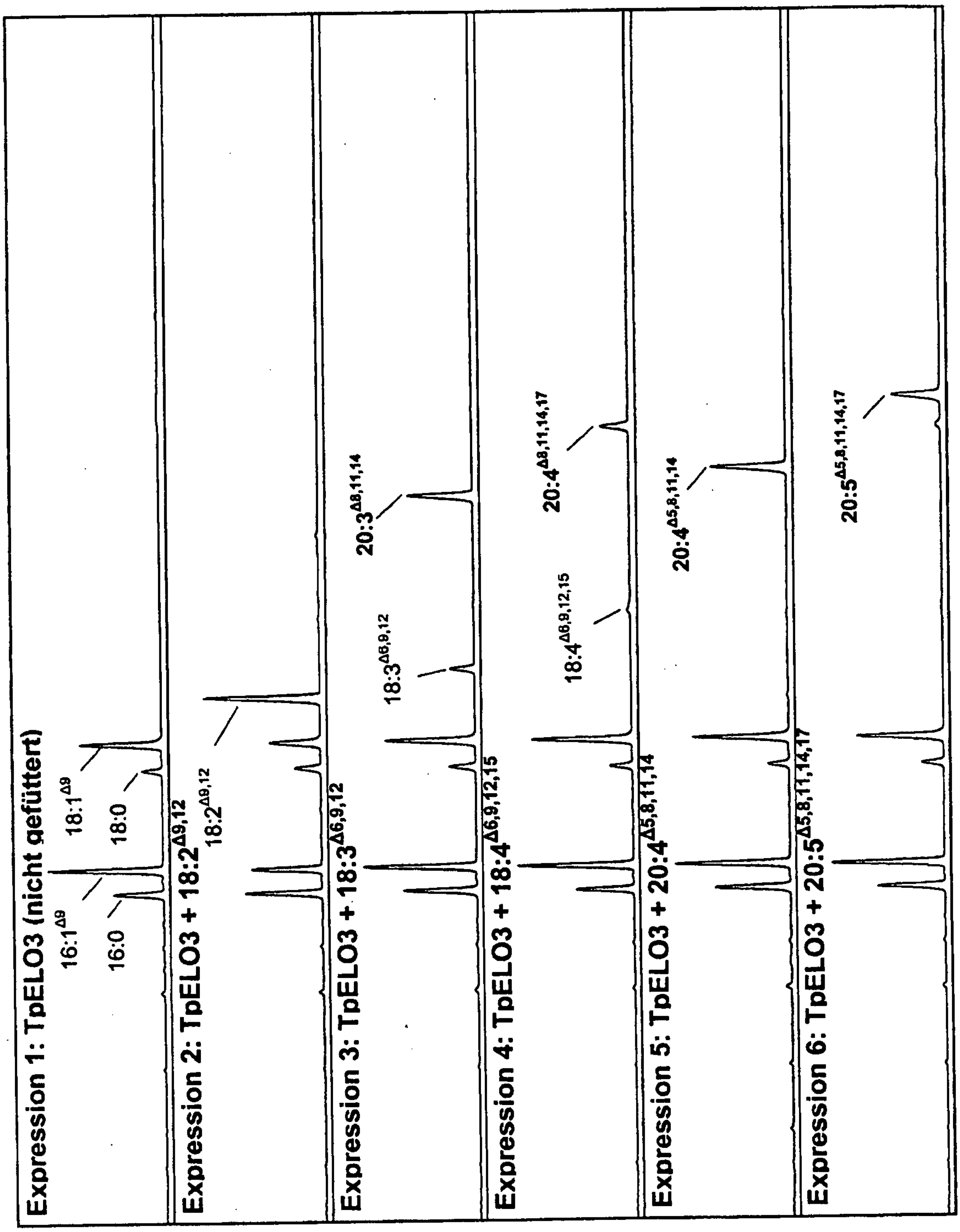

Figur 11: Expression von Thraustochytrium Δ5-Elongase TL16/pYES2.1 in Hefe.

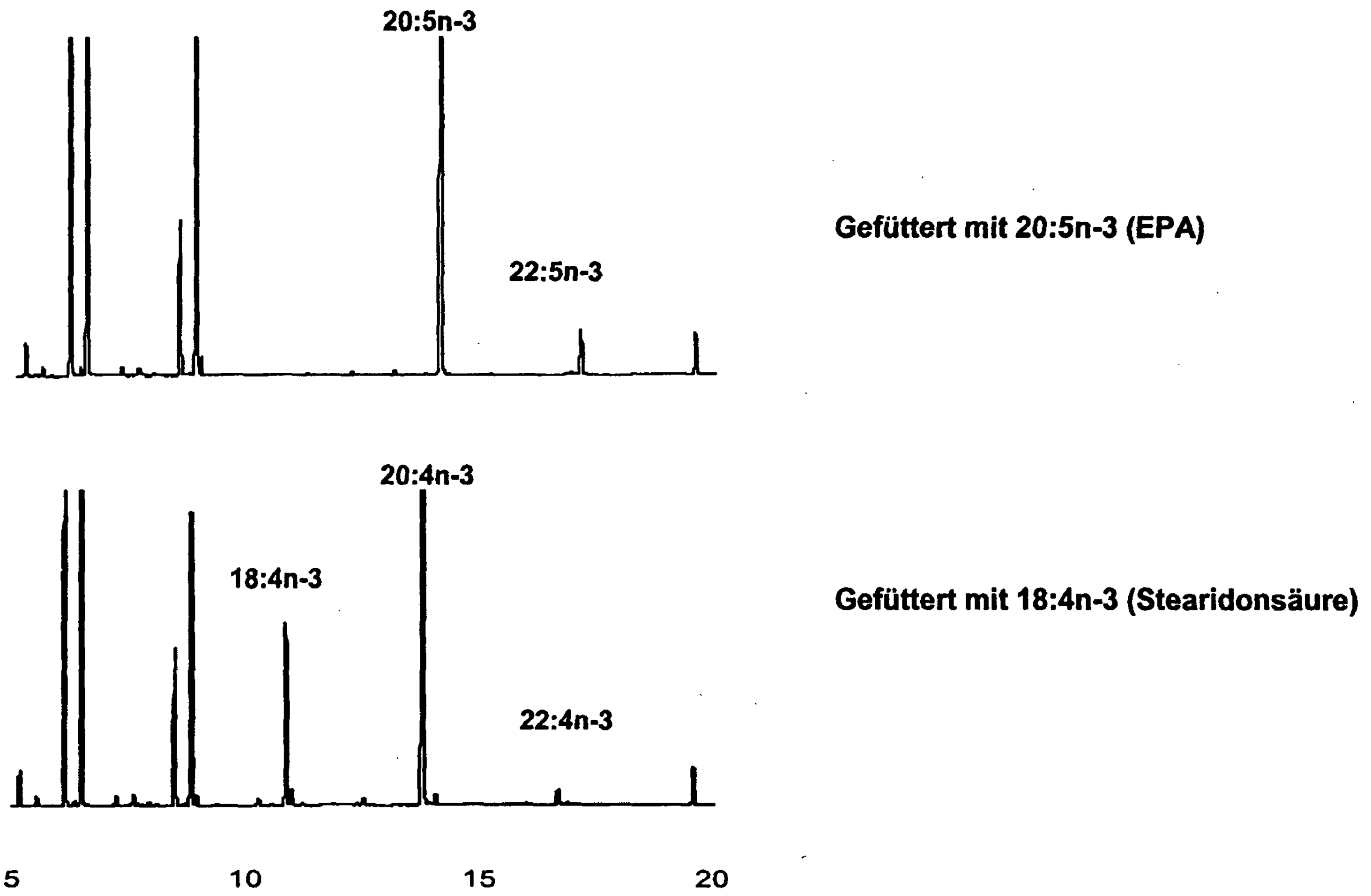

Figur 12: Desaturierung von Linolsäure (18:2 ω-6-Fettsäure) zu α-Linolensäure (18:3 ω-3-Fettsäure) durch Pi-omega3Des.

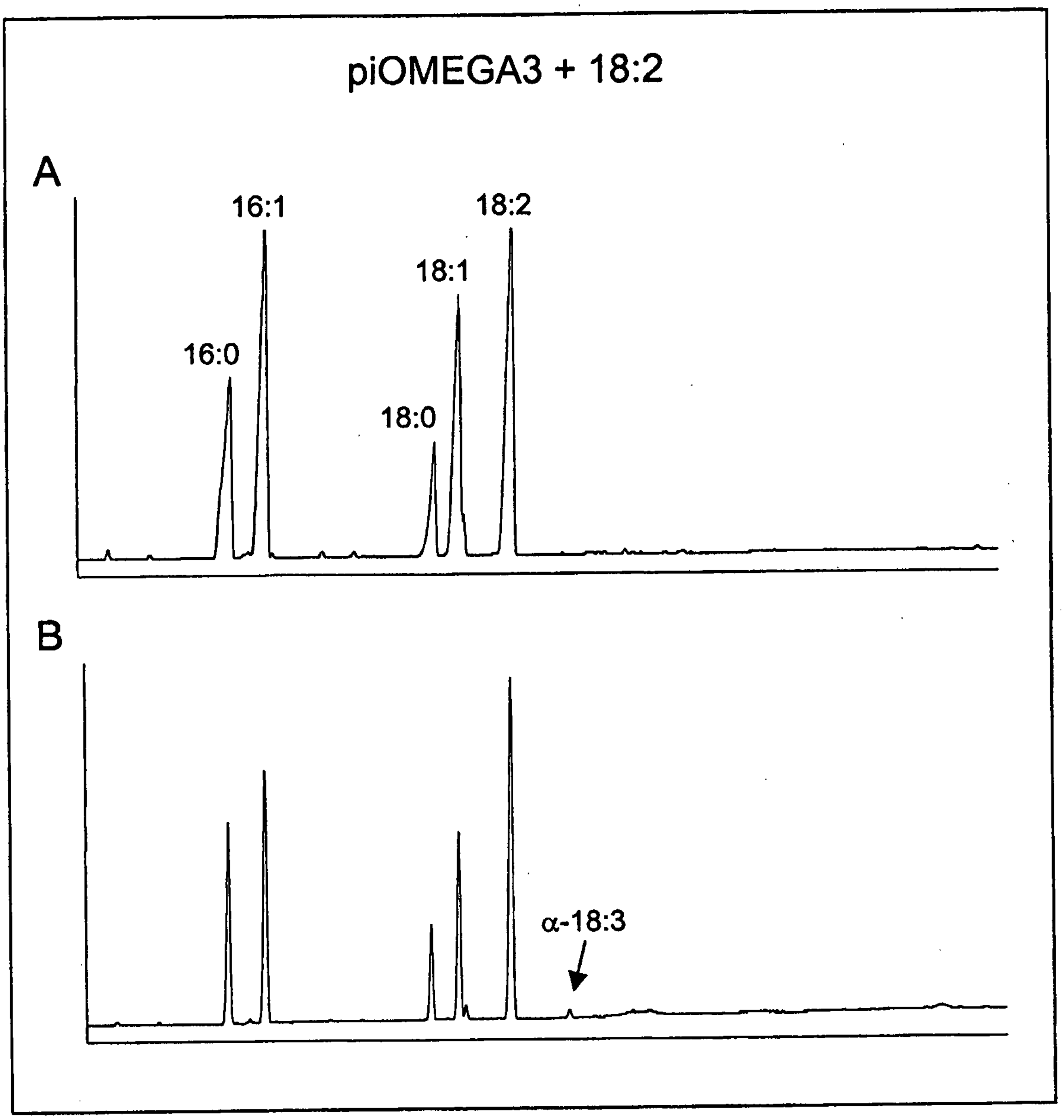

Figur 13: Desaturierung von γ-Linolensäure (18:3 ω-6-Fettsäure) zu Stearidonsäure (18:4 ω-3-Fettsäure) durch Pi-omega3Des.

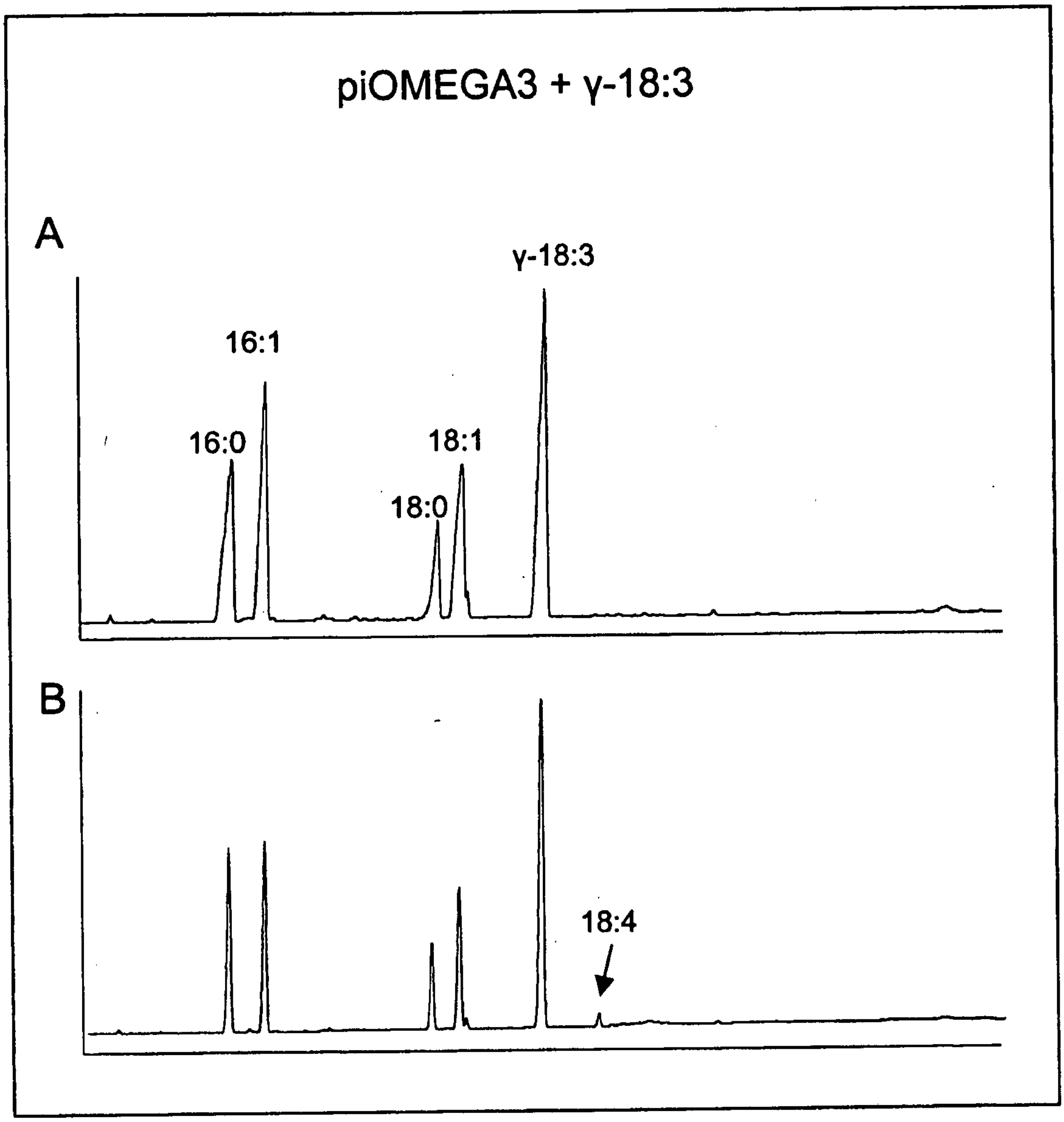

Figur 14: Desaturierung von C20:2 ω-6-Fettsäure zu C20:3 ω-3-Fettsäure durch Pi-omega3Des.

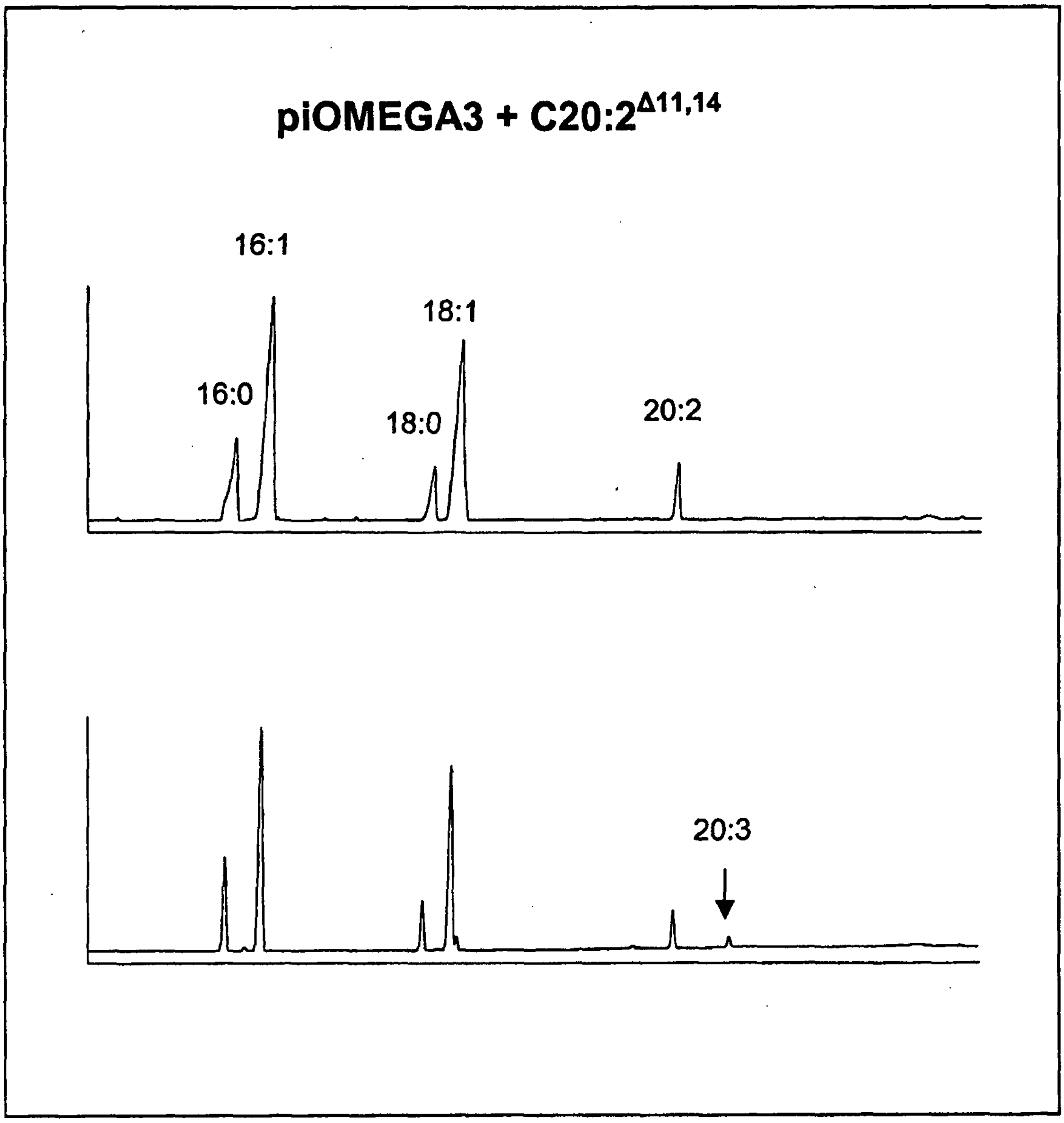

Figur 15: Desaturierung von C20:3-ω-6-Fettsäure zu C20:4-ω-3-Fettsäure durch Pi-omega3Des.

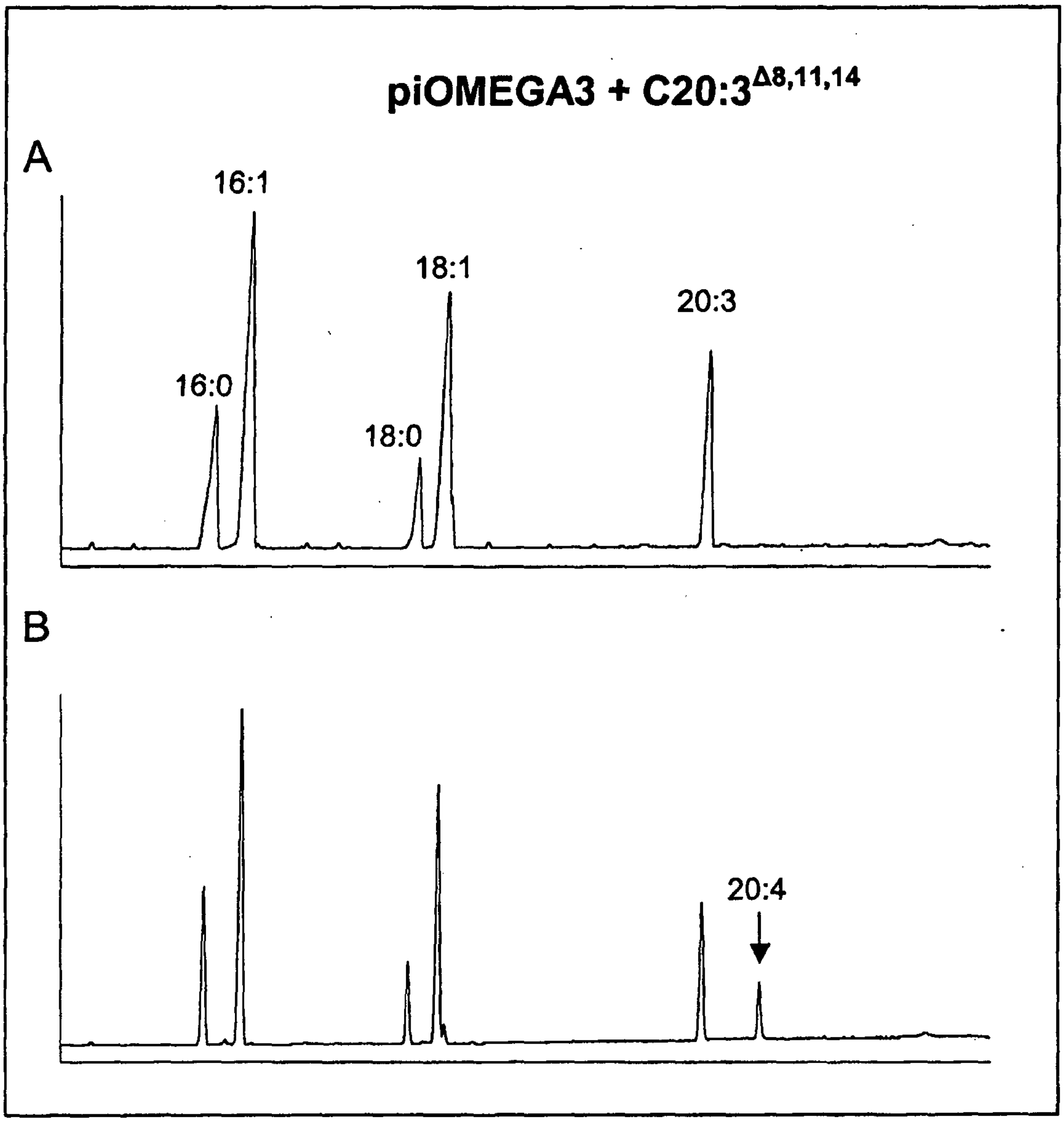

Figur 16: Desaturierung von Arachidonsäure (C20:4-ω-6-Fettsäure) zu Eicosapentaensäure (C20:5-ω-3-Fettsäure) durch die Pi-omega3Des.

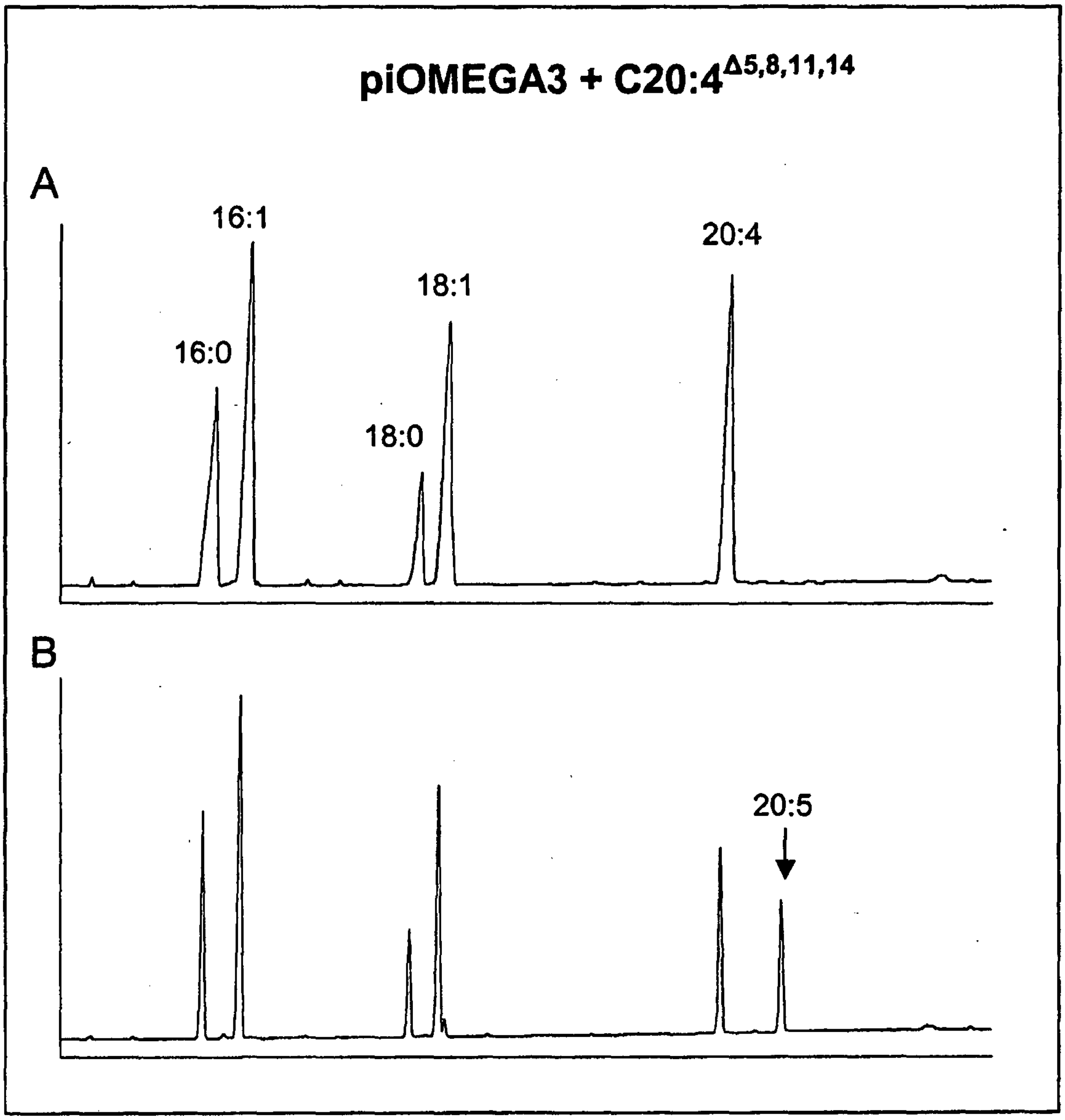

Figur 17: Desaturierung von Docosatetraensäure (C22:4-ω-6-Fettsäure) zu Docosapentaensäure (C22:5-ω-3-Fettsäure) durch Pi-omega3Des.

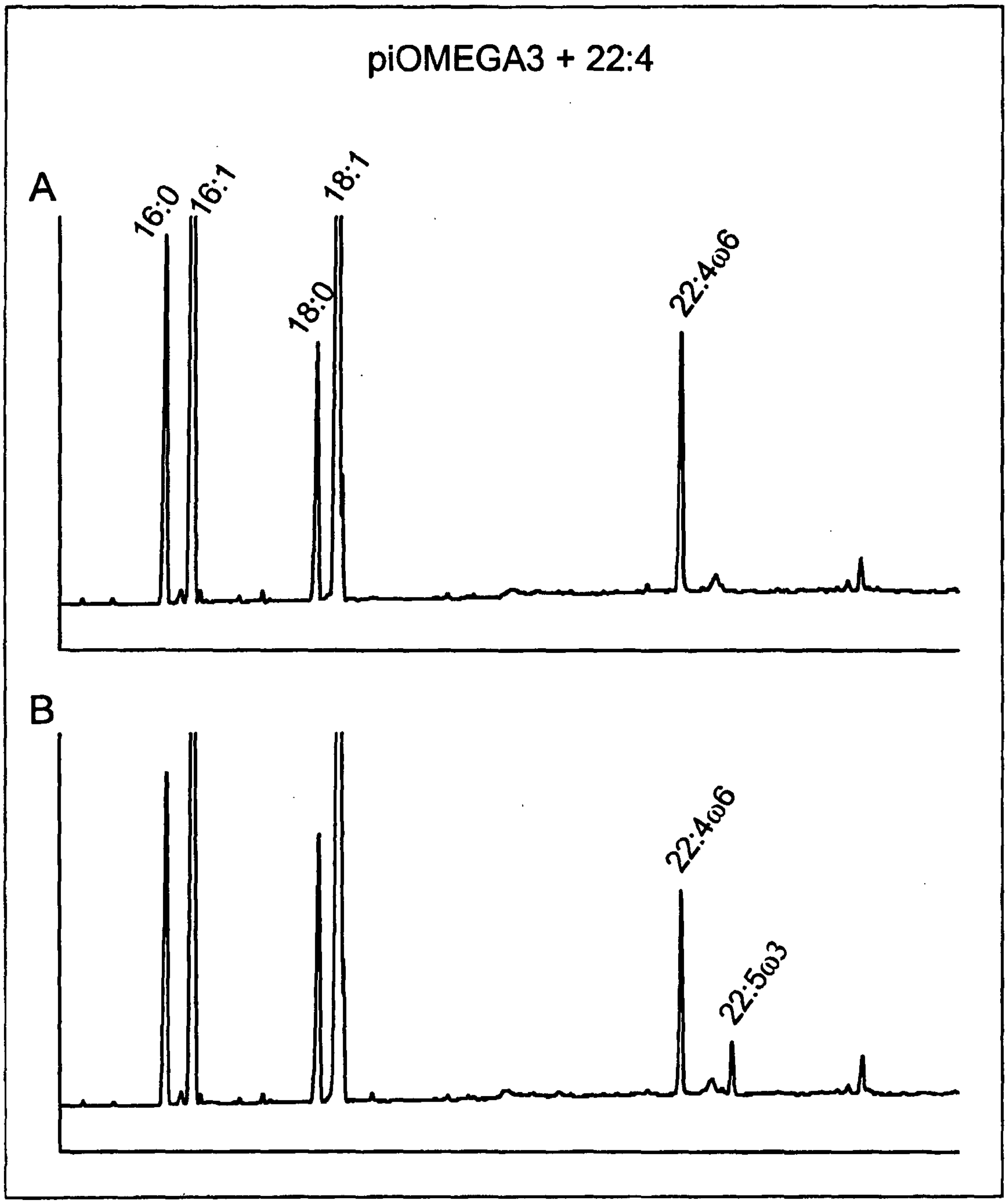

Figur 18: Substratspezifität der Pi-omega3Des gegenüber verschiedenen Fettsäuren

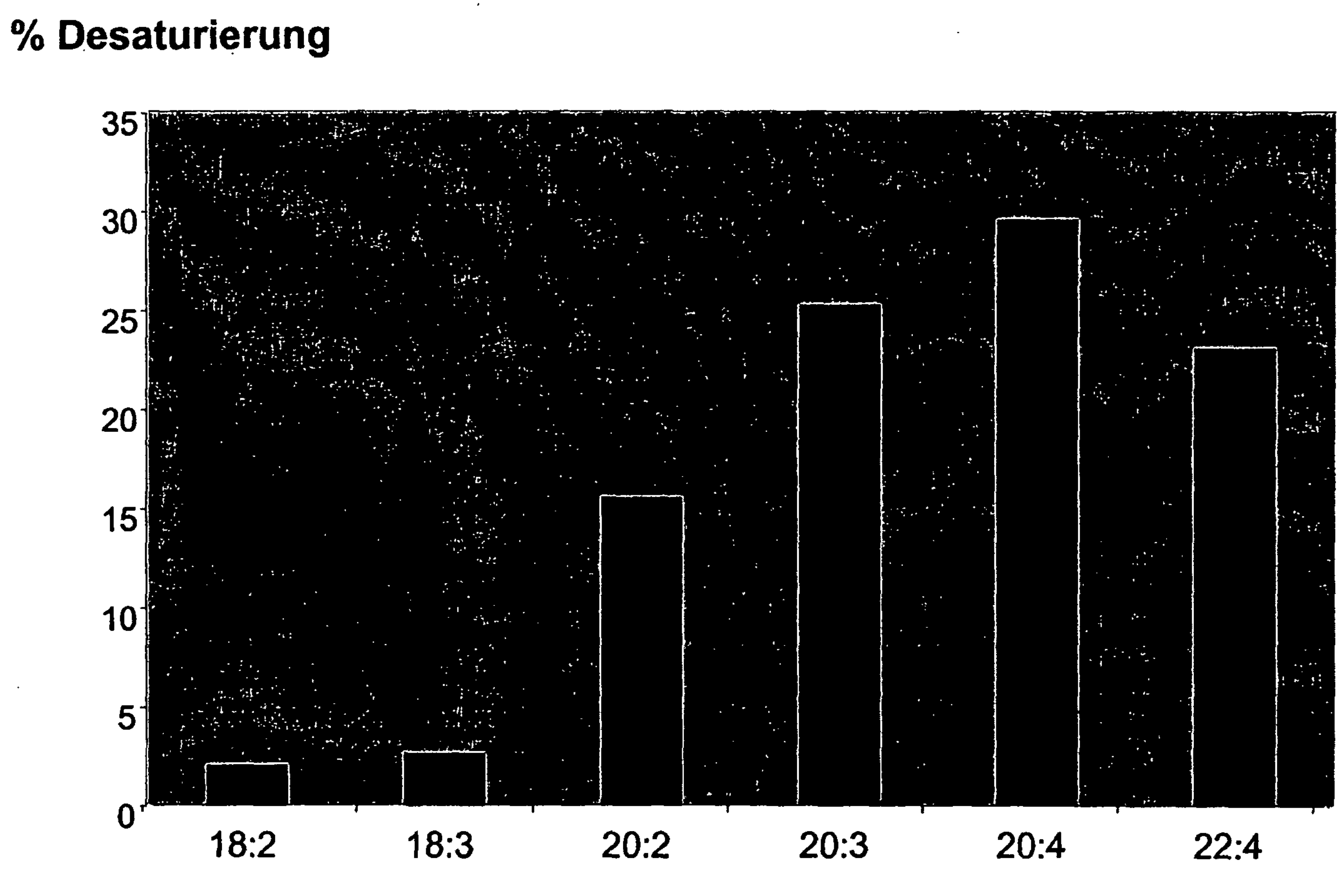

Figur 19: Desaturierung von Phospholipid gebundener Arachidonsäure zu EPA durch die Pi-Omega3Des

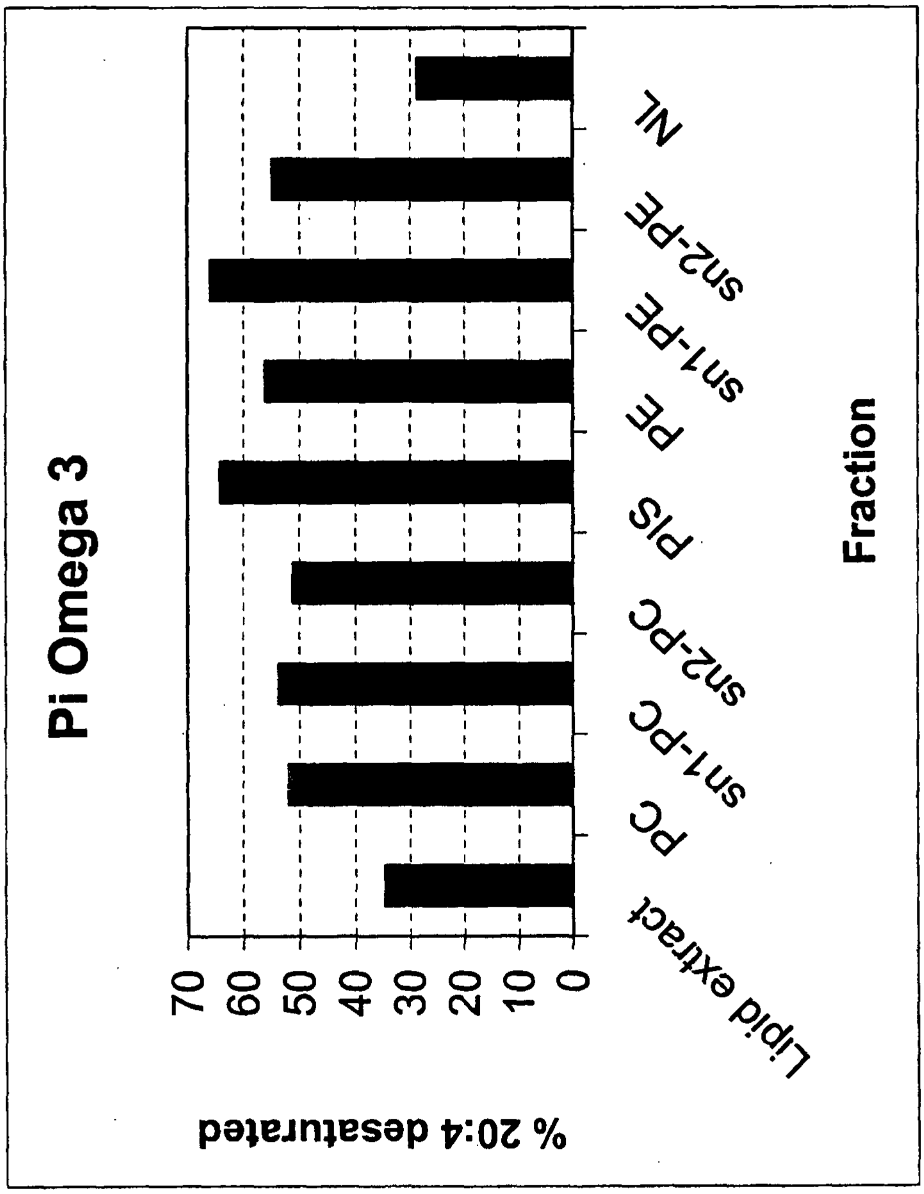

Figur 20: Umsetzung von Linolsäure (Pfeil) zu γ-Linolensäure (γ-18:3) durch Ot-Des6.1.

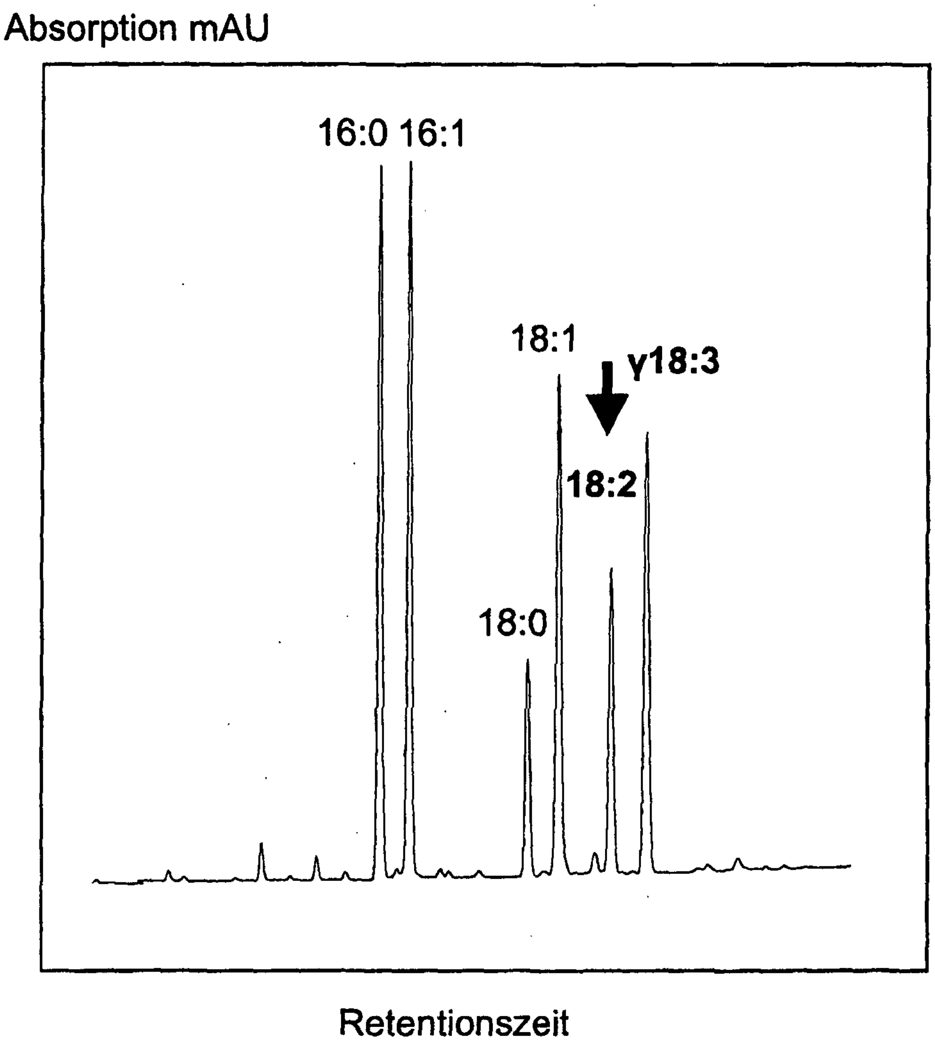

Figur 21: Umsetzung von Linolsäure und α-Linolensäure (A und C), sowie Rekonstitution des ARA- bzw. EPA-Syntheseweges in Hefe (B und D) in Gegenwart von OtD6.1.

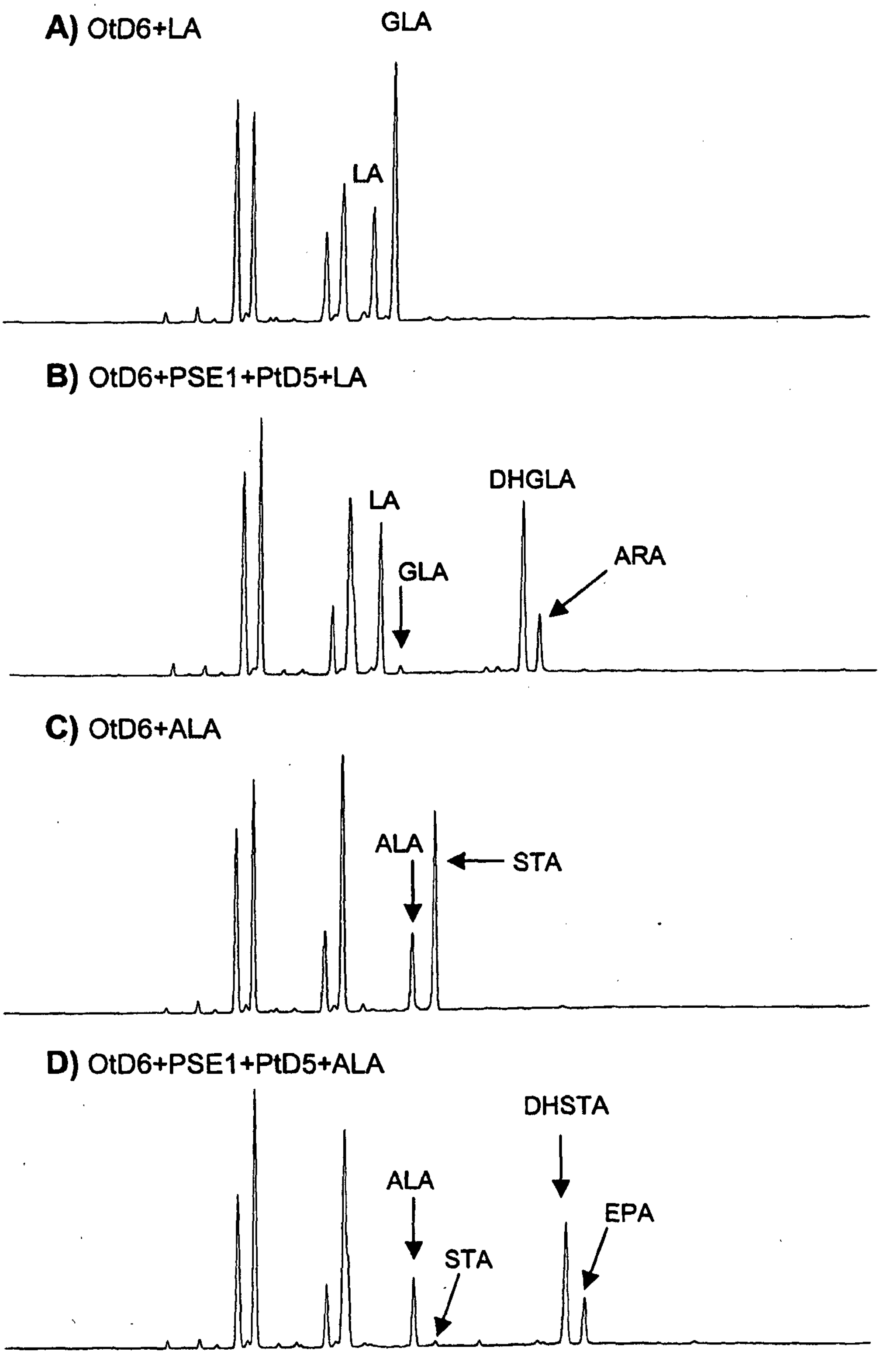

Figur 22: Expression von ELO(XI) in Hefe.

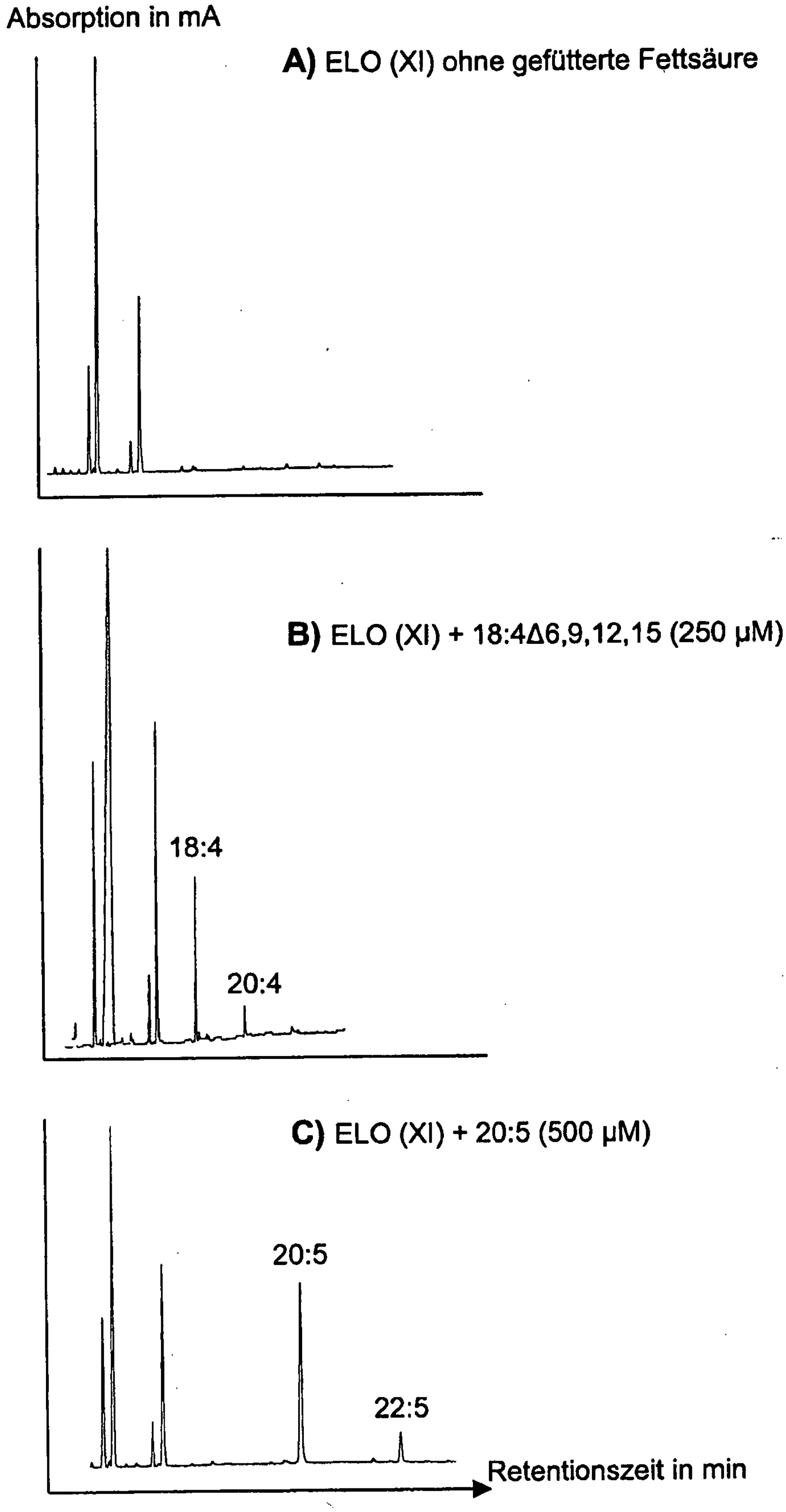

Figur 23:

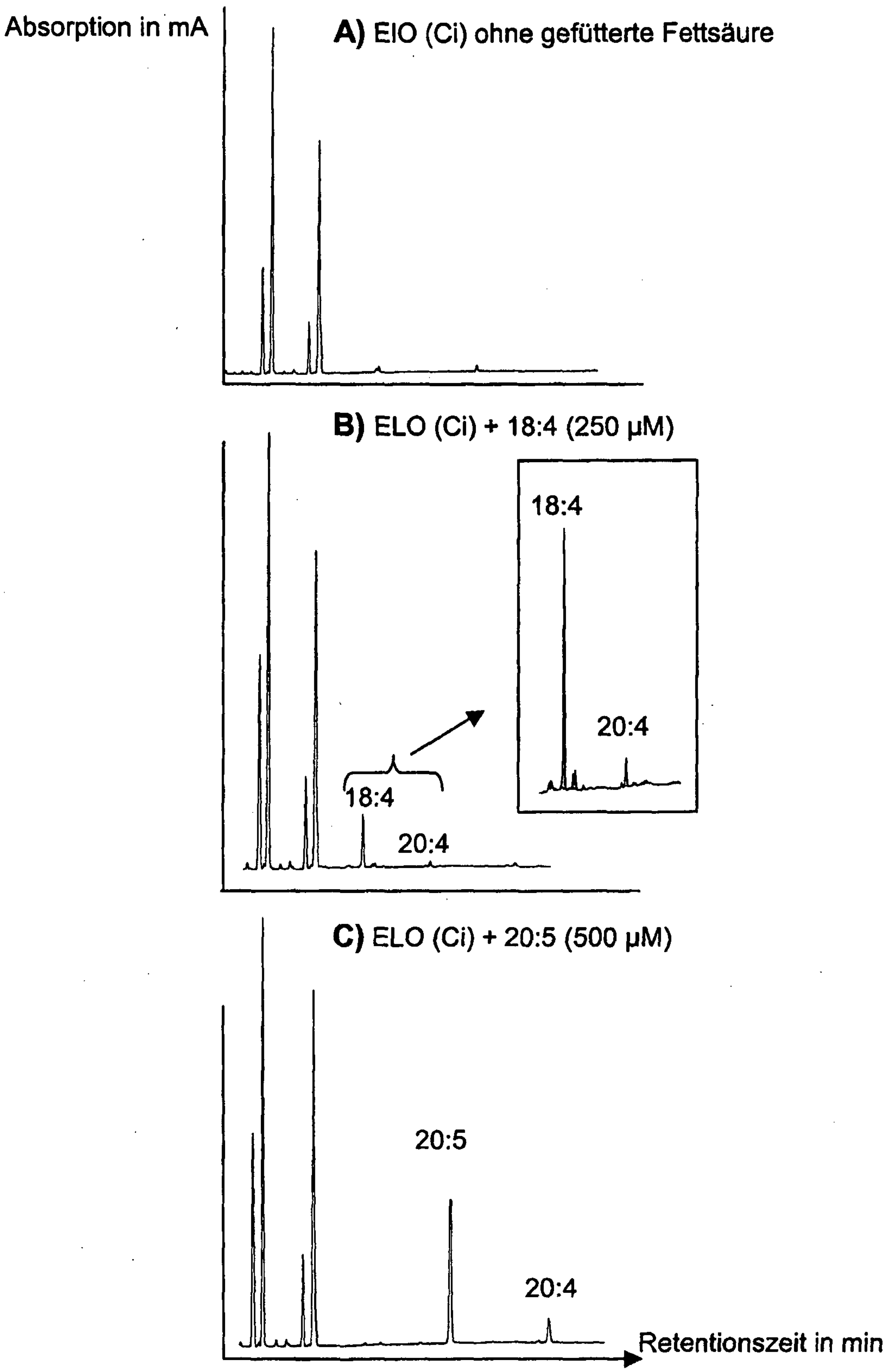
Absorption in mA
A) ElO (Ci) ohne gefütterte Fettsäure
B) ELO (Ci) + 18:4 (250 µM)
18:4
20:4
18:4
20:4
C) ELO (Ci) + 20:5 (500 µM)
20:5
20:4
Retentionszeit in min

Figur 24: Elongation von Eicosapentaensäure durch OtElo1 (B) bzw. OtElo1.2 (D). Die Kontrollen (A, C) zeigen nicht das Produkt der Elongation (22:5ω3).

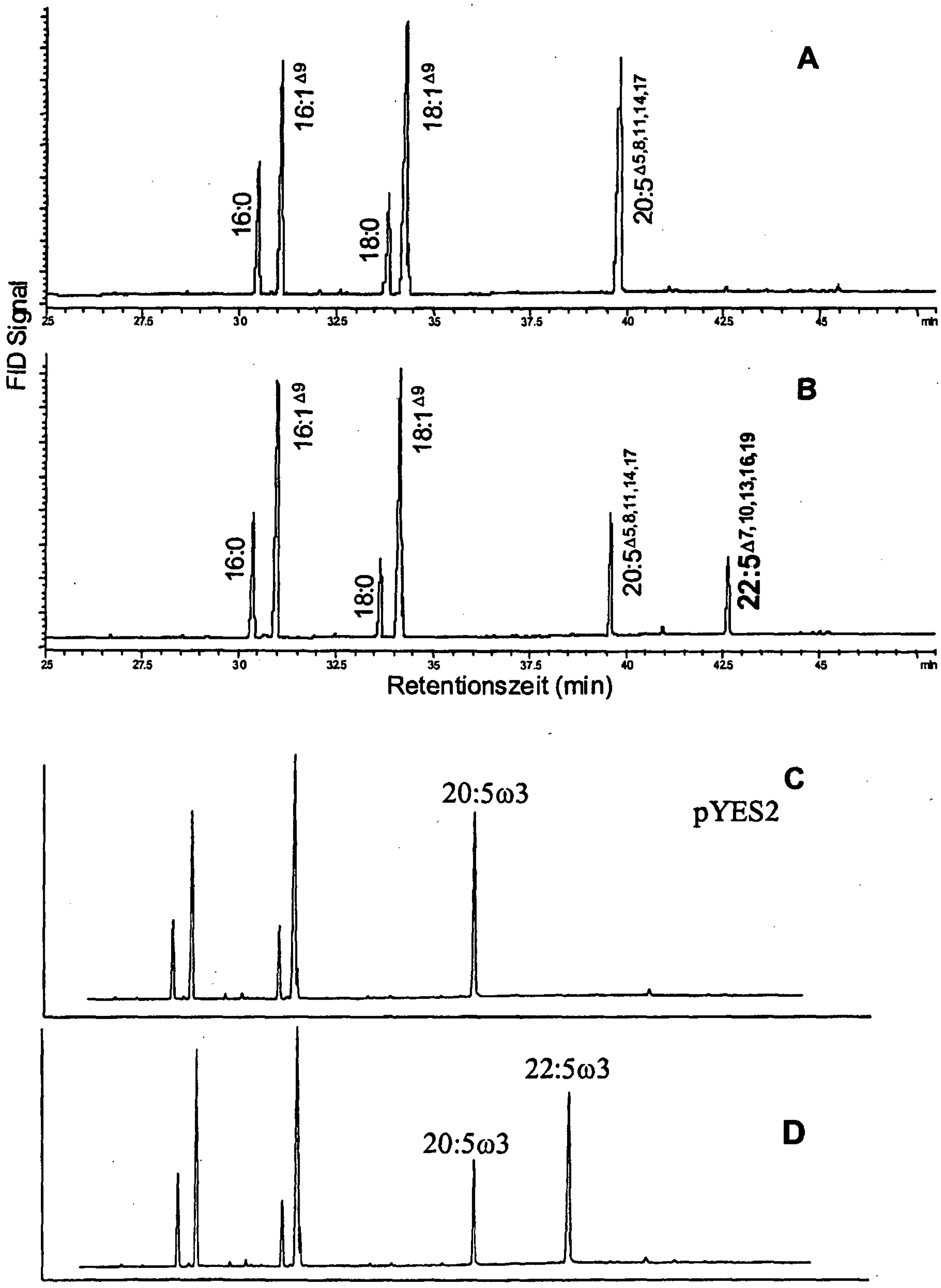

Figur 25: Elongation von Arachidonsäure durch OtElo1 (B) bzw. OtElo1.2 (D). Die Kontrollen (A, C) zeigen nicht das Produkt der Elongation (22:4ω6).

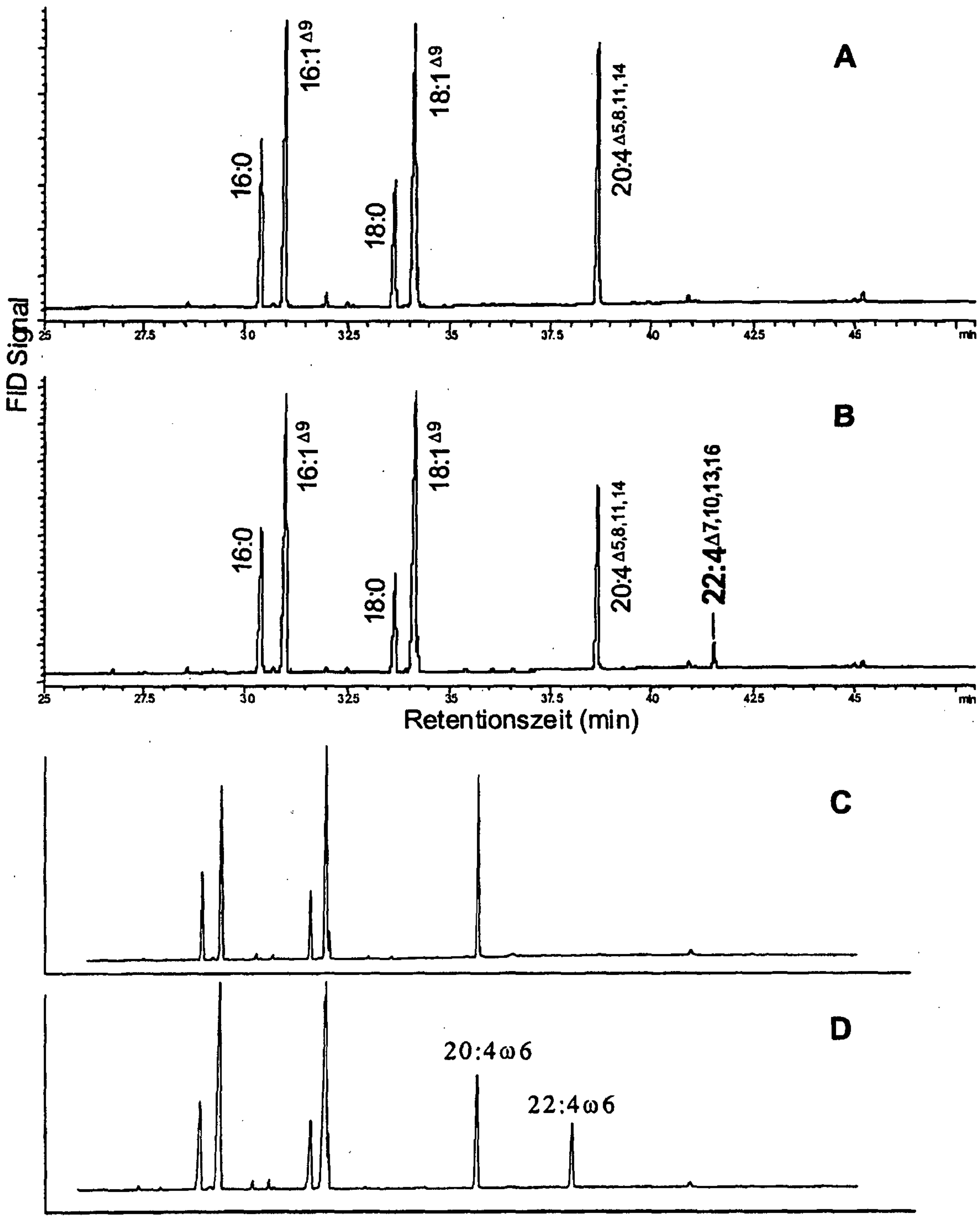

Figur 26: Elongation von 20:5n-3 durch die Elongasen At3g06470.

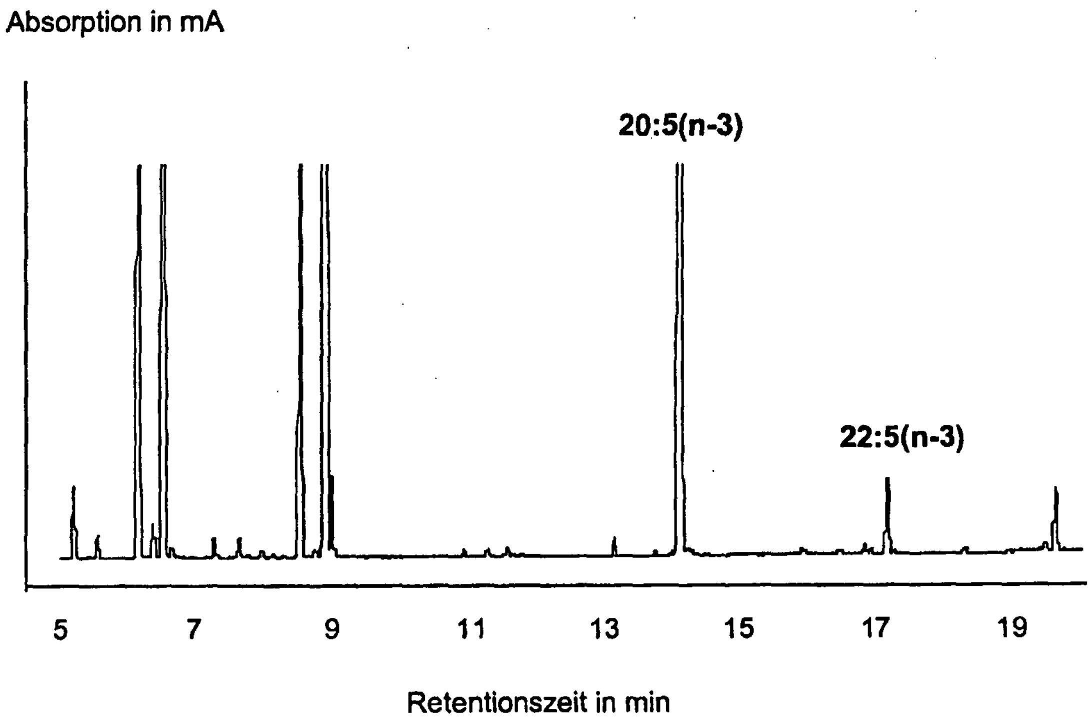

Figur 27: Substratspezifität der Xenopus Elongase (A), Ciona Elongase (B) und Oncorhynchus Elongase (C)

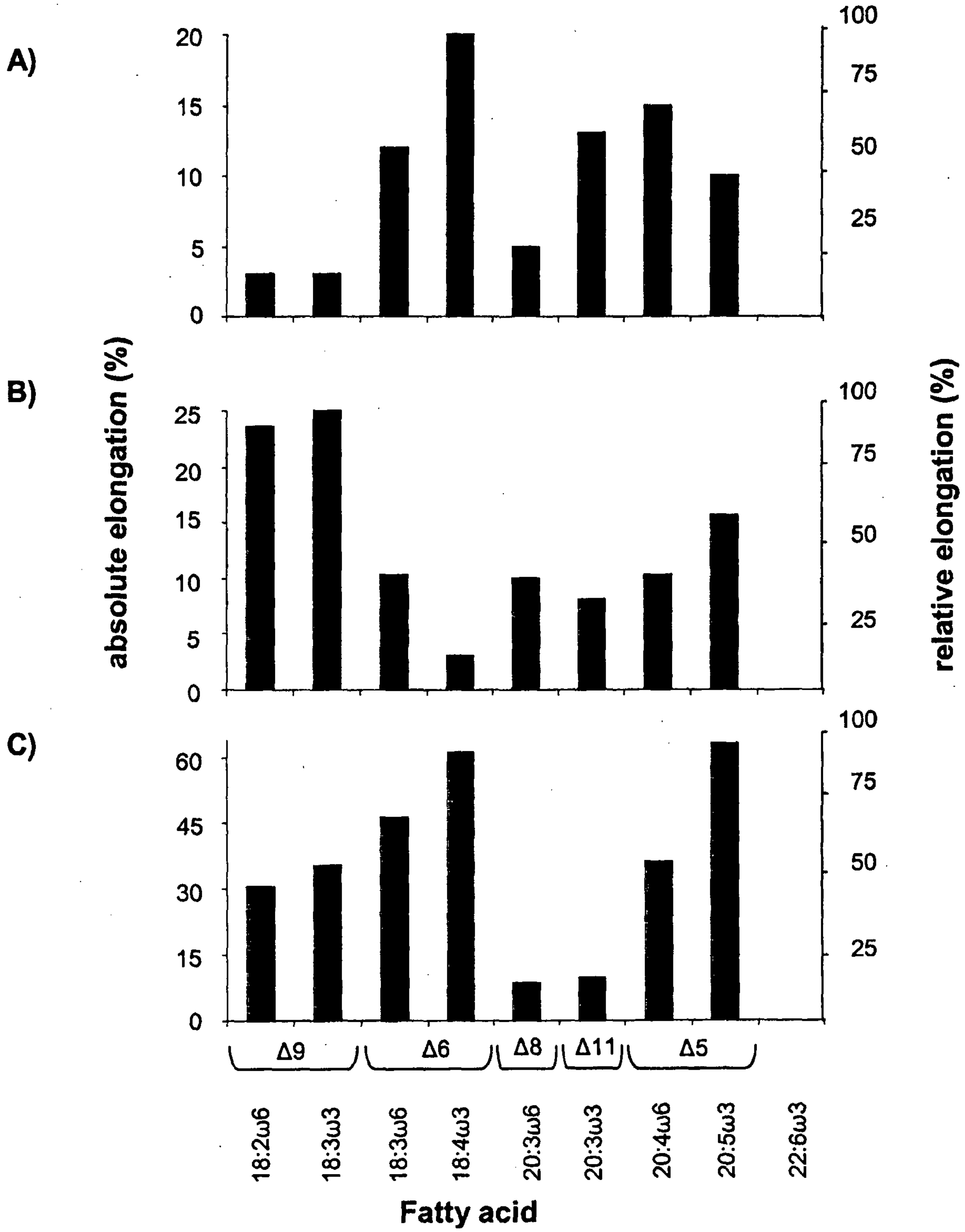

Figur 28: Substratspezifität der Ostreococcus Δ-5-Elongase (A), der Ostreococcus Δ-6-Elongase (B), der Thalassiosira Δ-5-Elongase (C) und Thalassiosira Ostreococcus Δ-6-Elongase (D)

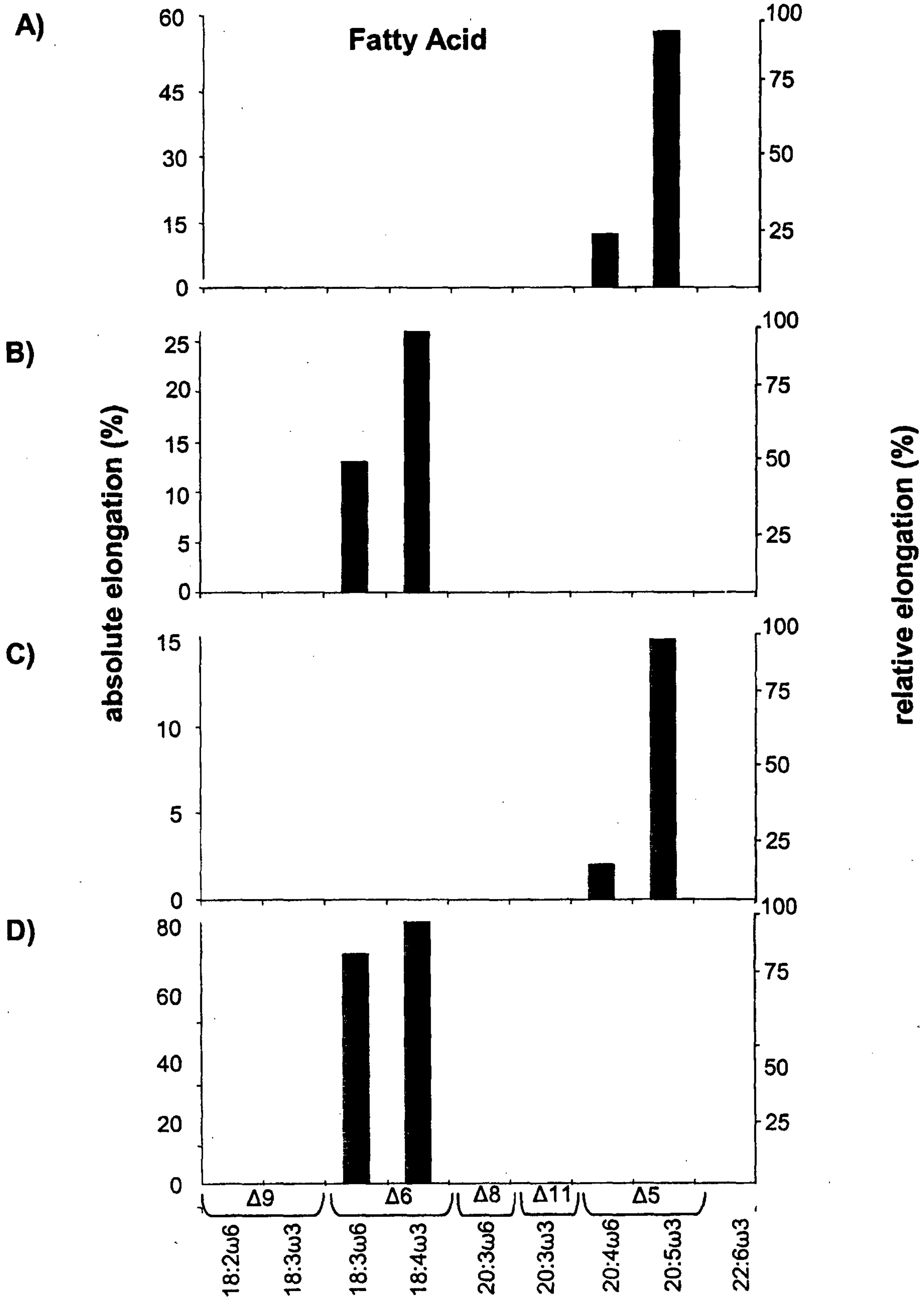

Figur 29: Expression der Phaeodactylum tricornutum Δ-6-Elongase (PtELO6) in Hefe. A) zeigt die Elongation der C18:3$^{\Delta6,9,12}$ Fettsäure und B) die Elongation der C18:4$^{\Delta6,9,12,15}$ Fettsäure

A)

B)

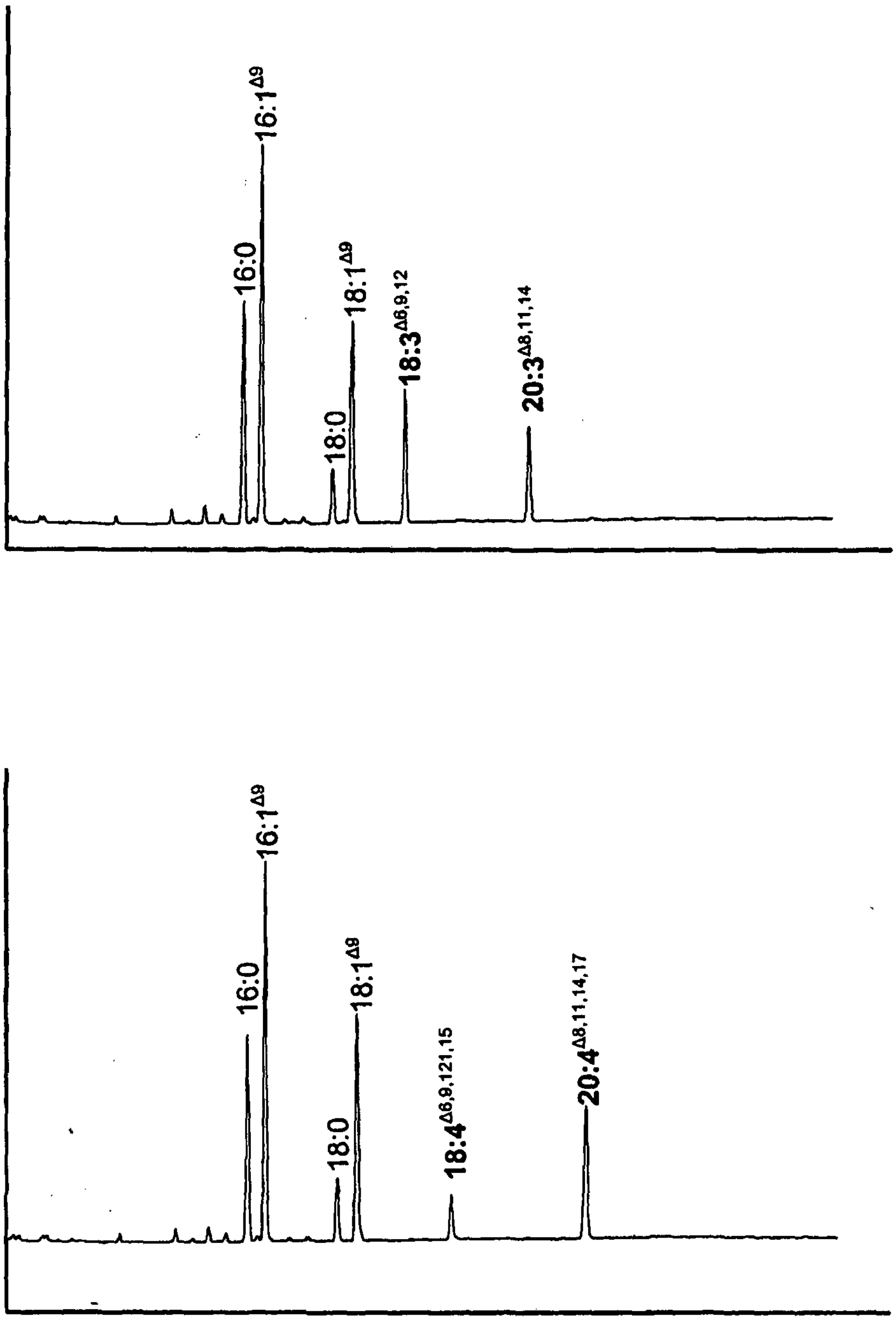

Figur 30: Figur 30 zeigt die Substratspezifität von PtELO6 in Bezug auf die gefütterten Substrate.

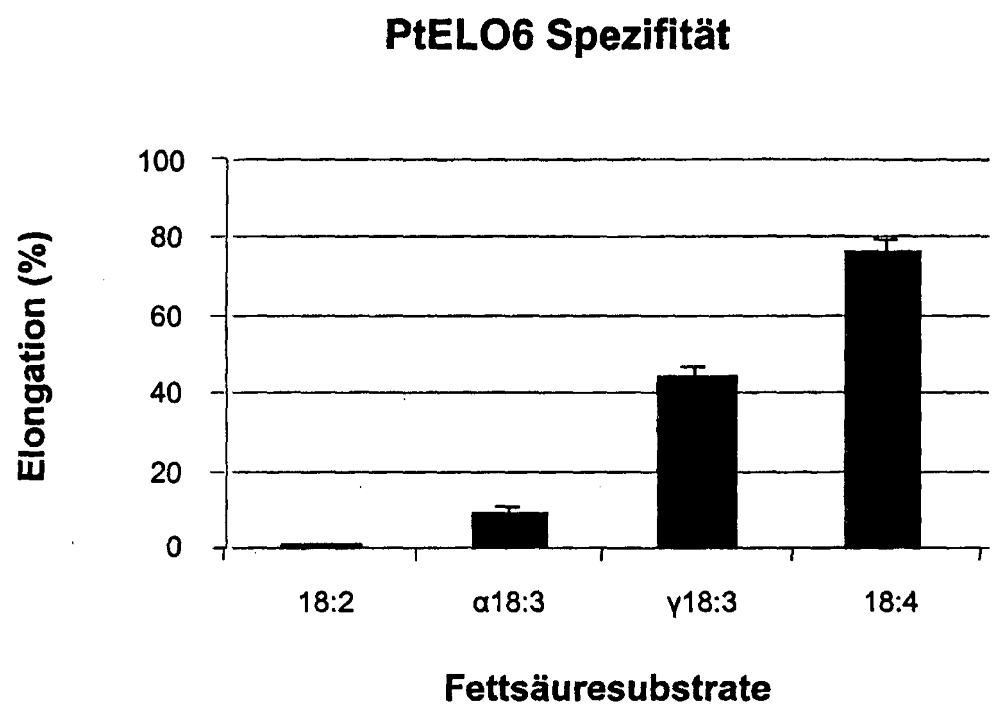

Figur 31: Gaschromatographische Analyse des Samens einer transgenen Pflanze, transformiert mit pSUN-5G.

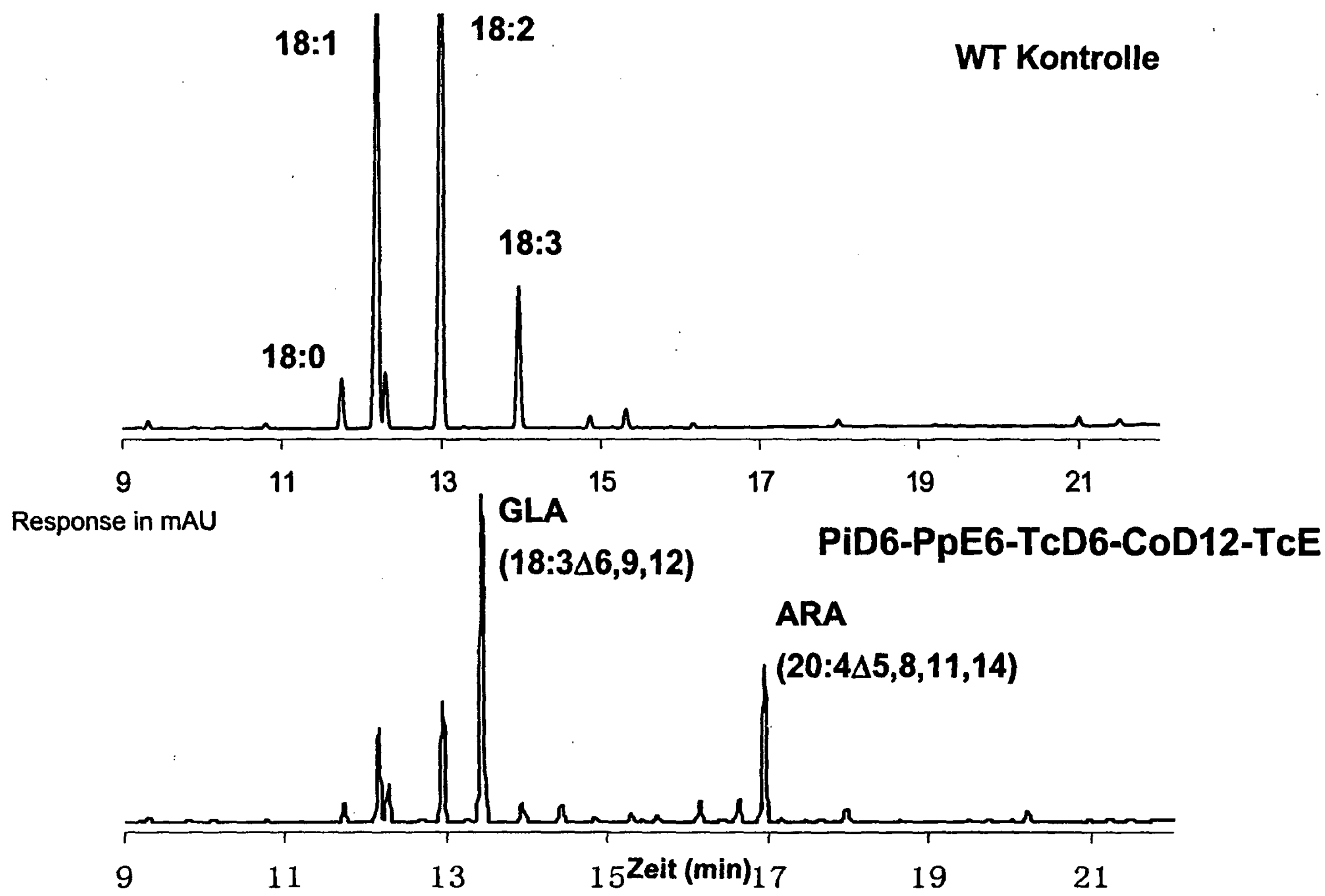

Figur 32: Gaschromatographische Analyse des Samens einer transgenen Pflanze, transformiert mit pGPTV-D6Des(Pir)_D5Des(Tc)_D6Elo(PP)_12Des(Co).

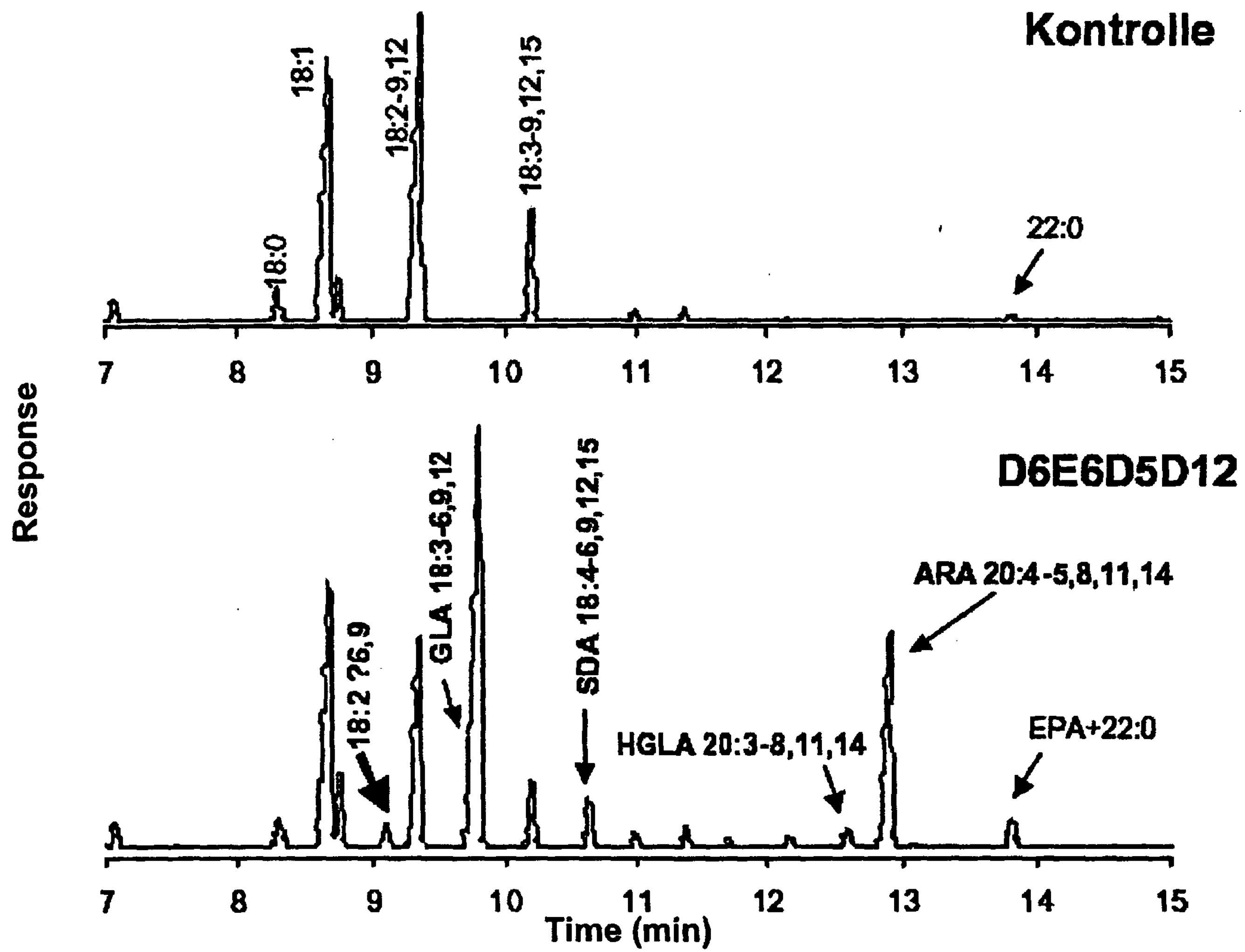

Figur 33: DHA in transgenen Samen von Brassica juncea. Die Pflanzen wurden mit dem Konstrukt pSUN-8G transformiert.

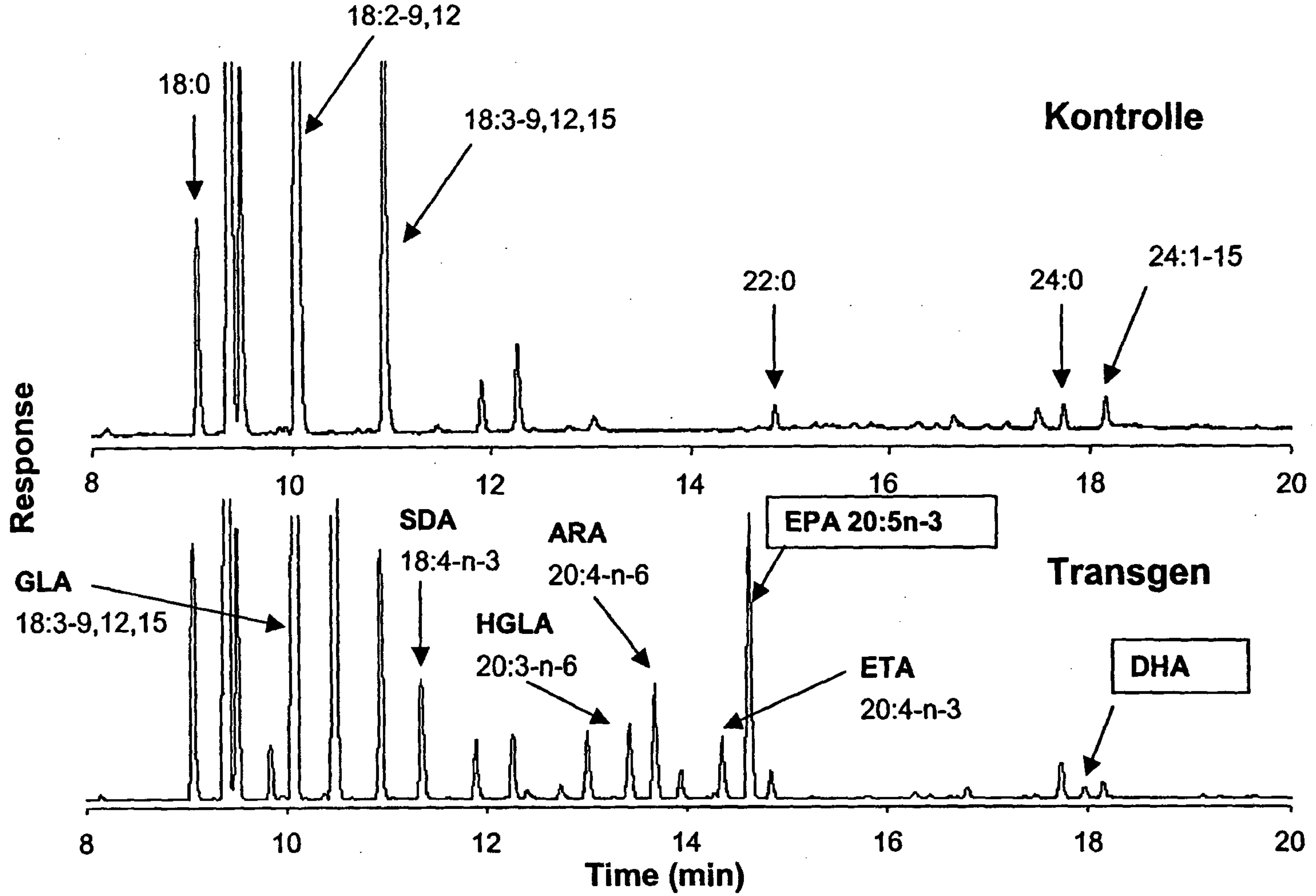

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9113972 A
- WO 9311245 A
- WO 9411516 A
- EP 0550162 A
- WO 9418337 A
- WO 9730582 A
- WO 9721340 A
- WO 9518222 A
- EP 0794250 A
- WO 9306712 A
- US 5614393 A
- WO 9621022 A
- WO 0021557 A
- WO 9927111 A
- WO 9846763 A
- WO 9846764 A
- WO 9846765 A
- WO 9964616 A
- WO 9846776 A
- WO 0159128 A
- WO 0012720 A
- WO 02077213 A
- WO 0208401 A
- WO 0244320 A
- DE 10219203
- WO 2004071467 A
- DE 10219203 A
- WO 0226946 A
- WO 0185968 A
- WO 9916890 A
- US 5608152 A
- WO 02102970 A
- US 5315001 A
- WO 9218634 A
- WO 9845461 A
- WO 9320216 A
- US 5504200 A
- WO 9113980 A
- WO 9515389 A
- WO 9523230 A
- US 5677474 A
- US 5530149 A
- EP 571741 A
- JP 6062870 A
- WO 9808962 A
- US 5689040 A
- EP 781849 A
- WO 9519443 A
- DE 10102337
- DE 10102338
- US 5352605 A
- WO 8402913 A
- US 4962028 A
- US 5187267 A
- WO 9612814 A
- EP 0375091 A
- WO 9516783 A
- WO 9706250 A
- WO 9946394 A
- US 5565350 A
- WO 0015815 A
- EP 0388186 A
- EP 0335528 A
- WO 9321334 A
- EP 0249676 A
- WO 9801572 A
- DE 10205607


### In der Beschreibung aufgeführte Nicht-Patentliteratur


- **F.C. NEIDHARDT et al.** E. coli und Salmonella. ASM Press, 1996, 612-636
- Biology of Procaryotes. Thieme, 1999
- **MAGNUSON, K. et al.** *Microbiological Reviews,* 1993, vol. 57, 522-542
- **FRENTZEN.** *Lipid,* 1998, vol. 100 (4-5), 161-166
- **KINNEY.** Genetic Engeneering. 1997, vol. 19, 149-166
- **OHLROGGE ; BROWSE.** *Plant Cell,* 1995, vol. 7, 957-970
- **SHANKLIN ; CAHOON.** *Annu. Rev. Plant Physiol. Plant Mol. Biol.,* 1998, vol. 49, 611-641
- **VOELKER.** Genetic Engeneering. 1996, vol. 18, 111-13
- **GERHARDT.** *Prog. Lipid R.,* 1992, vol. 31, 397-417
- **GÜHNEMANN-SCHÄFER ; KINDL.** *Biochim. Biophys Acta,* 1995, vol. 1256, 181-186
- **KUNAU et al.** *Prog. Lipid Res.,* 1995, vol. 34, 267-342

- **STYMNE et al.** Biochemistry and Molecular Biology of Membrane and Storage Lipids of Plants. American Society of Plant Physiologists, 1993, 150-158
- **MURPHY ; ROSS.** *Plant Journal,* 1998, vol. 13 (1), 1-16
- **SHIMIKAWA.** *World Rev. Nutr. Diet.,* 2001, vol. 88, 100-108
- **CALDER.** *Proc. Nutr. Soc.,* 2002, vol. 61, 345-358
- **CLELAND ; JAMES.** *J. Rheumatol.,* 2000, vol. 27, 2305-2307
- **POULOS, A LIPIDS.** *Krebs und Diabetes umfassen,* 1995, vol. 30, 1-14
- *Horrocks, LA und Yeo YK Pharmacol Res,* 1999, vol. 40, 211-225
- **STUKEY et al.** *J. Biol. Chem.,* 1990, vol. 265, 20144-20149
- **WADA et al.** *Nature,* 1990, vol. 347, 200-203
- **HUANG et al.** *Lipids,* 1999, vol. 34, 649-659
- **MCKEON et al.** *Methods in Enzymol.,* 1981, vol. 71, 12141-12147
- **WANG et al.** *Plant Physiol. Biochem.,* 1988, vol. 26, 777-792
- **MILLAR ; KUNST.** *Plant Journal,* 1997, vol. 12, 121-131
- **MILLAR et al.** *Plant Cell,* 1999, vol. 11, 825-838
- **TVRDIK et al.** *J. Cell Biol.,* 2000, vol. 149, 707-718
- **R. VAZHAPPILLY ; F. CHEN.** *Botanica Marina,* 1998, vol. 41, 553-558
- **K. TOTANI ; K. OBA.** *Lipids,* 1987, vol. 22, 1060-1062
- **M. AKIMOTO et al.** *Appl. Biochemistry and Biotechnology,* 1998, vol. 73, 269-278
- **E. UCCIANI.** Nouveau Dictionnaire des Huiles Végétales. *Technique & Documentation - Lavoisier,* 1995, ISBN 2-7430-0009-0
- **YU, R. et al.** *Lipids,* 2000, vol. 35, 1061-1064
- **TAKEYAMA, H. et al.** *Microbiology,* 1997, vol. 143, 2725-2731
- **ZANK, T.K. et al.** *Plant Journal,* 2002, vol. 31, 255-268
- **SAKURADANI, E. et al.** *Gene,* 1999, vol. 238, 445-453
- **SPRECHER.** *Biochim. Biophys. Acta,* 2000, vol. 1486, 219-231
- **TOCHER et al.** *Prog. Lipid Res.,* 1998, vol. 37, 73-117
- **DOMERGUE et al.** *Eur. J. Biochem.,* 2002, vol. 269, 4105-4113
- **MIKOKLAJCZAK et al.** *Journal of the American Oil Chemical Society,* 1961, vol. 38, 678-681
- *J. Mol. Evolution.,* 1987, vol. 25, 351-360
- **HIGGINS et al.** *CABIOS,* 1989, vol. 5, 151-153
- **NEEDLEMAN ; WUNSCH.** *J. Mol. Biol.,* 1970, vol. 48, 443-453
- **SMITH ; WATERMAN.** *Adv. Appl. Math.,* 1981, vol. 2, 482-489
- *Genetics Computer Group, 575 Science Drive, Madison, Wisconsin,* 1991, vol. 575, 53711
- Current Protocols in Molecular Biology. John Wiley & Sons, 1989, 6.3.1-6.3.6
- **SAMBROOK et al.** Molecular Cloning. Cold Spring Harbor Laboratory, 1989
- Nucleic Acids Hybridization: A Practical Approach. IRL Press at Oxford University Press, 1985
- Essential Molecular Biology: A Practical Approach. IRL Press at Oxford University Press, 1991
- **HELLENS et al.** *Trends in Plant Science,* 2000, vol. 5, 446-451
- Transformation von Pflanzen verwendet werden, wie denjenigen, die veröffentlicht und dort zitiert sind. Plant Molecular Biology and Biotechnology. CRC Press, 1993, 71-119
- Vectors for Gene Transfer in Higher Plants. **F.F. WHITE.** Transgenic Plants, Bd. 1, Engineering and Utilization. Academic Press, 1993, vol. 1, 15-38
- Techniques for Gene Transfer. **B. JENES et al.** Transgenic Plants, Bd. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BÄUMLEIN et al.** *Mol. Gen Genet.,* 1991, vol. 225 (3
- **BÄUMLEIN et al.** *Plant J.,* 1992, vol. 2,2
- **GATZ.** *Annu. Rev. Plant Physiol. Plant Mol. Biol.,* 1997, vol. 48, 89-108
- **GATZ et al.** *Plant J.,* 1992, vol. 2, 397-404
- Gene Expression Technology. **GOEDDEL.** Methods in Enzymology. Academic Press, 1990, vol. 185
- **GRUBER ; CROSBY.** Methods in Plant Molecular Biology and Biotechnolgy. CRC Press, vol. 7, 89-108
- **FALCIATORE et al.** *Marine Biotechnology,* 1999, vol. 1 (3), 239-251
- **BECKER, D. ; KEMPER, E. ; SCHELL, J. ; MASTERSON, R.** New plant binary vectors with selectable markers located proximal to the left border. *Plant Mol. Biol.,* 1992, vol. 20, 1195-1197
- **BEVAN, M.W.** Binary Agrobacterium vectors for plant transformation. *Nucl. Acids Res.,* 1984, vol. 12, 8711-8721
- Vectors for Gene Transfer in Higher Plants. Transgenic Plants, Bd. 1, Engineering and Utilization. Academic Press, 1993, vol. 1, 15-38
- **GIELEN et al.** *EMBO J.,* 1984, vol. 3, 835ff

- **GALLIE et al.** *Nucl. Acids Research,* 1987, vol. 15, 8693-8711
- **BENFEY et al.** *EMBO J.,* 1989, vol. 8, 2195-2202
- **FRANCK et al.** *Cell,* 1980, vol. 21, 285-294
- **WARD et al.** *Plant. Mol. Biol.,* 1993, vol. 22, 361-366
- **BAEUMLEIN et al.** *Mol Gen Genet,* 1991, vol. 225 (3), 459-67
- **BAEUMLEIN et al.** *Plant Journal,* 1992, vol. 2 (2), 233-9
- **KERMODE.** *Crit. Rev. Plant Sci.,* 1996, vol. 15 (4), 285-423
- **GOEDDEL.** Gene Expression Technology: Methods in Enzymology. Academic Press, 1990, vol. 185
- **GOTTESMAN, S.** Gene Expression Technology: Methods in Enzymology. Academic Press, 1990, vol. 185, 119-128
- *Genetics Computer Group, 575 Science Drive, Madison, Wisconsin,* 1991, 53711
- **WARD et al.** *Plant. Mol. Biol.,* 1993, vol. 22
- **GATZ.** *Plant J.,* 1992, vol. 2, 397-404
- **STOCKHAUS et al.** *EMBO J.,* 1989, vol. 8, 2445
- **BAEUMLEIN et al.** *Plant J.,* 1992, vol. 2 (2), 233-239
- **ROMANOS, M.A. et al.** Foreign gene expression in yeast: a review. *Yeast,* 1992, vol. 8, 423-488
- Heterologous gene expression in filamentous fungi. **VAN DEN HONDEL, C.A.M.J.J. et al.** More Gene Manipulations in Fungi. Academic Press, 1991, 396-428
- Gene transfer systems and vector development for filamentous fungi. **VAN DEN HONDEL, C.A.M.J.J. ; PUNT, P.J. et al.** Applied Molecular Genetics of Fungi. Cambridge University Press, 1991, 1-28
- **SCHMIDT, R. ; WILLMITZER, L.** High efficiency Agrobacterium tumefaciens-mediated transformation of Arabidopsis thaliana leaf and cotyledon explants. *Plant Cell Rep.,* 1988, 583-586
- Plant Molecular Biology and Biotechnology. C Press, 1993, 71-119
- Techniques for Gene Transfer. **F.F. WHITE ; B. JENES et al.** Transgenic Plants, Bd. 1, Engineering and Utilization. Academic Press, 1993, vol. 1, 128-43
- **SMITH, D.B. ; JOHNSON, K.S.** Gene. Pharmacia Biotech Inc, 1988, vol. 67, 31-40
- **AMANN et al.** *Gene,* 1988, vol. 69, 301-315
- Gene Expression Technology. **STUDIER et al.** Methods in Enzymology. Academic Press, 1990, vol. 185, 60-89
- **BALDARI et al.** *Embo J.,* 1987, vol. 6, 229-234
- **KURJAN ; HERSKOWITZ.** *Cell,* 1982, vol. 30, 933-943
- **SCHULTZ et al.** *Gene,* 1987, vol. 54, 113-123
- Gene transfer systems and vector development for filamentous fungi. **VAN DEN HONDEL, C.A.M.J.J. ; PUNT, P.J. et al.** Applied Molecular Genetics of fungi. Cambridge University Press, 1991, 1-28
- More Gene Manipulations in Fungi. Academic Press, 396-428
- **SMITH et al.** *Mol. Cell Biol.,* 1983, vol. 3, 2156-2165
- **LUCKLOW ; SUMMERS.** *Virology,* 1989, vol. 170, 31-39
- Cloning Vectors. Elsevier, 1985
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning: A Laboratory Manual. Cold Spring Harbor Laboratory Press, 1989
- Agrobacterium protocols. Methods in Molecular Biology. Humana Press, 1995, vol. 44
- **SAMBROOK et al.** Molecular Cloning: A Laboratory Manual. Cold Spring Harbor Laboratory Press, 1989
- **CHIRGWIN et al.** *Biochemistry,* 1979, vol. 18, 5294-5299
- **SANGER et al.** *Proc. Natl. Acad. Sci. USA,* 1977, vol. 74, 5463-5467
- **HAJDUKIEWICZ,P ; SVAB, Z ; MALIGA, P.** The small versatile pPZP family of Agrobacterium binary vectors forplant transformation. *Plant Mol Biol,* 1994, vol. 25, 989-994
- **DE GREVE,H. ; DHAESE,P. ; SEURINCK,J. ; LEMMERS,M. ; VAN MONTAGU,M. ; SCHELL,J.** Nucleotide sequence and transcript map of the Agrobacterium tumefaciens Ti plasmid-encoded octopine synthase gene. *J. Mol. Appl. Genet.,* 1982, vol. 1 (6), 499-511
- **ULLMAN.** Encyclopedia of Industrial Chemistry. VCH, 1985, vol. A2, S. 89-90, S. 443-613
- **FALLON, A. et al.** Applications of HPLC in Biochemistry. *Laboratory Techniques in Biochemistry and Molecular Biology,* 1987, vol. 17
- Product recovery and purification. **REHM et al.** Biotechnology. VCH, 1993, vol. 3, 469-714
- **BELTER, P.A. et al.** Bioseparations: downstream processing for Biotechnology. John Wiley and Sons, 1988
- **KENNEDY, J.F. ; CABRAL, J.M.S.** Recovery processes for biological Materials. John Wiley and Sons, 1992
- Biochemical Separations. **SHAEIWITZ, J.A. ; HENRY, J.D.** Ullmann's Encyclopedia of Industrial Chemistry. VCH, 1988, vol. B3, 1-27
- **DECHOW, F.J.** Separation and purification techniques in biotechnology. Noyes Publications, 1989

- **CAHOON et al.** *Proc. Natl. Acad. Sci. USA,* 1999, vol. 96 (22), 12935-12940
- **BROWSE et al.** *Analytic Biochemistry,* 1986, vol. 152, 141-145
- **CHRISTIE, WILLIAM W.** Advances in Lipid Methodology. Oily Press Lipid Library
- Gas Chromatography and Lipids. **CHRISTIE, WILLIAM W.** A Practical Guide. Oily Press Lipid Library, 1989, vol. IX, 307
- Progress in Lipid Research. Progress in the Chemistry of Fats and Other Lipids CODEN. Pergamon Press, 1952, vol. 1
- A Practical Approach. Applied Microbial Physiology. IRL Press, 103-129, 131-163, 165-192
- **CHRISTIE.** Advances on Lipid Methodology. Christie, Oily Press, 1997, 119-169
- Gaschromatographie-Massenspektrometrie-Verfahren. *Lipide,* 1998, vol. 33, 343-353
- **NAPIER ; MICHAELSON.** *Lipids,* 2001, vol. 36 (8), 761-766
- **SAYANOVA et al.** *Journal of Experimental Botany,* 2001, vol. 52 (360), 1581-1585
- **SPERLING et al.** *Arch. Biochem. Biophys.,* 2001, vol. 388 (2), 293-298
- **MICHAELSON et al.** *FEBS Letters,* 1998, vol. 439 (3), 215-218
- **DEBLAERE et al.** *Nucl. Acids. Res.,* 1984, vol. 13, 4777-4788
- **MURASHIGE ; SKOOG.** *Physiol. Plant.,* 1962, vol. 15, 473
- **BELL et al.** *In Vitro Cell. Dev. Biol.-Plant.,* 1999, vol. 35 (6), 456-465
- **MLYNAROVA et al.** *Plant Cell Report,* 1994, vol. 13, 282-285
- **ALTSCHUL et al.** *J. Mol. Biol.,* 1990, vol. 215, 403-410
- **KOZAK.** *Cell,* 1986, vol. 44, 283-292
- **HAJDUKIEWICZ,P ; SVAB, Z ; MALIGA, P.** The small versatile pPZP family of Agrobacterium binary vectors for plant transformation. *Plant Mol Biol,* 1994, vol. 25, 989-994
- Culture of phytoplankton for feeding marine invertebrates. **GUILLARD, R.R.L.** Culture of Marine Invertebrate Animals. Plenum Press, 1975, 29-60
- **HIS-BOXEN, DOMERGUE et al.** *Eur. J. Biochem.,* 2002, vol. 269, 4105-4113
- **QIU et al.** *J. Biol. Chem.,* 2001, vol. 276, 31561-31566
- **HONG et al.** *Lipids,* 2002, vol. 37, 863-868
- **ZANK et al.** *Plant J.,* 2002, vol. 31, 255-268
- Genetic and genomic tools for Xenopus research: The NIH Xenopus initiative. *Dev. Dyn.,* 2002, vol. 225 (4), 384-391
- **SATOU,Y. ; YAMADA,L. ; MOCHIZUKI,Y. ; TAKATORI,N. ; KAWASHIMA,T. ; SASAKI,A. ; HAMAGUCHI,M. ; AWAZU,S. ; YAGI,K. ; SASAKURA,Y.** A cDNA resource from the basal chordate Ciona intestinalis. *JOURNAL Genesis,* 2002, vol. 33 (4), 153-154
- **ALTSCHUL et al.** *J. Mol. Biol.,* 1990, vol. 215, 403-410
- **CALVAYRAC R ; DOUCE R.** *FEBS Letters,* 1970, vol. 7, 259-262
- **DE GREVE, H. ; DHAESE, P. ; SEURINCK, J. ; LEMMERS, M. ; VAN MONTAGU, M. ; SCHELL, J.** Nucleotide sequence and transcript map of the Agrobacterium tumefaciens Ti plasmid-encoded octopine synthase gene. *J. Mol. Appl. Genet.,* 1982, vol. 1 (6), 499-511
- **ULLMAN.** Encyclopedia of Industrial Chemistry. VCH, 1985, vol. A2, S.89-90, S.443-613
- Applied Microbial Physiology. A Practical Approach. IRL Press, 103-129, 131-163, 165-192
- **CHRISTIE.** Advances on Lipid Methodology. Oily Press, 1997, 119-169